# EUROPEAN PATENT SPECIFICATION

(11) **EP 3 930 849 B1**
(45) Date of publication and mention of the grant of the patent: **19.02.2025**
(21) Application number: 20715580.5
(22) Date of filing: 28.02.2020
(51) Int. Cl.: A61P 35/00, C07D 277/18, C07D 401/06, C07D 413/14, C07D 417/12, C07D 417/14, A61K 31/427

(54) **THIAZOLE DERIVATIVES AS PROTEIN SECRETION INHIBITORS**
THIAZOLDERIVATE ALS PROTEINSEKRETIONSINHIBITOREN
DÉRIVÉS DE THIAZOLE EN TANT QU'INHIBITEURS DE SÉCRÉTION DE PROTÉINES

(30) Priority: 28.02.2019 US 201962812013 P
(43) Date of publication of application: 05.01.2022
(73) Proprietor: Kezar Life Sciences, South San Francisco, CA 94080 (US)
(72) Inventor: MCMINN, Dustin, Pacifica, CA 94044 (US); RAO, Meera, San Francisco, CA 94110 (US)
(74) Representative: Mewburn Ellis LLP
(86) International application number: PCT/US2020/020419
(87) International publication number: WO 2020/176863

(56) References cited:
- WO-A1-03/015778
- WO-A1-2005/095386
- WO-A1-2010/101949
- WO-A1-2010/126914
- WO-A2-2014/145642
- VAN PUYENBROECK VICTOR ET AL: "Inhibitors of protein translocation across membranes of the secretory pathway: novel antimicrobial and anticancer agents", CMLS CELLULAR AND MOLECULAR LIFE SCIENCES, BIRKHAUSER VERLAG, HEIDELBERG, DE, vol. 75, no. 9, 5 January 2018 (2018-01-05), pages 1541 - 1558, XP036478836, ISSN: 1420-682X, [retrieved on 20180105], DOI: 10.1007/S00018-017-2743-2
- JUN ZUO ET AL: "Discovery of Structurally Diverse Small-Molecule Compounds with Broad Antiviral Activity against Enteroviruses", ANTIMICROBIAL AGENTS AND CHEMOTHERAPY, vol. 60, no. 3, 1 March 2016 (2016-03-01), US, pages 1615 - 1626, XP055694837, ISSN: 0066-4804, DOI: 10.1128/AAC.02646-15
- KAI-UWE KALIES ET AL: "Inhibitors of Protein Translocation Across the ER Membrane : ER Protein Translocation Inhibitors", TRAFFIC, vol. 16, no. 10, 27 July 2015 (2015-07-27), DK, pages 1027 - 1038, XP055694929, ISSN: 1398-9219, DOI: 10.1111/tra.12308
- DATABASE REGISTRY [online] CHEMICAL ABSTRACTS SERVICE, COLUMBUS, OHIO, US; 1 May 2011 (2011-05-01), XP002799007, Database accession no. 1287868-13-6
- DATABASE REGISTRY [online] CHEMICAL ABSTRACTS SERVICE, COLUMBUS, OHIO, US; 27 May 2011 (2011-05-27), XP002799008, Database accession no. 1301352-46-4
- DATABASE REGISTRY [online] CHEMICAL ABSTRACTS SERVICE, COLUMBUS, OHIO, US; 10 November 2010 (2010-11-10), XP002799009, Database accession no. 1252203-85-2
- DATABASE REGISTRY [online] CHEMICAL ABSTRACTS SERVICE, COLUMBUS, OHIO, US; 12 April 2011 (2011-04-12), XP002799010, Database accession no. 1278765-37-9
- DATABASE REGISTRY [online] CHEMICAL ABSTRACTS SERVICE, COLUMBUS, OHIO, US; 18 November 2018 (2018-11-18), XP002799011, Database accession no. 2249352-34-7
- DATABASE REGISTRY [online] CHEMICAL ABSTRACTS SERVICE, COLUMBUS, OHIO, US; 24 May 2011 (2011-05-24), XP002799012, Database accession no. 1299339-46-0

## Description

### BACKGROUND

### Field of the Invention

The present disclosure relates to compounds and their use in treating cancer, inflammation, an autoimmune disease, an immune-related disease, a neurodegenerative disease or an inflammatory disease.

### Description of Related Technology

Protein translocation into the endoplasmic reticulum ("ER") constitutes the first step of protein secretion. ER protein import is essential in all eukaryotic cells and is particularly important in fast-growing tumor cells. Thus, the process of protein secretion can serve as a target both for potential cancer drugs and for bacterial virulence factors. See Kalies and Römisch, Traffic, 16(10):1027-1038 (2015).

Protein transport to the ER is initiated in the cytosol when N-terminal hydrophobic signal peptides protrude from the ribosome. Binding of signal recognition particle ("SRP") to the signal sequence allows targeting of the ribosome-nascent chain-SRP complex to the ER membrane where contact of SRP with its receptor triggers handing over of the signal peptide to Sec61. Sec61 is an ER membrane protein translocator (aka translocon) that is doughnut-shaped with 3 major subunits (heterotrimeric). It includes a "plug," which blocks transport into or out of the ER. The plug is displaced when the hydrophobic region of a nascent polypeptide interacts with the "seam" region of Sec61, allowing translocation of the polypeptide into the ER lumen. In mammals, only short proteins (<160 amino acids) can enter the ER posttranslationally, and proteins smaller than 120 amino acids are obliged to use this pathway. Some of the translocation competence is maintained by the binding of calmodulin to the signal sequence. Upon arrival at the Sec61 channel, the signal peptide or signal anchor intercalates between transmembrane domains ("TMDs") 2 and 7 of Sec61α, which form the lateral portion of the gate, allowing the channel to open for soluble secretory proteins. As the Sec61 channel consists of 10 TMDs (Sec61α) surrounded by a hydrophobic clamp formed by Sec61γ, channel opening is dependent on conformational changes that involve practically all TMDs.

Inhibition of protein transport across the ER membrane has the potential to treat or prevent diseases, such as the growth of cancer cells and inflammation. Known secretion inhibitors, which range from broad-spectrum to highly substrate-specific, can interfere with virtually any stage of this multistep process, and even with transport of endocytosed antigens into the cytosol for cross-presentation. These inhibitors interact with the signal peptide, chaperones, or the Sec61 channel to block substrate binding or to prevent the

conformational changes needed for protein import into the ER. Examples of protein secretion inhibitors include, calmodulin inhibitors (e.g., E6 Berbamine and Ophiobolin A), Lanthanum, sterols, cyclodepsipeptides (e.g., HUN-7293, CAM741, NFI028, Cotrainsin, Apratoxin A, Decatransin, Valinomycin), CADA, Mycolactone, Eeyarestatin I ("ESI"), and Exotoxin A. However, the above secretion inhibitors suffer from one or more of the following: lack selectivity for the Sec61 channel, challenging manufacture due to structural complexity, and molecular weight limited administration, bio-availability and distribution.

Kalies et al, Traffic 2015, vol. 16, p. 1027-1038 discloses small compounds as protein secretion inhibitors.

Thus, a need exits for new inhibitors of protein secretion.

### SUMMARY

Provided herein are compounds having a structure of Formula (III), or a pharmaceutically acceptable salt thereof: 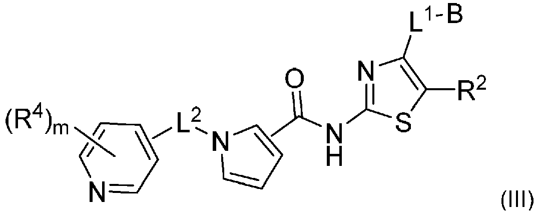 wherein L¹ is a bond, C₁₋₆alkylene, or 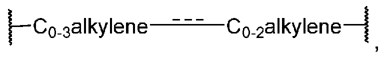 , wherein - - - indicates a double bond, a triple bond, or a fused cyclopropyl; B is C₀₋₃alkylene-X; X is an aromatic or nonaromatic C₄₋₁₀carbocycle, or an aromatic or nonaromatic 4-10 membered heterocycle having 1-3 ring heteroatoms selected from N, O, and S; X is substituted with 1-3 G; each G is independently selected from the group consisting of halo, OH, =O, CN, NO₂, N(R^{N})₂, N(R^{N})C(O)C₁₋₃alkyl, C₁₋₃alkyl, C₁₋₃alkoxy, C₁₋₃ haloalkyl, C₁₋₃ haloalkoxy, C(O)C₁₋₃alkyl, S(O₂)-Z, C(O)-Z, C(O)N(R^{N})₂, silyl ether, and [O]₀₋₁-C₀₋₃alkylene-Z; each R^{N} independently is H or C₁₋₄alkyl; Z is aromatic or nonaromatic C₃₋₁₀carbocycle, or aromatic or nonaromatic 4-10 membered heterocycle having 1-3 heteroatoms selected from the group consisting of N, O, and S; Z is optionally substituted with 1-3 E; and, each E independently is selected from C₁₋₃alkyl, C₁₋₃alkoxy, =O, C₁₋₃haloalkoxy, CN, and halo; R² is H; L² is C₀₋₃alkylene; m is 0 to 2; and each R⁴ independently is C₁₋₃alkyl, C₂₋₃alkynyl, C₁₋₃haloalkyl, C₁₋₃alkoxy, halo, or NHC₁₋₃alkylene-aryl; or a pharmaceutically acceptable salt thereof wherein the compound or salt thereof is not N-[4-[(3-methyl-1-piperidinyl)methyl]2-thiazolyl)-1-(4-pyridinylmethyl)-1H-pyrrole-2-carboxamide.

In various embodiments, L¹ is a bond. In various embodiments, L¹ is C₁₋₆alkylene. In some cases, L¹ is CH₂, CH₂CH₂, C(CH₃)₂, C(CH₃)₂CH₂, or C(CH₃)₂CH₂CH₂. In various embodiments, L¹ is 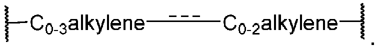 In some cases, L¹ is 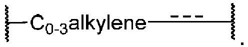 In some cases, L¹ is 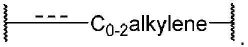 In some cases, L¹ is 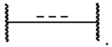 In various embodiments, - - - indicates a double bond. In some cases, the double bond is further substituted with C₁₋₃alkyl. In various embodiments, - - - indicates a triple bond. In various embodiments, - - - indicates a fused cyclopropyl, e.g.,

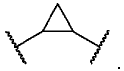

In various embodiments, B is C₁₋₃alkylene-X. In various embodiments, B is X. In various embodiments, X is pyrrolidinyl, cyclobutyl, cyclopentyl, cyclohexyl, cyclohexenyl, phenyl, piperidinyl, pyridinyl, piperazinyl, tetrahydrofuranyl, furanyl, tetrahydropyranyl, quinolinyl, morpholinyl, pyrrolidonyl, pyrimidinyl, pyridazinyl, indenyl, dihydroindenyl, dihydrobenzofuranyl, chromanyl, isochromanyl, dihydroisoquinolinyl, or indolyl.

In various embodiments, L¹-B is selected from the group consisting of 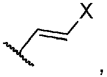 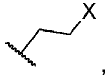 and 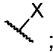 X is aromatic or nonaromatic C₄₋₇ carbocycle, or an aromatic or nonaromatic 4-9 membered heterocycle having 1 ring heteroatom; X is optionally substituted with 1-3 G; each G independently is selected from the group consisting of halo, OH, =O, CN, NO₂, N(R^{N})₂, N(R^{N})C(O)C₁₋₃alkyl, C₁₋₃alkyl, C₁₋₃alkoxy, C₁₋₃ haloalkyl, C₁₋₃ haloalkoxy, C(O)C₁₋₃alkyl, S(O₂)-Z, C(O)-Z, C(O)N(R^{N})₂, silyl ether, and [O]₀₋₁-C₀₋₃alkylene-Z; each R^{N} independently is H or C₁₋₄alkyl; Z is aromatic or nonaromatic C₃₋₁₀carbocycle, or aromatic or nonaromatic 4-10 membered heterocycle having 1-3 heteroatoms selected from the group consisting of N, O, and S; Z is optionally substituted with 1-3 E; and, each E independently is selected from C₁₋₃alkyl, C₁₋₃alkoxy, =O, C₁₋₃haloalkoxy, CN, and halo.

In some cases, L¹-B is selected from the group consisting of 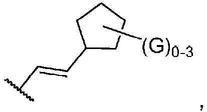 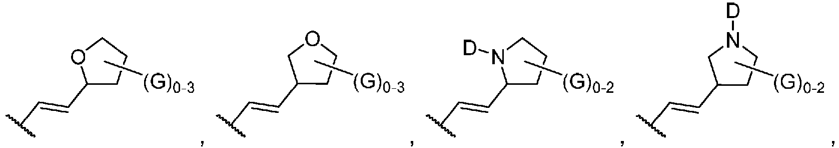 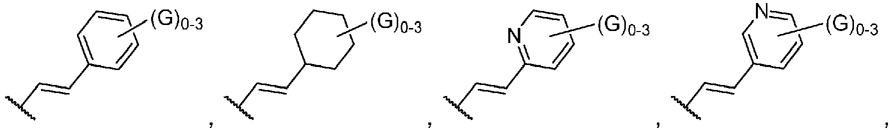 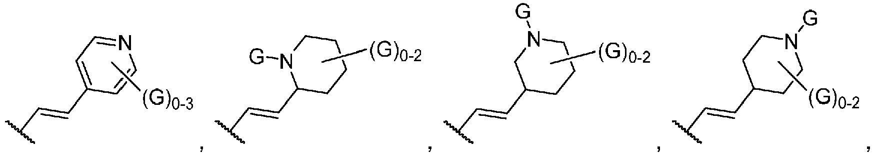 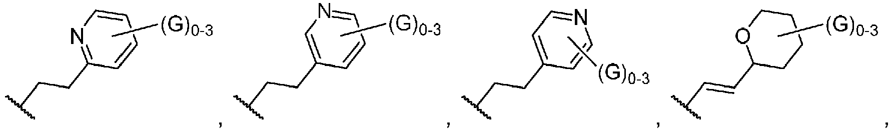 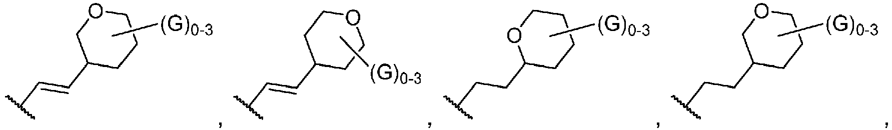 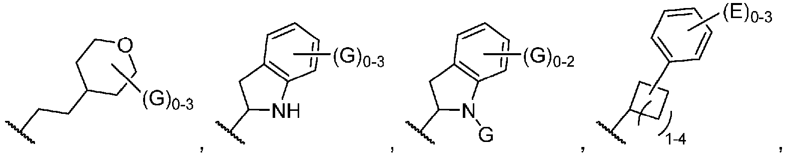 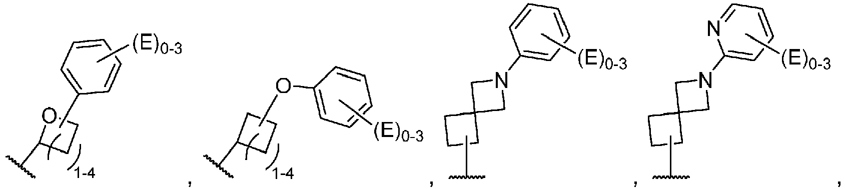 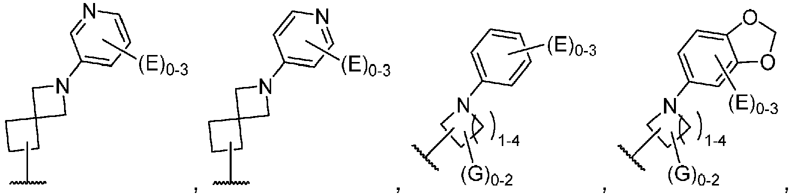 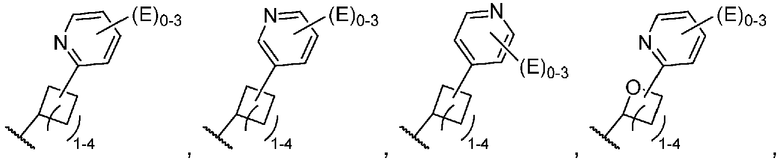 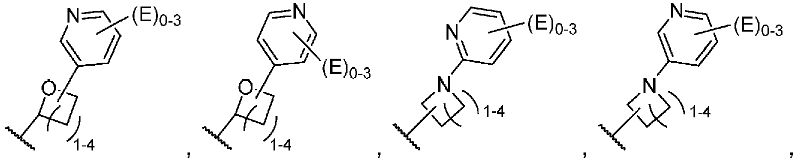 and

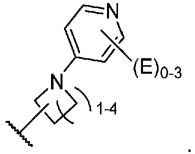

In various embodiments, L¹-B is selected from the group consisting of

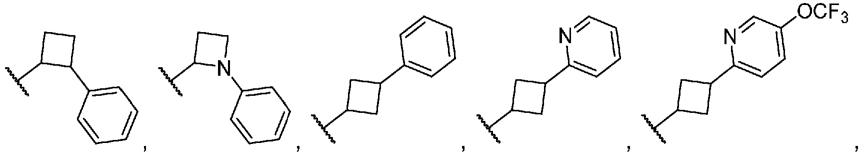

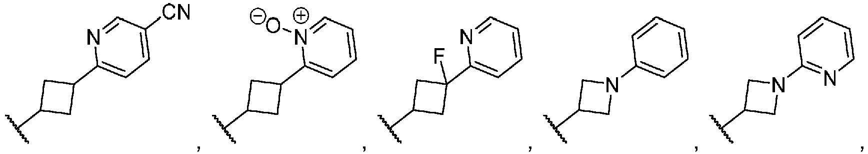

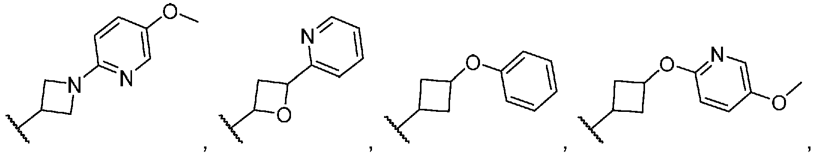

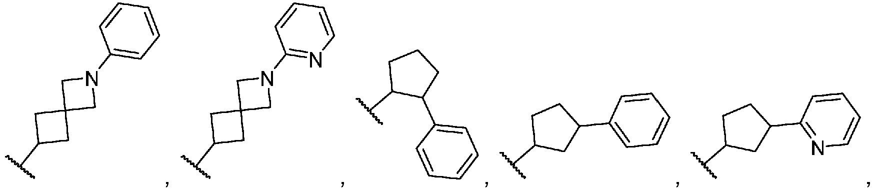

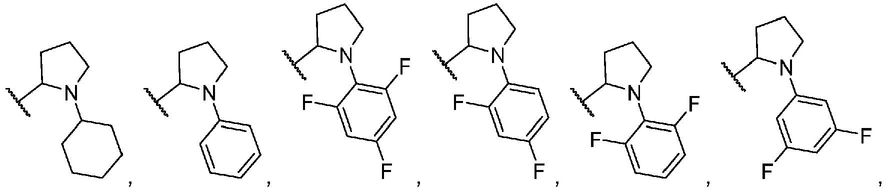

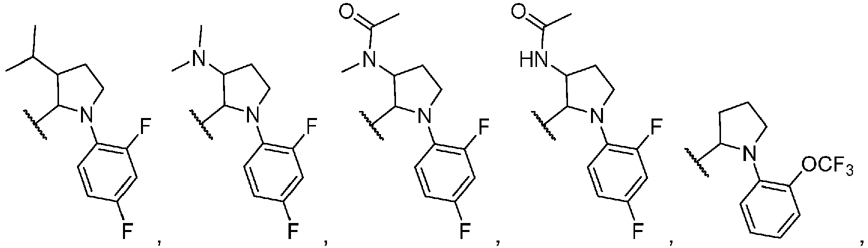

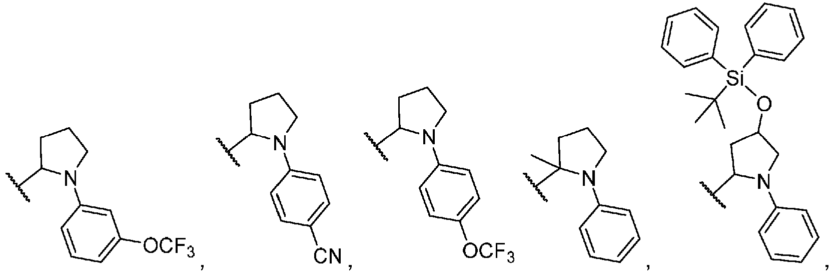

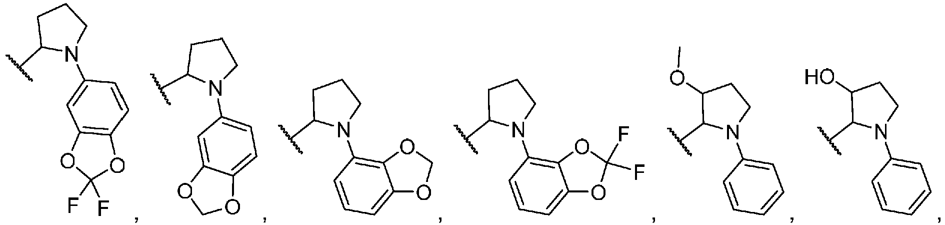

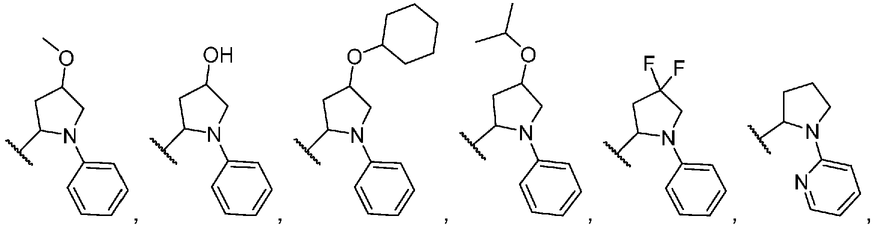

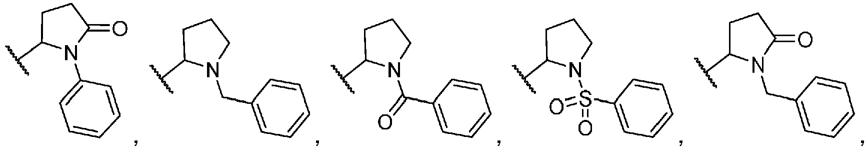

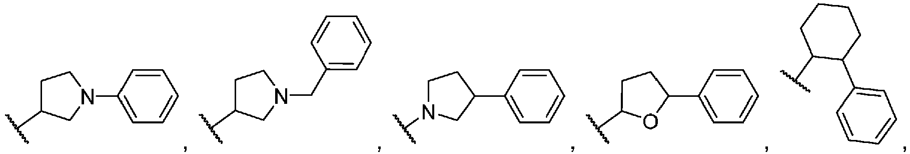

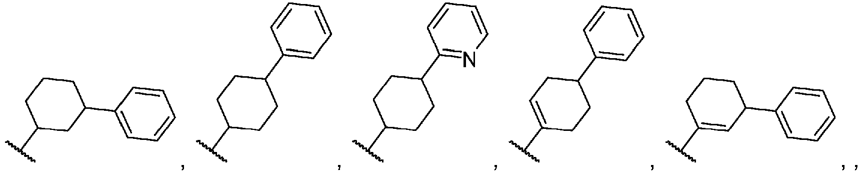

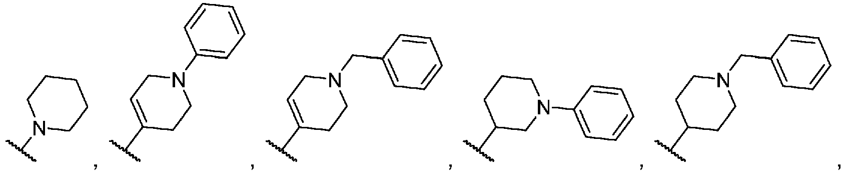

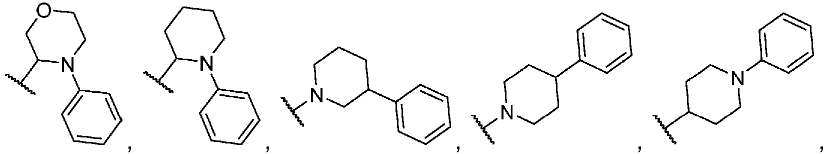

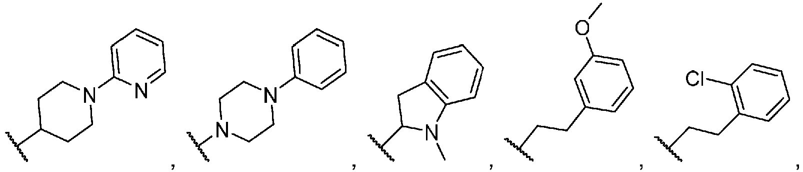

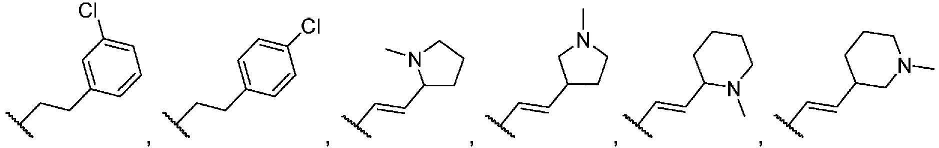

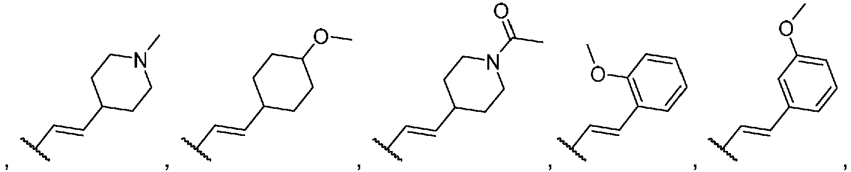

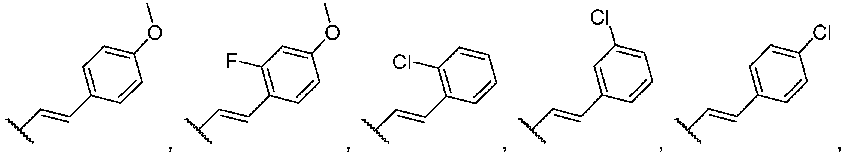

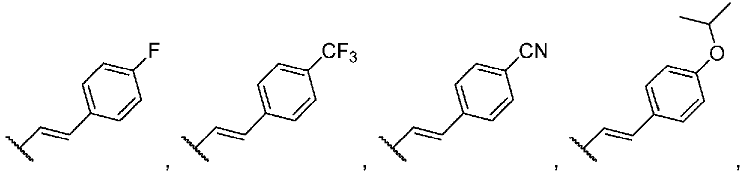

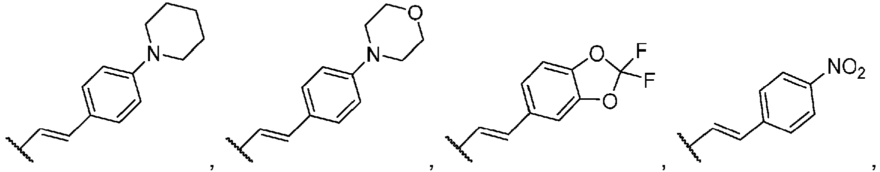

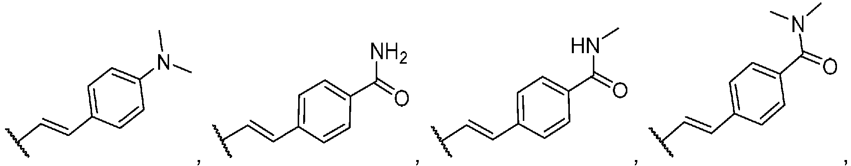

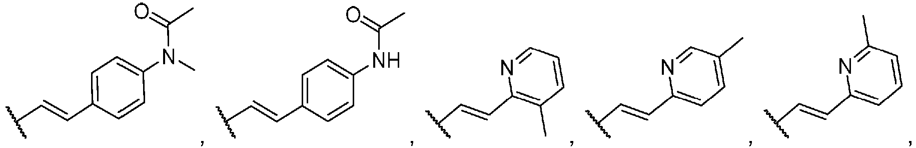

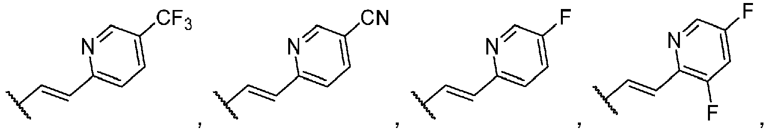

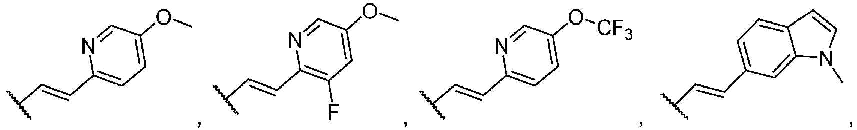

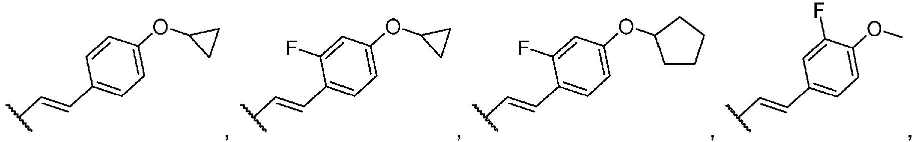

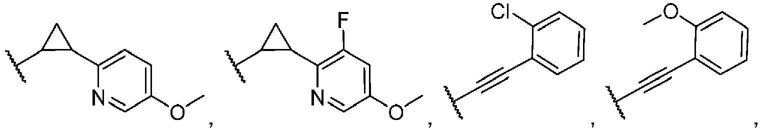

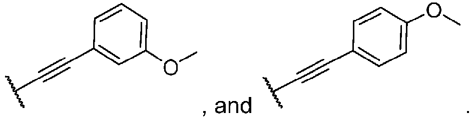

In all embodiments, R² is H.

In various embodiments, L² is C₀alkylene. In various embodiments, L² is C₁alkylene. In various embodiments, L² is C₂alkylene. In various embodiments, L² is C₃alkylene.

In various embodiments, m is 0. In various embodiments, m is 1 or 2.

In various embodiments, R⁴ is C₁₋₃alkyl. In some cases, R⁴ is methyl or ethyl. In various embodiments, R⁴ is halo. In some cases, R⁴ is F. In some cases, R⁴ is Cl. In various embodiments, R⁴ is C₂₋₃alkynyl. In some cases, R⁴ is C₂alkynyl. In various embodiments, R⁴ is C₁₋₃haloalkyl. In some cases R⁴ is CF₃. In various embodiments, R⁴ is C₁₋₃alkoxy. In some cases, R⁴ is methoxy. In various embodiments, R⁴ is NHC₁₋₃alkylene-aryl. In some cases, R⁴ is NH-CH₂-phenyl.

In various embodiments, m is 2, and one R⁴ is halo, and the other R⁴ is halo or methyl.

In various embodiments, the compound or salt has a structure of Formula (IIIA):

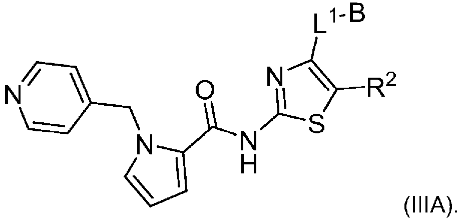

In various embodiments, L¹-B is selected from the group consisting of

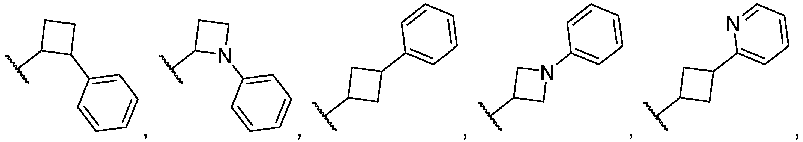

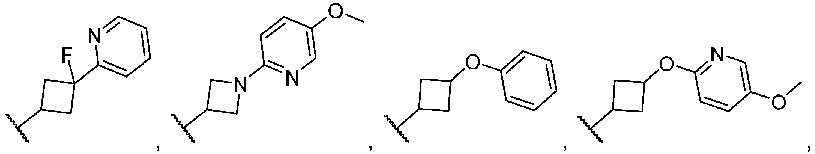

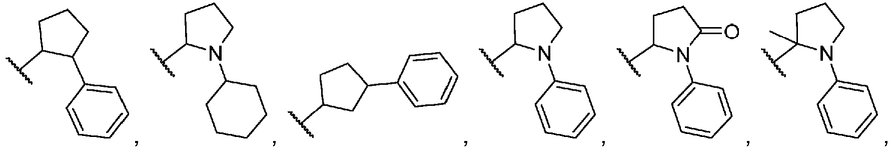

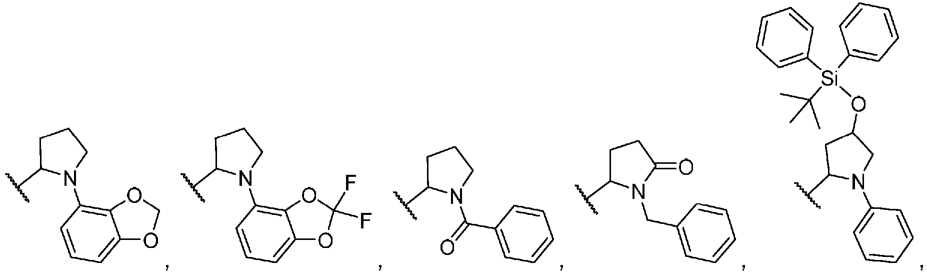

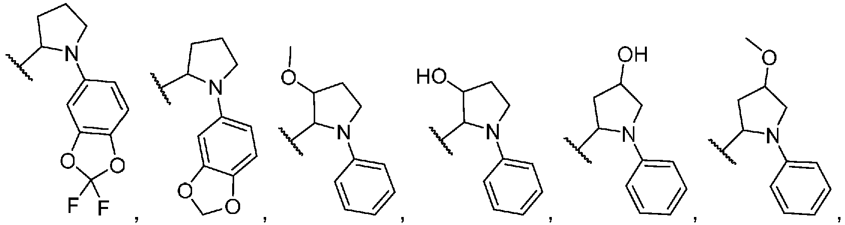

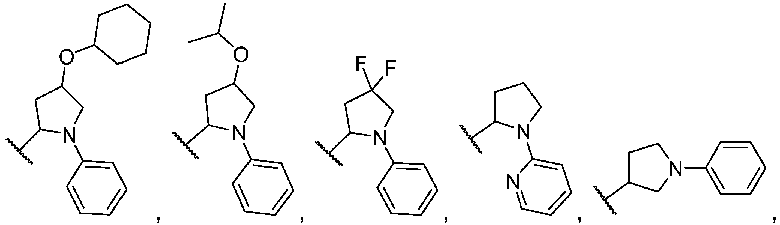

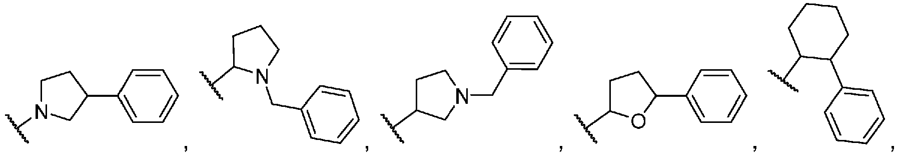

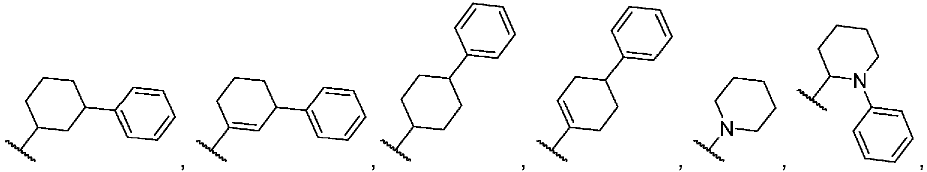

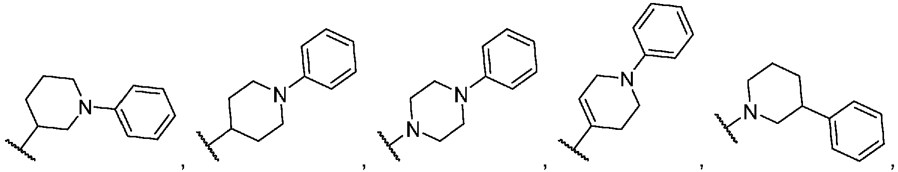

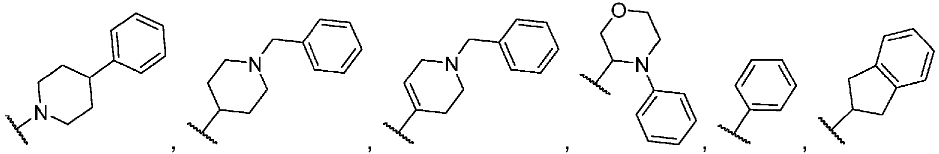

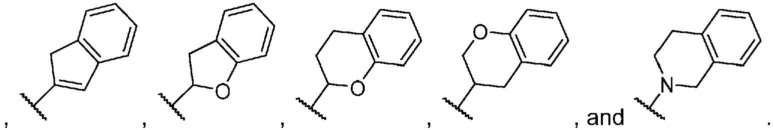

In some cases, L¹-B is selected from the group consisting of

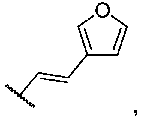

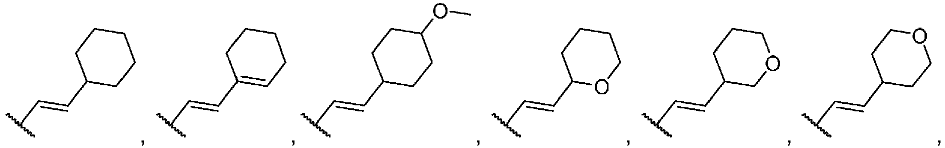

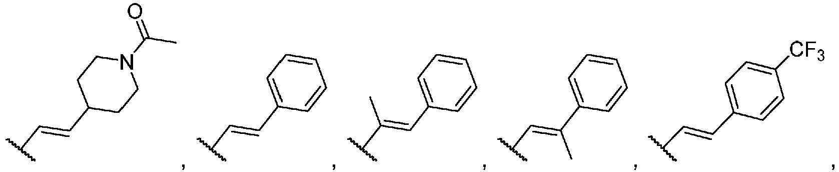

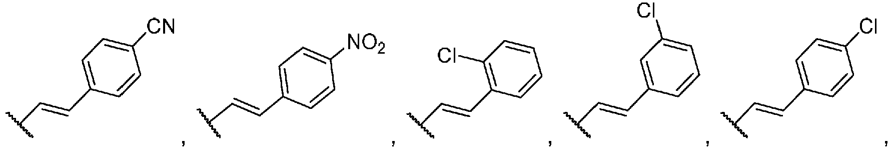

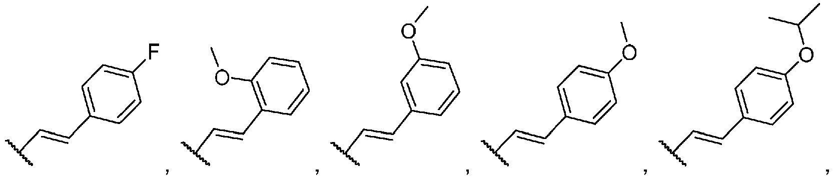

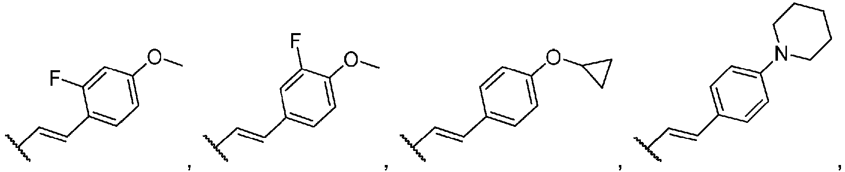

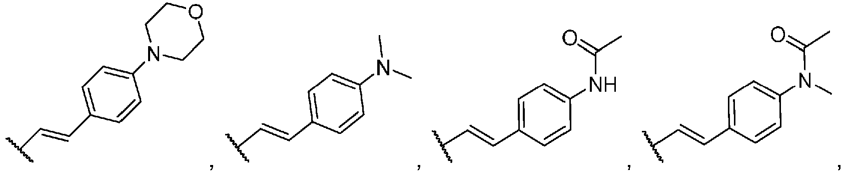

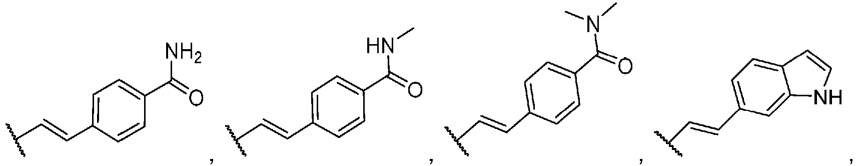

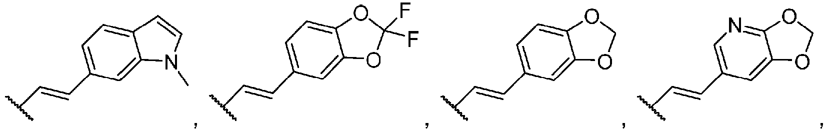

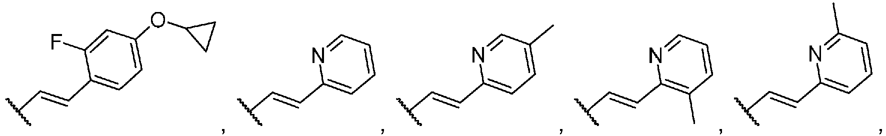

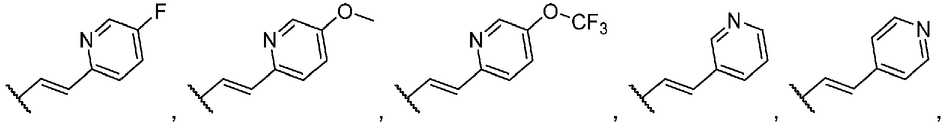

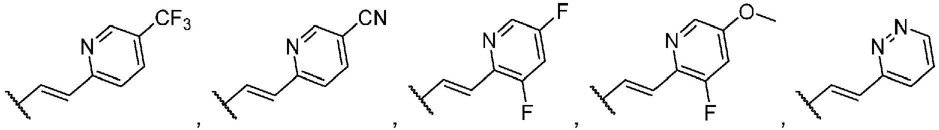

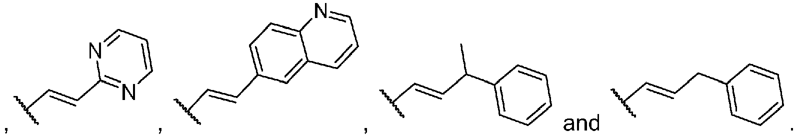

In some cases, the compound or salt is selected from the group consisting of

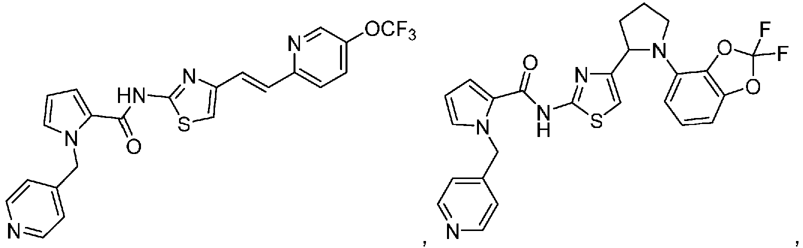

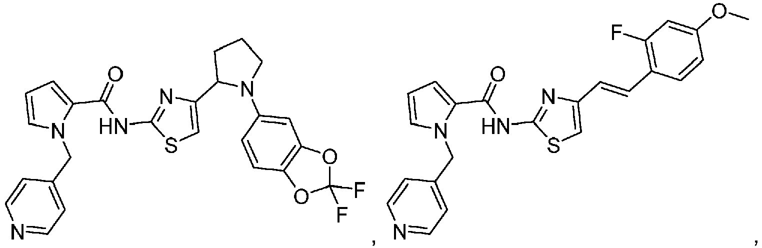

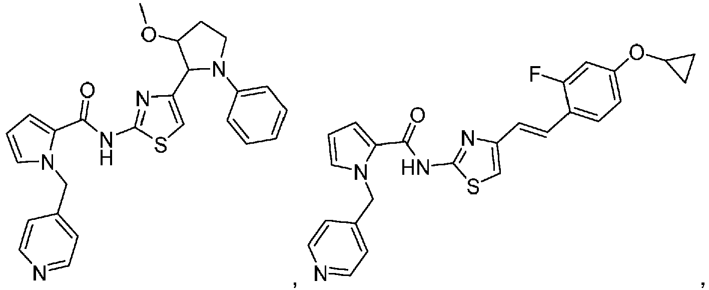

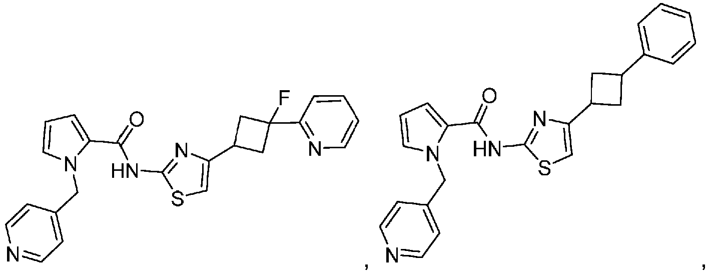

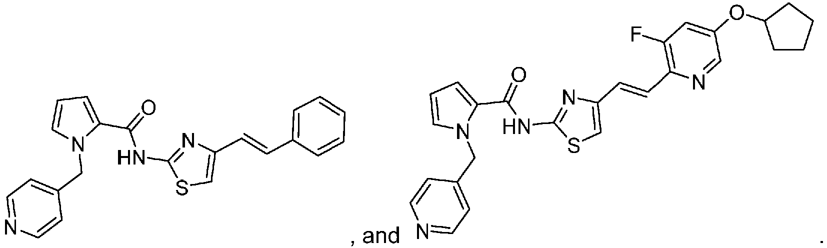

The disclosure further provides the compounds listed in Table A, or a pharmaceutically salt thereof. In some embodiments, the compound or salt is selected from A1-A210. In some embodiments, the compound or salt is selected from A211-A403. Further described are the compounds listed in Table B, or a pharmaceutically acceptable salt thereof, such as the compound or salt is selected from B1-B29. In some cases, the compound or salt is selected from the group consisting of 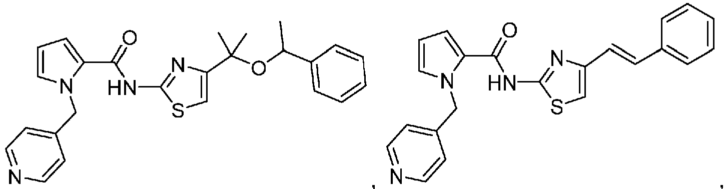 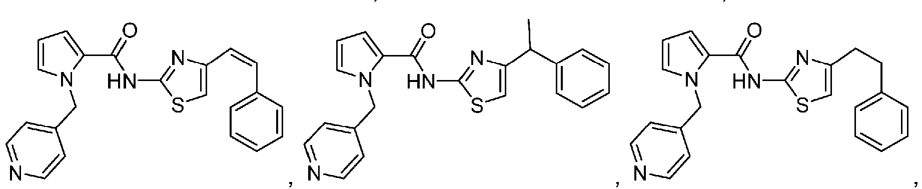 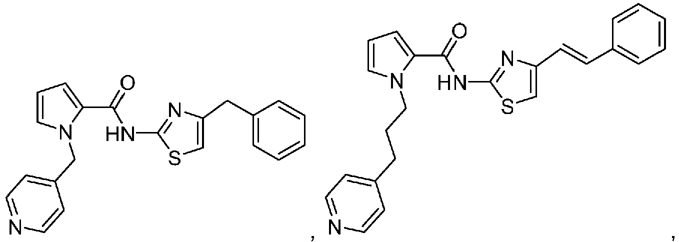 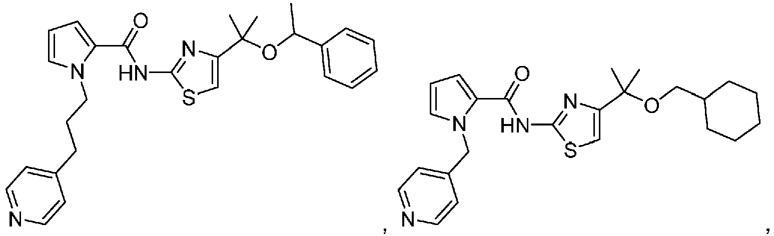 and 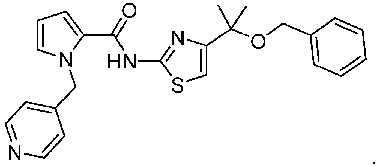 In some cases, the compound or salt is selected from the group consisting of 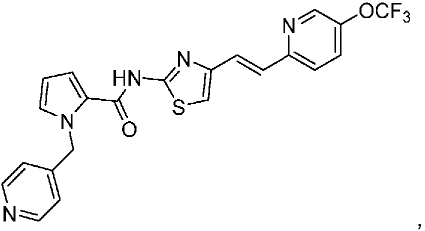 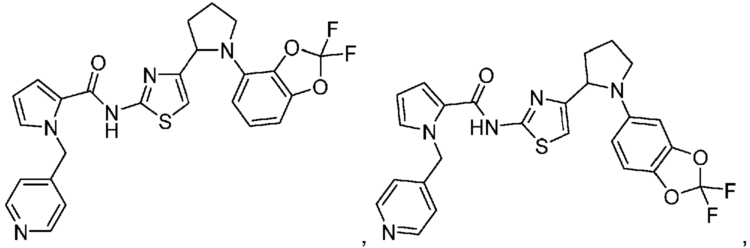 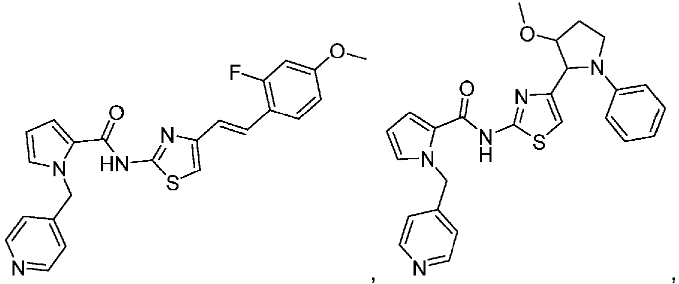 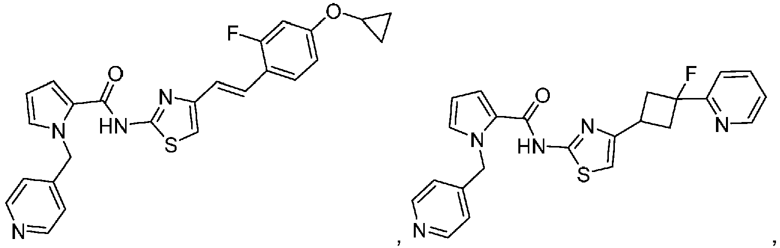 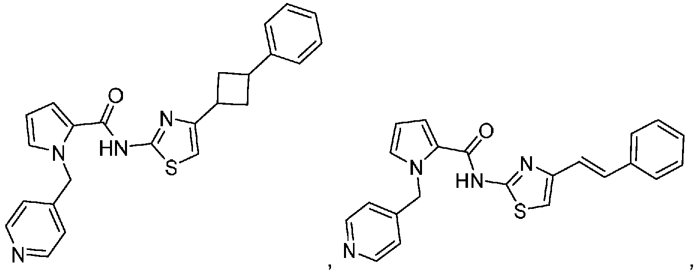 and

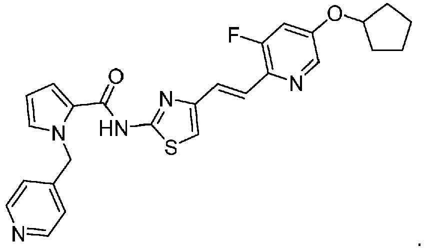

Also described are pharmaceutical compositions comprising the compound or salt described herein and a pharmaceutically acceptable carrier.

Further described are methods of inhibiting protein secretion in a cell comprising contacting the cell with the compound, salt, or pharmaceutical composition described herein in an amount effective to inhibit secretion. In some descriptions, the protein is a checkpoint protein. In some descriptions, the protein is a cell-surface protein, endoplasmic reticulum associated protein, or secreted protein involved in regulation of anti-tumor immune response. In various descriptions, the protein is at least one of PD-1, PD-L1, TIM-1, LAG-3, CTLA4, BTLA, OX-40, B7H1, B7H4, CD137, CD47, CD96, CD73, CD40, VISTA, TIGIT, LAIR1, CD160, 2B4, TGFRβ and combinations thereof. In some descriptions, the protein is selected from the group consisting of HER3, TNFα, IL2, and PD1. In some descriptions, the contacting comprises administering the compound or the composition to a subject in need thereof.

The disclosure also provides compounds for use in treating inflammation.

The disclosure further provides compounds for use in treating cancer. In some embodiments, the cancer is melanoma, multiple myeloma, prostate cancer, lung cancer, pancreatic cancer, squamous cell carcinoma, leukemia, lymphoma, a neuroendocrine tumor, bladder cancer, or colorectal cancer. In some cases, the cancer is selected from the group consisting of prostate, lung, bladder, colorectal, and multiple myeloma. In some cases, the cancer is non-small cell lung carcinoma, squamous cell carcinoma, leukemia, acute myelogenous leukemia, chronic myelogenous leukemia, lymphoma, NPM/ALK-transformed anaplastic large cell lymphoma, diffuse large B cell lymphoma, neuroendocrine tumors, breast cancer, mantle cell lymphoma, renal cell carcinoma, rhabdomyosarcoma, ovarian cancer, endometrial cancer, small cell carcinoma, adenocarcinoma, gastric carcinoma, hepatocellular carcinoma, pancreatic cancer, thyroid carcinoma, anaplastic large cell lymphoma, hemangioma, or head and neck cancer. In various cases, the cancer is a solid tumor. In various cases, the cancer is head and neck cancer, squamous cell carcinoma, gastric carcinoma, or pancreatic cancer.

Further provided are compounds for use in treating an autoimmune disease. In some embodiments, the autoimmune disease is psoriasis, dermatitis, systemic scleroderma, sclerosis, Crohn's disease, ulcerative colitis; respiratory distress syndrome, meningitis; encephalitis; uveitis; colitis; glomerulonephritis; eczema, asthma, chronic inflammation; atherosclerosis; leukocyte adhesion deficiency; rheumatoid arthritis; systemic lupus erythematosus (SLE); diabetes mellitus; multiple sclerosis; Reynaud's syndrome; autoimmune thyroiditis; allergic encephalomyelitis; Sjorgen's syndrome; juvenile onset diabetes; tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia.

The disclosure also provides compounds for use in the treatment of an immune-related disease. In some embodiments, the immune-related disease is rheumatoid arthritis, lupus, inflammatory bowel disease, multiple sclerosis, or Crohn's disease.

Further provided are compounds for use in treating a neurodegenerative disease. In some cases, the neurodegenerative disease is multiple sclerosis.

Also provided are compounds for use in treating an inflammatory disease. In some embodiments, the inflammatory disease is bronchitis, conjunctivitis, myocarditis, pancreatitis, chronic cholecstitis, bronchiectasis, aortic valve stenosis, restenosis, psoriasis or arthritis.

Further aspects and advantages will be apparent to those of ordinary skill in the art from a review of the following detailed description. The description hereafter includes specific embodiments with the understanding that the disclosure is illustrative, and is not intended to limit the disclosure to the specific embodiments described herein.

### DETAILED DESCRIPTION

Provided herein are compounds that inhibit protein secretion. The compounds described herein can be used to treat or prevent diseases associated with excessive protein secretion, such as inflammation and cancer, improving the quality of life for afflicted individuals.

Compounds disclosed herein can have a structure of Formula (III):

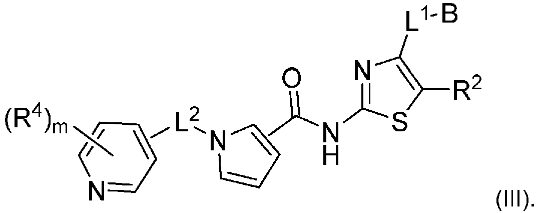

In some cases, the compound has a structure of Formula (IIIA):

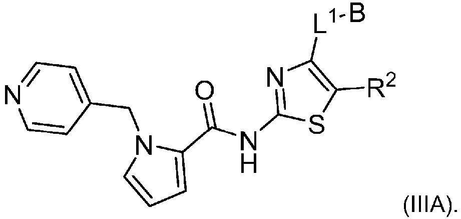

Without being bound by any particular theory, the compounds described herein inhibit protein secretion by binding to and disabling components of the translocon, including but not limited to Sec61, and in some cases, disrupting in a sequence specific fashion interactions between the nascent signaling sequence of translated proteins with components of the translocon including but not limited to Sec61.

The compounds described herein can advantageously inhibit the secretion of a protein of interest with an IC50 of up to 5 µM, or up to 3µM, or up to 1 µM. In various cases, the compounds disclosed herein can inhibit the secretion of TNFα with an IC50 of up to 5 µM, or up to 3µM, or up to 1 µM. In various cases, the compounds disclosed herein can inhibit the secretion of Her3 with an IC50 of up to 5 µM, or up to 3µM, or up to 1 µM. In some cases, the compounds disclosed herein can inhibit the secretion of IL2 with an IC50 of up to 5 µM, or up to 3µM, or up to 1 µM. In various cases, the compounds disclosed herein can inhibit the secretion of PD-1 with an IC50 of up to 5 µM, or up to 3µM, or up to 1 µM.

### Chemical Definitions

As used herein, the term "alkyl" refers to straight chained and branched saturated hydrocarbon groups containing one to thirty carbon atoms, for example, one to twenty carbon atoms, or one to ten carbon atoms. The term Cₙ means the alkyl group has "n" carbon atoms. For example, C₄alkyl refers to an alkyl group that has 4 carbon atoms. C₁₋₆alkyl refers to an alkyl group having a number of carbon atoms encompassing the entire range (i.e., 1 to 6 carbon atoms), as well as all subgroups (e.g., 1-5, 2-5, 1-4, 2-5, 1, 2, 3, 4, 5, and 6 carbon atoms). Nonlimiting examples of alkyl groups include, methyl, ethyl, n-propyl, isopropyl, n-butyl, sec-butyl (2-methylpropyl), and t-butyl (1,1-dimethylethyl). Unless otherwise indicated, an alkyl group can be an unsubstituted alkyl group or a substituted alkyl group.

As used herein, the term "alkylene" refers to a bivalent saturated aliphatic radical. The term Cₙ means the alkylene group has "n" carbon atoms. For example, C₁₋₆alkylene refers to an alkylene group having a number of carbon atoms encompassing the entire range, as well as all subgroups, as previously described for "alkyl" groups.

As used herein, the term "alkene" or "alkenyl" is defined identically as "alkyl" except for containing at least one carbon-carbon double bond, and having two to thirty carbon atoms, for example, two to twenty carbon atoms, or two to ten carbon atoms. The term Cₙ means the alkenyl group has "n" carbon atoms. For example, C₄alkenyl refers to an alkenyl group that has 4 carbon atoms. C₂₋₇alkenyl refers to an alkenyl group having a number of carbon atoms encompassing the entire range (i.e., 2 to 7 carbon atoms), as well as all subgroups (e.g., 2-6, 2-5, 3-6, 2, 3, 4, 5, 6, and 7 carbon atoms). Specifically contemplated alkenyl groups include ethenyl, 1-propenyl, 2-propenyl, and butenyl. Unless otherwise indicated, an alkenyl group can be an unsubstituted alkenyl group or a substituted alkenyl group. Unless otherwise indicated, an alkenyl group can be a cis-alkenyl or trans-alkenyl.

As used herein, the term "alkyne" or "alkynyl" is defined identically as "alkyl" except for containing at least one carbon-carbon triple bond, and having two to thirty carbon atoms, for example, two to twenty carbon atoms, or two to ten carbon atoms. The term Cₙ means the alkynyl group has "n" carbon atoms. For example, C₄alkynyl refers to an alkynyl group that has 4 carbon atoms. C₂₋₇alkynyl refers to an alkynyl group having a number of carbon atoms encompassing the entire range (i.e., 2 to 7 carbon atoms), as well as all subgroups (e.g., 2-6, 2-5, 3-6, 2, 3, 4, 5, 6, and 7 carbon atoms). Specifically contemplated alkynyl groups include ethynyl, 1-propynyl, 2-propynyl, and butynyl. Unless otherwise indicated, an alkynyl group can be an unsubstituted alkynyl group or a substituted alkynyl group.

As used herein, the term "carbocycle" refers to an aromatic or nonaromatic ring in which each atom of the ring is carbon. A carbocycle can include, for example, from three to ten carbon atoms, four to eight carbon atoms, or five to six carbon atoms. As used herein, the term "carbocycle" also includes polycyclic ring systems having two or more cyclic rings in which two or more carbons are common to two adjoining rings wherein at least one of the rings is carbocyclic, e.g., the other cyclic rings can be cycloalkyls, cycloalkenyls, aryls, heteroaryls, and/or heterocycles.

As used herein, the term "cycloalkyl" specifically refers to a non-aromatic carbocycle. The term Cₙ means the cycloalkyl group has "n" carbon atoms. For example, C₅ cycloalkyl refers to a cycloalkyl group that has 5 carbon atoms in the ring. C₅₋₈ cycloalkyl refers to cycloalkyl groups having a number of carbon atoms encompassing the entire range (i.e., 5 to 10 carbon atoms), as well as all subgroups (e.g., 5-10, 5-9, 5-8, 5-6, 6-8, 7-8, 5-7, 5, 6, 7, 8, 9 and 10 carbon atoms). Nonlimiting examples of cycloalkyl groups include cyclopropyl, cyclobutyl, cyclopentyl, cyclohexyl, cycloheptyl, and cyclooctyl. Unless otherwise indicated, a cycloalkyl group can be an unsubstituted cycloalkyl group or a substituted cycloalkyl group.

As used herein, the term "aryl" refers to an aromatic carbocycle, and can be monocyclic or polycyclic (e.g., fused bicyclic and fused tricyclic) carbocyclic aromatic ring systems. Examples of aryl groups include, but are not limited to, phenyl, naphthyl, tetrahydronaphthyl, phenanthrenyl, biphenylenyl, indanyl, indenyl, anthracenyl, fluorenyl, tetralinyl. Unless otherwise indicated, an aryl group can be an unsubstituted aryl group or a substituted aryl group.

As used herein, the term "heterocycle" is defined similarly as carbocycle, except the ring contains one to four heteroatoms independently selected from oxygen, nitrogen, and sulfur. For example, a heterocycle can be a 5-10 membered ring having 1 or 2 heteroatoms selected from N, O, and S. As another example, a heterocycle can be a 5-6 membered ring having 1 or 2 ring heteroatoms selected from N, O, and S. Nonlimiting examples of heterocycle groups include piperdine, tetrahydrofuran, tetrahydropyran, dihydrofuran, morpholine, oxazepaneyl, thiazole, pyrrole, and pyridine. Carbocyclic and heterocyclic groups can be saturated or partially unsaturated ring systems optionally substituted with, for example, one to three groups, independently selected alkyl, alkoxy, alkyleneOH, C(O)NH₂, NH₂, oxo (=O), aryl, haloalkyl, haloalkoxy, C(O)-alkyl, SO₂alkyl, halo, OH, NHC₁₋₃alkylene-aryl, OC₁₋₃alkylene-aryl, C₁₋₃alkylene-aryl, and C₃₋₆heterocycloalkyl having 1-3 heteroatoms selected from N, O, and S. Heterocyclic groups optionally can be further N-substituted as described herein.

As used herein, the term "heteroaryl" refers to an aromatic heterocycle, and can be monocyclic or polycyclic (e.g., fused bicyclic and fused tricyclic) aromatic ring systems, wherein one to four-ring atoms are selected from oxygen, nitrogen, or sulfur, and the remaining ring atoms are carbon, said ring system being joined to the remainder of the molecule by any of the ring atoms. Nonlimiting examples of heteroaryl groups include, but are not limited to, pyridyl, pyridazinyl, pyrazinyl, pyrimidinyl, pyrrolyl, pyrazolyl, imidazolyl, thiazolyl, tetrazolyl, oxazolyl, isooxazolyl, thiadiazolyl, oxadiazolyl, furanyl, thienyl, quinolinyl, isoquinolinyl, benzoxazolyl, benzimidazolyl, benzofuranyl, benzothiazolyl, triazinyl, triazolyl, purinyl, pyrazinyl, purinyl, indolinyl, phthalzinyl, indazolyl, quinolinyl, isoquinolinyl, cinnolinyl, quinazolinyl, naphthyridinyl, pyridopyridinyl, indolyl, 3H-indolyl, pteridinyl, and quinooxalinyl. Unless otherwise indicated, a heteroaryl group can be an unsubstituted heteroaryl group or a substituted heteroaryl group.

As used herein, the term "hydroxy" or "hydroxyl" as used herein refers to an "-OH" group. Accordingly, a "hydroxyalkyl" refers to an alkyl group substituted with one or more - OH groups.

As used herein, the term "alkoxy" or "alkoxyl" refers to a "-O-alkyl" group.

As used herein, the term "halo" is defined as fluoro, chloro, bromo, and iodo. Accordingly, a "haloalkyl" refers to an alkyl group substituted with one or more halo atoms. A "haloalkoxy" refers to an alkoxy group that is substituted with one or more halo atoms.

A "substituted" functional group (e.g., a substituted alkyl, cycloalkyl, aryl, or heteroaryl) is a functional group having at least one hydrogen radical that is substituted with a non-hydrogen radical (i.e., a substituent). Examples of non-hydrogen radicals (or substituents) include, but are not limited to, alkyl, cycloalkyl, alkenyl, cycloalkenyl, alkynyl, ether, aryl, O-alkylene aryl, N-alkylene aryl, alkylene aryl, heteroaryl, heterocycloalkyl, hydroxy, hydroxyalkyl, haloalkoxy, amido, oxy (or oxo), alkoxy, ester, thioester, acyl, carboxyl, cyano, nitro, amino, sulfhydryl, and halo. When a substituted alkyl group includes more than one non-hydrogen radical, the substituents can be bound to the same carbon or two or more different carbon atoms.

### Protein Secretion Inhibitors - Formula (III)

Provided herein are compounds having a structure of Formula (III), or a pharmaceutically acceptable salt thereof: 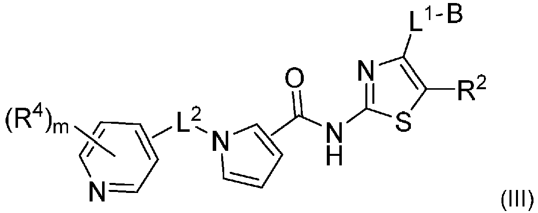 wherein L¹ is a bond, C₁₋₆alkylene, or 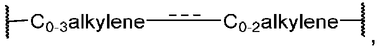 wherein - - - indicates a double bond, a triple bond, or a fused cyclopropyl; B is C₀₋₃alkylene-X; X is an aromatic or nonaromatic C₄₋₁₀carbocycle, or an aromatic or nonaromatic 4-10 membered heterocycle having 1-3 ring heteroatoms selected from N, O, and S; X is substituted with 1-3 G; each G is independently selected from the group consisting of halo, OH, =O, CN, NO₂, N(R^{N})₂, N(R^{N})C(O)C₁₋₃alkyl, C₁₋₃alkyl, C₁₋₃alkoxy, C₁₋₃ haloalkyl, C₁₋₃ haloalkoxy, C(O)C₁₋₃alkyl, S(O₂)-Z, C(O)-Z, C(O)N(R^{N})₂, silyl ether, and [O]₀₋₁-C₀₋₃alkylene-Z; each R^{N} independently is H or C₁₋₄alkyl; Z is aromatic or nonaromatic C₃₋₁₀carbocycle, or aromatic or nonaromatic 4-10 membered heterocycle having 1-3 heteroatoms selected from the group consisting of N, O, and S; Z is optionally substituted with 1-3 E; and, each E independently is selected from C₁₋₃alkyl, C₁₋₃alkoxy, =O, C₁₋₃haloalkoxy, CN, and halo; R² is H; L² is C₀₋₃alkylene; m is 0 to 2; and each R⁴ independently is C₁₋₃alkyl, C₂₋₃alkynyl, C₁₋₃haloalkyl, C₁₋₃alkoxy, halo, or NHC₁₋₃alkylene-aryl, wherein the compound or salt thereof is not N-[4-[(3-methyl-1-piperidinyl)methyl]2-thiazolyl)-1-(4-pyridinylmethyl)-1H-pyrrole-2-carboxamide.

As provided herein, L¹ is a bond, C₁₋₆alkylene, or 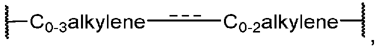 wherein - - - indicates a double bond, a triple bond, or a fused cyclopropyl (e.g., 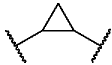 ).

In some embodiments, L¹ is a bond.

In some embodiments, L¹ is C₁₋₆alkylene. In various cases, L¹ is CH₂, CH₂CH₂, C(CH₃)₂, C(CH₃)₂CH₂, or C(CH₃)₂CH₂CH₂.

In some embodiments, L¹ is 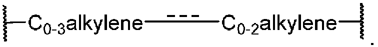 In various cases, L¹ is 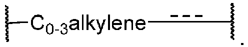 In various cases, L¹ is 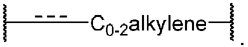 In various cases, L¹ is 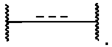 In various cases, - - - indicates a double bond. The double bond can be further substituted, for example, with C₁₋₃alkyl, e.g., methyl, ethyl, n-propyl, or isopropyl. In some embodiments, where - - - indicates a double bond, the double bond is substituted with a methyl. The double bond orientation can be cis or trans, or when substituted, E- or Z-. In various cases, - - - indicates a triple bond. In various cases, - - - indicates a fused cyclopropyl, e.g.,

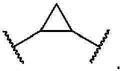

As provided herein, B is C₀₋₃alkylene-X, for example, X, C₁alkylene-X, C₂alkylene-X, or C₃alkylene-X. In various cases, B is C₁₋₃alkylene-X. In various cases, B is X.

As provided herein, X is an aromatic or nonaromatic C₄₋₁₀carbocycle, or an aromatic or nonaromatic 4-10 membered heterocycle having 1-3 ring heteroatoms selected from N, O, and S. In some embodiments, X is aromatic or nonaromatic C₄₋₇ carbocycle, or an aromatic or nonaromatic 4-9 membered heterocycle having 1 ring heteroatom. In various cases, X is an aromatic C₆₋₁₀carbocycle, e.g., phenyl or naphthyl. In various cases, X is an aromatic C₆₋₇ carbocycle, e.g., phenyl. In various cases, X is a nonaromatic C₄₋₁₀carbocycle, e.g., cyclobutyl, cyclohexanyl, or cyclohexenyl. In various cases, X is a nonaromatic C₄₋₇carbocycle. In various cases, X is an aromatic 6-10 membered heterocycle having 1-3 ring heteroatoms selected from N, O, and S. In various cases, X is a nonaromatic 4-10 membered heterocycle having 1-3 ring heteroatoms selected from N, O, and S. In various cases, X is an aromatic 6-9 membered heterocycle having 1 ring heteroatom. In various cases, X is a nonaromatic 4-9 membered heterocycle having 1 ring heteroatom.

Examples of suitable X include, but are not limited to, pyrrolidinyl, cyclobutyl, cyclopentyl, cyclohexyl, cyclohexenyl, phenyl, piperidinyl, pyridinyl, piperazinyl, tetrahydrofuranyl, furanyl, tetrahydropyranyl, quinolinyl, morpholinyl, pyrrolidonyl, pyrimidinyl, pyridazinyl, indenyl, dihydroindenyl, dihydrobenzofuranyl, chromanyl, isochromanyl, dihydroisoquinolinyl, or indolyl.

X can be substituted with 1-3 G. In various cases, X is substituted with 1 G. In various cases, X is substituted with 2 G. In various cases, X is substituted with 3 G. Each G can be independently selected from the group consisting of halo, OH, =O, CN, NO₂, N(R^{N})₂, N(R^{N})C(O)C₁₋₃alkyl, C₁₋₃alkyl, C₁₋₃alkoxy, C₁₋₃ haloalkyl, C₁₋₃ haloalkoxy, C(O)C₁₋₃alkyl, S(O₂)-Z, C(O)-Z, C(O)N(R^{N})₂, silyl ether, and [O]₀₋₁-C₀₋₃alkylene-Z. As provided herein, each R^{N} independently is H or C₁₋₄alkyl.

In various cases, G is halo, e.g., F, Cl, or Br. In various cases, G is OH. In various cases, G is =O. In various cases, G is CN. In various cases, G is NO₂. In various cases, G is N(R^{N})₂, e.g., NH₂, NHC₁₋₃alkyl, or N(C₁₋₃alkyl)₂. In various cases, G is N(R^{N})C(O)C₁₋₃alkyl, e.g., NHC(O)CH₃. In various cases, G is C₁₋₃alkyl, e.g., methyl, ethyl, n-propyl, or isopropyl. In various cases, G is C₁₋₃alkoxy, e.g., methoxy, ethoxy, n-propoxy, or isopropoxy. In various cases, G is C₁₋₃ haloalkyl, such as CF₃, CHF₂, or CH₂F. In various cases, G is C₁₋₃ haloalkoxy, e.g., OCF₃, OCH₂CF₃, or OCF₂CF₃. In various cases, G is C(O)C₁₋₃alkyl, e.g., C(O)CH₃, C(O)CH₂CH₃, or C(O)CH₂CH₂CH₃. In various cases, G is S(O₂)-Z. In various cases, G is C(O)-Z. In various cases, G is C(O)N(R^{N})₂, e.g., C(O)NH₂, C(O)NHC₁₋₃alkyl, or C(O)N(C₁₋₃alkyl)₂. In various cases, G is silyl ether, e.g. tert-butyldiphenylsilyl ether. In various cases, G is [O]₀₋₁-C₀₋₃alkylene-Z, e.g., O-C₁₋₃alkylene-Z, O-Z, C₁₋₃alkylene-Z, or Z.

As provided herein, Z is aromatic or nonaromatic C₃₋₁₀carbocycle, or aromatic or nonaromatic 4-10 membered heterocycle having 1-3 heteroatoms selected from the group consisting of N, O, and S. In some embodiments, Z is aromatic C₆₋₁₀carbocycle, e.g., phenyl or naphthyl. In some embodiments, Z is nonaromatic C₃₋₁₀carbocycle, e.g., cyclopropyl, cyclobutyl, cyclopropyl, or cyclohexanyl. In some embodiments, Z is aromatic 6-10 membered heterocycle having 1-3 heteroatoms selected from the group consisting of N, O, and S. In some embodiments, Z is nonaromatic 4-10 membered heterocycle having 1-3 heteroatoms selected from the group consisting of N, O, and S.

Examples of suitable Z include, but are not limited to, pyrrolidinyl, cyclobutyl, cyclopentyl, cyclohexyl, cyclohexenyl, phenyl, piperidinyl, pyridinyl, piperazinyl, tetrahydrofuranyl, furanyl, tetrahydropyranyl, quinolinyl, morpholinyl, pyrrolidonyl, pyrimidinyl, pyridazinyl, indenyl, dihydroindenyl, dihydrobenzofuranyl, chromanyl, isochromanyl, dihydroisoquinolinyl, or indolyl.

In some embodiments, Z is substituted with 1-3 E. In some embodiments, Z is not substituted. In various cases, Z is substituted with 1 E. In various cases, Z is substituted

with 2 E. In various cases, Z is substituted with 3 E. Each E can be independently selected from C₁₋₃alkyl, C₁₋₃alkoxy, =O, C₁₋₃haloalkoxy, CN, and halo.

In various cases, E is C₁₋₃alkyl, e.g., methyl, ethyl, n-propyl, or isopropyl. In various cases, E is C₁₋₃alkoxy, e.g., methoxy, ethoxy, n-propoxy, or isopropoxy. In various cases, E is =O. In various cases, E is C₁₋₃haloalkoxy, e.g., OCF₃, OCH₂CF₃, or OCF₂CF₃. In various cases, E is CN. In various cases, E is halo, e.g., F, Cl, or Br.

In some embodiments, L¹-B is selected from the group consisting of 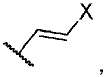 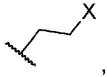 and 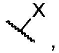 wherein X is as described herein. For example, in embodiments, X is aromatic or nonaromatic C₄₋₇ carbocycle, or an aromatic or nonaromatic 4-9 membered heterocycle having 1 ring heteroatom. In some embodiments, X is substituted with 1-3 G, wherein G can be as described herein.

As provided herein, R² is H.

As provided herein, L² is C₀₋₃alkylene. In various cases, L² is a bond (i.e., C₀alkylene). In various cases, L² is C₁alkylene. In various cases, L² is C₂alkylene. In various cases, L² is C₃alkylene.

As provided herein, m is 0 to 2. In various cases, m is 0. In various cases, m is 1. In various cases, m is 2. In various cases, m is 1 or 2.

As provided herein, each R⁴ independently is C₁₋₃alkyl, C₂₋₃alkynyl, C₁₋₃haloalkyl, C₁₋₃alkoxy, halo, or NHC₁₋₃alkylene-aryl.

In some embodiments, at least one R⁴ is C₁₋₃alkyl, e.g., methyl, ethyl, n-propyl, or isopropyl. In various cases, at least one R⁴ is methyl or ethyl. In some embodiments, at least one R⁴ is C₂₋₃alkynyl. In various cases, at least one R⁴ is C₂alkynyl. In various cases, at least one R⁴ is C₃alkynyl. In some embodiments, at least one R⁴ is C₁₋₃haloalkyl, e.g., CF₃, CHF₂, or CH₂F. In various cases, at least one R⁴ is CF₃. In some embodiments, at least one R⁴ is C₁₋₃alkoxy, e.g., methoxy, ethoxy, n-propoxy, or isopropoxy. In various cases, at least one R⁴ is methoxy. In some embodiments, at least one R⁴ is halo, e.g., F, Cl, or Br. In various cases, at least one R⁴ is F. In various cases, at least one R⁴ is Cl. In some embodiments, NHC₁₋₃alkylene-aryl. In various cases, at least one R⁴ is NH-CH₂-phenyl.

In some embodiments, one R⁴ is halo, and the other R⁴ is halo or methyl. In various cases, one R⁴ is halo, and the other R⁴ is halo. For example, in various cases, one R⁴ is F, and the other R⁴ is F. In various cases, one R⁴ is halo, and the other R⁴ is methyl. For example, in various cases, one R⁴ is F, and the other R⁴ is methyl.

In some embodiments, the compound has a structure of Formula (IIIA): 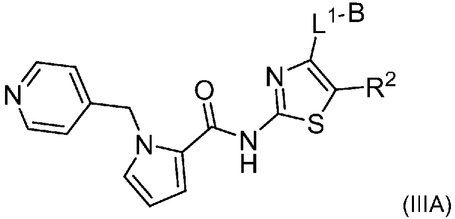 wherein each of L¹-B and R³ are as described herein.

In some embodiments, L¹-B is selected from the group consisting of

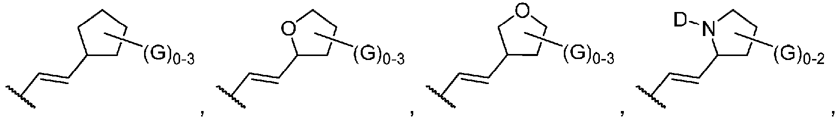

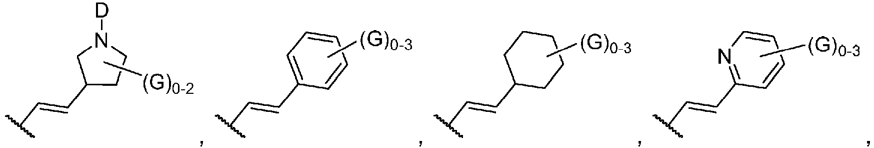

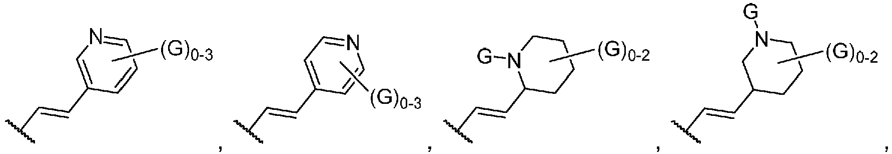

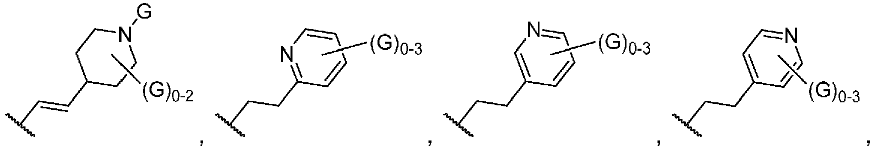

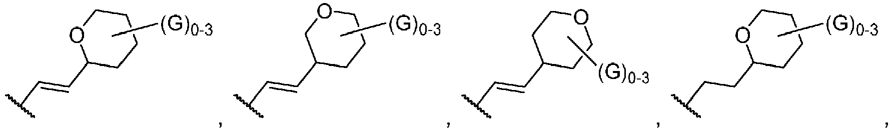

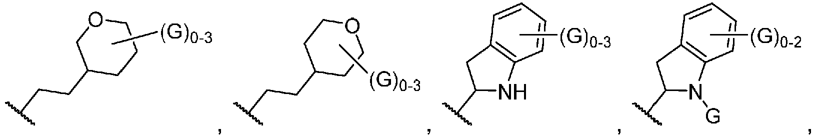

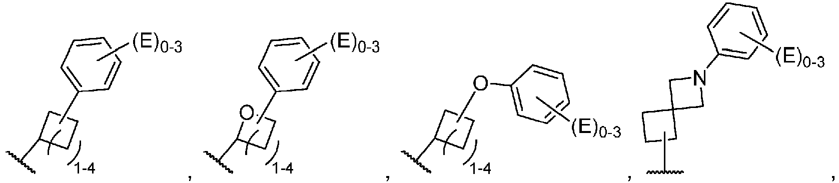

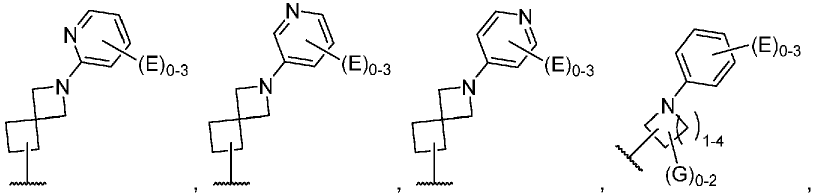

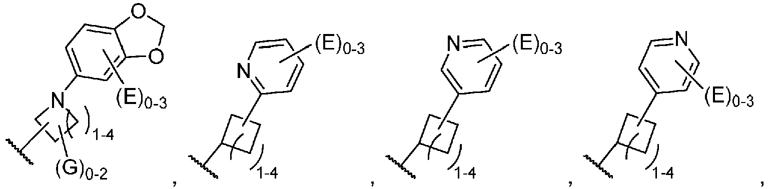

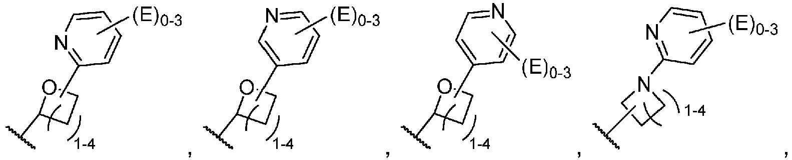

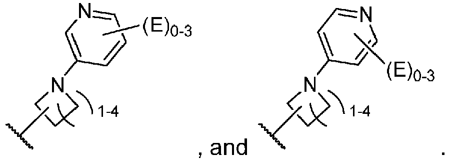

In some embodiments, L¹-B is selected from the group consisting of

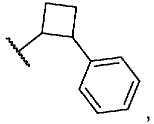

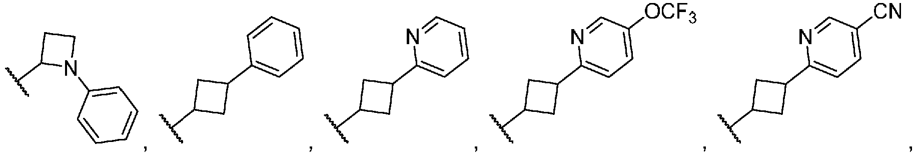

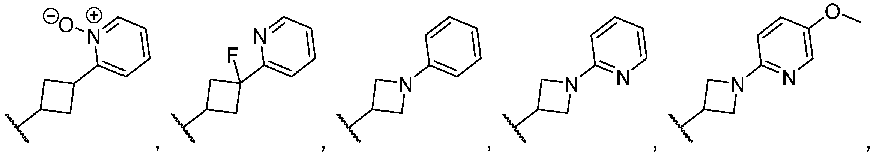

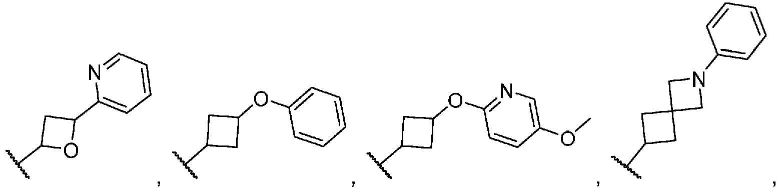

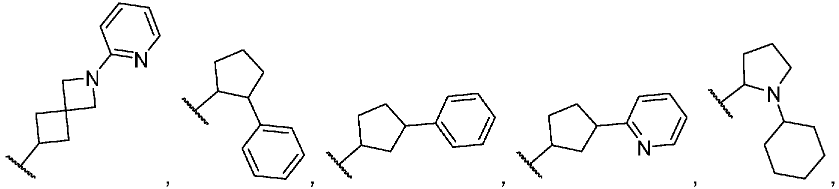

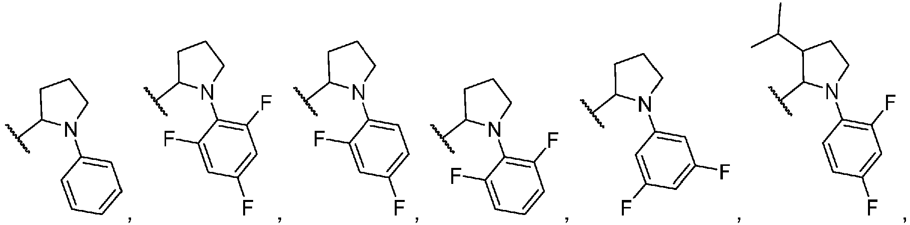

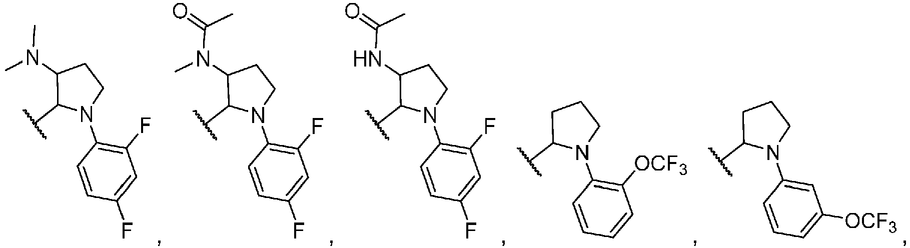

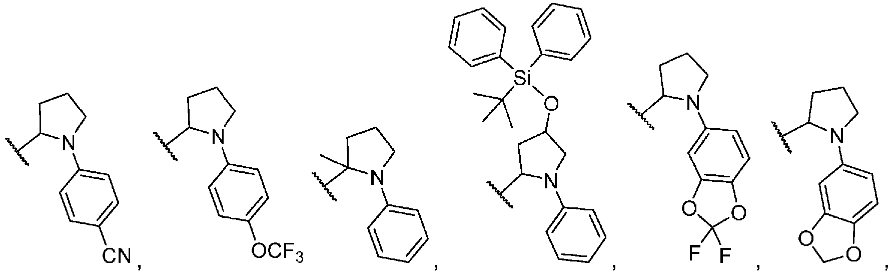

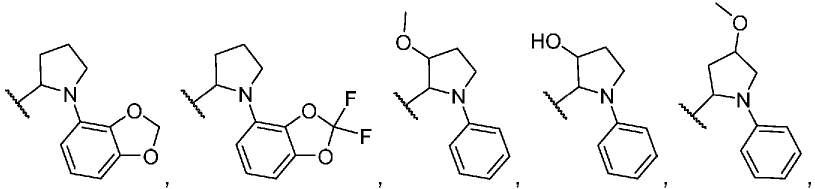

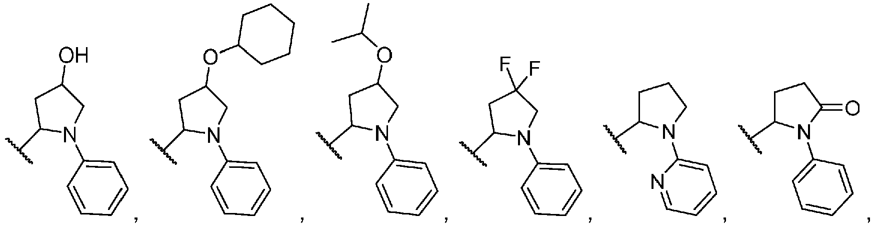

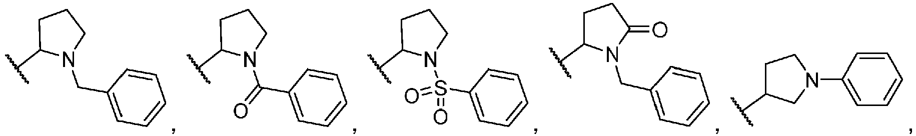

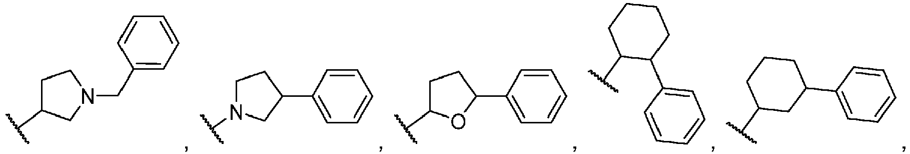

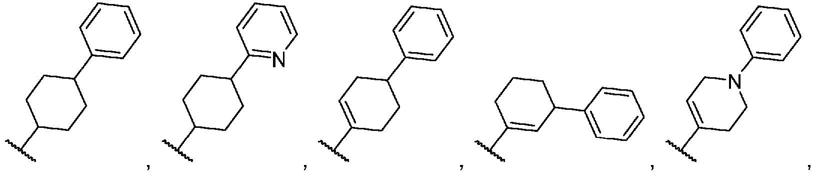

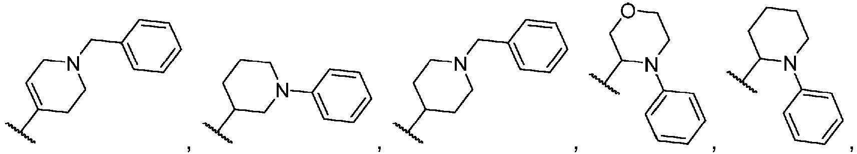

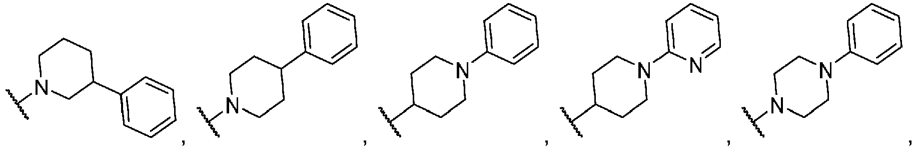

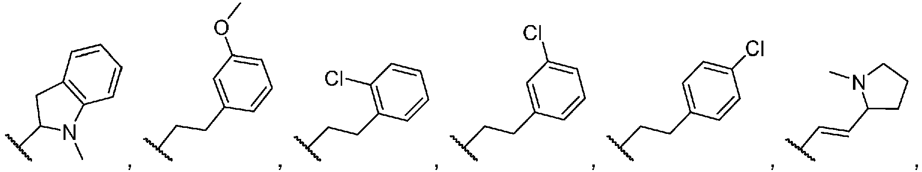

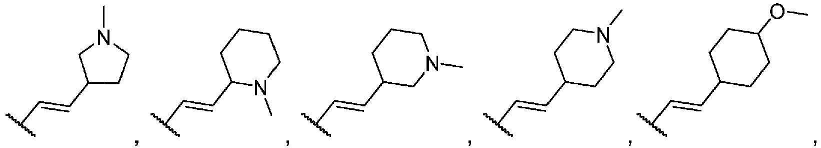

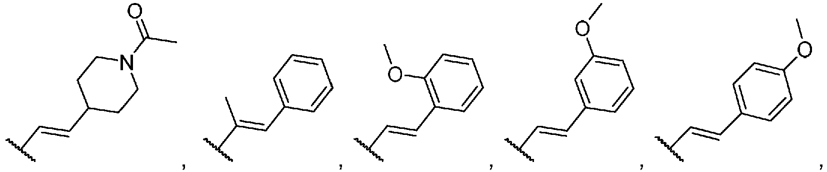

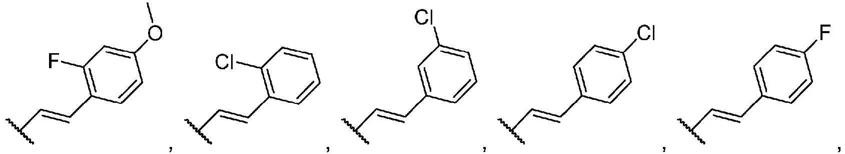

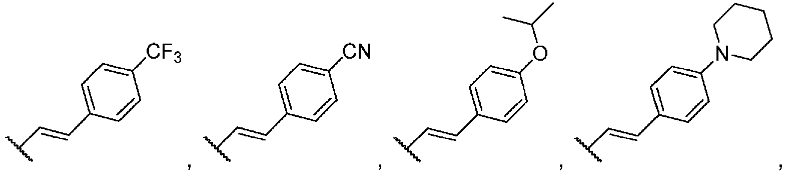

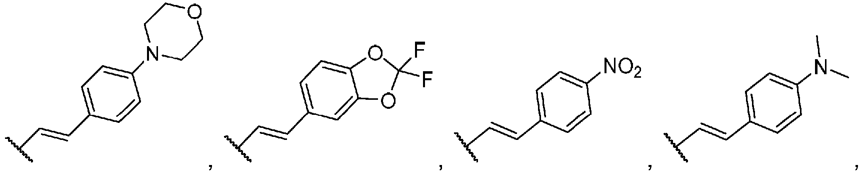

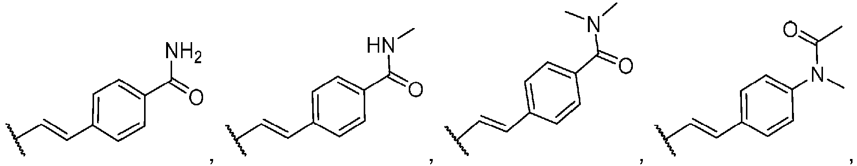

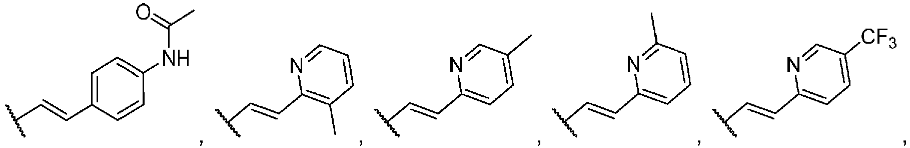

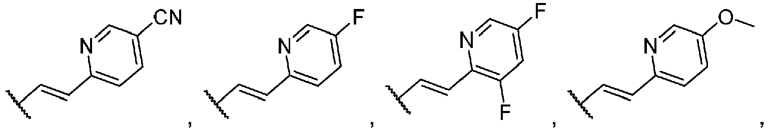

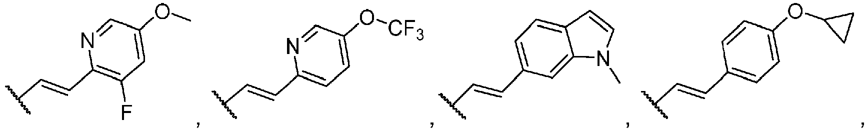

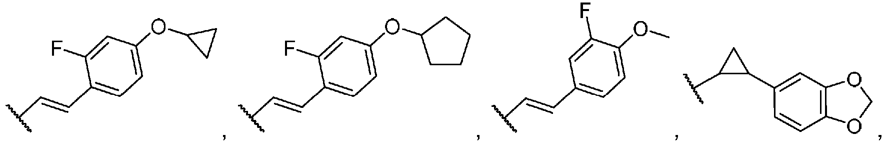

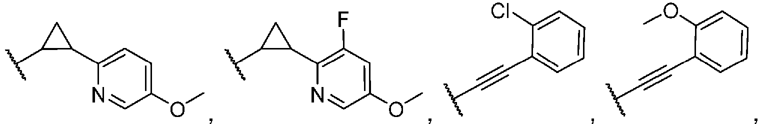

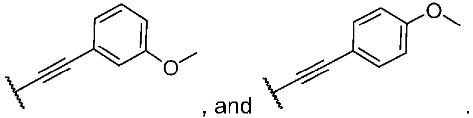

In some embodiments, L¹-B is selected from the group consisting of

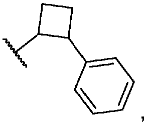

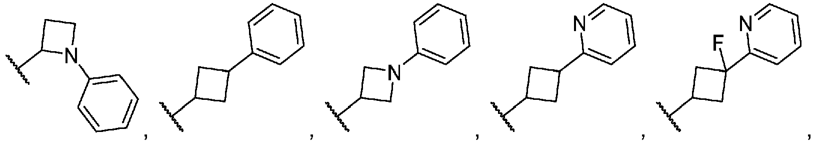

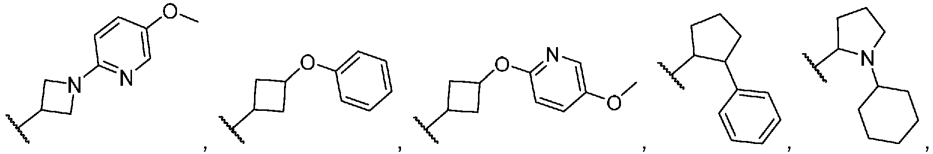

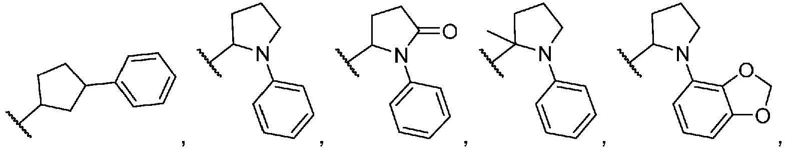

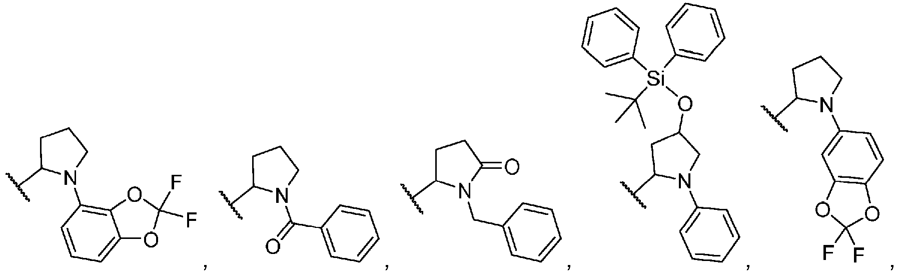

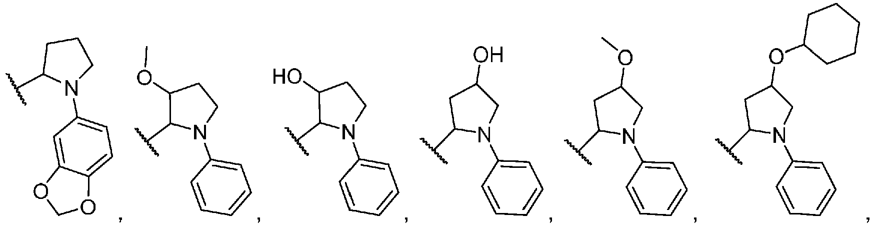

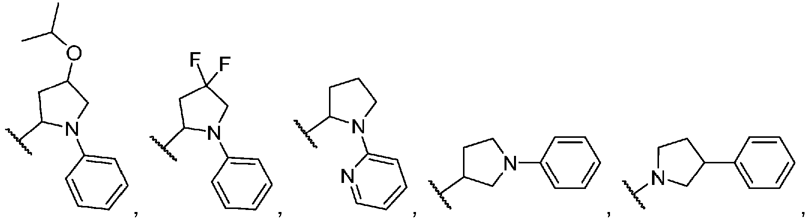

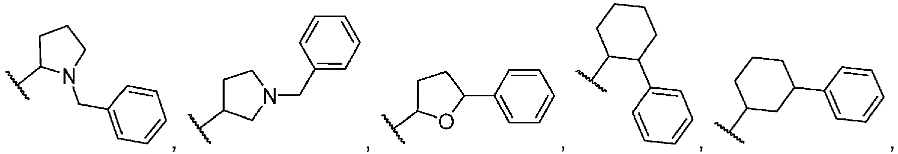

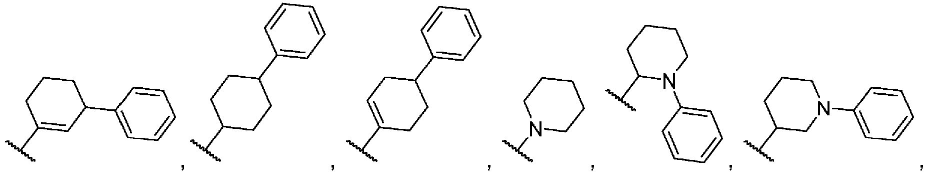

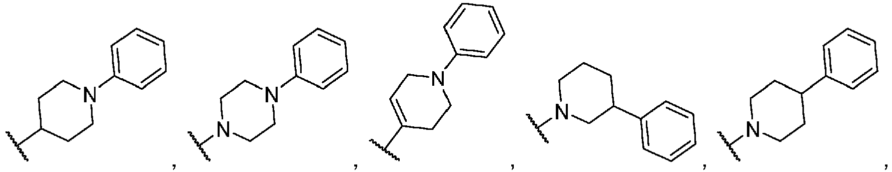

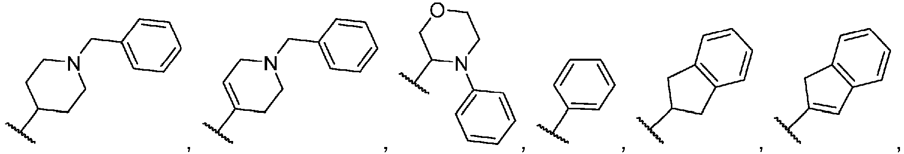

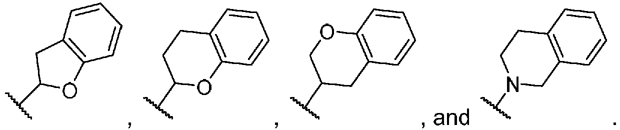

In some embodiments, L¹-B is selected from the group consisting of

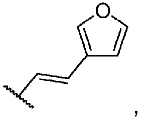

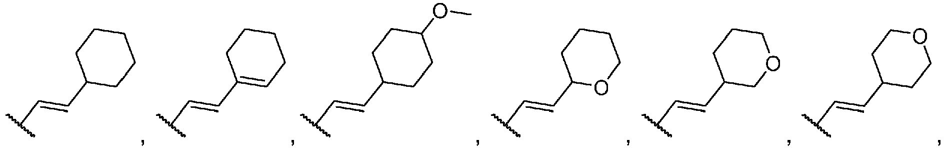

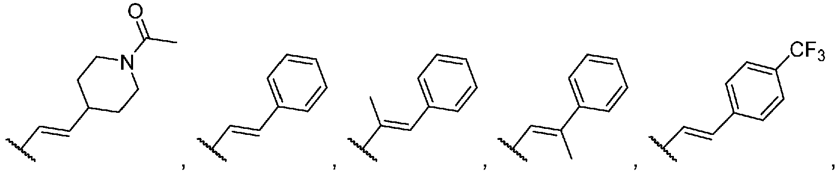

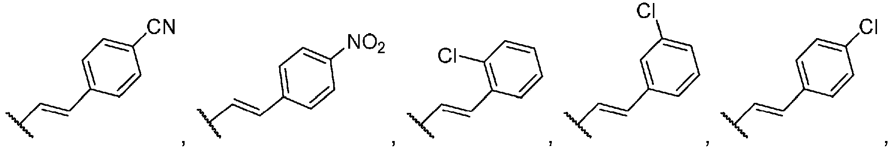

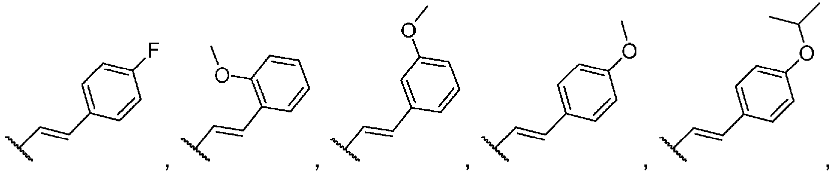

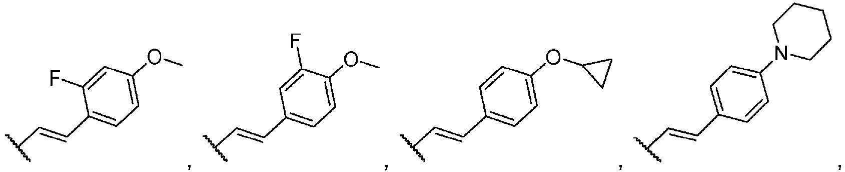

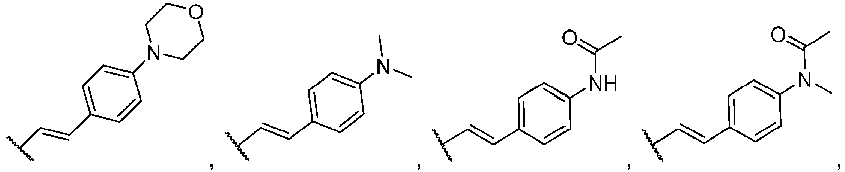

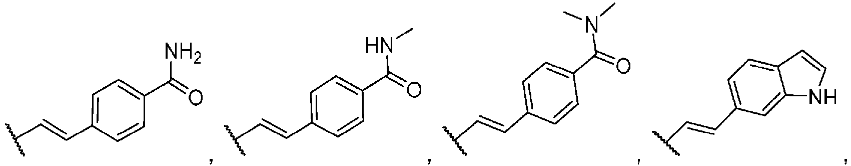

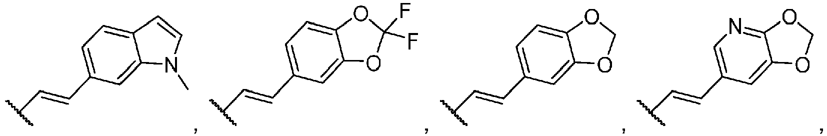

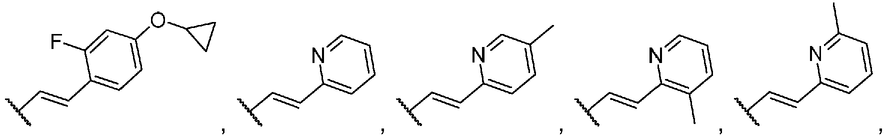

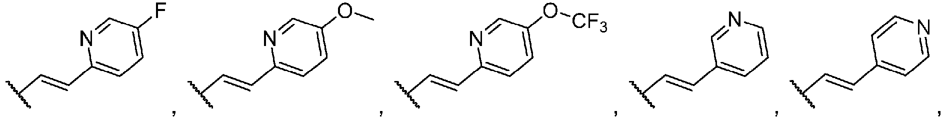

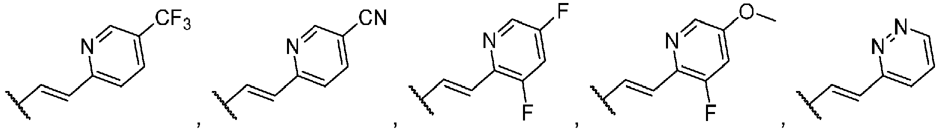

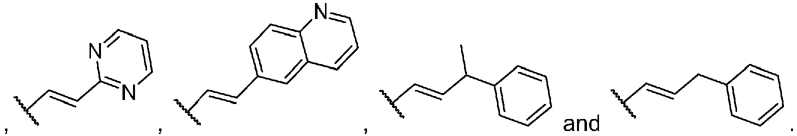

Examples of compounds according to Formula(III) of the disclosure are shown in Table A, below, as compounds A1-A403. In some embodiments, a compound of the disclosure is one of A1-A210. Additional compounds are shown in Table B, below, as compounds B1-B29.

**Table A**

| | |
|---|---|
| **A1** | 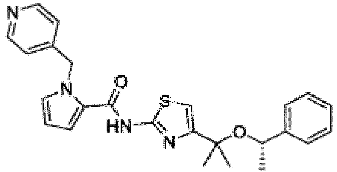 |
| **A2** | 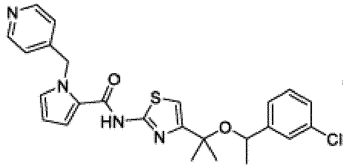 |
| **A3** | 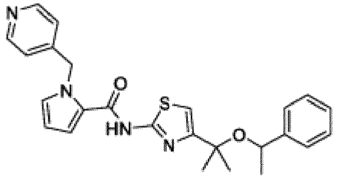 |
| **A4** | 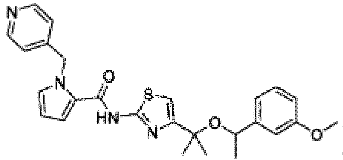 |
| **A5** | 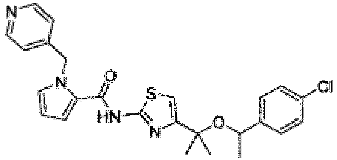 |
| **A6** | 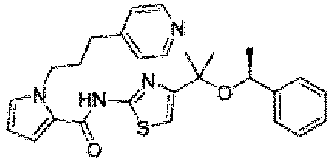 |
| **A7** | 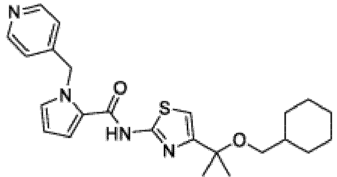 |
| **A8** | 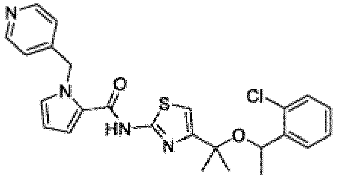 |
| **A9** | 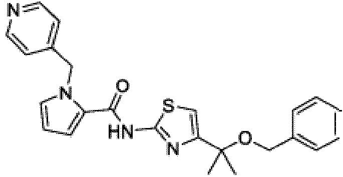 |
| **A10** | 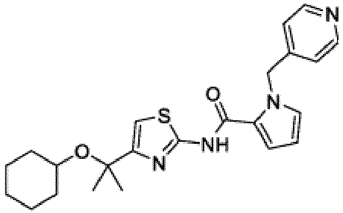 |
| **A11** | 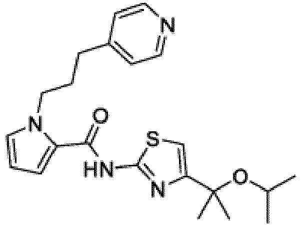 |
| **A12** | 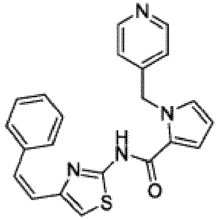 |
| **A13** | 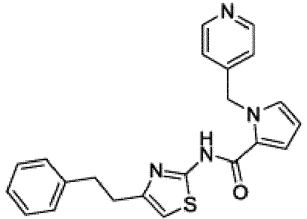 |
| **A14** | 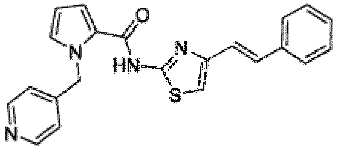 |
| **A15** | 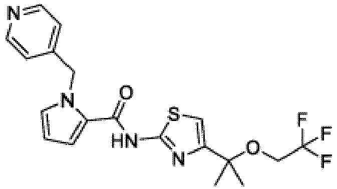 |
| **A16** | 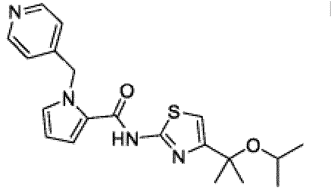 |
| **A17** | 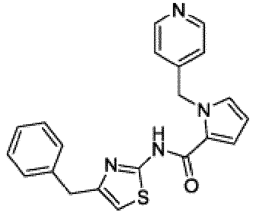 |
| **A18** | 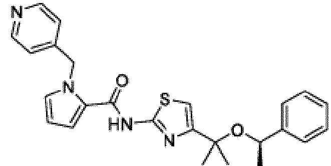 |
| **A19** | 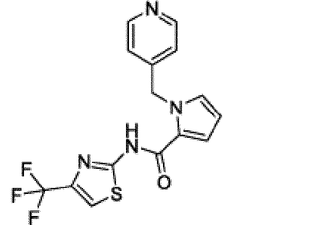 |
| **A20** | 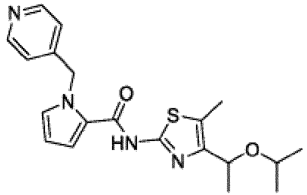 |
| **A21** | 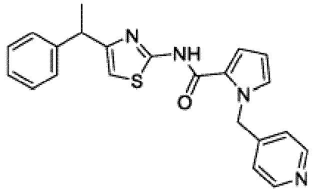 |
| **A22** | 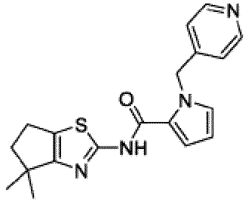 |
| **A23** | 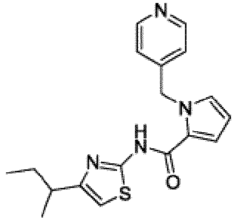 |
| **A24** | 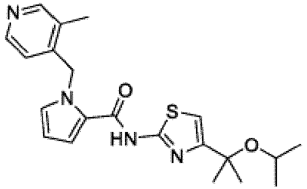 |
| **A25** | 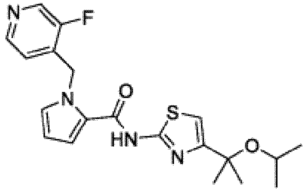 |
| **A26** | 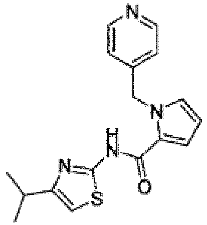 |
| **A27** | 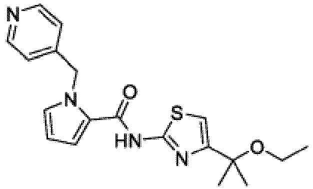 |
| **A28** | 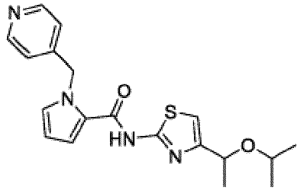 |
| **A29** | 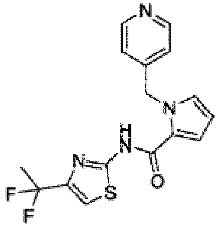 |
| **A30** | 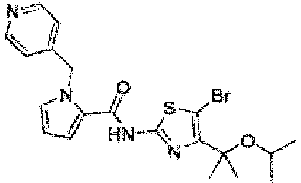 |
| **A31** | 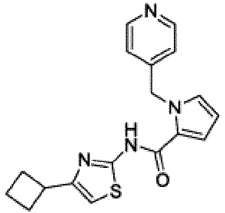 |
| **A32** | 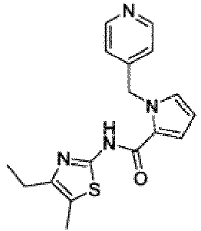 |
| **A33** | 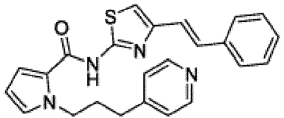 |
| **A34** | 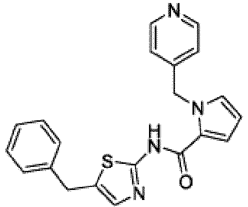 |
| **A35** | 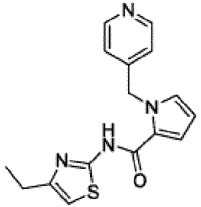 |
| **A36** | 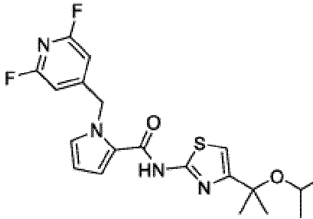 |
| **A37** | 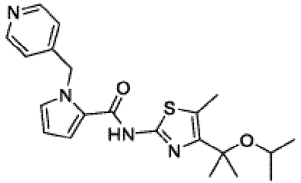 |
| **A38** | 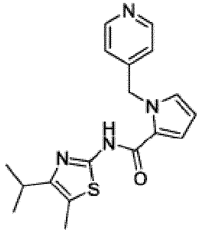 |
| **A39** | 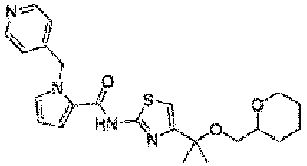 |
| **A40** | 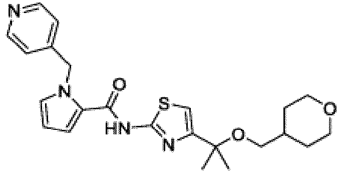 |
| **A41** | 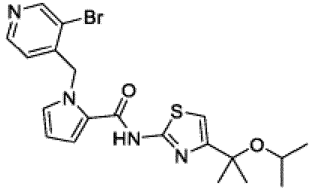 |
| **A42** | 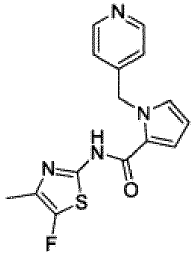 |
| **A43** | 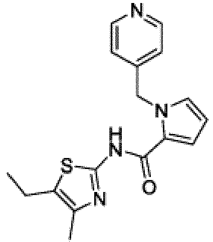 |
| **A44** | 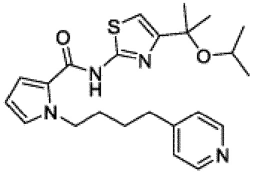 |
| **A45** | 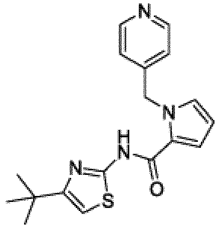 |
| **A46** | 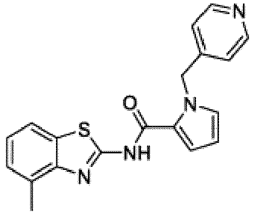 |
| **A47** | 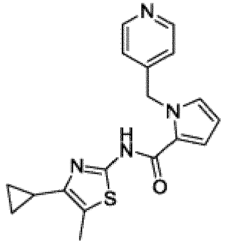 |
| **A48** | 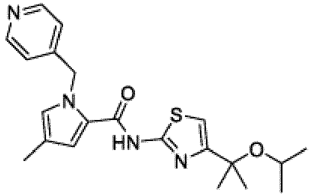 |
| **A49** | 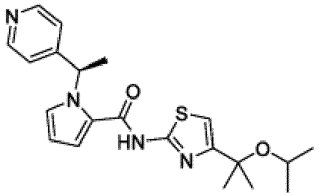 |
| **A50** | 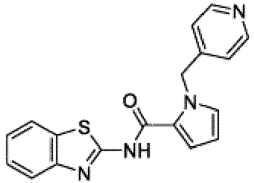 |
| **A51** | 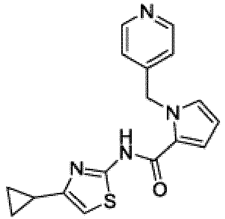 |
| **A52** | 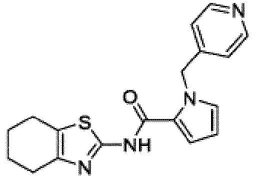 |
| **A53** | 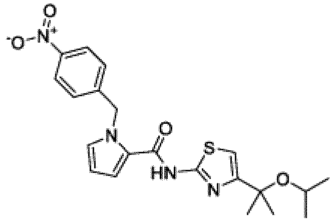 |
| **A54** | 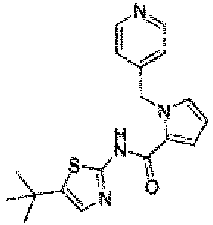 |
| **A55** | 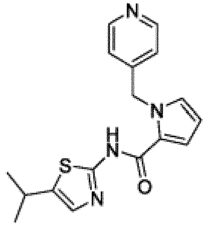 |
| **A56** | 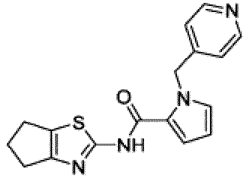 |
| **A57** | 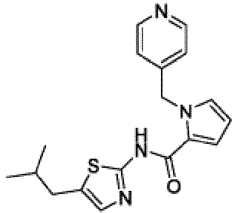 |
| **A58** | 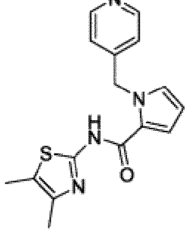 |
| **A59** | 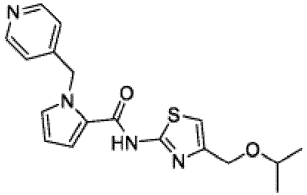 |
| **A60** | 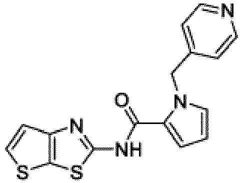 |
| **A61** | 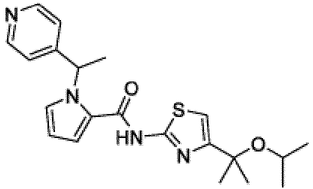 |
| **A62** | 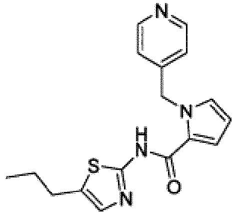 |
| **A63** | 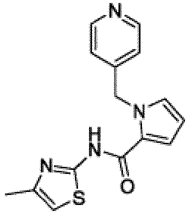 |
| **A64** | 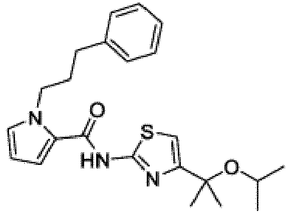 |
| **A65** | 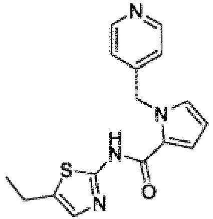 |
| **A66** | 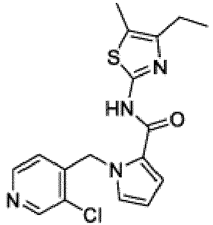 |
| **A67** | 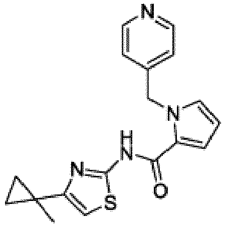 |
| **A68** | 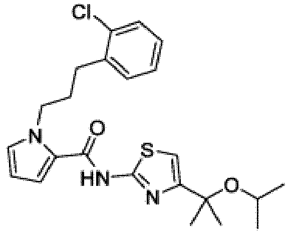 |
| **A69** | 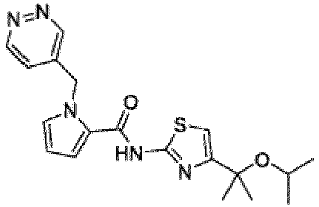 |
| **A70** | 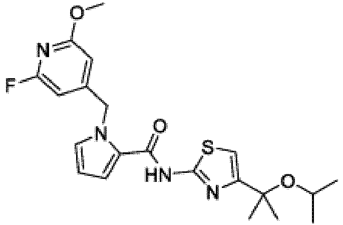 |
| **A71** | 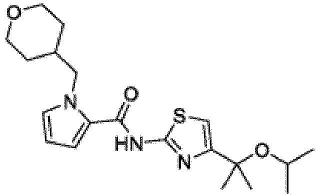 |
| **A72** | 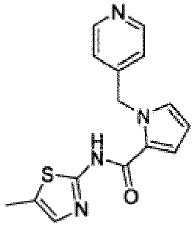 |
| **A73** | 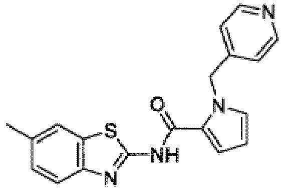 |
| **A74** | 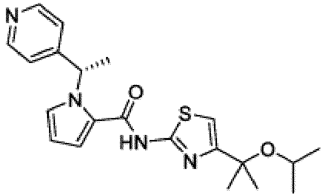 |
| **A75** | 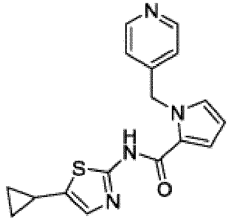 |
| **A76** | 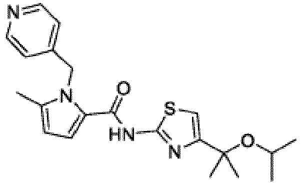 |
| **A77** | 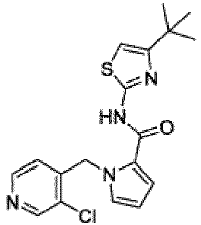 |
| **A78** | 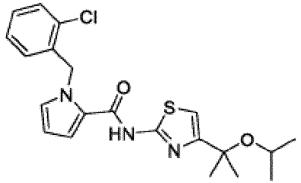 |
| **A79** | 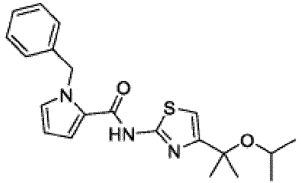 |
| **A80** | 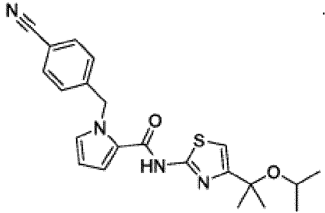 |
| **A81** | 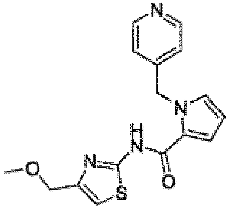 |
| **A82** | 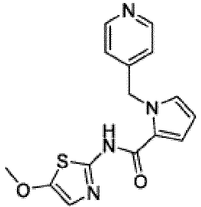 |
| **A83** | 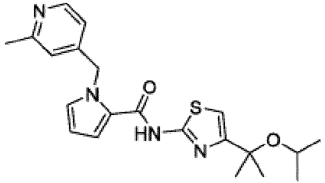 |
| **A84** | 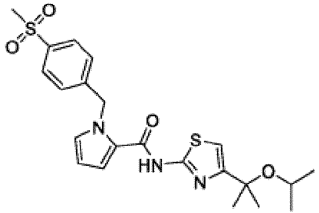 |
| **A85** | 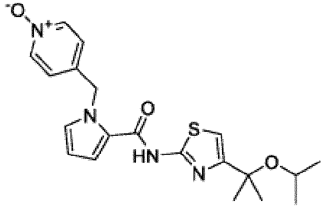 |
| **A86** | 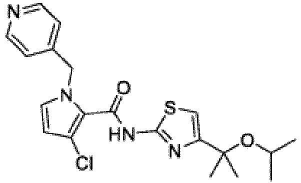 |
| **A87** | 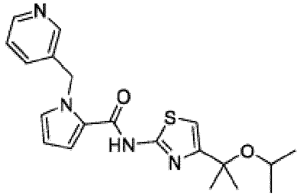 |
| **A88** | 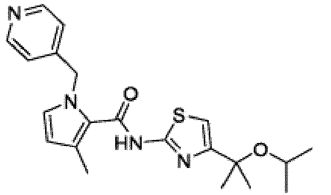 |
| **A89** | 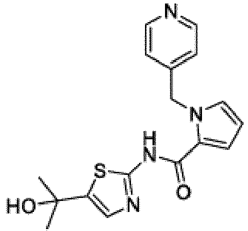 |
| **A90** | 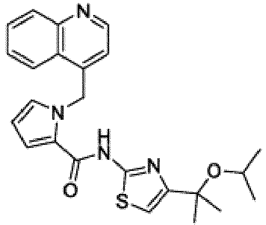 |
| **A91** | 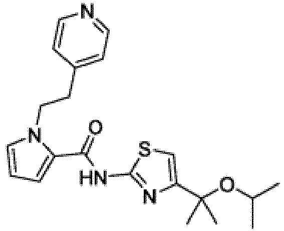 |
| **A92** | 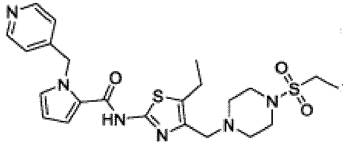 |
| **A93** | 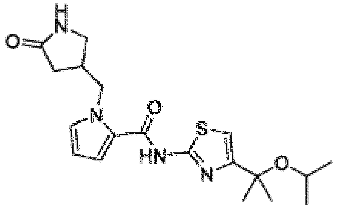 |
| **A94** | 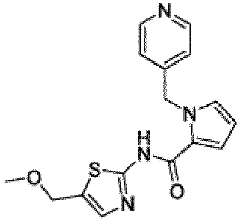 |
| **A95** | 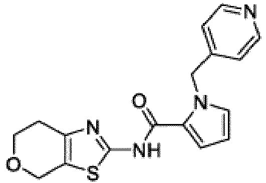 |
| **A96** | 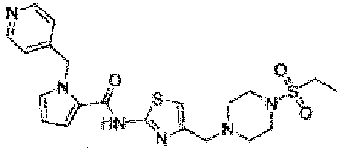 |
| **A97** | 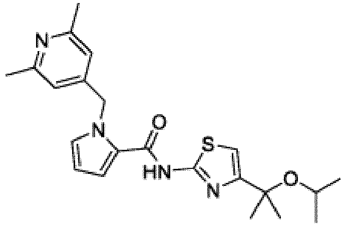 |
| **A98** | 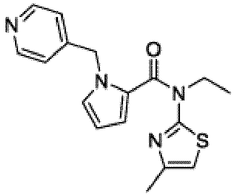 |
| **A99** | 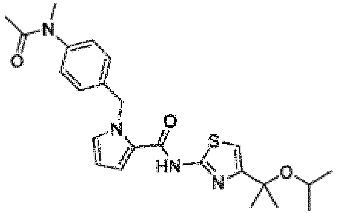 |
| **A100** | 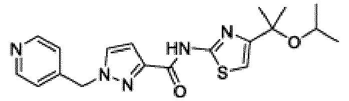 |
| **A101** | 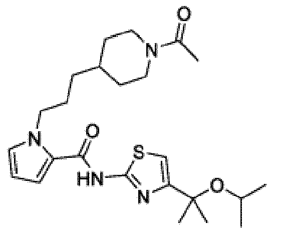 |
| **A102** | 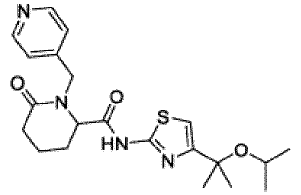 |
| **A103** | 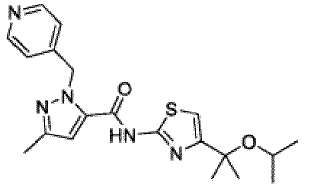 |
| **A104** | 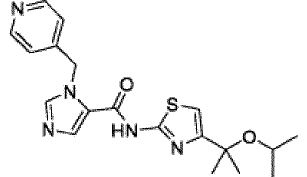 |
| **A105** | 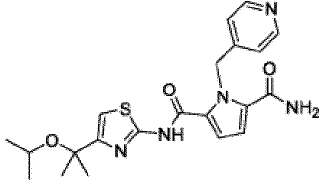 |
| **A106** | 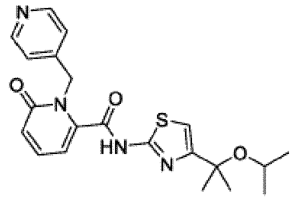 |
| **A107** | 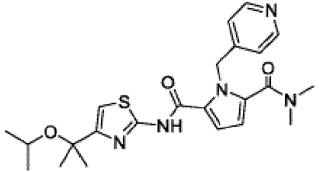 |
| **A108** | 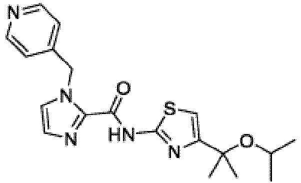 |
| **A109** | 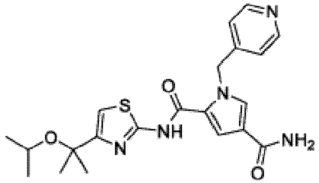 |
| **A110** | 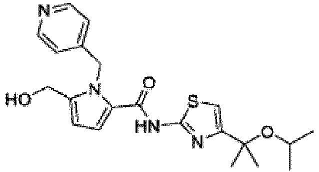 |
| **A111** | 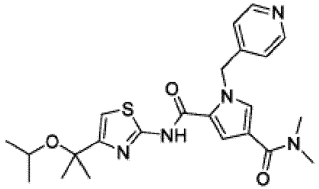 |
| **A112** | 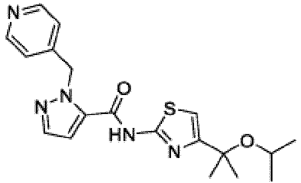 |
| **A113** | 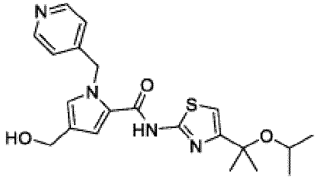 |
| **A114** | 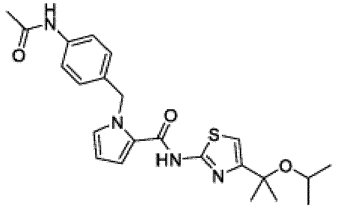 |
| **A115** | 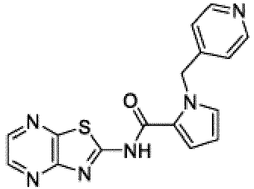 |
| **A116** | 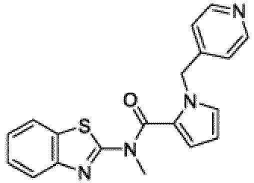 |
| **A117** | 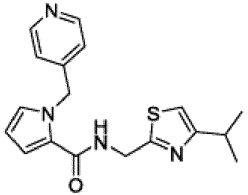 |
| **A118** | 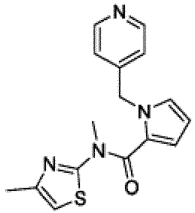 |
| **A119** | 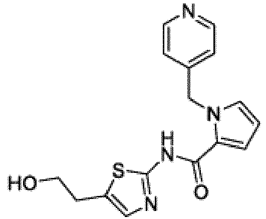 |
| **A120** | 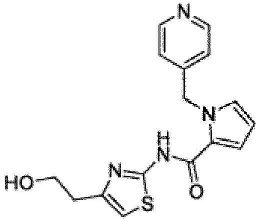 |
| **A121** | 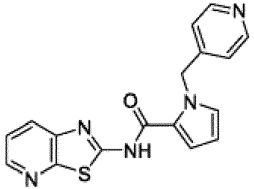 |
| **A122** | 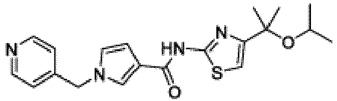 |
| **A123** | 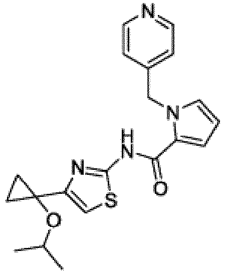 |
| **A124** | 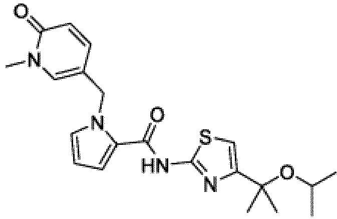 |
| **A126** | 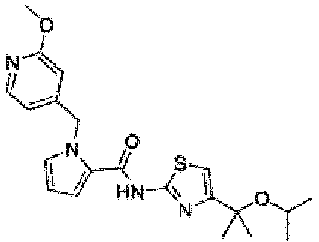 |
| **A126** | 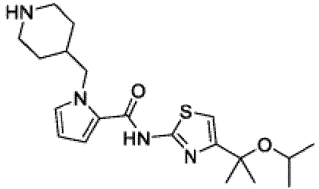 |
| **A127** | 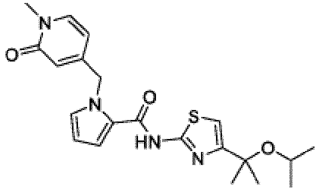 |
| **A129** | 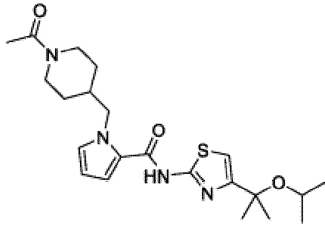 |
| **A129** | 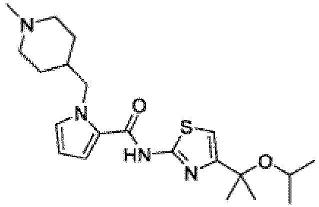 |
| **A130** | 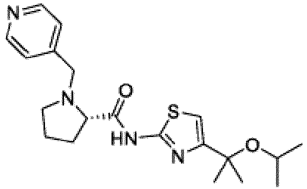 |
| **A131** | 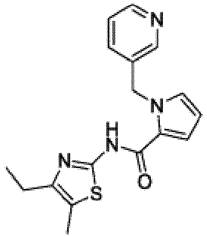 |
| **A132** | 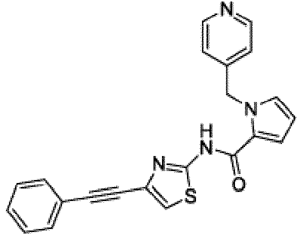 |
| **A133** | 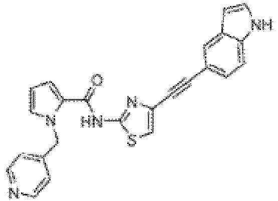 |
| **A134** | 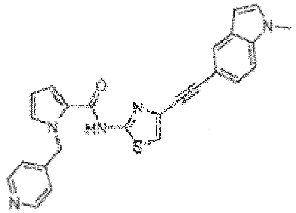 |
| **A135** | 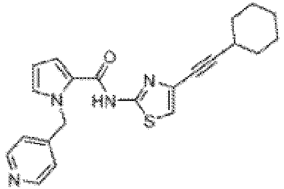 |
| **A136** | 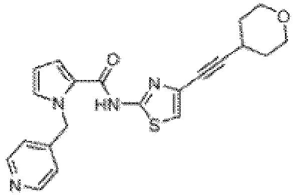 |
| **A137** | 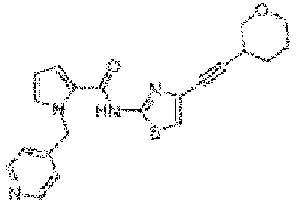 |
| **A138** | 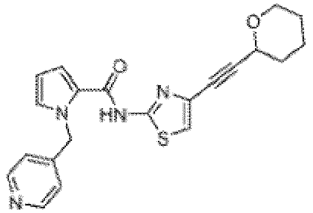 |
| **A139** | 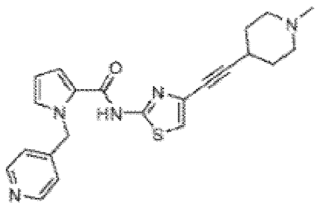 |
| **A140** | 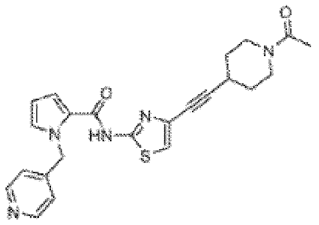 |
| **A141** | 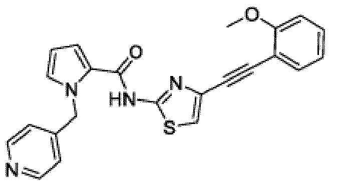 |
| **A142** | 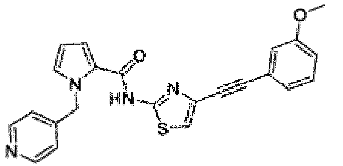 |
| **A143** | 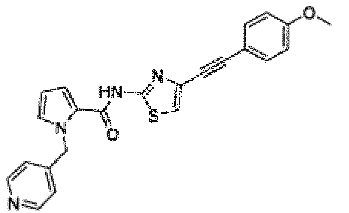 |
| **A144** | 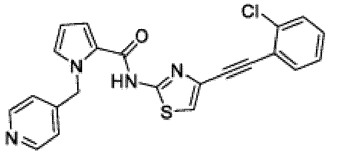 |
| **A145** | 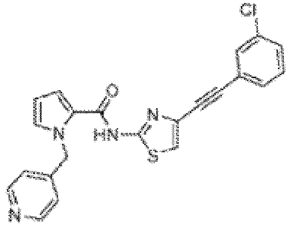 |
| **A146** | 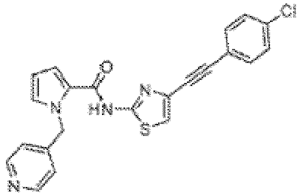 |
| **A147** | 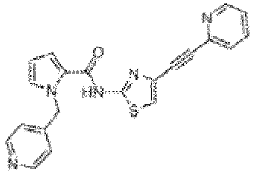 |
| **A148** | 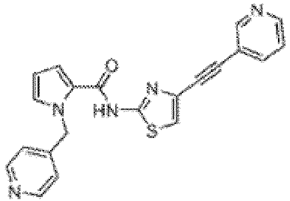 |
| **A149** | 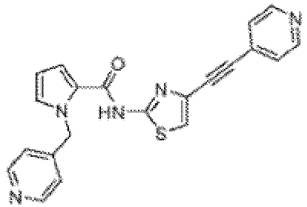 |
| **A1**50 | 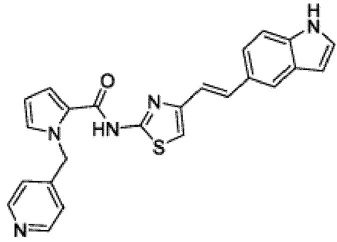 |
| **A151** | 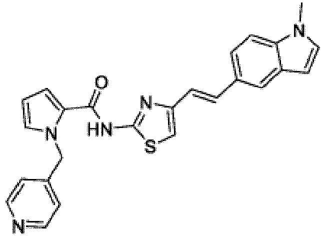 |
| **A152** | 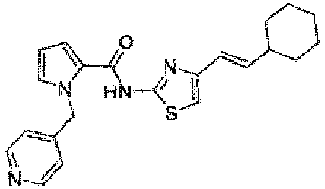 |
| **A153** | 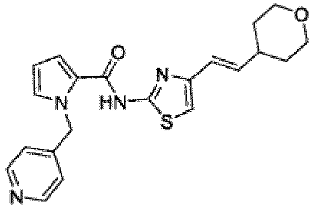 |
| **A154** | 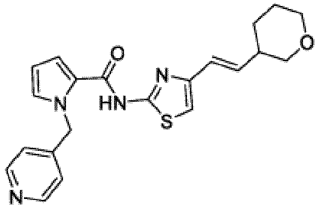 |
| **A165** | 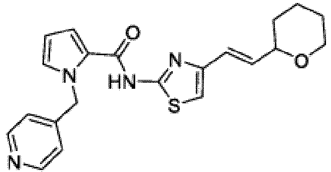 |
| **A156** | 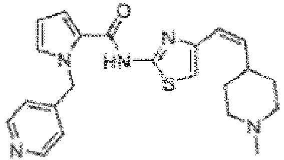 |
| **A167** | 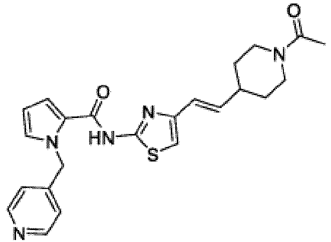 |
| **A158** | 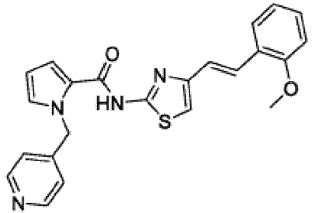 |
| **A159** | 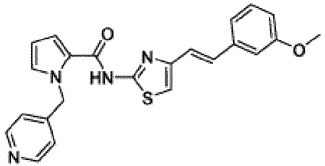 |
| **A160** | 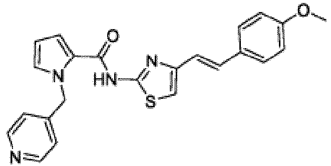 |
| **A161** | 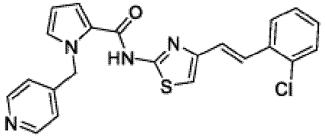 |
| **A162** | 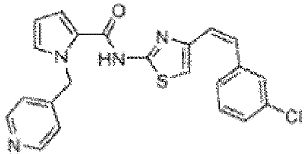 |
| **A163** | 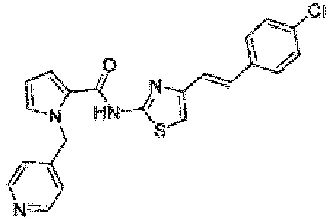 |
| **A164** | 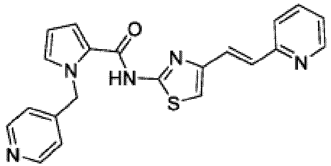 |
| **A165** | 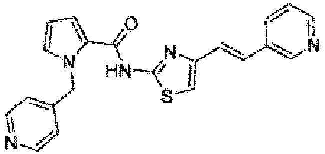 |
| **A166** | 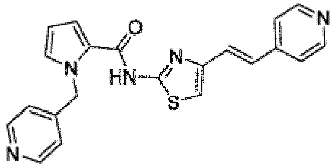 |
| **A167** | 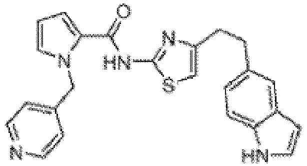 |
| **A168** | 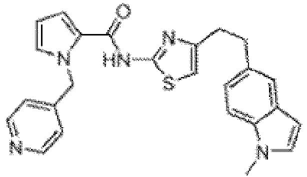 |
| **A169** | 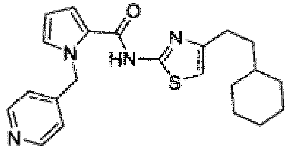 |
| **A170** | 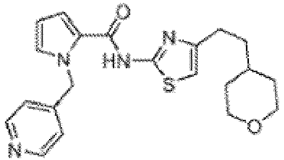 |
| **A171** | 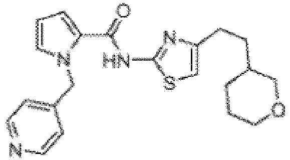 |
| **A172** | 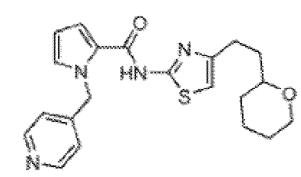 |
| **A173** | 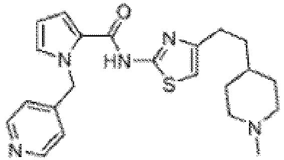 |
| **A174** | 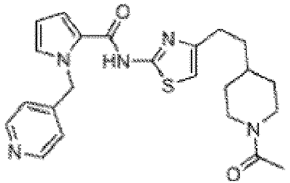 |
| **A175** | 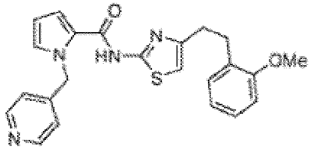 |
| **A176** | 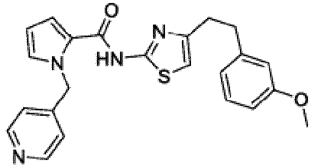 |
| **A177** | 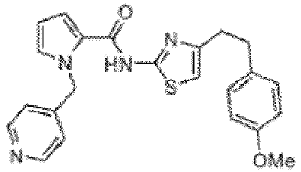 |
| **A178** | 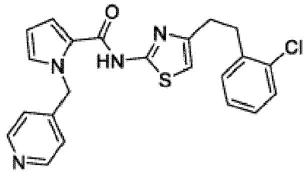 |
| **A179** | 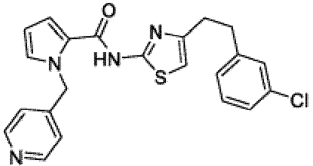 |
| **A180** | 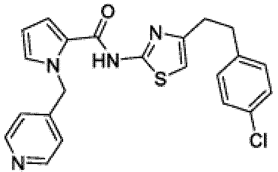 |
| **A181** | 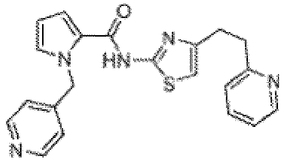 |
| **A182** | 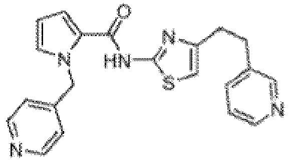 |
| **A183** | 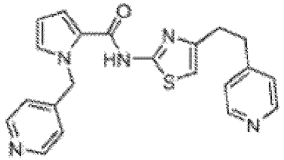 |
| **A184** | 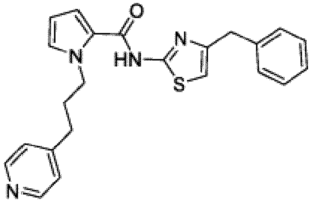 |
| **A185** | 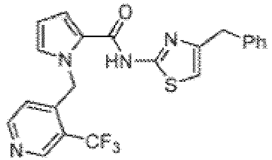 |
| **A186** | 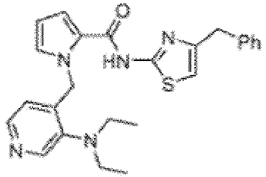 |
| **A187** | 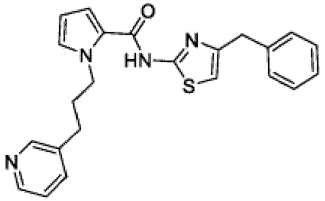 |
| **A188** | 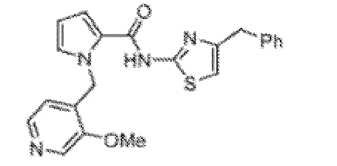 |
| **A189** | 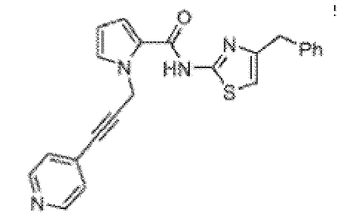 |
| **A190** | 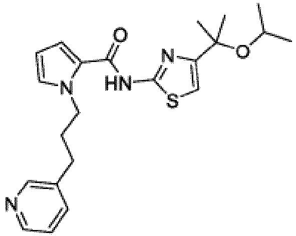 |
| **A191** | 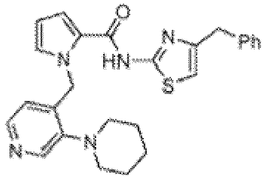 |
| **A192** | 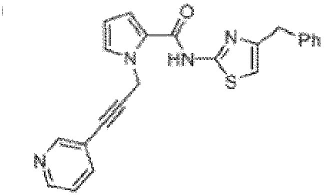 |
| **A193** | 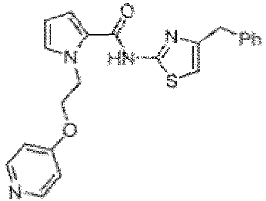 |
| **A194** | 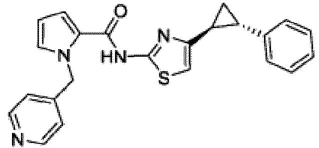 |
| **A195** | 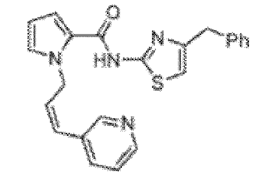 |
| **A196** | 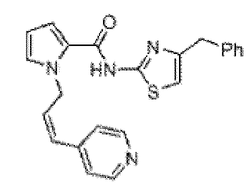 |
| **A197** | 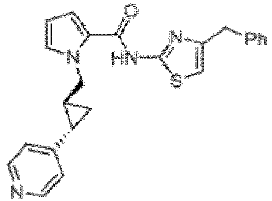 |
| **A198** | 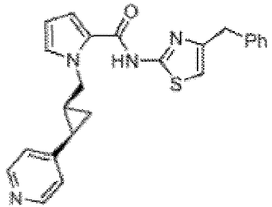 |
| **A199** | 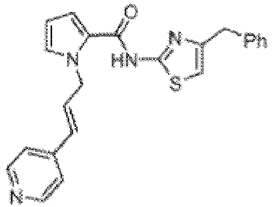 |
| **A200** | 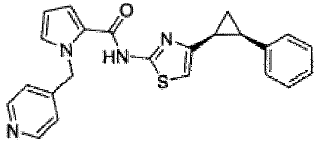 |
| **A201** | 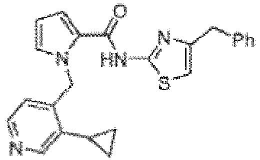 |
| **A202** | 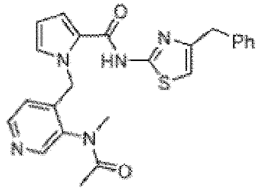 |
| **A203** | 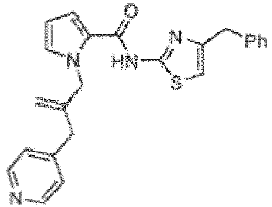 |
| **A204** | 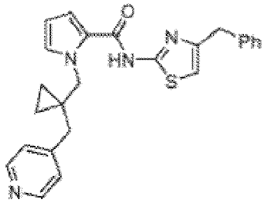 |
| **A205** | 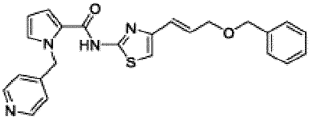 |
| **A206** | 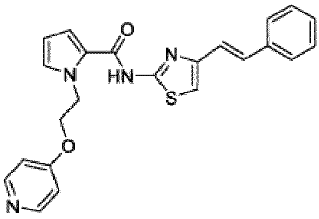 |
| **A207** | 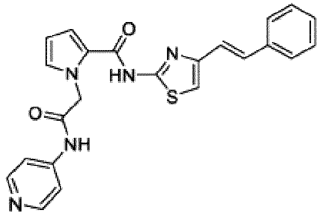 |
| **A208** | 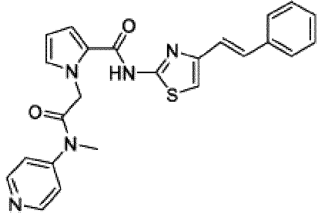 |
| **A209** | 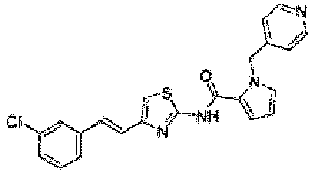 |
| **A210** | 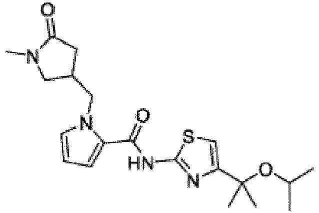 |
| **A211** | 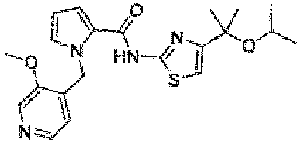 |
| **A212** | 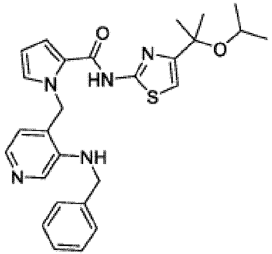 |
| **A213** | 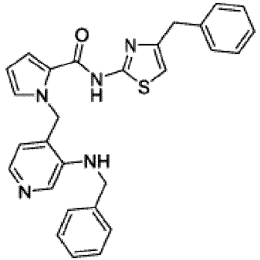 |
| **A214** | 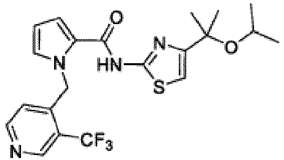 |
| **A215** | 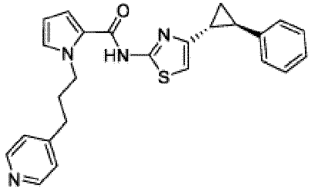 |
| **A216** | 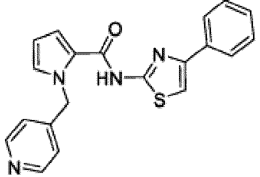 |
| **A217** | 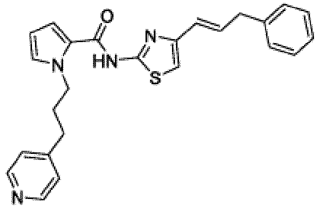 |
| **A218** | 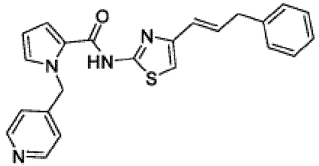 |
| **A219** | 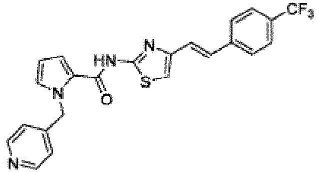 |
| **A220** | 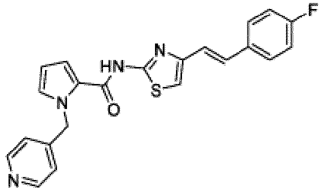 |
| **A221** | 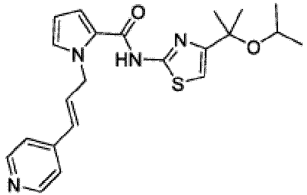 |
| **A222** | 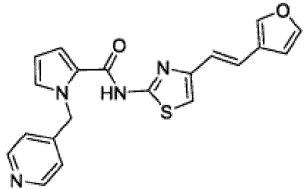 |
| **A223** | 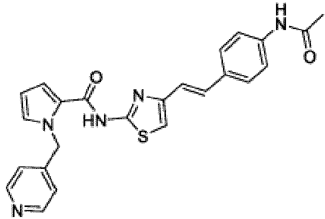 |
| **A224** | 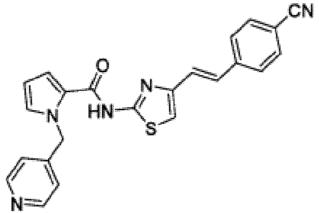 |
| **A226** | 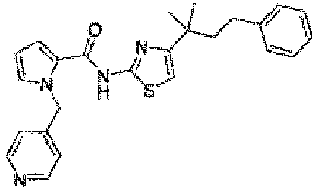 |
| **A226** | 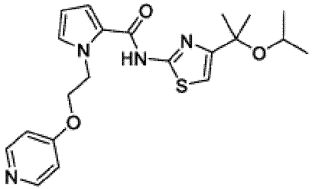 |
| **A227** | 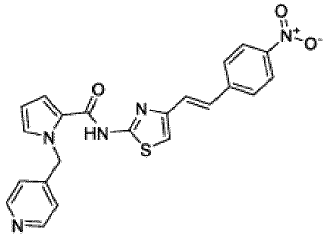 |
| **A228** | 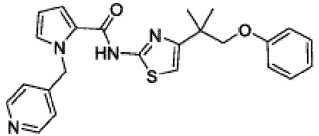 |
| **A229** | 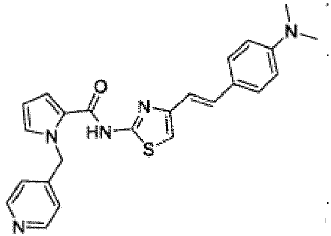 |
| **A230** | 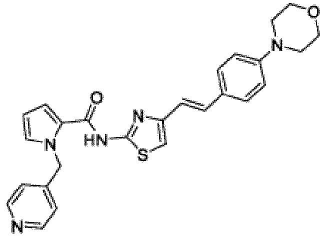 |
| **A231** | 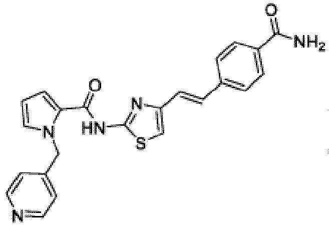 |
| **A232** | 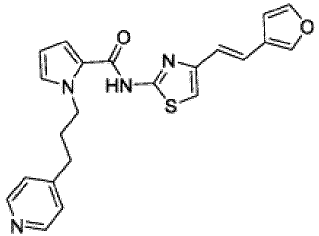 |
| **A233** | 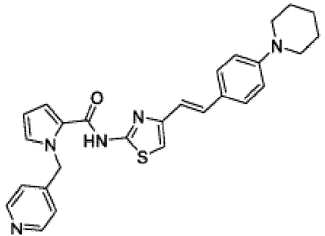 |
| **A234** | 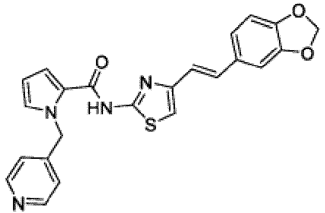 |
| **A235** | 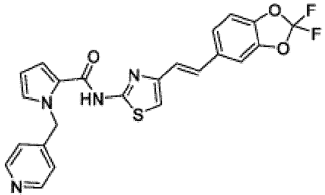 |
| **A236** | 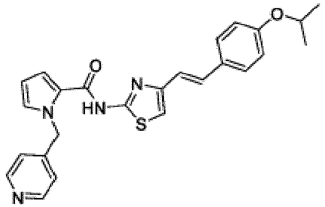 |
| **A237** | 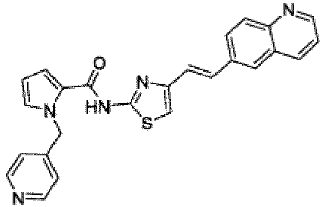 |
| **A238** | 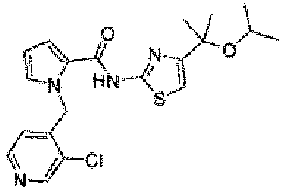 |
| **A239** | 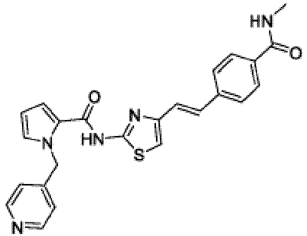 |
| **A240** | 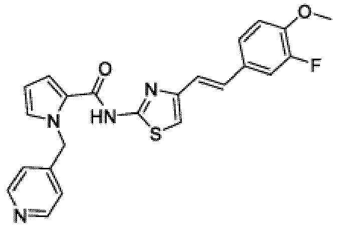 |
| **A241** | 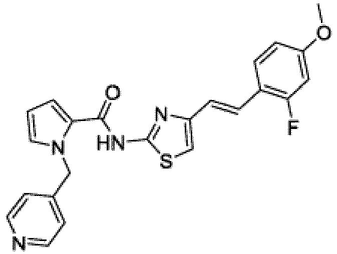 |
| **A242** | 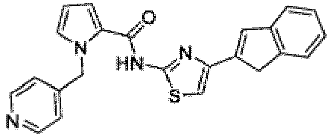 |
| **A243** | 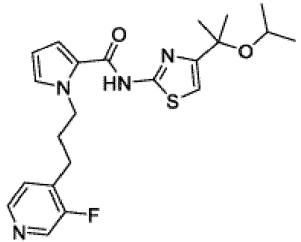 |
| **A244** | 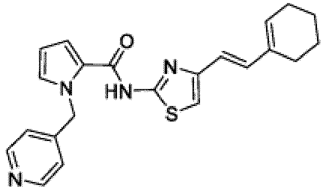 |
| **A245** | 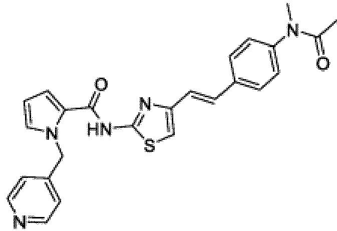 |
| **A246** | 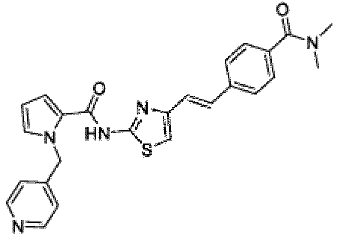 |
| **A247** | 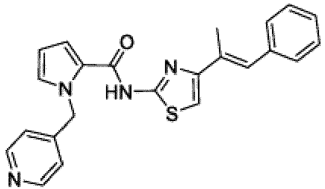 |
| **A248** | 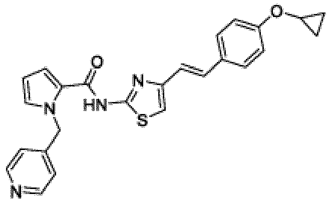 |
| **A249** | 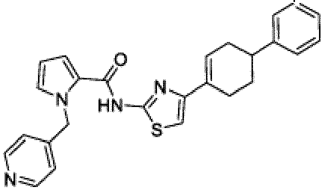 |
| **A250** | 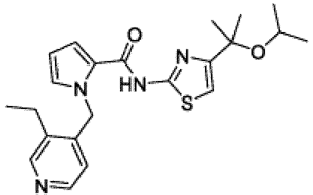 |
| **A251** | 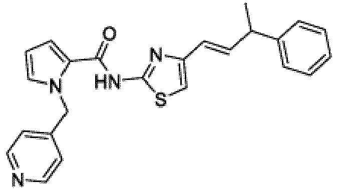 |
| **A252** | 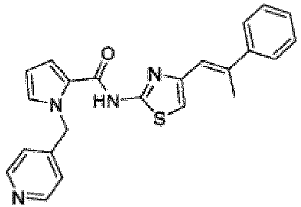 |
| **A253** | 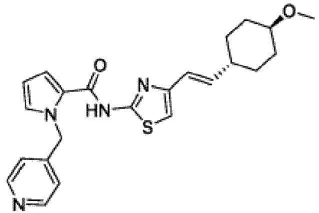 |
| **A254** | 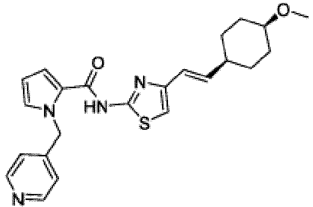 |
| **A255** | 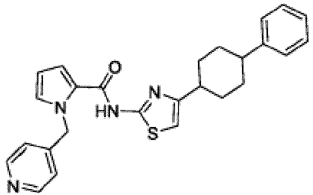 |
| **A256** | 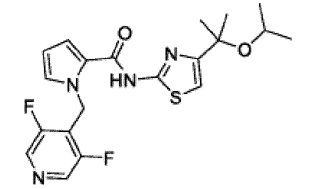 |
| **A267** | 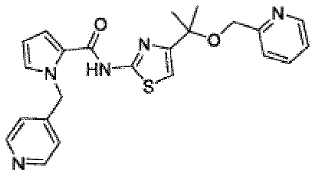 |
| **A258** | 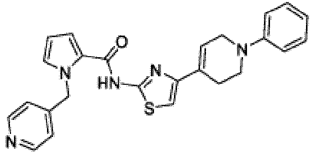 |
| **A259** | 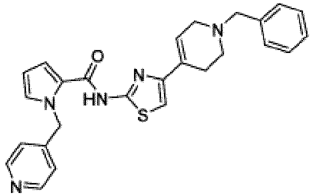 |
| **A260** | 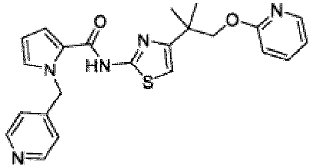 |
| **A261** | 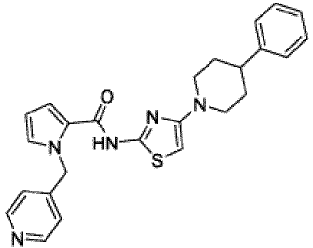 |
| **A262** | 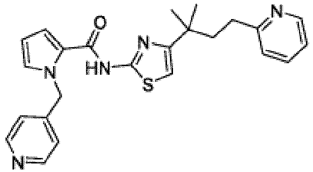 |
| **A263** | 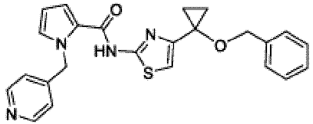 |
| **A264** | 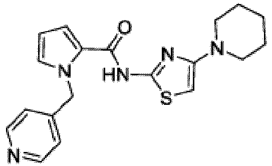 |
| **A265** | 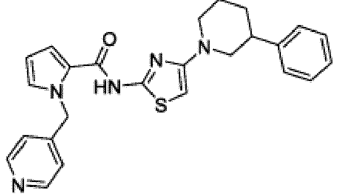 |
| **A266** | 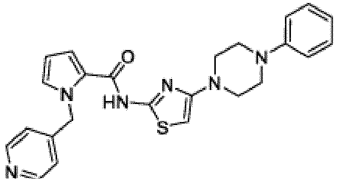 |
| **A267** | 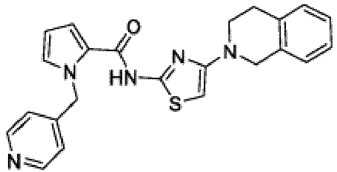 |
| **A268** | 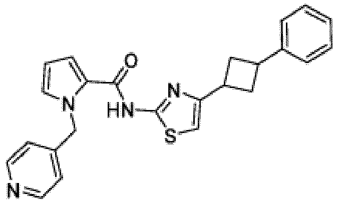 |
| **A269** | 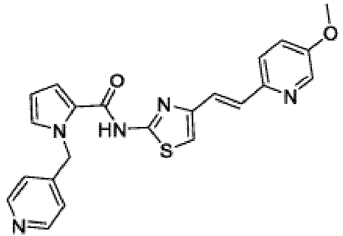 |
| **A270** | 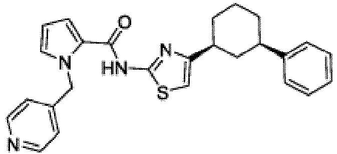 |
| **A271** | 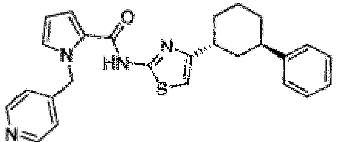 |
| **A272** | 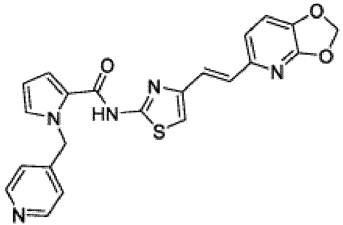 |
| **A273** | 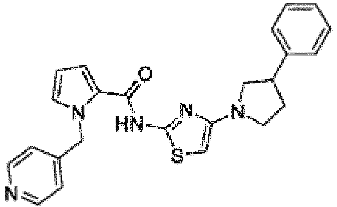 |
| **A274** | 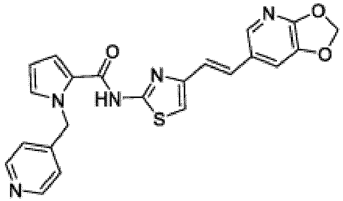 |
| **A275** | 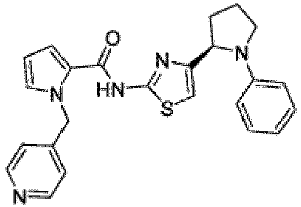 |
| **A276** | 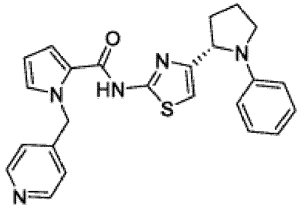 |
| **A277** | 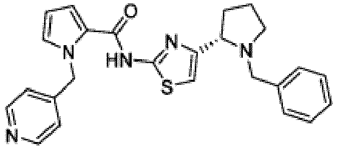 |
| **A278** | 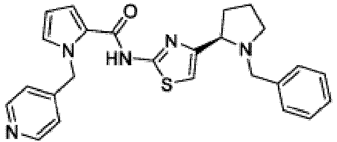 |
| **A279** | 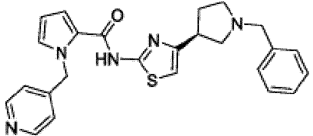 |
| **A280** | 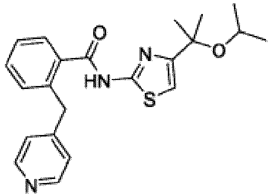 |
| **A281** | 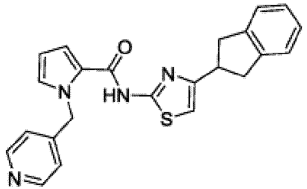 |
| **A282** | 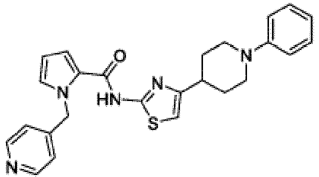 |
| **A283** | 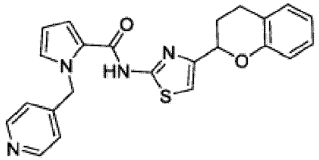 |
| **A284** | 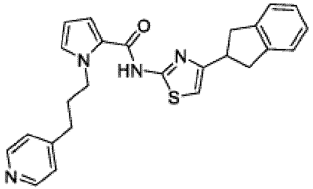 |
| **A285** | 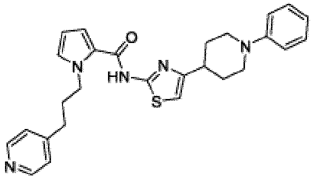 |
| **A286** | 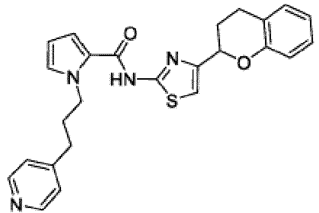 |
| **A287** | 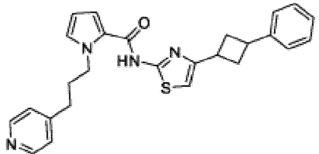 |
| **A288** | 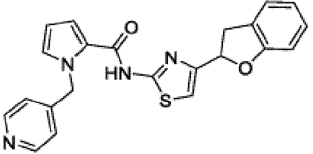 |
| **A289** | 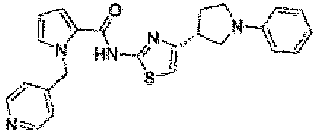 |
| **A290** | 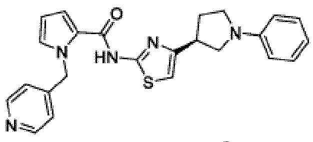 |
| **A291** | 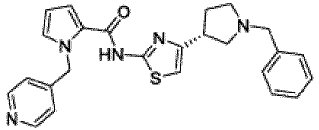 |
| **A292** | 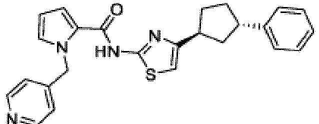 |
| **A293** | 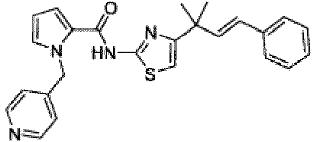 |
| **A294** | 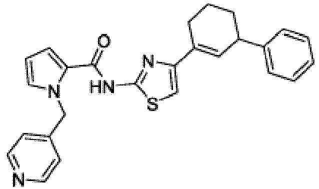 |
| **A295** | 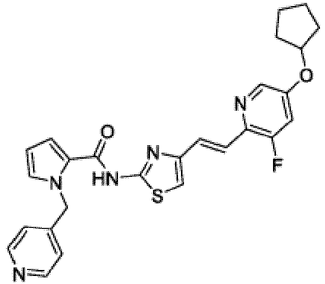 |
| **A296** | 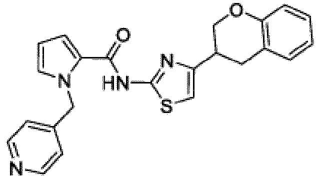 |
| **A297** | 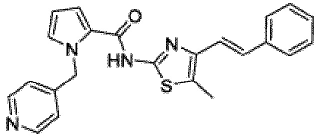 |
| **A298** | 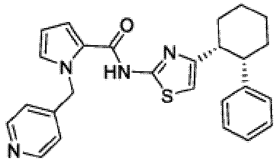 |
| **A299** | 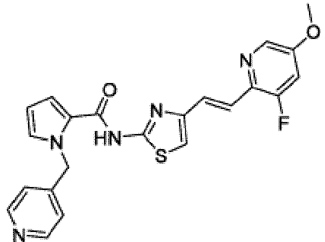 |
| **A300** | 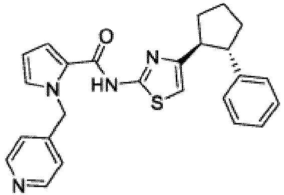 |
| **A301** | 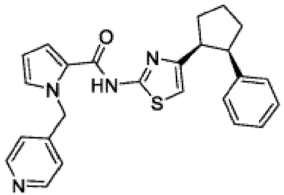 |
| **A302** | 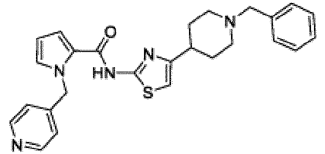 |
| **A303** | 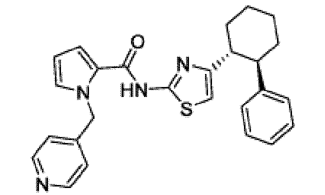 |
| **A304** | 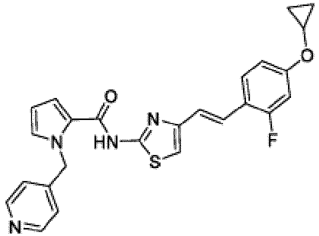 |
| **A305** | 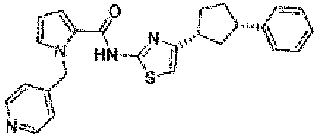 |
| **A306** | 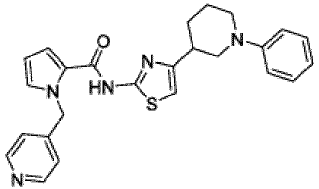 |
| **A307** | 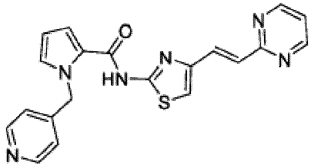 |
| **A308** | 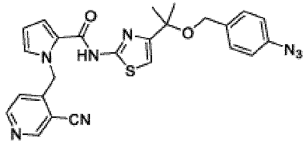 |
| **A309** | 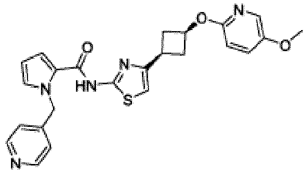 |
| **A310** | 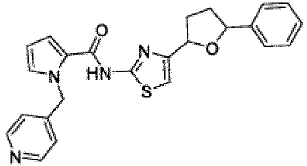 |
| **A311** | 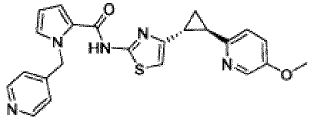 |
| **A312** | 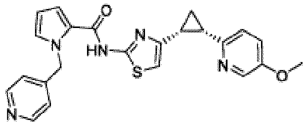 |
| **A313** | 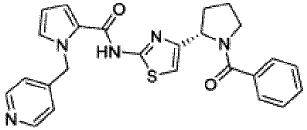 |
| **A314** | 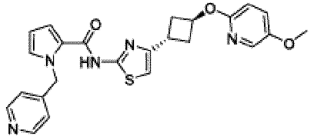 |
| **A315** | 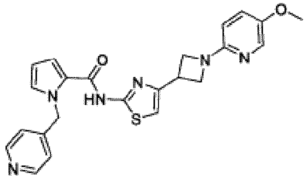 |
| **A316** | 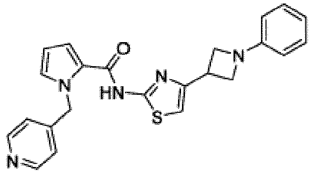 |
| **A317** | 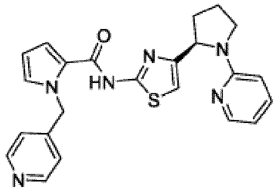 |
| **A318** | 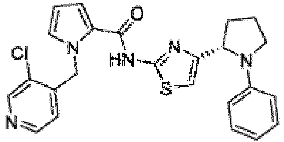 |
| **A319** | 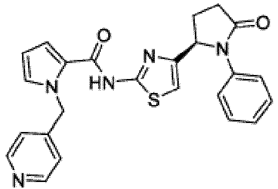 |
| **A320** | 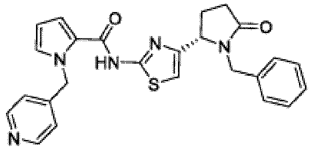 |
| **A321** | 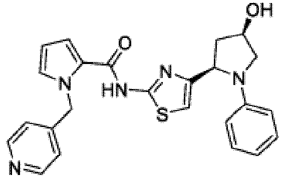 |
| **A322** | 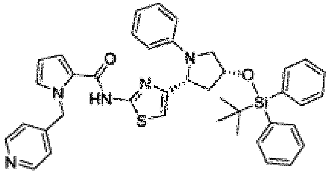 |
| **A323** | 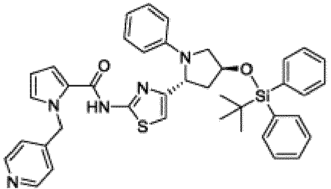 |
| **A324** | 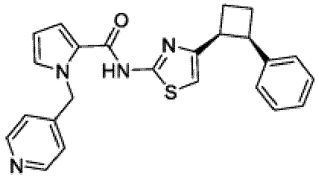 |
| **A326** | 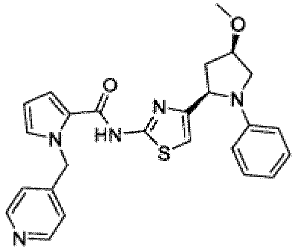 |
| **A326** | 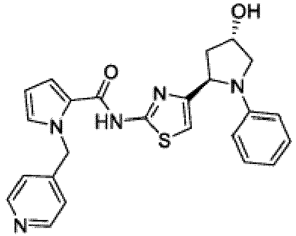 |
| **A327** | 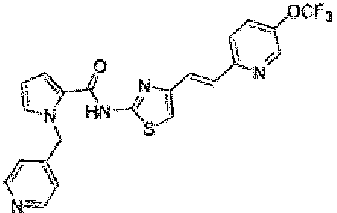 |
| **A328** | 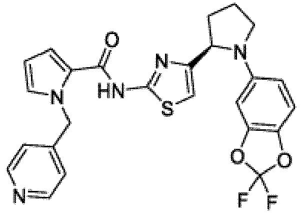 |
| **A329** | 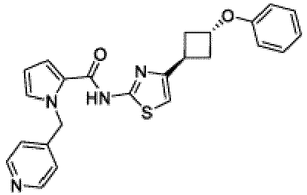 |
| **A330** | 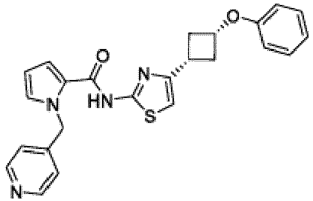 |
| **A331** | 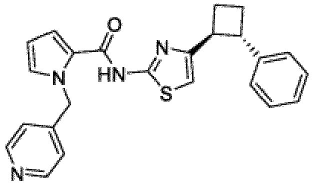 |
| **A332** | 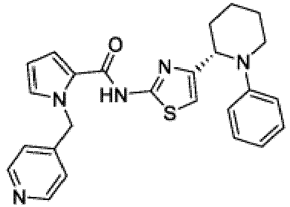 |
| **A333** | 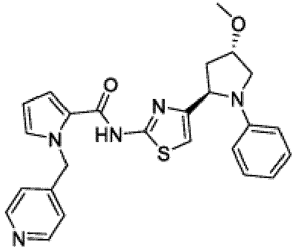 |
| **A334** | 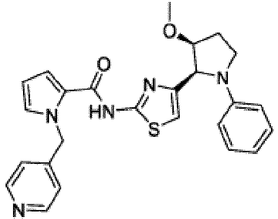 |
| **A335** | 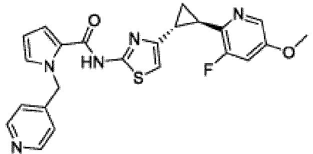 |
| **A336** | 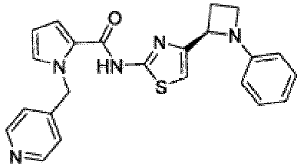 |
| **A337** | 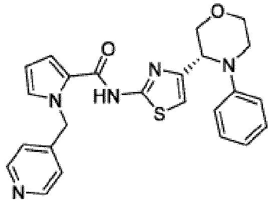 |
| **A338** | 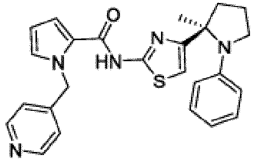 |
| **A339** | 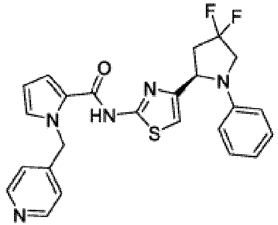 |
| **A340** | 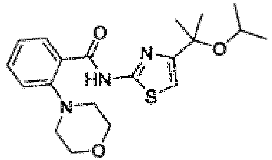 |
| **A341** | 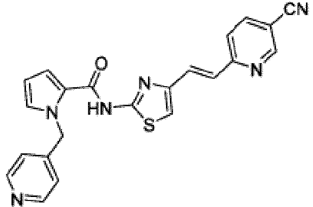 |
| **A342** | 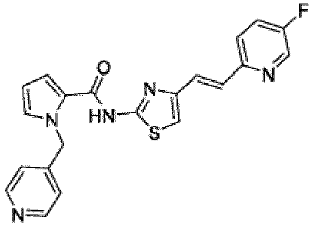 |
| **A343** | 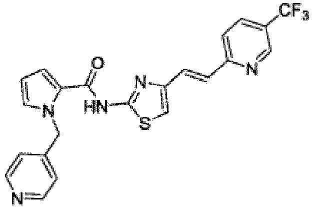 |
| **A344** | 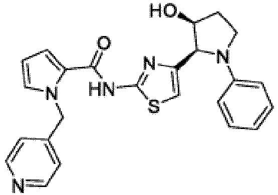 |
| **A346** | 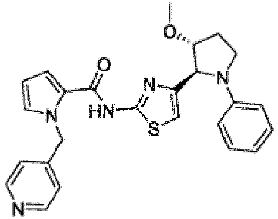 |
| **A346** | 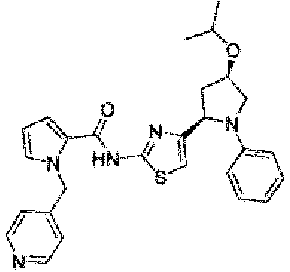 |
| **A347** | 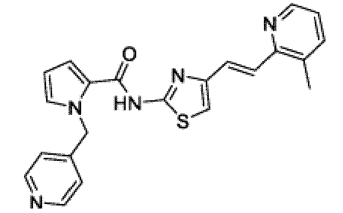 |
| **A348** | 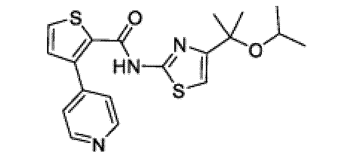 |
| **A349** | 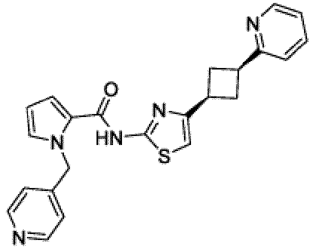 |
| **A350** | 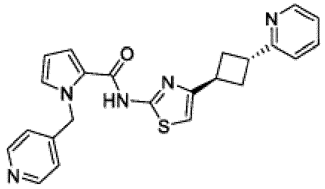 |
| **A351** | 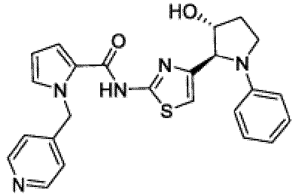 |
| **A352** | 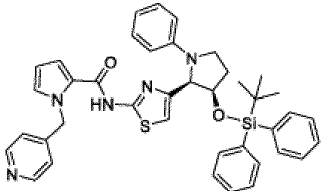 |
| **A353** | 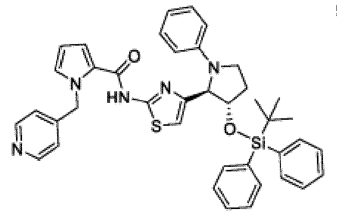 |
| **A354** | 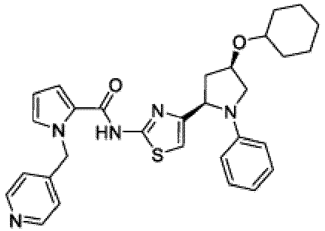 |
| **A355** | 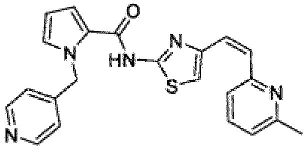 |
| **A356** | 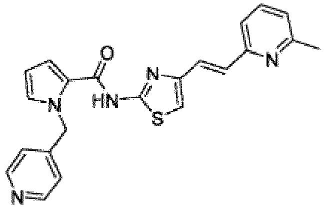 |
| **A357** | 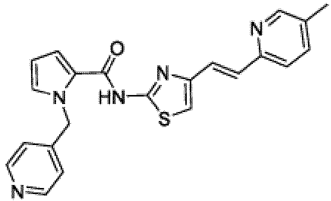 |
| **A358** | 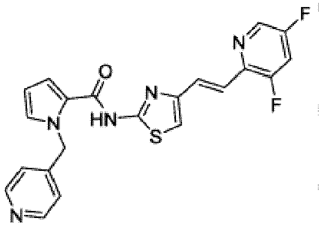 |
| **A359** | 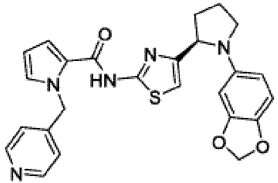 |
| **A360** | 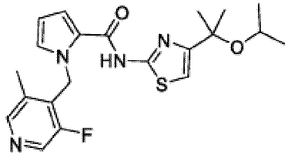 |
| **A361** | 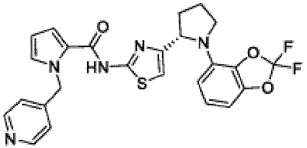 |
| **A362** | 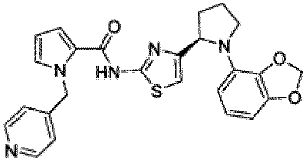 |
| **A363** | 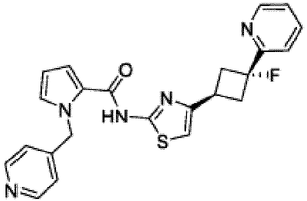 |
| **A364** | 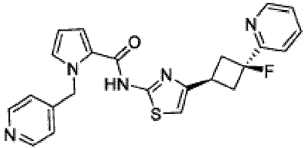 |
| **A356** | 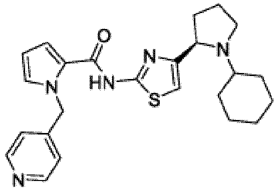 |
| **A366** | 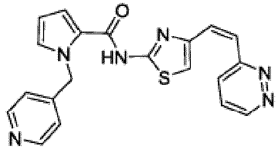 |
| **A367** | 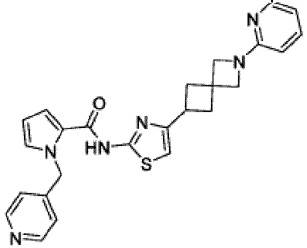 |
| **A368** | 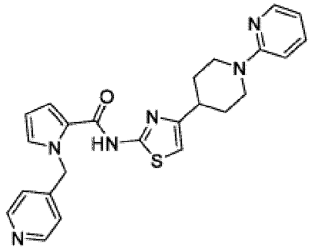 |
| **A369** | 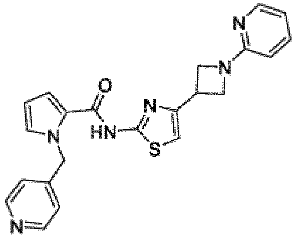 |
| **A370** | 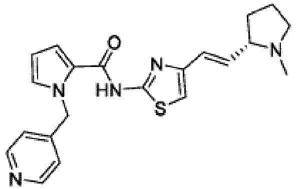 |
| **A371** | 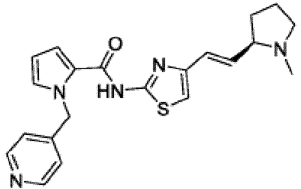 |
| **A372** | 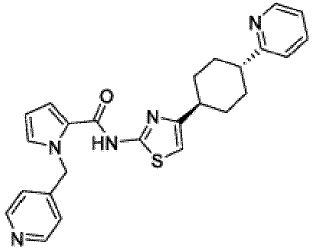 |
| **A373** | 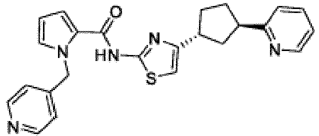 |
| **A374** | 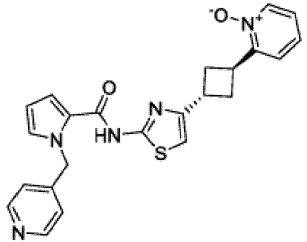 |
| **A375** | 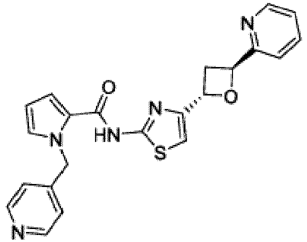 |
| **A376** | 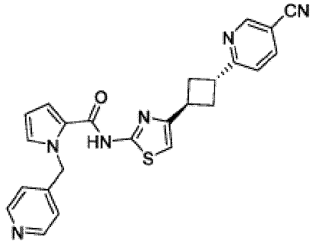 |
| **A377** | 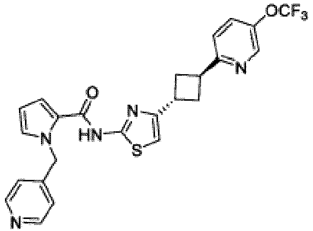 |
| **A378** | 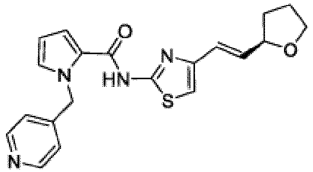 |
| **A379** | 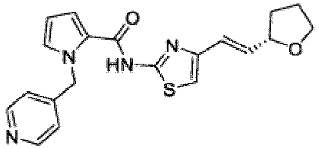 |
| **A380** | 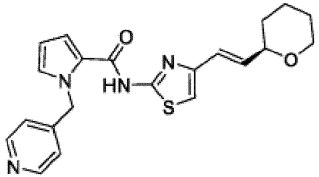 |
| **A381** | 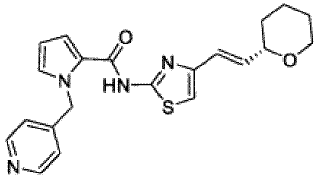 |
| **A382** | 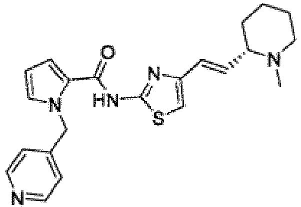 |
| **A383** | 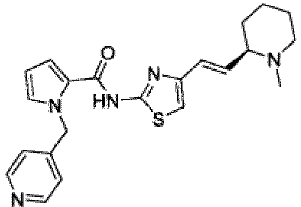 |
| **A384** | 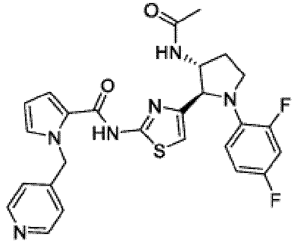 |
| **A385** | 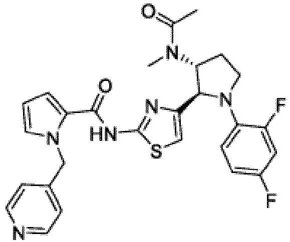 |
| **A386** | 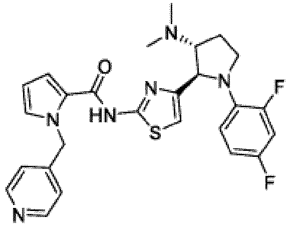 |
| **A367** | 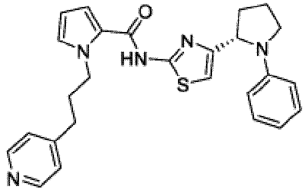 |
| **A388** | 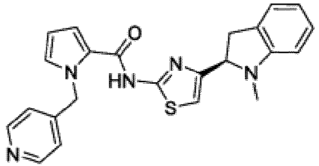 |
| **A389** | 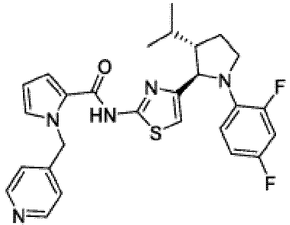 |
| **A390** | 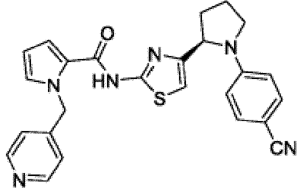 |
| **A391** | 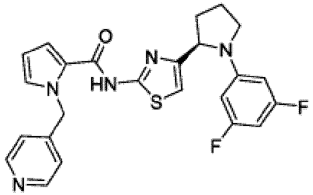 |
| **A392** | 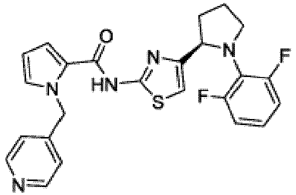 |
| **A393** | 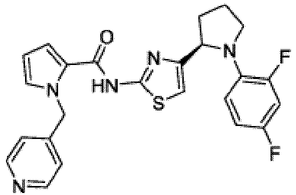 |
| **A394** | 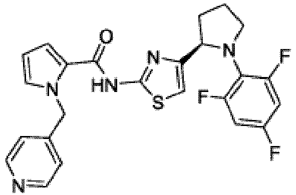 |
| **A395** | 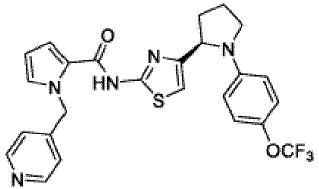 |
| **A396** | 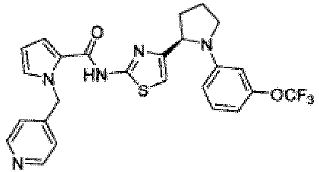 |
| **A397** | 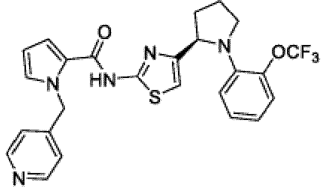 |
| **A398** | 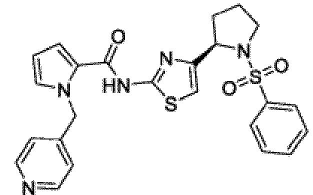 |
| **A399** | 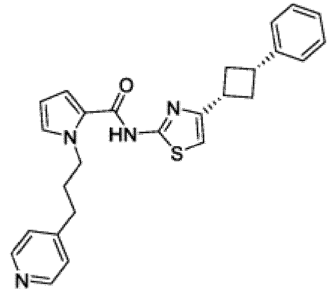 |
| **A400** | 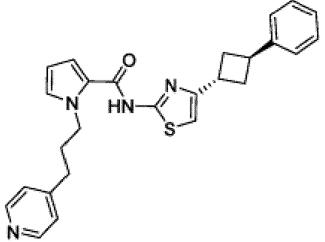 |
| **A401** | 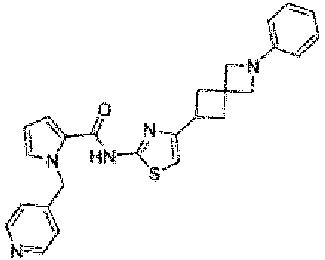 |
| **A402** | 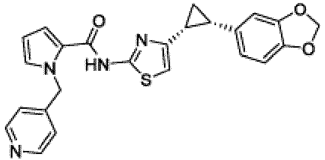 |
| **A403** | 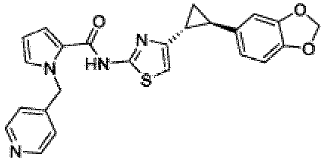 |

**Table B**

| | |
|---|---|
| **Ref. B1** | 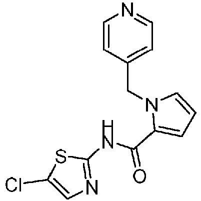 |
| **B2** | 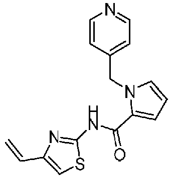 |
| **Ref. B3** | 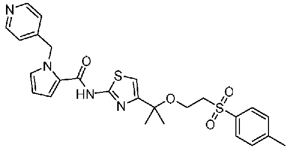 |
| **Ref. B4** | 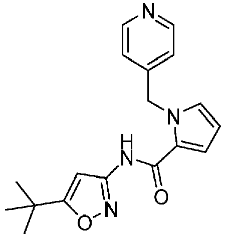 |
| **Ref. B5** | 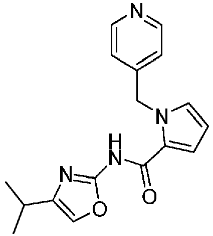 |
| **Ref. B6** | 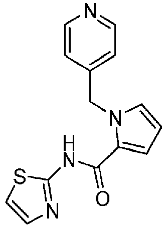 |
| **Ref. B7** | 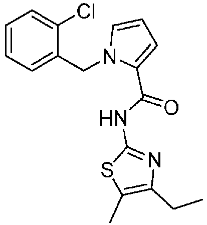 |
| **Ref. B8** | 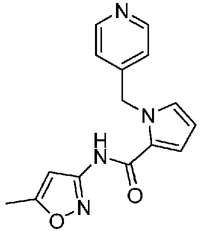 |
| **Ref. B9** | 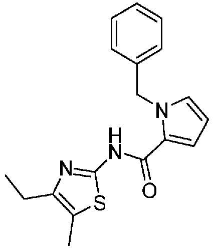 |
| **Ref. B10** | 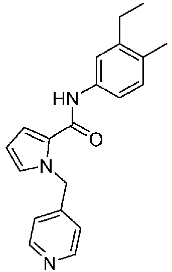 |
| **Ref. B11** | 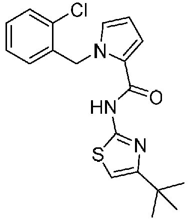 |
| **Ref. B12** | 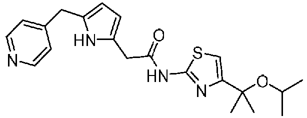 |
| **Ref. B13** | 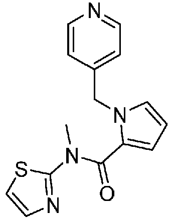 |
| **Ref. B14** | 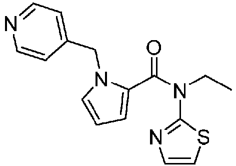 |
| **Ref. B15** | 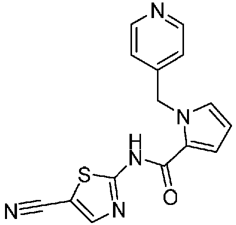 |
| **Ref. B16** | 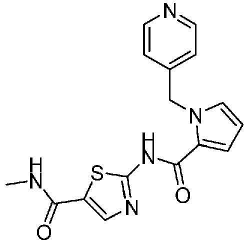 |
| **Ref. B17** | 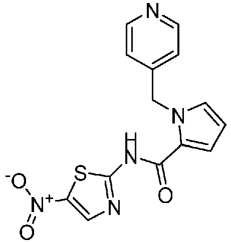 |
| **Ref. B18** | 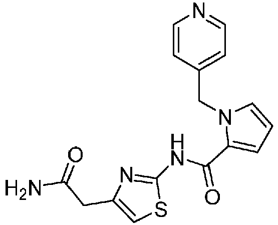 |
| **Ref. B19** | 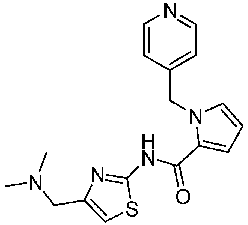 |
| **Ref. B20** | 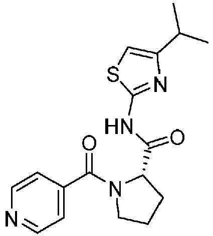 |
| **Ref. B21** | 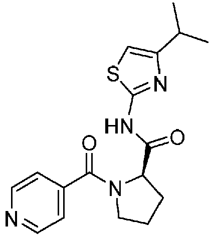 |
| **Ref. B22** | 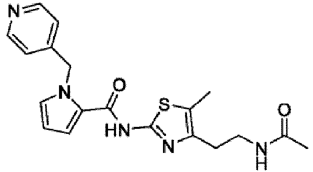 |
| **Ref. B23** | 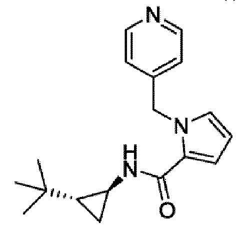 |
| **Ref. B24** | 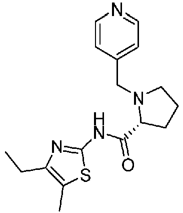 |
| **Ref. B25** | 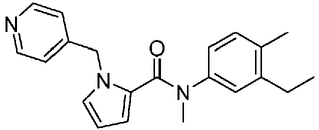 |
| **Ref. B26** | 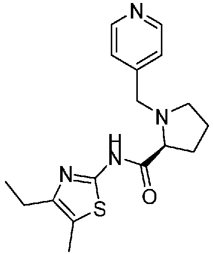 |
| **Ref. B27** | 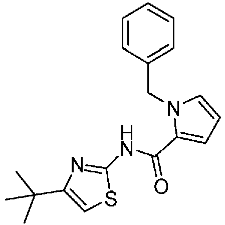 |
| **Ref. B28** | 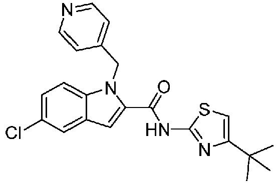 |
| **Ref. B29** | 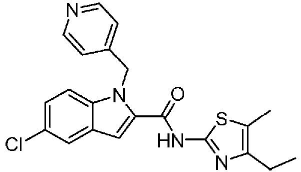 |

In embodiments, the compound is selected from a compound listed in Table A, or a pharmaceutically acceptable salt thereof. In some embodiments, the compound or salt is selected from A1-A210. In some embodiments, the compound or salt is selected from A211-A403. In some cases, the compound is selected from the group consisting of 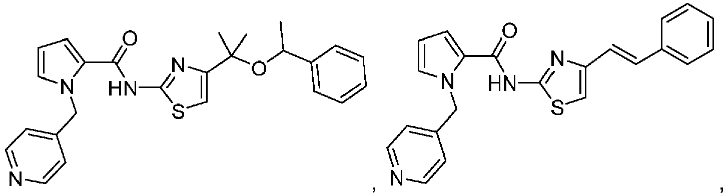 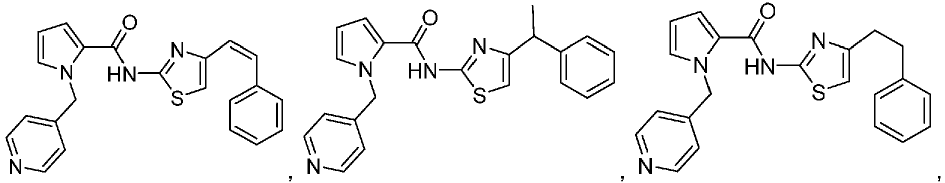 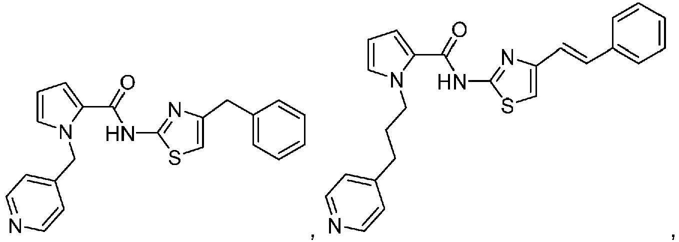 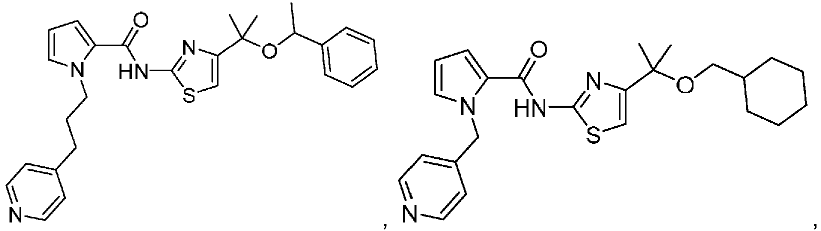 and 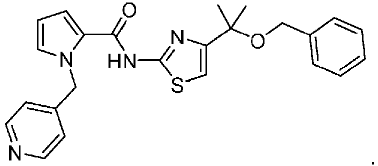 In some cases, the compound or salt is selected from the group consisting of 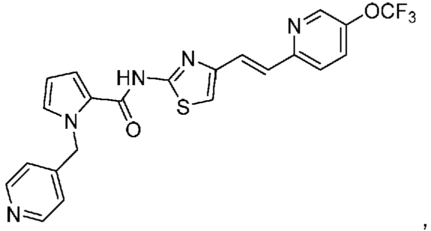 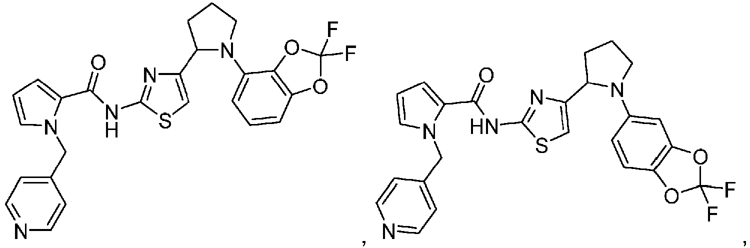 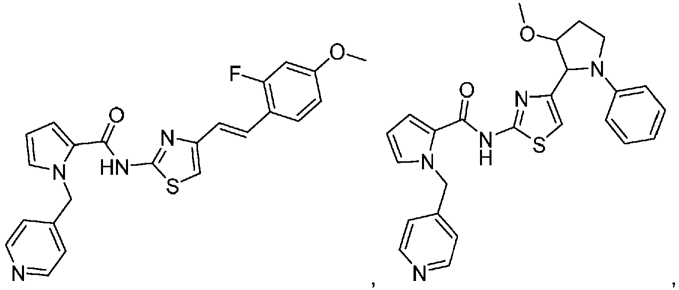 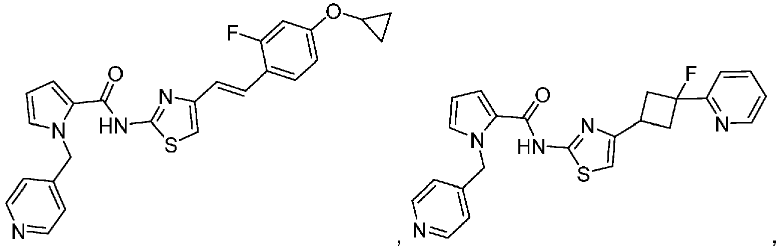 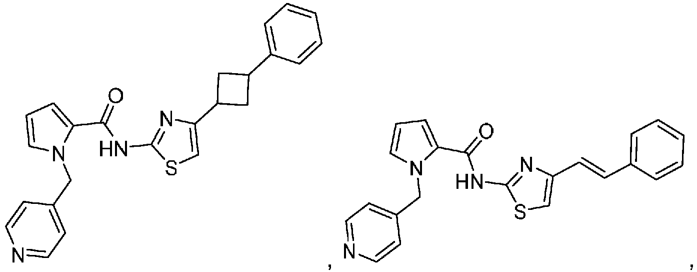 and The chemical structures having one or more stereocenters depicted with dashed and bold wedged bonds (i.e., 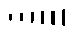 and 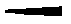 ) are meant to indicate absolute stereochemistry of the stereocenter(s) present in the chemical structure. Bonds symbolized by a simple line do not indicate a stereo-preference. Bonds symbolized by dashed or bold straight bonds (i.e., 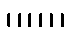 and 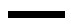 ) are meant to indicate a relative stereochemistry of the stereocenter(s) present in the chemical structure. Unless otherwise indicated to the contrary, chemical structures that include one or more stereocenters which are illustrated herein without indicating absolute or relative stereochemistry, encompass all possible stereoisomeric forms of the compound (e.g., diastereomers, enantiomers) and mixtures thereof. Structures with a single bold or dashed wedged line, and at least one additional simple line, encompass a single enantiomeric series of all possible diastereomers. Similarly, the chemical structures having alkenyl groups are meant to encompass both cis and trans orientations, or when substituted, E- and Z-isomers of the chemical structure.

### Synthesis of Protein Secretion Inhibitors

The compounds provided herein can be synthesized using conventional

techniques readily available starting materials known to those skilled in the art. In general, the compounds provided herein are conveniently obtained via standard organic chemistry synthesis methods.

Although not limited to any one or several sources, classic texts such as Smith, M. B., March, J., March' s Advanced Organic Chemistry: Reactions, Mechanisms, and Structure, 5th edition, John Wiley & Sons: New York, 2001 ; and Greene, T.W., Wuts, P.G. M., Protective Groups in Organic Synthesis, 3rd edition, John Wiley & Sons: New York, 1999, are useful and recognized reference textbooks of organic synthesis known to those in the art. The following descriptions of synthetic methods are designed to illustrate, but not to limit, general procedures for the preparation of compounds of the present disclosure.

The synthetic processes disclosed herein can tolerate a wide variety of functional groups; therefore, various substituted starting materials can be used. The processes generally provide the desired final compound at or near the end of the overall process, although it may be desirable in certain instances to further convert the compound to a pharmaceutically acceptable salt thereof.

In general, the compounds of Formula (III) can be synthesized in line with the examples shown below. For example, the compounds can be prepared by alkylation of the appropriate amine having a carboxyl group, with appropriate protecting groups as necessary. The intermediate can be saponified, for example, to expose a reactive carboxylate. Then, amide coupling between the appropriate amine and the free carboxylate can occur.

The amine for the amide coupling noted above can be prepared via known synthetic techniques using appropriate starting materials and protecting groups, as necessary.

Further modifications can be performed, e.g., to introduce additional substituents such as halo groups or alkyl groups.

### Useof the Compounds

The compounds disclosed herein (i.e., the compounds of Formula (III), the compounds listed in Table A and pharmaceutically acceptable salts of any of the foregoing) can inhibit protein secretion of a protein of interest. The compounds disclosed herein can interfere with the Sec61 protein secretion machinery of a cell. In some cases, a compound as disclosed herein inhibits secretion of one or more of TNFα, IL2, Her3, and PD-1, or each of TNFα, IL2, Her3, and PD-1. Protein secretion activity can be assessed in a manner as described in the Examples section below.

As used herein, the term "inhibitor" is meant to describe a compound that blocks or reduces an activity of a pharmacological target (for example, a compound that inhibits Sec61 function in the protein secretion pathway). An inhibitor can act with competitive, uncompetitive, or noncompetitive inhibition. An inhibitor can bind reversibly or irreversibly, and therefore, the term includes compounds that are suicide substrates of a protein or enzyme. An inhibitor can modify one or more sites on or near the active site of the protein, or it can cause a conformational change elsewhere on the enzyme. The term inhibitor is used more broadly herein than scientific literature so as to also encompass other classes of pharmacologically or therapeutically useful agents, such as agonists, antagonists, stimulants, co-factors.

Thus, described herein are methods of inhibiting protein secretion in a cell. In these methods, a cell is contacted with a compound described herein (i.e., the compounds of Formula (III), the compounds listed in Table A and compound B2, and pharmaceutically acceptable salts of any of the foregoing), or pharmaceutical composition thereof, in an amount effective to inhibit secretion of the protein of interest. In some descriptions, the cell is contacted *in vitro.* In various descriptions, the cell is contacted *in vivo.* In various descriptions, the contacting includes administering the compound or pharmaceutical composition to a subject.

The biological consequences of Sec61 inhibition are numerous. For example, Sec61 inhibition has been suggested for the treatment or prevention of inflammation and/or cancer in a subject. Therefore, pharmaceutical compositions for Sec61 specific compounds, provide a means of administering a drug to a subject and treating these conditions. As used herein, the terms "treat," "treating," "treatment," refer to eliminating, reducing, or ameliorating a disease or condition, and/or symptoms associated therewith. Although not precluded, treating a disease or condition does not require that the disease, condition, or symptoms associated therewith be completely eliminated. As used herein, the terms "treat," "treating," "treatment," may include "prophylactic treatment," which refers to reducing the probability of redeveloping a disease or condition, or of a recurrence of a previously-controlled disease or condition, in a subject who does not have, but is at risk of or is susceptible to, redeveloping a disease or condition or a recurrence of the disease or condition. The term "treat" and synonyms contemplate administering a therapeutically effective amount of a compound of the disclosure to an individual in need of such treatment. Within the meaning of the disclosure, "treatment" also includes relapse prophylaxis or phase prophylaxis, as well as the treatment of acute or chronic signs, symptoms and/or malfunctions. The treatment can be orientated symptomatically, for example, to suppress symptoms. It can be effected over a short period, be oriented over a medium term, or can be a long-term treatment, for example within the context of a maintenance therapy. As used herein, the terms "prevent," "preventing," "prevention," are art-recognized, and when used in relation to a condition, such as a local recurrence (e.g., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount. As used herein, the terms "patient" and "subject" may be used interchangeably and mean animals, such as dogs, cats, cows, horses, and sheep (i.e., non-human animals) and humans. Particular patients are mammals (e.g., humans). The term patient includes males and females.

Inhibition of Sec61-mediated secretion of inflammatory proteins (e.g., TNFα) can disrupt inflammation signaling. Thus, provided herein is the use of a compound described herein, (i.e., a compound of Formula (III), or a compound listed in Table A), or a pharmaceutically acceptable salt thereof in treating inflammation.

Further, the viability of cancer cells relies upon increased protein secretion into the ER for survival. Therefore, non-selective or partially selective inhibition of Sec61 mediated protein secretion may inhibit tumor growth. Alternatively, in the immune-oncology setting, selective secretion inhibitors of known secreted immune checkpoints proteins (e.g., PD-1, TIM-3, LAG3) can result in activation of the immune system to against various cancers.

Accordingly, also provided herein is the use of a compound described herein, (i.e., a compound of Formula (III), or a compound listed in Table A), or a pharmaceutically acceptable salt thereof in treating cancer. Specifically contemplated cancers that can be treated using the compounds and compositions described herein include, but are not limited to melanoma, multiple myeloma, prostate, lung, non small cell lung carconimoa (NSCLC), squamous cell carcinoma, leukemia, acute myelogenous leukemia, chronic myelogenous leukemia, lymphoma, NPM/ALK-transformed anaplastic large cell lymphoma, renal cell carcinoma, rhabdomyosarcoma, ovarian cancer, endometrial cancer, small cell carcinoma, adenocarcinoma, gastric carcinoma, hepatocellular carcinoma, pancreatic cancer, thyroid carcinoma, anaplastic large cell lymphoma, hemangioma, head and neck cancer, bladder, and colorectal cancers.

The compounds described herein are also contemplated to be used in the prevention and/or treatment of a multitude of diseases including, but not limited to, proliferative diseases, neurotoxic/degenerative diseases, ischemic conditions, autoimmune and autoinflammatory disorders, inflammation, immune-related diseases, HIV, cancers, organ graft rejection, septic shock, viral and parasitic infections, conditions associated with acidosis, macular degeneration, pulmonary conditions, muscle wasting diseases, fibrotic diseases, bone and hair growth diseases.

Examples of proliferative diseases or conditions include diabetic retinopathy, macular degeneration, diabetic nephropathy, glomerulosclerosis, IgA nephropathy, cirrhosis, biliary atresia, congestive heart failure, scleroderma, radiation-induced fibrosis, and lung fibrosis (idiopathic pulmonary fibrosis, collagen vascular disease, sarcoidosis, interstitial lung diseases and extrinsic lung disorders).

Inflammatory diseases include acute (e.g., bronchitis, conjunctivitis, myocarditis, pancreatitis) and chronic conditions (e.g., chronic cholecstitis, bronchiectasis, aortic valve stenosis, restenosis, psoriasis and arthritis), along with conditions associated with inflammation such as fibrosis, infection and ischemia.

Immunodeficiency disorders occur when a part of the immune system is not working properly or is not present. They can affect B lymophyctes, T lymphocytes, or phagocytes and be either inherited (e.g., IgA deficiency, severe combined immunodeficiency (SCID), thymic dysplasia and chronic granulomatous) or acquired (e.g., acquired immunodeficiency syndrome (AIDS), human immunodeficiency virus (HIV) and drug-induced immunodeficiencies). Immune-related conditions include allergic disorders such as allergies, asthma and atopic dermatitis like eczema. Other examples of such immune-related conditions include lupus, rheumatoid arthritis, scleroderma, ankylosing spondylitis, dermatomyositis, psoriasis, multiple sclerosis and inflammatory bowel disease (such as ulcerative colitis and Crohn's disease).

Tissue/organ graft rejection occurs when the immune system mistakenly attacks the cells being introduced to the host's body. Graft versus host disease (GVHD), resulting from allogenic transplantation, arises when the T cells from the donor tissue go on the offensive and attack the host's tissues. In all three circumstances, autoimmune disease, transplant rejection and GVHD, modulating the immune system by treating the subject with a compound or composition of the disclosure could be beneficial.

Also provided herein is the use of a compound described herein, (i.e., a compound of Formula (III), or a compound listed in Table A), or a pharmaceutically acceptable salt thereof in treating an autoimmune disease in a patient comprising administering a therapeutically effective amount of the compound described herein. An "autoimmune disease" as used herein is a disease or disorder arising from and directed against an individual's own tissues. Examples of autoimmune diseases include, but are not limited to, inflammatory responses such as inflammatory skin diseases including psoriasis and dermatitis (e.g., atopic dermatitis); systemic scleroderma and sclerosis; responses associated with inflammatory bowel disease (such as Crohn's disease and ulcerative colitis); respiratory distress syndrome (including adult respiratory distress syndrome(ARDS)); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (e.g., Type I diabetes mellitus or insulin dependent diabetes mellitus); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia); myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia. Compounds provided herein may be useful for the treatment of conditions associated with inflammation, including, but not limited to COPD, psoriasis, asthma, bronchitis, emphysema, and cystic fibrosis.

Also provided herein is the use of a compound as disclosed herein for the treatment of neurodegenerative diseases. Neurodegenerative diseases and conditions includes, but not limited to, stroke, ischemic damage to the nervous system, neural trauma (e.g., percussive brain damage, spinal cord injury, and traumatic damage to the nervous system), multiple sclerosis and other immune-mediated neuropathies (e.g., Guillain-Barre syndrome and its variants, acute motor axonal neuropathy, acute inflammatory demyelinating polyneuropathy, and Fisher Syndrome), HIV/AIDS dementia complex, axonomy, diabetic neuropathy, Parkinson's disease, Huntington's disease, multiple sclerosis, bacterial, parasitic, fungal, and viral meningitis, encephalitis, vascular dementia, multi-infarct dementia, Lewy body dementia, frontal lobe dementia such as Pick's disease, subcortical dementias (such as Huntington or progressive supranuclear palsy), focal cortical atrophy syndromes (such as primary aphasia), metabolic-toxic dementias (such as chronic hypothyroidism or B12 deficiency), and dementias caused by infections (such as syphilis or chronic meningitis).

Further guidance for using compounds and compositions described herein (i.e., a compound of Formula (III), or a compound listed in Table A, or a pharmaceutically acceptable salt thereof) for inhibiting protein secretion can be found in the Examples section, below.

### Pharmaceutical Compositions and Administration

Described herein is the manufacture and use of pharmaceutical compositions, which include one or more of the compounds provided herein. Also described are the pharmaceutical compositions themselves. Pharmaceutical compositions typically include a pharmaceutically acceptable carrier. Thus, described herein are pharmaceutical compositions that include a compound described herein (i.e., a compound of Formula (III), or a compound listed in Table A, or a pharmaceutically acceptable salt thereof), as previously described herein, and one or more pharmaceutically acceptable carriers.

The phrase "pharmaceutically acceptable" is employed herein to refer to those ligands, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition, or vehicle, such as a liquid or solid filler, diluent, excipient, solvent or encapsulating material. As used herein the language "pharmaceutically acceptable carrier" includes buffer, sterile water for injection, solvents, dispersion media, coatings, antibacterial and antifungal agents, isotonic and absorption delaying agents, compatible with pharmaceutical administration. Each carrier must be "acceptable" in the sense of being compatible with the other ingredients of the composition and not injurious to the patient. Some examples of materials which can serve as pharmaceutically acceptable carriers include: (1) sugars, such as lactose, glucose, and sucrose; (2) starches, such as corn starch, potato starch, and substituted or unsubstituted β-cyclodextrin; (3) cellulose, and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical compositions. In certain embodiments, pharmaceutical compositions provided herein are non-pyrogenic, i.e., do not induce significant temperature elevations when administered to a patient.

The term "pharmaceutically acceptable salt" refers to the relatively non-toxic, inorganic and organic acid addition salts of a compound provided herein. These salts can be prepared *in situ* during the final isolation and purification of a compound provided herein, or by separately reacting the compound in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, laurylsulphonate salts, and amino acid salts. (See, for example, Berge et al. (1977) "Pharmaceutical Salts", J. Pharm. Sci. 66: 1-19.)

In some embodiments, a compound provided herein may contain one or more acidic functional groups and, thus, is capable of forming pharmaceutically acceptable salts with pharmaceutically acceptable bases. The term "pharmaceutically acceptable salts" in these instances refers to the relatively non-toxic inorganic and organic base addition salts of a compound provided herein. These salts can likewise be prepared *in situ* during the final isolation and purification of the compound, or by separately reacting the purified compound in its free acid form with a suitable base, such as the hydroxide, carbonate, or bicarbonate of a pharmaceutically acceptable metal cation, with ammonia, or with a pharmaceutically acceptable organic primary, secondary, or tertiary amine. Representative alkali or alkaline earth salts include the lithium, sodium, potassium, calcium, magnesium, and aluminum salts. Representative organic amines useful for the formation of base addition salts include ethylamine, diethylamine, ethylenediamine, ethanolamine, diethanolamine, piperazine (see, for example, Berge et al., supra).

Wetting agents, emulsifiers, and lubricants, such as sodium lauryl sulfate and magnesium stearate, as well as coloring agents, release agents, coating agents, sweetening, flavoring, and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite; (2) oil-soluble antioxidants, such as ascorbyl palmitate, butylated hydroxyanisole (BHA), butylated hydroxytoluene (BHT), lecithin, propyl gallate, alpha-tocopherol; and (3) metal chelating agents, such as citric acid, ethylenediamine tetraacetic acid (EDTA), sorbitol, tartaric acid, phosphoric acid.

A pharmaceutical composition may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid,. It may also be desirable to include tonicity-adjusting agents, such as sugars into the compositions. In addition, prolonged absorption of the injectable pharmaceutical form may be brought about by the inclusion of agents which delay absorption such as aluminum monostearate and gelatin.

In some cases, in order to prolong the effect of one or more compounds provided herein, it is desirable to slow the absorption of the compound from subcutaneous or intramuscular injection. For example, delayed absorption of a parenterally administered compound can be accomplished by dissolving or suspending the compound in an oil vehicle.

Compositions prepared as described herein can be administered in various forms, depending on the disorder to be treated and the age, condition, and body weight of the patient, as is well known in the art. For example, where the compositions are to be administered orally, they may be formulated as tablets, capsules, granules, powders, or syrups; or for parenteral administration, they may be formulated as injections (intravenous, intramuscular, or subcutaneous), drop infusion preparations, or suppositories. For application by the ophthalmic mucous membrane route, they may be formulated as eye drops or eye ointments. These compositions can be prepared by conventional means in conjunction with the methods described herein, and, if desired, the active ingredient may be mixed with any conventional additive or excipient, such as a binder, a disintegrating agent, a lubricant, a corrigent, a solubilizing agent, a suspension aid, an emulsifying agent, or a coating agent.

Compositions suitable for oral administration may be in the form of capsules (e.g., gelatin capsules), cachets, pills, tablets, lozenges (using a flavored basis, usually sucrose and acacia or tragacanth), powders, troches, granules, or as a solution or a suspension in an aqueous or non-aqueous liquid, or as an oil-in-water or water-in-oil liquid emulsion, or as an elixir or syrup, or as pastilles (using an inert matrix, such as gelatin and glycerin, or sucrose and acacia) and/or as mouthwashes, each containing a predetermined amount of a compound provided herein as an active ingredient. A composition may also be administered as a bolus, electuary, or paste. Oral compositions generally include an inert diluent or an edible carrier.

Pharmaceutically compatible binding agents, and/or adjuvant materials can be included as part of an oral composition. In solid dosage forms for oral administration (capsules, tablets, pills, dragees, powders, granules), the active ingredient can be mixed with one or more pharmaceutically acceptable carriers, such as sodium citrate or dicalcium phosphate, and/or any of the following: (1) fillers or extenders, such as starches, cyclodextrins, lactose, sucrose, saccharin, glucose, mannitol, and/or silicic acid; (2) binders, such as, for example, carboxymethylcellulose, microcrystalline cellulose, gum tragacanth, alginates, gelatin, polyvinyl pyrrolidone, sucrose, and/or acacia; (3) humectants, such as glycerol; (4) disintegrating agents, such as agar-agar, calcium carbonate, potato, corn, or tapioca starch, alginic acid, Primogel, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, Sterotes, solid polyethylene glycols, sodium lauryl sulfate, and mixtures thereof; (10) a glidant, such as colloidal silicon dioxide; (11) coloring agents; and (12) a flavoring agent such as peppermint, methyl salicylate, or orange flavoring. In the case of capsules, tablets, and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of a powdered compound moistened with an inert liquid diluent.

Tablets, and other solid dosage forms, such as dragees, capsules, pills, and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes, microspheres, and/or nanoparticles. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents, and emulsifiers such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols, and fatty acid esters of sorbitan, and mixtures thereof.

Besides inert diluents, the oral compositions can also include adjuvants such as wetting agents, emulsifying and suspending agents, sweetening, flavoring, coloring, perfuming, and preservative agents.

Suspensions, in addition to the active compound(s) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions suitable for parenteral administration can include one or more compounds provided herein in combination with one or more pharmaceutically acceptable sterile aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the composition isotonic with the blood of the intended recipient or suspending or thickening agents.

Examples of suitable aqueous and nonaqueous carriers which may be employed in the pharmaceutical compositions provided herein include water for injection (e.g., sterile water for injection), bacteriostatic water, ethanol, polyols (such as glycerol, propylene glycol, polyethylene glycol such as liquid polyethylene glycol), sterile buffer (such as citrate buffer), and suitable mixtures thereof, vegetable oils, such as olive oil, injectable organic esters, such as ethyl oleate, and Cremophor EL^{™} (BASF, Parsippany, NJ). In all cases, the composition must be sterile and should be fluid to the extent that easy syringability exists. Proper fluidity can be maintained, for example, by the use of coating materials, such as lecithin, by the maintenance of the required particle size in the case of dispersions, and by the use of surfactants.

The composition should be stable under the conditions of manufacture and storage and must be preserved against the contaminating action of microorganisms such as bacteria and fungi. Prevention of the action of microorganisms can be achieved by various antibacterial and antifungal agents, for example, parabens, chlorobutanol, phenol, ascorbic acid, thimerosal. In many cases, it will be preferable to include isotonic agents, for example, sugars, polyalcohols such as mannitol, sorbitol, and sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the methods of preparation are freeze-drying (lyophilization), which yields a powder of the active ingredient plus any additional desired ingredient from a previously sterile-filtered solution thereof.

Injectable depot forms can be made by forming microencapsule or nanoencapsule matrices of a compound provided herein in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable compositions are also prepared by entrapping the drug in liposomes,microemulsions or nanoemulsions, which are compatible with body tissue.

For administration by inhalation, the compounds can be delivered in the form of an aerosol spray from a pressured container or dispenser that contains a suitable propellant, e.g., a gas such as carbon dioxide, or a nebulizer. Such methods include those described in U.S. Patent No. 6,468,798. Additionally, intranasal delivery can be accomplished, as described in, inter alia, Hamajima et al., Clin. Immunol. Immunopathol., 88(2), 205-10 (1998). Liposomes (e.g., as described in U.S. Patent No. 6,472,375), microencapsulation and nanoencapsulation can also be used. Biodegradable targetable microparticle delivery systems or biodegradable targetable nanoparticle delivery systems can also be used (e.g., as described in U.S. Patent No. 6,471,996).

Systemic administration of a therapeutic compound as described herein can also be by transmucosal or transdermal means. Dosage forms for the topical or transdermal administration of a compound provided herein include powders, sprays, ointments, pastes, creams, lotions, gels, solutions, patches, and inhalants. The active component may be mixed under sterile conditions with a pharmaceutically acceptable carrier, and with any preservatives, buffers, or propellants which may be required. For transmucosal or transdermal administration, penetrants appropriate to the barrier to be permeated are used in the composition. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The ointments, pastes, creams, and gels may contain, in addition to one or more compounds provided herein, excipients, such as animal and vegetable fats, oils, waxes, paraffins, starch, tragacanth, cellulose derivatives, polyethylene glycols, silicones, bentonites, silicic acid, talc, and zinc oxide, or mixtures thereof.

Powders and sprays can contain, in addition to a compound provided herein, excipients such as lactose, talc, silicic acid, aluminum hydroxide, calcium silicates, and polyamide powder, or mixtures of these substances. Sprays can additionally contain customary propellants, such as chlorofluorohydrocarbons and volatile unsubstituted hydrocarbons, such as butane and propane.

A compound provided herein can be administered by aerosol. This is accomplished by preparing an aqueous aerosol, liposomal preparation, or solid particles containing a compound or composition provided herein. A nonaqueous (e.g., fluorocarbon propellant) suspension could be used. In some embodiments, sonic nebulizers are used because they minimize exposing the agent to shear, which can result in degradation of the compound.

Ordinarily, an aqueous aerosol can be made by formulating an aqueous solution or suspension of the agent together with conventional pharmaceutically acceptable carriers and stabilizers. The carriers and stabilizers vary with the requirements of the particular composition, but typically include nonionic surfactants (TWEEN^{®} (polysorbates), PLURONIC^{®} (poloxamers), sorbitan esters, lecithin, CREMOPHOR^{®} (polyethoxylates)), pharmaceutically acceptable co-solvents such as polyethylene glycol, innocuous proteins like serum albumin, sorbitan esters, oleic acid, lecithin, amino acids such as glycine, buffers, salts, sugars, or sugar alcohols. Aerosols generally are prepared from isotonic solutions.

Transdermal patches have the added advantage of providing controlled delivery of a compound provided herein to the body. Such dosage forms can be made by dissolving or dispersing the agent in the proper medium. Absorption enhancers can also be used to increase the flux of the compound across the skin. The rate of such flux can be controlled by either providing a rate controlling membrane or dispersing the compound in a polymer matrix or gel.

The pharmaceutical compositions can also be prepared in the form of suppositories or retention enemas for rectal and/or vaginal delivery. Compositions presented as a suppository can be prepared by mixing one or more compounds provided herein with one or more suitable nonirritating excipients or carriers comprising, for example, cocoa butter, glycerides, polyethylene glycol, a suppository wax or a salicylate, which is solid at room temperature, but liquid at body temperature and, therefore, will melt in the rectum or vaginal cavity and release the active agent. Compositions which are suitable for vaginal administration also include pessaries, tampons, creams, gels, pastes, foams, or spray compositions containing such carriers as are known in the art to be appropriate.

In one embodiment, the therapeutic compounds are prepared with carriers that will protect the therapeutic compounds against rapid elimination from the body, such as a controlled release composition, including implants and microencapsulated delivery systems. Biodegradable, biocompatible polymers can be used, such as ethylene vinyl acetate, polyanhydrides, polyglycolic acid, collagen, polyorthoesters, and polylactic acid. Such compositions can be prepared using standard techniques, or obtained commercially, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

As described above, the preparations of one or more compounds provided herein may be given orally, parenterally, topically, or rectally. They are, of course, given by forms suitable for each administration route. For example, they are administered in tablets or capsule form, by injection, inhalation, eye lotion, ointment, suppository, infusion; topically by lotion or ointment; and rectally by suppositories. In some embodiments, administration is oral.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrastemal injection, and infusion.

The phrases "systemic administration", "administered systemically", "peripheral administration", and "administered peripherally" as used herein mean the administration of a ligand, drug, or other material via route other than directly into the central nervous system, such that it enters the patient's system and thus, is subject to metabolism and other like processes, for example, subcutaneous administration.

A compound provided herein may be administered to humans and other animals for therapy by any suitable route of administration, including orally, nasally, as by, for example, a spray, rectally, intravaginally, parenterally, intracistemally, and topically, as by powders, ointments or drops, including buccally and sublingually. Regardless of the route of administration selected, a compound provided herein, which may be used in a suitable hydrated form, and/or the pharmaceutical compositions provided herein, is formulated into a pharmaceutically acceptable dosage form by conventional methods known to those of skill in the art. In another embodiment, the pharmaceutical composition is an oral solution or a parenteral solution. Another embodiment is a freeze-dried preparation that can be reconstituted prior to administration. As a solid, this composition may also include tablets, capsules or powders.

Actual dosage levels of the active ingredients in the pharmaceutical compositions provided herein may be varied so as to obtain "therapeutically effective amount," which is an amount of the active ingredient effective to achieve the desired therapeutic response for a particular patient, composition, and mode of administration, without being toxic to the patient.

The concentration of a compound provided herein in a pharmaceutically acceptable mixture will vary depending on several factors, including the dosage of the compound to be administered, the pharmacokinetic characteristics of the compound(s) employed, and the route of administration. In some embodiments, the compositions provided herein can be provided in an aqueous solution containing about 0.1-10% w/v of a compound disclosed herein, among other substances, for parenteral administration. Typical dose ranges can include from about 0.01 to about 50 mg/kg of body weight per day, given in 1-4 divided doses. Each divided dose may contain the same or different compounds. The dosage will be a therapeutically effective amount depending on several factors including the overall health of a patient, and the composition and route of administration of the selected compound(s).

Dosage forms or compositions containing a compound as described herein in the range of 0.005% to 100% with the balance made up from non-toxic carrier may be prepared. Methods for preparation of these compositions are known to those skilled in the art. The contemplated compositions may contain 0.001%-100% active ingredient, in one embodiment 0.1-95%, in another embodiment 75-85%. Although the dosage will vary depending on the symptoms, age and body weight of the patient, the nature and severity of the disorder to be treated or prevented, the route of administration and the form of the drug, in general, a daily dosage of from 0.01 to 2000 mg of the compound is recommended for an adult human patient, and this may be administered in a single dose or in divided doses. The amount of active ingredient which can be combined with a carrier material to produce a single dosage form will generally be that amount of the compound which produces a therapeutic effect.

The pharmaceutical composition may be administered at once, or may be divided into a number of smaller doses to be administered at intervals of time. It is also noted that the dose of the compound can be varied over time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated. It is to be further understood that for any particular patient, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

The precise time of administration and/or amount of the composition that will yield the most effective results in terms of efficacy of treatment in a given patient will depend upon the activity, pharmacokinetics, and bioavailability of a particular compound, physiological condition of the patient (including age, sex, disease type and stage, general physical condition, responsiveness to a given dosage, and type of medication), route of administration. However, the above guidelines can be used as the basis for fine-tuning the treatment, e.g., determining the optimum time and/or amount of administration, which will require no more than routine experimentation consisting of monitoring the patient and adjusting the dosage and/or timing.

The pharmaceutical compositions can be included in a container, pack, or dispenser together with instructions for administration.

In jurisdictions that forbid the patenting of methods that are practiced on the human body, the meaning of "administering" of a composition to a human subject shall be restricted to prescribing a controlled substance that a human subject will self-administer by any technique (e.g., orally, inhalation, topical application, injection, insertion). The broadest reasonable interpretation that is consistent with laws or regulations defining patentable subject matter is intended. In jurisdictions that do not forbid the patenting of methods that are practiced on the human body, the "administering" of compositions includes both methods practiced on the human body and also the foregoing activities.

It is to be understood that while the disclosure is read in conjunction with the detailed description thereof, the foregoing description is intended to illustrate and not limit the scope of the disclosure, which is defined by the scope of the appended claims. Other aspects, advantages, and modifications are within the scope of the following claims.

### EXAMPLES

The following examples are provided for illustration and are not intended to limit the scope of the disclosure in any way.

As used throughout these examples, common organic abbreviations are defined as follows:

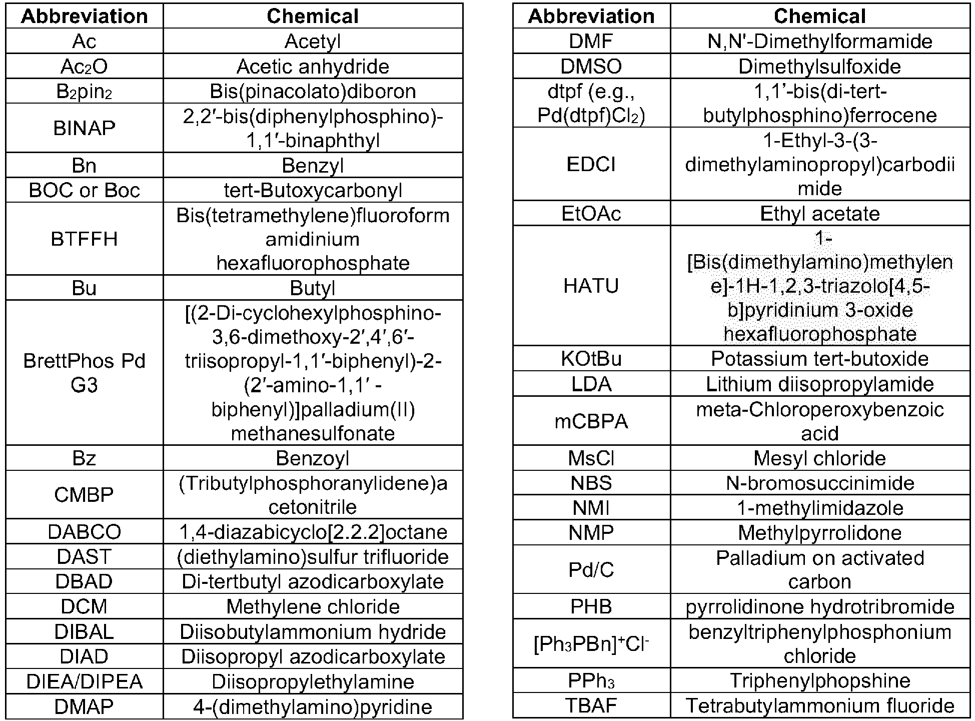

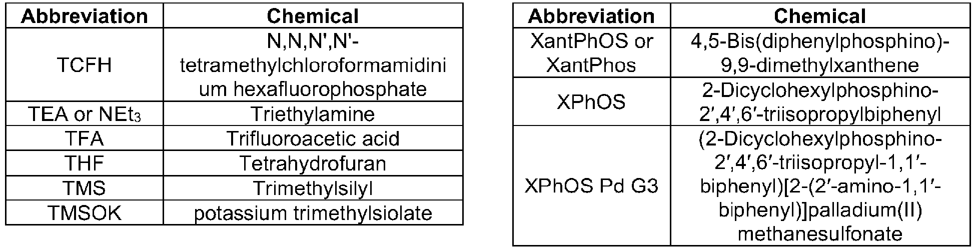

### Example 1: Synthesis of Intermediate Compounds via Alkylation

### Procedure 1 - Intermediate for A91

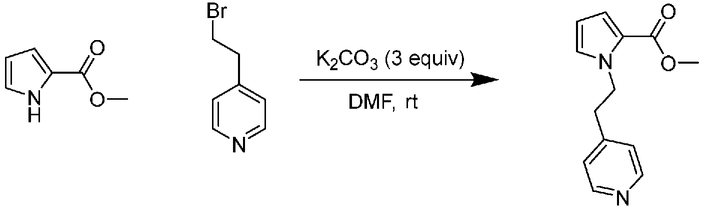

A 100 mL roundbottom flask with stir bar was charged with methyl 1H-pyrrole-2-carboxylate (581 mg, 4.65 mmol, 1.1 equiv), 4-(2-bromoethyl)pyridine (786 mg, 4.22 mmol, 1.0 equiv), K₂CO₃ (1.75 g, 12.7 mmol, 3.0 equiv) and DMF (20 mL). The resulting reaction mixture was stirred at room temperature for 2 days. After this time, the reaction mixture was diluted with DCM (200 mL) and washed with water (3 x 200 mL). The organic layer was dried over Na₂SO₄, filtered and concentrated in vacuo. The resulting crude material was purified via silica gel chromatography (50-100% EtOAc in hexanes) to yield the desired product.

The following intermediate compounds were synthesized in a similar manner:

| **Intermediate for:** | **Product name** |
|---|---|
| **A41** | methyl 1-((3-bromopyridin-4-yl)methyl)-1H-pyrrole-2-carboxylate |
| **A100** | ethyl 1-(pyridin-4-ylmethyl)-1H-pyrazole-3-carboxylate |
| **A112** | ethyl1-(pyridin-4-ylmethyl)-1H-pyrazole-5-carboxylate |
| **A122** | methyl1-(pyridin-4-ylmethyl)-1H-pyrrole-3-carboxylate |
| **A87** | methyl1-(pyridin-3-ylmethyl)-1H-pyrrole-2-carboxylate |
| **A102** | methyl6-oxo-1-(pyridin-4-ylmethyl)piperidine-2-carboxylate |
| **A103** | methyl3-methyl-1-(pyridin-4-ylmethyl)-1H-pyrazole-5-carboxylate |
| **A104** | methyl1-(pyridin-4-ylmethyl)-1H-imidazole-5-carboxylate |
| **A106** | methyl6-oxo-1-(pyridin-4-ylmethyl)-1,6-dihydropyridine-2-carboxylate |
| **A108** | methyl1-(pyridin-4-ylmethyl)-1H-imidazole-2-carboxylate |
| **A86** | methyl 3-chloro-1-(pyridin-4-ylmethyl)-1 H-pyrrole-2-carboxylate |
| **A88** | methyl3-methyl-1-(pyridin-4-ylmethyl)-1H-pyrrole-2-carboxylate |
| **A24** | methyl1-((3-methylpyridin-4-yl)methyl)-1H-pyrrole-2-carboxylate |
| **A48** | methyl4-methyl-1-(pyridin-4-ylmethyl)-1H-pyrrole-2-carboxylate |
| **A76** | methyl 5-methyl-1-(pyridin-4-ylmethyl)-1H-pyrrole-2-carboxylate |
| **A83** | methyl1-((2-methylpyridin-4-yl)methyl)-1H-pyrrole-2-carboxylate |
| **A125** | methyl 1-((2-methoxypyridin-4-yl)methyl)-1H-pyrrole-2-carboxylate |
| **A211** | methyl 1-((3-methoxypyridin-4-yl)methyl)-1H-pyrrole-2-carboxylate |
| **A214** | methyl1-((3-(trifluoromethyl)pyridin-4-yl)methyl)-1H-pyrrole-2-carboxylate |
| **A212 and A190** | methyl1-((3-bromopyridin-4-yl)methyl)-1H-pyrrole-2-carboxylate |
| **A221** | methyl1-(3-(pyridin-4-yl)allyl)-1H-pyrrole-2-carboxylate |
| **A238 and A318** | methyl 1-((3-chloropyridin-4-yl)methyl)-1H-pyrrole-2-carboxylate |
| **A243** | methyl 1-(3-(3-fluoropyridin-4-yl)propyl)-1H-pyrrole-2-carboxylate |
| **A360** | methyl1-((3-fluoro-5-methylpyridin-4-yl)methyl)-1H-pyrrole-2-carboxylate |

### Procedure 2 - Intermediate for A64

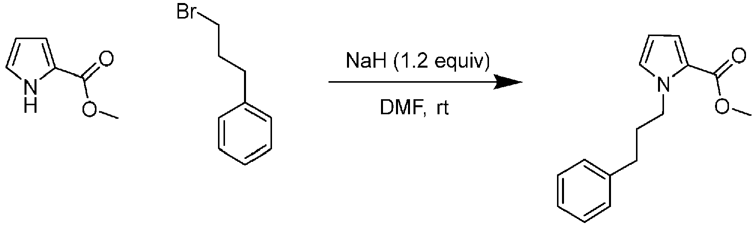

A roundbottom flask with stir bar was charged with NaH (210 mg, 60 wt%, 5.13 mmol, 1.2 equiv) and DMF (20 mL). Methyl 1H-pyrrole-2-carboxylate (535 mg, 4.27 mmol, 1.0 equiv) was slowly added to the reaction mixture, and the solution was stirred at room temperature for 30 min. After 30 min, (3-bromopropyl)benzene (936 mg, 4.70 mmol, 1.1 equiv) was added, and the reaction mixture was allowed to stir at room temperature overnight. The next morning, the reaction mixture was diluted with DCM (200 mL) and washed with water (3 x 200 mL). The organic layer was dried over Na₂SO₄, filtered and concentrated in vacuo. The resulting crude material was purified via silica gel chromatography (0-15% EtOAc in hexanes) to yield the desired product.

The following intermediate compounds were synthesized in a similar manner:

| **Intermediate for:** | **Product name** |
|---|---|
| **A190** | methyl 1-(3-(pyridin-3-yl)propyl)-1H-pyrrole-2-carboxylate |
| **A101** | methyl 1-(3-(1-acetylpiperidin-4-yl)propyl)-1H-pyrrole-2-carboxylate ) |
| **A68** | methyl 1-(3-(2-chlorophenyl)propyl)-1H-pyrrole-2-carboxylate |
| **A44** | methyl 1-(4-(pyridin-4-yl)butyl)-1H-pyrrole-2-carboxylate |
| **A126** | benzyl 4-((2-(methoxycarbonyl)-1H-pyrrol-1-yl)methyl)piperidine-1-carboxylate |
| **A11** | methyl 1-(3-(pyridin-4-yl)propyl)-1H-pyrrole-2-carboxylate |
| **A71** | methyl 1-((tetrahydro-2H-pyran-4-yl)methyl)-1H-pyrrole-2-carboxylate |
| **A128 and A129** | tert-butyl 4-((2-(methoxycarbonyl)-1H-pyrrol-1-yl)methyl)piperidine-1-carboxylate |
| **A78** | methyl 1-(2-chlorobenzyl)-1H-pyrrole-2-carboxylate |
| **A79** | methyl 1-benzyl-1H-pyrrole-2-carboxylate |
| **A84** | methyl 1-(4-(methylsulfonyl)benzyl)-1H-pyrrole-2-carboxylate |
| **A80** | methyl 1-(4-cyanobenzyl)-1H-pyrrole-2-carboxylate |
| **A53** | methyl 1-(4-nitrobenzyl)-1H-pyrrole-2-carboxylate |
| **A187 and A190** | methyl 1-(3-(pyridin-3-yl)propyl)-1H-pyrrole-2-carboxylate |
| **A256** | methyl 1-((3,5difluoropyridin-4-yl)methyl)-1H-pyrrole-2-carboxylate |
| **A226** | methyl 1-(2-((tert-butyldimethylsilyl)oxy)ethyl)-1H-pyrrole-2-carboxylate |

### Procedure 3 - Intermediate for A90

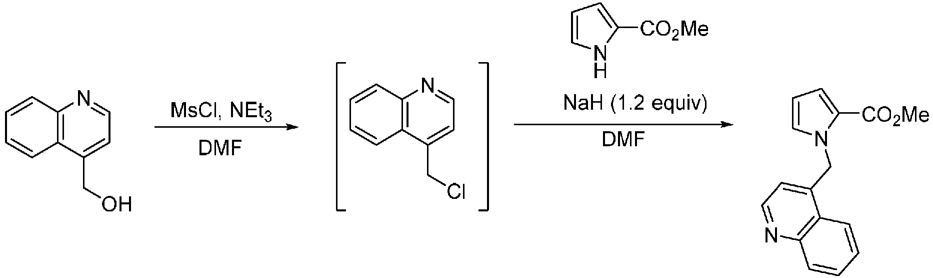

A 40 mL scintillation vial with stir bar was charged with quinolin-4-ylmethanol (500 mg, 3.14 mmol, 1.1 equiv) and DMF (7 mL). Triethylamine (0.65 mL, 4.71 mmol, 1.5 equiv) was added to the reaction mixture, followed by the slow addition of mesyl chloride (0.269 mL, 3.45 mmol, 1.2 equiv) at room temperature. The reaction mixture was allowed to stir at room temperature overnight. The next morning, in a separate flask, a slurry of NaH (140 mg, 3.43 mmol, 60 wt%, 1.2 equiv) and DMF (7 mL) was prepared. Methyl 1H-pyrrole-2-carboxylate (357 mg, 2.86 mmol, 1.0 equiv) was added, and the reaction mixture was allowed to stir at room temperature for 30 min. After 30 min, the solution of crude activated alcohol was slowly added to the pyrrole solution in four portions over 1 h. The resulting solution was allowed to stir at room temperature overnight. The next morning, the reaction mixture was diluted with DCM (150 mL) and washed with water (3 x 150 mL). The organic layer was dried over Na₂SO₄, filtered and concentrated in vacuo. The resulting crude material was purified via silica gel chromatography (10-40% EtOAc in hexanes) to yield the desired product.

The following intermediate compounds were synthesized in a similar manner:

| **Intermediate for:** | **Product name** |
|---|---|
| **A25** | methyl 1-((3-fluoropyridin-4-yl)methyl)-1H-pyrrole-2-carboxylate |
| **A90** | methyl 1-(quinolin-4-ylmethyl)-1H-pyrrole-2-carboxylate |
| **A124** | methyl 1-((1-methyl-6-oxo-1,6-dihydropyridin-3-yl)methyl)-1H-pyrrole-2-carboxylate |
| **A69** | methyl 1-(pyridazin-4-ylmethyl)-1H-pyrrole-2-carboxylate |
| **A127** | methyl 1-((1-methyl-2-oxo-1,2-dihydropyridin-4-yl)methyl)-1 H-pyrrole-2-carboxylate |
| **A97** | methyl 1-((2,6-dimethylpyridin-4-yl)methyl)-1H-pyrrole-2-carboxylate |
| **A36** | methyl 1-((2,6-difluoropyridin-4-yl)methyl)-1H-pyrrole-2-carboxylate |

### Procedure 4 - Intermediate for A111

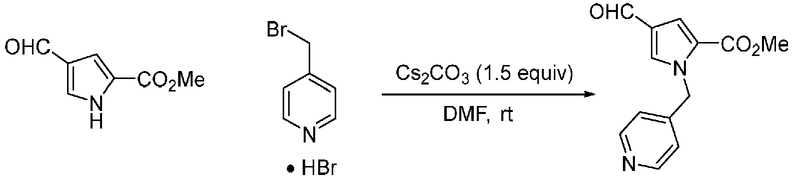

Methyl 4-formyl-1H-pyrrole-2-carboxylate (2 g, 13 mmol, 1 equiv) was dissolved in DMF (65 mL), and Cs₂CO₃ (6.37 g, 19 mmol, 1.5 equiv) and (bromomethyl)pyridine hydrobromide (3.3g, 13 mmol, 1.0 equiv) were added. The resulting reaction mixture was stirred at room temperature overnight. The next morning, the reaction mixture was diluted with water (20 mL) and extracted with EtOAc (4 x 30mL). The organic layer was dried over Mg₂SO₄, filtered and concentrated in vacuo. The resulting crude product was purified via silica gel chromatography (25-100% EtOAc in hexanes) to yield the desired product.

The following intermediate compounds were synthesized in a similar manner:

| **Intermediate for:** | **Product name** |
|---|---|
| **A105** | methyl 5-formyl-1-(pyridin-4-ylmethyl)-1H-pyrrole-2-carboxylate |
| **A109** | methyl 4-formyl-1-(pyridin-4-ylmethyl)-1H-pyrrole-2-carboxylate |

### Procedure 5 - Intermediate for A74 and A49

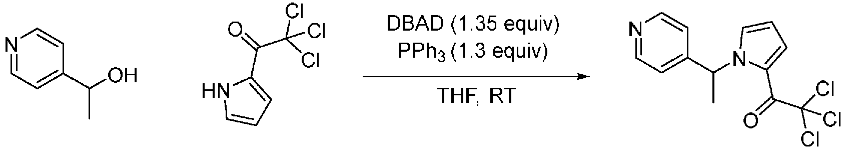

2-(trichloroacetyl)pyrrole (1.0 g, 4.71 mmol, 1.0 eq), triphenylphosphine (0.527 g, 6.12 mmol, 1.3 equiv) and 1-(4-pyridinyl)ethanol (0.638 g, 5.18 mmol, 1.10 eq) were dissoled in anhydrous THF (15.8 mL, 0.3 M). Then di-tertbutylazodicarboxylate (DBAD) (1.463 g, 6.35 mmol, 1.35 eq) dissolved in anhydrous THF (2 mL) was added under argon atmosphere. The reaction was allowed to stir at room temperature overnight. The next morning, the THF was evaporated under reduced preassure and the resulting crude material was purified via silica gel chromatography (0-100% EtOAc in DCM) to give the desired product.

### Procedure 6 - Intermediate for A93

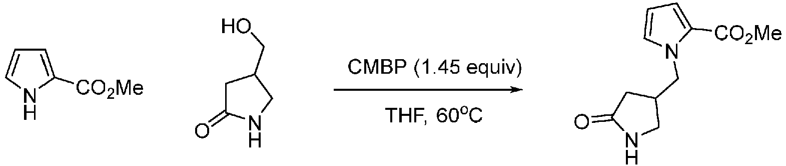

Methyl 1H-pyrrole-2-carboxylate (1 g, 8 mmol, 1 equiv) and 4-(hydroxymethyl)pyrrolidin-2-one (1.38 g, 12 mmol, 1.5 equiv) were dissolved in dry THF (16 mL) followed by addition of CMBP (2.85g, 11.6 mmol, 1.45 equiv) under Ar. The tube was sealed and heated to 60°C overnight. The next morning, the THF was removed under reduced pressure and the crude mixture was purified via silica gel chromatography (1-5% methanol in DCM), which yielded the desired product mixed with tributylphosphine oxide. This mixture washed with hexane and filtered to provide the pure desired product.

### Procedure 7 - Intermediate for B26

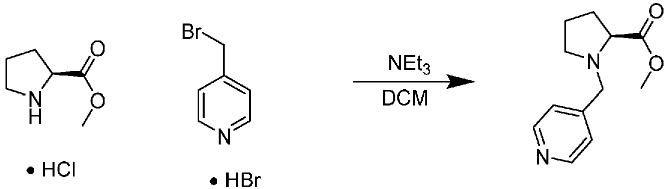

A 40 mL vial with stir bar was charged with methyl prolinate hydrochloride (500 mg, 3.02 mmol, 1.0 equiv) and DCM (15 mL, 0.2 M). Triethylamine (1.7 mL, 12.1 mmol, 4.0 equiv) was slowly added at room temperature. 4-(bromomethyl)pyridine hydrobromide (840 mg, 3.32 mmol, 1.1 equiv) was added portion-wise over 1 h. The resulting reaction mixture was allowed to stir at room temperature overnight. The next morning, the reaction mixture was concentrated in vacuo and the resulting crude product was purified via silica gel chromatography (50-100% EtOAc in hexanes) to yield the desired product.

### Procedure 8 - Intermediate for A210

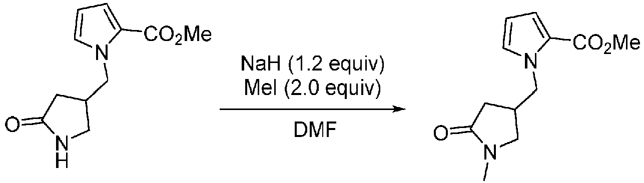

Methyl 1-((5-oxopyrrolidin-3-yl)methyl)-1H-pyrrole-2-carboxylate (100 mg, 0.45 mmol, 1.0 equiv) was dissolved in THF, and cooled to 0 °C. NaH (21.6 mg, 0.54 mmol, 1.2 equiv) was added, and the reaction stirred at 0 °C for 15 min. After this time, Mel (127.8 mg, 0.9 mmol, 2.0 equiv) was added, and the reaction mixture was warmed to room temperature for 30 min. After 30 min at room temperature, the reaction was quenched with water (5 mL) and concentrated in vacuo to yield the desired product.

### Procedure 9 - Intermediate for A212 & A213

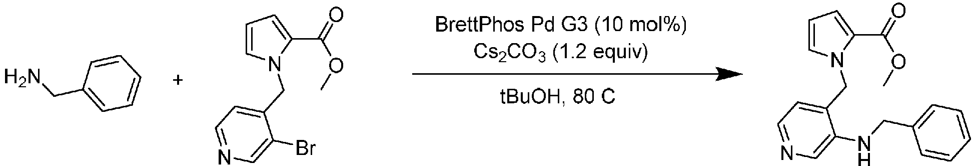

A 20 mL vial with stir bar was charged with bromide (461 mg, 1.56 mmol, 1.0 equiv), Cs₂CO₃ (611 mg, 1.87 mmol, 1.2 equiv), and BrettPhos Pd G3 (142 mg, 0.156 mmol, 0.1 equiv). The vial was evacuated and backflushed with nitrogen. Freshly-sparged tBuOH (7 mL) was added, followed by benzylamine (0.2 mL, 1.87 mmol, 1.2 equiv). The vial was capped, and the reaction mixture was allowed to stir at 80 C overnight. The next morning, the reaction mixture was cooled to room temperature and diluted with DCM (100 mL). The organic layer was washed with brine (2 x 100 mL), and the combined aqueous layers were extracted with DCM (1 × 100 mL). The combined organic layers were dried over Na₂SO₄, filtered and concentrated in vacuo. The resulting material was purified by silica gel chromatography to yield the desired product.

### Procedure 10 - Intermediate for A226

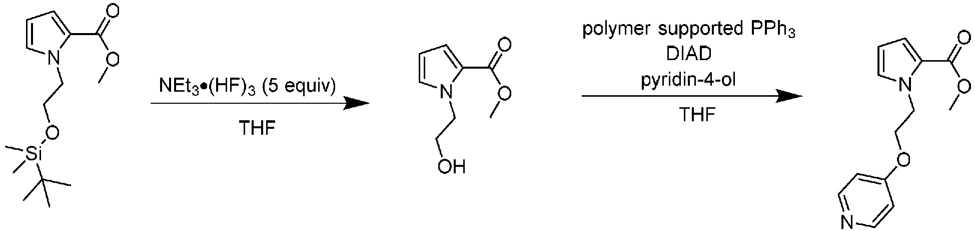

A roundbottom flask with stir bar was charged with silyl ether (1.74 g, 6.14 mmol, 1.0 equiv) and THF (20 mL). The reaction mixture was cooled to 0 C, and triethylamine trihydrofluoride (5.00 mL, 30.7 mmol, 5.0 equiv) was added at 0 C. The reaction mixture was allowed to warm to room temperature overnight. The next morning, the reaction mixture was diluted with DCM (150 mL) and washed with saturated NaHCO₃ (2 x 150 mL). The combined aqueous layers were extracted with DCM (1 × 150 mL), and the combined organic layers were dried over Na₂SO₄, filtered and concentrated in vacuo. The resulting material was used in the next step without further purification.

A roundbottom flask with stir bar was charged with alcohol (2.30 g, 13.6 mmol, 1.0 equiv), pyridin-4-ol (1.29 g, 13.6 mmol, 1.0 equiv), and polymer-bound PPh3 (3 mmol/g loading, 9.05 g, 27.2 mmol, 2.0 equiv) and THF (30 mL). The reaction mixture was cooled to 0 C. DIAD (5.34 mL, 27.2 mmol, 2.0 equiv) was slowly added at 0 C, and the reaction mixture was allowed to warm to room temperature overnight. The next morning, the reaction mixture filtered through a plug of Celite and washed with EtOAc. The filtrate was concentrated in vacuo, and the resulting crude material was purified via silica gel chromatography to yield the desired product.

### Example 2: Synthesis of Intermediate Compounds via Saponification Procedure 1 - Intermediate for A6

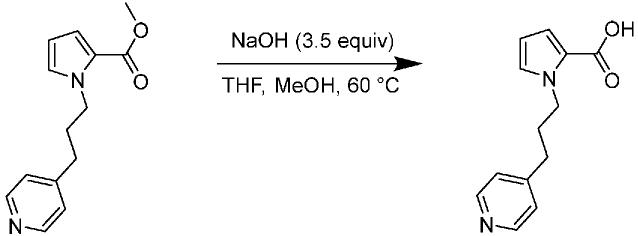

A 40 mL vial with stir bar was charged with methyl 1-(3-(pyridin-4-yl)propyl)-1H-pyrrole-2-carboxylate (559 mg, 2.29 mmol, 1.0 equiv). MeOH (4 mL) and THF (4 mL) were added, followed by NaOH (1.6 mL, 5.0 M in water, 3.5 equiv). The resulting solution was heated at 60 ⁰C overnight. The next morning, the solvents were removed in vacuo and the resulting aqueous solution was acidified to ~ pH 3 via addition of 1 M HCl. The resulting precipitate was filtered and collected, and any residual water was removed in vacuo to yield the desired product.

The following compounds were prepared in a similar manner:

| **Intermediate for:** | **Compound name** |
|---|---|
| **A190** | 1-(3-(pyridin-3-yl)propyl)-1H-pyrrole-2-carboxylic acid |
| **A41** | 1-((3-bromopyridin-4-yl)methyl)-1H-pyrrole-2-carboxylic acid |
| **A101** | 1-(3-(1-acetylpiperidin-4-yl)propyl)-1H-pyrrole-2-carboxylic acid |
| **A68** | 1-(3-(2-chlorophenyl)propyl)-1H-pyrrole-2-carboxylic acid |
| **A64** | 1-(3-phenylpropyl)-1H-pyrrole-2-carboxylicacid |
| **A100** | 1-(pyridin-4-ylmethyl)-1H-pyrazole-3-carboxylic acid |
| **A44** | 1-(4-(pyridin-4-yl)butyl)-1H-pyrrole-2-carboxylic acid |
| **A25** | 1-((3-fluoropyridin-4-yl)methyl)-1H-pyrrole-2-carboxylicacid |
| **A112** | 1-(pyridin-4-ylmethyl)-1H-pyrazole-5-carboxylic acid |
| **A91** | 1-(2-(pyridin-4-yl)ethyl)-1H-pyrrole-2-carboxylic acid |
| **A90** | 1-(quinolin-4-ylmethyl)-1H-pyrrole-2-carboxylic acid |
| **A122** | 1-(pyridin-4-ylmethyl)-1H-pyrrole-3-carboxylic acid |
| **A124** | 1-((1-methyl-6-oxo-1,6-dihydropyridin-3-yl)methyl)-1H-pyrrole-2-carboxylic acid |
| **A69** | 1-(pyridazin-4-ylmethyl)-1H-pyrrole-2-carboxylic acid |
| **A126** | 1-((1-((benzyloxy)carbonyl)piperidin-4-yl)methyl)-1H-pyrrole-2-carboxylic acid |
| **A127** | 1-((1-methyl-2-oxo-1,2-dihydropyridin-4-yl)methyl)-1H-pyrrole-2-carboxylic acid |
| **A71** | 1-((tetrahydro-2H-pyran-4-yl)methyl)-1H-pyrrole-2-carboxylic acid |
| **A128** | 1-((1-acetylpiperidin-4-yl)methyl)-1H-pyrrole-2-carboxylic acid |
| **A129** | 1-((1-methylpiperidin-4-yl)methyl)-1H-pyrrole-2-carboxylic acid |
| **A130** | (pyridin-4-ylmethyl)-L-proline |
| **A87** | 1-(pyridin-3-ylmethyl)-1H-pyrrole-2-carboxylic acid |
| **A16** | 1-(pyridin-4-ylmethyl)-1H-pyrrole-2-carboxylic acid |
| **A78** | 1-(2-chlorobenzyl)-1H-pyrrole-2-carboxylicacid |
| **A79** | 1-benzyl-1H-pyrrole-2-carboxylic acid |
| **A211** | 1-((3-methoxypyridin-4-yl)methyl)-1H-pyrrole-2-carboxylic acid |
| **A214** | 1-((3-(trifluoromethyl)pyridin-4-yl)methyl)-1H-pyrrole-2-carboxylicacid |
| **A187 and A190** | 1-(3-(pyridin-3-yl)propyl)-1H-pyrrole-2-carboxylic acid |
| **A212 and A213** | 1-((3-(benzylamino)pyridin-4-yl)methyl)-1H-pyrrole-2-carboxylicacid |
| **A221** | 1-(3-(pyridin-4-yl)allyl)-1H-pyrrole-2-carboxylic acid |
| **A226** | 1-(2-(pyridin-4-yloxy)ethyl)-1H-pyrrole-2-carboxylic acid |
| **A238 and A318** | 1-((3-chloropyridin-4-yl)methyl)-1H-pyrrole-2-carboxylic acid |

### Procedure 2 - Intermediate for A102

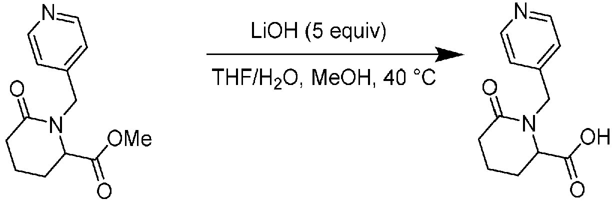

To the solution of methyl ester (0.210 g, 0.85 mmol, 1.0 eq.) in THF/water and few drops of MeOH was added LiOH monohydrate (0.177 g, 4.00 mmol, 5.0 eq.). Reaction was continued at 40°C for 18 hours. The next morning, the THF was evaporated, and the water layer was acidified to ~ pH 4-5 via addition of 1 M HCl. The water layer was then evaporated, and the crude product was purified by ionic resin (Amberlite IR 120) to give the desired product.

The following compounds were prepared in a similar manner:

| **Intermediate for:** | **Compound name** |
|---|---|
| **A210** | 1-((1-methyl-5-oxopyrrolidin-3-yl)methyl)-1H-pyrrole-2-carboxylic acid |
| **A93** | 1-((5-oxopyrrolidin-3-yl)methyl)-1H-pyrrole-2-carboxylic acid |
| **A104** | 1-(pyridin-4-ylmethyl)-1H-imidazole-5-carboxylic acid |
| **A105** | 5-carbamoyl-1-(pyridin-4-ylmethyl)-1H-pyrrole-2-carboxylic acid |
| **A107** | 5-(dimethylcarbamoyl)-1-(pyridin-4-ylmethyl)-1H-pyrrole-2-carboxylic acid |
| **A109** | 4-carbamoyl-1-(pyridin-4-ylmethyl)-1H-pyrrole-2-carboxylic acid |
| **A110** | 5-(hydroxymethyl)-1-(pyridin-4-ylmethyl)-1H-pyrrole-2-carboxylic acid |
| **A111** | 4-(dimethylcarbamoyl)-1-(pyridin-4-ylmethyl)-1H-pyrrole-2-carboxylic acid |
| **A113** | 4-(hydroxymethyl)-1-(pyridin-4-ylmethyl)-1H-pyrrole-2-carboxylic acid |
| **A86** | 3-chloro-1-(pyridin-4-ylmethyl)-1H-pyrrole-2-carboxylic acid |
| **A70** | 1-((2-fluoro-6-methoxypyridin-4-yl)methyl)-1H-pyrrole-2-carboxylic acid |
| **A97** | 1-((2,6-dimethylpyridin-4-yl)methyl)-1H-pyrrole-2-carboxylic acid |
| **A36** | 1-((2,6-difluoropyridin-4-yl)methyl)-1H-pyrrole-2-carboxylic acid |
| **A48** | 4-methyl-1-(pyridin-4-ylmethyl)-1H-pyrrole-2-carboxylic acid |
| **A53** | 1-(4-nitrobenzyl)-1H-pyrrole-2-carboxylic acid |
| **A256** | 1-((3,5-difluoropyridin-4-yl)methyl)-1H-pyrrole-2-carboxylic acid |
| **A210** | 1-((1-methyl-5-oxopyrrolidin-3-yl)methyl)-1H-pyrrole-2-carboxylic acid |
| **A360** | 1-((3-fluoro-5-methylpyridin-4-yl)methyl)-1H-pyrrole-2-carboxylic acid |

### Procedure 3 - Intermediate for A112

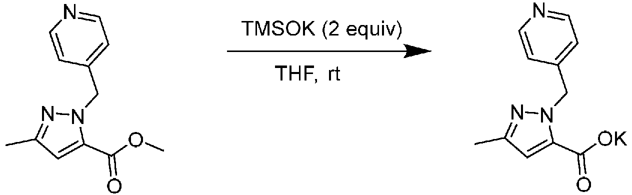

To a solution of methyl ester (0.175 g, 0.76 mmol, 1.0 eq) THF (5.8 mL), potassium trimethylsiolate (0.194 g, 1.51 mmol, 2.0 eq) was added portion-wise. The reaction mixture was stirred at room temperature for 4 h. After this time, the precipitate was filtered, collected and dried under vacuum. The desired product was obtained as the potassium salt.

The following compounds were prepared in a similar manner:

| **Intermediate for:** | **Compound name** |
|---|---|
| **A103** | 3-methyl-1-(pyridin-4-ylmethyl)-1H-pyrazole-5-carboxylic acid |
| **A106** | 6-oxo-1-(pyridin-4-ylmethyl)-1,6-dihydropyridine-2-carboxylic acid |
| **A108** | 1-(pyridin-4-ylmethyl)-1H-imidazole-2-carboxylic acid |
| **A88** | 3-methyl-1-(pyridin-4-ylmethyl)-1H-pyrrole-2-carboxylic acid |
| **A84** | 1-(4-(methylsulfonyl)benzyl)-1H-pyrrole-2-carboxylic acid |
| **A24** | 1-((3-methylpyridin-4-yl)methyl)-1H-pyrrole-2-carboxylic acid |
| **A76** | 5-methyl-1-(pyridin-4-ylmethyl)-1H-pyrrole-2-carboxylic acid |
| **A83** | 1-((2-methylpyridin-4-yl)methyl)-1H-pyrrole-2-carboxylic acid |
| **A125** | 1-((2-methoxypyridin-4-yl)methyl)-1H-pyrrole-2-carboxylic acid |
| **A80** | 1-(4-cyanobenzyl)-1H-pyrrole-2-carboxylic acid |
| **A243** | 1-(3-(3-fluoropyridin-4-yl)propyl)-1H-pyrrole-2-carboxylic acid |

### Procedure 4 - Intermediates for A114 and A99

Synthesis of the intermediates for A114 and A99 followed the scheme below:

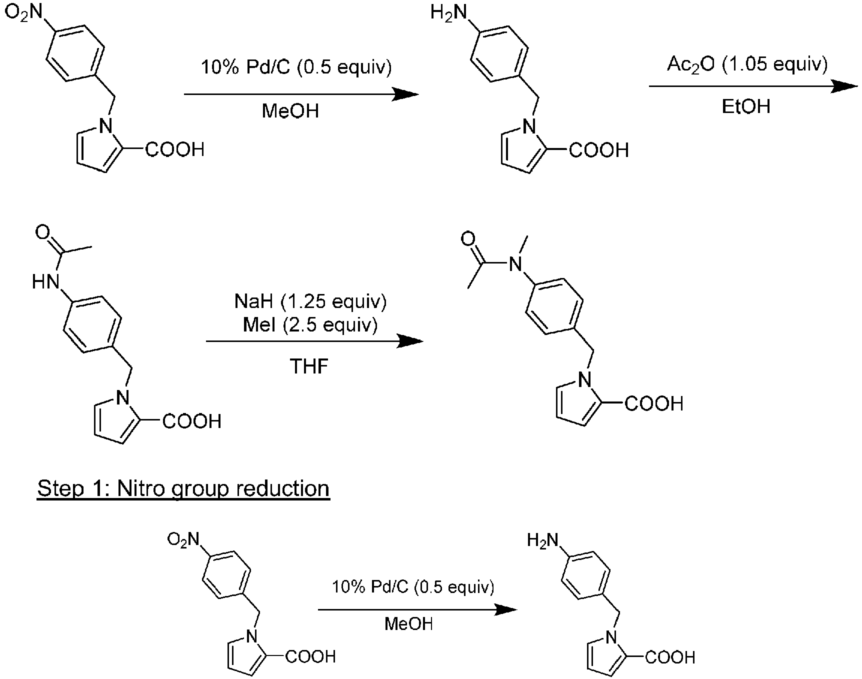

1-(4-nitrobenzyl)-1H-pyrrole-2-carboxylic acid (0.200 g, 0.81 mmol, 1.0 eq.) was dissolved in methanol (10 mL). Then, Pd/C (10 wt%, 0.414 g, 0.41 mmol, 0.5 eq.) was added and reaction was continued under hydrogen atmosphere overnight at room temperature. The next morning, the reaction mixture was filtered through Celite and washed with EtOAc. The filtrate was concentrated to give the desired product, which was used in the next step without further purification.

### Step 2: Aniline acylation

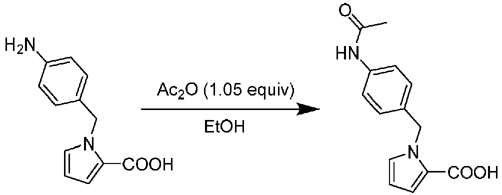

1-(4-acetamidobenzyl)-1H-pyrrole-2-carboxylic acid (0.150 g, 0.69 mmol, 1.0 eq.) was dissolved in ethanol (5 mL). Then, acetic anhydride (0.069 mL, 0.73 mmol, 1.05 eq.) was added, and reaction was stirred overnight at room temperature. The next morning, the volatile materials were evaporated. Water was added, and solution was acidified to pH 4 with 4N HCl. The resulting precipitate was filtered off and dried to yield the desired product (Intermediate for A114).

### Step 3: Amide methylation

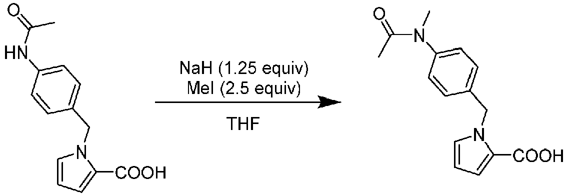

A solution of 1-(4-acetamidobenzyl)-1H-pyrrole-2-carboxylic acid (0.163 g, 0,63 mmol, 1.0 eq) in THF (4 mL) was added dropwise to a slurry of NaH (0.032 g, 0.79 mmol, 1.25 eq) in THF (4 mL) at 0 °C. The mixture was stirred at this temperature for 30 min. After this time, Mel (0.098 ml, 1.58 mmol, 2.5 eq) was added dropwise to the reactinon mixture at 0 °C. The reaction mixture was allowed to warm to room temperature overnight. The next morning, the THF was evaporated, and the resulting material was diluted with water. The aqueous layer was extracted with EtOAc (3 x 30 mL). The aqueous layer was then acidified to pH 4 with 4 N HCl, and the resulting precipitate was filtered off and dried to yield the desired product (Intermediate for A99)

### Example 3: Synthesis of Thiazole Amine Intermediate Compounds

### Procedure 1 - Intermediate for A41

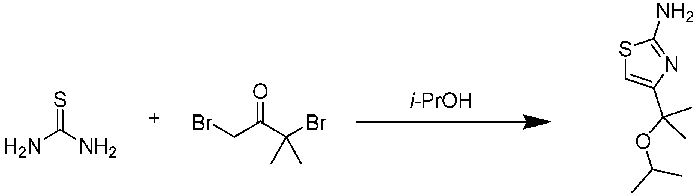

A 100 mL roundbottom flask with stir bar was charged with 1,3-dibromo-3-methylbutan-2-one (1.95 g, 7.99 mmol, 1.0 equiv) and isopropanol (40 mL, 0.2 M). Thiourea (669 mg, 8.79 mmol, 1.1 equiv) was added, and the reaction mixture was stirred at room temperature overnight. The next morning, the solvent was evaporated, and the reaction mixture was diluted with DCM (100 mL). The organic layer was washed with saturated NaHCO₃ (3 x 100 mL), and subsequently dried over Na₂SO₄. The organic layer was filtered and concentrated in vacuo. The resulting crude material was purified via silica gel chromatography (0-50% EtOAc in hexanes) to yield the desired product.

| **Intermediate for:** | **Compound name** |
|---|---|
| **A28** | 4-(1-isopropoxyethyl)thiazol-2-amine |
| **A20** | 4-(1-isopropoxyethyl)-5-methylthiazol-2-amine |
| **A15** | 4-(2-(2,2,2-trifluoroethoxy)propan-2-yl)thiazol-2-amine |
| **A37** | 4-(2-isopropoxypropan-2-yl)-5-methylthiazol-2-amine |
| **A27** | 4-(2-ethoxypropan-2-yl)thiazol-2-amine |
| **A59** | 4-(isopropoxymethyl)thiazol-2-amine |

### Procedure 2 - Intermediate for A39

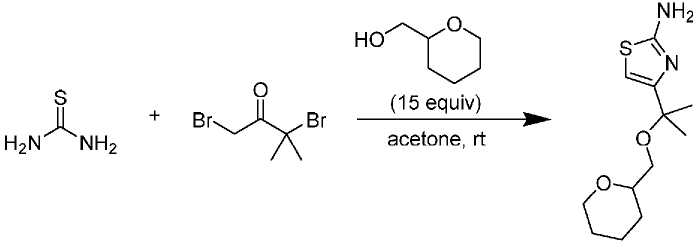

A 40 mL vial with stir bar was charged with 1,3-dibromo-3-methylbutan-2-one (600 mg, 2.46 mmol, 1.0 equiv) and acetone (8 mL, 0.2 M). (Tetrahydro-2H-pyran-2-yl)methanol (4.17 mL, 36.9 mmol, 15 equiv) was added, followed by thiourea (206 mg, 2.71 mmol, 1.1 equiv), and the reaction mixture was stirred at room temperature overnight. The next morning, the reaction mixture was diluted with DCM (100 mL). The organic layer was washed with saturated NaHCO₃ (3 x 100 mL), and subsequently dried over Na₂SO₄. The organic layer was filtered and concentrated in vacuo. The resulting crude material was purified via silica gel chromatography (40-80% EtOAc in hexanes) to yield the desired product.

| **Intermediate for:** | **Compound name** |
|---|---|
| **A2** | 4-(2-(1-(3-chlorophenyl)ethoxy)propan-2-yl)thiazol-2-amine |
| **A8** | 4-(2-(1-(2-chlorophenyl)ethoxy)propan-2-yl)thiazol-2-amine |
| **A5** | 4-(2-(1-(4-chlorophenyl)ethoxy)propan-2-yl)thiazol-2-amine |
| **A4** | 4-(2-(1-(3-methoxyphenyl)ethoxy)propan-2-yl)thiazol-2-amine |
| **A40** | 4-(2-((tetrahydro-2H-pyran-4-yl)methoxy)propan-2-yl)thiazol-2-amine |
| **A18** | (R)-4-(2-(1-phenylethoxy)propan-2-yl)thiazol-2-amine |
| **A1** | (S)-4-(2-(1-phenylethoxy)propan-2-yl)thiazol-2-amine |
| **Ref. B3** | 4-(2-(2-tosylethoxy)propan-2-yl)thiazol-2-amine |
| **A7** | 4-(2-(cyclohexylmethoxy)propan-2-yl)thiazol-2-amine |
| **A10** | 4-(2-(cyclohexyloxy)propan-2-yl)thiazol-2-amine |
| **A9** | 4-(2-(benzyloxy)propan-2-yl)thiazol-2-amine |

### Procedure 3 - Intermediate for A14

Synthesis of these intermediates followed the scheme below:

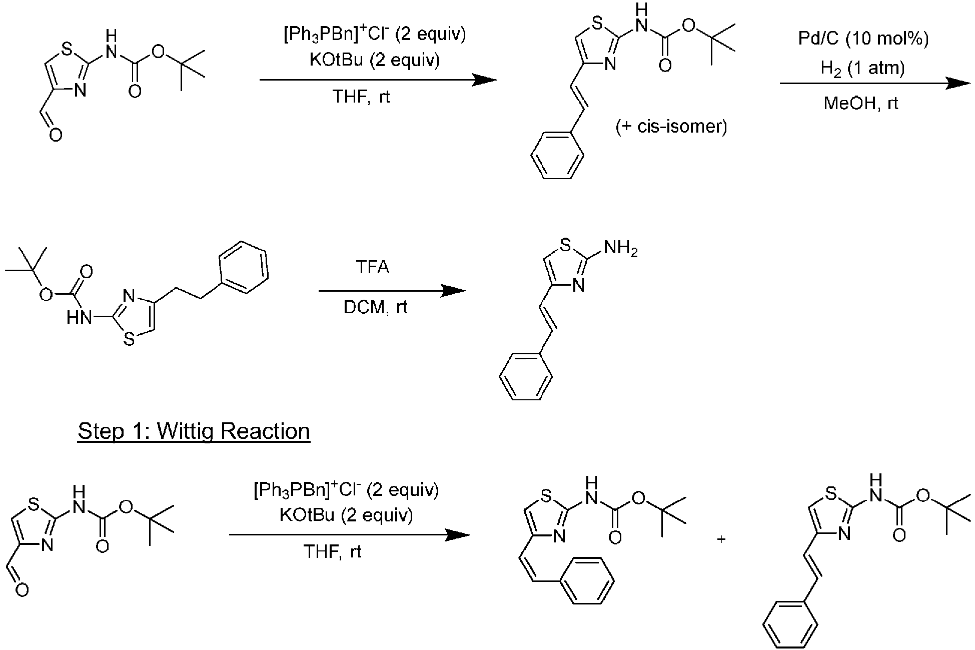

A 100 mL roundbottom flask was charged with benzyltriphenylphosphonium chloride (3.41 g, 8.76 mmol, 2.0 equiv) and THF (20 mL, 0.2 M). Potassium tert-butoxide (1.03 g, 9.20 mmol, 2.1 equiv) was added, and the reaction mixture was allowed to stir at room temperature for 15 min. Tert-butyl (4-formylthiazol-2-yl)carbamate (1.00 g, 4.38 mmol, 1.0 equiv) was then added, and the reaction mixture was allowed to stir at room temperature overnight. The next morning, the solvent was removed in vacuo, and the resulting material was taken up in DCM (100 mL). The organic layer was washed with saturated NH₄Cl (3 x 100 mL). The organic layer was then dried over Na₂SO₄, filtered and concentrated in vacuo. The resulting crude material was purified via silica gel chromatography (0-10% EtOAc in hexanes) to yield the desired cis- and trans- isomers separately.

### Step 2: Hydrogenation

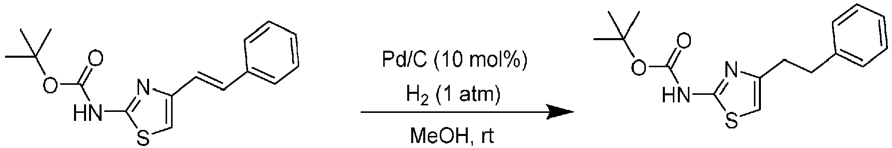

A 20 mL scintillation vial with stir bar was charged with tert-butyl (4-styrylthiazol-2-yl)carbamate (187 mg, 0.618 mmol, 1.0 equiv) and Pd/C (10 wt%, 66 mg, 0.0618 mmol, 0.1 equiv). The solids were evacuated and backflushed with hydrogen (1 atm, 3x). Methanol (5 mL, 0.1 M) was added to the reaction mixture, and the resulting suspension was stirred at room temperature overnight. The next morning, the solids were filtered off, and the resulting crude material was purified via silica gel chromatography (0-30% EtOAc in hexanes) to yield the desired product.

### Step 3: Boc Deprotection

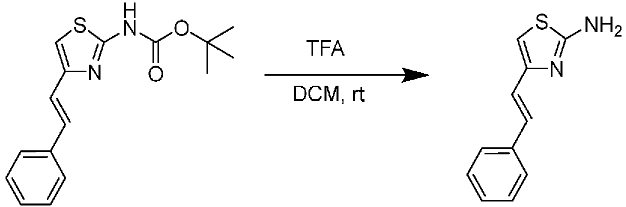

A 20 mL scintillation vial with stir bar was charged with tert-butyl (4-styrylthiazol-2-yl)carbamate (176 mg, 0.582 mmol). DCM (2.7 mL) and TFA (600 uL, 20 vol%) were added, and the reaction mixture was stirred at room temperature overnight. The next morning, the reaction mixture was diluted with DCM (50 mL) and washed with saturated NaHCO₃ (3 x 50 mL). The organic layer was dried over Na₂SO₄, filtered and concentrated in vacuo. The resulting product was used in the next step without further purification.

The following compounds were prepared in a similar manner:

| **Intermediate for:** | **Compound name** |
|---|---|
| **A13** | 4-phenethylthiazol-2-amine |
| **A12** | 4-styrylthiazol-2-amine |

### Procedure 4 - Intermediate for A29

Synthesis of this intermediate followed the scheme, below:

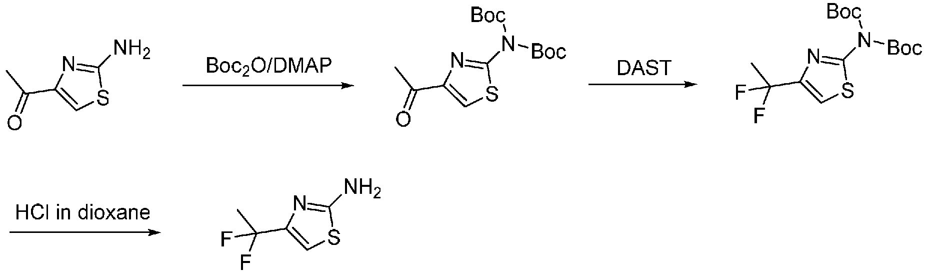

### Step 1: Boc Protection

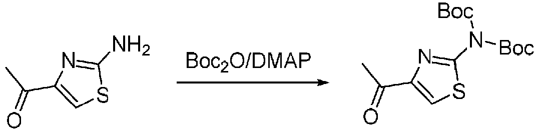

1-(2-aminothiazol-4-yl)ethan-1-one (1.0 g, 2.06 mmol, 1 equiv) was suspended in neat Boc₂O (1.79g, 8.24mmol, 4 equiv) at 60°C, then a catalytic amount of DMAP (0.024g; 0.02 mmol, 0.01 equiv) was added. Gas evolution started. The reaction mixture was left stirring overnight at 60 °C. The reaction mixture was concentrated and directly used into column chromatography. The resulting crude product was purified via silica gel chromatography (10-50% EtOAc in hexanes) to yield the desired product.

### Step 2: Fluorination

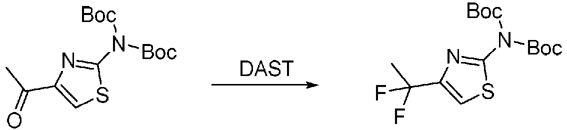

DAST (0.456 g, 2.83 mmol, 3.0 equiv) was mixed with ketone (0.323 g, 0.943 mmol, 1.0 equiv) and left stirring at 50°C for 2 days. After this time, the reaction mixture was diluted with DCM (15 mL), and carefully washed with NaHCO₃ (2 x 15 mL). The organic layer was dried over MgSO₄, filtered and concentrated in vacuo. The resulting crude product was used in the next step without further purification.

### Step 3: Boc deprotection

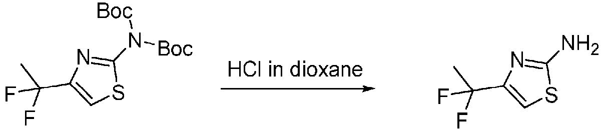

Tert-butyl (tert-butoxycarbonyl)(4-(1,1-difluoroethyl)thiazol-2-yl)carbamate (0.38 g, 1.04 mmol, 1.0 equiv) was dissolved in HCl in dioxane (4M, 20 mL) and heated to 50 °C overnight. The next morning, the volatile materials were removed under reduced pressure and the crude product was used in the next step without further purification.

### Procedure 5 - Intermediate for A132

Synthesis of the intermediate for A132 followed the scheme, below:

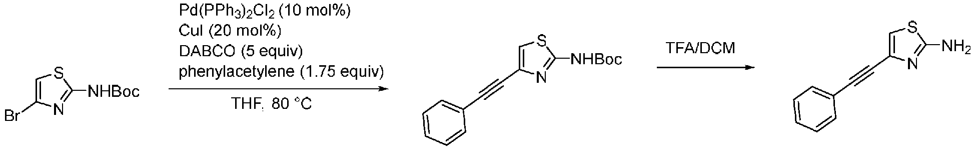

### Step 1: Sonogashira coupling

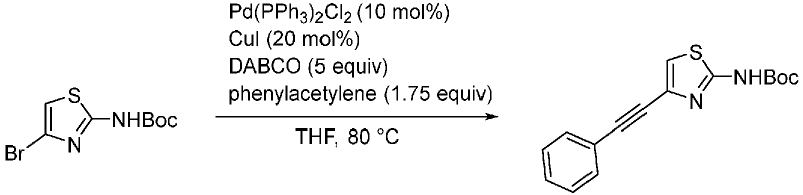

A solution of tert-Butyl (4-bromo-5-formylthiazol-2-yl)carbamate (0.500 g, 1.79 mmol, 1.0 equiv), phenylacetylene (0.573 g, 3.13 mmol, 1.75 equiv) and DABCO (1.005 g, 8.96 mmol, 5.0 equiv) in anhydrous THF (15.0 mL) was purged three times with Ar (g), then Cul (0.003 g, 0.02 mmol, 0.01 equiv) and Pd(PPh₃)₂Cl₂ (0.025 g, 0.04 mmol, 0.02 eq) were added. The reaction mixture was again purged with Ar (g) and then tube was closed and heated overnight at 80 °C. The next morning, the solvent was evaporated and the resulting crude product was purified via silica gel chromatography (0-2% EtOAc in hexanes) to yield the desired product.

### Step 2: General Boc Deprotection Procedure*

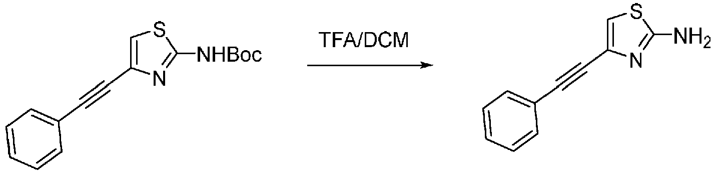

Tert-butyl (4-(phenylethynyl)thiazol-2-yl)carbamate (0.269 g, 0.96 mmol, 1.0 equiv) was dissolved in DCM (8.1 mL), and the solution was cooled to 0 ⁰C. TFA (1.22 mL, 9.64 mmol, 10.0 eq) was added at 0 ⁰C. TLC showed no reaction progress so another portion of TFA (1.22 mL, 9.64 mmol, 10.0 eq) was added after 2h. The reaction mixture was stirred at room temperature for the next 17 h. After this time, the reaction mixture was was cooled down to 0 ⁰C, basified with NaHCO₃ sat. solution (50 mL) and extracted with DCM (3x30 mL). The organic phases were combined, dried over MgSO₄, filtered and evaporated to dryness. Solid residues were washed with pentane and drop of DCM to obtain the desired product, which was used in the next reaction without further purification.

The following compounds were deprotected in a similar manner:

| **Intermediate for:** | **Compound Name** |
|---|---|
| **Ref. B22** | N-(2-(2-amino-5-methylthiazol-4-yl)ethyl)acetamide |
| **A96** | 4-((4-(ethylsulfonyl)piperazin-1-yl)methyl)thiazol-2-amine |
| **A92** | 5-ethyl-4-((4-(ethylsulfonyl)piperazin-1-yl)methyl)thiazol-2-amine |

### Procedure 6 - Intermediate for Reference Compound B22

Synthesis of intermediate for B22 followed the scheme, below

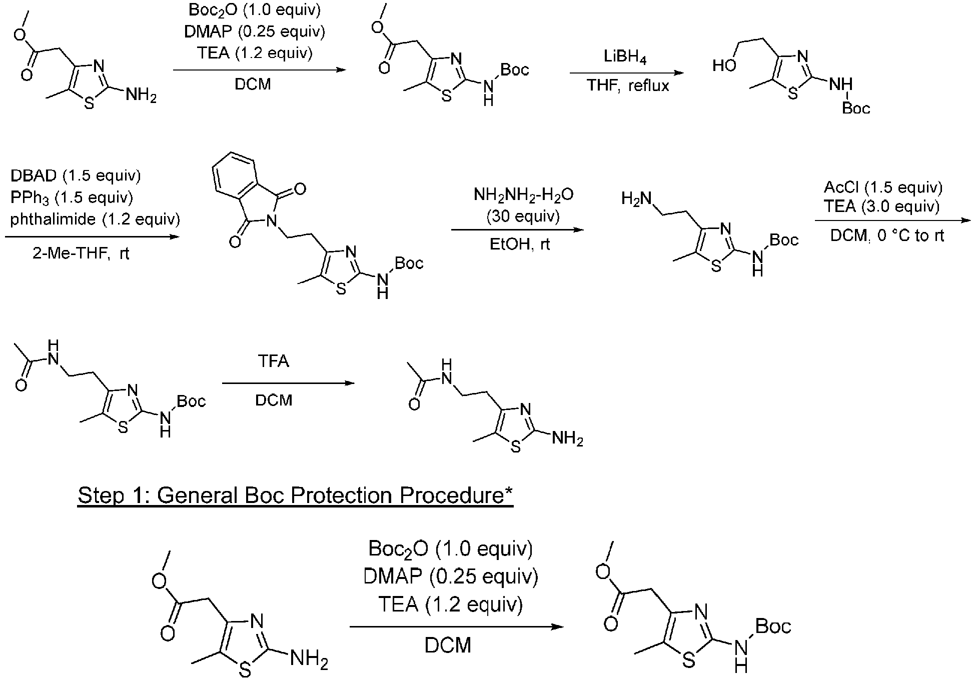

Methyl 2-(2-amino-5-methylthiazol-4-yl)acetate (1.0 g, 5.37 mmol, 1.0 eq.) was dissolved in DCM (10 mL) , then di-tert-butyl dicarbonate (1.41 g, 6.44 mmol, 1.0 eq.) was added followed by TEA (0.90 mL, 6.44 mmol, 1.2 eq.) and DMAP (0.171 g, 1.34 mmol, 0.25 eq.). The reaction was stirred at room temterature overnight. The next morning, the reaction was diluted with DCM (50 mL) and washed with water (2x20 mL). The organic layer was dried over MgSO₄, filtered and evaporated. The resulting crude material was purified by FC eluting with hexanes/EtOAc to yield the desired product.

The following compound was protected in a similar manner:

| **Intemediate for:** | **Compound Name** |
|---|---|
| **A92** | methyl 2-((tert-butoxycarbonyl)amino)-5-ethylthiazole-4-carboxylate |

### Step 2: Ester Reduction

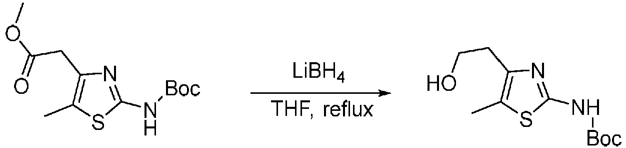

LiBH₄ in THF (0.3 mL, 2 N, 0.6 mmol, 0.6 eq) was added via syringe to a stirred solution of methyl 2-(2-((tert-butoxycarbonyl)amino)-5-methylthiazol-4-yl)acetate (0.285 g, 1.0 mmol, 1.0 eq.) in anhydrous THF (2.0 mL). The reaction was then slowly heated to reflux (initial exotherm). Reaction was continued under reflux for 16 h. After this time, the reaction was cooled down to 0°C and quenched with water (10 mL). The aqueous layer was extracted with EtOAc (4 x 30 mL) and the organic layer was dried over MgSO₄, filtered and evaporated. The resulting crude product was purified by silica gel chromatography (EtOAc: hexane 1:9 to 2:1) to yield the desired product.

### Step 3: Mitsunobu Reaction

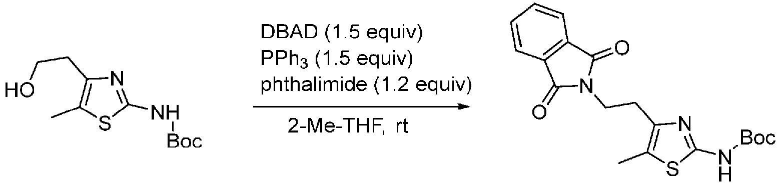

Di-tert-butyl azodicarboxylate (0.319 g, 1.38 mmol, 1.5 eq.) was added portion-wise to a solution of starting material (0.207g, 0.92 mmol, 1.0 eq.), phthalimide (0.163g; 1.11 mmol, 1.2 eq.), and PPh₃ (0.363g, 1.38 mmol, 1.5 eq.) in Me-THF (10 mL) at room temperature under N₂ (g). The reaction mixture was stirred at room temperature overnight. The next morning, the solution was diluted with DCM (10 mL) and washed with 10 percent aqueous solution of K₂CO₃ (2 x 20 mL). The organic layer was dried over MgSO₄, filtered and evaporated to dryness. The resulting crude product was purified by silica gel chromatography (EtOAc: hexane 1:9 to 1:4) to yield the desired product.

### Step 4: Phthalimide hydrolysis

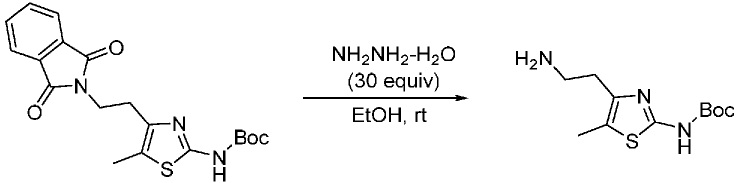

Tert-butyl (4-(2-(1,3-dioxoisoindolin-2-yl)ethyl)-5-methylthiazol-2-yl)carbamate (0.170 g, 1.0 eq.) was dissolved in 2 mL of EtOH. Then, hydrazine monohydrate (0.617 g, 30.0 eq.) was added at 0°C. Reaction was continued at room temperature for 17 hours. The resulting mixture was concentrated and then diluted with DCM (20 mL) and washed with water (2 x 10 mL). The organic layers were combined, dried over MgSO₄, filtered and concentrated in vacuo. The resulting crude product was used in the next step without further purification.

### Step 5: Acetylation

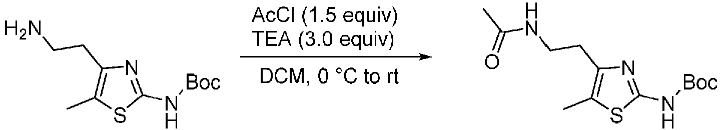

Tert-butyl (4-(2-aminoethyl)-5-methylthiazol-2-yl)carbamate (0.077 g, 0.30 mmol, 1.0 eq.) was dissolved in 2 mL of DCM. Then, TEA (0.125 mL, 0.90 mmol, 3.0 eq.) was added, followed by acetyl chloride (0.032 mL, 0.45 mmol, 1.5 eq.) at 0°C. The reaction was continued at room temperature for 17 hours. The resulting mixture was diluted with water (3 mL) and extracted with DCM (3 x 10 mL). The combined organic layers were dried over MgSO₄, filtered and concentrated in vacuo. The resulting crude product was used to the next step without further purification.

### Step 6: Boc Deprotection

See General Boc Deprotection Procedure, above.

### Procedure 7 - Intermediate for A92

Synthesis of intermediate for A92 followed the scheme, below.

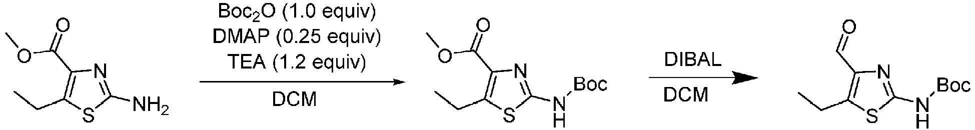

### Step 1: Boc Protection

See General Boc Protection Procedure, above.

### Step 2: DIBAL Reduction

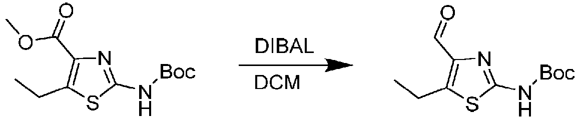

Dried starting material (0.780 g, 2.72 mmol, 1.0 eq.) was dissolved in DCM (8 mL) and diisobutylaluminum hydride (1M in DCM, 8.17 mL, 8.17 mmol, 3.0 eq.) was added at - 78°C to the solution. It was stirred for 5 h. After 5 h, the solution was quenched with methanol and 1N HCl. After the temperature of the reaction mixture was elevated to room temperature, water was added (15 mL) and extracted with DCM (3 x 20 mL). The organic layer was dried over MgSO₄, filtered and evaporated. The resulting crude product was used in the next step without further purification.

### Procedure 8 - Intermediate for A96

Synthesis of intermediate for A96 followed the scheme, below

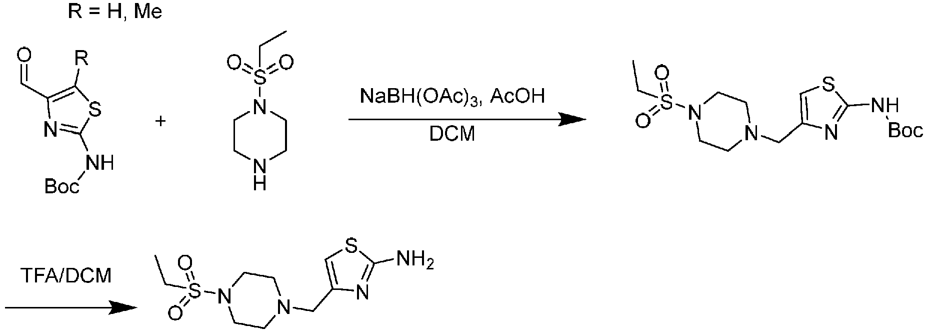

### Step 1: Reductive Amination

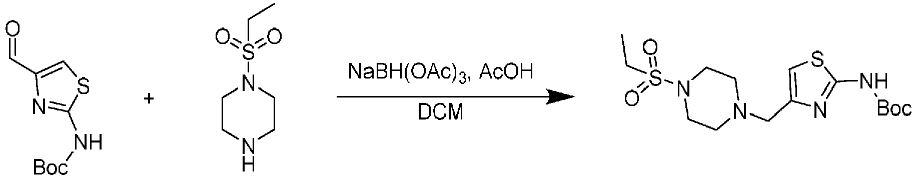

To a stirred solution of 1-(ethanesulfonyl)piperazine (0.500g, 2.8 mmol, 1.0 equiv), in 7 mL of DCM at room temperature was added (4-formylthiazol-2-yl)carbamic acid tert-butyl ester (0.650g, 3.65 mmol, 1.3 equiv). The mixture was then treated with sodium triacetoxyborohydride (0.773g, 3.65 mmol, 1.3 equiv) and a catalytic amount of acetic acid. The reaction was allowed to stir for about 15 hours. The resulting mixture was diluted with NaHCO₃ (40 mL) and extracted with DCM (3 x 30 mL). The organic layers were combined, dried over MgSO₄, filtered and concentrated in vacuo. The resulting crude material was purified via silica gel chromatography (0-100% EtOAc in hexanes) to give desired product.

The following compounds were prepared in a similar manner:

| **Intermediate for:** | **Compound Name** |
|---|---|
| **A92** | tert-butyl (5-ethyl-4-((4-(ethylsulfonyl)piperazin-1-yl)methyl)thiazol-2-yl)carbamate |

### Step 2: Boc deprotection

See General Boc Deprotection Procedure, above.

### Procedure 9 - Intermediate for A209

Synthesis of the intermediate for A209 was prepared according to the scheme, below:

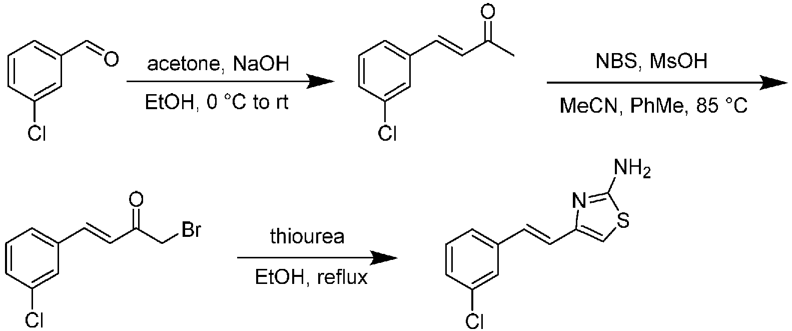

### Step 1: Condensation

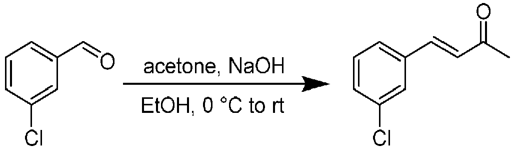

To a solution of NaOH solid (0.569 g, 14.23 mmol, 1.0 eq.) and acetone (4.22 mL, 56.9 mmol, 4.0 eq.) was added water and ethanol. The aldehyde (2.0 g, 14.23 mmol, 1.0 eq.) was added dropwise within 20 min at 0°C. The reaction mixture was allowed to warm to room temperature and was stirred. TLC analysis indicated completion of the reaction after 2 h. After this time, the reaction mixture was quenched with aqueous HCl (1N), adjusted the pH to 6, and then evaporated to remove the residual ethanol. The residue was extracted by ethyl acetate (3 x 40 mL). The combined organic phase was washed with brine (2 x 15 mL), dried over MgSO₄ and concentrated under reduced pressure to yield the desired product, which was used without further purification in the next step.

### Step 2: Bromination

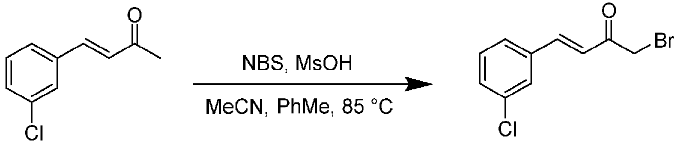

To a solution of enone (1.91 g, 10.57 mmol, 1.0 eq.) in 60 mL ACN:toluene (1:1) was added methanesulfonic acid (1.72 mL, 26.44 mmol, 2.5 eq.) and NBS (1.98g, 11.1 mmol, 1.05 eq.) and heated to 85°C for 6 hours. Sat. NaHCO₃ (70 mL) and EtOAc (4 x 30 mL) were added to the above mixture. The organic layer was separated, dried over magnesium sulfate and evaporated. The resulting crude material was purified via silica gel chromatography (0-25% EtOAc in hexanes) to yield the desired product.

### Step 3: Thiazole condensation

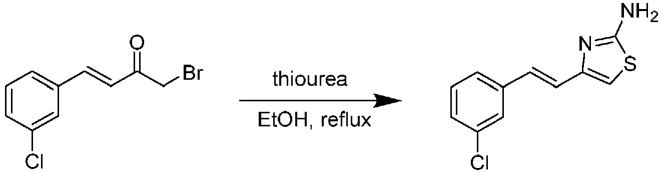

Thiourea (0.442g, 5.81 mmol, 1.1 eq.) was dissolved in absolute ethanol (20 mL). To this solution were added starting material (1.37g, 5.28 mmol, 1.0 eq.). The mixture was refluxed for 3 hours. After this time, the solution was cooled down, and the ethanol was evaporated. Water was added and the resulting solution was basified to pH 10. The aqueous layer was extracted with EtOAc (4 x 30 mL), and the combined organic layers were dried over MgSO₄, filtered and evaporated. The resulting crude product was used in the next step without further purification.

### Procedure 10 - Synthesis of Intermediate for A21

Synthesis of intermediate for A21 followed the scheme, below

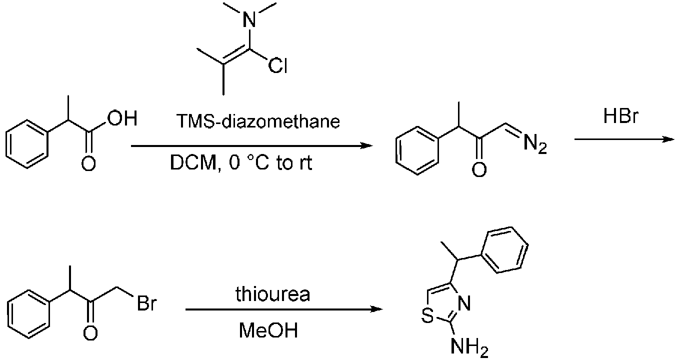

### Step 1: Diazoketone formation

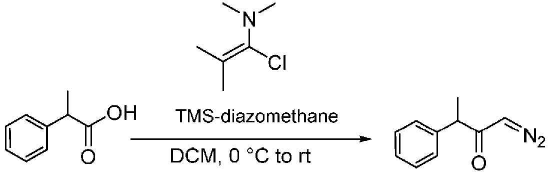

To a stirred solusion of 2-phenylpropionoic acid (1.0 g, 6.62 mmol, 1.0 eq) in DCM (20 mL) at 0 ⁰C were added 4 Å molecular sieves and 1-chloro-N,N,2-trimethyl-1-propenylamine (0.99 mL, 7.54 mmol, 1.14 eq). After 15 min, the reaction mixture was cooled to -20 ⁰C and slowly added to a solution of TMS-diazomethane (3.47 mL, 21.9 mmol, 2.9 eq) in DCM. The reaction mixture was allowed to warm to room temperature and stirred overnight. The reaction mixture was filtered on a sintered funnel and the filtrate was quenched by the addition of saturated NH₄Cl (30 mL). The mixture was extracted with DCM (4 × 40 mL)and the organic layers were separated, and washed with brine. The organic layer was then dried with MgSO4, filtered and concentrated to dryness. The resulting crude material was used in the next step without further purification.

### Step 2: Bromination

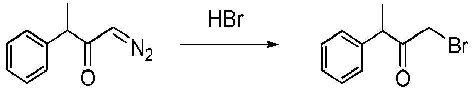

HBr (40% solusion in water, 45 mL) was poured into the round-bottom flask containing crude diazoketone (1.5g, 8.56 mmol, 1.0 eq). The reaction mixture was allowed to be stirred at room temperature for 17 h. After this time, the reaction mixture was quenched with NaHCO₃ sat. solution (100 mL). The mixture was extracted with DCM (4 × 30 mL) and the organic layers were separated, washed with brine, dried with MgSO₄ filtered and concentrated to dryness. The resulting crude material was used without further purification.

### Step 3: Thiazole condensation

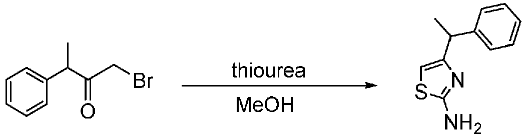

To a solution of crude bromoketone (1.03g , 4.52 mmol, 1.0 eq) in MeOH (10.3 mL), thiourea (0.344g, 4.52 mmol, 1.0 eq) was added. The reaction mixture was allowed to be stirred overnight. LCMS analysis showed consumption of SM and formation of a new product. The next morning, the MeOH was evaporated and crude product was purified by preparatory HPLC (C18 column-prep Mobile phase: ACN + 0.1% FA, H₂O + 0.1% FA) to yield the desired product.

### Procedure 11A - Precursor for Intermediate for A307

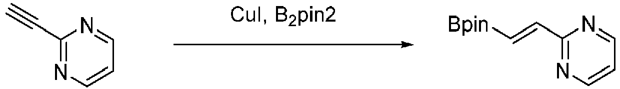

Cul (24.36 mg, 0.243 mmol, 0.05 eq.), XantPhos (140 mg, 0.243 mmol, 0.05 eq.), and NaOtBu (46.6 mg, 0.485 mmol, 0.10 eq.) were mixed in a dry THF (20 mL) in a flame dried Schlenk under argon. The reaction mixture was stirred at room temperature for 30 min. Then B₂pin₂ (1.35g, 5.34 mmol, 1.1 eq.) solution in dry THF (10 mL) was added to mixture and stirred for further 10 min. Then alkyne (500 mg, 4.85 mmol, 1.0 eq.), MeOH (0.441 ml, 9.709 mmol, 2 eq.), and dry THF (5 mL) were added successively. The resulting mixture was stirred for 18 h at room temperature, filtered through Celite and evaporated to dryness. The crude product was purified by column chromatography on silica (Hex/EtOAc) to yield pure product.

### Procedure 11B - Precursor for Intermediate for A157

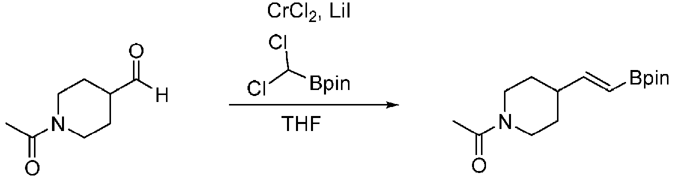

Anhydrous CrCl₂ (950 mg, 7.73 mmol, 8 eq.) was suspended in THF (10 mL) under an argon atmosphere. A solution of aldehyde (150 mg, 0.96 mmol, 1.0 eq.) and dichloromethylboronic ester (407 mg, 1.93 mmol, 2 eq.) in THF (5 mL) and a THF solution of Lil (517 mg, 3.86 mmol, 4 eq.) were added at 25 °C to the suspension successively. After being stirred at 25 °C for 16 h, the reaction mixture was poured into water (25 mL) and extracted with ether (3x10 mL). The combined extracts were dried over Na₂SO₄ and concentrated. Crude product was pure enough to be used in the next step.

The following compounds were prepared via a similar method:

| **Precursor for Intermediate for** | **Compound name** |
|---|---|
| **A155** | 4,4,5,5-tetramethyl-2-(2-(tetrahydro-2H-pyran-2-yl)vinyl)-1,3,2-dioxaborolane |
| **A254** | 2-(2-((1s,4s)-4-methoxycyclohexyl)vinyl)-4,4,5,5-tetramethyl-1,3,2-dioxaborolane |
| **A253** | 2-(2-((1r,4r)-4-methoxycyclohexyl)vinyl)-4,4,5,5-tetramethyl-1,3,2-dioxaborolane |

### Procedure 11C - Precursor for Intermediate for A245

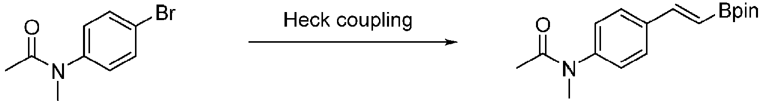

Aryl bromide (1.39 g, 6.09 mmol, 1.0 eq), vinyl boronic pinacol ester (1.06 ml, 6.09 mmol, 1.0 eq) and TEA (1.69 ml, 12.19 mmol, 2.0 eq) were dissolved in toluene (24.5 mL). The mixture was purged with argon for 5 min. After that time Pd(tBu₃P)₂ (0.156 g, 0.30 mmol, 0.05 eq) was added, and the reaction mixture was purged for 2 min with argon. The pressure vessel was closed, and the reaction was heated at 90 C for two hours. LCMS analysis showed consumption of starting material and formation of desired product. The reaction was cooled down to the room temperature and filtered through a plug of Celite. The filtrate was evaporated, and the desired product was recrystallized from Et₂O and washed with pentane. In case of necessity, additional purification by flash chromatography on silica (Hex/EtOAc) was performed.

The following compounds were prepared via a similar method:

| **Precursor for Intermediate for** | **Compound Name** |
|---|---|
| **A248** | 2-(4-cyclopropoxystyryl)-4,4,5,5-tetramethyl-1,3,2-dioxaborolane |
| **A247** | 4,4,5,5-tetramethyl-2-(1-phenylprop-1-en-2-yl)-1,3,2-dioxaborolane |
| **A246** | N,N-dimethyl-4-(2-(4,4,5,5-tetramethyl-1,3,2-dioxaborolan-2-yl)vinyl)benzamide |
| **A245** | N-methyl-N-(4-(2-(4,4,5,5-tetramethyl-1,3,2-dioxaborolan-2-yl)vinyl)phenyl)acetamide |
| **A241** | 2-(2-fluoro-4-methoxystyryl)-4,4,5,5-tetramethyl-1,3,2-dioxaborolane |
| **A240** | 2-(3-fluoro-4-methoxystyryl)-4,4,5,5-tetramethyl-1,3,2-dioxaborolane |
| **A150** | 5-(2-(4,4,5,5-tetramethyl-1,3,2-dioxaborolan-2-yl)vinyl)-1H-indole |
| **A239** | N-methyl-4-(2-(4,4,5,5-tetramethyl-1,3,2-dioxaborolan-2-yl)vinyl)benzamide |
| **A237** | 6-(2-(4,4,5,5-tetramethyl-1,3,2-dioxaborolan-2-yl)vinyl)quinoline |
| **A236** | 2-(4-isopropoxystyryl)-4,4,5,5-tetramethyl-1,3,2-dioxaborolane |
| **A235** | 2-(2-(2,2-difluorobenzo[d][1,3]dioxol-5-yl)vinyl)-4,4,5,5-tetramethyl-1,3,2-dioxaborolane |
| **A234** | 2-(2-(benzo[d][1,3]dioxol-5-yl)vinyl)-4,4,5,5-tetramethyl-1,3,2-dioxaborolane |
| **A233** | 1-(4-(2-(4,4,5,5-tetramethyl-1,3,2-dioxaborolan-2-yl)vinyl)phenyl)piperidine |
| **A232 and A222** | 2-(2-(furan-3-yl)vinyl)-4,4,5,5-tetramethyl-1,3,2-dioxaborolane |
| **A230** | 4-(4-(2-(4,4,5,5-tetramethyl-1,3,2-dioxaborolan-2-yl)vinyl)phenyl)morpholine |
| **A229** | N,N-dimethyl-4-(2-(4,4,5,5-tetramethyl-1,3,2-dioxaborolan-2-yl)vinyl)aniline |
| **A151** | 1-methyl-5-(2-(4,4,5,5-tetramethyl-1,3,2-dioxaborolan-2-yl)vinyl)-1H-indole |
| **A366 and A307** | tert-butyl (4-(2-(4,4,5,5-tetramethyl-1,3,2-dioxaborolan-2-yl)vinyl)thiazol-2-yl)carbamate |

### Procedure 11D - Precursor for Intermediate for A343

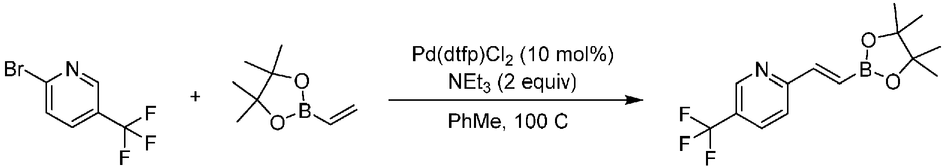

A 20 mL vial with stir bar was charged with bromide (1.3 g, 5.9 mmol, 1.0 equiv) and Pd(dtfp)Cl₂ (380 mg, 0.59 mmol, 0.1 equiv). The vial was evacuated and backflushed with nitrogen. Freshly-sparged toluene (5 mL) was added, followed by vinyl pinacol boronic ester (3.5 mL, 21 mmol, 3.5 equiv) and NEt₃ (1.6 mL, 12 mmol, 2.0 equiv). The vial was capped, and the reaction mixture was allowed to stir at 100 C overnight. The next morning, the reaction mixture was cooled to room temperature and diluted with EtOAc (100 mL). The organic layer was washed with brine (2 × 100 mL), and the combined aqueous layers were extracted with EtOAc (1 × 100 mL). The combined organic layers were dried over Na₂SO₄, filtered and concentrated in vacuo. The resulting crude material was purified by either silca gel chromatography or RP-silica gel chromatography (C18) to yield the desired product.

The following compounds were prepared via a similar method:

| **Precursor for Intermediate for** | **Compound Name** |
|---|---|
| **A358** | 3,5-difluoro-2-(2-(4,4,5,5-tetramethyl-1,3,2-dioxaborolan-2-yl)vinyl)pyridine |
| **A357** | 5-methyl-2-(2-(4,4,5,5-tetramethyl-1,3,2-dioxaborolan-2-yl)vinyl)pyridine |
| **A356** | 2-methyl-6-(2-(4,4,5,5-tetramethyl-1,3,2-dioxaborolan-2-yl)vinyl)pyridine |
| **A347** | 3-methyl-2-(2-(4,4,5,5-tetramethyl-1,3,2-dioxaborolan-2-yl)vinyl)pyridine |
| **A342** | 5-fluoro-2-(2-(4,4,5,5-tetramethyl-1,3,2-dioxaborolan-2-yl)vinyl)pyridine |
| **A341** | 6-(2-(4,4,5,5-tetramethyl-1,3,2-dioxaborolan-2-yl)vinyl)nicotinonitrile |
| **A327** | 2-(2-(4,4,5,5-tetramethyl-1,3,2-dioxaborolan-2-yl)vinyl)-5-(trifluoromethoxy)pyridine |
| **A304** | 2-(4-cyclopropoxy-2-fluorostyryl)-4,4,5,5-tetramethyl-1,3,2-dioxaborolane |
| **A299** | 3-fluoro-5-methoxy-2-(2-(4,4,5,5-tetramethyl-1,3,2-dioxaborolan-2-yl)vinyl)pyridine |

### Procedure 11E - Intermediate for A248 (Route A)

The reactions followed the general reaction scheme, below:

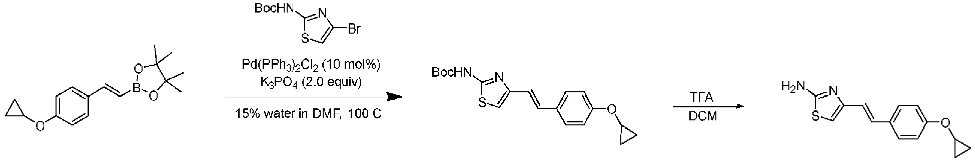

### Step 1A: Suzuki Coupling Procedure

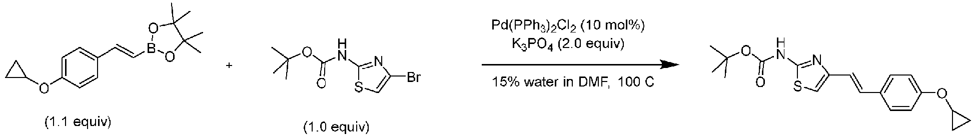

A vial with stir bar was charged with bromide (388 mg, 1.39 mmol, 1.0 equiv), boronic ester (437 mg, 1.53 mmol, 1.1 equiv), potassium phosphate (589 mg, 2.78 mmol, 2.0 equiv) and Pd(PPh₃)₂Cl₂ (97.4 mg, 0.139 mmol, 0.1 equiv). The vial was evacuated and backflushed with nitrogen. 15% water in DMF (sparged with nitrogen for 1 h, 4 mL, 0.4 M) was added. The vial was capped, and the reaction mixture was stirred at 100 C overnight. The next morning, the reaction mixture was poured into EtOAc (50 mL) and washed with 1:1 water:brine (2 × 50 mL). The combined aqueous layers were extracted with EtOAc (1 × 50 mL). The combined organic layers were dried over Na₂SO₄, filtered and concentrated in vacuo. The resulting crude material was purified by silica gel chromatography to yield the desired product.

The following compounds were prepared via the same method:

| **Intermediate for** | **Compound Name** |
|---|---|
| **A269** | tert-butyl (4-(2-(5-methoxypyridin-2-yl)vinyl)thiazol-2-yl)carbamate |
| **A155** | tert-butyl (4-(2-(tetrahydro-2H-pyran-2-yl)vinyl)thiazol-2-yl)carbamate |
| **A254** | tert-butyl (4-(2-((1s,4s)-4-methoxycyclohexyl)vinyl)thiazol-2-yl)carbamate |
| **A253** | tert-butyl (4-(2-((1r,4r)-4-methoxycyclohexyl)vinyl)thiazol-2-yl)carbamate |
| **A246** | tert-butyl (4-(4-(dimethylcarbamoyl)styryl)thiazol-2-yl)carbamate |
| **A245** | tert-butyl (4-(4-(N-methylacetamido)styryl)thiazol-2-yl)carbamate |
| **A157** | tert-butyl (4-(2-(1-acetylpiperidin-4-yl)vinyl)thiazol-2-yl)carbamate |
| **A241** | tert-butyl (4-(2-fluoro-4-methoxystyryl)thiazol-2-yl)carbamate |
| **A240** | tert-butyl (4-(3-fluoro-4-methoxystyryl)thiazol-2-yl)carbamate |
| **A150** | tert-butyl (4-(2-(1H-indol-5-yl)vinyl)thiazol-2-yl)carbamate |
| **A239** | tert-butyl (4-(4-(methylcarbamoyl)styryl)thiazol-2-yl)carbamate |
| **A237** | tert-butyl (4-(2-(quinolin-6-yl)vinyl)thiazol-2-yl)carbamate |
| **A230** | tert-butyl (4-(4-morpholinostyryl)thiazol-2-yl)carbamate |
| **A151** | tert-butyl (4-(2-(1-methyl-1H-indol-5-yl)vinyl)thiazol-2-yl)carbamate |
| **A217 and A218** | tert-butyl (4-(3-phenylprop-1-en-1-yl)thiazol-2-yl)carbamate |
| **A222 and A232** | tert-butyl (4-(2-(furan-3-yl)vinyl)thiazol-2-yl)carbamate |
| **A234** | tert-butyl (4-(2-(benzo[d][1,3]dioxol-5-yl)vinyl)thiazol-2-yl)carbamate |
| **A235** | tert-butyl (4-(2-(2,2-difluorobenzo[d][1,3]dioxol-5-yl)vinyl)thiazol-2-yl)carbamate |
| **A236** | tert-butyl (4-(4-isopropoxystyryl)thiazol-2-yl)carbamate |
| **A242** | tert-butyl (4-(1H-inden-2-yl)thiazol-2-yl)carbamate |
| **A244** | tert-butyl (4-(2-(cyclohex-1-en-1-yl)vinyl)thiazol-2-yl)carbamate |
| **A247** | tert-butyl (4-(1-phenylprop-1-en-2-yl)thiazol-2-yl)carbamate |
| **A248** | tert-butyl (4-(4-cyclopropoxystyryl)thiazol-2-yl)carbamate |
| **A249** | tert-butyl (4-(1,2,3,6-tetrahydro-[1,1'-biphenyl]-4-yl)thiazol-2-yl)carbamate |
| **A258** | tert-butyl (4-(1-phenyl-1,2,3,6-tetrahydropyridin-4-yl)thiazol-2-yl)carbamate |
| **A259** | tert-butyl (4-(1-benzyl-1,2,3,6-tetrahydropyridin-4-yl)thiazol-2-yl)carbamate |
| **A327** | tert-butyl (4-(2-(5-(trifluoromethoxy)pyridin-2-yl)vinyl)thiazol-2-yl)carbamate |
| **A341** | tert-butyl (4-(2-(5-cyanopyridin-2-yl)vinyl)thiazol-2-yl)carbamate |
| **A342** | tert-butyl (4-(2-(5-fluoropyridin-2-yl)vinyl)thiazol-2-yl)carbamate |
| **A343** | tert-butyl (4-(2-(5-(trifluoromethyl)pyridin-2-yl)vinyl)thiazol-2-yl)carbamate |
| **A272** | tert-butyl (4-(2-([1,3]dioxolo[4,5-b]pyridin-5-yl)vinyl)thiazol-2-yl)carbamate |
| **A274** | tert-butyl (4-(2-([1,3]dioxolo[4,5-b]pyridin-6-yl)vinyl)thiazol-2-yl)carbamate |
| **A294** | tert-butyl (4-(1,4,5,6-tetrahydro-[1,1'-biphenyl]-3-yl)thiazol-2-yl)carbamate |
| **A229** | tert-butyl (4-(4-(dimethylamino)styryl)thiazol-2-yl)carbamate |
| **A233** | tert-butyl (4-(4-(piperidin-1-yl)styryl)thiazol-2-yl)carbamate |
| **A223** | tert-butyl (4-(4-acetamidostyryl)thiazol-2-yl)carbamate |
| **A299** | tert-butyl (4-(2-(3-fluoro-5-methoxypyridin-2-yl)vinyl)thiazol-2-yl)carbamate |
| **A304** | tert-butyl (4-(4-cyclopropoxy-2-fluorostyryl)thiazol-2-yl)carbamate |
| **A347** | tert-butyl (4-(2-(3-methylpyridin-2-yl)vinyl)thiazol-2-yl)carbamate |
| **A356** | tert-butyl (4-(2-(6-methylpyridin-2-yl)vinyl)thiazol-2-yl)carbamate |
| **A357** | tert-butyl (4-(2-(5-methylpyridin-2-yl)vinyl)thiazol-2-yl)carbamate |
| **A358** | tert-butyl (4-(2-(3,5-difluoropyridin-2-yl)vinyl)thiazol-2-yl)carbamate |

### Step 1A: Reverse Suzuki Coupling

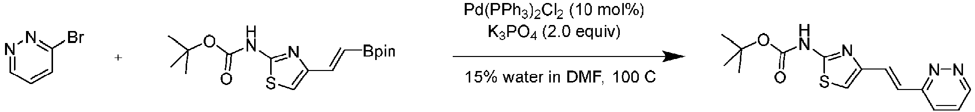

A vial with stir bar was charged with bromide (51.7 mg, 0.325 mmol, 1.0 equiv), boronic ester (126 mg, 0.358 mmol, 1.1 equiv), K₃PO₄ (138 mg, 0.650 mmol, 2.0 equiv) and Pd(PPh₃)₂Cl₂ (22.8 mg, 0.0325 mmol, 0.1 equiv). The vial was evacuated and backflushed with nitrogen. Freshly-sparged 15% water in DMF (2 mL) was added, and the vial was capped. The reaction mixture was stirred at 100 C for 2 h. After 2 h, the reaction mixture was cooled to room temperature and poured into EtOAc (50 mL). The resulting solution was washed with brine (2 × 50 mL), and the combined aqueous layers were extracted with EtOAc (1 × 50 mL). The combined organic layers were dried over Na₂SO₄, filtered and concentrated in vacuo. The resulting crude material was purified via silica gel chromatography.

The following compounds were prepared via a similar method:

| **Intermediate for** | **Compound Name** |
|---|---|
| **A307** | tert-butyl (4-(2-(pyrimidin-2-yl)vinyl)thiazol-2-yl)carbamate |

### Step 2: Boc Deprotection

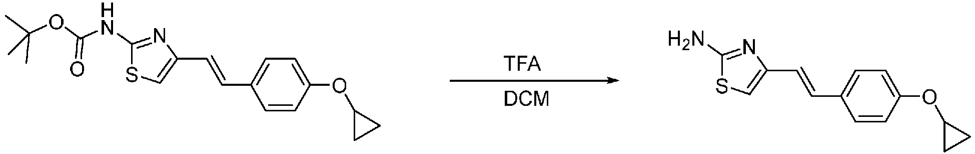

A 20 mL vial with stir bar was charged with carbamate (270 mg, 0.753 mmol, 1.0 equiv) and DCM (4 mL). TFA (1 mL, 13 mmol, 17 equiv) was added, and the reaction mixture was stirred at room temperature overnight. The next morning, the reaction mixture was poured into DCM (50 mL) and washed with saturated NaHCO₃ (2 × 50 mL). The combined aqueous layers were extracted with DCM (1 × 50 mL), and the combined organic layers were dried over Na₂SO₄, filtered and concentrated in vacuo. The resulting material was used in the next step without further purification.

The following compounds were prepared via a similar method:

| **Intermediate for** | **Compound Name** |
|---|---|
| **A269** | 4-(2-(5-methoxypyridin-2-yl)vinyl)thiazol-2-amine |
| **A155** | 4-(2-(tetrahydro-2H-pyran-2-yl)vinyl)thiazol-2-amine |
| **A254** | 4-(2-((1s,4s)-4-methoxycyclohexyl)vinyl)thiazol-2-amine |
| **A253** | 4-(2-((1r,4r)-4-methoxycyclohexyl)vinyl)thiazol-2-amine |
| **A246** | 4-(2-(2-aminothiazol-4-yl)vinyl)-N,N-dimethylbenzamide |
| **A245** | N-(4-(2-(2-aminothiazol-4-yl)vinyl)phenyl)-N-methylacetamide |
| **A157** | 1-(4-(2-(2-aminothiazol-4-yl)vinyl)piperidin-1-yl)ethan-1-one |
| **A241** | 4-(2-fluoro-4-methoxystyryl)thiazol-2-amine |
| **A240** | 4-(3-fluoro-4-methoxystyryl)thiazol-2-amine |
| **A150** | 4-(2-(1H-indol-5-yl)vinyl)thiazol-2-amine |
| **A239** | 4-(2-(2-aminothiazol-4-yl)vinyl)-N-methylbenzamide |
| **A237** | 4-(2-(quinolin-6-yl)vinyl)thiazol-2-amine |
| **A230** | 4-(4-morpholinostyryl)thiazol-2-amine |
| **A151** | 4-(2-(1-methyl-1H-indol-5-yl)vinyl)thiazol-2-amine |
| **A217 and A218** | 4-(3-phenylprop-1-en-1-yl)thiazol-2-amine |
| **A222 and A232** | 4-(2-(furan-3-yl)vinyl)thiazol-2-amine |
| **A234** | 4-(2-(benzo[d][1,3]dioxol-5-yl)vinyl)thiazol-2-amine |
| **A235** | 4-(2-(2,2-difluorobenzo[d][1,3]dioxol-5-yl)vinyl)thiazol-2-amine |
| **A236** | 4-(4-isopropoxystyryl)thiazol-2-amine |
| **A242** | 4-(1H-inden-2-yl)thiazol-2-amine |
| **A244** | 4-(2-(cyclohex-1-en-1-yl)vinyl)thiazol-2-amine |
| **A247** | 4-(1-phenylprop-1-en-2-yl)thiazol-2-amine |
| **A248** | 4-(4-cyclopropoxystyryl)thiazol-2-amine |
| **A249** | 4-(1,2,3,6-tetrahydro-[1,1'-biphenyl]-4-yl)thiazol-2-amine |
| **A258** | 4-(1-phenyl-1,2,3,6-tetrahydropyridin-4-yl)thiazol-2-amine |
| **A259** | 4-(1-benzyl-1,2,3,6-tetrahydropyridin-4-yl)thiazol-2-amine |
| **A327** | 4-(2-(5-(trifluoromethoxy)pyridin-2-yl)vinyl)thiazol-2-amine |
| **A341** | 6-(2-(2-aminothiazol-4-yl)vinyl)nicotinonitrile |
| **A342** | 4-(2-(5-fluoropyridin-2-yl)vinyl)thiazol-2-amine |
| **A343** | 4-(2-(5-(trifluoromethyl)pyridin-2-yl)vinyl)thiazol-2-amine |
| **A272** | 4-(2-([1,3]dioxolo[4,5-b]pyridin-5-yl)vinyl)thiazol-2-amine |
| **A274** | 4-(2-([1,3]dioxolo[4,5-b]pyridin-6-yl)vinyl)thiazol-2-amine |
| **A294** | 4-(1,4,5,6-tetrahydro-[1,1'-biphenyl]-3-yl)thiazol-2-amine |
| **A366** | 4-(2-(pyridazin-3-yl)vinyl)thiazol-2-amine |
| **A229** | 4-(4-(dimethylamino)styryl)thiazol-2-amine |
| **A233** | 4-(4-(piperidin-1-yl)styryl)thiazol-2-amine |
| **A307** | 4-(2-(pyrimidin-2-yl)vinyl)thiazol-2-amine |
| **A223** | N-(4-(2-(2-aminothiazol-4-yl)vinyl)phenyl)acetamide |
| **A299** | 4-(2-(3-fluoro-5-methoxypyridin-2-yl)vinyl)thiazol-2-amine |
| **A304** | 4-(4-cyclopropoxy-2-fluorostyryl)thiazol-2-amine |
| **A347** | 4-(2-(3-methylpyridin-2-yl)vinyl)thiazol-2-amine |
| **A356** | 4-(2-(6-methylpyridin-2-yl)vinyl)thiazol-2-amine |
| **A357** | 4-(2-(5-methylpyridin-2-yl)vinyl)thiazol-2-amine |
| **A358** | 4-(2-(3,5-difluoropyridin-2-yl)vinyl)thiazol-2-amine |

### Procedure 12A - Precursor for Intermediate for A289

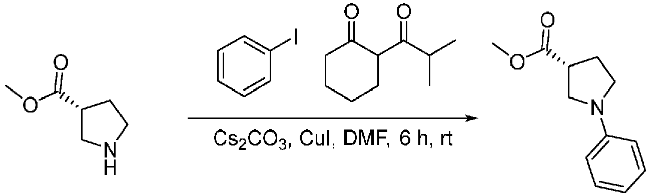

A 100 mL roundbottom flask with stir bar was charged with D-proline ester (1.00 g, 7.7 mmol, 1.00 equiv), iodobenzene (1.90 g, 9.3 mmol, 1.2 equiv), 2-(2-methylpropanoyl)cyclohexan-1-one (521 mg, 3.1 mmol, 0.4 equiv), Cs₂CO₃ (7.57 g, 23.227 mmol, 3 equiv), Cul (147.45 mg, 0.774 mmol, 0.1 equiv) and DMF (30 mL, 0.26 M) under nitrogen atmosphere, the vial was capped and placed in an 50°C bath. The reaction mixture was stirred at 50 °C overnight. The next morning, the reaction mixture was poured into DCM (200 mL) and washed with brine (3 × 100 mL). The organic layer was dried over Na₂SO₄, filtered and concentrated in vacuo. The resulting crude material was purified via silica gel chromatography to yield the desired product.

The following compounds were prepared via a similar method:

| **Precursor for Intermediate for** | **Compound name** |
|---|---|
| **A290** | methyl (R)-1-phenylpyrrolidine-3-carboxylate |

### Procedure 12B - Precursor for Intermediate for A359

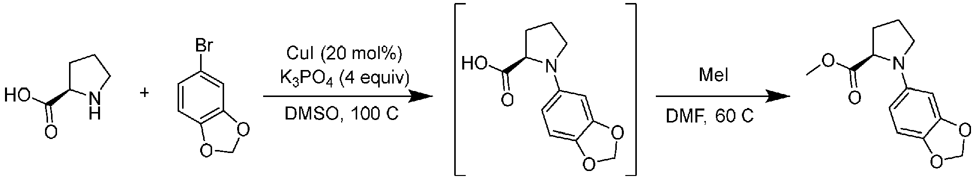

A 40 mL vial with stir bar was charged with D-proline (1.43 g, 12.4 mmol, 2.5 equiv), aryl bromide (1.00 g, 4.97 mmol, 1.0 equiv), Cul (189 mg, 0.994 mmol, 0.2 equiv) and K₃PO₄ (4.22 g, 19.9 mmol, 4.0 equiv). The vial was evacuated and backflushed with nitrogen. Freshly-sparged DMSO (6 mL) was added. The vial was capped, and the reaction mixture was allowed to stir at 100 C overnight. The next morning, the reaction mixture was cooled to 60 C and diluted with DMF (10 mL). Mel (1.55 mL, 24.8 mmol, 5.0 equiv) was added, and the reaction mixture was allowed to stir at 60 C for 2 h. After 2 h, the reaction mixture was poured into EtOAc (150 mL) and washed with brine (2 × 150 mL). The combined aqueous layers were extracted with EtOAc (1 × 50 mL). The combined organic layers were dried over Na₂SO₄, filtered and concentrated in vacuo. The resulting crude material was purified via silica gel chromatography to yield the desired product.

The following compounds were prepared via a similar method:

| **Precursor for Intermediate for** | **Compound name** |
|---|---|
| **A328** | methyl (2,2-difluorobenzo[d][1,3]dioxol-5-yl)-D-prolinate |

### Procedure 12C - Precursor for Intermediate for A319

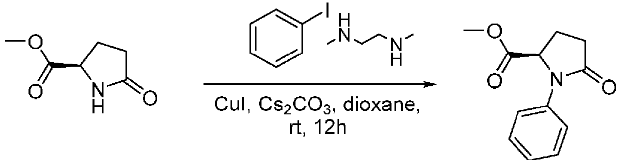

A 100 mL roundbottom flask with stir bar was charged with D-proline ester (500 mg, 3.2 mmol, 1.00 equiv), iodobenzene (649 mg, 3.181 mmol, 1.00 equiv), methyl[2-(methylamino)ethyl]amine (280 mg, 3.2 mmol, 1 equiv), Cul (303 mg, 1.6 mmol, 0.5 equiv), Cs₂CO₃ (2.59 g, 8.0 mmol, 2.5 equiv) and dioxane (20 mL, 0.16 M) under nitrogen atmosphere, the vial was capped and placed in an 25°C bath. The reaction mixture was stirred at 25°C overnight. The next morning, the reaction mixture was poured into DCM (80 mL) and washed with brine (2 × 40 mL). The organic layer was dried over Na₂SO₄, filtered and concentrated in vacuo. The resulting crude material was purified via silica gel chromatography to yield the desired product.

### Procedure 12D - Procedure for Intermediate for A279

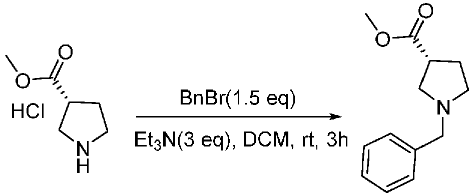

A 100 mL roundbottom flask with stir bar was charged with pyrrolidine ester (1.50 g, 11.6 mmol, 1.00 equiv), TEA (3.53 g, 34 mmol, 3.00 equiv) and DCM (20 mL, 0.58 M). Benzyl bromide (2.38 g, 14.0 mmol, 1.20 equiv) was added, and the vial was capped and placed in a 25°C bath. The reaction mixture was stirred at 25°C for 2 h. The reaction mixture was poured into DCM (50 mL) and washed with H₂O (3 × 50 mL). The organic layer was dried over Na₂SO₄, filtered and concentrated in vacuo. The resulting crude material was purified via silica gel chromatography to yield the desired product.

The following compounds were prepared via a similar method:

| **Precursor for Intermediate for** | **Compound Name** |
|---|---|
| **A277** | methyl benzyl-D-prolinate |
| **A278** | methyl benzyl-L-prolinate |
| **A291** | methyl (R)-1-benzylpyrrolidine-3-carboxylate |
| **A302** | methyl 1-benzylpiperidine-4-carboxylate |

### Procedure 12E - Precursor for Intermediate for A320

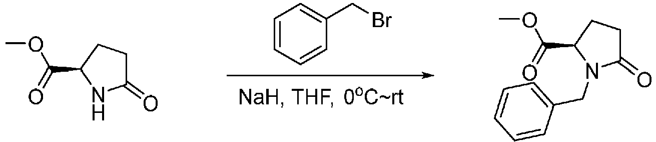

A 50 mL roundbottom flask with stir bar was charged with D-proline ester (880 mg, 6.1 mmol, 1.00 equiv), benzyl bromide (1.15 g, 6.7 mmol, 1.09 equiv) and THF (12 mL, 0.51 M). NaH (60%, 369 mg, 9.2 mmol,1.5 equiv) was added in portions at 0°C. The vial was capped and placed in an 25°C bath. The reaction mixture was stirred at 25°C for 5h. The reaction mixture was quenched by NH₄Cl (aq) (25 mL). The resulting solution was extracted with ethyl acetate (3 × 30 mL) and washed with brine (2 × 30 mL). The combined organic layers were dried over Na₂SO₄, filtered and concentrated in vacuo. The resulting crude material was purified via silica gel chromatography to yield the desired product.

### Procedure 12F - Precursor for Intermediate for A323

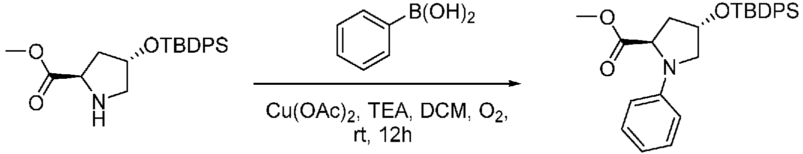

A 100 mL roundbottom flask with stir bar was charged with pyrrolidine ester (2.00 g, 4.4 mmol, 1.00 equiv), phenyl boronic acid (1.59 g, 13.0 mmol, 3 equiv), Cu(OAc)₂ (2.37 g, 13.0 mmol, 3 equiv), TEA (2.20 g, 21.8 mmol, 5 equiv) and DCM (30 mL, 0.15 M) under nitrogen atmosphere. The reaction flask was then vacuumed and flushed with oxygen, and the sequence was repeated twice. The vial was capped and placed in a 25°C bath. The reaction mixture was stirred at 25°C for 48 h under oxygen atmosphere using an oxygen balloon. The reaction mixture was poured into DCM (150 mL) and quenched by the addition of NH₃.H₂O (20 mL), washed with H₂O (1 × 50 mL) and brine (3 × 50 mL). The organic layer was dried over Na₂SO₄, filtered and concentrated in vacuo. The resulting crude material was purified via silica gel chromatography to yield the desired product.

The following compounds were prepared via a similar method:

| **Precursor for Intermediate for** | **Compound Name** |
|---|---|
| **A332** | methyl (R)-1-phenylpiperidine-2-carboxylate |
| **A333** | methyl (2R,4S)-4-methoxy-1-phenylpyrrolidine-2-carboxylate |
| **A334** | methyl (2R,3S)-3-methoxy-1-phenylpyrrolidine-2-carboxylate |
| **A336** | methyl (R)-1-phenylazetidine-2-carboxylate |
| **A337** | methyl (R)-4-phenylmorpholine-3-carboxylate |
| **A339** | methyl (R)-4,4-difluoro-1-phenylpyrrolidine-2-carboxylate |
| **A344 and A352** | methyl (2R,3S)-3-((tert-butyldiphenylsilyl)oxy)-1-phenylpyrrolidine-2-carboxylate |
| **A345** | methyl (2R,3R)-3-methoxy-1-phenylpyrrolidine-2-carboxylate |
| **A346** | methyl (2R,4R)-4-isopropoxy-1-phenylpyrrolidine-2-carboxylate |
| **A351 and A353** | methyl (2R,3R)-3-((tert-butyldiphenylsilyl)oxy)-1-phenylpyrrolidine-2-carboxylate |
| **A354** | methyl (2R,4R)-4-(cyclohexyloxy)-1-phenylpyrrolidine-2-carboxylate |
| **A306** | methyl 1-phenylpiperidine-3-carboxylate |
| **A338** | methyl (R)-2-methyl-1-phenylpyrrolidine-2-carboxylate |

### Procedure 12G - Precursor for Intermediate for A285

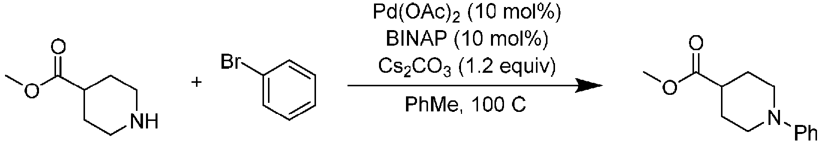

A vial with stir bar was charged with amine (1.4 g, 9.7 mmol, 1.2 equiv), Cs₂CO₃ (3.2 g, 9.7 mmol, 1.2 equiv), Pd(OAc)₂ (180 mg, 0.81 mmol, 0.1 equiv), and BINAP (510 mg, 0.81 mmol, 0.1 equiv). The vial was evacuated and backflushed with nitrogen. Freshly sparged toluene (15 mL) was added, followed by bromobenzene (0.85 mL, 8.1 mmol, 1.0 equiv). The reaction mixture was capped and allowed to stir at 100 C overnight. The next morning, the reaction mixture was cooled to room temperature and poured into EtOAc (100 mL). The organic layer was washed with brine (2 × 100 mL) and the combined aqueous layers were extracted with EtOAc (1 × 100 mL). The combined organic layers were dried over Na₂SO₄, filtered and concentrated in vacuo. The resulting crude material was purified via silica gel chromatography to yield the desired product.

The following compounds were prepared via a similar method:

| **Precursor for Intermediate for** | **Compound name** |
|---|---|
| **A315** | methyl 1-(5-methoxypyridin-2-yl)azetidine-3-carboxylate |
| **A361** | methyl (2,2-difluorobenzo[d][1,3]dioxol-4-yl)-D-prolinate |
| **A362** | methyl benzo[d][1,3]dioxoi-d-yl-D-prolinate |

### Procedure 12H - Precursor for Intermediate for A322

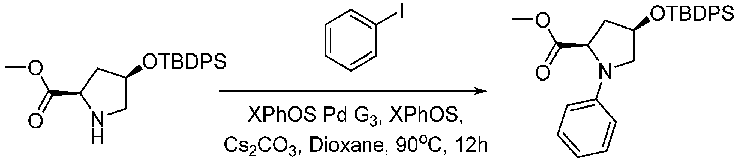

A 250 mL roundbottom flask with stir bar was charged with pyrrolidine ester (2.40 g, 6.257 mmol, 1.00 equiv), iodobenzene (1.91 g, 9.362 mmol, 1.50 equiv), Cs₂CO₃ (6.10 g, 18.722 mmol, 2.99 equiv), XPhOS (595.00 mg, 1.248 mmol, 0.20 equiv), XPhOS Pd G3 (1.06 g, 1.252 mmol, 0.20 equiv) and dioxane (20 mL, 0.16 M) under nitrogen atmosphere, the vial was capped and placed in an 90°C bath. The reaction mixture was stirred at 90°C overnight. The next morning, the reaction mixture was cooled to room temperature and quenched by H₂O (50 mL). The resulting solution was extracted with (3 × 50 mL) of ethyl acetate. The combined organic layers were dried over Na₂SO₄, filtered and concentrated in vacuo. The resulting crude material was purified via prep-TLC to yield the desired product.

The following compounds were prepared via a similar method:

| **Precursor for Intermediate for** | **Compound Name** |
|---|---|
| **A323 and A326** | methyl (2R,4S)-4-((tert-butyldiphenylsilyl)oxy)-1-phenylpyrrolidine-2-carboxylate |
| **A322 and A321** | methyl (2R,4R)-4-((tert-butyldiphenylsilyl)oxy)-1-phenylpyrrolidine-2-carboxylate |

### Procedure 12I - Precursor for Intermediate for A365

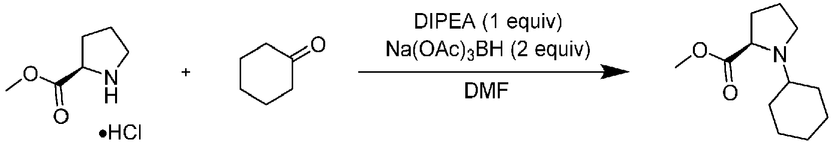

A vial with stir bar was charged with methyl D-prolinate hydrochloride (100 mg, 0.604 mmol, 1.0 equiv), DIPEA (0.11 mL, 0.604 mmol, 1.0 equiv), cyclohexanone (62.6 uL, 0.604 mmol, 1.0 equiv) and DMF (1 mL). The reaction mixture was cooled to 0 C. Na(OAc)₃BH () was added, and the reaction mixture was allowed to warm to room temperature overnight. The next morning, the reaction mixture was diluted with EtOAc (50 mL) and washed with saturated NaHCO₃ (3 × 50 mL). The combined aqueous layers were extracted with EtOAc (1 × 50 mL), and the combined organic layers were dried over Na₂SO₄, filtered and concentrated in vacuo. The resulting material was used without further purification in the next step.

### Procedure 12J - Precursor for Intermediate for A313

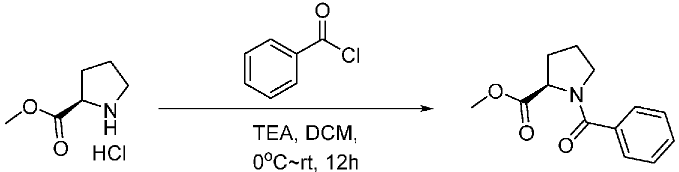

A 250 mL roundbottom flask with stir bar was charged with pyrrolidine ester (4.00 g, 24 mmol, 1.00 equiv), TEA (8.40 mL, 60 mmol, 2.50 equiv) and DCM (100 mL, 0.24 M). Benzoyl chloride (2.8 mL, 24 mmol, 1.01 eq) was added, and the vial was capped and placed in a 0°C bath. The reaction mixture was stirred at 25°C overnight. The next morning, the reaction mixture was poured into DCM (50 mL) and washed with H₂O (2 × 70 mL). The organic layer was dried over Na₂SO₄, filtered and concentrated in vacuo. The resulting crude material was purified via silica gel chromatography to yield the desired product.

### Procedure 12K - Synthesis of Intermediates via Halogenation (e.g., for A163, A154, A158, & A292) (Route B)

The reactions followed the general reaction scheme, below:

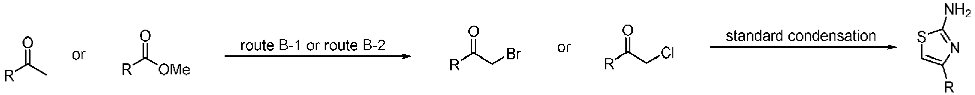

In accordance with this procedure, compounds having a single stereocenter were prepared and evaluated as a single enantiomer. Unless indicated otherwise, compounds having two stereocenters were prepared and evaluated as a single diastereomer, but as a mixture of enantiomers. Each of compounds A321-A323, A325, A326, A333, A334, A344-A346, and A351-A354 (having two stereocenters) where prepared and evaluated as pure diastereomers and pure enantiomers.

### Step 1A: Route B-1 (Bromoketone Synthesis)

Route B-1 followed the general reaction scheme, below:

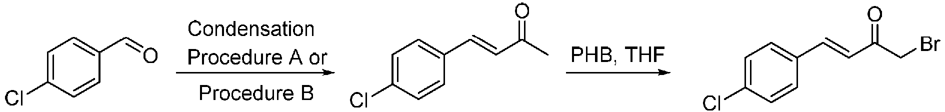

### Condensation Procedure A

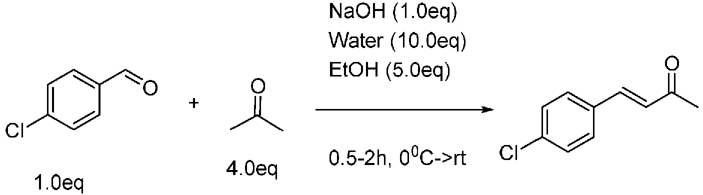

Solid NaOH (0.356g, 8.89mmol, 1.0eq) was dissolved in the mixture of acetone (2.63mL, 35.57mmol, 4.0eq), water (12.5mL) and EtOH (6.3mL). Then the 4-chlorobenzaldehyde (1.250g, 8.89mmol, 1.0eq) was added dropwise within 20 min at 0 °C. The reaction mixture was allowed to warm to room temperature and was stirred until TLC analysis indicated completion of the reaction (0.5-2 h). The reaction mixture was quenched with aqueous HCl (1 N), adjusted the pH to 6 and evaporated to remove the residual EtOH and acetone. The residue was extracted by EtOAc (3 × 40 mL). The combined organic phases were washed with brine (50 mL), dried over anhydrous Na₂SO₄ and concentrated under reduced pressure. Crude product was purified by column chromatography on silica gel.

The following compounds were prepared via a similar method:

| **Intermediate for** | **Compound name** |
|---|---|
| **A161** | 4-(2-chlorophenyl)but-3-en-2-one |
| **A159** | 4-(3-methoxyphenyl)but-3-en-2-one |
| **A160** | 4-(4-methoxyphenyl)but-3-en-2-one |
| **A152** | 4-cyclohexylbut-3-en-2-one |
| **A219** | 4-(4-(trifluoromethyl)phenyl)but-3-en-2-one |
| **A220** | 4-(4-fluorophenyl)but-3-en-2-one |
| **A224 and A231** | 4-(3-oxobut-1-en-1-yl)benzonitrile |
| **A227** | 4-(4-nitrophenyl)but-3-en-2-one |
| **A251** | 5-phenylhex-3-en-2-one |
| **A252** | 4-phenylpent-3-en-2-one |
| **A297** | 1-phenylpent-1-en-3-one |
| **A209** | 4-(3-chlorophenyl)but-3-en-2-one |
| **A163** | 4-(4-chlorophenyl)but-3-en-2-one |
| **A223** | N-(4-(3-oxobut-1-en-1-yl)phenyl)acetamide |

### Condensation Procedure B

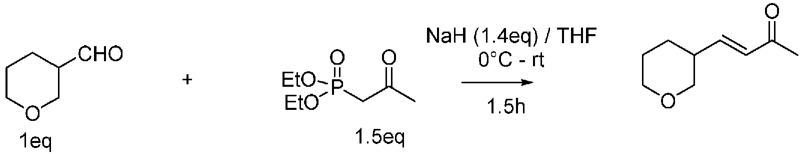

Diethyl (2-oxopropyl)-phosphonate (1.34 mL, 6.97 mmol, 1.5 equiv) was added to a slurry of NaH (250 mg of a 60 % mineral oil suspension, 6.51 mmol, 1.4 equiv) in THF (10.3 mL) at 0°C, and stirred for 1 hr. Aldehyde (0.5ml, 4.65 mmol, 1.0 equiv) was added dropwise, the cooling bath was removed and the resulting solution stirred for an additional 1.5 hr at room temperature. H₂O (10 mL) was added and the layers were separated. The aqueous phase was extracted with Et₂O (3 × 10 mL), and the combined organic extracts were dried (MgSO₄) and concentrated under reduced pressure. The crude residue was purified by flash chromatography.

The following compounds were prepared via a similar method:

| **Intermediate for** | **Compound name** |
|---|---|
| **A158** | 4-(2-methoxyphenyl)but-3-en-2-one |
| **A153** | 4-(tetrahydro-2H-pyran-4-yl)but-3-en-2-one |
| **A166** | 4-(pyridin-4-yl)but-3-en-2-one |
| **A165** | 4-(pyridin-3-yl)but-3-en-2-one |
| **A164** | 4-(pyridin-2-yl)but-3-en-2-one |

### Bromination

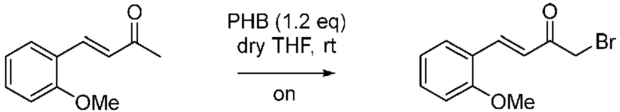

Ketone (730mg, 4.15 mmol, 1.0 equiv) was dissolved in dry THF (10ml) followed by slow (1h) dropwise addition of pyrrolidone hydrotribromide (2468 mg, 4.98 mmol, 1.2 equiv) in THF (15 ml). Reaction was stirred overnight at rt, the solid residue was filtered off and filtrate was evaporated. The oily residue was dissolved in Et₂O (50ml), washed with saturated NaHCO₃ (20ml), water (20ml) and brine (20ml). Organic layer was separated, dried by MgSO₄ and evaporated to yield crude product which was further purified by flash chromatography on silica gel.

The following compounds were prepared via a similar method:

| **Intermediate for** | **Compound Name** |
|---|---|
| **A161** | 1-bromo-4-(2-chlorophenyl)but-3-en-2-one |
| **A159** | 1-bromo-4-(3-methoxyphenyl)but-3-en-2-one |
| **A160** | 1-bromo-4-(4-methoxyphenyl)but-3-en-2-one |
| **A154** | 1-bromo-4-(tetrahydro-2H-pyran-3-yl)but-3-en-2-one |
| **A158** | 1-bromo-4-(2-methoxyphenyl)but-3-en-2-one |
| **A152** | 1-bromo-4-cyclohexylbut-3-en-2-one |
| **A153** | 1-bromo-4-(tetrahydro-2H-pyran-4-yl)but-3-en-2-one |
| **A219** | 1-bromo-4-(4-(trifluoromethyl)phenyl)but-3-en-2-one |
| **A220** | 1-bromo-4-(4-fluorophenyl)but-3-en-2-one |
| **A166** | 1-bromo-4-(pyridin-4-yl)but-3-en-2-one |
| **A224** | 4-(4-bromo-3-oxobut-1-en-1-yl)benzonitrile |
| **A227** | 1-bromo-4-(4-nitrophenyl)but-3-en-2-one |
| **A165** | 1-bromo-4-(pyridin-3-yl)but-3-en-2-one |
| **A164** | 1-bromo-4-(pyridin-2-yl)but-3-en-2-one |
| **A251** | 1-bromo-5-phenylhex-3-en-2-one |
| **A252** | 1-bromo-4-phenylpent-3-en-2-one |
| **A297** | 4-bromo-1-phenylpent-1-en-3-one |
| **A209** | 1-bromo-4-(3-chlorophenyl)but-3-en-2-one |
| **A163** | 1-bromo-4-(4-chlorophenyl)but-3-en-2-one |
| **A225** | 1-bromo-3,3-dimethyl-5-phenylpentan-2-one |
| **A228** | 1-bromo-3,3-dimethyl-4-phenoxybutan-2-one |
| **A257** | 1-bromo-3-methyl-3-(pyridin-2-ylmethoxy)butan-2-one |
| **A260** | 1-bromo-3,3-dimethyl-4-(pyridin-2-yloxy)butan-2-one |
| **A215 and A194** | 2-bromo-1-((1S,2S)-2-phenylcyclopropyl)ethan-1-one |
| **A223** | N-(4-(4-bromo-3-oxobut-1-en-1-yl)phenyl)acetamide |

### Step 1B: Route B-2 (Chloroketone Synthesis)

Route B-2 followed the general reaction scheme, below:

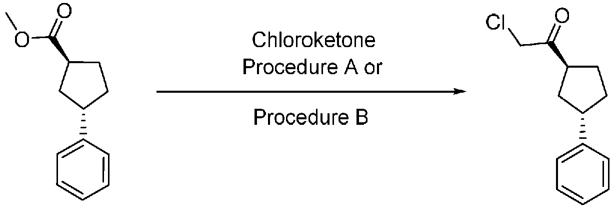

### Chloroketone Procedure A

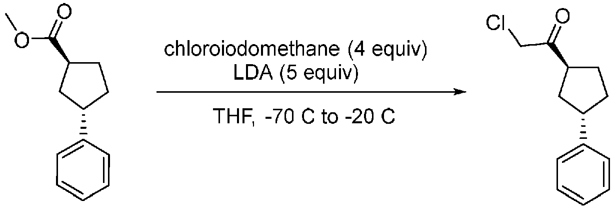

A roundbottom flask with stir bar was charged with starting material (0.50 g, 2.4 5mmol, 1.0 equiv) dissolved in THF (6.1 mL) under Ar atmosphere. Then to reaction mixture was added chloroiodomethane (0.71 mL, 9.79 mmol, 4.0 equiv). When the flask was cooled to -72°C, solution of LDA (12.24 mL, 12.24 mmol, 5.0 equiv, 1M in THF) was added dropwise maintaining the temperature below -68°C. Upon completion of the addition, the solution was stirred at -72°C for 15 min. An acetic acid solution (1.4 mL in 1.4 mL of THF) was added dropwise at a rate sufficient to keep the internal temperature below -60°C and the reaction mixture was stirred for 1 hour while flask was slowly allowed to warm. Then toluene (6 mL) was added and when temperature reached -20°C, a portion of water (15 mL) was slowly added and the solution was stirred for 20 minutes. Layers were separated and the organic layer was washed with NaHCO₃ (2 × 100 ml), dried over MgSO4, filtered and concentrated. The crude product was purified by flash chromatography on silica gel using Hex/EtOAc as eluent.

The following compounds were prepared in a similar way:

| **Intermediate for** | **Compound Name** |
|---|---|
| **A263** | 1-(1-(benzyloxy)cyclopropyl)-2-chloroethan-1-one |
| **A292** | 2-chloro-1-((1R,3R)-3-phenylcyclopentyl)ethan-1-one |
| **A293** | 1-chloro-3,3-dimethyl-5-phenylpent-4-en-2-one |
| **A305** | 2-chloro-1-((1S,3R)-3-phenylcyclopentyl)ethan-1-one |
| **A310** | 2-chloro-1-(5-phenyltetrahydrofuran-2-yl)ethan-1-one |
| **A306** | 2-chloro-1-(1-phenylpiperidin-3-yl)ethan-1-one |
| **A338** | (R)-2-chloro-1-(2-methyl-1-phenylpyrrolidin-2-yl)ethan-1-one |

### Chloroketone Procedure B

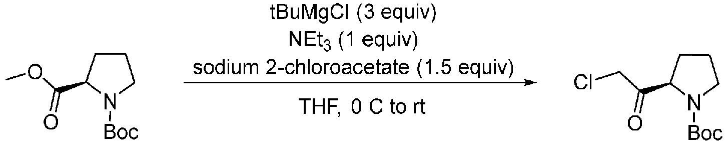

A 20 mL vial with stir bar was charged with methyl ester (182 mg, 0.887 mmol, 1.0 equiv), sodium 2-chloroacetate (155 mg, 1.33 mmol, 1.5 equiv), triethylamine (0.124 mL, 0.887 mmol, 1.0 equiv) and THF (1.0 mL, 0.2 M). The reaction mixture was cooled to 0 C, and tert-butylmagnesium chloride (1.0 M in THF, 2.7 mL, 2.70 mmol, 3.0 equiv) was added. The reaction mixture was allowed to warm to room temperature overnight. The next morning, the reaction mixture was poured into EtOAc (50 mL). The organic layer was washed with saturated NaHCO₃ (2 × 50 mL). The combined aqueous layers were extracted with EtOAc (1 × 50 mL), and the combined organic layers were dried over Na₂SO₄, filtered and concentrated in vacuo. The resulting crude material was used in the next step without further purification.

The following compounds were prepared in a similar way:

| **Intermediate for** | **Compound Name** |
|---|---|
| **A309** | 2-chloro-1-((1s,3s)-3-((5-methoxypyridin-2-yl)oxy)cyclobutyl)ethan-1-one |
| **A314** | 2-chloro-1-((1r,3r)-3-((5-methoxypyridin-2-yl)oxy)cyclobutyl)ethan-1-one |
| **A316** | 2-chloro-1-(1-phenylazetidin-3-yl)ethan-1-one |
| **A329** | 2-chloro-1-((1r,3r)-3-phenoxycyclobutyl)ethan-1-one |
| **A330** | 2-chloro-1-(1s,3s)-3-phenoxycyclobutyl)ethan-1-one |
| **A300** | 2-chloro-1-((1S,2S)-2-phenylcyclopentyl)ethan-1-one |
| **A301** | 2-chloro-1-((1S,2R)-2-phenylcyclopentyl)ethan-1-one |
| **A268 and A287** | 2-chloro-1-(3-phenylcyclobutyl)ethan-1-one |
| **A281 and A284** | 2-chloro-1-(2,3-dihydro-1H-inden-2-yl)ethan-1-one |
| **A282 and A285** | 2-chloro-1-(1-phenylpiperidin-4-yl)ethan-1-one |
| **A283 and A286** | 2-chloro-1-(chroman-2-yl)ethan-1-one |
| **A302** | 1-(1-benzylpiperidin-4-yl)-2-chloroethan-1-one |
| **A318 and A275** | (R)-2-chloro-1-(1-phenylpyrrolidin-2-yl)ethan-1-one |
| **A328** | (R)-2-chloro-1-(1-(2,2-difluorobenzo[d][1,3]dioxol-5-yl)pyrrolidin-2-yl)ethan-1-one |
| **A359** | (R)-1-(1-(benzo[d][1,3]dioxol-5-yl)pyrrolidin-2-yl)-2-chloroethan-1-one |
| **A365** | (R)-2-chloro-1-(1-cyclohexylpyrrolidin-2-yl)ethan-1-one |
| **A262** | 1-chloro-3,3-dimethyl-5-(pyridin-2-yl)pentan-2-one |
| **A270** | 2-chloro-1-((1S,3R)-3-phenylcyclohexyl)ethan-1-one |
| **A271** | 2-chloro-1-((1R,3R)-3-phenylcyclohexyl)ethan-1-one |
| **A276** | (S)-2-chloro-1-(1-phenylpyrrolidin-2-yl)ethan-1-one |
| **A277** | (R)-1-(1-benzylpyrrolidin-2-yl)-2-chloroethan-1-one |
| **A278** | (S)-1-(1-benzylpyrrolidin-2-yl)-2-chloroethan-1-one |
| **A279** | (S)-1-(1-benzylpyrrolidin-3-yl)-2-chloroethan-1-one |
| **A288** | 2-chloro-1-(2,3-dihydrobenzofuran-2-yl)ethan-1-one |
| **A289** | (S)-2-chloro-1-(1-phenylpyrrolidin-3-yl)ethan-1-one |
| **A290** | (R)-2-chloro-1-(1-phenylpyrrolidin-3-yl)ethan-1-one |
| **A291** | (R)-1-(1-benzylpyrrolidin-3-yl)-2-chloroethan-1-one |
| **A200** | 2-chloro-1-((1S,2R)-2-phenylcyclopropyl)ethan-1-one |
| **A296** | 2-chloro-1-(chroman-3-yl)ethan-1-one |
| **A298** | 2-chloro-1-((1S,2R)-2-phenylcyclohexyl)ethan-1-one |
| **A303** | 2-chloro-1-((1S,2S)-2-phenylcyclohexyl)ethan-1-one |
| **A313** | (R)-1-(1-benzoylpyrrolidin-2-yl)-2-chloroethan-1-one |
| **A315** | 2-chloro-1-(1-(5-methoxypyridin-2-yl)azetidin-3-yl)ethan-1-one |
| **A319** | (R)-5-(2-chloroacetyl)-1-phenylpyrrolidin-2-one |
| **A320** | (R)-1-benzyl-5-(2-chloroacetyl)pyrrolidin-2-one |
| **A322 and A321** | 1-((2R,4R)-4-((tert-butyldiphenylsilyl)oxy)-1-phenylpyrrolidin-2-yl)-2-chloroethan-1-one |
| **A323 and A326** | 1-((2R,4S)-4-((tert-butyldiphenylsilyl)oxy)-1-phenylpyrrolidin-2-yl)-2-chloroethan-1-one |
| **A324** | 2-chloro-1-((1S,2R)-2-phenylcyclobutyl)ethan-1-one |
| **A325** | 2-chloro-1-((2R,4R)-4-methoxy-1-phenylpyrrolidin-2-yl)ethan-1-one |
| **A331** | 2-chloro-1-((1S,2S)-2-phenylcyclobutyl)ethan-1-one |
| **A332** | (R)-2-chloro-1-(1-phenylpiperidin-2-yl)ethan-1-one |
| **A333** | 2-chloro-1-((2R,4S)-4-methoxy-1-phenylpyrrolidin-2-yl)ethan-1-one |
| **A334** | 2-chloro-1-((2R,3S)-3-methoxy-1-phenylpyrrolidin-2-yl)ethan-1-one |
| **A336** | (R)-2-chloro-1-(1-phenylazetidin-2-yl)ethan-1-one |
| **A337** | (R)-2-chloro-1-(4-phenylmorpholin-3-yl)ethan-1-one |
| **A339** | (R)-2-chloro-1-(4,4-difluoro-1-phenylpyrrolidin-2-yl)ethan-1-one |
| **A344 and A352** | 2-chloro-1-((2R,3S)-3-hydroxy-1-phenylpyrrolidin-2-yl)ethan-1-one |
| **A345** | 2-chloro-1-((2R,3R)-3-methoxy-1-phenylpyrrolidin-2-yl)ethan-1-one |
| **A346** | 2-chloro-1-((2R,4R)-4-isopropoxy-1-phenylpyrrolidin-2-yl)ethan-1-one |
| **A349** | 2-chloro-1-((1s,3s)-3-(pyridin-2-yl)cyclobutyl)ethan-1-one |
| **A350** | 2-chloro-1-((1r,3r)-3-(pyridin-2-yl)cyclobutyl)ethan-1-one |
| **A351 and A353** | 2-chloro-1-((2R,3R)-3-hydroxy-1-phenylpyrrolidin-2-yl)ethan-1-one |
| **A354** | 2-chloro-1-((2R,4R)-4-(cyclohexyloxy)-1-phenylpyrrolidin-2-yl)ethan-1-one |
| **A361** | (R)-2-chloro-1-(1-(2,2-difluorobenzo[d][1,3]dioxol-4-yl)pyrrolidin-2-yl)ethan-1-one |
| **A362** | (R)-1-(1-(benzo[d][1,3]dioxol-4-yl)pyrrolidin-2-yl)-2-chloroethan-1-one |
| **A363** | 2-chloro-1-((1r,3r)-3-fluoro-3-(pyridin-2-yl)cyclobutyl)ethan-1-one |
| **A364** | 2-chloro-1-((1s,3s)-3-fluoro-3-(pyridin-2-yl)cyclobutyl)ethan-1-one |

### Step 2: Standard Condensation

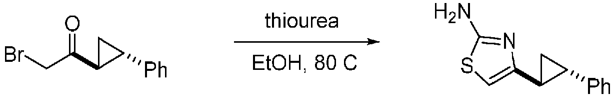

A 20 mL vial with stir bar was charged with bromoketone (307 mg, 1.28 mmol, 1.0 equiv), thiourea (108 mg, 1.41 mmol, 1.1 equiv) and EtOH (7 mL). The resulting solution was stirred at 80 C overnight. The next morning, the resulting solution was cooled and poured into EtOAc (100 mL). The mixture was washed with saturated NaHCO₃ (2 × 100 mL), and the combined aqueous layers were extracted with EtOAc (1 × 100 mL). The combined organic layers were dried over Na₂SO₄, filtered and concentrated in vacuo. The resulting crude material was purified via silica gel chromatography to yield the desired product.

The following compounds were prepared via a similar method:

| **Intermediate for** | **Compound Name** |
|---|---|
| **A161** | 4-(2-chlorostyryl)thiazol-2-amine |
| **A159** | 4-(3-methoxystyryl)thiazol-2-amine |
| **A160** | 4-(4-methoxystyryl)thiazol-2-amine |
| **A154** | 4-(2-(tetrahydro-2H-pyran-3-yl)vinyl)thiazol-2-amine |
| **A158** | 4-(2-methoxystyryl)thiazol-2-amine |
| **A152** | 4-(2-cyclohexylvinyl)thiazol-2-amine |
| **A153** | 4-(2-(tetrahydro-2H-pyran-4-yl)vinyl)thiazol-2-amine |
| **A219** | 4-(4-(trifluoromethyl)styryl)thiazol-2-amine |
| **A220** | 4-(4-fluorostyryl)thiazol-2-amine |
| **A166** | 4-(2-(pyridin-4-yl)vinyl)thiazol-2-amine |
| **A224** | 4-(2-(2-aminothiazol-4-yl)vinyl)benzonitrile |
| **A227** | 4-(4-nitrostyryl)thiazol-2-amine |
| **A165** | 4-(2-(pyridin-3-yl)vinyl)thiazol-2-amine |
| **A164** | 4-(2-(pyridin-2-yl)vinyl)thiazol-2-amine |
| **A251** | 4-(3-phenylbut-1-en-1-yl)thiazol-2-amine |
| **A252** | 4-(2-phenylprop-1-en-1-yl)thiazol-2-amine |
| **A297** | 5-methyl-4-styrylthiazol-2-amine |
| **A209** | 4-(3-chlorostyryl)thiazol-2-amine |
| **A163** | 4-(4-chlorostyryl)thiazol-2-amine |
| **A225** | 4-(2-methyl-4-phenylbutan-2-yl)thiazol-2-amine |
| **A228** | 4-(2-methyl-1-phenoxypropan-2-yl)thiazol-2-amine |
| **A257** | 4-(2-(pyridin-2-ylmethoxy)propan-2-yl)thiazol-2-amine |
| **A260** | 4-(2-methyl-1-(pyridin-2-yloxy)propan-2-yl)thiazol-2-amine |
| **A215 and A194** | 4-((1R,2R)-2-phenylcyclopropyl)thiazol-2-amine |
| **A263** | 4-(1-(benzyloxy)cyclopropyl)thiazol-2-amine |
| **A292** | 4-((1R,3R)-3-phenylcyclopentyl)thiazol-2-amine |
| **A293** | 4-(2-methyl-4-phenylbut-3-en-2-yl)thiazol-2-amine |
| **A305** | 4-((1S,3R)-3-phenylcyclopentyl)thiazol-2-amine |
| **A309** | 4-((1s,3s)-3-((5-methoxypyridin-2-yl)oxy)cyclobutyl)thiazol-2-amine |
| **A314** | 4-((1r,3r)-3-((5-methoxypyridin-2-yl)oxy)cyclobutyl)thiazol-2-amine |
| **A316** | 4-(1-phenylazetidin-3-yl)thiazol-2-amine |
| **A329** | 4-((1r,3r)-3-phenoxycyclobutyl)thiazol-2-amine |
| **A330** | 4-((1s,3s)-3-phenoxycyclobutyl)thiazol-2-amine |
| **A300** | 4-((1S,2S)-2-phenylcyclopentyl)thiazol-2-amine |
| **A301** | 4-((1S,2R)-2-phenylcyclopentyl)thiazol-2-amine |
| **A310** | 4-(5-phenyltetrahydrofuran-2-yl)thiazol-2-amine |
| **A268 and A287** | 4-(3-phenylcyclobutyl)thiazol-2-amine |
| **A281 and A284** | 4-(2,3-dihydro-1H-inden-2-yl)thiazol-2-amine |
| **A282 and A285** | 4-(1-phenylpiperidin-4-yl)thiazol-2-amine |
| **A283 and A286** | 4-(chroman-2-yl)thiazol-2-amine |
| **A302** | 4-(1-benzylpiperidin-4-yl)thiazol-2-amine |
| **A306** | 4-(1-phenylpiperidin-3-yl)thiazol-2-amine |
| **A318 and A275** | (R)-4-(1-phenylpyrrolidin-2-yl)thiazol-2-amine |
| **A328** | (R)-4-(1-(2,2-difluorobenzo[d][1,3]dioxol-5-yl)pyrrolidin-2-yl)thiazol-2-amine |
| **A359** | (R)-4-(1-(benzo[d][1,3]dioxol-5-yl)pyrrolidin-2-yl)thiazol-2-amine |
| **A365** | (R)-4-(1-cyclohexylpyrrolidin-2-yl)thiazol-2-amine |
| **A262** | 4-(2-methyl-4-(pyridin-2-yl)butan-2-yl)thiazol-2-amine |
| **A270** | 4-((1S,3R)-3-phenylcyclohexyl)thiazol-2-amine |
| **A271** | 4-((1R,3R)-3-phenylcyclohexyl)thiazol-2-amine |
| **A276** | (S 4-(1-phenylpyrrolidin-2-yl)thiazol-2-amine |
| **A277** | (R)-4-(1-benzylpyrrolidin-2-yl)thiazol-2-amine |
| **A278** | (S-4-(1-benzylpyrrolidin-2-yl)thiazol-2-amine |
| **A279** | (S)-4-(1-benzylpyrrolidin-3-yl)thiazol-2-amine |
| **A288** | 4-(2,3-dihydrobenzofuran-2-yl)thiazol-2-amine |
| **A289** | (S)-4-(1-phenylpyrrolidin-3-yl)thiazol-2-amine |
| **A290** | (R)-4-(1-phenylpyrrolidin-3-yl)thiazol-2-amine |
| **A291** | (R)-4-(1-benzylpyrrolidin-3-yl)thiazol-2-amine |
| **A200** | 4-((1S,2R)-2-phenylcyclopropyl)thiazol-2-amine |
| **A296** | 4-(chroman-3-yl)thiazol-2-amine |
| **A298** | 4-((1S,2R)-2-phenylcyclohexyl)thiazol-2-amine |
| **A303** | 4-((1S,2S)-2-phenylcyclohexyl)thiazol-2-amine |
| **A313** | (R)-(2-(2-aminothiazol-4-yl)pyrrolidin-1-yl)(phenyl)methanone |
| **A315** | 4-(1-(5-methoxypyridin-2-yl)azetidin-3-yl)thiazol-2-amine |
| **A319** | (R)-5-(2-aminothiazol-4-yl)-1-phenylpyrrolidin-2-one |
| **A320** | (R)-5-(2-aminothiazol-4-yl)-1-benzylpyrrolidin-2-one |
| **A322 and A321** | 4-((2R,4R)-4-((tert-butyldiphenylsilyl)oxy)-1-phenylpyrrolidin-2-yl)thiazol-2-amine |
| **A323 and A326** | 4-((2R,4S)-4-((tert-butyldiphenylsilyl)oxy)-1-phenylpyrrolidin-2-yl)thiazol-2-amine |
| **A324** | 4-((1S,2R)-2-phenylcyclobutyl)thiazol-2-amine |
| **A325** | 4-((2R,4R)-4-methoxy-1-phenylpyrrolidin-2-yl)thiazol-2-amine |
| **A331** | 4-((1S,2S)-2-phenylcyclobutyl)thiazol-2-amine |
| **A332** | (R)-4-(1-phenylpiperidin-2-yl)thiazol-2-amine |
| **A333** | 4-((2R,4S)-4-methoxy-1-phenylpyrrolidin-2-yl)thiazol-2-amine |
| **A334** | 4-((2S,3S)-3-methoxy-1-phenylpyrrolidin-2-yl)thiazol-2-amine |
| **A336** | (R)-4-(1-phenylazetidin-2-yl)thiazol-2-amine |
| **A337** | (S)-4-(4-phenylmorpholin-3-yl)thiazol-2-amine |
| **A338** | (R)-4-(2-methyl-1-phenylpyrrolidin-2-yl)thiazol-2-amine |
| **A339** | (R)-4-(4,4-difluoro-1-phenylpyrrolidin-2-yl)thiazol-2-amine |
| **A344 and A352** | (2S,3S)-2-(2-aminothiazol-4-yl)-1-phenylpyrrolidin-3-ol |
| **A345** | 4-((2S,3R)-3-methoxy-1-phenylpyrrolidin-2-yl)thiazol-2-amine |
| **A346** | 4-((2R,4R)-4-isopropoxy-1-phenylpyrrolidin-2-yl)thiazol-2-amine |
| **A349** | 4-((1s,3s)-3-(pyridin-2-yl)cyclobutyl)thiazol-2-amine |
| **A350** | 4-((1r,3r)-3-(pyridin-2-yl)cyclobutyl)thiazol-2-amine |
| **A351 and A353** | (2S,3R)-2-(2-aminothiazol-4-yl)-1-phenylpyrrolidin-3-ol |
| **A354** | 4-((2R,4R)-4-(cyclohexyloxy)-1-phenylpyrrolidin-2-yl)thiazol-2-amine |
| **A361** | (R)-4-(1-(2,2-difluorobenzo[d][1,3]dioxol-4-yl)pyrrolidin-2-yl)thiazol-2-amine |
| **A362** | (R)-4-(1-(benzo[d][1,3]dioxol-4-yl)pyrrolidin-2-yl)thiazol-2-amine |
| **A363** | 4-((1r,3r)-3-fluoro-3-(pyridin-2-yl)cyclobutyl)thiazol-2-amine |
| **A364** | 4-((1s,3s)-3-fluoro-3-(pyridin-2-yl)cyclobutyl)thiazol-2-amine |
| **A223** | N-(4-(2-(2-aminothiazol-4-yl)vinyl)phenyl)acetamide |

### Procedure 13 - Intermediate for A267 (Route C)

The reactions followed the general reaction scheme, below:

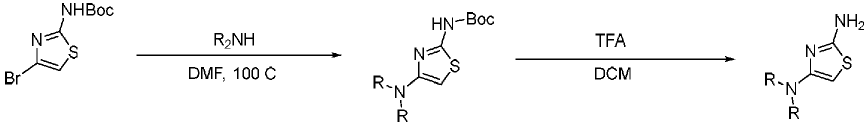

### Step 1: Amine Installation

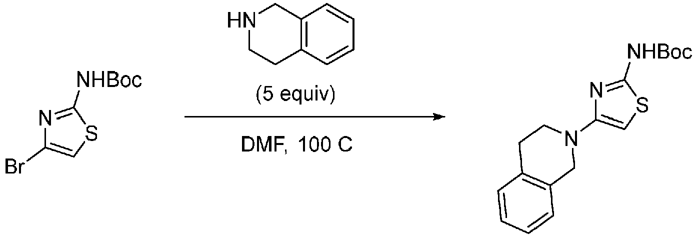

A 50 mL vial with stir bar was charged with thiazole (300 mg, 1.08 mmol, 1.0 equiv), amine (869 mg, 5.39 mmol, 5.0 equiv) and DMF (9.0 mL, 0.12 M) under nitrogen atmosphere, the vial was capped and placed in an 100°C bath. The reaction mixture was stirred at 100°C for 4 h. The reaction mixture was cooled to room temperature and quenched by H₂O (50 mL). The resulting solution was extracted with ethyl acetate (3 × 40 mL) and washed with brine (3 × 40 mL). The organic layer was then dried over Na₂SO₄, filtered and concentrated in vacuo. The resulting crude material was purified via prep-TLC to yield the desired product.

The following compounds were prepared via a similar method:

| **Intermediate for** | **Compound name** |
|---|---|
| **A261** | tert-butyl (4-(4-phenylpiperidin-1-yl)thiazol-2-yl)carbamate |
| **A264** | tert-butyl (4-(piperidin-1-yl)thiazol-2-yl)carbamate |
| **A265** | tert-butyl (4-(3-phenylpiperidin-1-yl)thiazol-2-yl)carbamate |
| **A266** | tert-butyl (4-(4-phenylpiperazin-1-yl)thiazol-2-yl)carbamate |
| **A273** | tert-butyl (4-(3-phenylpyrrolidin-1-yl)thiazol-2-yl)carbamate |

### Step 2: Deprotection

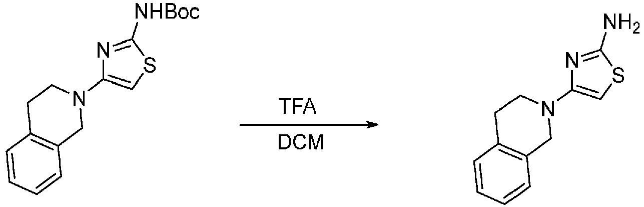

A 20 mL vial with stir bar was charged with thiazole (90 mg, 0.25 mmol, 1.00 equiv) and DCM (2.0 mL, 0.125 M). TFA (2 mL, 27.0 mmol, 108 equiv) was added, and the vial was capped and placed in a 25°C bath. The reaction mixture was stirred at 25°C for 1 h. The resulting mixture was concentrated under vacuum and quenched by sat. NaHCO₃ (aq) (10 mL). The combined aqueous layers were extracted with EtOAc (4 × 20 mL), the combined organic layers were dried over Na₂SO₄, filtered and concentrated in vacuo. The resulting crude material was used directly for next step.

The following compounds were prepared via a similar method:

| **Intermediate for** | **Compound Name** |
|---|---|
| **A261** | 4-(4-phenylpiperidin-1-yl)thiazol-2-amine |
| **A264** | 4-(piperidin-1-yl)thiazol-2-amine |
| **A265** | 4-(3-phenylpiperidin-1-yl)thiazol-2-amine |
| **A266** | 4-(4-phenylpiperazin-1-yl)thiazol-2-amine |
| **A273** | 4-(3-phenylpyrrolidin-1-yl)thiazol-2-amine |

### Example 4: Amide Coupling Reactions

### Procedure 1 - Synthesis of Compounds A74 and A49

Synthesis of A74 and A49 followed the scheme, below.

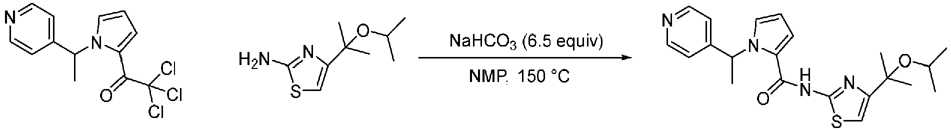

N-(2-(4-pyridinylethyl)-2-(trichloroacetyl)pyrrole (0.53 g, 1.67 mmol, 1.0 eq), amine (0.334 g, 1.67 mmol, 1.0 eq) and NaHCO₃ (0.911 g, 10.9 mmol, 6. 5eq) were taken up in NMP (6.5 mL). The reaction mixture was heated at 150 °C for 17h. After this time, the NMP was evaporated, and the resulting crude material was purified on pTLC (60% EtOAc in hexanes). LCMS (+ESI): calc. [M+H]⁺= 396; found 397. The enantiomers were resolved via SFC (Chiralpak IF 3, 5% MeOH+TEA in CO₂).

### Procedure 2 - Synthesis of Compound A71

Synthesis of A71 followed the scheme, below

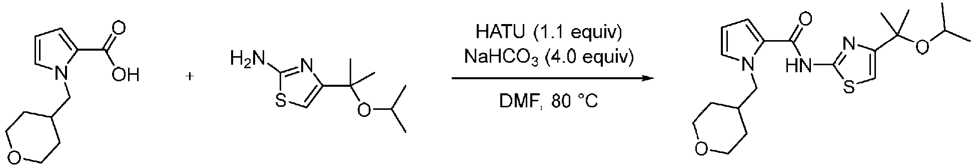

A 20 mL vial with stir bar was charged with acid (71.3 mg, 0.341 mmol, 1.0 equiv), amine (115 mg, 0.574 mmol, 1.7 equiv), sodium bicarbonate (115 mg, 1.36 mmol, 4.0 equiv) and HATU (143 mg, 0.375 mmol, 1.1 equiv). DMF (2 mL) was added, and the vial was capped and placed in an 80 ⁰C bath. The reaction mixture was stirred at 80 ⁰C overnight. The next morning, the reaction mixture was poured into DCM (50 mL) and washed with 0.5 M NaOH (2 × 50 mL), followed by water (1 × 50 mL). The organic layer was then dried over Na₂SO₄, filtered and concentrated in vacuo. The resulting crude material was purified via silica gel chromatography (20-50% EtOAc in hexanes) to yield the desired product. LCMS (+ESI): calc. [M+H]⁺= 392; found 392.

The following compounds were prepared in a similar manner:

| **Compound** | **Compound name** | **Exact mass [M]** | **Observed molecular ion [M+H]** |
|---|---|---|---|
| **A1** | (S)-N-(4-(2-(1-phenylethoxy)propan-2-yl)thiazol-2-yl)-1-(pyridin-4-ylmethyl)-1H-pyrrole-2-carboxamide | 446 | 447 |
| **A16** | N-(4-(2-isopropoxypropan-2-yl)thiazol-2-yl)-1-(pyridin-4-ylmethyl)-1H-pyrrole-2-carboxamide | 384 | 385 |
| **A2** | N-(4-(2-(1-(3-chlorophenyl)ethoxy)propan-2-yl)thiazol-2-yl)-1-(pyridin-4-ylmethyl)-1H-pyrrole-2-carboxamide | 480 | 481 |
| **A3** | N-(4-(2-(1-phenylethoxy)propan-2-yl)thiazol-2-yl)-1-(pyridin-4-ylmethyl)-1H-pyrrole-2-carboxamide | 446 | 447 |
| **A4** | N-(4-(2-(1-(3-methoxyphenyl)ethoxy)propan-2-yl)thiazol-2-yl)-1-(pyridin-4-ylmethyl)-1H-pyrrole-2-carboxamide | 476 | 477 |
| **A5** | N-(4-(2-(1-(4-chlorophenyl)ethoxy)propan-2-yl)thiazol-2-yl)-1-(pyridin-4-ylmethyl)-1H-pyrrole-2-carboxamide | 480 | 481 |
| **A6** | (S)-N-(4-(2-(1-phenylethoxy)propan-2-yl)thiazol-2-yl)-1-(3-(pyridin-4-yl)propyl)-1H-pyrrole-2-carboxamide | 474 | 475 |
| **A7** | N-(4-(2-(cyclohexylmethoxy)propan-2-yl)thiazol-2-yl)-1-(pyridin-4-ylmethyl)-1 H-pyrrole-2-carboxamide | 438 | 439 |
| **A8** | N-(4-(2-(1-(2-chlorophenyl)ethoxy)propan-2-yl)thiazol-2-yl)-1-(pyridin-4-ylmethyl)-1H-pyrrole-2-carboxamide | 480 | 481 |
| **A9** | N-(4-(2-(benzyloxy)propan-2-yl)thiazol-2-yl)-1-(pyridin-4-ylmethyl)-1H-pyrrole-2-carboxamide | 432 | 433 |
| **A10** | N-(4-(2-(cyclohexyloxy)propan-2-yl)thiazol-2-yl)-1-(pyridin-4-ylmethyl)-1H-pyrrole-2-carboxamide | 424 | 425 |
| **A11** | N-(4-(2-isopropoxypropan-2-yl)thiazol-2-yl)-1-(3-(pyridin-4-yl)propyl)-1H-pyrrole-2-carboxamide | 412 | 413 |
| **A12** | 1-(pyridin-4-ylmethyl)-N-(4-styrylthiazol-2-yl)-1H-pyrrole-2-carboxamide | 386 | 387 |
| **A13** | N-(4-phenethylthiazol-2-yl)-1-(pyridin-4-ylmethyl)-1H-pyrrole-2-carboxamide | 388 | 389 |
| **A14** | 1-(pyridin-4-ylmethyl)-N-(4-styrylthiazol-2-yl)-1H-pyrrole-2-carboxamide | 386 | 387 |
| **A15** | 1-(pyridin-4-ylmethyl)-N-(4-(2-(2,2,2-trifluoroethoxy)propan-2-yl)thiazol-2-yl)-1H-pyrrole-2-carboxamide | 424 | 425 |
| **A18** | (R)-N-(4-(2-(1-phenylethoxy)propan-2-yl)thiazol-2-yl)-1-(pyridin-4-ylmethyl)-1H-pyrrole-2-carboxamide | 446 | 447 |
| **A21** | N-(4-(1-phenylethyl)thiazol-2-yl)-1-(pyridin-4-ylmethyl)-1H-pyrrole-2-carboxamide | 388 | 389 |
| **A22** | N-(4,4-dimethyl-5,6-dihydro-4H-cyclopenta[d]thiazol-2-yl)-1-(pyridin-4-ylmethyl)-1H-pyrrole-2-carboxamide | 352 | 353 |
| **A25** | 1-((3-fluoropyridin-4-yl)methyl)-N-(4-(2-isopropoxypropan-2-yl)thiazol-2-yl)-1H-pyrrole-2-carboxamide | 402 | 403 |
| **A26** | N-(4-isopropylthiazol-2-yl)-1-(pyridin-4-ylmethyl)-1H-pyrrole-2-carboxamide | 326 | 327 |
| **A27** | N-(4-(2-ethoxypropan-2-yl)thiazol-2-yl)-1-(pyridin-4-ylmethyl)-1H-pyrrole-2-carboxamide | 370 | 371 |
| **A32** | N-(4-ethyl-5-methylthiazol-2-yl)-1-(pyridin-4-ylmethyl)-1 H-pyrrole-2-carboxamide | 326 | 327 |
| **A33** | 1-(3-(pyridin-4-yl)propyl)-N-(4-styrylthiazol-2-yl)-1 H-pyrrole-2-carboxamide | 414 | 415 |
| **A34** | N-(5-benzylthiazol-2-yl)-1-(pyridin-4-ylmethyl)-1H-pyrrole-2-carboxamide | 374 | 375 |
| **A37** | N-(4-(2-isopropoxypropan-2-yl)-5-methylthiazol-2-yl)-1-(pyridin-4-ylmethyl)-1H-pyrrole-2-carboxamide | 398 | 399 |
| **A39** | 1-(pyridin-4-ylmethyl)-N-(4-(2-((tetrahydro-2H-pyran-2-yl)methoxy)propan-2-yl)thiazol-2-yl)-1H-pyrrole-2-carboxamide | 440 | 441 |
| **A40** | 1-(pyridin-4-ylmethyl)-N-(4-(2-((tetrahydro-2H-pyran-4-yl)methoxy)propan-2-yl)thiazol-2-yl)-1H-pyrrole-2-carboxamide | 440 | 441 |
| **A41** | 1-((3-bromopyridin-4-yl)methyl)-N-(4-(2-isopropoxypropan-2-yl)thiazol-2-yl)-1H-pyrrole-2-carboxamide | 462 | 463 |
| **A44** | N-(4-(2-isopropoxypropan-2-yl)thiazol-2-yl)-1-(4-(pyridin-4-yl)butyl)-1H-pyrrole-2-carboxamide | 426 | 427 |
| **A45** | N-(4-(tert-butyl)thiazol-2-yl)-1-(pyridin-4-ylmethyl)-1H-pyrrole-2-carboxamide | 340 | 341 |
| **A50** | N-(benzo[d]thiazol-2-yl)-1-(pyridin-4-ylmethyl)-1H-pyrrole-2-carboxamide | 334 | 335 |
| **A58** | N-(4,5-dimethylthiazol-2-yl)-1-(pyridin-4-ylmethyl)-1H-pyrrole-2-carboxamide | 312 | 313 |
| **A59** | N-(4-(isopropoxymethyl)thiazol-2-yl)-1-(pyridin-4-ylmethyl)-1H-pyrrole-2-carboxamide | 356 | 357 |
| **A64** | N-(4-(2-isopropoxypropan-2-yl)thiazol-2-yl)-1-(3-phenylpropyl)-1H-pyrrole-2-carboxamide | 411 | 412 |
| **A66** | 1-((3-chloropyridin-4-yl)methyl)-N-(4-ethyl-5-methylthiazol-2-yl)-1H-pyrrole-2-carboxamide | 360 | 361 |
| **A68** | 1-(3-(2-chlorophenyl)propyl)-N-(4-(2-isopropoxypropan-2-yl)thiazol-2-yl)-1H-pyrrole-2-carboxamide | 445 | 446 |
| **A69** | N-(4-(2-isopropoxypropan-2-yl)thiazol-2-yl)-1-(pyridazin-4-ylmethyl)-1H-pyrrole-2-carboxamide | 385 | 386 |
| **Ref. B3** | 1-(pyridin-4-ylmethyl)-N-(4-(2-(2-tosylethoxy)propan-2-yl)thiazol-2-yl)-1H-pyrrole-2-carboxamide | 524 | 525 |
| **Ref. B4** | N-(5-(tert-butyl)isoxazol-3-yl)-1-(pyridin-4-ylmethyl)-1H-pyrrole-2-carboxamide | 324 | 325 |
| A77 | N-(4-(tert-butyl)thiazol-2-yl)-1-((3-chloropyridin-4-yl)methyl)-1H-pyrrole-2-carboxamide | 374 | 375 |
| **A78** | 1-(2-chlorobenzyl)-N-(4-(2-isopropoxypropan-2-yl)thiazol-2-yl)-1H-pyrrole-2-carboxamide | 417 | 418 |
| **A79** | 1-benzyl-N-(4-(2-isopropoxypropan-2-yl)thiazol-2-yl)-1H-pyrrole-2-carboxamide | 383 | 384 |
| **Ref. B5** | N-(4-isopropyloxazol-2-yl)-1-(pyridin-4-ylmethyl)-1H-pyrrole-2-carboxamide | 310 | 311 |
| **A87** | N-(4-(2-isopropoxypropan-2-yl)thiazol-2-yl)-1-(pyridin-3-ylmethyl)-1H-pyrrole-2-carboxamide | 384 | 385 |
| **Ref. B7** | 1-(2-chlorobenzyl)-N-(4-ethyl-5-methylthiazol-2-yl)-1H-pyrrole-2-carboxamide | 359 | 360 |
| **Ref. B8** | N-(5-methylisoxazol-3-yl)-1-(pyridin-4-ylmethyl)-1H-pyrrole-2-carboxamide | 282 | 283 |
| **Ref. B9** | 1-benzyl-N-(4-ethyl-5-methylthiazol-2-yl)-1H-pyrrole-2-carboxamide | 325 | 326 |
| **A90** | N-(4-(2-isopropoxypropan-2-yl)thiazol-2-yl)-1-(quinolin-4-ylmethyl)-1H-pyrrole-2-carboxamide | 434 | 435 |
| **A91** | N-(4-(2-isopropoxypropan-2-yl)thiazol-2-yl)-1-(2-(pyridin-4-yl)ethyl)-1H-pyrrole-2-carboxamide | 398 | 399 |
| **Ref. B11** | N-(4-(tert-butyl)thiazol-2-yl)-1-(2-chlorobenzyl)-1H-pyrrole-2-carboxamide | 373 | 374 |
| **A100** | N-(4-(2-isopropoxypropan-2-yl)thiazol-2-yl)-1-(pyridin-4-ylmethyl)-1H-pyrazole-3-carboxamide | 385 | 386 |
| **A101** | 1-(3-(1-acetylpiperidin-4-yl)propyl)-N-(4-(2-isopropoxypropan-2-yl)thiazol-2-yl)-1H-pyrrole-2-carboxamide | 460 | 461 |
| **A106** | N-(4-(2-isopropoxypropan-2-yl)thiazol-2-yl)-6-oxo-1-(pyridin-4-ylmethyl)-1,6-dihydropyridine-2-carboxamide | 412 | 411 [M-H]- |
| **A112** | N-(4-(2-isopropoxypropan-2-yl)thiazol-2-yl)-1-(pyridin-4-ylmethyl)-1H-pyrazole-5-carboxamide | 385 | 386 |
| **A113** | 4-(hydroxymethyl)-N-(4-(2-isopropoxypropan-2-yl)thiazol-2-yl)-1-(pyridin-4-ylmethyl)-1H-pyrrole-2-carboxamide | 414 | 415 |
| **Ref. B12** | N-(4-(2-isopropoxypropan-2-yl)thiazol-2-yl)-2-(5-(pyridin-4-ylmethyl)-1H-pyrrol-2-yl)acetamide | 398 | 399 |
| **A117** | N-((4-isopropylthiazol-2-yl)methyl)-1-(pyridin-4-ylmethyl)-1H-pyrrole-2-carboxamide | 340 | 341 |
| **A122** | N-(4-(2-isopropoxypropan-2-yl)thiazol-2-yl)-1-(pyridin-4-ylmethyl)-1H-pyrrole-3-carboxamide | 384 | 385 |
| **A124** | N-(4-(2-isopropoxypropan-2-yl)thiazol-2-yl)-1-((1-methyl-6-oxo-1,6-dihydropyridin-3-yl)methyl)-1H-pyrrole-2-carboxamide | 414 | 415 |
| **A125** | N-(4-(2-isopropoxypropan-2-yl)thiazol-2-yl)-1-((2-methoxypyridin-4-yl)methyl)-1H-pyrrole-2-carboxamide | 414 | 415 |
| **Ref. B20** | (S)-1-isonicotinoyl-N-(4-isopropylthiazol-2-yl)pyrrolidine-2-carboxamide | 344 | 345 |
| **Ref. B21** | (R)-1-isonicotinoyl-N-(4-isopropylthiazol-2-yl)pyrrolidine-2-carboxamide | 344 | 345 |
| **A126** | benzyl 4-((2-((4-(2-isopropoxypropan-2-yl)thiazol-2-yl)carbamoyl)-1H-pyrrol-1-yl)methyl)piperidine-1-carboxylate | 524 | 525 |
| **Ref. B22** | N-(4-(2-acetamidoethyl)-5-methylthiazol-2-yl)-1-(pyridin-4-ylmethyl)-1H-pyrrole-2-carboxamide | 383 | 384 |
| **Ref. B23** | N-((1S,2R)-2-(tert-butyl)cyclopropyl)-1-(pyridin-4-ylmethyl)-1H-pyrrole-2-carboxamide | 297 | 298 |
| **A127** | N-(4-(2-isopropoxypropan-2-yl)thiazol-2-yl)-1-((1-methyl-2-oxo-1,2-dihydropyridin-4-yl)methyl)-1H-pyrrole-2-carboxamide | 414 | 415 |
| **Intermediate for Ref. B24** | tert-butyl (R)-2-((4-ethyl-5-methylthiazol-2-yl)carbamoyl)pyrrolidine-1-carboxylate | 339 | 340 |
| **Ref. B25** | N-(3-ethyl-4-methylphenyl)-N-methyl-1-(pyridin-4-ylmethyl)-1H-pyrrole-2-carboxamide | 333 | 334 |
| **A128** | 1-((1-acetylpiperidin-4-yl)methyl)-N-(4-(2-isopropoxypropan-2-yl)thiazol-2-yl)-1H-pyrrole-2-carboxamide | 432 | 433 |
| **A129** | N-(4-(2-isopropoxypropan-2-yl)thiazol-2-yl)-1-((1-methylpiperidin-4-yl)methyl)-1H-pyrrole-2-carboxamide | 404 | 405 |
| **Ref. B26** | (S)-N-(4-ethyl-5-methylthiazol-2-yl)-1-(pyridin-4-ylmethyl)pyrrolidine-2-carboxamide | 330 | 331 |
| **A130** | (S)-N-(4-(2-isopropoxypropan-2-yl)thiazol-2-yl)-1-(pyridin-4-ylmethyl)pyrrolidine-2-carboxamide | 388 | 389 |
| **A131** | N-(4-ethyl-5-methylthiazol-2-yl)-1-(pyridin-3-ylmethyl)-1H-pyrrole-2-carboxamide | 326 | 327 |
| **Ref. B27** | 1-benzyl-N-(4-(tert-butyl)thiazol-2-yl)-1H-pyrrole-2-carboxamide | 339 | 340 |
| **Ref. B11** | N-(4-(tert-butyl)thiazol-2-yl)-1-(2-chlorobenzyl)-1H-pyrrole-2-carboxamide | 373 | 374 |
| **Ref. B28** | N-(4-(tert-butyl)thiazol-2-yl)-5-chloro-1-(pyridin-4-ylmethyl)-1H-indole-2-carboxamide | 424 | 425 |
| **Ref. B29** | 5-chloro-N-(4-ethyl-5-methylthiazol-2-yl)-1-(pyridin-4-ylmethyl)-1H-indole-2-carboxamide | 410 | 411 |
| **A293** | N-(4-(2-methyl-4-phenylbut-3-en-2-yl)thiazol-2-yl)-1-(pyridin-4-ylmethyl)-1H-pyrrole-2-carboxamide | 428 | 429 |
| **A292** | N-(4-((1R,3R)-3-phenylcyclopentyl)thiazol-2-yl)-1-(pyridin-4-ylmethyl)-1H-pyrrole-2-carboxamide | 428 | 429 |
| **A280** | N-(4-(2-isopropoxypropan-2-yl)thiazol-2-yl)-2-(pyridin-4-ylmethyl)benzamide | 395 | 396 |
| **A269** | N-(4-(2-(5-methoxypyridin-2-yl)vinyl)thiazol-2-yl)-1-(pyridin-4-ylmethyl)-1H-pyrrole-2-carboxamide | 417 | 418 |
| **A263** | N-(4-(1-(benzyloxy)cyclopropyl)thiazol-2-yl)-1-(pyridin-4-ylmethyl)-1H-pyrrole-2-carboxamide | 430 | 431 |
| **A155** | 1-(pyridin-4-ylmethyl)-N-(4-(2-(tetrahydro-2H-pyran-2-yl)vinyl)thiazol-2-yl)-1H-pyrrole-2-carboxamide | 394 | 395 |
| **A256** | 1-((3,5-difluoropyridin-4-yl)methyl)-N-(4-(2-isopropoxypropan-2-yl)thiazol-2-yl)-1H-pyrrole-2-carboxamide | 420 | 421 |
| **A254** | N-(4-(2-((1s,4s)-4-methoxycyclohexyl)vinyl)thiazol-2-yl)-1-(pyridin-4-ylmethyl)-1H-pyrrole-2-carboxamide | 422 | 423 |
| **A253** | N-(4-(2-((1r,4r)-4-methoxycyclohexyl)vinyl)thiazol-2-yl)-1-(pyridin-4-ylmethyl)-1H-pyrrole-2-carboxamide | 422 | 423 |
| **A250** | 1-((3-ethylpyridin-4-yl)methyl)-N-(4-(2-isopropoxypropan-2-yl)thiazol-2-yl)-1H-pyrrole-2-carboxamide | 412 | 413 |
| **A243** | 1-(3-(3-fluoropyridin-4-yl)propyl)-N-(4-(2-isopropoxypropan-2-yl)thiazol-2-yl)-1H-pyrrole-2-carboxamide | 430 | 431 |
| **A241** | N-(4-(2-fluoro-4-methoxystyryl)thiazol-2-yl)-1-(pyridin-4-ylmethyl)-1H-pyrrole-2-carboxamide | 434 | 435 |
| **A240** | N-(4-(3-fluoro-4-methoxystyryl)thiazol-2-yl)-1-(pyridin-4-ylmethyl)-1H-pyrrole-2-carboxamide | 434 | 435 |
| **A238** | 1-((3-chloropyridin-4-yl)methyl)-N-(4-(2-isopropoxypropan-2-yl)thiazol-2-yl)-1H-pyrrole-2-carboxamide | 418 | 419 |
| **A165** | N-(4-(2-(pyridin-3-yl)vinyl)thiazol-2-yl)-1-(pyridin-4-ylmethyl)-1H-pyrrole-2-carboxamide | 387 | 388 |
| **A224** | N-(4-(4-cyanostyryl)thiazol-2-yl)-1-(pyridin-4-ylmethyl)-1H-pyrrole-2-carboxamide | 411 | 410 [M-H]- |
| **A166** | N-(4-(2-(pyridin-4-yl)vinyl)thiazol-2-yl)-1-(pyridin-4-ylmethyl)-1H-pyrrole-2-carboxamide | 387 | 388 |
| **A220** | N-(4-(4-fluorostyryl)thiazol-2-yl)-1-(pyridin-4-ylmethyl)-1H-pyrrole-2-carboxamide | 404 | 405 |
| **A219** | 1-(pyridin-4-ylmethyl)-N-(4-(4-(trifluoromethyl)styryl)thiazol-2-yl)-1H-pyrrole-2-carboxamide | 454 | 455 |
| **A153** | 1-(pyridin-4-ylmethyl)-N-(4-(2-(tetrahydro-2H-pyran-4-yl)vinyl)thiazol-2-yl)-1H-pyrrole-2-carboxamide | 394 | 395 |
| **A152** | N-(4-(2-cyclohexylvinyl)thiazol-2-yl)-1-(pyridin-4-ylmethyl)-1 H-pyrrole-2-carboxamide | 392 | 393 |
| **A158** | N-(4-(2-methoxystyryl)thiazol-2-yl)-1-(pyridin-4-ylmethyl)-1H-pyrrole-2-carboxamide | 416 | 417 |
| **A160** | N-(4-(4-methoxystyryl)thiazol-2-yl)-1-(pyridin-4-ylmethyl)-1H-pyrrole-2-carboxamide | 416 | 418 |
| **A154** | 1-(pyridin-4-ylmethyl)-N-(4-(2-(tetrahydro-2H-pyran-3-yl)vinyl)thiazol-2-yl)-1H-pyrrole-2-carboxamide | 394 | 395 |
| **A159** | N-(4-(3-methoxystyryl)thiazol-2-yl)-1-(pyridin-4-ylmethyl)-1H-pyrrole-2-carboxamide | 416 | 417 |
| **A161** | N-(4-(2-chlorostyryl)thiazol-2-yl)-1-(pyridin-4-ylmethyl)-1H-pyrrole-2-carboxamide | 420 | 421 |
| **A163** | N-(4-(4-chlorostyryl)thiazol-2-yl)-1-(pyridin-4-ylmethyl)-1H-pyrrole-2-carboxamide | 420 | 421 |
| **A209** | N-(4-(3-chlorostyryl)thiazol-2-yl)-1-(pyridin-4-ylmethyl)-1H-pyrrole-2-carboxamide | 420 | 421 |
| **A210** | N-(4-(2-isopropoxypropan-2-yl)thiazol-2-yl)-1-((1-methyl-5-oxopyrrolidin-3-yl)methyl)-1 H-pyrrole-2-carboxamide | 404 | 405 |
| **A300** | N-(4-((1S,2S)-2-phenylcyclopentyl)thiazol-2-yl)-1-(pyridin-4-ylmethyl)-1H-pyrrole-2-carboxamide | 428 | 429 |
| **A301** | N-(4-((1S,2R)-2-phenylcyclopentyl)thiazol-2-yl)-1-(pyridin-4-ylmethyl)-1H-pyrrole-2-carboxamide | 428 | 429 |
| **A305** | N-(4-(1S,3R)-3-phenylcyclopentyl)thiazol-2-yl)-1-(pyridin-4-ylmethyl)-1H-pyrrole-2-carboxamide | 428 | 429 |
| **A306** | N-(4-(1-phenylpiperidin-3-yl)thiazol-2-yl)-1-(pyridin-4-ylmethyl)-1H-pyrrole-2-carboxamide | 443 | 444 |
| **A310** | N-(4-(5-phenyltetrahydrofuran-2-yl)thiazol-2-yl)-1-(pyridin-4-ylmethyl)-1H-pyrrole-2-carboxamide | 430 | 431 |
| **A184** | N-(4-benzylthiazol-2-yl)-1-(3-(pyridin-4-yl)propyl)-1H-pyrrole-2-carboxamide | 402 | 403 |
| **A187** | N-(4-benzylthiazol-2-yl)-1-(3-(pyridin-3-yl)propyl)-1H-pyrrole-2-carboxamide | 402 | 403 |
| **A190** | N-(4-(2-isopropoxypropan-2-yl)thiazol-2-yl)-1-(3-(pyridin-3-yl)propyl)-1H-pyrrole-2-carboxamide | 412 | 413 |
| **A211** | N-(4-(2-isopropoxypropan-2-yl)thiazol-2-yl)-1-((3-methoxypyridin-4-yl)methyl)-1H-pyrrole-2-carboxamide | 414 | 415 |
| **A194** | N-(4-((1S,2S)-2-phenylcyclopropyl)thiazol-2-yl)-1-(pyridin-4-ylmethyl)-1H-pyrrole-2-carboxamide | 400 | 401 |
| **A212** | 1-((3-(benzylamino)pyridin-4-yl)methyl)-N-(4-(2-isopropoxypropan-2-yl)thiazol-2-yl)-1H-pyrrole-2-carboxamide | 489 | 490 |
| **A213** | 1-((3-(benzylamino)pyridin-4-yl)methyl)-N-(4-benzylthiazol-2-yl)-1H-pyrrole-2-carboxamide | 479 | 480 |
| **A214** | N-(4-(2-isopropoxypropan-2-yl)thiazol-2-yl)-1-((3-(trifluoromethyl)pyridin-4-yl)methyl)-1H-pyrrole-2-carboxamide | 452 | 453 |
| **A215** | N-(4-((1R,2R)-2-phenylcyclopropyl)thiazol-2-yl)-1-(3-(pyridin-4-yl)propyl)-1H-pyrrole-2-carboxamide | 428 | 429 |
| **A216** | N-(4-phenylthiazol-2-yl)-1-(pyridin-4-ylmethyl)-1 H-pyrrole-2-carboxamide | 360 | 361 |
| **A217** | N-(4-(3-phenylprop-1-en-1-yl)thiazol-2-yl)-1-(3-(pyridin-4-yl)propyl)-1H-pyrrole-2-carboxamide | 428 | 429 |
| **A218** | N-(4-(3-phenylprop-1-en-1-yl)thiazol-2-yl)-1-(pyridin-4-ylmethyl)-1H-pyrrole-2-carboxamide | 400 | 401 |
| **A221** | N-(4-(2-isopropoxypropan-2-yl)thiazol-2-yl)-1-(3-(pyridin-4-yl)allyl)-1H-pyrrole-2-carboxamide | 410 | 411 |
| **A222** | N-(4-(2-(furan-3-yl)vinyl)thiazol-2-yl)-1-(pyridin-4-ylmethyl)-1H-pyrrole-2-carboxamide | 376 | 377 |
| **A225** | N-(4-(2-methyl-4-phenylbutan-2-yl)thiazol-2-yl)-1-(pyridin-4-ylmethyl)-1H-pyrrole-2-carboxamide | 430 | 431 |
| **A226** | N-(4-(2-isopropoxypropan-2-yl)thiazol-2-yl)-1-(2-(pyridin-4-yloxy)ethyl)-1 H-pyrrole-2-carboxamide | 414 | 415 |
| **A232** | N-(4-(2-(furan-3-yl)vinyl)thiazol-2-yl)-1-(3-(pyridin-4-yl)propyl)-1H-pyrrole-2-carboxamide | 404 | 405 |
| **A234** | N-(4-(2-(benzo[d][1,3]dioxol-5-yl)vinyl)thiazol-2-yl)-1-(pyridin-4-ylmethyl)-1H-pyrrole-2-carboxamide | 430 | 431 |
| **A235** | N-(4-(2-(2,2-difluorobenzo[d][1,3]dioxol-5-yl)vinyl)thiazol-2-yl)-1-(pyridin-4-ylmethyl)-1H-pyrrole-2-carboxamide | 466 | 467 |
| **A236** | N-(4-(4-isopropoxystyryl)thiazol-2-yl)-1-(pyridin-4-ylmethyl)-1H-pyrrole-2-carboxamide | 444 | 445 |
| **A242** | N-(4-(1H-inden-2-yl)thiazol-2-yl)-1-(pyridin-4-ylmethyl)-1H-pyrrole-2-carboxamide | 398 | 399 |
| **A244** | N-(4-(2-(cyclohex-1-en-1-yl)vinyl)thiazol-2-yl)-1-(pyridin-4-ylmethyl)-1H-pyrrole-2-carboxamide | 390 | 391 |
| **A247** | N-(4-(1-phenylprop-1-en-2-yl)thiazol-2-yl)-1-(pyridin-4-ylmethyl)-1H-pyrrole-2-carboxamide | 400 | 401 |
| **A248** | N-(4-(4-cyclopropoxystyryl)thiazol-2-yl)-1-(pyridin-4-ylmethyl)-1H-pyrrole-2-carboxamide | 442 | 443 |
| **A249** | 1-(pyridin-4-ylmethyl)-N-(4-(1,2,3,6-tetrahydro-[1,1'-biphenyl]-4-yl)thiazol-2-yl)-1H-pyrrole-2-carboxamide | 440 | 441 |
| **A223** | N-(4-(4-acetamidostyryl)thiazol-2-yl)-1-(pyridin-4-ylmethyl)-1H-pyrrole-2-carboxamide | 443 | 444 |
| **Intermediate for A228** | N-(4-(1 -(2-bromophenoxy)-2-methylpropan-2-yl)thiazol-2-yl)-1-(pyridin-4-ylmethyl)-1H-pyrrole-2-carboxamide | 510 | 511 |
| **Intermediate for A231** | N-(4-(4-cyanostyryl)thiazol-2-yl)-1-(pyridin-4-ylmethyl)-1H-pyrrole-2-carboxamide | 411 | 410 [M-H]- |

### Procedure 3 - Synthesis of Compound A103

Synthesis of A103 followed the scheme, below:

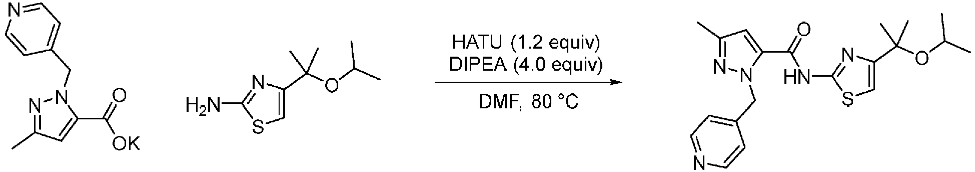

To the solution of potassium salt of acid (0.145 g, 0.57 mmol, 1.0 equiv), DIPEA (0.395 mL, 2.27 mmol, 4.0 equiv) and HATU (0.266 g, 0.70 mmol, 1.23 equiv) in DMF (2.9 mL) were added. After 15 min, amine (0.114 g, 0.57 mmol, 1.0 equiv) was added. The reaction was allowed to stir at 80 ⁰C overnight. The next morning, the reaction mixture was diluted wth EtOAc (50 mL) and washed with saturated NaHCO₃ (2 × 50 mL), followed by water (1 × 50 mL). The organic layer was then dried over MgSO₄, filtered and concentrated in vacuo. The crude product was purified via silica gel chromatography eluting with EtOAc. LCMS (+ESI): calc. [M+H]⁺= 400; found 400.

The following compounds were prepared in a similar manner:

| **Compound** | **Compound name** | **Exact mass [M]** | **Observed molecular ion [M+H]** |
|---|---|---|---|
| **A19** | 1-(pyridin-4-ylmethyl)-N-(4-(trifluoromethyl)thiazol-2-yl)-1H-pyrrole-2-carboxamide | 352 | 353 |
| **A20** | N-(4-(1-isopropoxyethyl)-5-methylthiazol-2-yl)-1-(pyridin-4-ylmethyl)-1H-pyrrole-2-carboxamide | 384 | 385 |
| **A35** | N-(4-ethylthiazol-2-yl)-1-(pyridin-4-ylmethyl)-1H-pyrrole-2-carboxamide | 312 | 313 |
| **A53** | N-(4-(2-isopropoxypropan-2-yl)thiazol-2-yl)-1-(4-nitrobenzyl)-1H-pyrrole-2-carboxamide | 428 | 429 |
| **A63** | N-(4-methylthiazol-2-yl)-1-(pyridin-4-ylmethyl)-1H-pyrrole-2-carboxamide | 298 | 299 |
| **A67** | N-(4-(1-methylcyclopropyl)thiazol-2-yl)-1-(pyridin-4-ylmethyl)-1H-pyrrole-2-carboxamide | 338 | 339 |
| **A72** | N-(5-methylthiazol-2-yl)-1-(pyridin-4-ylmethyl)-1H-pyrrole-2-carboxamide | 298 | 299 |
| **A76** | N-(4-(2-isopropoxypropan-2-yl)thiazol-2-yl)-5-methyl-1-(pyridin-4-ylmethyl)-1H-pyrrole-2-carboxamide | 398 | 399 |
| **A86** | 3-chloro-N-(4-(2-isopropoxypropan-2-yl)thiazol-2-yl)-1-(pyridin-4-ylmethyl)-1H-pyrrole-2-carboxamide | 418 | 419 |
| **A88** | N-(4-(2-isopropoxypropan-2-yl)thiazol-2-yl)-3-methyl-1-(pyridin-4-ylmethyl)-1H-pyrrole-2-carboxamide | 398 | 399 |
| **Ref. B10** | N-(3-ethyl-4-methylphenyl)-1-(pyridin-4-ylmethyl)-1H-pyrrole-2-carboxamide | 319 | 320 |
| **A92** | N-(5-ethyl-4-((4-(ethylsulfonyl)piperazin-1-yl)methyl)thiazol-2-yl)-1-(pyridin-4-ylmethyl)-1H-pyrrole-2-carboxamide | 502 | 503 |
| **A109** | N2-(4-(2-isopropoxypropan-2-yl)thiazol-2-yl)-1-(pyridin-4-ylmethyl)-1H-pyrrole-2,4-dicarboxamide | 427 | 428 |

### Procedure 4 - Synthesis of Compound A97

Synthesis of A97 followed the scheme, below:

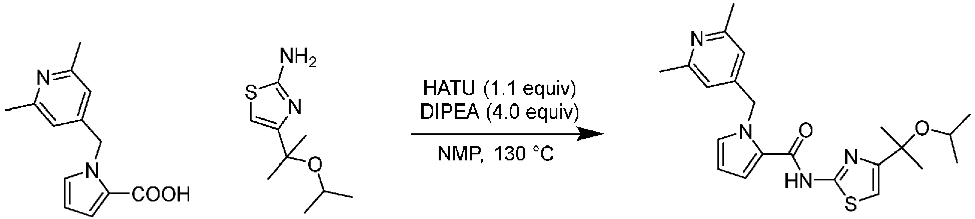

To the solution of the acid (0.090 g, 0.39 mmol, 1.0 equiv) in NMP (1 mL) were added HATU (0.163 g, 0.43 mmol, 1.1 equiv), DIPEA (0.272 mL, 1.56 mmol, 4.0 equiv) and amine (0.117 g, 0.59 mmol, 1.5 eq.). Reaction was continued either with conventional heating or in the microwave at 130°C for 1 h. After that the reaction mixture was diluted with EtOAc (50 mL) and washed with saturated NaHCO₃ (2 × 50 mL), followed by water (1 × 50 mL). The organic layer was then dried over MgSO₄, filtered and concentrated in vacuo. Crude product was purified via silica gel chromatography (1-10% MeOH in DCM). LCMS (+ESI): calc. [M+H]⁺= 413; found 413.

The following compounds were prepared in a similar manner:

| **Compound** | **Compound name** | **Exact mass [M]** | **Observed molecular ion [M+H]** | **Heating Protocol** |
|---|---|---|---|---|
| **A17** | N-(4-benzylthiazol-2-yl)-1-(pyridin-4-ylmethyl)-1H-pyrrole-2-carboxamide | 374 | 375 | conventional heating, 130 ⁰C |
| **A24** | N-(4-(2-isopropoxypropan-2-yl)thiazol-2-yl)-1-((3-methylpyridin-4-yl)methyl)-1H-pyrrole-2-carboxamide | 398 | 399 | conventional heating, 100 ⁰C |
| **A28** | N-(4-(1-isopropoxyethyl)thiazol-2-yl)-1-(pyridin-4-ylmethyl)-1H-pyrrole-2-carboxamide | 370 | 371 | microwave heating, 130 ⁰C |
| **A29** | N-(4-(1,1-difluoroethyl)thiazol-2-yl)-1-(pyridin-4-ylmethyl)-1H-pyrrole-2-carboxamide | 348 | 349 | conventional heating, 100°C |
| **A36** | 1-((2,6-difluoropyridin-4-yl)methyl)-N-(4-(2-isopropoxypropan-2-yl)thiazol-2-yl)-1H-pyrrole-2-carboxamide | 420 | 421 | microwave heating, 130 ⁰C |
| **A48** | N-(4-(2-isopropoxypropan-2-yl)thiazol-2-yl)-4-methyl-1-(pyridin-4-ylmethyl)-1H-pyrrole-2-carboxamide | 398 | 399 | microwave heating, 130 ⁰C |
| **A49** | (R)-N-(4-(2-isopropoxypropan-2-yl)thiazol-2-yl)-1-(1-(pyridin-4-yl)ethyl)-1H-pyrrole-2-carboxamide | 398 | 397 [M-H]- | conventional heating, 150 °C |
| **A70** | 1-((2-fluoro-6-methoxypyridin-4-yl)methyl)-N-(4-(2-isopropoxypropan-2-yl)thiazol-2-yl)-1H-pyrrole-2-carboxamide | 432 | 433 | microwave heating, 130 ⁰C |
| **A74** | (S)-N-(4-(2-isopropoxypropan-2-yl)thiazol-2-yl)-1-(1-(pyridin-4-yl)ethyl)-1H-pyrrole-2-carboxamide | 398 | 399 | conventional heating, 150°C |
| **A80** | 1-(4-cyanobenzyl)-N-(4-(2-isopropoxypropan-2-yl)thiazol-2-yl)-1H-pyrrole-2-carboxamide | 408 | 407 [M-H]- | conventional heating, 100°C |
| **A83** | N-(4-(2-isopropoxypropan-2-yl)thiazol-2-yl)-1-((2-methylpyridin-4-yl)methyl)-1H-pyrrole-2-carboxamide | 398 | 399 | conventional heating, 100°C |
| **A84** | N-(4-(2-isopropoxypropan-2-yl)thiazol-2-yl)-1-(4-(methylsulfonyl)benzyl)-1H-pyrrole-2-carboxamide | 461 | 462 | conventional heating, 100°C |
| **A93** | N-(4-(2-isopropoxypropan-2-yl)thiazol-2-yl)-1-((5-oxopyrrolidin-3-yl)methyl)-1H-pyrrole-2-carboxamide | 390 | 392 | conventional heating, 60°C |
| **A96** | N-(4-((4-(ethylsulfonyl)piperazin-1-yl)methyl)thiazol-2-yl)-1-(pyridin-4-ylmethyl)-1H-pyrrole-2-carboxamide | 474 | 475 | microwave heating, 130 ⁰C |
| **A99** | N-(4-(2-isopropoxypropan-2-yl)thiazol-2-yl)-1-(4-(N-methylacetamido)benzyl)-1H-pyrrole-2-carboxamide | 454 | 455 | microwave heating, 130 ⁰C |
| **A102** | N-(4-(2-isopropoxypropan-2-yl)thiazol-2-yl)-6-oxo-1-(pyridin-4-ylmethyl)piperidine-2-carboxamide | 416 | 417 | microwave heating, 130 ⁰C |
| **A104** | N-(4-(2-isopropoxypropan-2-yl)thiazol-2-yl)-1-(pyridin-4-ylmethyl)-1H-imidazole-5-carboxamide | 385 | 387 | microwave heating, 130 ⁰C |
| **A105** | N-(4-(2-isopropoxypropan-2-yl)thiazol-2-yl)-1-(pyridin-4-ylmethyl)-1H-pyrrole-2,5-dicarboxamide | 427 | 429 | microwave heating, 130 ⁰C |
| **A107** | N2-(4-(2-isopropoxypropan-2-yl)thiazol-2-yl)-N5,N5-dimethyl-1-(pyridin-4-ylmethyl)-1H-pyrrole-2,5-dicarboxamide | 455 | 456 | **microwave** heating, 130 ⁰C |
| **A108** | N-(4-(2-isopropoxypropan-2-yl)thiazol-2-yl)-1-(pyridin-4-ylmethyl)-1H-imidazole-2-carboxamide | 385 | 386 | conventional heating, 130°C |
| **A110** | 5-(hydroxymethyl)-N-(4-(2-isopropoxypropan-2-yl)thiazol-2-yl)-1-(pyridin-4-ylmethyl)-1H-pyrrole-2-carboxamide | 414 | 415 | microwave heating, 130 ⁰C |
| **A111** | N2-(4-(2-isopropoxypropan-2-yl)thiazol-2-yl)-N4,N4-dimethyl-1-(pyridin-4-ylmethyl)-1H-pyrrole-2,4-dicarboxamide | 455 | 457 | conventional heating, 130°C |
| **A114** | 1-(4-acetamidobenzyl)-N-(4-(2-isopropoxypropan-2-yl)thiazol-2-yl)-1H-pyrrole-2-carboxamide | 440 | 441 | microwave heating, 130 ⁰C |

### Procedure 5 - Synthesis of Compound A281

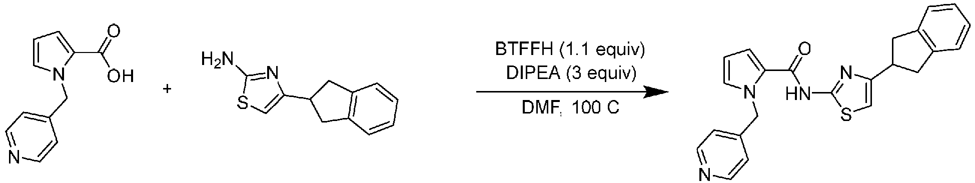

A 20 mL vial with stir bar was charged with acid (100 mg, 0.495 mmol, 1.1 equiv), amine (97.2 mg, 0.450 mmol, 1.0 equiv), BTFFH (156 mg, 0.495 mmol, 1.1 equiv) and DMF (1.0 mL, 0.4 M). DIPEA (160 uL, 0.899 mmol, 2.0 equiv) was added, and the vial was capped and placed in a 100 C bath. The reaction mixture was stirred at 100 C overnight. The next morning, the reaction mixture was poured into EtOAc (50 mL) and washed with 1:1 1 M NaOH:brine (2 x 50 mL). The combined aqueous layers were extracted with EtOAc (1 × 50 mL), and the combined organic layers were dried over Na2SO4, filtered and concentrated in vacuo. The resulting crude material was purified via silica gel chromatography to yield the desired product.

The following compounds were prepared via a similar method:

| **Compound** | **Observed molecular ion** |
|---|---|
| **A246** | 458 |
| **A245** | 458 |
| **A157** | 364 |
| **A150** | 364 |
| **A239** | 444 |
| **A230** | 364 |
| **A151** | 364 |
| **A227** | 430 |
| **A307** | 389 |
| **A314** | 462 |
| **A316** | 416 |
| **A330** | 431 |
| **A329** | 431 |
| **A255** | 443 |
| **A258** | 442 |
| **A259** | 456 |
| **A268** | 415 |
| **A281** | 401 |
| **A282** | 444 |
| **A283** | 417 |
| **A284** | 429 |
| **A285** | 472 |
| **A286** | 445 |
| **A287** | 443 |
| **A302** | 458 |
| **A318** | 464 |
| **A327** | 472 |
| **A328** | 510 |
| **A341** | 413 |
| **A342** | 406 |
| **A343** | 456 |
| **A359** | 474 |
| **A365** | 436 |
| **A366** | 389 |
| **Intermediate for A317** | 454 |

### Procedure 7 - Synthesis of Compound A309

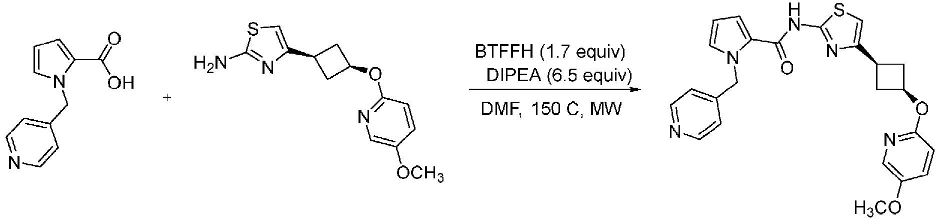

A 20 mL microwave vial (G30) with stir bar was charged with acid (109.95 mg, 0.544 mmol, 1.3 equiv), amine (116 mg, 0.418 mmol, 1.0 equiv), BTFFH (224.83 mg, 0.711 mmol, 1.7 equiv) and DMF (8.0 mL, 0.052 M). DIPEA (0.474 mL, 2.719 mmol, 6.5 equiv) was added, and the vial was capped and placed in a microwave reactor at 150 C. The reaction mixture was stirred at 150 C for 1 h. The reaction mixture was poured into EtOAc (50 mL) and washed with 1:1 a saturated NaHCO₃: brine (2 × 20 mL). The combined aqueous layers were extracted with EtOAc (1 × 50 mL), and the combined organic layers were dried over Na₂SO₄, filtered and concentrated in vacuo. The resulting crude material was purified via silica gel chromatography to yield the desired product.

The following compounds were prepared via a similar method:

| **Compound** | **Observed molecular ion** |
|---|---|
| **A309** | 462 |
| **A315** | 447 |

### Procedure 8 - Synthesis of Compound A251

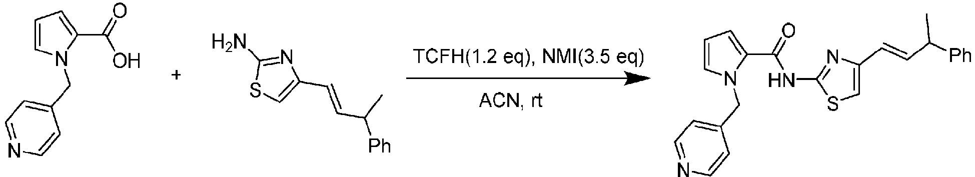

A 25 mL vial with stir bar was charged with acid (110.00 mg, 0.544 mmol, 1.00 equiv), amine (162.80 mg, 0.707 mmol, 1.30 equiv), NMI (156.30 mg, 1.904 mmol, 3.50 equiv) and ACN (4.0 mL, 0.14 M). TCFH (184.60 mg, 0.660 mmol, 1.21 equiv) was added, and the vial was capped and placed in a 25°C bath. The reaction mixture was stirred at 25°C overnight. The next morning, the reaction mixture was poured into EtOAc (30 mL) and washed with brine (2 × 30 mL). The combined aqueous layers were extracted with EtOAc (1 × 30 mL), and the combined organic layers were dried over Na₂SO₄, filtered and concentrated in vacuo. The resulting crude material was purified via silica gel chromatography & Prep-HPLC or RP column to yield the desired product.

The following compounds were prepared via a similar method:

| **Compound** | **Observed molecular ion** |
|---|---|
| **A251** | 415 |
| **A252** | 401 |
| **A257** | 434 |
| **A260** | 434 |
| **A262** | 432 |
| **A267** | 416 |
| **A270** | 443 |
| **A271** | 443 |
| **A272** | 432 |
| **A274** | 432 |
| **A275** | 430 |
| **A276** | 430 |
| **A277** | 444 |
| **A278** | 444 |
| **A279** | 444 |
| **A288** | 403 |
| **A289** | 430 |
| **A290** | 430 |
| **A291** | 444 |
| **A294** | 441 |
| **A200** | 401 |
| **A296** | 417 |
| **A297** | 401 |
| **A298** | 443 |
| **A299** | 436 |
| **A303** | 443 |
| **A304** | 461 |
| **A311** | 432 |
| **A312** | 432 |
| **A313** | 458 |
| **A319** | 444 |
| **A320** | 458 |
| **Intermediate for A321** | 684 |
| **A322** | 684 |
| **A323** | 684 |
| **A324** | 415 |
| **A325** | 460 |
| **Intermediate for A326** | 684 |
| **A331** | 415 |
| **A332** | 444 |
| **A333** | 460 |
| **A334** | 460 |
| **A335** | 450 |
| **A336** | 416 |
| **A337** | 446 |
| **A338** | 444 |
| **A339** | 466 |
| **Intermediate for A344** | 684 |
| **A345** | 460 |
| **A346** | 488 |
| **A347** | 402 |
| **A349** | 416 |
| **A350** | 416 |
| **Intermediate for A351** | 684 |
| **A352** | 684 |
| **A353** | 684 |
| **A354** | 528 |
| **A355** | 402 |
| **A356** | 402 |
| **A357** | 402 |
| **A358** | 424 |
| **A360** | 417 |
| **A361** | 510 |
| **A362** | 474 |
| **A363** | 434 |
| **A364** | 434 |

### Procedure 9 - Synthesis of A261

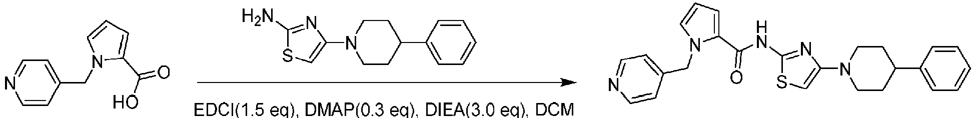

A 25 mL vial with stir bar was charged with acid (54.60 mg, 0.27 mmol, 1.00 equiv), EDCI (77.70 mg, 0.41 mmol, 1.50 equiv), DIEA (104.60 mg, 0.81 mmol, 3.00 equiv), DMAP (10.00 mg, 0.08 mmol, 0.30 equiv) and DCM (4.0 mL, 0.068 M). Amine (70.00 mg, 0.27 mmol, 1.00 equiv) was added, and the vial was capped and placed in a 25°C bath. The reaction mixture was stirred at 25°C for 4 h. The resulting solution was concentrated in vacuo. The resulting crude material was purified via silica gel chromatography & Prep-HPLC or RP column to yield the desired product.

The following compounds were prepared via a similar method:

| **Compound** | **Observed molecular ion** |
|---|---|
| **A261** | 444 |
| **A264** | 368 |
| **A265** | 444 |
| **A266** | 445 |
| **A273** | 430 |

### Procedure 10 - Synthesis of Intermediate for A141-A144, A164, A229, & A237

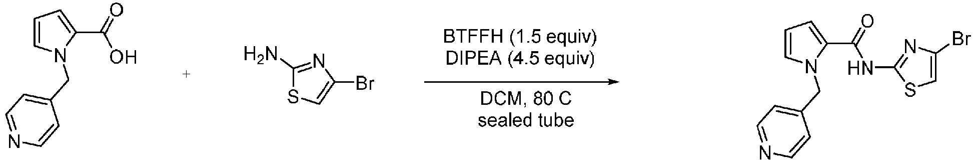

A vial with stir bar was chaged with amine (1.0 g, 5.59 mmol, 1.0 equiv) and DCM (10 mL). BTFFH (2.7 g, 8.4 mmol, 1.5 equiv), DIPEA (4.4 mL, 25 mmol, 4.5 equiv) and acid (1.5 g, 7.26 mmol, 1.1 equiv) were added. The reaction mixture was sealed and stirred at 80 C for 20 h. After this time, the reaction mixture was poured into water (100 mL). The water layer was extracted with EtOAc (2 × 100 mL), and the combined organic layers were washed with water (3 × 100 mL). The combined organic layers were then dred over MgSO4, filtered and concentrated in vacuo. The resulting crude material was purified via silica gel chromatography, followed by recrystallization from hot EtOH.

### Example 5: Post-Amide Coupling Modifications

### Procedure 1 - Synthesis of Compound A30

Synthesis of A30 followed the scheme, below:

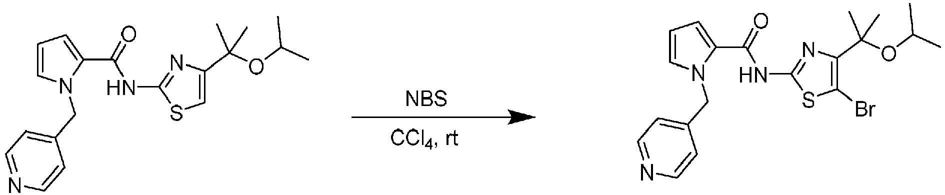

A 20 mL scintillation vial with stir bar was charged with N-(4-(2-isopropoxypropan-2-yl)thiazol-2-yl)-1-(pyridin-4-ylmethyl)-1H-pyrrole-2-carboxamide (131 mg, 0.341 mmol, 1.0 equiv) and CCl₄ (2 mL, 0.2 M). N-bromosuccinimide (66.7 mg, 0.375 mmol, 1.1 equiv) was added, and the reaction mixture was stirred at room temperature overnight. The next morning, the solvent was evaporated, and the crude material was purified directly via silica gel chromatography (70-100% EtOAc in hexanes) to yield the desired product. LCMS (+ESI): calc. [M+H]⁺= 463; found 463.

### Procedure 2 - Synthesis of Compound A126

Synthesis of A126 followed the scheme, below:

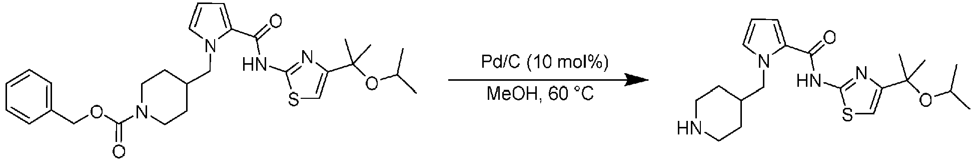

A 4 mL vial with stir bar was charged with benzyl 4-((2-((4-(2-isopropoxypropan-2-yl)thiazol-2-yl)carbamoyl)-1H-pyrrol-1-yl)methyl)piperidine-1-carboxylate (28 mg, 0.053 mmol, 1.0 equiv) and Pd/C (10 wt%, 5.7 mg, 0.0053 mmol, 0.1 equiv). The solids were evacuated and backflushed with hydrogen (1 atm, 3x). Methanol (0.5 mL, 0.1 M) was added, and the reaction mixture was heated to 60 ⁰C over 3 days. After 3 days, the reaction mixture was filtered and concentrated in vacuo. The resulting crude material was purified via

silica gel chromatography (5% MeOH in DCM with 1% NEt₃) to yield the desired product. LCMS (+ESI): calc. [M+H]⁺= 391; found 391.

### Procedure 3 - Synthesis of Reference Compound B24

Synthesis of B24 followed the scheme, below:

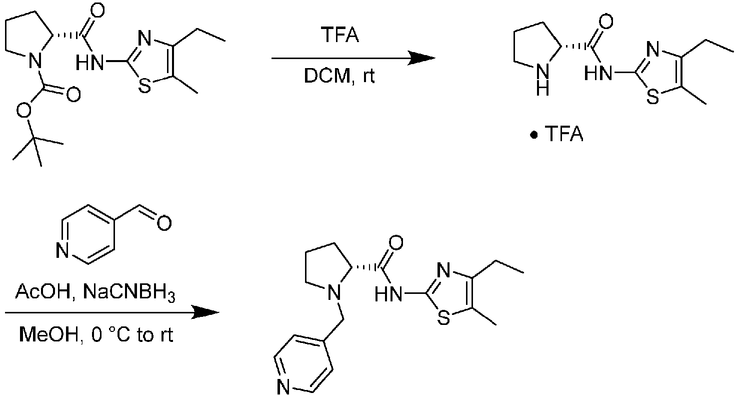

### Step 1: Boc Deprotection

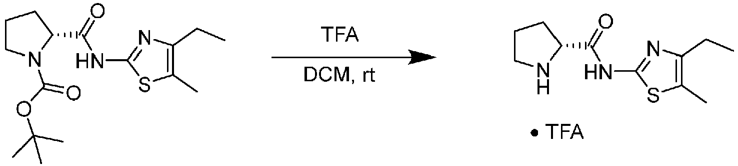

A 20 mL scintillation vial with stir bar was charged with tert-butyl (R)-2-((4-ethyl-5-methylthiazol-2-yl)carbamoyl)pyrrolidine-1-carboxylate (196 mg, 0.577 mmol, 1.0 equiv). DCM (2.4 mL) and TFA (600 uL, 20 vol%) were added, and the reaction mixture was stirred at room temperature overnight. The next morning, the reaction mixture was concentrated in vacuo. The resulting product was used directly in the next step without further purification.

### Step 2: Reductive amination

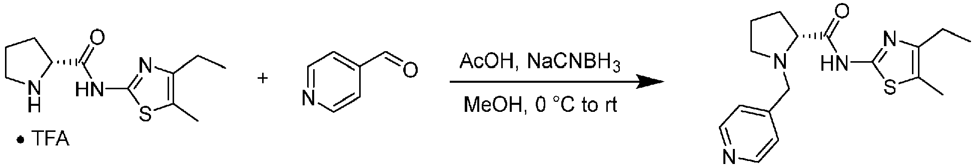

A vial with stir bar was charged with (R)-N-(4-ethyl-5-methylthiazol-2-yl)pyrrolidine-2-carboxamide 2,2,2-trifluoroacetate (204 mg, 0.577 mmol, 1.0 equiv) and methanol (2.4 mL, 0.2 M). Isonicotinaldehyde (0.326 mL, 6.46 mmol, 6.0 equiv) was added, followed by acetic acid (0.149 mL, 2.60 mmol, 4.5 equiv). The reaction mixture was cooled to 0 °C. Sodium cyanoborohydride (218 mg, 3.46 mmol, 6.0 equiv) was slowly added at 0 ⁰C, and the reaction was warmed to room temperature overnight. The next morning, the reaction mixture was diluted with DCM (50 mL) and washed with saturated NaHCO₃ (3 × 50 mL). The organic layer was dried over Na₂SO₄, filtered and concentrated in vacuo. The resulting crude material was purified via silica gel chromatography (5% MeOH in DCM with 1% NEt₃) to yield the desired product. LCMS (+ESI): calc. [M+H]⁺= 331; found 331.

### Procedure 4 - Synthesis of Compound A85

Synthesis of A85 followed the scheme, below:

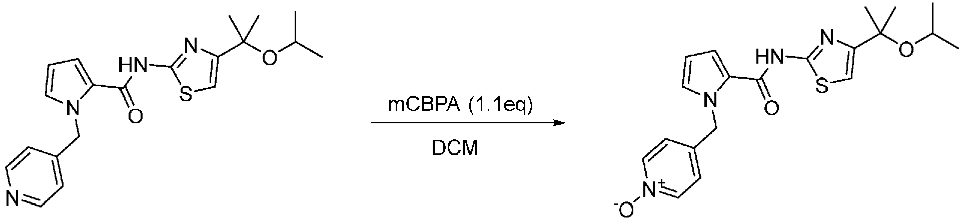

Amide (100 mg, 0.26 mmol, 1 equiv) was dissolved in DCM (2.6mL). mCPBA (64.1 mg, 0.28 mmol, 1.1 equiv) was added at room temperature, and the reaction mixture was stirred overnight. The next morning, the solvent was evaporated, and the resulting crude material was purified via pTLC (3% NEt₃ in EtOAc), followed by trituration with Et₂O and filtration, to yield the desired product. LCMS (+ESI): calc. [M+H]⁺= 401; found 401.

### Procedure 5 - Synthesis of Compound A228

Synthesis of Compound A228 followed the scheme, below:

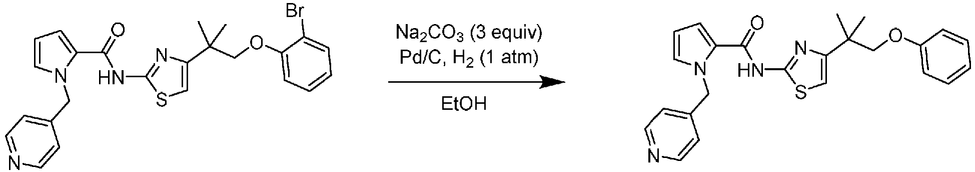

A vial with stir bar was charged with aryl bromide (127 mg, 0.248 mmol, 1.0 equiv), sodium carbonate (79 mg, 0.745 mmol, 3.0 equiv), and Pd/C (10 wt%, 26 mg, 0.1 equiv). The vial was evacuated and backflushed with hydrogen. EtOH (1 mL) was added, and the solution was allowed to stir at room temperature for 1 h. After 1 h, the solution was filtered through a plug of Celite, and the reaction mixture was concentrated in vacuo to yield the desired product (obs. [M+H] = 433).

### Procedure 6 - Synthesis of Compound A231

Synthesis of Compound A231 followed the scheme, below:

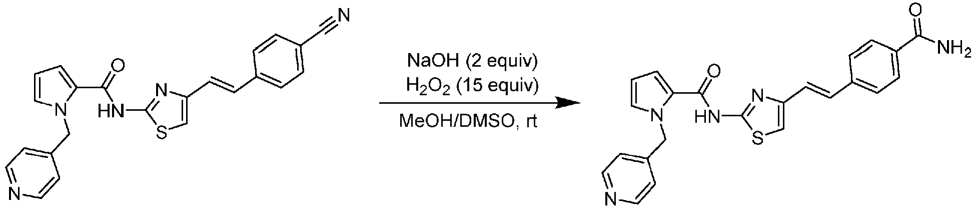

A vial with stir bar was charged with nitrile (20 mg, 0.0486 mmol, 1.0 equiv) in 1:1 DMSO:MeOH (1 mL). NaOH (1.0 M in water, 97 uL, 0.097 mmol, 2.0 equiv) was added, followed by H2O2 (30 wt% in water, 83 uL, 0.729 mmol, 15 equiv). The reaction mixture was allowed to stir at room temperature overnight. The next morning, the reaction mixture was poured into DCM (50 mL) and washed with saturated NaHCO₃ (2 × 50 mL). The combined aqueous layers were extracted with DCM (1 × 50 mL), and the combined organic layers were dried over Na₂SO₄, filtered and concentrated in vacuo to yield the desired product (obs. [M+H] = 430).

### Procedure 7 - Synthesis of Compound A317

Synthesis of Compound A317 followed the scheme, below:

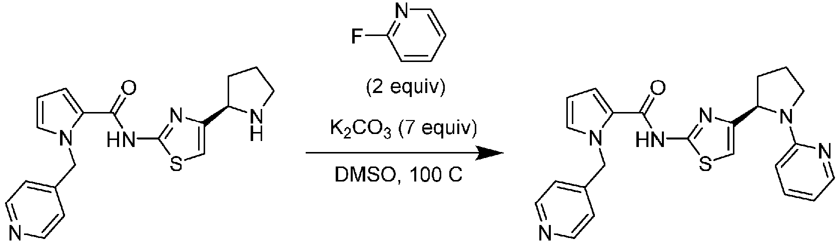

A vial with stir bar was charged with piperidine (74 mg, 0.208 mmol, 1.0 equiv), K₂CO₃ (201 mg, 1.46 mmol, 7 equiv), 2-fluoropyridine (36 uL, 0.416 mmol, 2.0 equiv) and DMSO (1 mL). The vial was capped and stirred at 100 C overnight. The next morning, the reaction mixture was poured into 10% MeOH in DCM (50 mL). The organic layer was washed with saturated NaHCO₃ (2 × 50 mL), and the combined aqueous layers were extracted with 10% MeOH in DCM (1 × 50 mL). The combined organic layers were dried over Na₂SO₄, filtered and concentrated in vacuo. The resulting crude material was purified via silica gel chromatography to yield the desired product (obs. [M+H] = 431).

### Procedure 8 - Synthesis of Compound A344

Synthesis of Compound A344 followed the scheme, below:

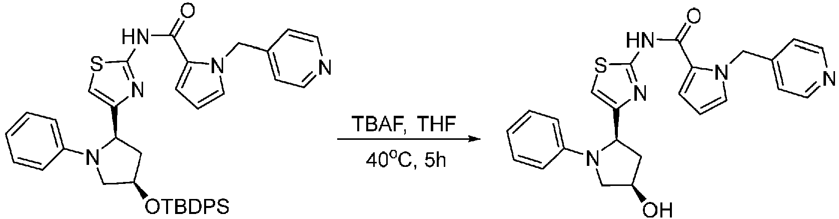

A 50 mL roundbottom flask with stir bar was charged with TBDPS-SM (220 mg, 0.322 mmol, 1.00 equiv), TBAF (841 mg, 3.22 mmol, 10 equiv) and THF (10.0 mL, 0.032 M). The vial was capped and placed in a 40°C bath. The reaction mixture was stirred at 40°C for 5h. The reaction mixture was cooled to room temperature and quenched by H₂O (30 mL). The resulting solution was extracted with (3 × 30 mL) of ethyl acetate and washed with (2x 30 mL) of brine. The organic layer was then dried over Na₂SO₄, filtered and concentrated in vacuo. The resulting crude material was purified via silica gel chromatography & Prep-HPLC or RP column to yield the desired product. (obs. [M+H] = 446).

The following compounds were prepared via a similar method:

| **Compound** | **Observed molecular ion** |
|---|---|
| **A326** | 446 |
| **A344** | 446 |
| **A351** | 446 |

### Procedure 9 - Synthesis of Compound A143

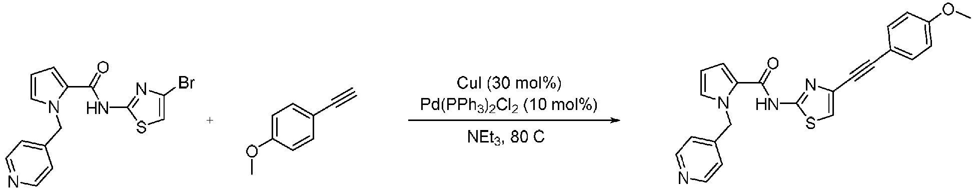

Acetylene (0.082 g, 0.28 mmol, 2.0 eq.), Cul (0.0236 g, 0.12 mmol, 0.3 eq.) and Pd(PPh₃)₂Cl₂ (0.029 g, 0.04 mmol, 0.1 eq.) were added in succession, under Ar, to the degassed solution of starting material (0.150 g, 0.41 mmol, 1.0 eq.) in TEA (1 mL). Reaction was continued at 80°C for 3h. Solution was filtered through Celite and washed with ethyl acetate. Filtrate was evaporated and crude product purified via FCC with hexane/ethyl acetate as eluent. In case of necessity final product was repurified by prep. HPLC

The following compounds were prepared in an analogous manner:

| **Compound** | **Observed molecular ion** |
|---|---|
| **A142** | 415 |
| **A141** | 415 |
| **A144** | 419 |
| **A143** | 415 |

### Procedure 10 - Synthesis of Compound A233

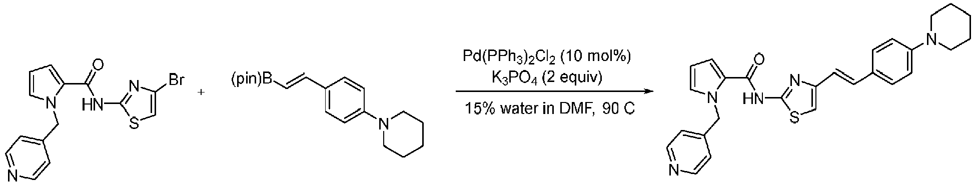

A 20 mL vial with stir bar was charged with boronic ester (70 mg, 0.26 mmol, 1.0 equiv), bromide (93 mg, 0.26 mmol, 1.0 equiv), K₃PO₄ (109 mg, 0.51 mmol, 2.0 equiv) and solution of 15% water in DMF (3.0 mL). The mixture was purged with argon for 5 min. After that time, Pd(PPh₃)₂Cl₂ (18 mg, 0.03 mmol, 0.10 equiv) was added, the vial was capped, purged with argon for 2 min and placed in a 90 C bath for 2 hours. Then reaction mixture was cooled down to the room temperature and palladium residues were filtered off through celite and washed with EtOAc. The resulting filtrate was extracted with water and brine. Organic phase was dried over MgSO₄, filtered and evaporated to dryness. The resulting crude material was purified via preparative HPLC to yield the desired product.

The following compounds were prepared in an analogous manner:

| **Compound** | **Observed molecular ion** |
|---|---|
| **A164** | 388 |
| **A229** | 430 |
| **A237** | 438 |

### Procedure 11 - Synthesis of Compound A180

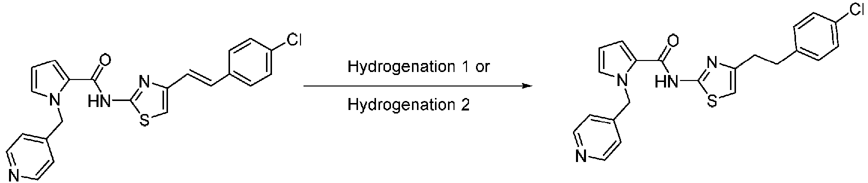

### Hydrogenation 1

Reaction was carried out in H-cube. Conditions: 10% Pd/C , 60°C, 30 bar, THF, dilution 0.05M. The resulting crude material was purified via preparative HPLC to yield the desired product.

The following compounds were prepared in an analogous manner:

| **Compound** | **Observed molecular ion** |
|---|---|
| **A179** | 423 |
| **A178** | 424 |

### Hydrogenation 2

Starting material (0.055 g, 0.13 mmol, 1.0 eq), Pd (0.21g, 10% on activated carbon) were mixed in ethanol. Reaction mixture was flushed with Ar, connected to H₂ ballon (1 atm) and stirred overnight at 40°C. The catalyst was removed by filtration, the filtrate was evaporated and the crude material was purified by preparative HPLC to yield the desired product.

The following compounds were prepared in an analogous manner:

| **Compound** | **Observed molecular ion** |
|---|---|
| **A180** | 423 |
| **A176** | 419 |

### Example 6: Biological Assays

### Dox-Induced PD1-ss-Gluc Assay

Flp-In 293 T-REx^{™} cells were transfected with pcDNA^{™}5/FRT/TO plasmid inserted with cDNA encoding Gaussia Luciferase fused to the 3' end of cDNA encoding PD1 signal sequence plus 10 amino acids (N-MQIPQAPWPVVWAVLQLGWRPGWFLDSPDR-C) (SEQ ID NO: 1). Transfected cells were selected for resistance to the selectable markers Hygromycin and Blasticidin to create a stable cell line that contained the PD1-ss+10aa/Gaussia Luciferase cDNA insert whose expression was regulated under the T-REx^{™} system. The day before assay, cells were trypsinized and plated in 384-well tissue culture plates. The next day, compound dilutions in DMSO/media containing doxycycline were added to the wells and incubated at 37°C, 5% CO₂. 24 hours later, coelenterazine substrate was added to each well and luciferase signal was quantified using Tecan Infinite M1000 Pro for potency determination.

Results for select compounds provided herein are shown in Table 1, below. For chemical structures that include one or more stereoisomers, but are illustrated without indicating stereochemistry, the assay data refers to a mixture of stereoisomers.

### Dox Induced TNFα-FL-Gluc Assay

Flp-In 293 T-REx^{™} cells were transfected with pcDNA^{™}5/FRT/TO plasmid inserted with cDNA encoding Gaussia Luciferase fused to the 3' end of cDNA encoding full length TNFα (amino acids 1-233). Transfected cells were selected for resistance to the selectable markers Hygromycin and Blasticidin to create a stable cell line that contained the TNFα-FL/Gaussia Luciferase cDNA insert whose expression was regulated under the T-REx^{™} system. The day before assay, cells were trypsinized and plated in 384-well tissue culture plates. The next day, compound dilutions in DMSO/media containing doxycycline were added to the wells and incubated at 37°C, 5% CO₂. 24 hours later, coelenterazine substrate was added to each well and luciferase signal was quantified using Tecan Infinite M1000 Pro for potency determination.

Results for select compounds provided herein are shown in Table 1, below. For chemical structures that include one or more stereoisomers, but are illustrated without indicating stereochemistry, the assay data refers to a mixture of stereoisomers.

### Dox-Induced Her3-ss-Gluc Assay

Flp-In 293 T-REx^{™} cells were transfected with pcDNA^{™}5/FRT/TO plasmid inserted with cDNA encoding Gaussia Luciferase fused to the 3' end of cDNA encoding HER3 signal sequence plus 4 amino acids (N- MRANDALQVLGLLFSLARGSEVG-C) (SEQ ID NO: 2). Transfected cells were selected for resistance to the selectable markers Hygromycin and Blasticidin to create a stable cell line that contained the HER3-ss+4aa/Gaussia Luciferase cDNA insert whose expression was regulated under the T-REx^{™} system. The day before assay, cells were trypsinized and plated in 384-well tissue culture plates. The next day, compound dilutions in DMSO/media containing doxycycline were added to the wells and incubated at 37°C, 5% CO₂. 24 hours later, coelenterazine substrate was added to each well and luciferase signal was quantified using Tecan Infinite M1000 Pro for potency determination.

Results for select compounds provided herein are shown in Table 1. For chemical structures that include one or more stereoisomers, but are illustrated without indicating stereochemistry, the assay data refers to a mixture of stereoisomers.

### Dox Induced IL2-FL-Gluc Assay

Flp-In 293 T-REx^{™} cells were transfected with pcDNA^{™}5/FRT/TO plasmid inserted with cDNA encoding Gaussia Luciferase fused to the 3' end of cDNA encoding full length IL-2 (amino acids 1-153). Transfected cells were selected for resistance to the selectable markers Hygromycin and Blasticidin to create a stable cell line that contained the IL-2-FL/Gaussia Luciferase cDNA insert whose expression was regulated under the T-REx^{™} system. The day before assay, cells were trypsinized and plated in 384-well tissue culture plates. The next day, compound dilutions in DMSO/media containing doxycycline were added to the wells and incubated at 37°C, 5% CO₂. 24 hours later, coelenterazine substrate was added to each well and luciferase signal was quantified using Tecan Infinite M1000 Pro for potency determination.

Results for select compounds provided herein are shown in Table 1, below. For chemical structures that include one or more stereoisomers, but are illustrated without indicating stereochemistry, the assay data refers to a mixture of stereoisomers.

### H929 Cell Viability Assay

The human multiple myeloma cell line NCI-H929 was cultured in Advanced RPMI 1640 media (Gibco^{®}) supplemented with 6% fetal bovine serum, 2mM Glutamine, and 1x Penicillin/Streptomycin. On the day of assay, cells were resuspended in RPMI 1640 media supplemented with 10% fetal bovine serum, 2mM Glutamine, and 1x Penicillin/Streptmycin and plated in 384-well tissue culture plates and treated with compound dilutions in DMSO/media. Plates were incubated at 37°C, 5% CO₂ for 48 hours. After 48 hours, Celltiter-Glo^{®} (Promega) was added to each well and luciferase signal was quantified using Tecan Infinite M1000 Pro for cell viability determination.

Results for select compounds provided herein are shown in Table 1, below. For chemical structures that include one or more stereoisomers, but are illustrated without indicating stereochemistry, the assay data refers to a mixture of stereoisomers.

### U266 Cell Viability Assay

The human multiple myeloma cell line U266B1 was cultured in RPMI 1640 media supplemented with 10% fetal bovine serum, 2mM Glutamine, and 1x Penicillin/Streptomycin. Cells were plated in 384-well tissue culture plates and treated with compound dilutions in DMSO/media. Plates were incubated at 37°C, 5% CO₂ for 48 hours. After 48 hours, Celltiter-Glo^{®} (Promega) was added to each well and luciferase signal was quantified using Tecan Infinite M1000 Pro for cell viability determination.

Results for select compounds provided herein are shown in Table 1, below. For chemical structures that include one or more stereoisomers, but are illustrated without indicating stereochemistry, the assay data refers to a mixture of stereoisomers.

**Table 1**

| **Compound ID** | **24hr Dox Inducible PD1ssGluc 293FRT/TO: Mean IC50 (nM)** | **24hr Dox Inducible TNFaFLGluc 293FRT/TO: Mean IC50 (nM)** | **24hr Dox Inducible Her3(ss+4)Glu c 293FRT/TO: Mean IC50 (nM)** | **24hr Dox Inducible IL2FLGluc 293FRTITO: Mean IC50 (nM)** | **48hr H929 Viability Celltiter-Glo: Mean EC50 (nM)** | **48hr U266 Viability Celltiter-Glo: Mean EC50 (nM)** |
|---|---|---|---|---|---|---|
| **A1** | 24 | 401 | 55 | 93 | 1877 | I.A. |
| **A2** | 29 | 391 | 62 | 105 | 1612 | 22304 |
| **A3** | 36 | 580 | 99 | 144 | 2910 | I.A. |
| **A4** | 38 | 377 | 91 | 148 | 2774 | I.A. |
| **A5** | 43 | 810 | 102 | 145 | 2435 | 23887 |
| **A6** | 46 | 541 | 88 | 78 | 2167 | 6225 |
| **A7** | 58 | 808 | 93 | 71 | 3850 | I.A. |
| **A8** | 58 | 420 | 174 | 165 | 3892 | 7869 |
| **A9** | 104 | 1623 | 202 | 197 | 9461 | I.A. |
| **A10** | 105 | 1579 | 195 | 158 | 15609 | I.A. |
| **A11** | 115 | 2392 | 128 | 61 | 8690 | I.A. |
| **A12** | 139 | 1755 | 338 | 566 | 18613 | I.A. |
| **A13** | 148 | 3305 | 382 | 648 | 18783 | I.A. |
| **A14** | 153 | 5107 | 568 | 1048 | I.A. | I.A. |
| **A15** | 169 | 5428 | 318 | 110 | 16591 | I.A. |
| **A16** | 185 | 5013 | 377 | 266 | > 23900 | I.A. |
| **A17** | 191 | 2235 | 560 | 804 | 20766 | I.A. |
| **A18** | 226 | 1353 | 461 | 718 | 15092 | I.A. |
| **A19** | 246 | 8535 | 492 | 529 | I.A. | I.A. |
| **A20** | 248 | 7166 | 510 | 351 | I.A. | I.A. |
| **A21** | 259 | 3676 | 590 | 502 | > 24966 | I.A. |
| **A22** | 271 | 8832 | 503 | 460 | I.A. | I.A. |
| **A23** | 290 | 5472 | 447 | 429 | I.A. | I.A. |
| **A24** | 303 | 11685 | 619 | 477 | I.A. | I.A. |
| **A25** | 314 | 9977 | 692 | 490 | I.A. | I.A. |
| **A26** | 319 | 10684 | 817 | 759 | I.A. | I.A. |
| **A27** | 445 | 9525 | 822 | 320 | I.A. | I.A. |
| **A28** | 462 | 3953 | 752 | 401 | I.A. | I.A. |
| **A29** | 488 | 19613 | 1243 | 935 | I.A. | I.A. |
| **A30** | 552 | 11818 | 809 | 927 | 22722 | I.A. |
| **A31** | 596 | 11814 | 1629 | 1340 | I.A. | I.A. |
| **A32** | 633 | > 20334 | 1331 | 1977 | I.A. | I.A. |
| **A33** | 663 | I.A. | 1623 | 2469 | 8992 | 6888 |
| **A34** | 674 | I.A. | 2419 | 4239 | I.A. | I.A. |
| **A35** | 692 | I.A. | 1938 | 2299 | I.A. | I.A. |
| **A36** | 698 | 20987 | 1137 | 896 | I.A. | I.A. |
| **A37** | 703 | 12119 | 1236 | 1074 | I.A. | I.A. |
| **A38** | 706 | 14775 | 1199 | 1407 | I.A. | I.A. |
| **A39** | 723 | 13069 | 1492 | 1339 | I.A. | I.A. |
| **A40** | 858 | 12581 | 1562 | 1206 | I.A. | I.A. |
| **A41** | 878 | 11999 | 2392 | 1981 | 9523 | I.A. |
| **A42** | 939 | I.A. | 2176 | 3791 | I.A. | I.A. |
| **A43** | 942 | I.A. | 2214 | 2672 | I.A. | I.A. |
| **A44** | 963 | 13290 | 1484 | 837 | > 24839 | I.A. |
| **A45** | 965 | 13802 | 1645 | 869 | I.A. | I.A. |
| **A46** | 1075 | 17825 | 2985 | 890 | I.A. | I.A. |
| **A47** | 1093 | 24952 | 2210 | 3111 | I.A. | I.A. |
| **A48** | 1186 | I.A. | 2689 | 2217 | I.A. | I.A. |
| **A49** | 1211 | 23621 | 2583 | 1891 | I.A. | I.A. |
| **A50** | 1288 | I.A. | 2932 | 1450 | I.A. | I.A. |
| **A51** | 1300 | I.A. | 3423 | 2978 | I.A. | I.A. |
| **A52** | 1357 | I.A. | 2835 | 1946 | I.A. | I.A. |
| **A53** | 1377 | 19100 | 1915 | 1200 | 17330 | I.A. |
| **A54** | 1383 | I.A. | 4048 | 4448 | I.A. | I.A. |
| **A55** | 1386 | I.A. | 3160 | 3990 | I.A. | I.A. |
| **A56** | 1419 | I.A. | 5312 | 6466 | I.A. | I.A. |
| **A57** | 1419 | I.A. | 4203 | 4930 | I.A. | I.A. |
| **A58** | 1503 | I.A. | 3530 | 7057 | I.A. | I.A. |
| **A59** | 1508 | 16636 | 11948 | 3625 | I.A. | I.A. |
| **A60** | 1566 | I.A. | 3751 | 2694 | I.A. | I.A. |
| **A61** | 1672 | I.A. | 3406 | 2381 | I.A. | I.A. |
| **A62** | 1694 | I.A. | 4298 | 5074 | I.A. | I.A. |
| **A63** | 1717 | I.A. | 10594 | I.A. | I.A. | I.A. |
| **A64** | 1732 | I.A. | 3483 | 2724 | 23220 | I.A. |
| **A65** | 1787 | I.A. | 4134 | 11229 | I.A. | I.A. |
| **A66** | 1840 | I.A. | 4152 | 4783 | I.A. | I.A. |
| **A67** | 1880 | I.A. | 3242 | 2083 | I.A. | I.A. |
| **A68** | 2072 | I.A. | 2725 | 2762 | I.A. | I.A. |
| **A69** | 2079 | I.A. | 6216 | 4171 | I.A. | I.A. |
| **A70** | 2229 | I.A. | 9390 | 5674 | I.A. | I.A. |
| **A71** | 2281 | I.A. | 5441 | 3541 | I.A. | I.A. |
| **A72** | 2466 | I.A. | 6107 | 22362 | I.A. | I.A. |
| **A73** | 2472 | I.A. | 7277 | 2023 | I.A. | I.A. |
| **A74** | 2573 | I.A. | 5389 | 4219 | I.A. | I.A. |
| **A75** | 2676 | I.A. | 15290 | 6945 | I.A. | I.A. |
| **A76** | 2725 | I.A. | 12510 | 3989 | I.A. | I.A. |
| **A77** | 3048 | I.A. | 4681 | 3099 | 24963 | I.A. |
| **A78** | 3163 | I.A. | 7876 | 6375 | 20890 | I.A. |
| **A79** | 3175 | I.A. | 5256 | 4146 | 24882 | I.A. |
| **A80** | 4218 | I.A. | 7884 | 3532 | 21927 | I.A. |
| **A81** | 4624 | I.A. | 23015 | I.A. | I.A. | I.A. |
| **A82** | 4663 | I.A. | I.A. | I.A. | I.A. | I.A. |
| **A83** | 5369 | I.A. | I.A. | 4517 | I.A. | I.A. |
| **A84** | 6339 | I.A. | I.A. | 5819 | I.A. | I.A. |
| **A85** | 7540 | I.A. | I.A. | I.A. | I.A. | I.A. |
| **A86** | 7995 | I.A. | I.A. | I.A. | I.A. | I.A. |
| **A87** | 8134 | I.A. | I.A. | 8589 | I.A. | I.A. |
| **A88** | 9653 | I.A. | I.A. | I.A. | I.A. | I.A. |
| **A89** | 9847 | I.A. | I.A. | I.A. | I.A. | I.A. |
| **A90** | 10049 | I.A. | I.A. | I.A. | 16900 | I.A. |
| **A91** | 11947 | I.A. | I.A. | > 21571 | I.A. | I.A. |
| **A92** | 13137 | I.A. | I.A. | I.A. | I.A. | I.A. |
| **A93** | 13225 | I.A. | I.A. | I.A. | I.A. | I.A. |
| **A94** | 14615 | I.A. | I.A. | I.A. | I.A. | I.A. |
| **A95** | 14671 | I.A. | I.A. | I.A. | I.A. | I.A. |
| **A96** | 15672 | I.A. | I.A. | I.A. | I.A. | I.A. |
| **A97** | 18860 | I.A. | I.A. | I.A. | I.A. | I.A. |
| **A98** | 19562 | I.A. | I.A. | I.A. | I.A. | I.A. |
| **A99** | 19876 | I.A. | I.A. | I.A. | I.A. | I.A. |
| **A100** | 24597 | I.A. | I.A. | I.A. | 24583 | I.A. |
| **A101** | I.A. | I.A. | I.A. | I.A. | I.A. | I.A. |
| **A102** | I.A. | I.A. | I.A. | I.A. | I.A. | I.A. |
| **A103** | I.A. | I.A. | I.A. | I.A. | I.A. | I.A. |
| **A104** | I.A. | I.A. | I.A. | I.A. | I.A. | I.A. |
| **A105** | I.A. | I.A. | I.A. | I.A. | I.A. | I.A. |
| **A106** | I.A. | I.A. | I.A. | I.A. | I.A. | I.A. |
| **A107** | I.A. | I.A. | I.A. | I.A. | I.A. | I.A. |
| **A108** | I.A. | I.A. | I.A. | 7270 | I.A. | I.A. |
| **A109** | I.A. | I.A. | I.A. | I.A. | 19462 | I.A. |
| **A110** | I.A. | I.A. | I.A. | I.A. | I.A. | I.A. |
| **A111** | I.A. | I.A. | I.A. | I.A. | I.A. | I.A. |
| **A112** | I.A. | I.A. | I.A. | I.A. | I.A. | I.A. |
| **A113** | I.A. | I.A. | I.A. | I.A. | I.A. | I.A. |
| **A114** | I.A. | I.A. | I.A. | I.A. | I.A. | I.A. |
| **A115** | I.A. | I.A. | I.A. | I.A. | I.A. | I.A. |
| **A116** | I.A. | I.A. | I.A. | I.A. | I.A. | I.A. |
| **A117** | I.A. | I.A. | I.A. | I.A. | I.A. | I.A. |
| **A118** | I.A. | I.A. | I.A. | I.A. | I.A. | I.A. |
| **A119** | I.A. | I.A. | I.A. | I.A. | I.A. | I.A. |
| **A120** | I.A. | I.A. | I.A. | I.A. | I.A. | I.A. |
| **A121** | I.A. | I.A. | I.A. | I.A. | I.A. | I.A. |
| **A122** | I.A. | I.A. | I.A. | I.A. | I.A. | I.A. |
| **A123** | I.A. | I.A. | I.A. | I.A. | I.A. | I.A. |
| **A124** | I.A. | I.A. | I.A. | I.A. | I.A. | I.A. |
| **A125** | I.A. | I.A. | I.A. | I.A. | I.A. | I.A. |
| **A126** | I.A. | I.A. | I.A. | I.A. | I.A. | I.A. |
| **A127** | I.A. | I.A. | I.A. | I.A. | I.A. | I.A. |
| **A128** | I.A. | I.A. | I.A. | I.A. | I.A. | I.A. |
| **A129** | I.A. | I.A. | I.A. | I.A. | I.A. | I.A. |
| **A130** | I.A. | I.A. | I.A. | I.A. | I.A. | |
| **A131** | I.A. | I.A. | I.A. | I.A. | I.A. | I.A. |
| **A132** | 150 | 1205 | 417 | 744 | 8853 | 9337 |
| **A141** | 277 | 1824 | 651 | 1579 | 7720 | 13093 |
| **A142** | 241 | 5410 | 565 | 1050 | I.A. | I.A. |
| **A143** | 89 | 1576 | 423 | 617 | 15198 | > 19801 |
| **A144** | 151 | 905 | 395 | 748 | 6860 | 10419 |
| **A150** | 122 | 14109 | 505 | 1284 | I.A. | I.A. |
| **A151** | 90 | 7132 | 386 | 691 | I.A. | I.A. |
| **A152** | 85 | 1577 | 280 | 854 | 10982 | I.A. |
| **A153** | 902 | I.A. | 1553 | 4071 | I.A. | I.A. |
| **A154** | 694 | 11452 | 2605 | 5419 | I.A. | I.A. |
| **A155** | 232 | 15896 | 2140 | 4371 | I.A. | I.A. |
| **A157** | 9056 | I.A. | 13449 | I.A. | I.A. | I.A. |
| **A158** | 293 | 2419 | 993 | 1474 | 15714 | I.A. |
| **A159** | 247 | 9007 | 948 | 2648 | 5708 | 16244 |
| **A160** | 51 | 1736 | 259 | 374 | 9097 | I.A. |
| **A161** | 134 | 2866 | 523 | 1072 | 5282 | 10879 |
| **A163** | 59 | 2469 | 305 | 652 | 7606 | 24495 |
| **A164** | 98 | 23683 | 8397 | 6770 | 17673 | I.A. |
| **A165** | 1648 | I.A. | 9828 | 10751 | 11086 | I.A. |
| **A166** | 13483 | I.A. | I.A. | I.A. | I.A. | I.A. |
| **A176** | 272 | 2871 | 643 | 1196 | 20801 | I.A. |
| **A178** | 152 | 929 | 367 | 500 | 9271 | 19881 |
| **A179** | 211 | 3584 | 541 | 1172 | 5752 | 10566 |
| **A180** | 57 | 736 | 156 | 281 | 7184 | 6464 |
| **A184** | 448 | 2412 | 807 | 1076 | 11804 | 13340 |
| **A187** | 10403 | I.A. | > 21927 | 11563 | 18316 | I.A. |
| **A190** | 5230 | I.A. | > 18142 | 3437 | I.A. | I.A. |
| **A194** | 280 | 2302 | 600 | 1143 | 6669 | 15986 |
| **A200** | 140 | 1610 | 332 | 251 | 15743 | I.A. |
| **A209** | 232 | 4740 | 578 | 1238 | I.A. | I.A. |
| **A210** | I.A. | I.A. | I.A. | I.A. | I.A. | I.A. |
| **A211** | 10036 | I.A. | I.A. | 8313 | I.A. | I.A. |
| **A212** | 4736 | I.A. | 4203 | 4574 | 2992 | 3758 |
| **A213** | 12086 | I.A. | 4939 | 6049 | 13827 | 17695 |
| **A214** | 4555 | I.A. | 6695 | 6451 | I.A. | I.A. |
| **A215** | 443 | 2492 | 720 | 1248 | 13059 | I.A. |
| **A216** | 318 | 18750 | 1910 | 1560 | I.A. | I.A. |
| **A217** | 245 | 3527 | 425 | 943 | > 21505 | I.A. |
| **A218** | 131 | 3028 | 315 | 779 | 17100 | I.A. |
| **A219** | 48 | 946 | 234 | 443 | 8601 | 13720 |
| **A220** | 86 | 3750 | 329 | 628 | I.A. | I.A. |
| **A221** | I.A. | I.A. | I.A. | I.A. | I.A. | I.A. |
| **A222** | 348 | 7703 | 1803 | 2274 | 19070 | I.A. |
| **A223** | 513 | I.A. | 3123 | 8038 | 22325 | I.A. |
| **A224** | 141 | I.A. | 296 | I.A. | 20548 | I.A. |
| **A225** | 324 | 3837 | 558 | 486 | 8894 | I.A. |
| **A226** | 302 | 7083 | 426 | 320 | 11938 | I.A. |
| **A227** | 53 | 1895 | 379 | 625 | 8946 | > 21399 |
| **A228** | 335 | 3593 | 730 | 933 | 9252 | I.A. |
| **A229** | 66 | 4585 | 568 | 1053 | 24853 | I.A. |
| **A230** | 1030 | I.A. | I.A. | I.A. | 12200 | I.A. |
| **A231** | 506 | 11158 | I.A. | 7068 | 16805 | I.A. |
| **A232** | 2992 | 21463 | 14886 | 8603 | 13366 | 19711.9 |
| **A233** | 56 | 2223 | 304 | 633 | 5786 | I.A. |
| **A234** | 63 | I.A. | 216 | 341 | 4487 | I.A. |
| **A235** | 63 | I.A. | 145 | 237 | 4150 | I.A. |
| **A236** | 87 | 3239 | 535 | 1009 | 7704 | I.A. |
| **A237** | 288 | I.A. | 1119 | 1869 | 18678 | I.A. |
| **A238** | 643 | 18327 | 1569 | 1199 | I.A. | I.A. |
| **A239** | 1508 | I.A. | I.A. | I.A. | I.A. | I.A. |
| **A240** | 55 | 7787 | 271 | 445 | 11770 | I.A. |
| **A241** | 27 | 1315 | 179 | 324 | 8366 | I.A. |
| **A242** | 281 | I.A. | 596 | I.A. | I.A. | I.A. |
| **A243** | 178 | 2361 | 199 | 136 | 14831 | I.A. |
| **A244** | 81 | 1434 | 225 | 648 | 17677 | 17388 |
| **A245** | 3181 | 22193 | I.A. | 14097 | 9057 | 17491 |
| **A246** | 526 | I.A. | 21868 | I.A. | 14039 | 10279 |
| **A247** | 146 | 2322 | 377 | 788 | I.A. | I.A. |
| **A248** | 38 | 1505 | 152 | 211 | I.A. | I.A. |
| **A249** | 163 | 1408 | 545 | 881 | 19865 | 14445 |
| **A266** | 5275 | I.A. | 15664 | 7395 | I.A. | I.A. |
| **A251** | 125 | 1418 | 344 | 549 | 11677 | I.A. |
| **A252** | 530 | 6928 | 1130 | 1281 | 13981 | I.A. |
| **A253** | 220 | 4197 | 672 | 1577 | 10753 | 15630 |
| **A254** | 74 | 1528 | 189 | 554 | 4096 | 4134 |
| **A255** | 102 | 1329 | 250 | 477 | 7209 | 18137 |
| **A256** | 2154 | I.A. | 4042 | 2866 | I.A. | I.A. |
| **A257** | 1880 | 24844 | 4007 | 3628 | I.A. | I.A. |
| **A258** | 701 | I.A. | 6400 | 14529 | 23776 | I.A. |
| **A259** | 516 | I.A. | 7112 | 10534 | I.A. | I.A. |
| **A260** | 478 | 4612 | 974 | 950 | 10916 | I.A. |
| **A261** | 2247 | I.A. | 2020 | I.A. | I.A. | I.A. |
| **A262** | 1461 | 24182 | 3142 | 3121 | I.A. | I.A. |
| **A263** | 381 | 5222 | 958 | 1042 | 21543 | I.A. |
| **A264** | I.A. | I.A. | I.A. | I.A. | I.A. | I.A. |
| **A265** | 9061 | I.A. | I.A. | 7301 | I.A. | I.A. |
| **A266** | 6254 | I.A. | 9004 | I.A. | I.A. | I.A. |
| **A267** | 15392 | I.A. | I.A. | I.A. | I.A. | I.A. |
| **A268** | 52 | 821 | 129 | 291 | 7293 | I.A. |
| **A269** | 13 | 20294 | 1574 | 2922 | > 21583 | I.A. |
| **A270** | 161 | 1736 | 463 | 599 | 10427 | I.A. |
| **A271** | 316 | 2871 | 912 | 951 | 15481 | I.A. |
| **A272** | 261 | I.A. | 1723 | I.A. | I.A. | I.A. |
| **A273** | I.A. | I.A. | I.A. | I.A. | I.A. | I.A. |
| **A274** | 363 | 22284 | 2549 | 3743 | 23859 | I.A. |
| **A275** | 15 | 147 | 32 | 29 | 1471 | I.A. |
| **A276** | 287 | 2471 | 859 | 1498 | 19371 | > 25825 |
| **A277** | 325 | 4062 | 819 | 769 | 18670 | I.A. |
| **A278** | 538 | 4997 | 1131 | 1270 | > 23903 | I.A. |
| **A279** | 1135 | I.A. | 4201 | 4968 | I.A. | I.A. |
| **A280** | 12714 | I.A. | I.A. | I.A. | I.A. | I.A. |
| **A281** | 244 | 1949 | 817 | 1157 | 11028 | I.A. |
| **A282** | 324 | 4660 | 864 | 1505 | 7076 | 12488 |
| **A283** | 1347 | 7181 | 3073 | 3865 | 22965 | I.A. |
| **A284** | 767 | 4072 | 1241 | 2316 | 4920 | 6104 |
| **A285** | 1228 | 16138 | 1836 | 2861 | 17813 | 16722 |
| **A286** | 1283 | 6173 | 2423 | 3934 | 9574 | 15733 |
| **A287** | 88 | 1155 | 181 | 376 | 5058 | I.A. |
| **A288** | 440 | 3028 | 1261 | 1537 | 23925 | I.A. |
| **A289** | 201 | 3610 | 512 | 856 | 22030 | I.A. |
| **A290** | 261 | 4023 | 648 | 1401 | 15162 | 20793 |
| **A291** | 918 | I.A. | 5277 | 6718 | I.A. | 22471 |
| **A292** | 211 | 2512 | 471 | 835 | 19006 | I.A. |
| **A293** | 367 | 3647 | 632 | 619 | 11427 | I.A. |
| **A294** | 499 | 4443 | 1052 | 939 | I.A. | I.A. |
| **A295** | 5 | 3563 | 692 | 846 | 18308 | 18864 |
| **A296** | 655 | 10290 | 1207 | 1146 | I.A. | I.A. |
| **A297** | 66 | 2823 | 251 | 687 | I.A. | I.A. |
| **A298** | 260 | 1665 | 482 | 450 | 13470 | I.A. |
| **A299** | 7 | I.A. | 214 | 219 | I.A. | I.A. |
| **A300** | 328 | 2417 | 589 | 450 | 10475 | I.A. |
| **A301** | 43 | 242 | 54 | 64 | 2769 | I.A. |
| **A302** | 1978 | I.A. | 6167 | 10583 | I.A. | I.A. |
| **A303** | 104 | 554 | 125 | 69 | 4504 | I.A. |
| **A304** | 20 | 236 | 108 | 178 | 6272 | 14342 |
| **A305** | 196 | 2182 | 414 | 657 | 12398 | I.A. |
| **A306** | 184 | 1790 | 495 | 489 | 11172 | I.A. |
| **A307** | 679 | I.A. | 8812 | I.A. | I.A. | I.A. |
| **A308** | 879 | 13504 | 1687 | 2369 | 17207 | I.A. |
| **A309** | 24 | 721 | 71 | 199 | 6109 | 14751 |
| **A310** | 2121 | 24571 | 5439 | 6992 | I.A. | I.A. |
| **A311** | 175 | 3333 | 510 | 874 | 6023 | 6225 |
| **A312** | 197 | 4132 | 646 | 1140 | I.A. | I.A. |
| **A313** | 3857 | I.A. | 7898 | 6361 | I.A. | I.A. |
| **A314** | 47 | 1466 | 145 | 424 | 9984 | 15995 |
| **A315** | 253 | 8030 | 827 | 1418 | I.A. | I.A. |
| **A316** | 117 | 3166 | 313 | 762 | I.A. | I.A. |
| **A317** | 25 | 427 | 64 | 57 | 2543 | I.A. |
| **A318** | 49 | 817 | 97 | 184 | 6044 | I.A. |
| **A319** | 1312 | 16038 | 2722 | 1845 | I.A. | I.A. |
| **A320** | 10354 | I.A. | I.A. | I.A. | I.A. | I.A. |
| **A321** | 215 | 4169 | 902 | 948 | 9727 | I.A. |
| **A322** | 3 | 14 | 9 | 9 | 315 | I.A. |
| **A323** | 7 | 11 | 13 | 10 | 118 | I.A. |
| **A324** | 289 | 3013 | 582 | 475 | 11328 | I.A. |
| **A325** | 26 | 206 | 46 | 58 | 3622 | I.A. |
| **A326** | 59 | 308 | 201 | 126 | 4999 | I.A. |
| **A327** | 16 | 4487 | 469 | 999 | 15077 | I.A. |
| **A328** | 9 | 105 | 12 | 14 | 381 | 16540 |
| **A329** | 202 | 3163 | 342 | 739 | 22796 | I.A. |
| **A330** | 218 | 3924 | 475 | 1023 | 19250 | I.A. |
| **A331** | 64 | 423 | 82 | 178 | 4719 | I.A. |
| **A332** | 64 | 516 | 97 | 82 | 4292 | I.A. |
| **A333** | 21 | 165 | 42 | 27 | 1480 | I.A. |
| **A334** | 80 | 775 | 138 | 110 | 6472 | 9048 |
| **A335** | 130 | 3009 | 411 | 752 | 11424 | I.A. |
| **A336** | 374 | 1347 | 835 | 334 | 17862 | I.A. |
| **A337** | 190 | 2849 | 548 | 379 | 21456 | I.A. |
| **A338** | 205 | 1853 | 264 | 345 | 14844 | I.A. |
| **A339** | 18 | 226 | 29 | 13 | 1312 | I.A. |
| **A340** | I.A. | I.A. | I.A. | I.A. | I.A. | I.A. |
| **A341** | 47 | I.A. | I.A. | I.A. | I.A. | I.A. |
| **A342** | 68 | I.A. | 952 | 1396 | 23747 | I.A. |
| **A343** | 17 | 3622 | 492 | 1149 | 7178 | 15932 |
| **A344** | 971 | 8859 | 2027 | 1348 | I.A. | I.A. |
| **A345** | 11 | 100 | 22 | 23 | 720 | I.A. |
| **A346** | 17 | 92 | 22 | 20 | 972 | I.A. |
| **A347** | 42 | 13107 | 6013 | 9581 | 23803 | 20318 |
| **A348** | I.A. | I.A. | I.A. | I.A. | 16970 | 18342 |
| **A349** | 364 | 6624 | 1262 | 1700 | 12532 | I.A. |
| **A350** | 707 | 14883 | 2394 | I.A. | I.A. | I.A. |
| **A351** | 167 | 954 | 450 | 259 | 7502 | I.A. |
| **A352** | 8 | 62 | 19 | 16 | 452 | I.A. |
| **A353** | 5 | 13 | 12 | 11 | 303 | I.A. |
| **A354** | 6 | 54 | 16 | 21 | 901 | I.A. |
| **A355** | 524 | 9744 | 3810 | 11319 | 22265 | 17198 |
| **A356** | 35 | 14435 | 5412 | I.A. | I.A. | 23817 |
| **A357** | 12 | 7478 | 2306 | 9073 | 10744 | 17903 |
| **A358** | 29 | 3572 | 443 | 765 | 17872 | I.A. |
| **A359** | 11 | 83 | 13 | 15 | 382 | I.A. |
| **Ref. B1** | 1889 | I.A. | 4319 | 6285 | I.A. | I.A. |
| **B2** | 1974 | I.A. | 12125 | 8420 | I.A. | I.A. |
| **Ref. B3** | 2841 | I.A. | 4240 | 2925 | I.A. | I.A. |
| **Ref. B4** | 3015 | I.A. | 11210 | 22501 | I.A. | I.A. |
| **Ref. B5** | 3238 | I.A. | 12572 | 10554 | I.A. | I.A. |
| **Ref. B6** | 6655 | I.A. | I.A. | I.A. | I.A. | I.A. |
| **Ref. B7** | 9074 | I.A. | 19959 | 24269 | I.A. | I.A. |
| **Ref. B8** | 9099 | I.A. | I.A. | I.A. | I.A. | I.A. |
| **Ref. B9** | 9426 | I.A. | I.A. | I.A. | I.A. | I.A. |
| **Ref. B10** | 11853 | I.A. | I.A. | I.A. | I.A. | I.A. |
| **Ref. B11** | 22747 | I.A. | 5565 | I.A. | I.A. | I.A. |
| **Ref. B12** | I.A. | I.A. | I.A. | I.A. | I.A. | I.A. |
| **Ref. B13** | I.A. | I.A. | I.A. | I.A. | I.A. | I.A. |
| **Ref. B14** | I.A. | I.A. | I.A. | I.A. | I.A. | I.A. |
| **Ref. B15** | I.A. | I.A. | I.A. | I.A. | I.A. | I.A. |
| **Ref. B16** | I.A. | I.A. | I.A. | I.A. | I.A. | I.A. |
| **Ref. B17** | I.A. | I.A. | I.A. | I.A. | I.A. | I.A. |
| **Ref. B18** | I.A. | I.A. | I.A. | I.A. | I.A. | I.A. |
| **Ref. B19** | I.A. | I.A. | I.A. | I.A. | I.A. | I.A. |
| **Ref. B20** | I.A. | I.A. | I.A. | I.A. | I.A. | I.A. |
| **Ref. B21** | I.A. | I.A. | I.A. | I.A. | I.A. | I.A. |
| **Ref. B22** | I.A. | I.A. | I.A. | I.A. | I.A. | I.A. |
| **Ref. B23** | I.A. | I.A. | I.A. | I.A. | I.A. | I.A. |
| **Ref. B24** | I.A. | I.A. | I.A. | I.A. | I.A. | I.A. |
| **Ref. B25** | I.A. | I.A. | I.A. | I.A. | I.A. | I.A. |
| **Ref. B26** | I.A. | I.A. | I.A. | I.A. | I.A. | I.A. |
| **Ref. B27** | I.A. | I.A. | I.A. | I.A. | I.A. | I.A. |
| **Ref. B28** | I.A. | I.A. | NT | I.A. | 23450 | I.A. |
| **Ref. B29** | I.A. | I.A. | NT | I.A. | I.A. | I.A. |

| | | | | | | |
|---|---|---|---|---|---|---|
| I.A. indicates IC50 > 25 µM; NT indicates not tested. | | | | | | |

## Claims

1. A compound having a structure of Formula (III): wherein: 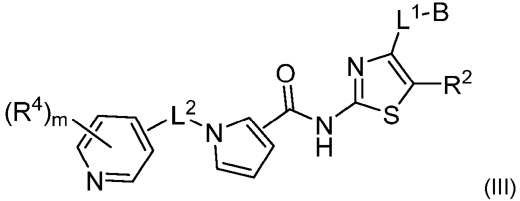
L¹ is a bond, C₁₋₆alkylene, or 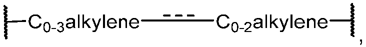
wherein - - - indicates a double bond, a triple bond, or a fused cyclopropyl;
B is C₀₋₃alkylene-X;
X is an aromatic or nonaromatic C₄₋₁₀carbocycle, or an aromatic or nonaromatic 4-10 membered heterocycle having 1-3 ring heteroatoms selected from N, O, and S;
X is substituted with 1-3 G;
Each G is independently selected from the group consisting of halo, OH, =O, CN, NO₂, N(R^{N})₂, N(R^{N})C(O)C₁₋₃alkyl, C₁₋₃alkyl, C₁₋₃alkoxy, C₁₋₃ haloalkyl, C₁₋₃ haloalkoxy, C(O)C₁₋₃alkyl, S(O₂)-Z, C(O)-Z, C(O)N(R^{N})₂, silyl ether, and [O]₀₋₁-C₀₋₃alkylene-Z;
each R^{N} independently is H or C₁₋₄alkyl;
Z is aromatic or nonaromatic C₃₋₁₀carbocycle, or aromatic or nonaromatic 4-10 membered heterocycle having 1-3 heteroatoms selected from the group consisting of N, O, and S;
Z is optionally substituted with 1-3 E; and,
each E independently is selected from C₁₋₃alkyl, C₁₋₃alkoxy, =O, C₁₋₃haloalkoxy, CN, and halo
R² is H;
L² is C₀₋₃alkylene;
m is 0 to 2; and
each R⁴ independently is C₁₋₃alkyl, C₂₋₃alkynyl, C₁₋₃haloalkyl, C₁₋₃alkoxy, halo, or NHC₁₋₃alkylene-aryl;
or a pharmaceutically acceptable salt thereof,
wherein the compound or salt thereof is not N-[4-[(3-methyl-1-piperidinyl)methyl]2-thiazolyl)-1-(4-pyridinylmethyl)-1H-pyrrole-2-carboxamide.

2. The compound or salt of claim 1, wherein L¹ is a bond or is 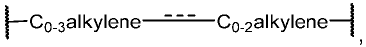 and - - - indicates a double bond.

3. The compound or salt of any one of claims 1-2, wherein X is pyrrolidinyl, cyclobutyl, cyclopentyl, cyclohexyl, cyclohexenyl, phenyl, piperidinyl, pyridinyl, piperazinyl, tetrahydrofuranyl, furanyl, tetrahydropyranyl, quinolinyl, morpholinyl, pyrrolidonyl, pyrimidinyl, pyridazinyl, indenyl, dihydroindenyl, dihydrobenzofuranyl, chromanyl, isochromanyl, dihydroisoquinolinyl, or indolyl.

4. The compound or salt of claim 1, wherein L¹-B is selected from the group consisting of:

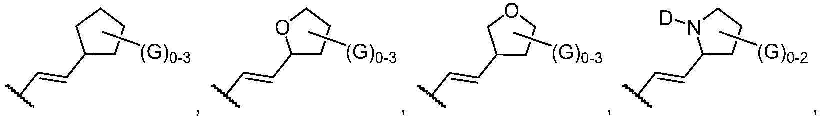

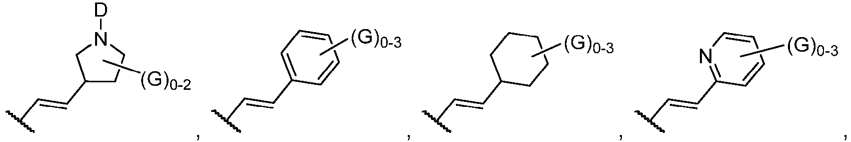

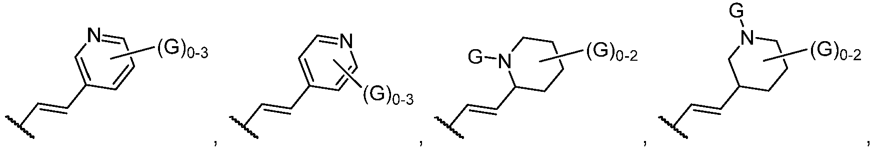

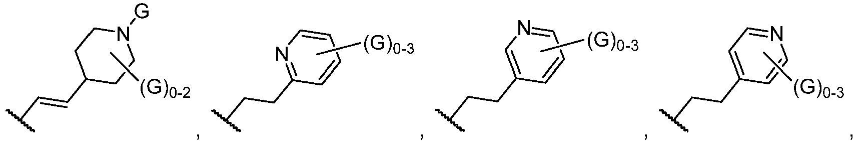

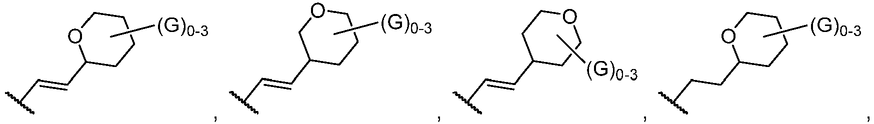

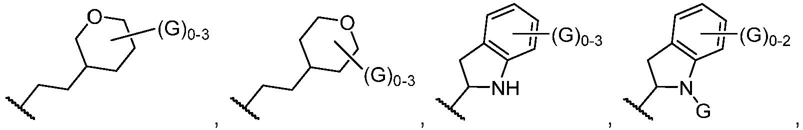

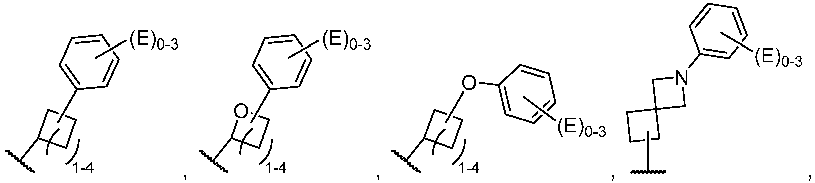

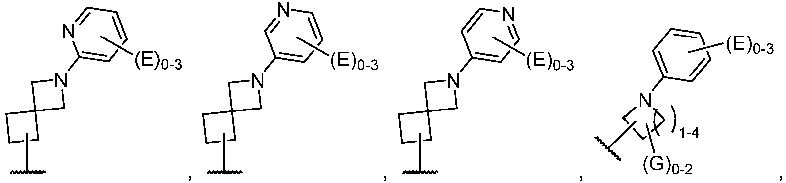

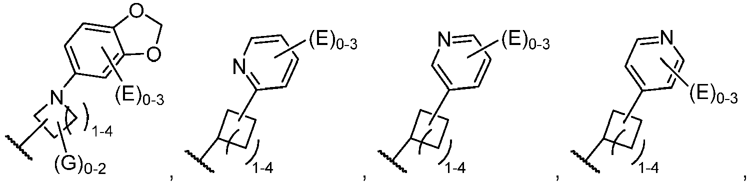

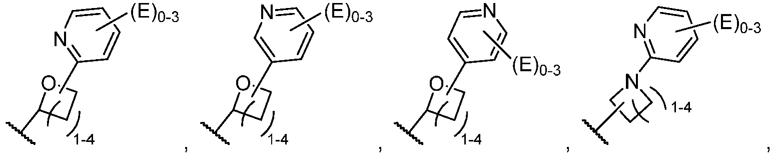

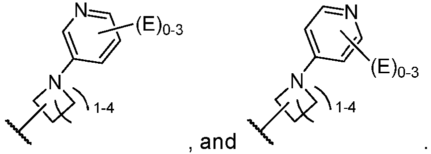

5. The compound or salt of claim 1, wherein L¹-B is selected from the group consisting of:.

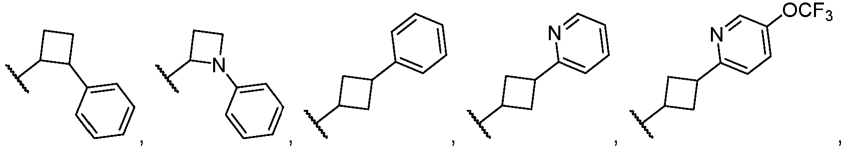

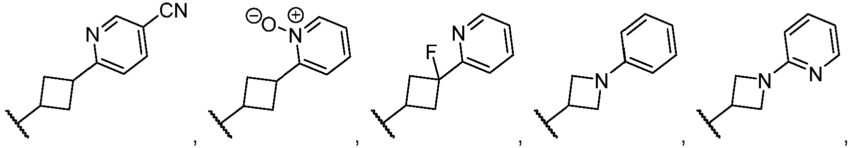

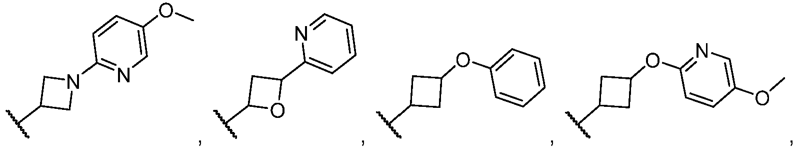

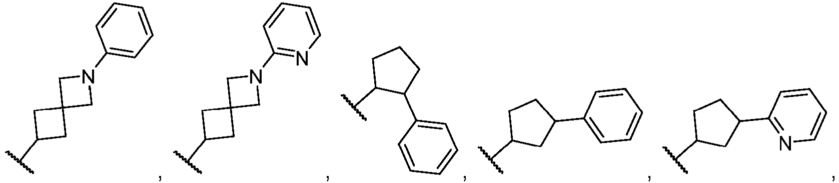

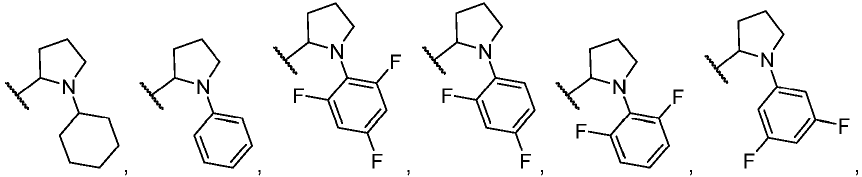

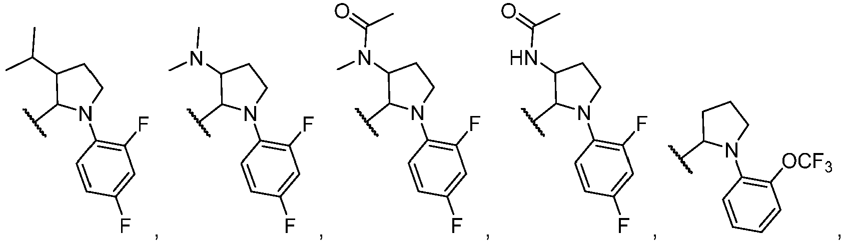

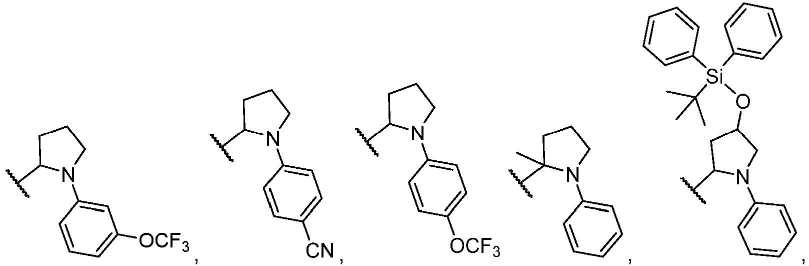

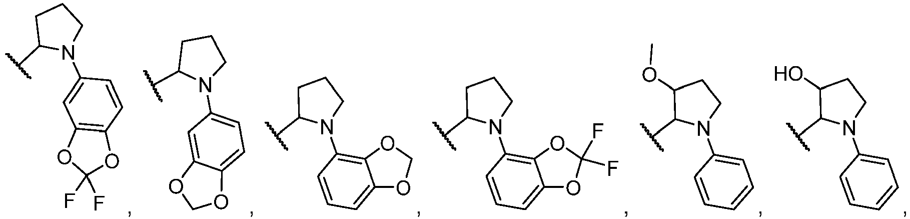

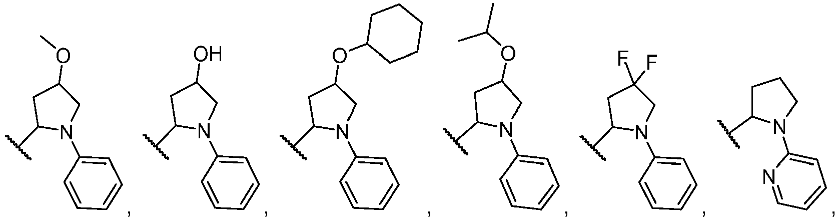

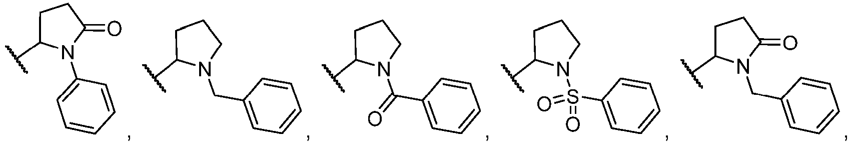

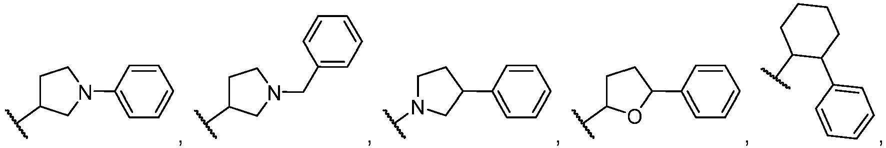

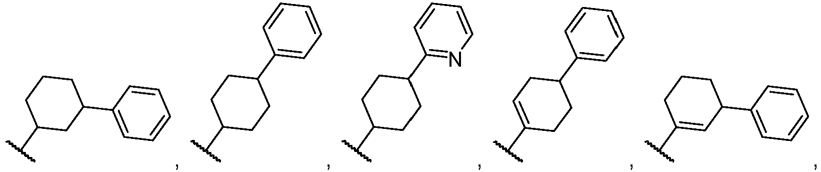

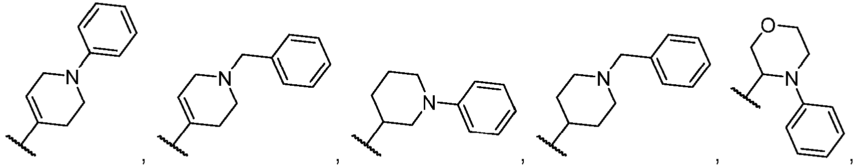

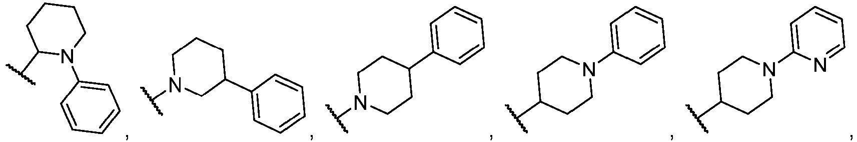

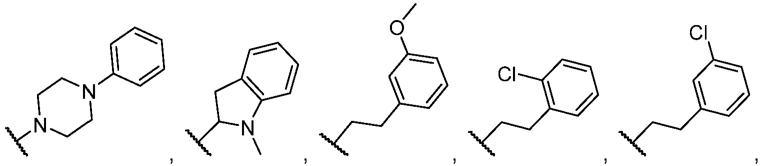

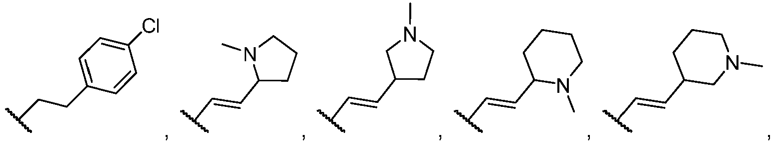

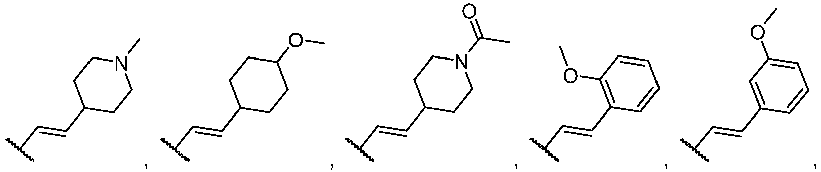

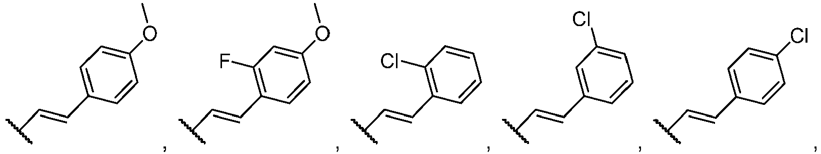

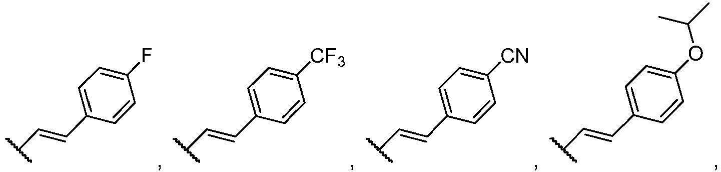

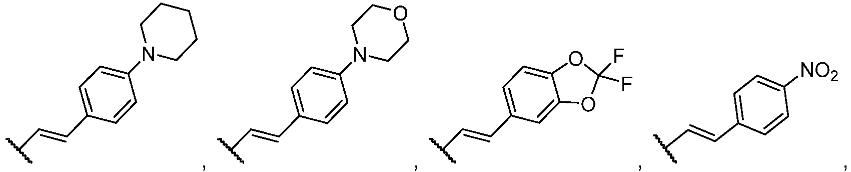

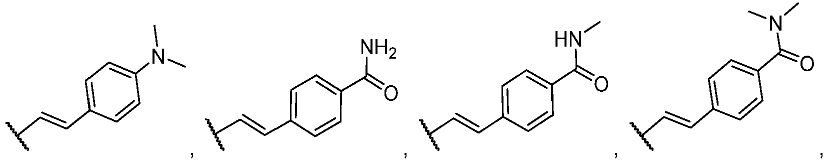

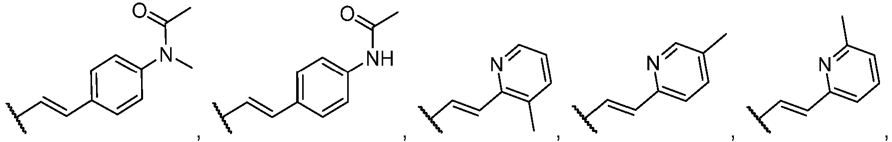

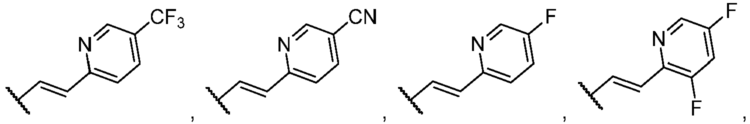

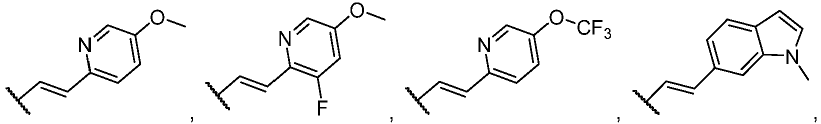

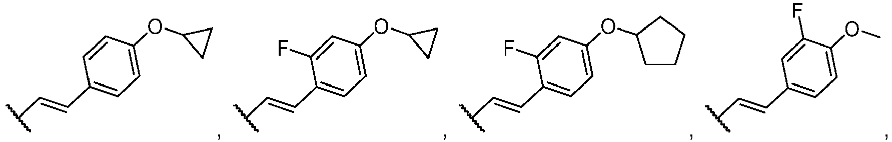

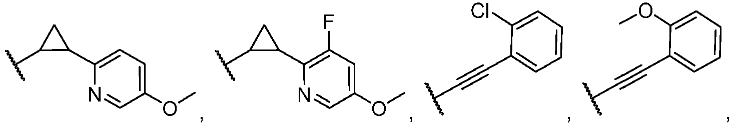

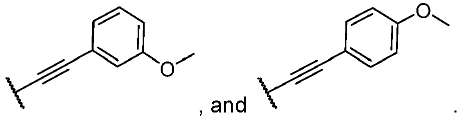

6. The compound or salt of any one of claims 1-5, wherein L² is C₁alkylene.

7. The compound or salt of any one of claims 1-6, wherein m is 1 or 2.

8. The compound or salt of claim 1, having the structure of Formula (IIIA): 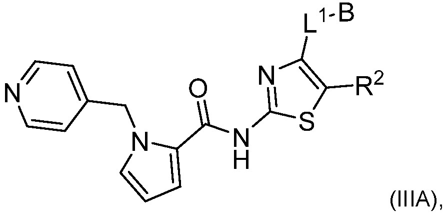 and wherein L¹-B is selected from the group consisting of:

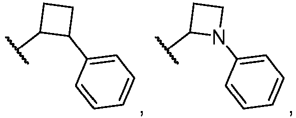

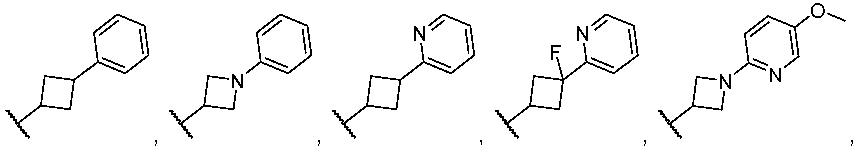

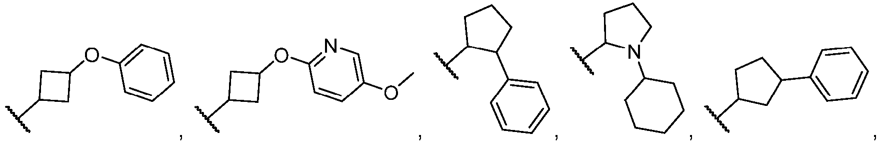

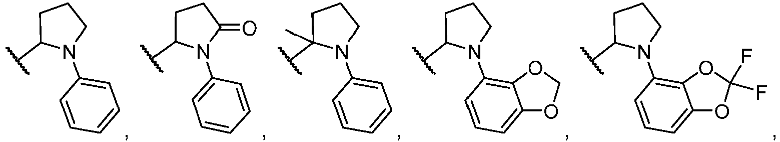

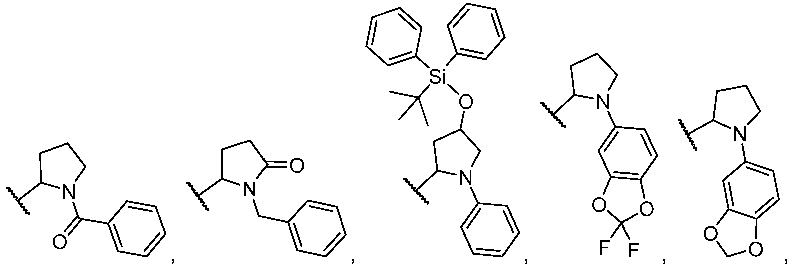

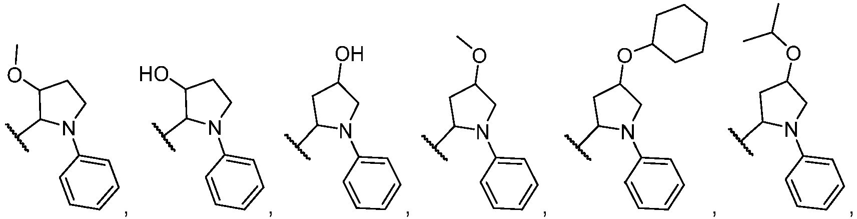

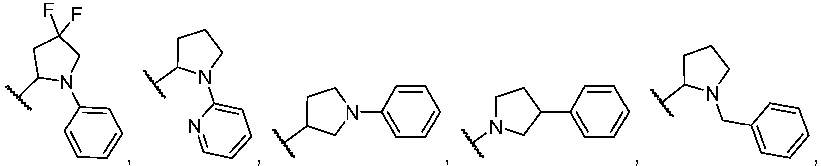

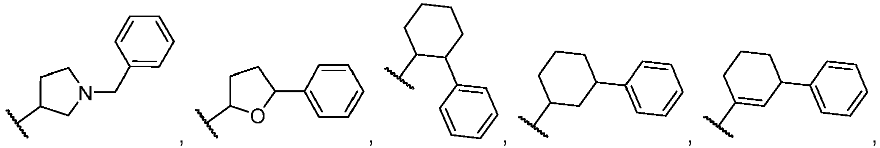

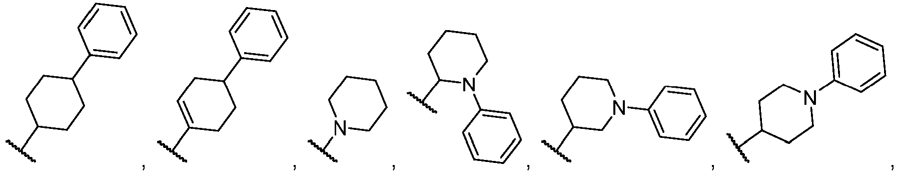

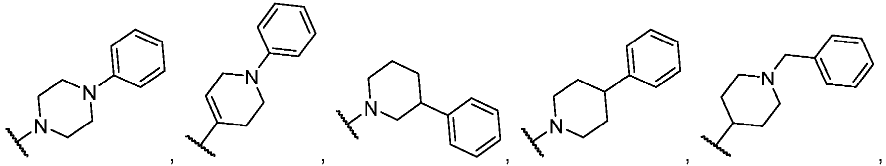

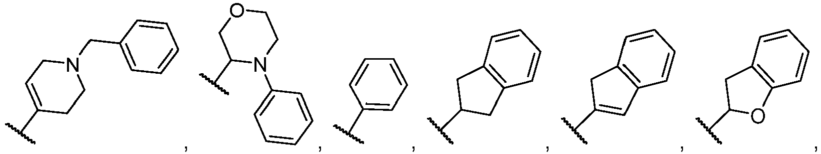

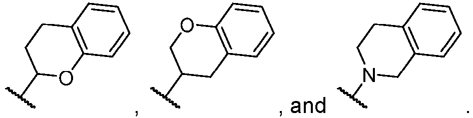

9. The compound or salt of claim 1, having the structure of Formula (IIIA): 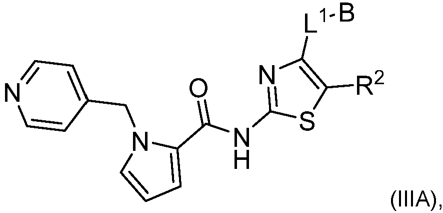 and wherein L¹-B is selected from the group consisting of:

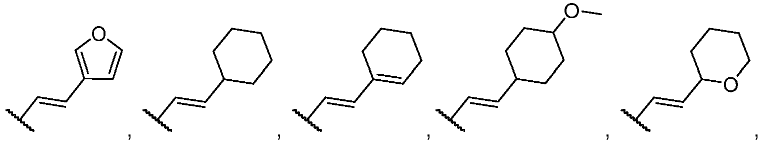

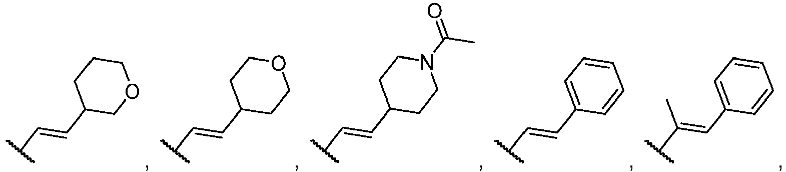

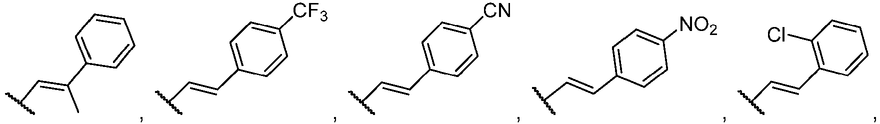

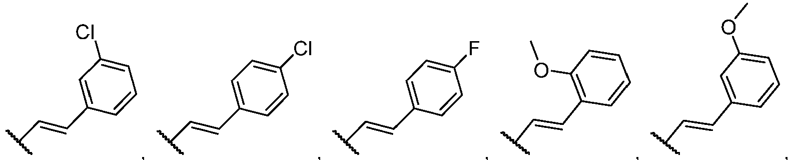

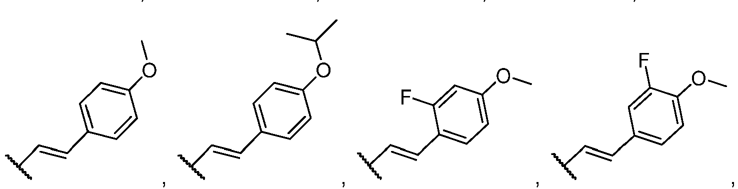

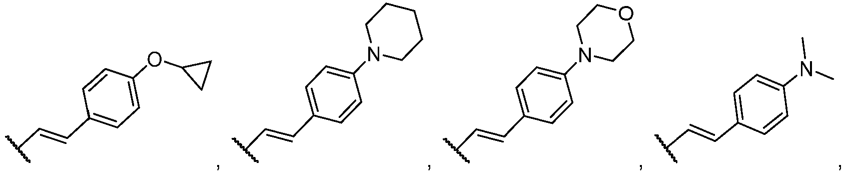

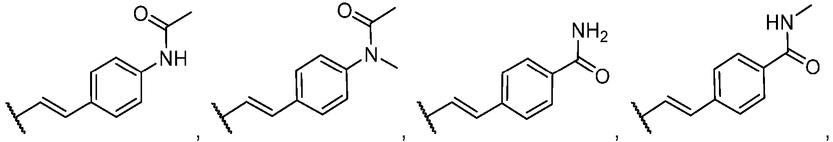

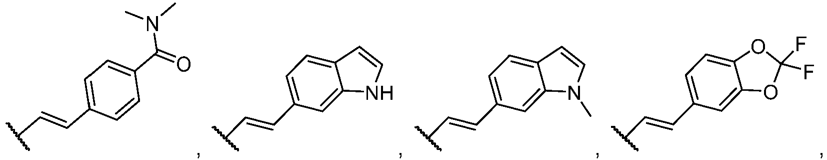

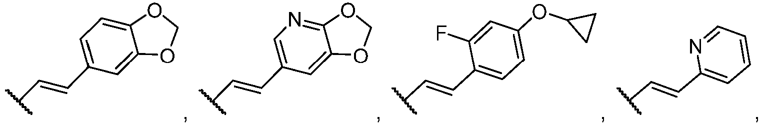

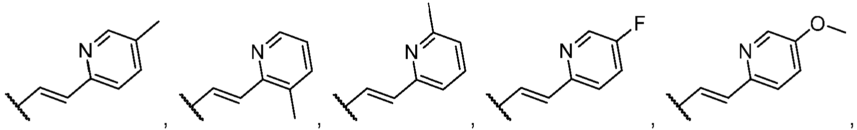

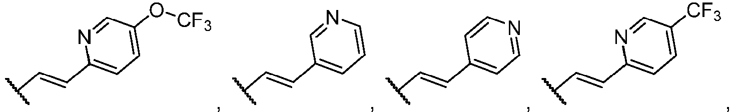

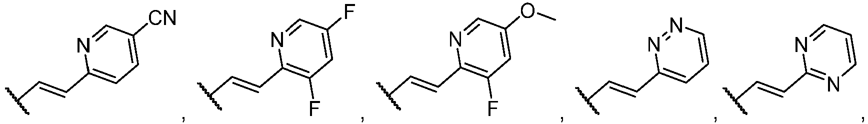

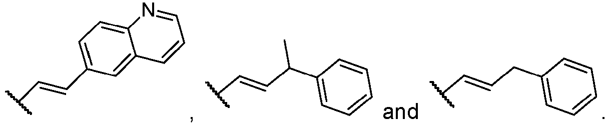

10. The compound or salt of claim 1, wherein the compound or salt is selected from the group consisting of:

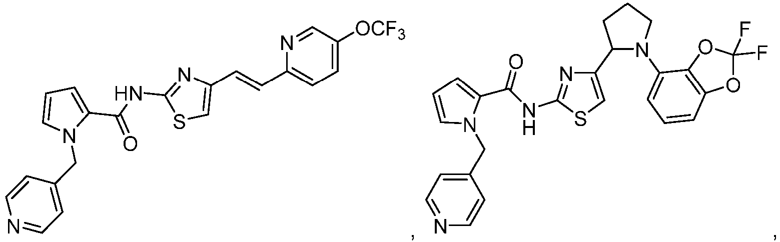

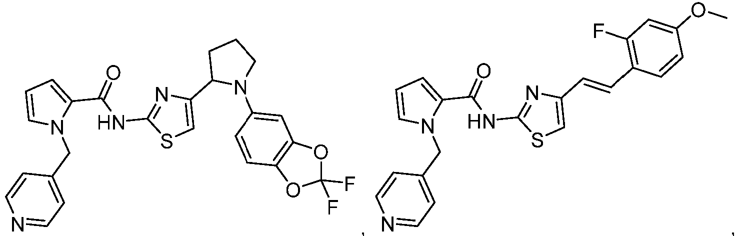

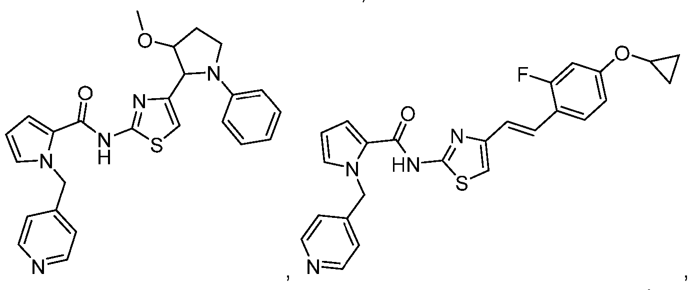

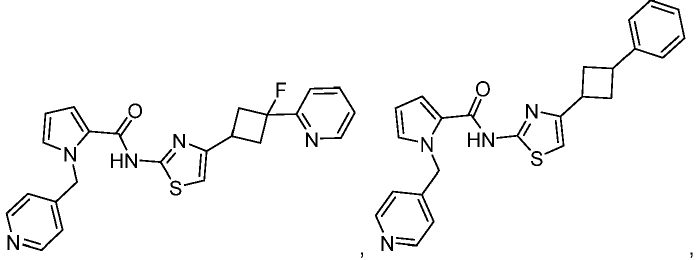

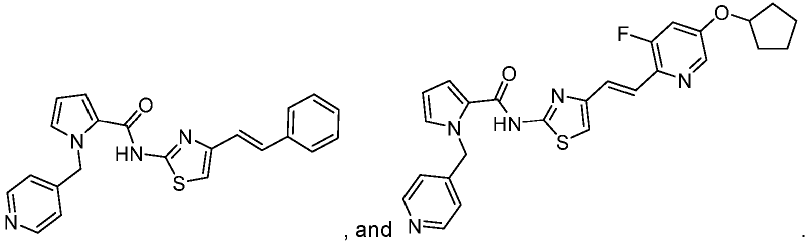

11. A compound listed in Table A, or a pharmaceutically salt thereof.
**Table A**
| | |
|---|---|
| **A1** | 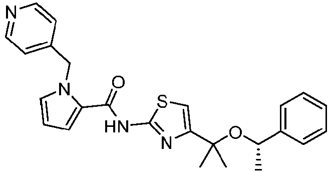 |
| **A2** | 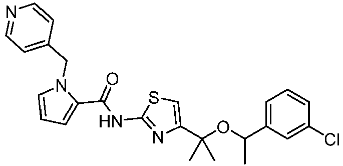 |
| **A3** | 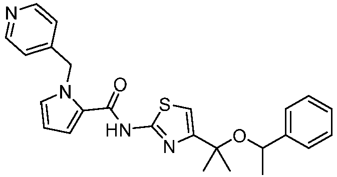 |
| **A4** | 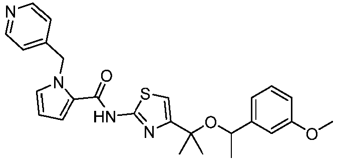 |
| **A5** | 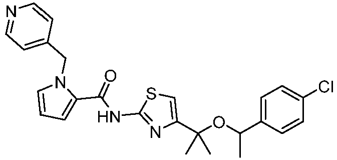 |
| **A6** | 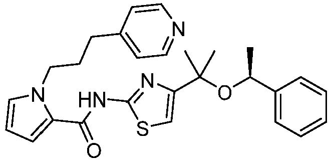 |
| **A7** | 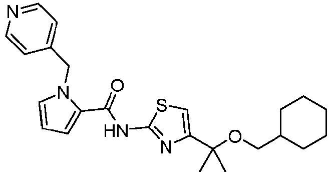 |
| **A8** | 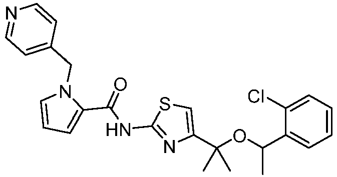 |
| **A9** | 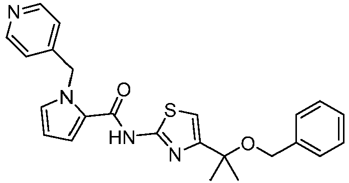 |
| **A10** | 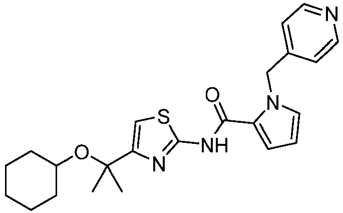 |
| **A11** | 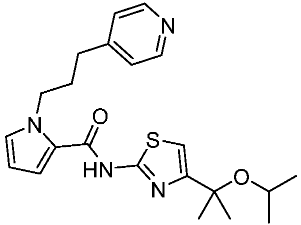 |
| **A12** | 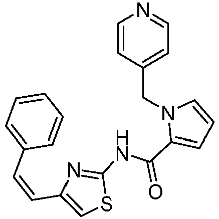 |
| **A13** | 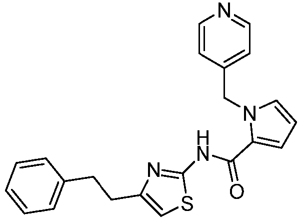 |
| **A14** | 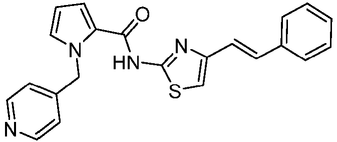 |
| **A15** | 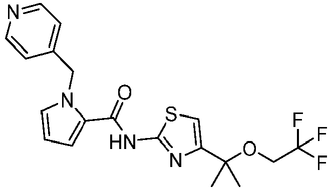 |
| **A16** | 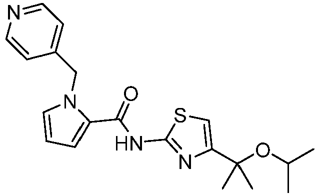 |
| **A17** | 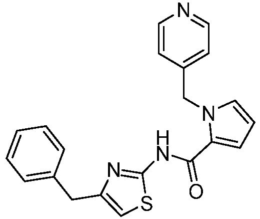 |
| **A18** | 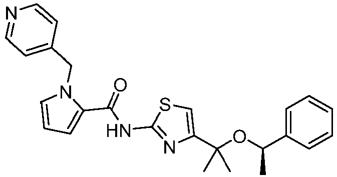 |
| **A19** | 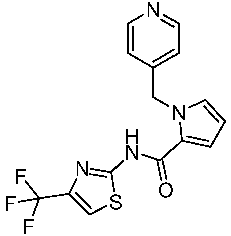 |
| **A20** | 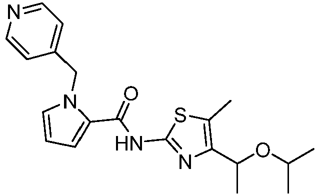 |
| **A21** | 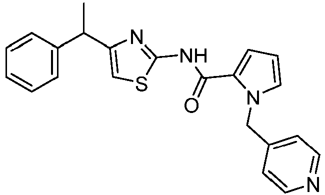 |
| **A22** | 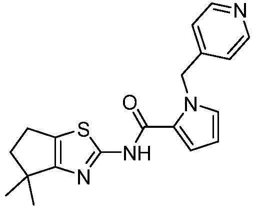 |
| **A23** | 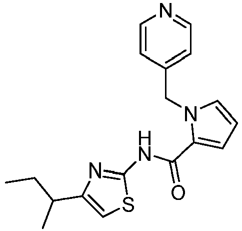 |
| **A24** | 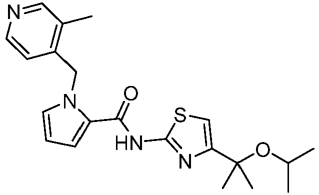 |
| **A25** | 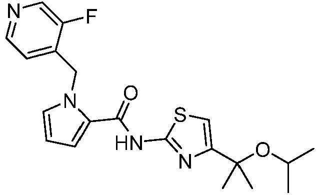 |
| **A26** | 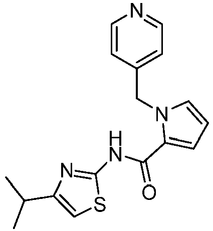 |
| **A27** | 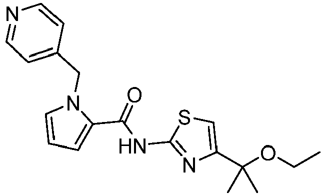 |
| **A28** | 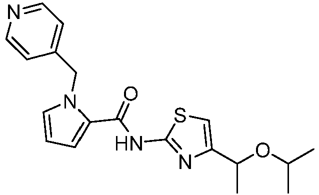 |
| **A29** | 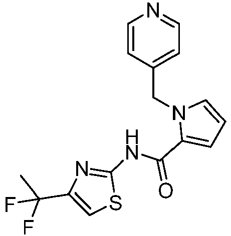 |
| **A30** | 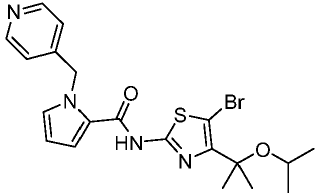 |
| **A31** | 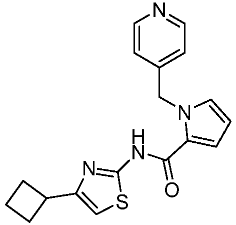 |
| **A32** | 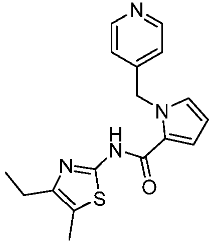 |
| **A33** | 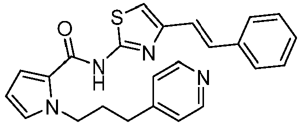 |
| **A34** | 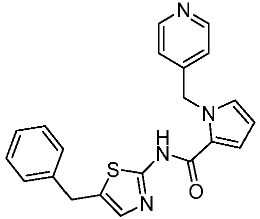 |
| **A35** | 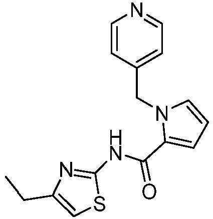 |
| **A36** | 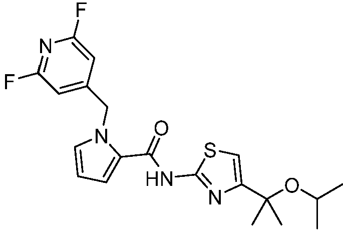 |
| **A37** | 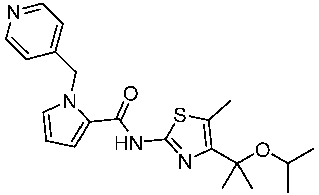 |
| **A38** | 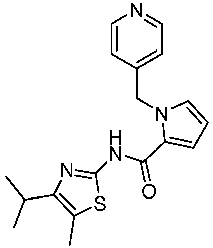 |
| **A39** | 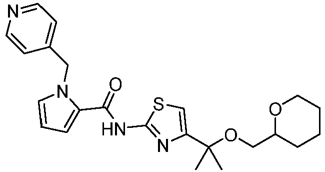 |
| **A40** | 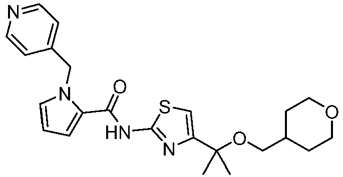 |
| **A41** | 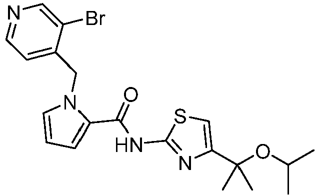 |
| **A42** | 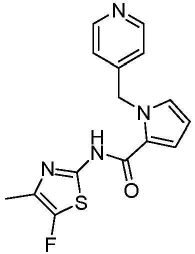 |
| **A43** | 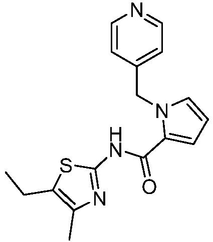 |
| **A44** | 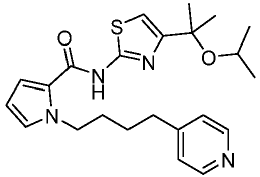 |
| **A45** | 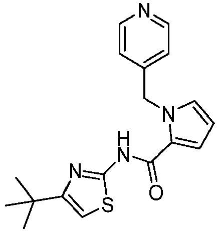 |
| **A46** | 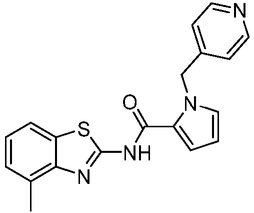 |
| **A47** | 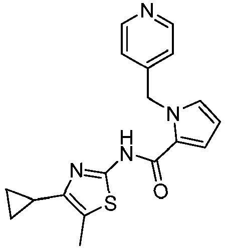 |
| **A48** | 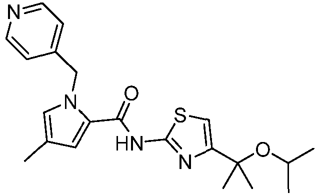 |
| **A49** | 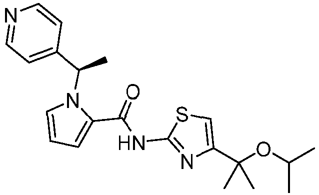 |
| **A50** | 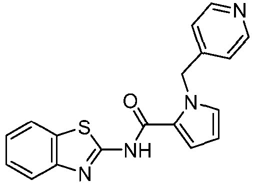 |
| **A51** | 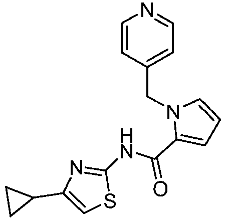 |
| **A52** | 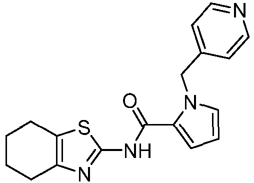 |
| **A53** | 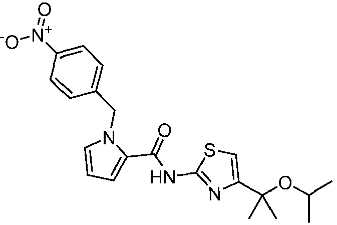 |
| **A54** | 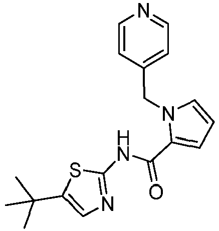 |
| **A55** | 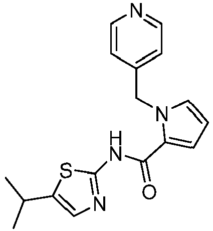 |
| **A56** | 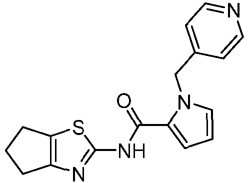 |
| **A57** | 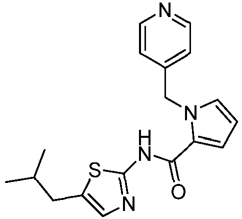 |
| **A58** | 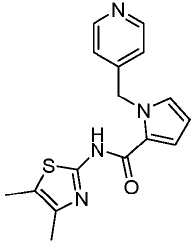 |
| **A59** | 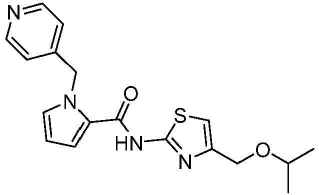 |
| **A60** | 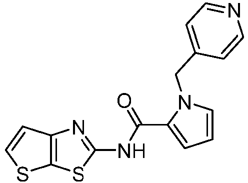 |
| **A61** | 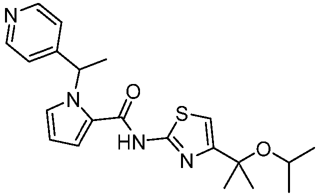 |
| **A62** | 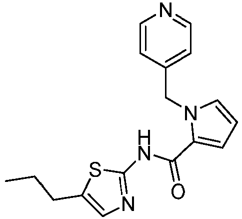 |
| **A63** | 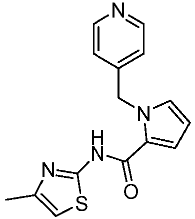 |
| **A64** | 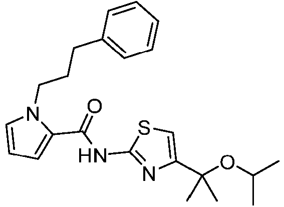 |
| **A65** | 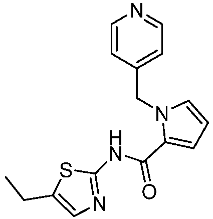 |
| **A66** | 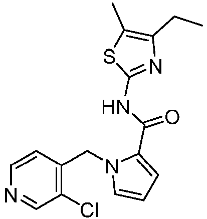 |
| **A67** | 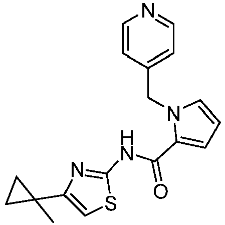 |
| **A68** | 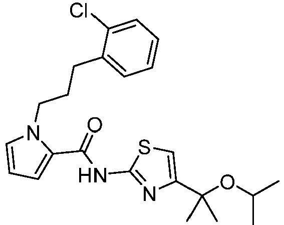 |
| **A69** | 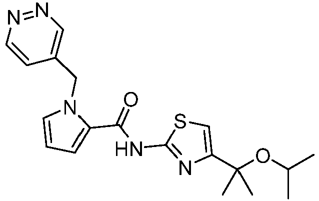 |
| **A70** | 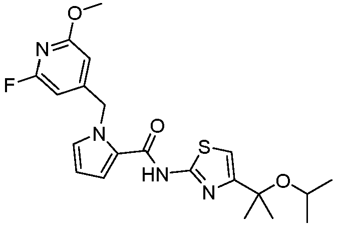 |
| **A71** | 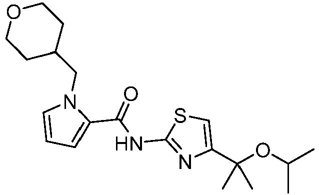 |
| **A72** | 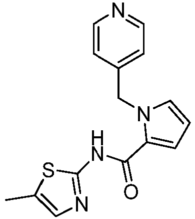 |
| **A73** | 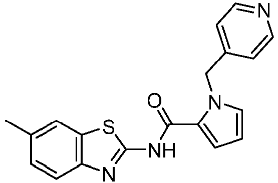 |
| **A74** | 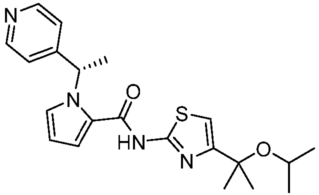 |
| **A75** | 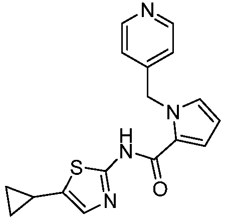 |
| **A76** | 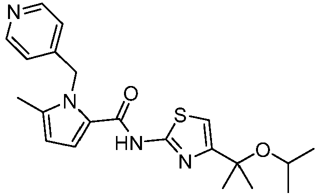 |
| **A77** | 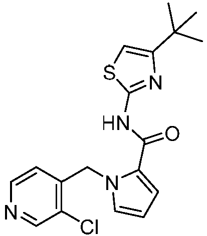 |
| **A78** | 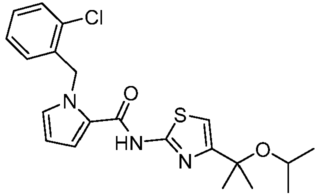 |
| **A79** | 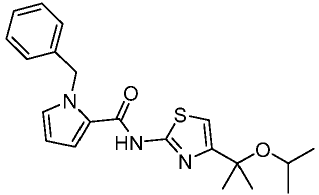 |
| **A80** | 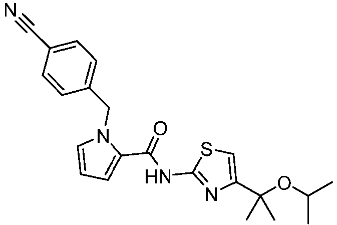 |
| **A81** | 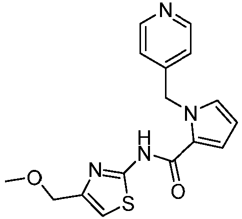 |
| **A82** | 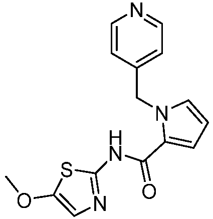 |
| **A83** | 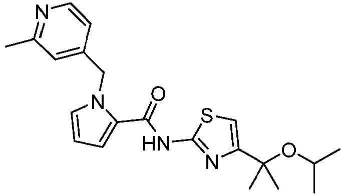 |
| **A84** | 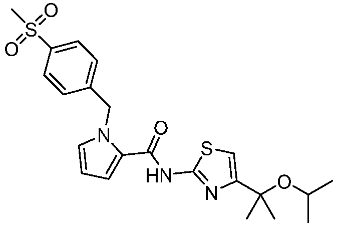 |
| **A85** | 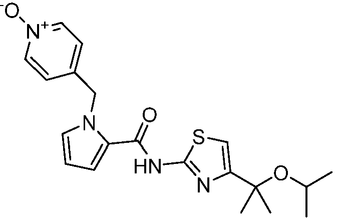 |
| **A86** | 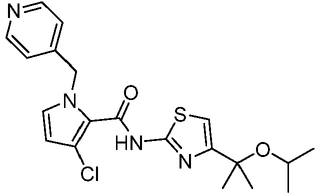 |
| **A87** | 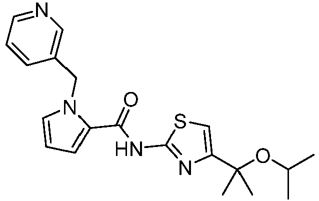 |
| **A88** | 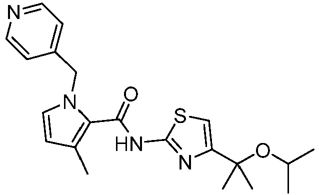 |
| **A89** | 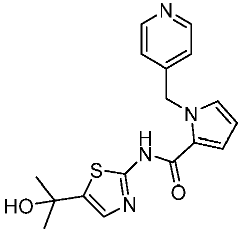 |
| **A90** | 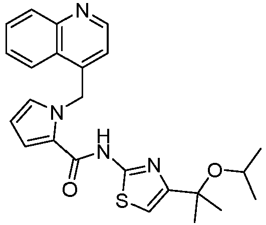 |
| **A91** | 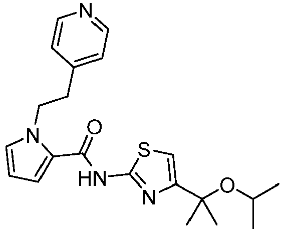 |
| **A92** | 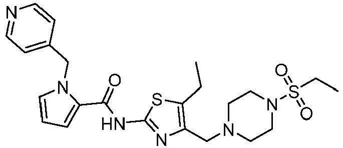 |
| **A93** | 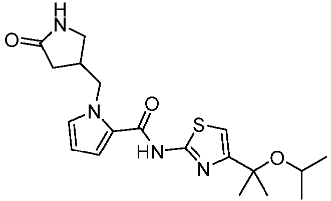 |
| **A94** | 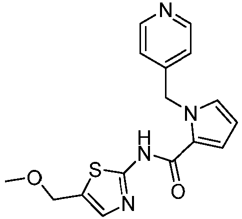 |
| **A95** | 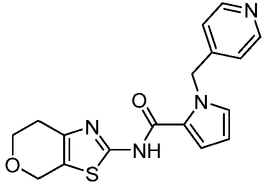 |
| **A96** | 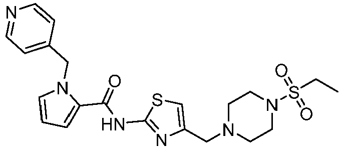 |
| **A97** | 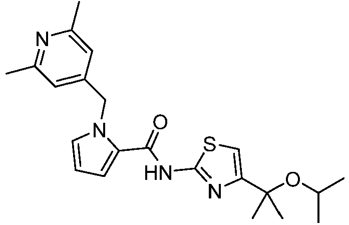 |
| **A98** | 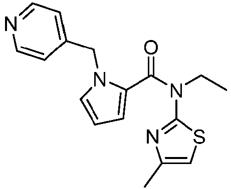 |
| **A99** | 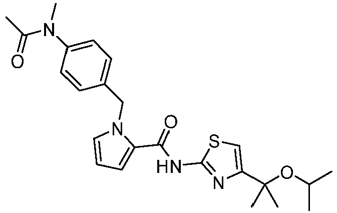 |
| **A100** | 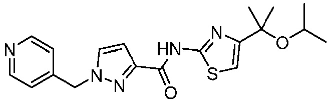 |
| **A101** | 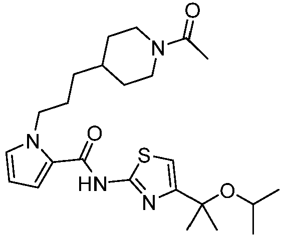 |
| **A102** | 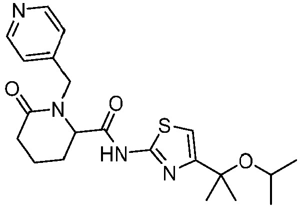 |
| **A103** | 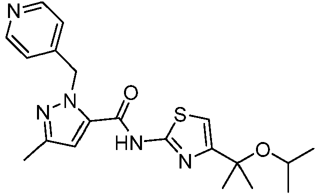 |
| **A104** | 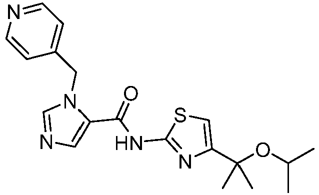 |
| **A105** | 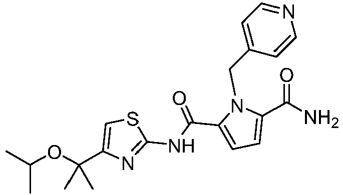 |
| **A106** | 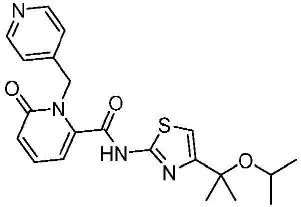 |
| **A107** | 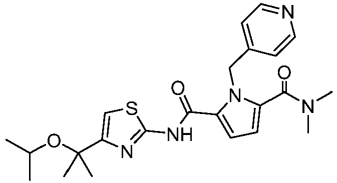 |
| **A108** | 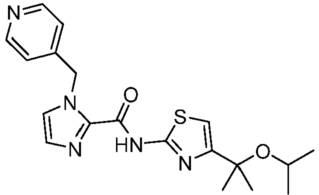 |
| **A109** | 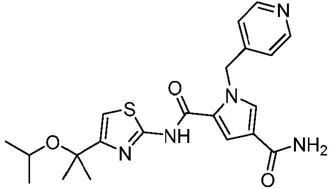 |
| **A110** | 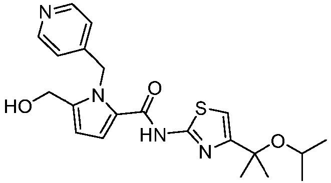 |
| **A111** | 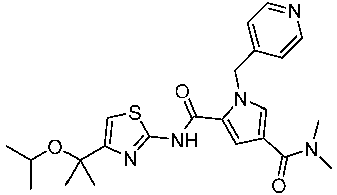 |
| **A112** | 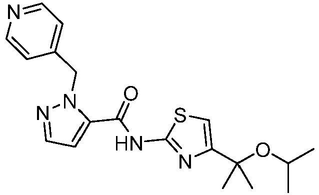 |
| **A113** | 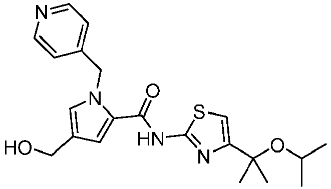 |
| **A114** | 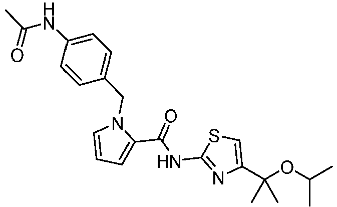 |
| **A115** | 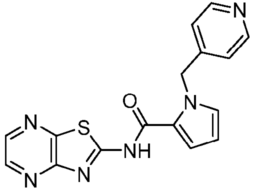 |
| **A116** | 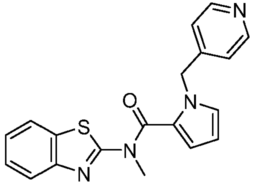 |
| **A117** | 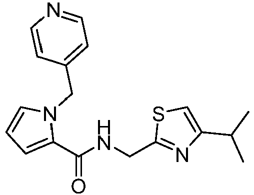 |
| **A118** | 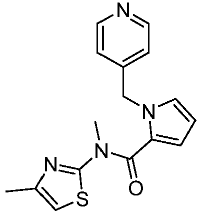 |
| **A119** | 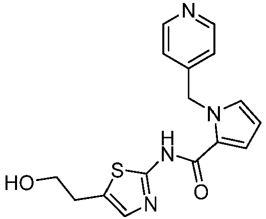 |
| **A120** | 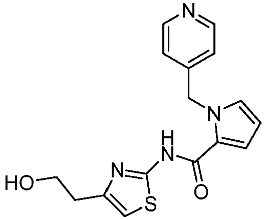 |
| **A121** | 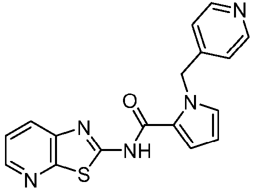 |
| **A122** | 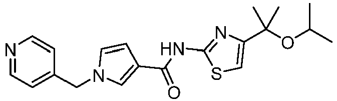 |
| **A123** | 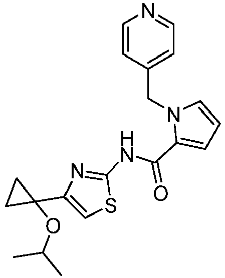 |
| **A124** | 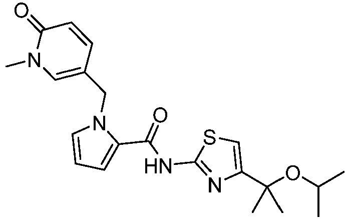 |
| **A125** | 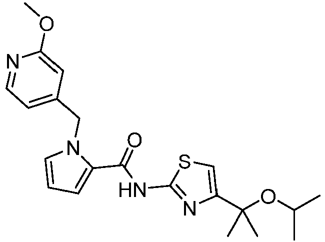 |
| **A126** | 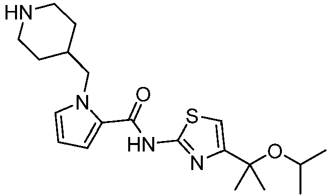 |
| **A127** | 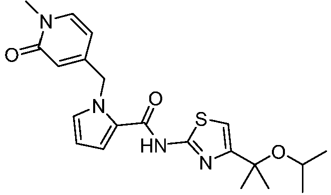 |
| **A128** | 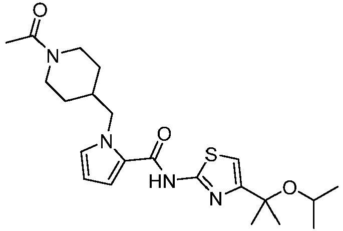 |
| **A129** | 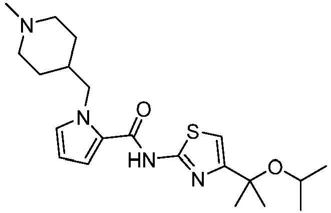 |
| **A130** | 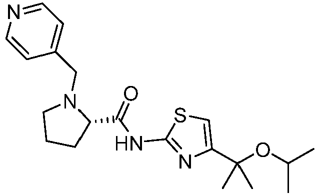 |
| **A131** | 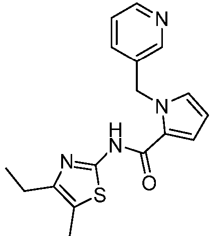 |
| **A132** | 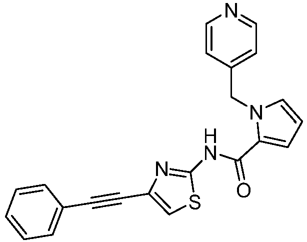 |
| **A133** | 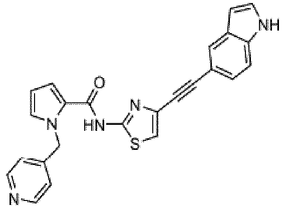 |
| **A134** | 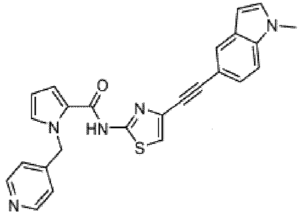 |
| **A135** | 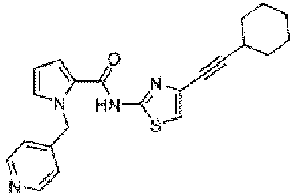 |
| **A136** | 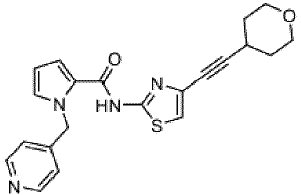 |
| **A137** | 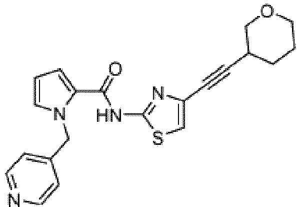 |
| **A138** | 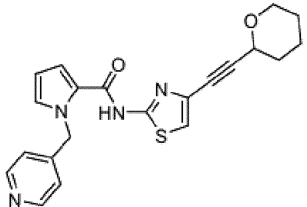 |
| **A139** | 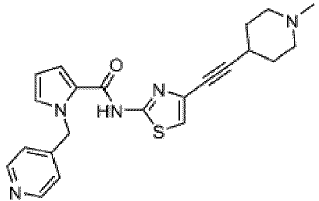 |
| **A140** | 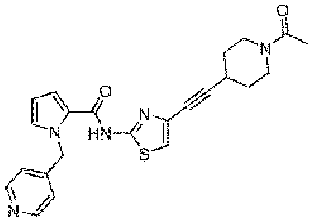 |
| **A141** | 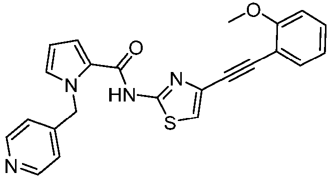 |
| **A142** | 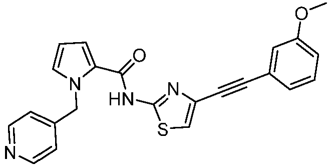 |
| **A143** | 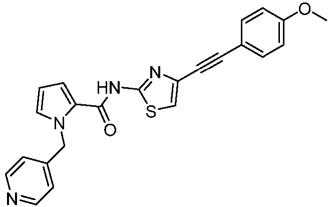 |
| **A144** | 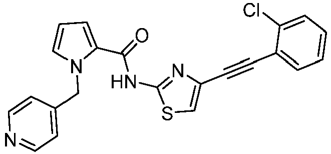 |
| **A145** | 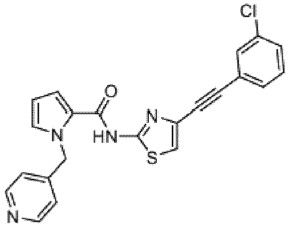 |
| **A146** | 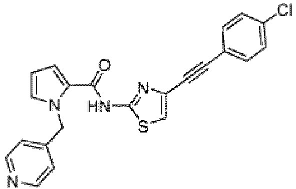 |
| **A147** | 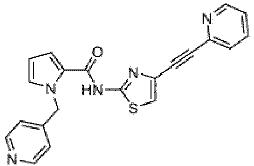 |
| **A148** | 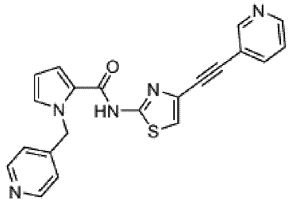 |
| **A149** | 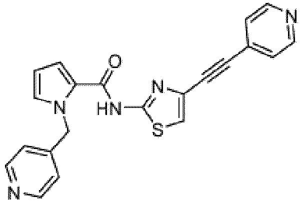 |
| **A150** | 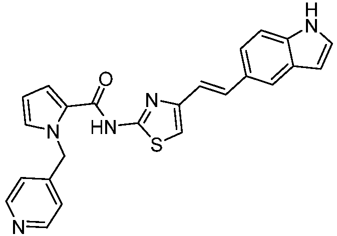 |
| **A151** | 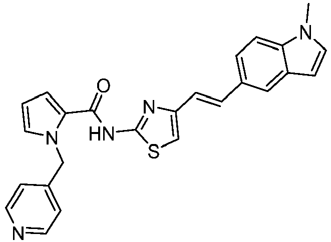 |
| **A152** | 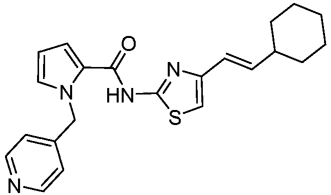 |
| **A153** | 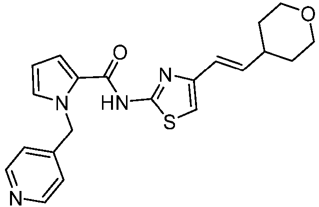 |
| **A154** | 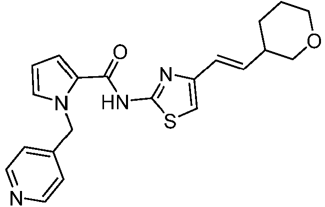 |
| **A155** | 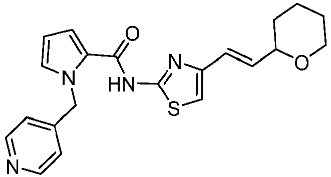 |
| **A156** | 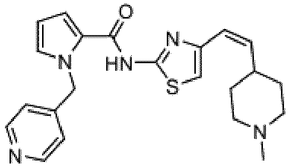 |
| **A157** | 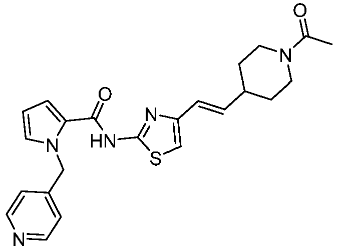 |
| **A158** | 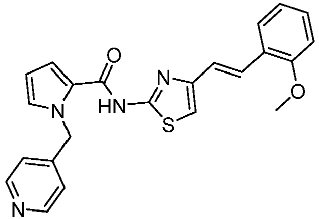 |
| **A159** | 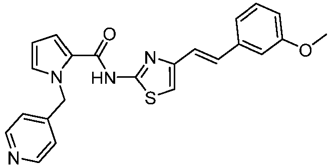 |
| **A160** | 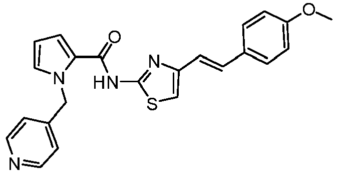 |
| **A161** | 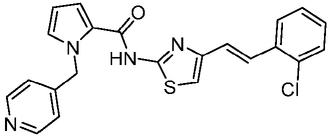 |
| **A162** | 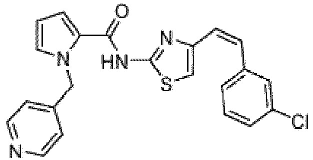 |
| **A163** | 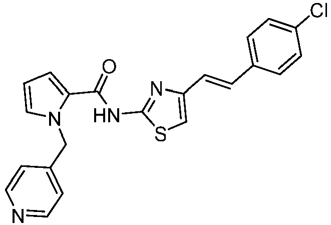 |
| **A164** | 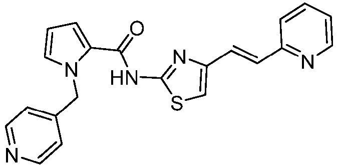 |
| **A165** | 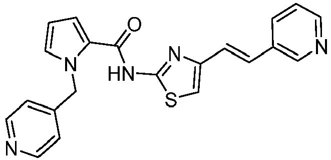 |
| **A166** | 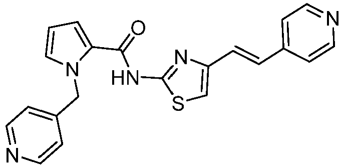 |
| **A167** | 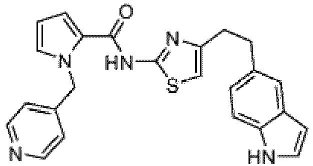 |
| **A168** | 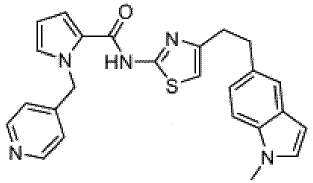 |
| **A169** | 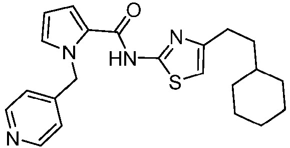 |
| **A170** | 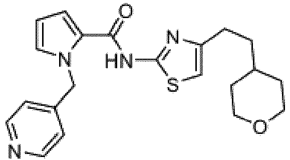 |
| **A171** | 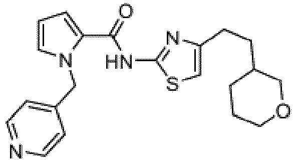 |
| **A172** | 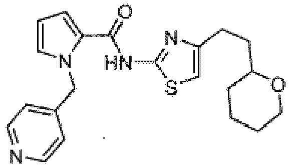 |
| **A173** | 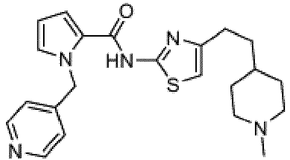 |
| **A174** | 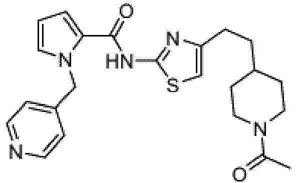 |
| **A175** | 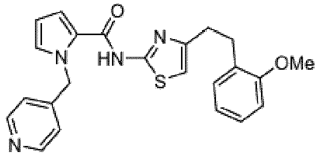 |
| **A176** | 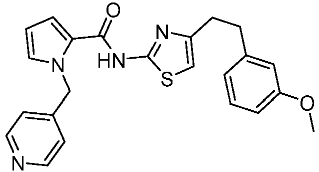 |
| **A177** | 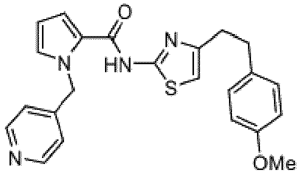 |
| **A178** | 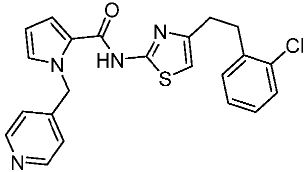 |
| **A179** | 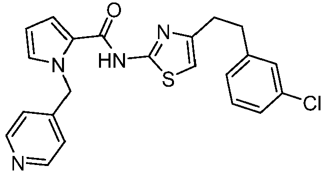 |
| **A180** | 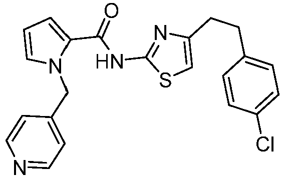 |
| **A181** | 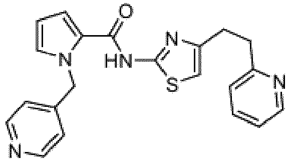 |
| **A182** | 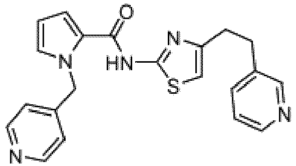 |
| **A183** | 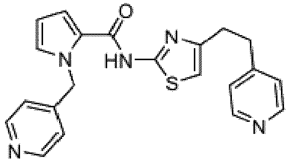 |
| **A184** | 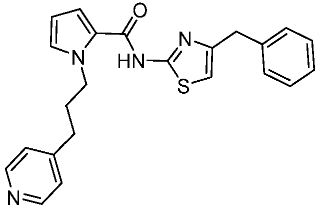 |
| **A185** | 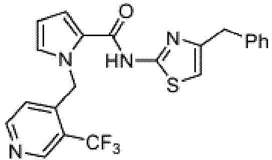 |
| **A186** | 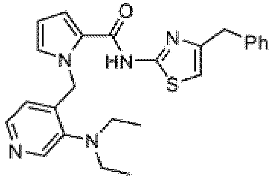 |
| **A187** | 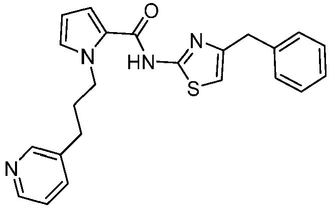 |
| **A188** | 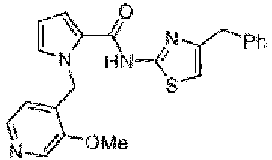 |
| **A189** | 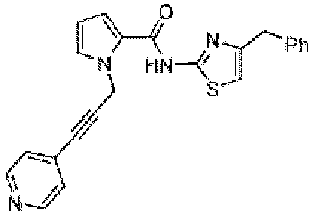 |
| **A190** | 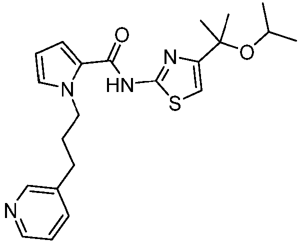 |
| **A191** | 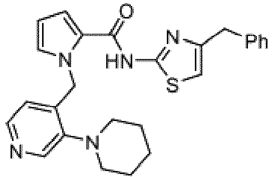 |
| **A192** | 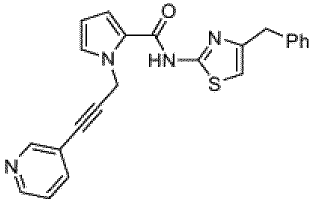 |
| **A193** | 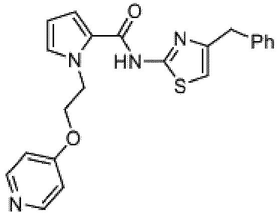 |
| **A194** | 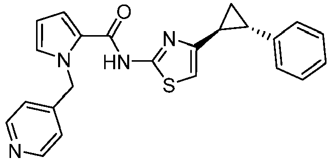 |
| **A195** | 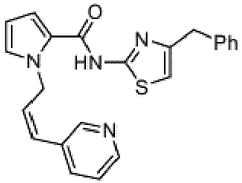 |
| **A196** | 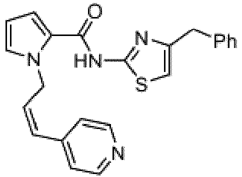 |
| **A197** | 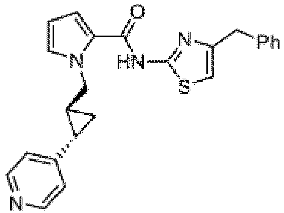 |
| **A198** | 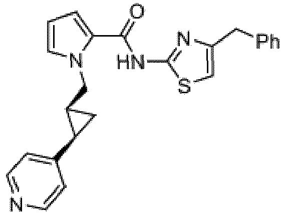 |
| **A199** | 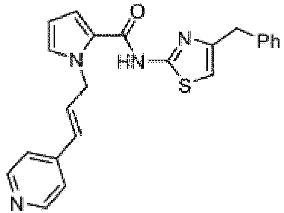 |
| **A200** | 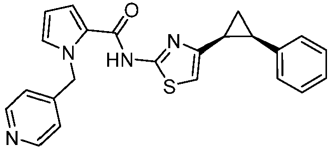 |
| **A201** | 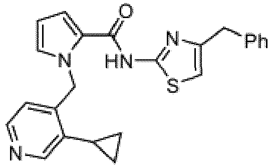 |
| **A202** | 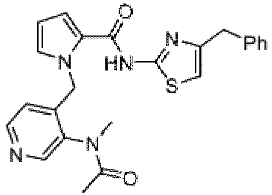 |
| **A203** | 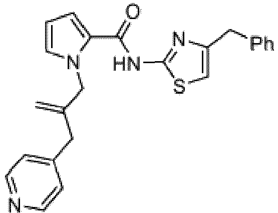 |
| **A204** | 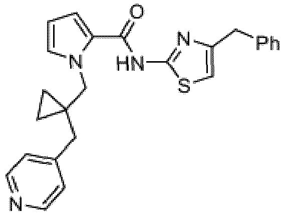 |
| **A205** | 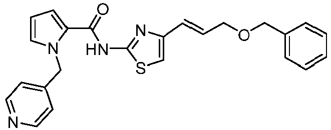 |
| **A206** | 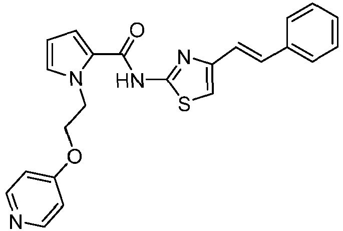 |
| **A207** | 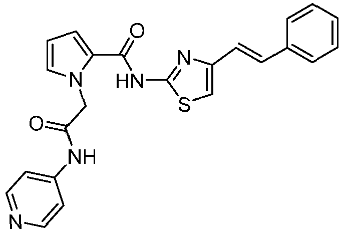 |
| **A208** | 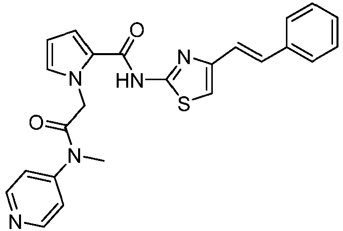 |
| **A209** | 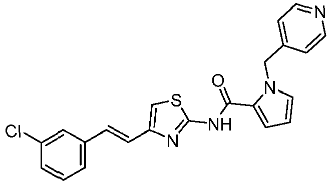 |
| **A210** | 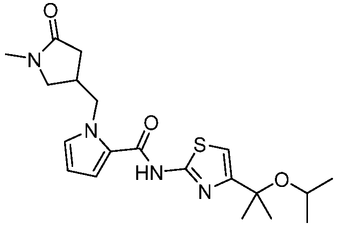 |
| **A211** | 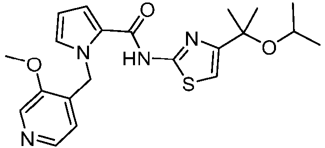 |
| **A212** | 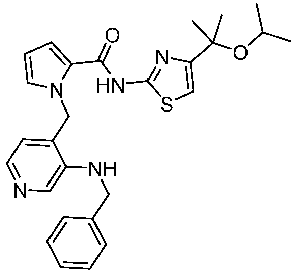 |
| **A213** | 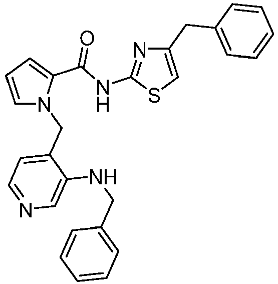 |
| **A214** | 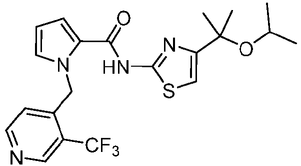 |
| **A215** | 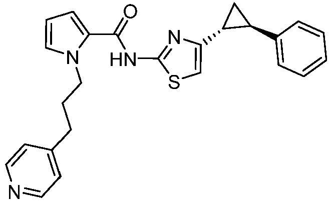 |
| **A216** | 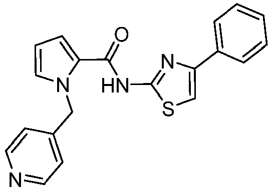 |
| **A217** | 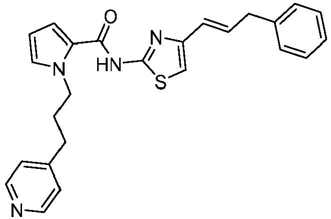 |
| **A218** | 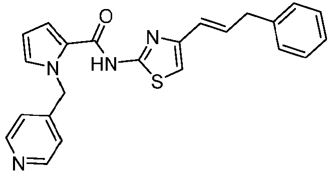 |
| **A219** | 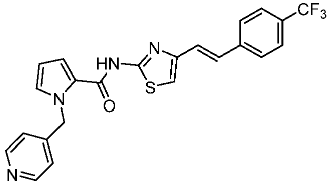 |
| **A220** | 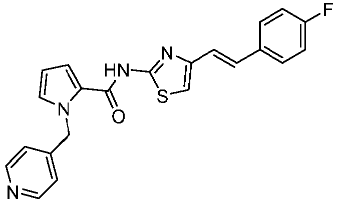 |
| **A221** | 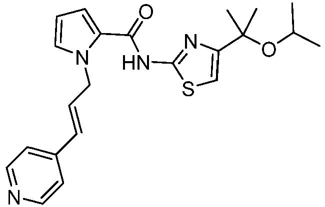 |
| **A222** | 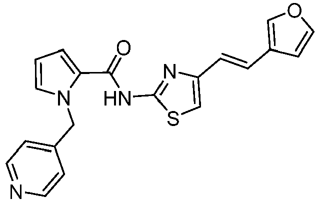 |
| **A223** | 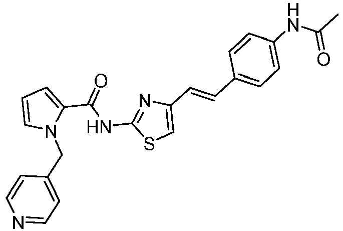 |
| **A224** | 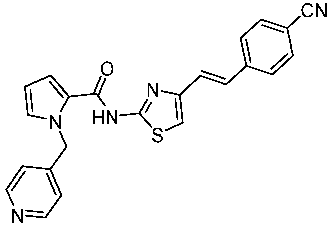 |
| **A225** | 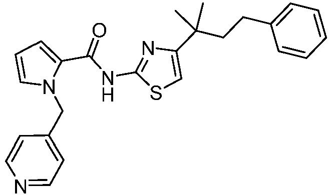 |
| **A226** | 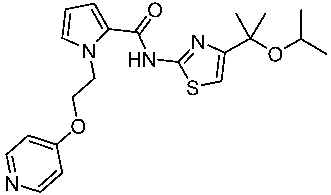 |
| **A227** | 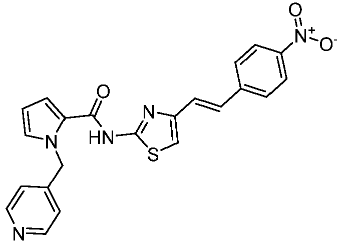 |
| **A228** | 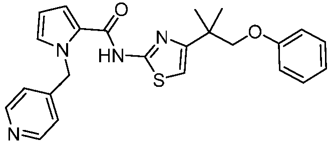 |
| **A229** | 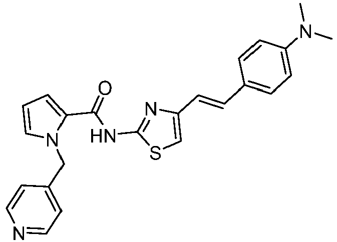 |
| **A230** | 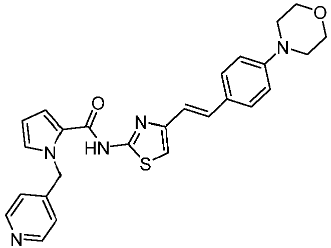 |
| **A231** | 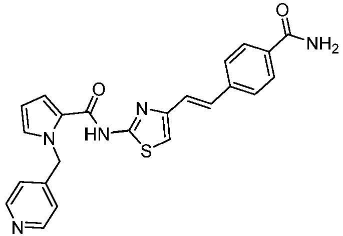 |
| **A232** | 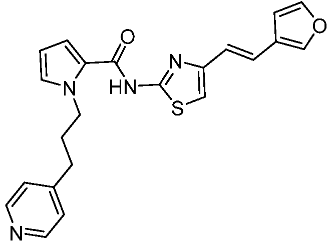 |
| **A233** | 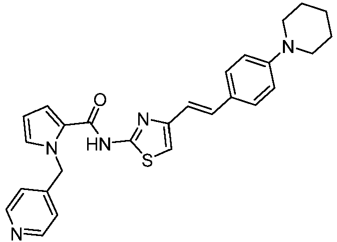 |
| **A234** | 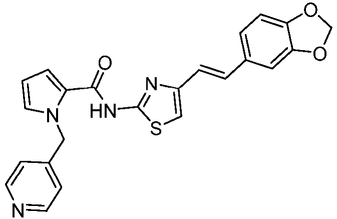 |
| **A235** | 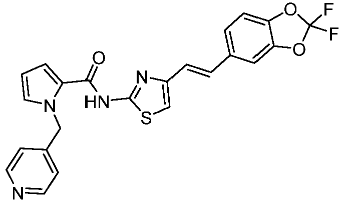 |
| **A236** | 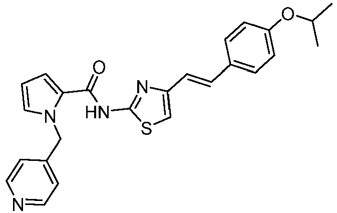 |
| **A237** | 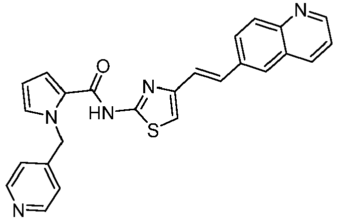 |
| **A238** | 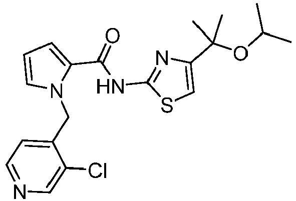 |
| **A239** | 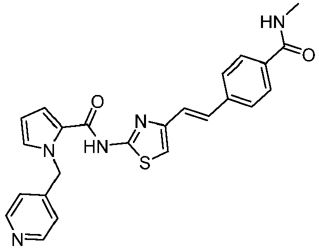 |
| **A240** | 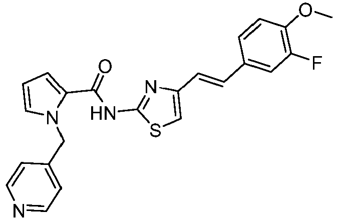 |
| **A241** | 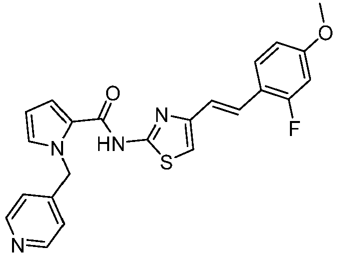 |
| **A242** | 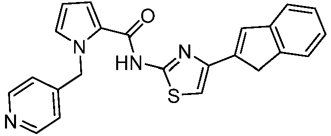 |
| **A243** | 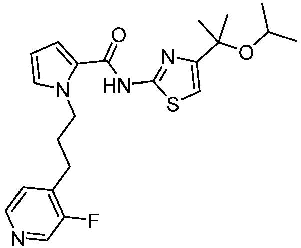 |
| **A244** | 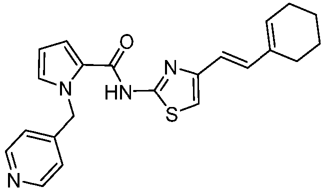 |
| **A245** | 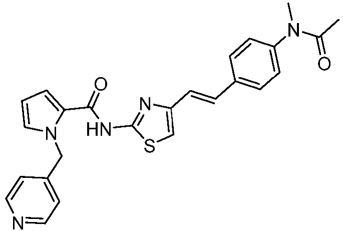 |
| **A246** | 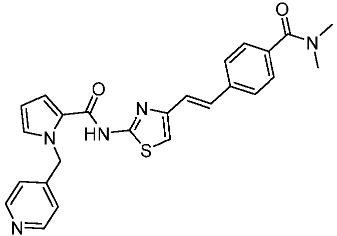 |
| **A247** | 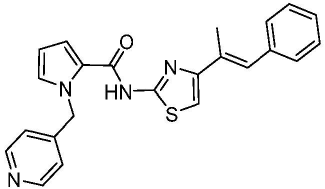 |
| **A248** | 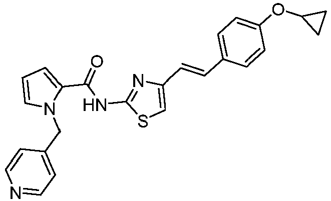 |
| **A249** | 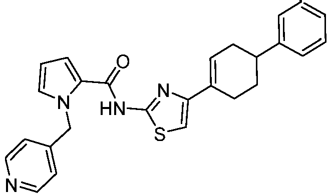 |
| **A250** | 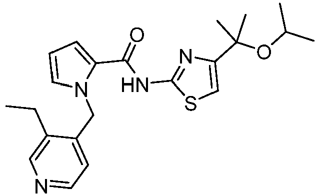 |
| **A251** | 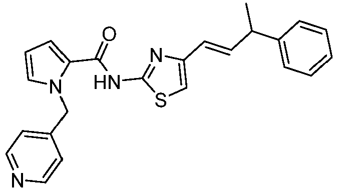 |
| **A252** | 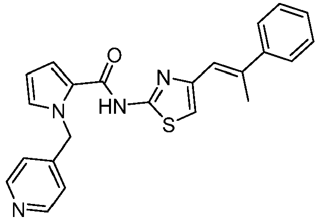 |
| **A253** | 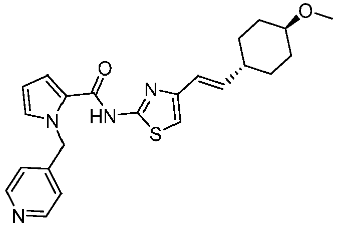 |
| **A254** | 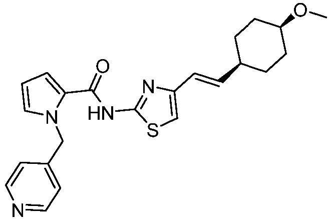 |
| **A255** | 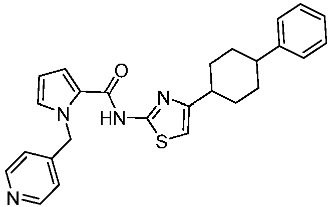 |
| **A256** | 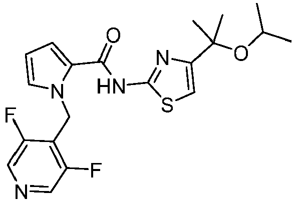 |
| **A257** | 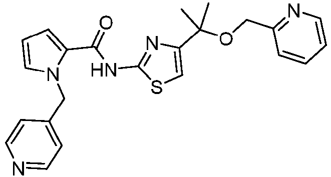 |
| **A258** | 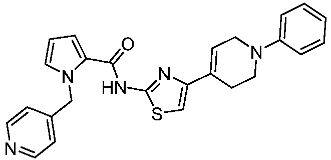 |
| **A259** | 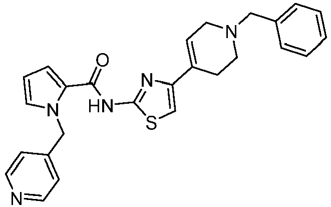 |
| **A260** | 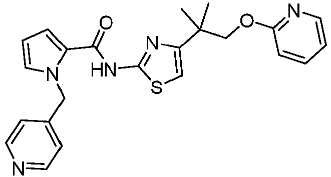 |
| **A261** | 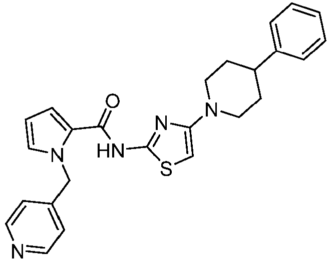 |
| **A262** | 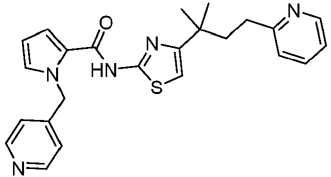 |
| **A263** | 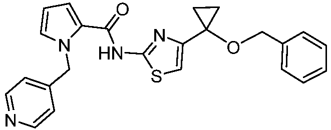 |
| **A264** | 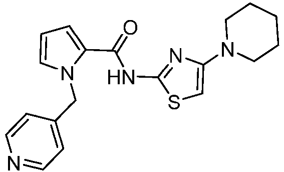 |
| **A265** | 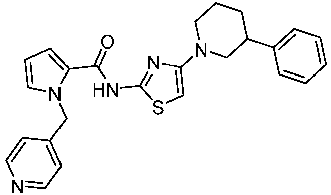 |
| **A266** | 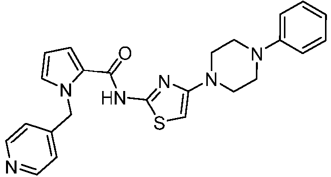 |
| **A267** | 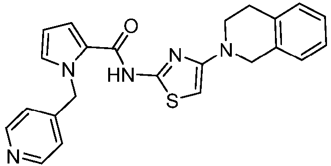 |
| **A268** | 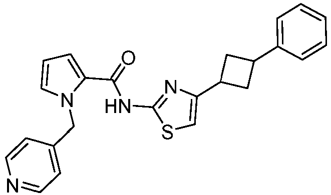 |
| **A269** | 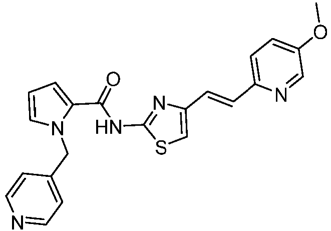 |
| **A270** | 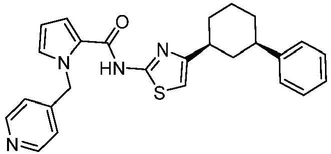 |
| **A271** | 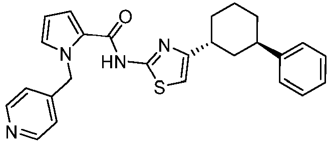 |
| **A272** | 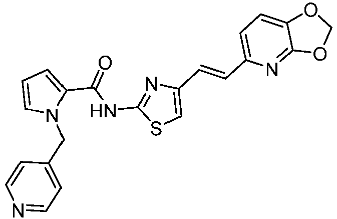 |
| **A273** | 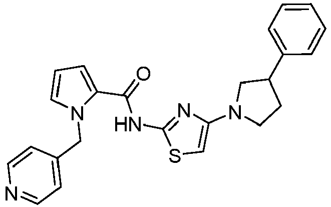 |
| **A274** | 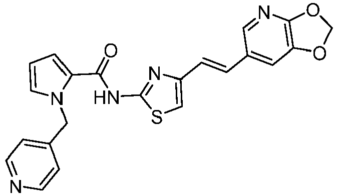 |
| **A275** | 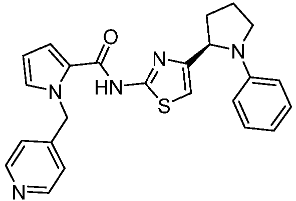 |
| **A276** | 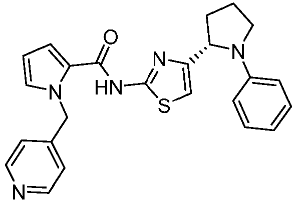 |
| **A277** | 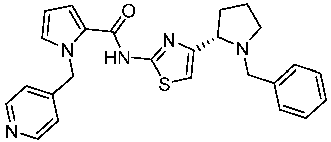 |
| **A278** | 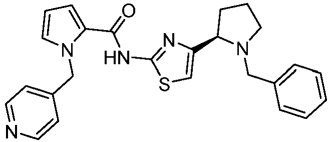 |
| **A279** | 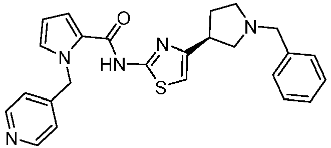 |
| **A280** | 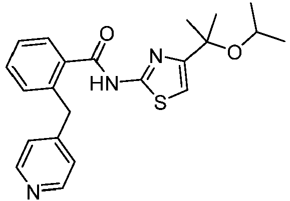 |
| **A281** | 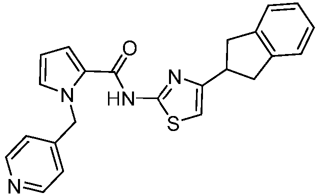 |
| **A282** | 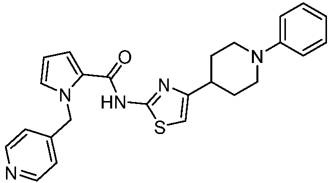 |
| **A283** | 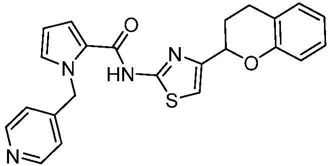 |
| **A284** | 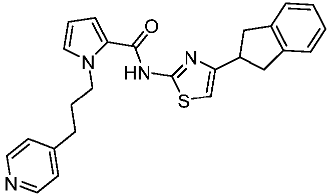 |
| **A285** | 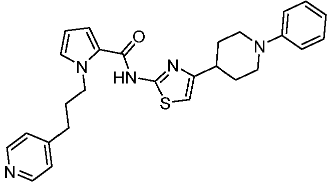 |
| **A286** | 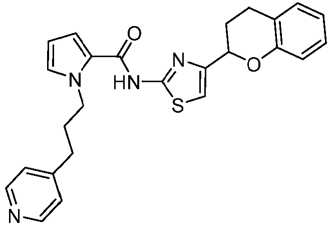 |
| **A287** | 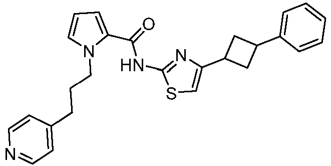 |
| **A288** | 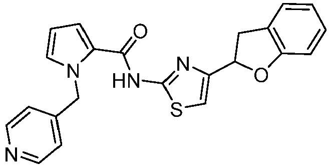 |
| **A289** | 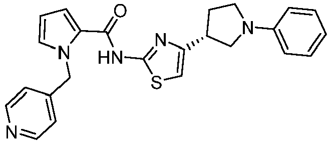 |
| **A290** | 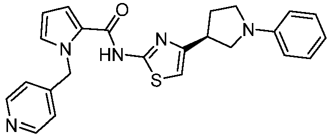 |
| **A291** | 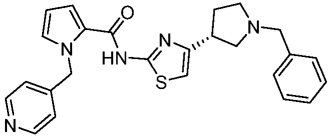 |
| **A292** | 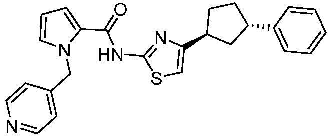 |
| **A293** | 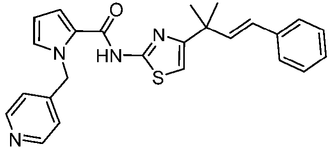 |
| **A294** | 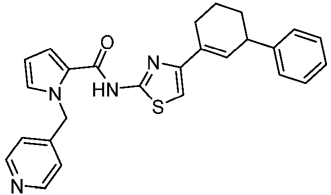 |
| **A295** | 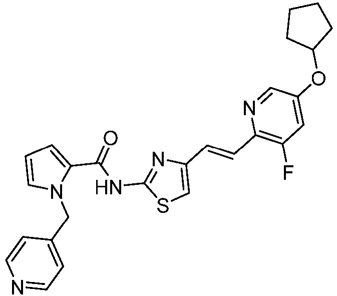 |
| **A296** | 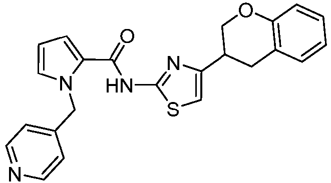 |
| **A297** | 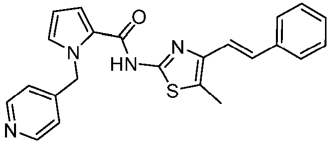 |
| **A298** | 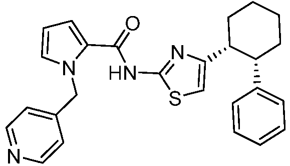 |
| **A299** | 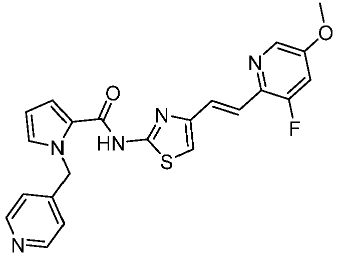 |
| **A300** | 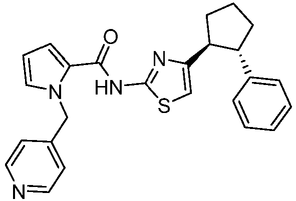 |
| **A301** | 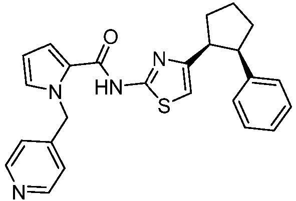 |
| **A302** | 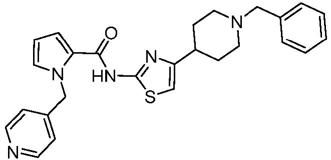 |
| **A303** | 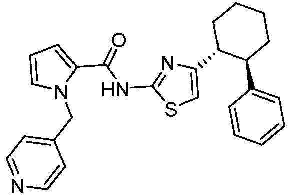 |
| **A304** | 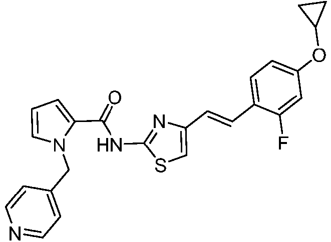 |
| **A305** | 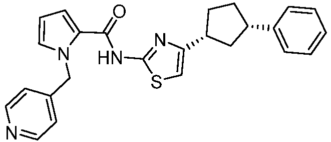 |
| **A306** | 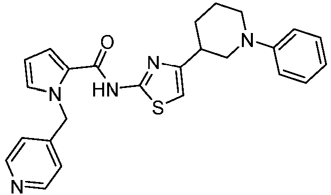 |
| **A307** | 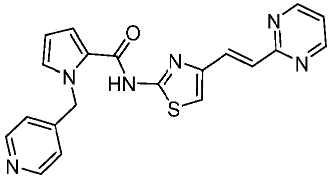 |
| **A308** | 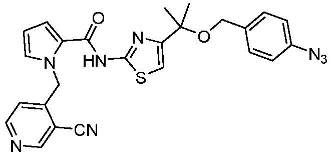 |
| **A309** | 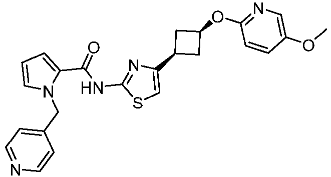 |
| **A310** | 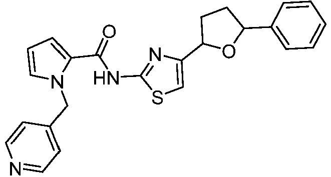 |
| **A311** | 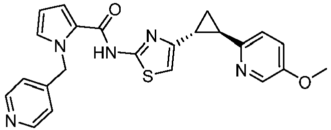 |
| **A312** | 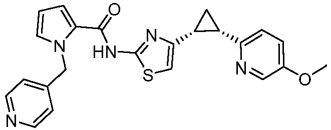 |
| **A313** | 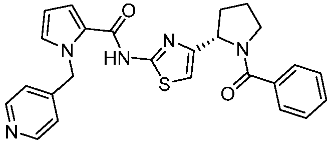 |
| **A314** | 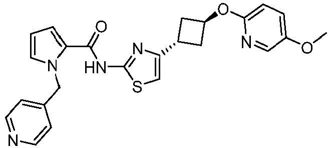 |
| **A315** | 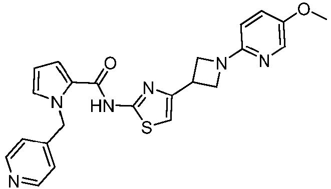 |
| **A316** | 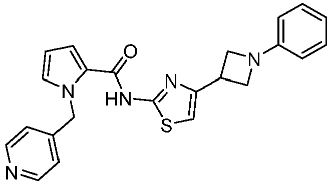 |
| **A317** | 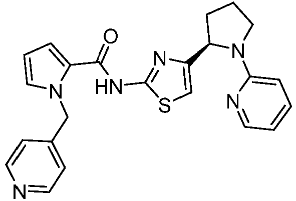 |
| **A318** | 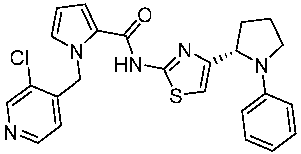 |
| **A319** | 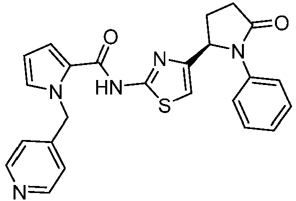 |
| **A320** | 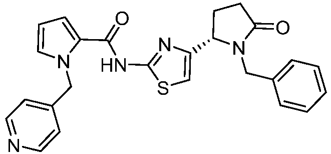 |
| **A321** | 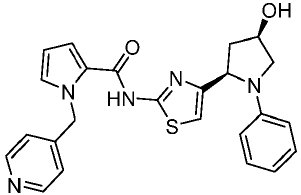 |
| **A322** | 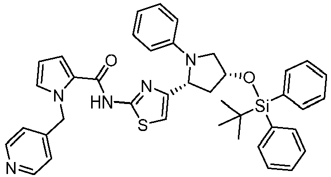 |
| **A323** | 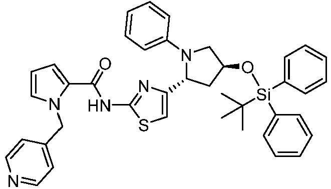 |
| **A324** | 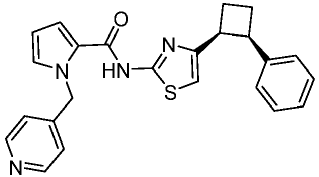 |
| **A325** | 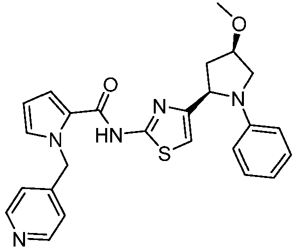 |
| **A326** | 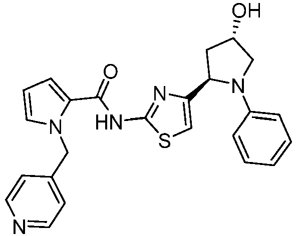 |
| **A327** | 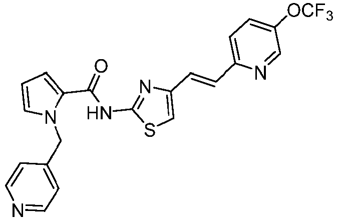 |
| **A328** | 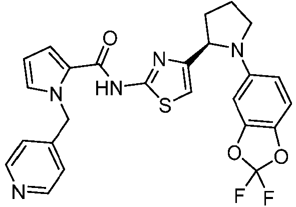 |
| **A329** | 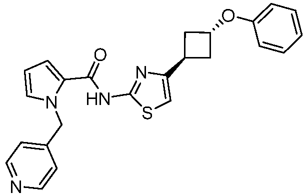 |
| **A330** | 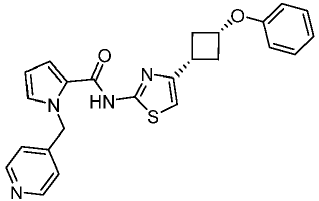 |
| **A331** | 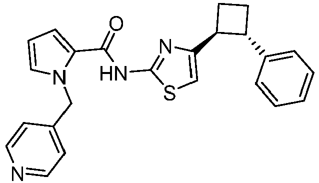 |
| **A332** | 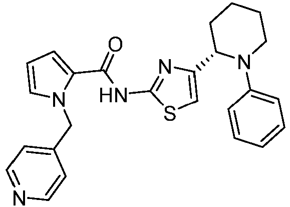 |
| **A333** | 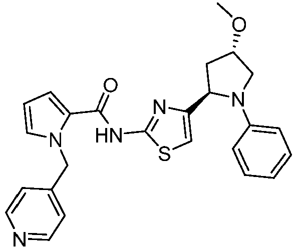 |
| **A334** | 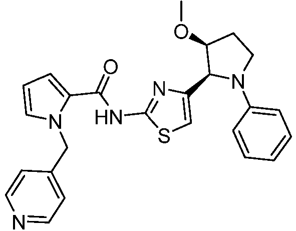 |
| **A335** | 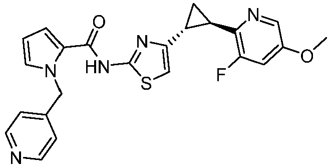 |
| **A336** | 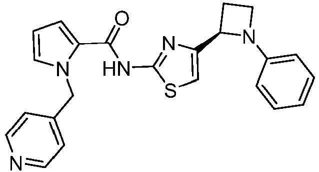 |
| **A337** | 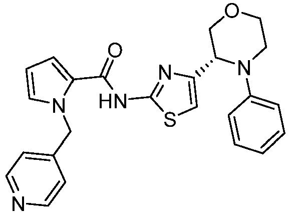 |
| **A338** | 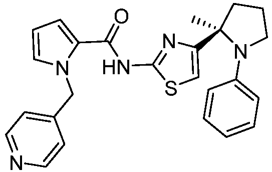 |
| **A339** | 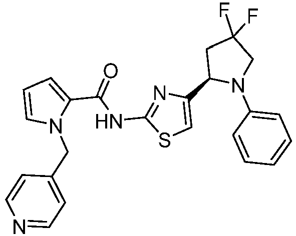 |
| **A340** | 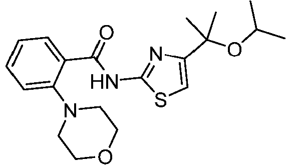 |
| **A341** | 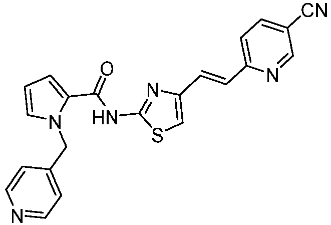 |
| **A342** | 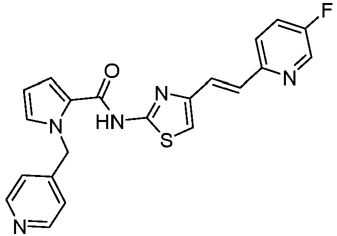 |
| **A343** | 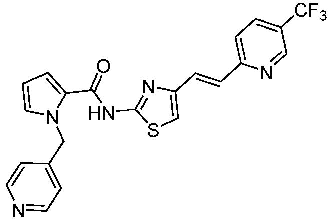 |
| **A344** | 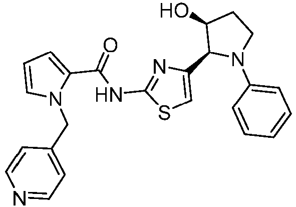 |
| **A345** | 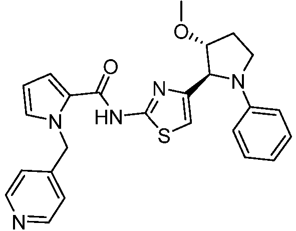 |
| **A346** | 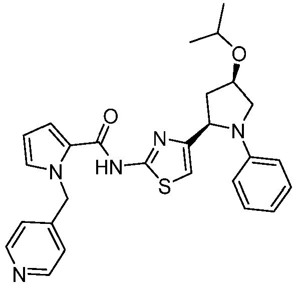 |
| **A347** | 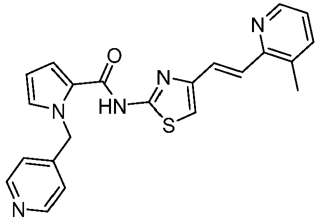 |
| **A348** | 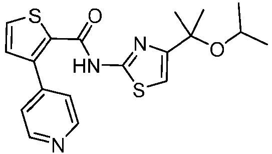 |
| **A349** | 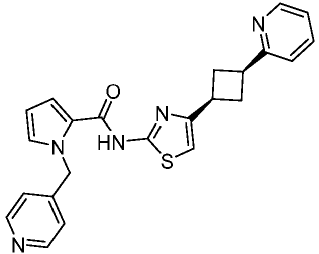 |
| **A350** | 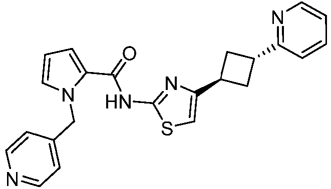 |
| **A351** | 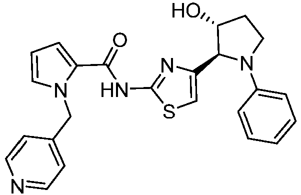 |
| **A352** | 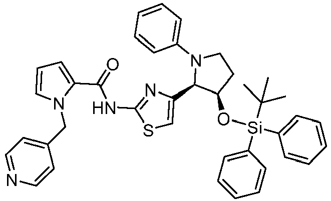 |
| **A353** | 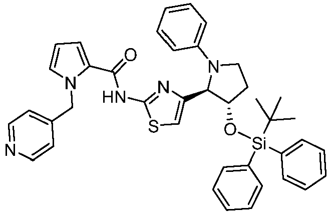 |
| **A354** | 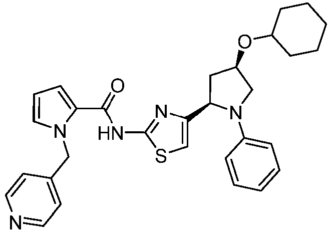 |
| **A355** | 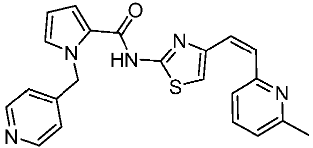 |
| **A356** | 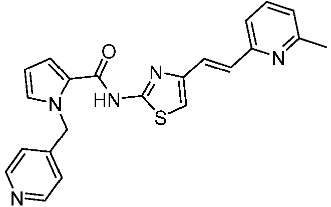 |
| **A357** | 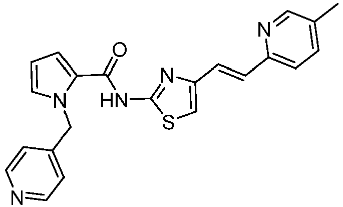 |
| **A358** | 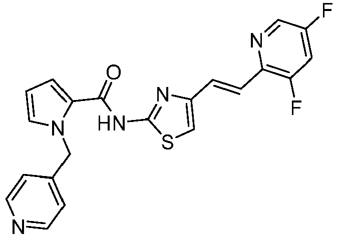 |
| **A359** | 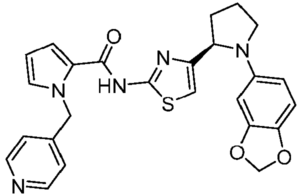 |
| **A360** | 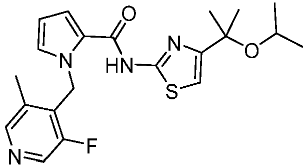 |
| **A361** | 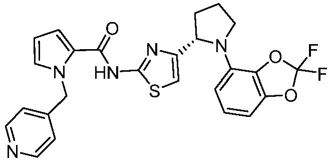 |
| **A362** | 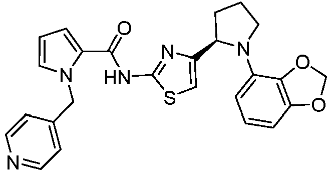 |
| **A363** | 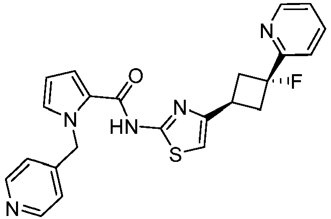 |
| **A364** | 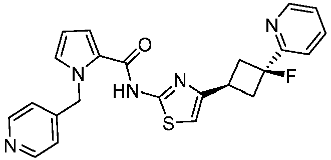 |
| **A356** | 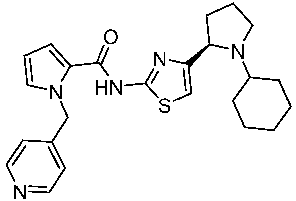 |
| **A366** | 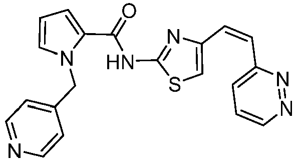 |
| **A367** | 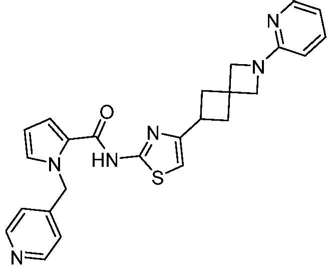 |
| **A368** | 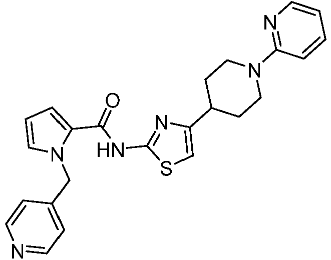 |
| **A369** | 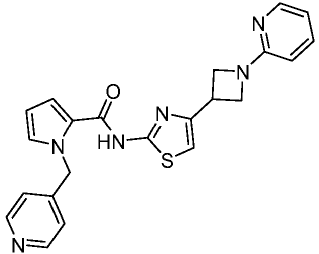 |
| **A370** | 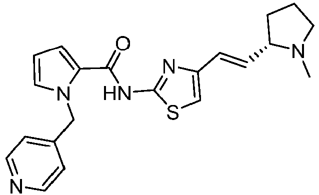 |
| **A371** | 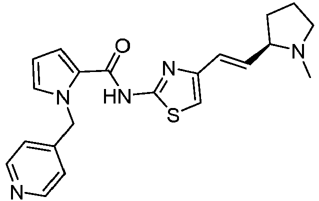 |
| **A372** | 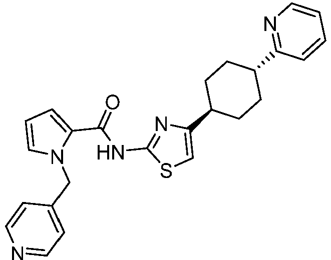 |
| **A373** | 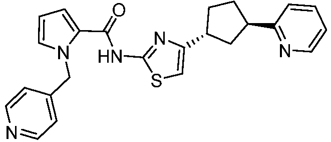 |
| **A374** | 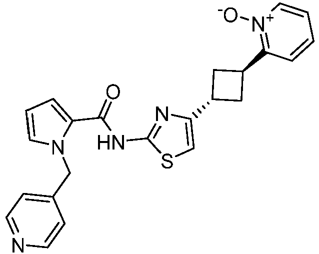 |
| **A375** | 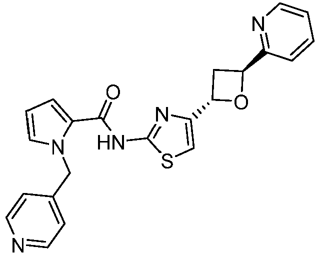 |
| **A376** | 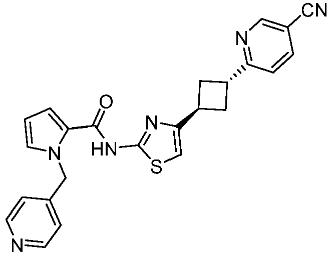 |
| **A377** | 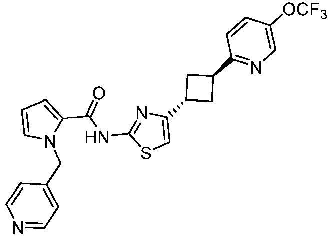 |
| **A378** | 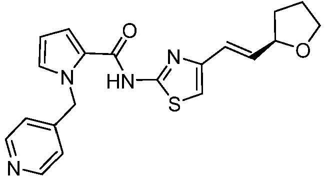 |
| **A379** | 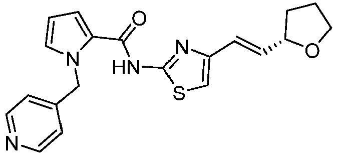 |
| **A380** | 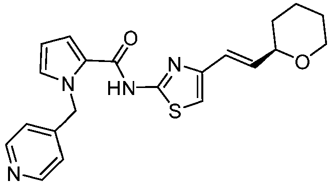 |
| **A381** | 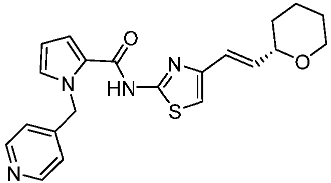 |
| **A382** | 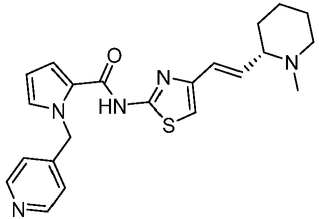 |
| **A383** | 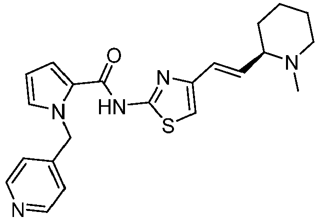 |
| **A384** | 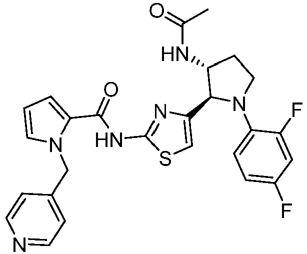 |
| **A385** | 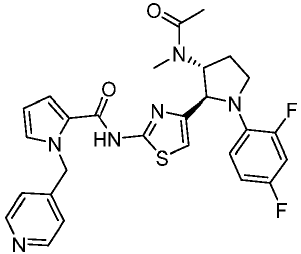 |
| **A386** | 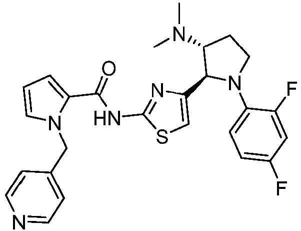 |
| **A387** | 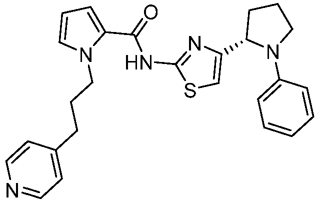 |
| **A388** | 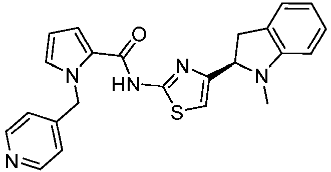 |
| **A389** | 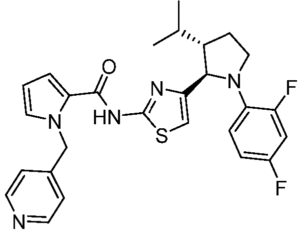 |
| **A390** | 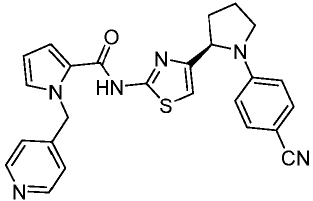 |
| **A391** | 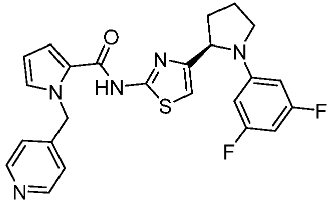 |
| **A392** | 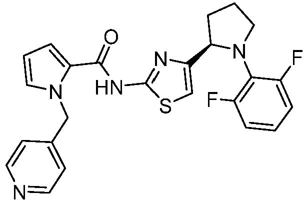 |
| **A393** | 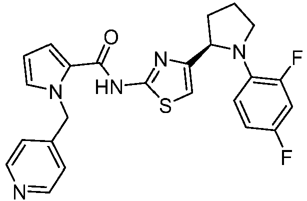 |
| **A394** | 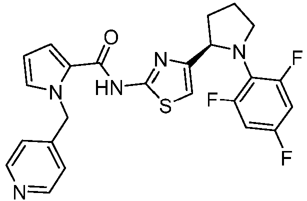 |
| **A395** | 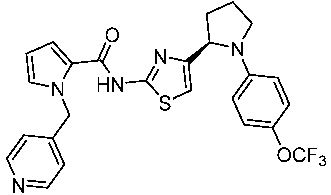 |
| **A396** | 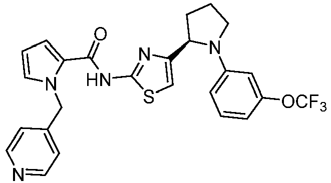 |
| **A397** | 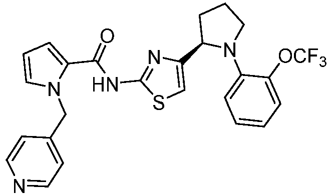 |
| **A398** | 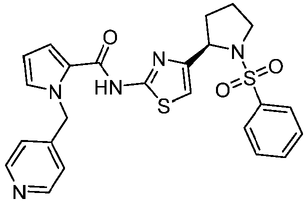 |
| **A399** | 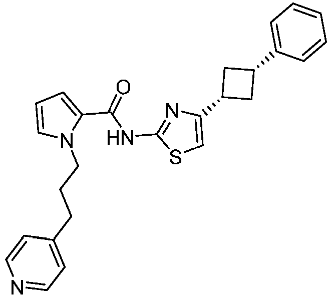 |
| **A400** | 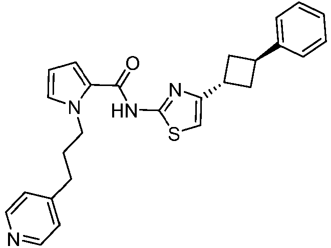 |
| **A401** | 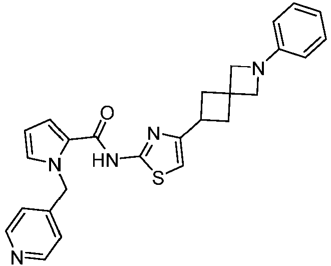 |
| **A402** | 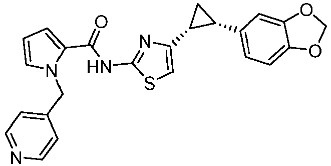 |
| **A403** | 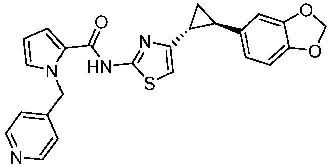 |

12. The compound or salt of claim 11, wherein the compound or salt is selected from the group consisting of: 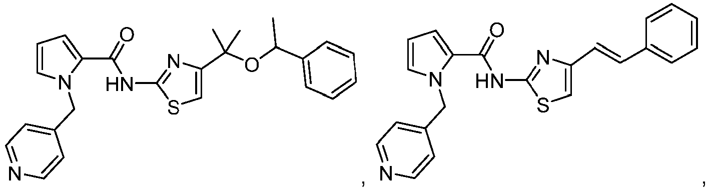 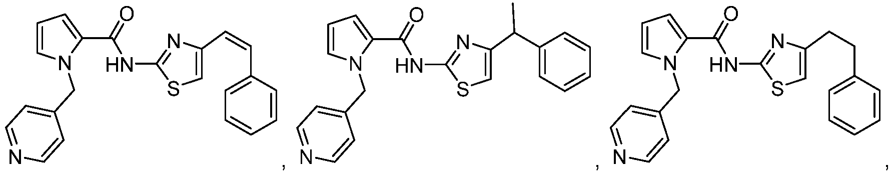 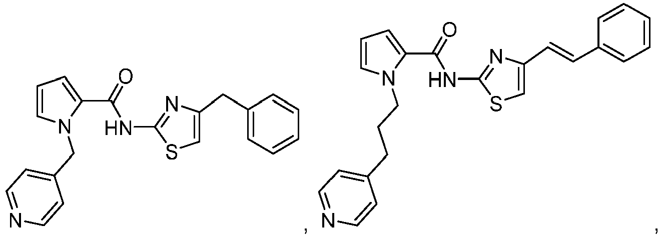 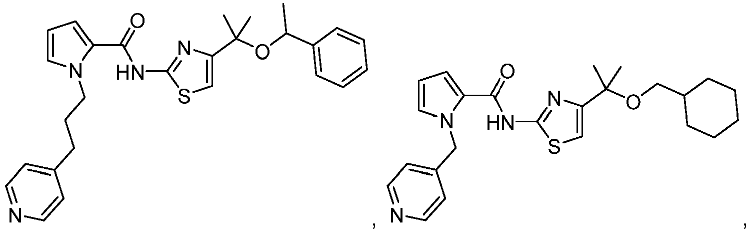 and 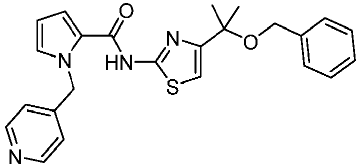 or the compound is selected from the group consisting of:

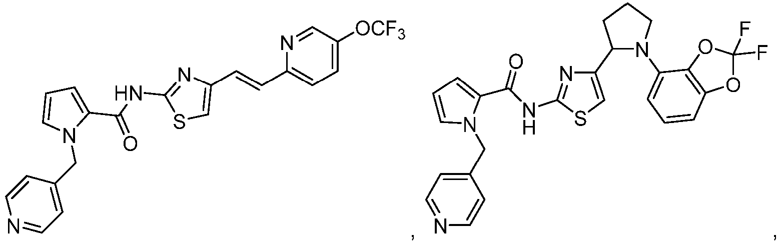

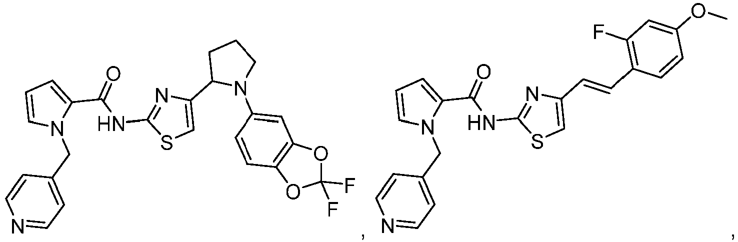

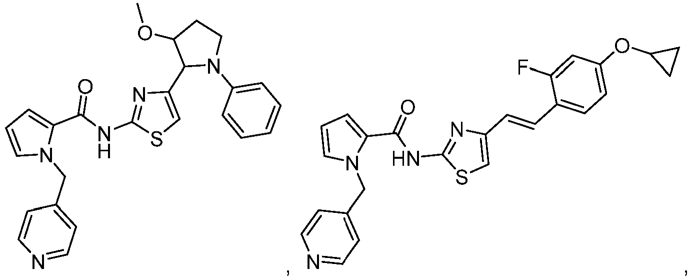

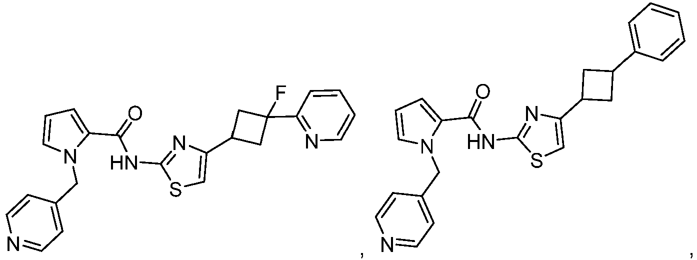

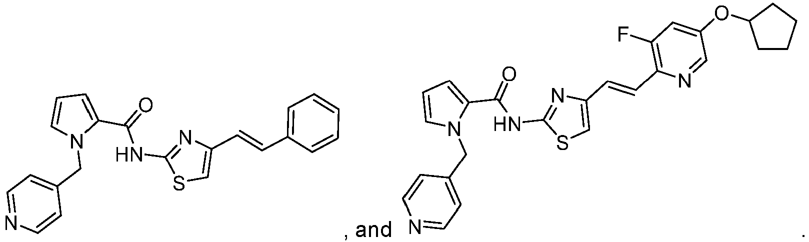

13. The compound or salt of any one of claims 1 to 12 for use in treating inflammation or an autoimmune disease or an immune-related disease or a neurodegenerative disease or an inflammatory disease.

14. The compound or salt of any one of claims 1 to 12 for use in treating cancer.

15. The compound or salt for use of claim 14, wherein the cancer is melanoma, multiple myeloma, prostate cancer, lung cancer, pancreatic cancer, squamous cell carcinoma, leukemia, lymphoma, a neuroendocrine tumor, bladder cancer, or colorectal cancer or the cancer is non-small cell lung carcinoma, squamous cell carcinoma, leukemia, acute myelogenous leukemia, chronic myelogenous leukemia, lymphoma, NPM/ALK-transformed anaplastic large cell lymphoma, diffuse large B cell lymphoma, neuroendocrine tumors, breast cancer, mantle cell lymphoma, renal cell carcinoma, rhabdomyosarcoma, ovarian cancer, endometrial cancer, small cell carcinoma, adenocarcinoma, gastric carcinoma, hepatocellular carcinoma, pancreatic cancer, thyroid carcinoma, anaplastic large cell lymphoma, hemangioma, or head and neck cancer.

16. The compound or salt for use of claim 14, wherein the cancer is a solid tumor.

## Patentansprüche

1. Verbindung, die eine Struktur der Formel (III) aufweist: worin: 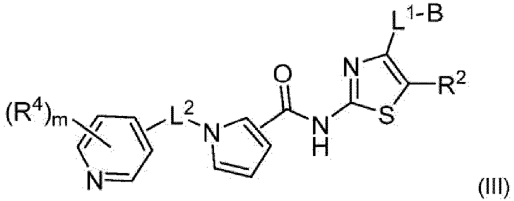
L¹ eine Bindung, C₁₋₆-Alkylen oder 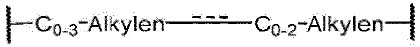
ist, worin --- für eine Doppelbindung, eine Dreifachbindung oder ein kondensiertes Cyclopropyl steht;
B C₀₋₃-Alkylen-X ist;
X ein aromatischer oder nichtaromatischer C₄₋₁₀-Carbocyclus oder ein aromatischer oder nichtaromatischer, 4- bis 10-gliedriger Heterocyclus mit 1 bis 3 Ring-Heteroatomen ist, die aus N, O und S ausgewählt sind;
X mit 1 bis 3 G substituiert ist; und
die G jeweils unabhängig aus der aus Halogen, OH, =O, CN, NO₂, N(R^{N})₂, N(R^{N})-C(O)C₁₋₃-Alkyl, C₁₋₃-Alkyl, C₁₋₃-Alkoxy, C₁₋₃-Halogenalkyl, C₁₋₃-Halogenalkoxy, C(O)C₁₋₃-Alkyl, S(O₂)-Z, C(O)-Z, C(O)N(R^{N})₂, Silylether und [O]₀₋₁-C₀₋₃-Alkylen-Z bestehenden Gruppe ausgewählt sind;
die R^{N} jeweils unabhängig H oder C₁₋₄-Alkyl sind;
Z ein aromatischer oder nichtaromatischer C₃₋₁₀-Carbocyclus oder ein aromatischer oder nichtaromatischer, 4- bis 10-gliedriger Heterocyclus mit 1 bis 3 Heteroatomen ist, die aus der aus N, O und S bestehenden Gruppe ausgewählt sind;
Z gegebenenfalls mit 1 bis 3 E substituiert ist; und
die E jeweils unabhängig aus der aus C₁₋₃-Alkyl, C₁₋₃-Alkoxy, =O, C₁₋₃-Halogenalkoxy, CN und Halogen bestehenden Gruppe ausgewählt sind;
R² H ist;
L² C₀₋₃-Alkylen ist;
m = 0 bis 2 ist; und
die R⁴ jeweils unabhängig C₁₋₃-Alkyl, C₂₋₃-Alkinyl, C₁₋₃-Halogenalkyl, C₁₋₃-Alkoxy, Halogen oder NHC₁₋₃-Alkylenaryl sind;
oder ein pharmazeutisch annehmbares Salz davon,
wobei die Verbindung oder das Salz davon nicht N-[4-[(3-methyl-1-piperidinyl)methyl]-2-thiazolyl)-1-(4-pyridinylmethyl)-1H-pyrrol-2-carboxamid ist.

2. Verbindung oder Salz nach Anspruch 1, worin L₁ eine Bindung oder 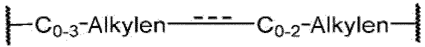 ist und --- für eine Doppelbindung steht.

3. Verbindung oder Salz nach einem der Ansprüche 1 bis 2, worin X Pyrrolidinyl, Cyclobutyl, Cyclopentyl, Cyclohexyl, Cyclohexenyl, Phenyl, Piperidinyl, Pyridinyl, Piperazinyl, Tetrahydrofuranyl, Furanyl, Tetrahydropyranyl, Chinolinyl, Morpholinyl, Pyrrolidonyl, Pyrimidinyl, Pyridazinyl, Indenyl, Dihydroindenyl, Dihydrobenzofuranyl, Chromanyl, Isochromanyl, Dihydroisochinolinyl oder Indolyl ist.

4. Verbindung oder Salz nach Anspruch 1, worin L¹-B aus der aus Folgendem bestehenden Gruppe ausgewählt ist:

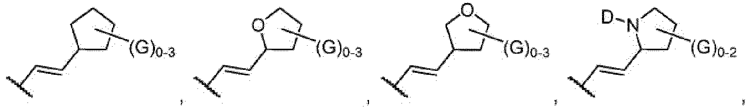

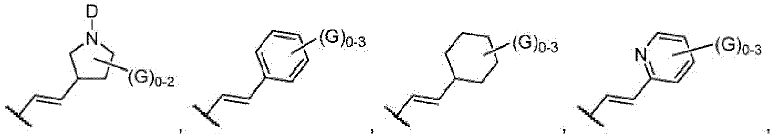

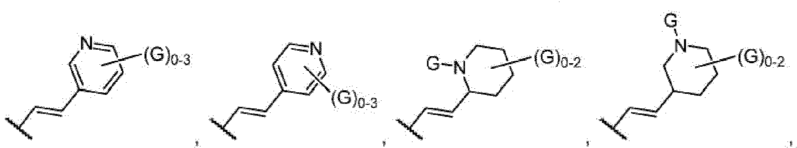

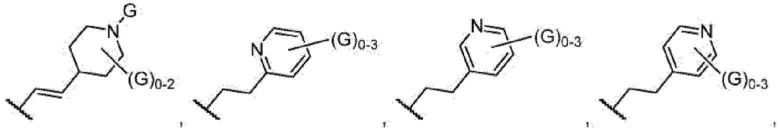

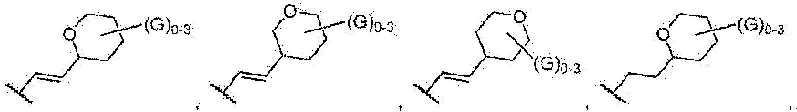

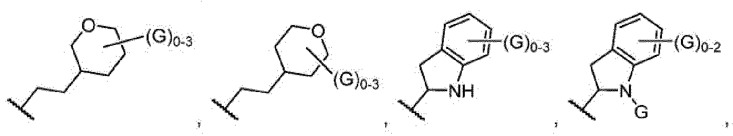

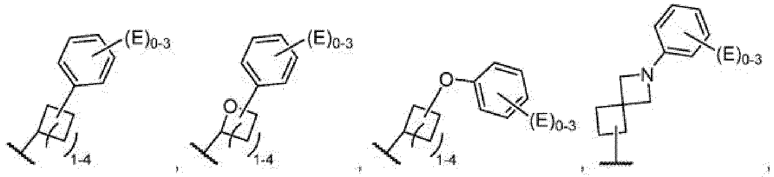

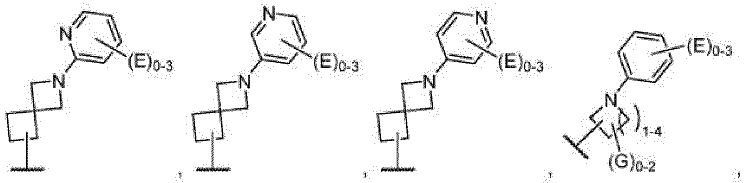

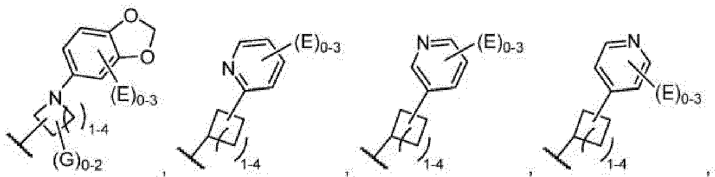

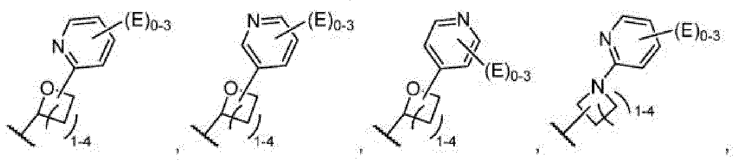

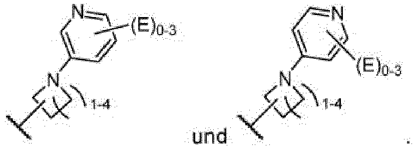

5. Verbindung oder Salz nach Anspruch 1, worin L¹-B aus der aus Folgendem bestehenden Gruppe ausgewählt ist:

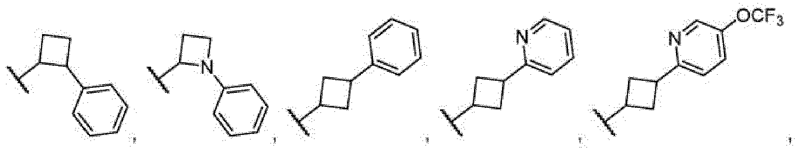

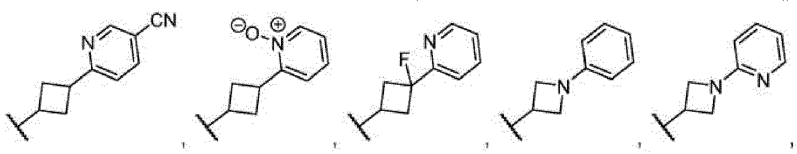

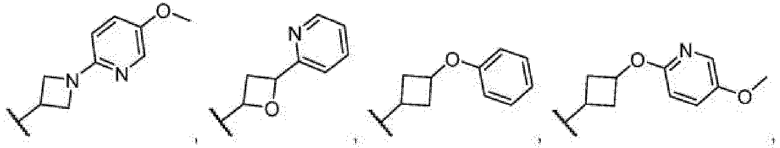

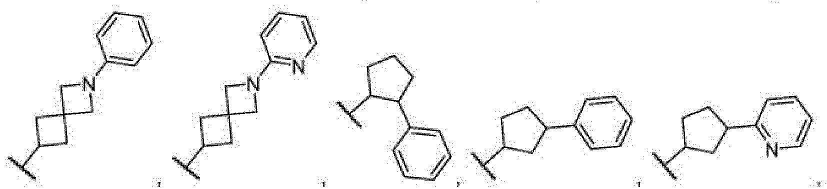

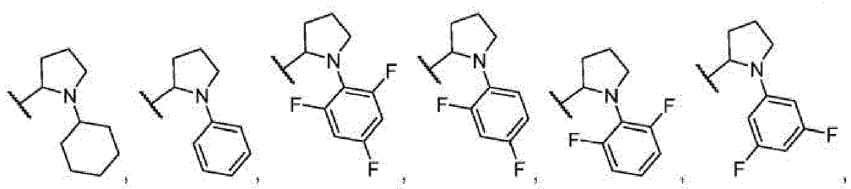

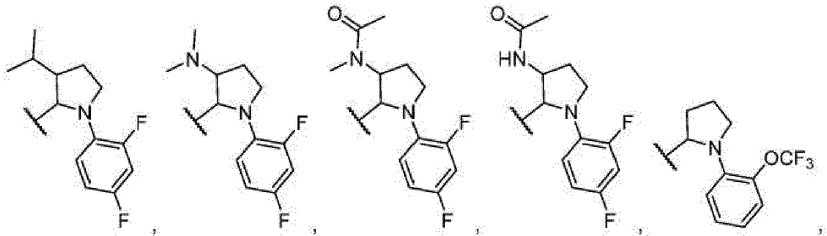

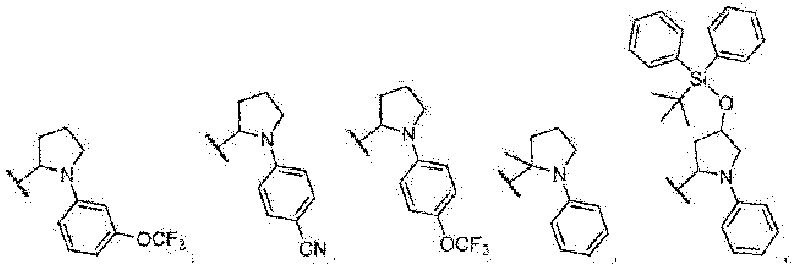

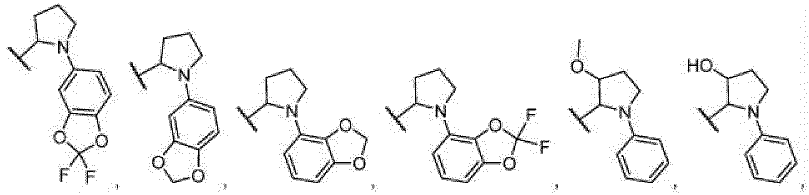

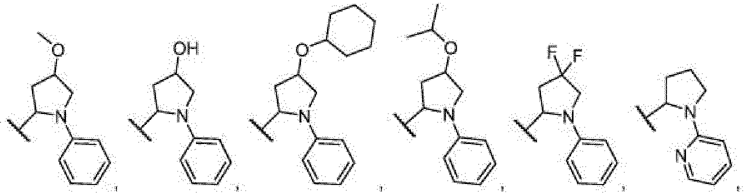

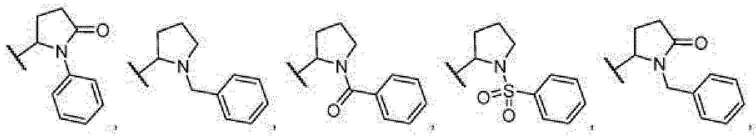

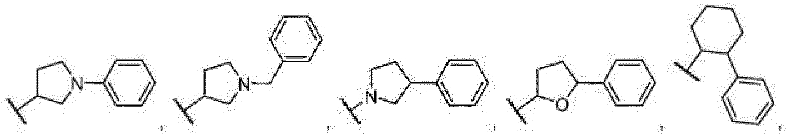

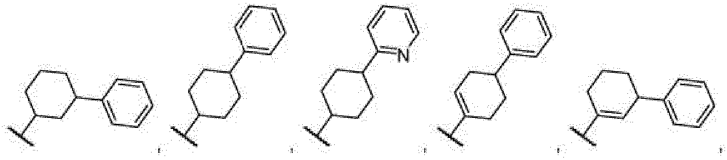

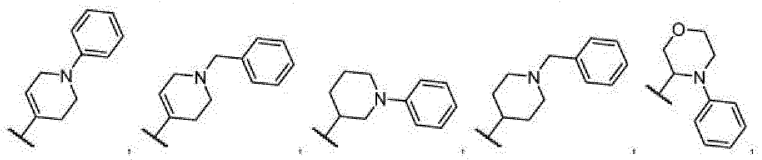

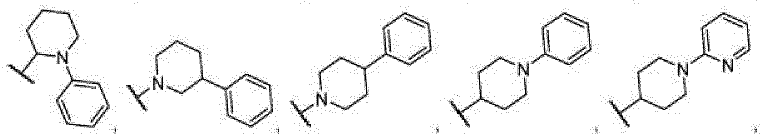

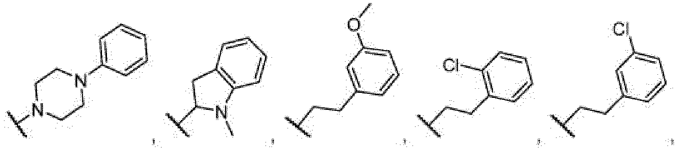

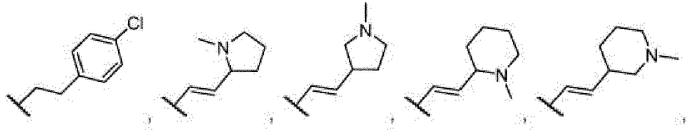

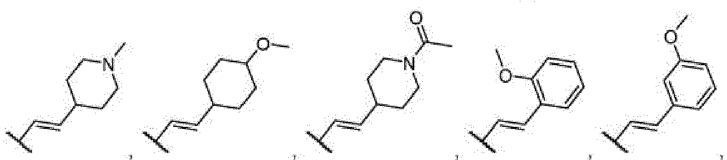

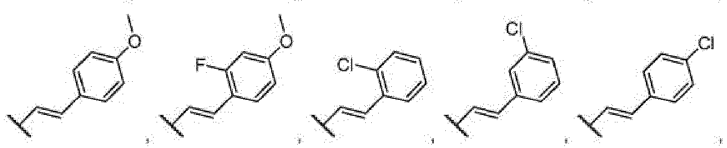

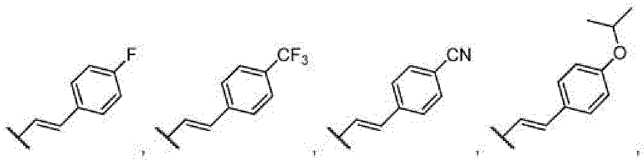

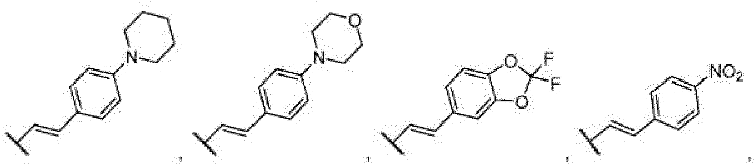

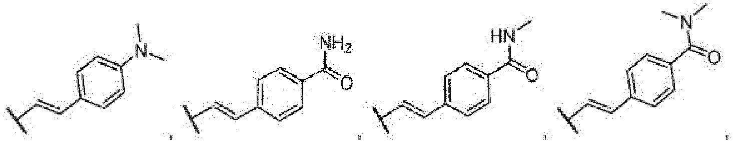

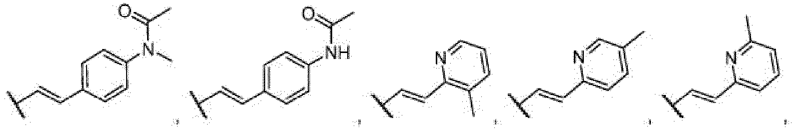

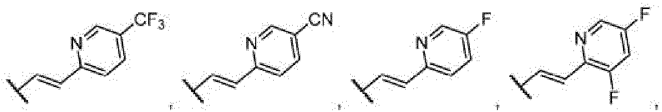

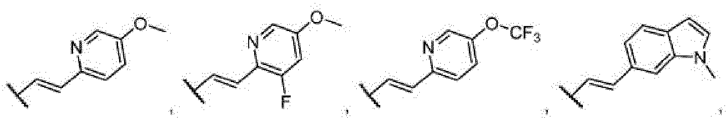

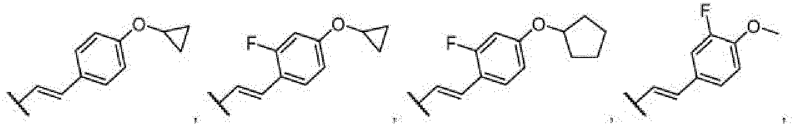

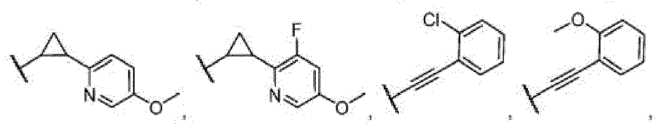

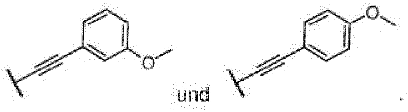

6. Verbindung oder Salz nach einem der Ansprüche 1 bis 5, worin L² C₁-Alkylen ist:

7. Verbindung oder Salz nach einem der Ansprüche 1 bis 5, worin m = 1 oder 2 ist.

8. Verbindung oder Salz nach Anspruch 1, die eine Struktur der Formel (IIIA) aufweisen: 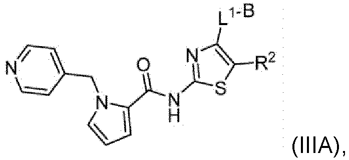 worin L¹-B aus der aus Folgendem bestehenden Gruppe ausgewählt ist:

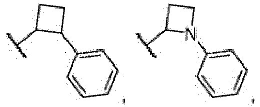

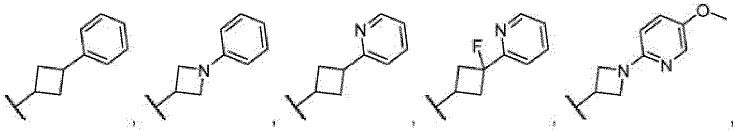

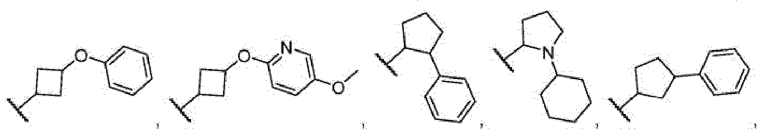

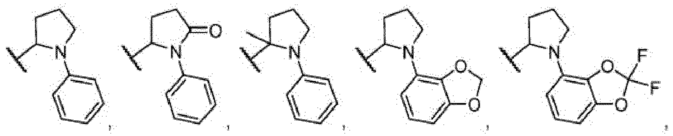

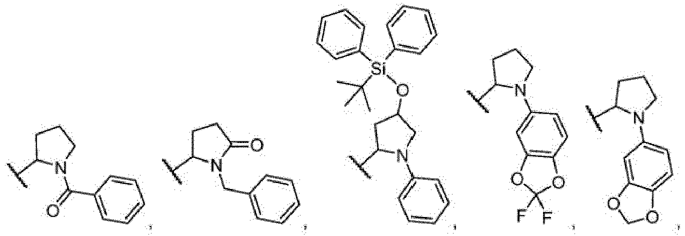

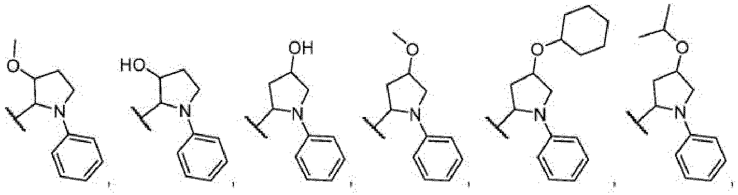

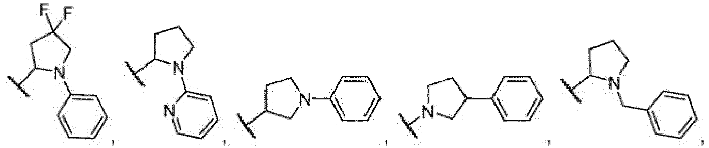

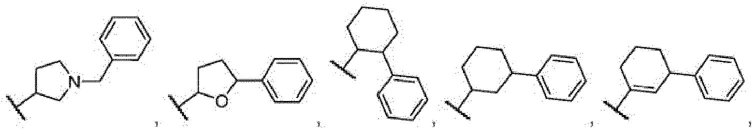

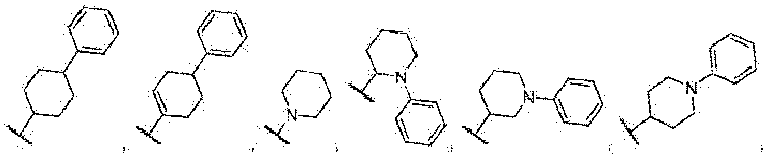

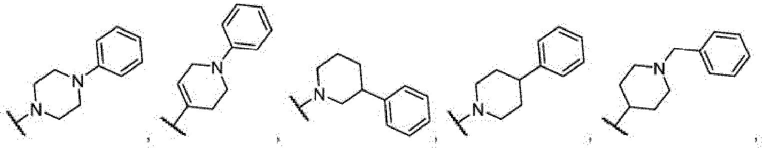

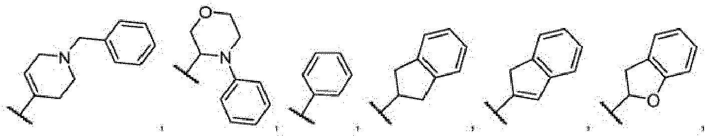

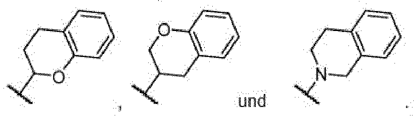

9. Verbindung oder Salz nach Anspruch 1, die einer Struktur der Formel (IIIA) aufweisen: 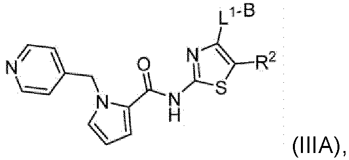 worin L¹-B aus der aus Folgendem bestehenden Gruppe ausgewählt ist:

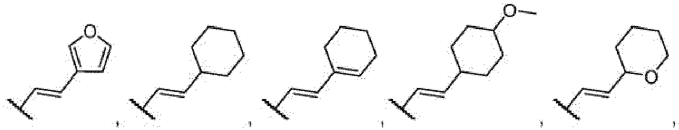

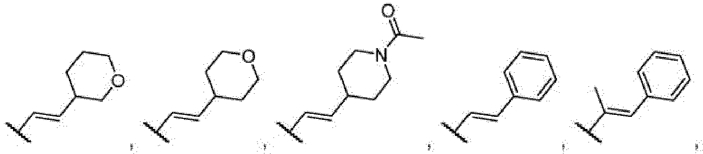

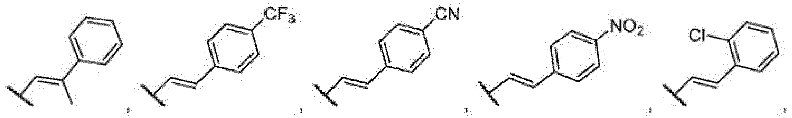

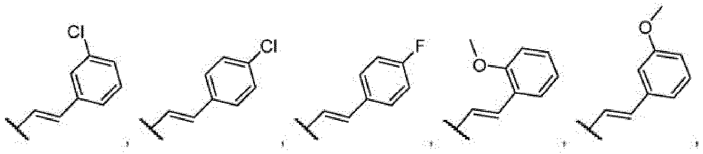

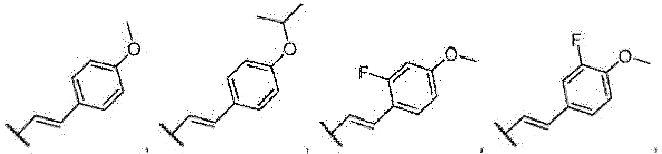

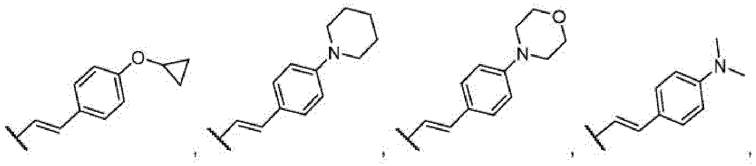

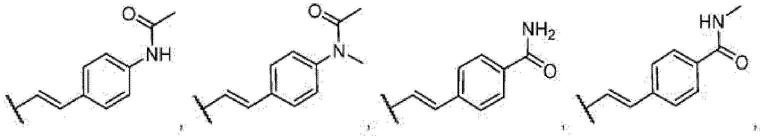

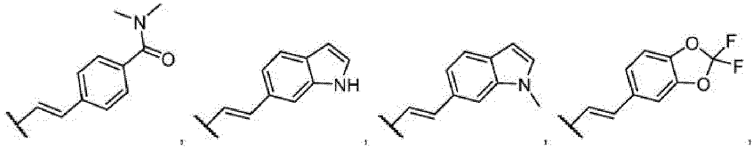

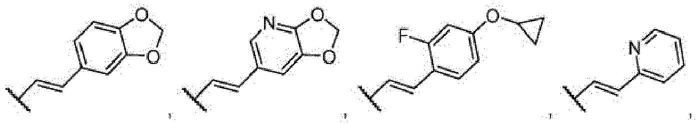

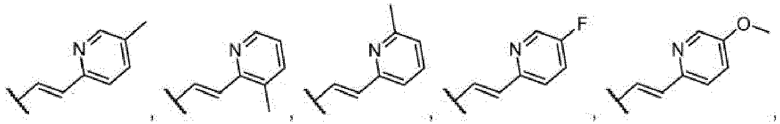

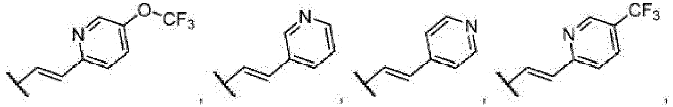

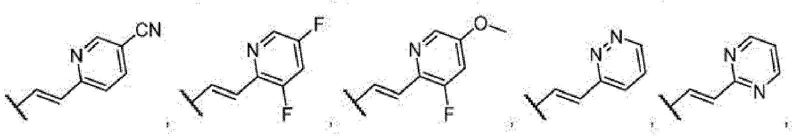

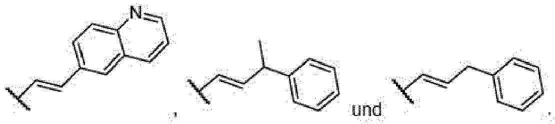

10. Verbindung oder Salz nach Anspruch 1, wobei die Verbindung oder das Salz aus der aus Folgendem bestehenden Gruppe ausgewählt ist:

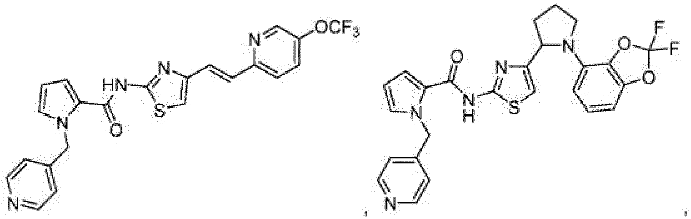

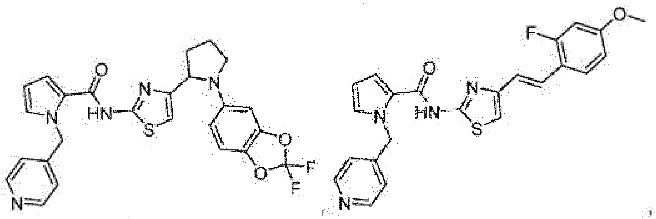

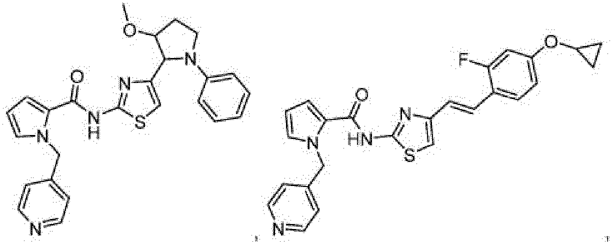

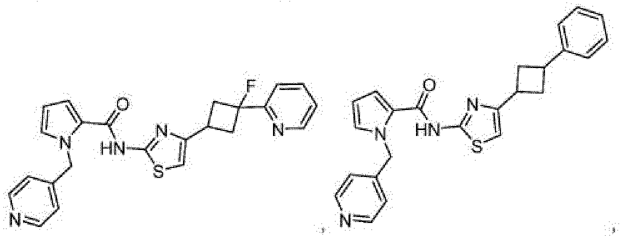

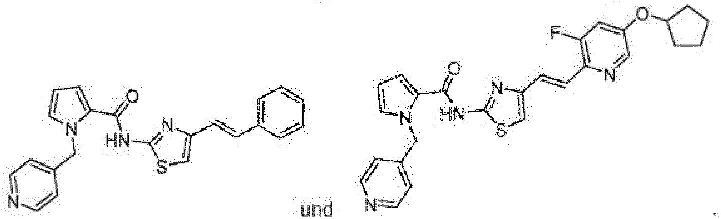

11. Verbindung wie in Tabelle A aufgelistet oder ein pharmazeutisch annehmbares Salz davon:
**Tabelle A**
| | |
|---|---|
| **A1** | 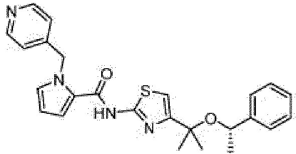 |
| **A2** | 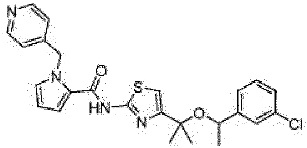 |
| **A3** | 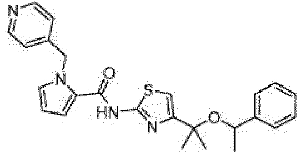 |
| **A4** | 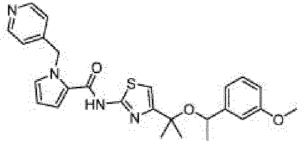 |
| **A5** | 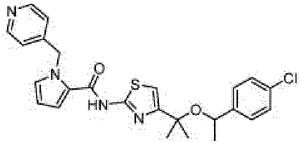 |
| **A6** | 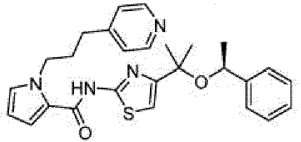 |
| **A7** | 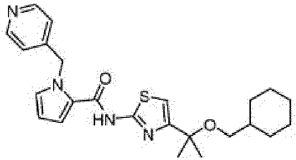 |
| **A8** | 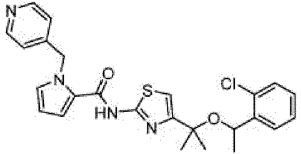 |
| **A9** | 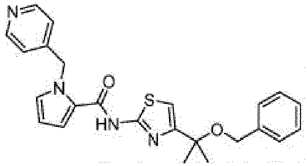 |
| **A10** | 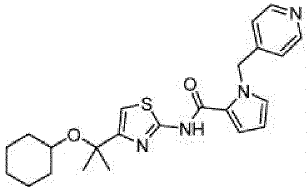 |
| **A11** | 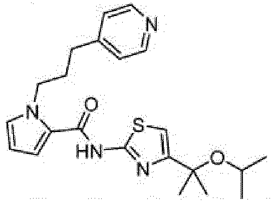 |
| **A12** | 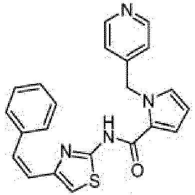 |
| **A13** | 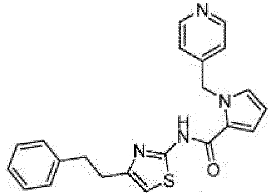 |
| **A14** | 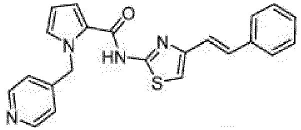 |
| **A15** | 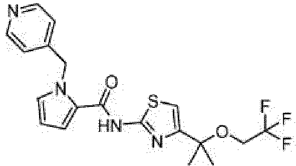 |
| **A16** | 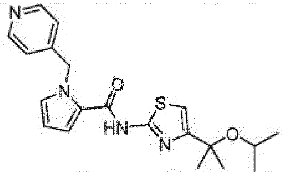 |
| **A17** | 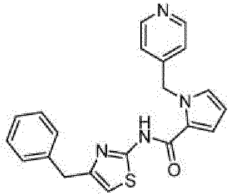 |
| **A18** | 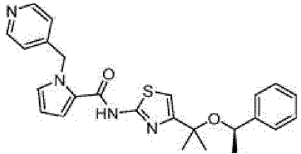 |
| **A19** | 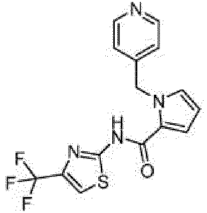 |
| **A20** | 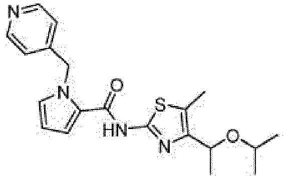 |
| **A21** | 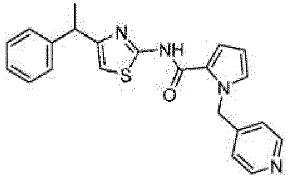 |
| **A22** | 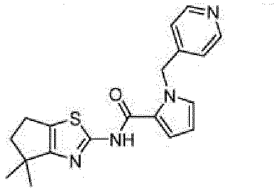 |
| **A23** | 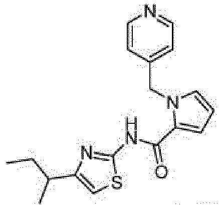 |
| **A24** | 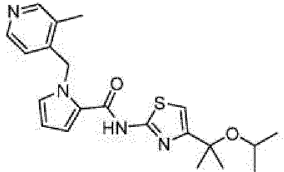 |
| **A25** | 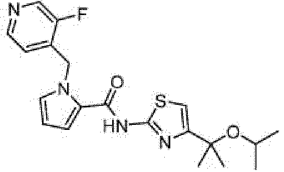 |
| **A26** | 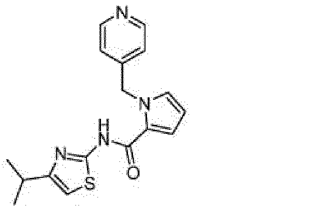 |
| **A27** | 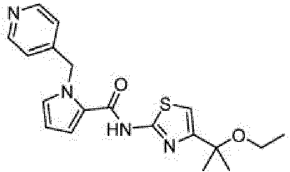 |
| **A28** | 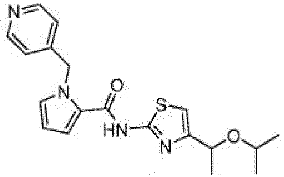 |
| **A29** | 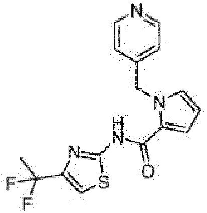 |
| **A30** | 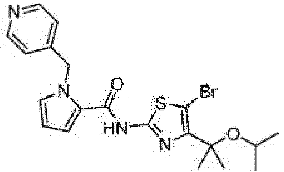 |
| **A31** | 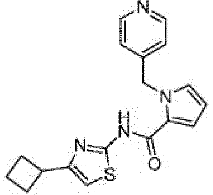 |
| **A32** | 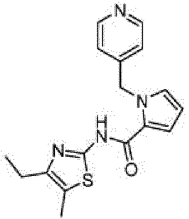 |
| **A33** | 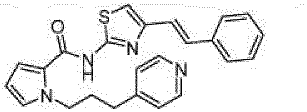 |
| **A34** | 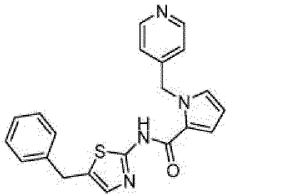 |
| **A35** | 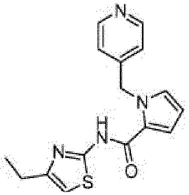 |
| **A36** | 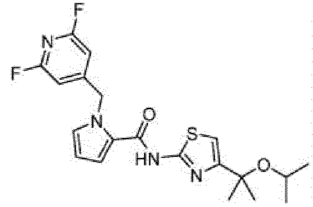 |
| **A37** | 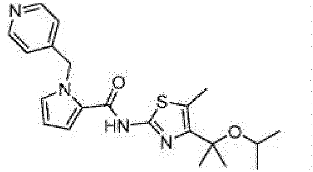 |
| **A38** | 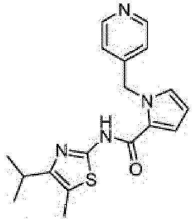 |
| **A39** | 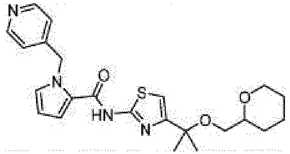 |
| **A40** | 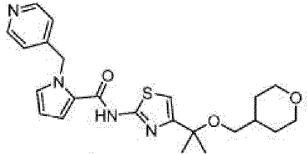 |
| **A41** | 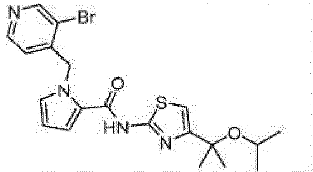 |
| **A42** | 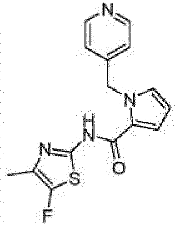 |
| **A43** | 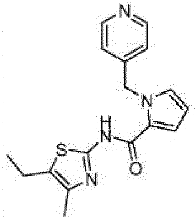 |
| **A44** | 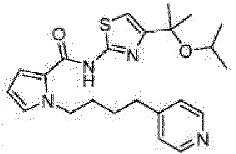 |
| **A45** | 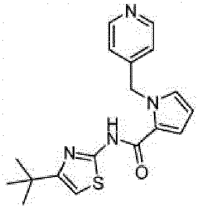 |
| **A46** | 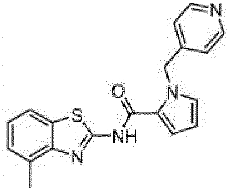 |
| **A47** | 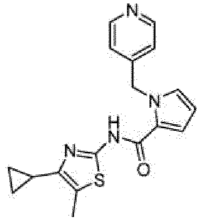 |
| **A48** | 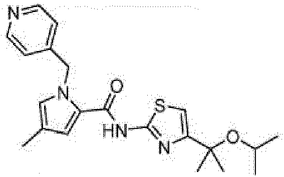 |
| **A49** | 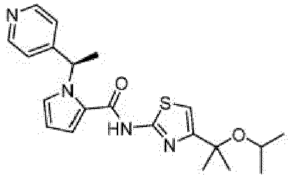 |
| **A50** | 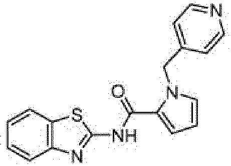 |
| **A51** | 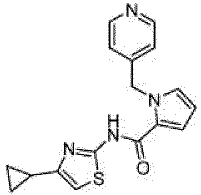 |
| **A52** | 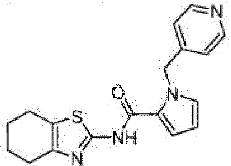 |
| **A53** | 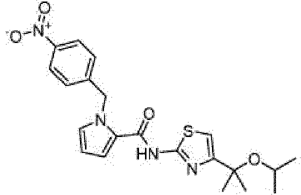 |
| **A54** | 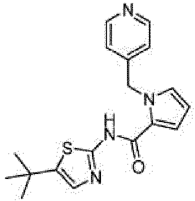 |
| **A55** | 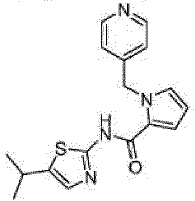 |
| **A56** | 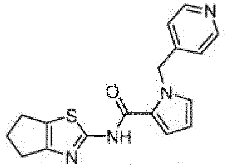 |
| **A57** | 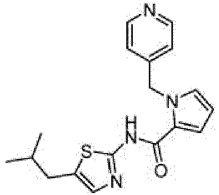 |
| **A58** | 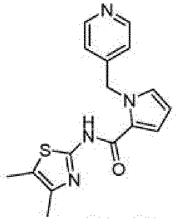 |
| **A59** | 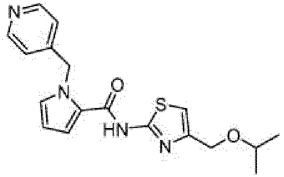 |
| **A60** | 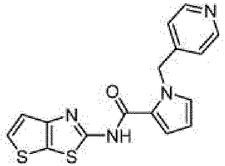 |
| **A61** | 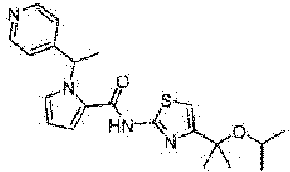 |
| **A62** | 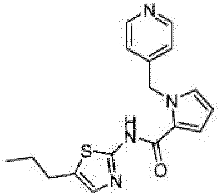 |
| **A63** | 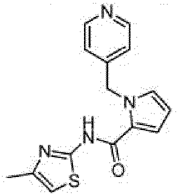 |
| **A64** | 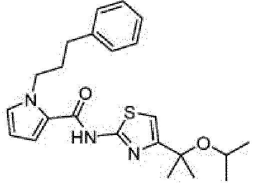 |
| **A65** | 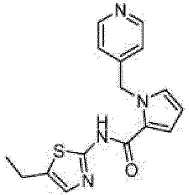 |
| **A66** | 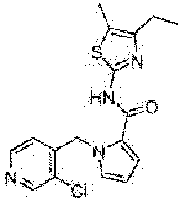 |
| **A67** | 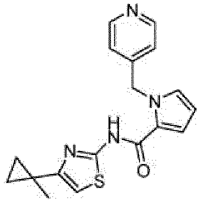 |
| **A68** | 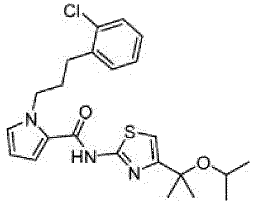 |
| **A69** | 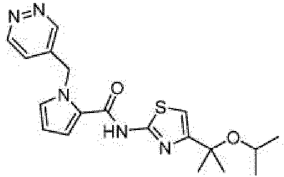 |
| **A70** | 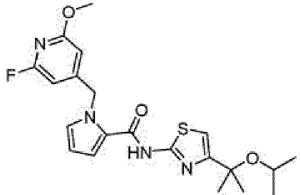 |
| **A71** | 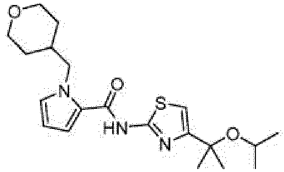 |
| **A72** | 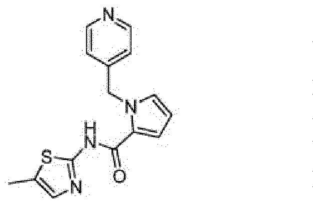 |
| **A73** | 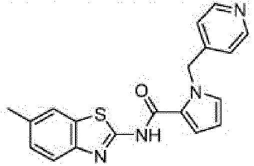 |
| **A74** | 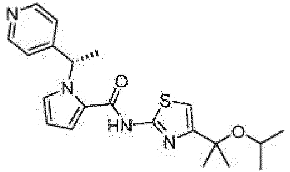 |
| **A75** | 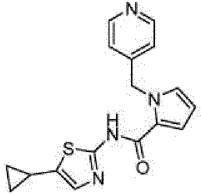 |
| **A76** | 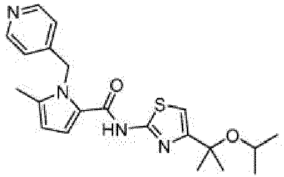 |
| **A77** | 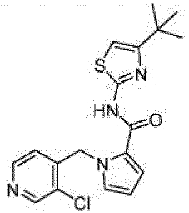 |
| **A78** | 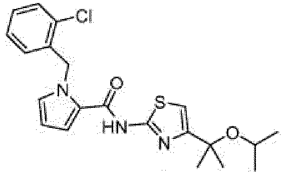 |
| **A79** | 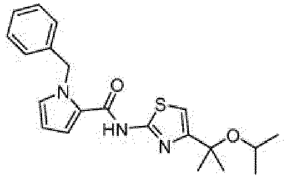 |
| **A80** | 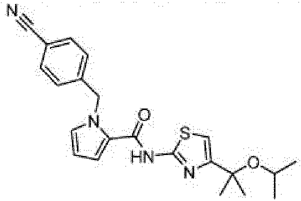 |
| **A81** | 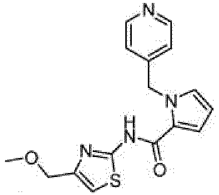 |
| **A82** | 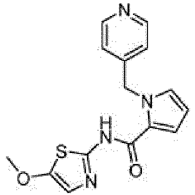 |
| **A83** | 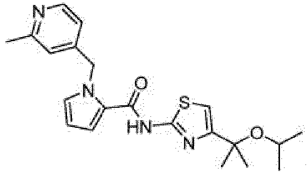 |
| **A84** | 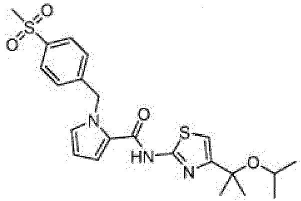 |
| **A85** | 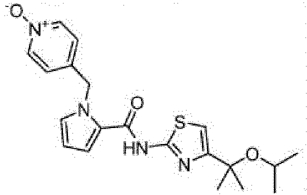 |
| **A86** | 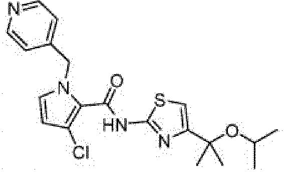 |
| **A87** | 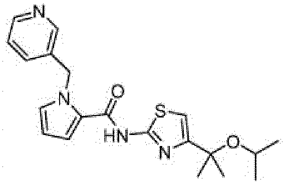 |
| **A88** | 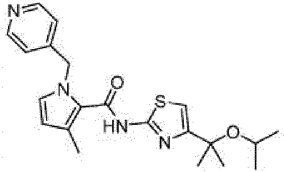 |
| **A89** | 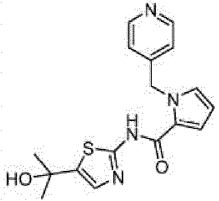 |
| **A90** | 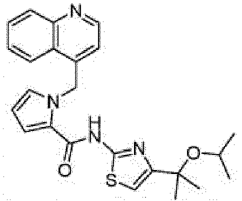 |
| **A91** | 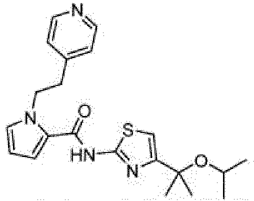 |
| **A92** | 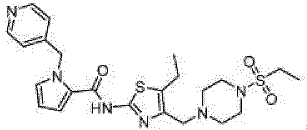 |
| **A93** | 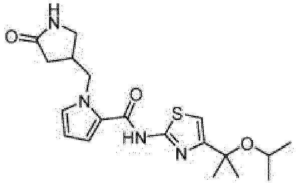 |
| **A94** | 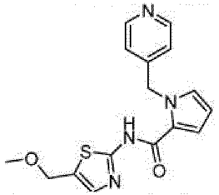 |
| **A95** | 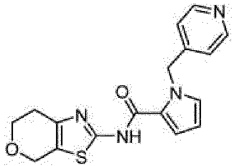 |
| **A96** | 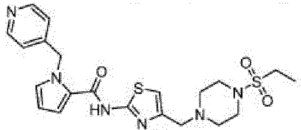 |
| **A97** | 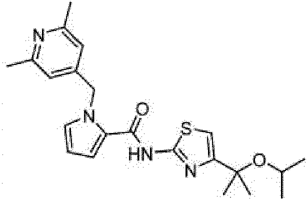 |
| **A98** | 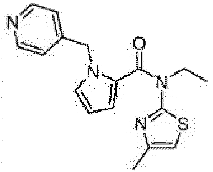 |
| **A99** | 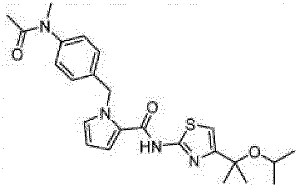 |
| **A100** | 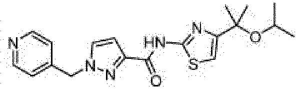 |
| **A101** | 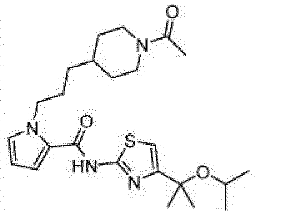 |
| **A102** | 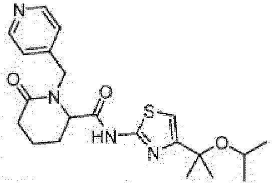 |
| **A103** | 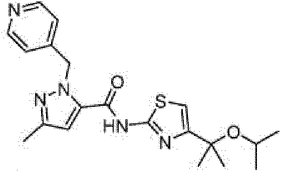 |
| **A104** | 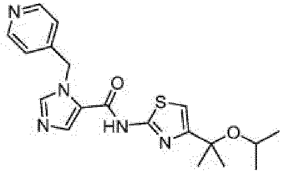 |
| **A105** | 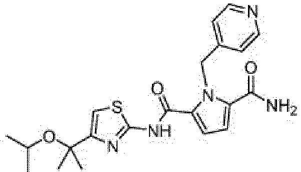 |
| **A106** | 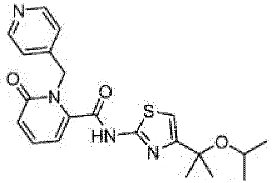 |
| **A107** | 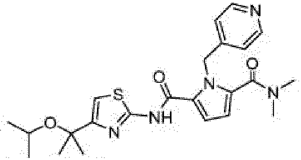 |
| **A108** | 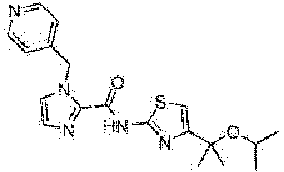 |
| **A109** | 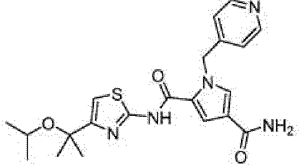 |
| **A110** | 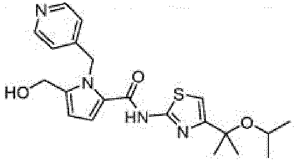 |
| **A111** | 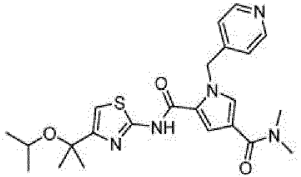 |
| **A112** | 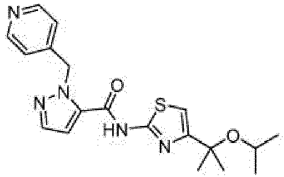 |
| **A113** | 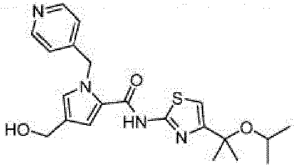 |
| **A114** | 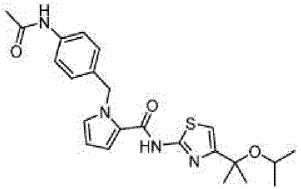 |
| **A115** | 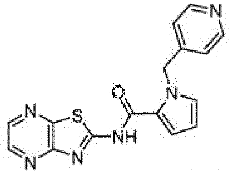 |
| **A116** | 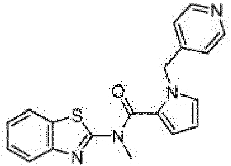 |
| **A117** | 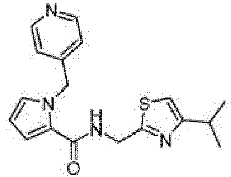 |
| **A118** | 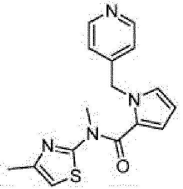 |
| **A119** | 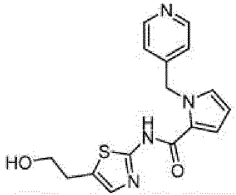 |
| **A120** | 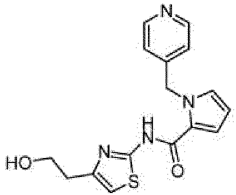 |
| **A121** | 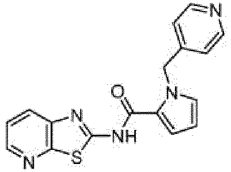 |
| **A122** | 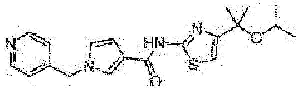 |
| **A123** | 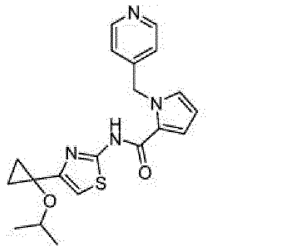 |
| **A124** | 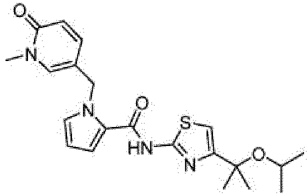 |
| **A125** | 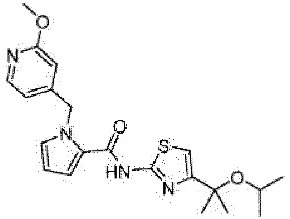 |
| **A126** | 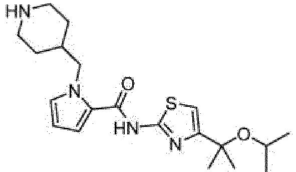 |
| **A127** | 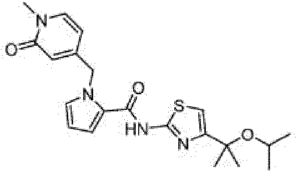 |
| **A128** | 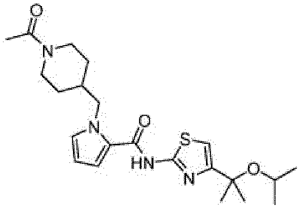 |
| **A129** | 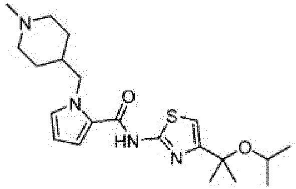 |
| **A130** | 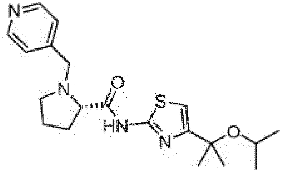 |
| **A131** | 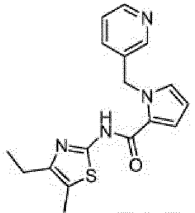 |
| **A132** | 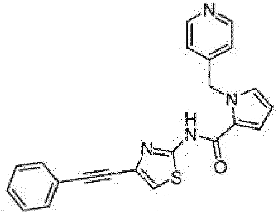 |
| **A133** | 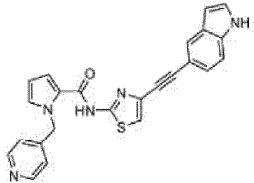 |
| **A134** | 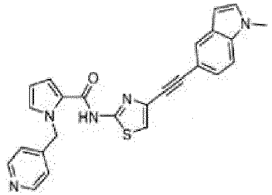 |
| **A135** | 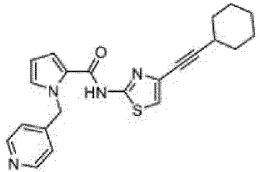 |
| **A136** | 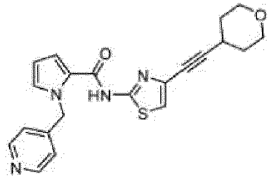 |
| **A137** | 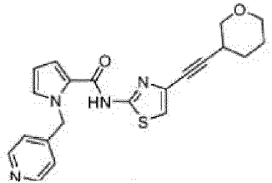 |
| **A138** | 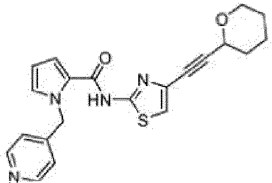 |
| **A139** | 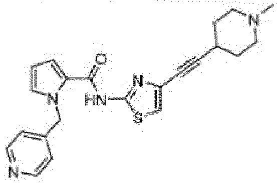 |
| **A140** | 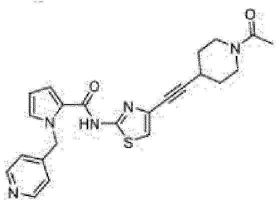 |
| **A141** | 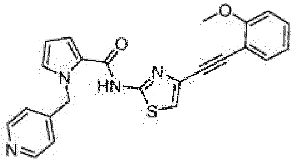 |
| **A142** | 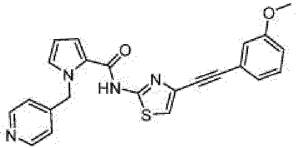 |
| **A143** | 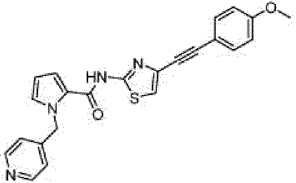 |
| **A144** | 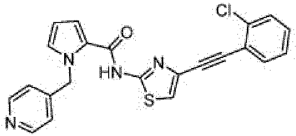 |
| **A145** | 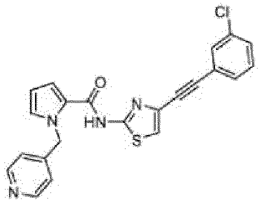 |
| **A146** | 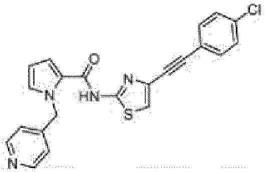 |
| **A147** | 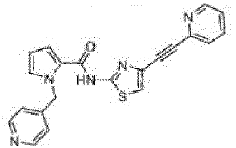 |
| **A148** | 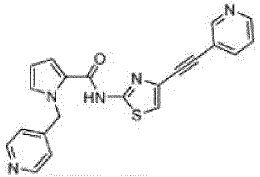 |
| **A149** | 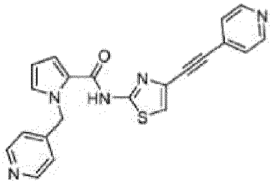 |
| **A150** | 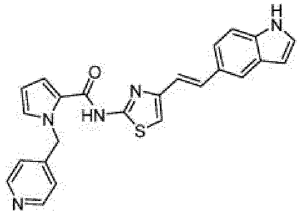 |
| **A151** | 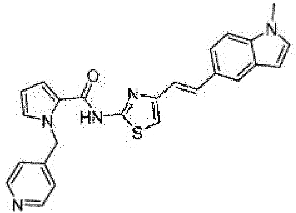 |
| **A152** | 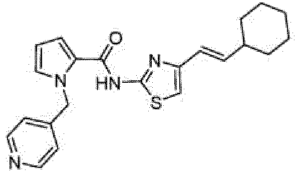 |
| **A153** | 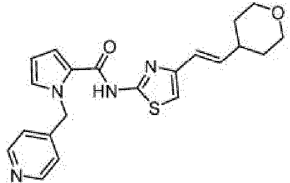 |
| **A154** | 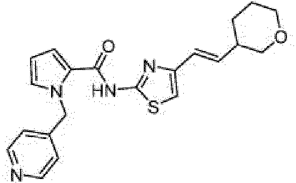 |
| **A155** | 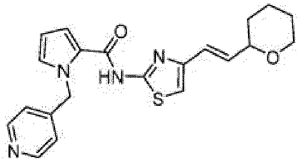 |
| **A156** | 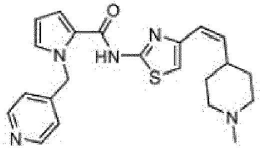 |
| **A157** | 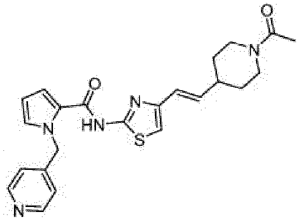 |
| **A158** | 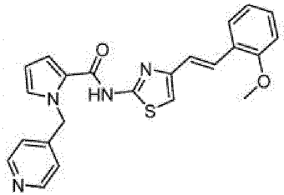 |
| **A159** | 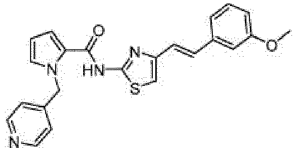 |
| **A160** | 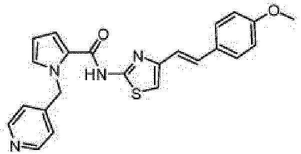 |
| **A161** | 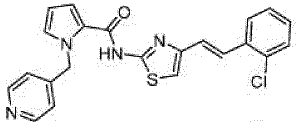 |
| **A162** | 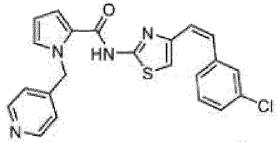 |
| **A163** | 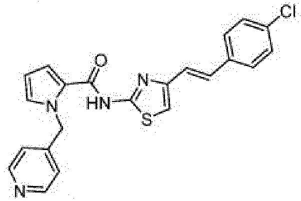 |
| **A164** | 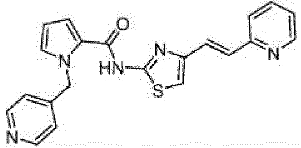 |
| **A165** | 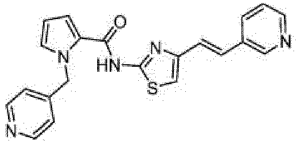 |
| **A166** | 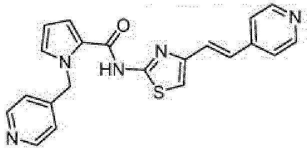 |
| **A167** | 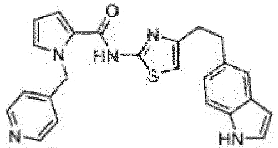 |
| **A168** | 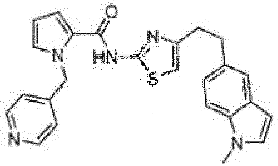 |
| **A169** | 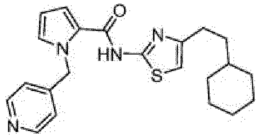 |
| **A170** | 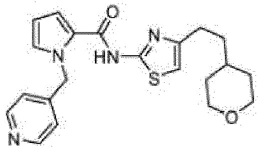 |
| **A171** | 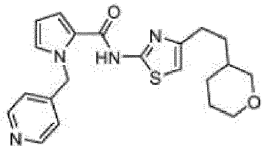 |
| **A172** | 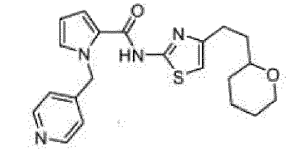 |
| **A173** | 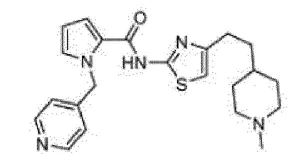 |
| **A180** | 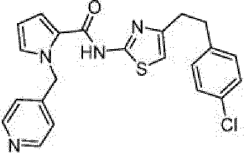 |
| **A181** | 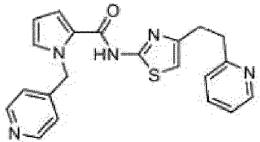 |
| **A182** | 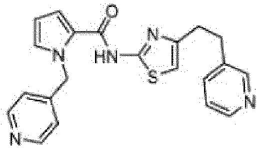 |
| **A183** | 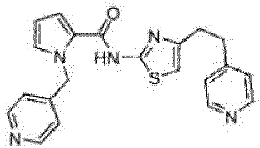 |
| **A184** | 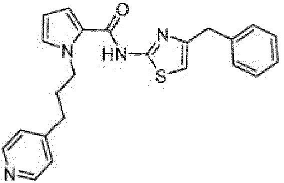 |
| **A185** | 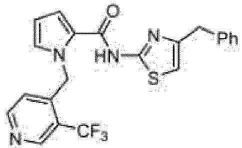 |
| **A174** | 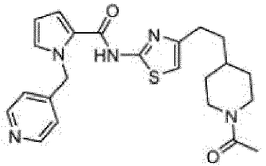 |
| **A175** | 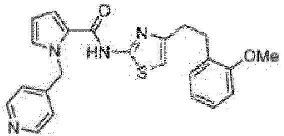 |
| **A176** | 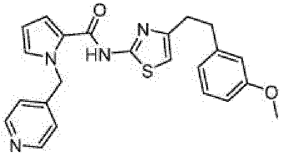 |
| **A177** | 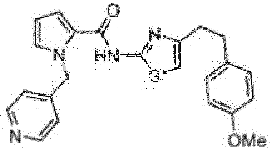 |
| **A178** | 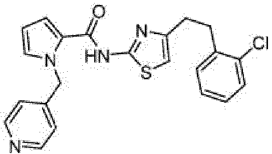 |
| **A179** | 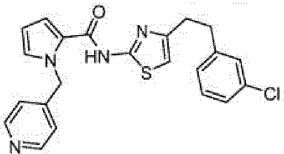 |
| **A186** | 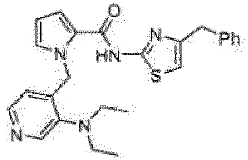 |
| **A187** | 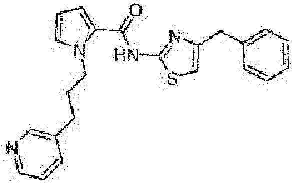 |
| **A188** | 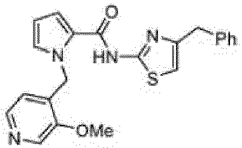 |
| **A189** | 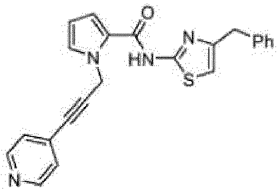 |
| **A190** | 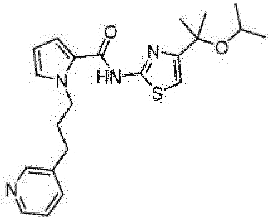 |
| **A191** | 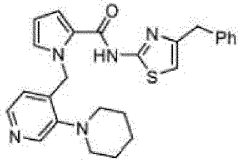 |
| **A192** | 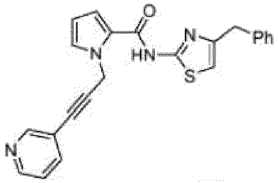 |
| **A193** | 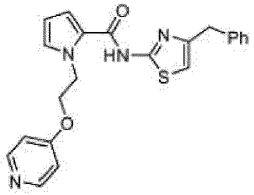 |
| **A194** | 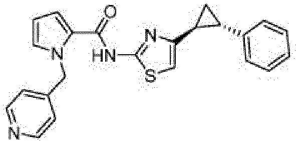 |
| **A195** | 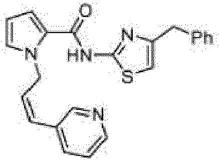 |
| **A196** | 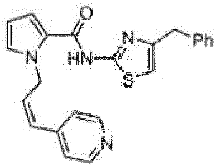 |
| **A197** | 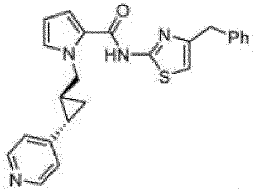 |
| **A198** | 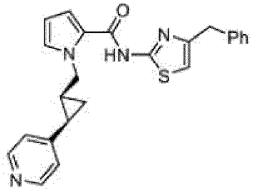 |
| **A199** | 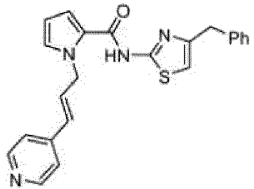 |
| **A200** | 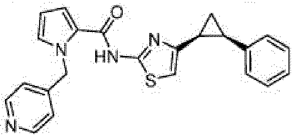 |
| **A201** | 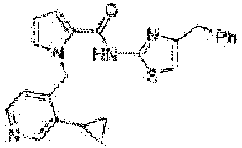 |
| **A202** | 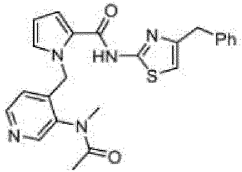 |
| **A203** | 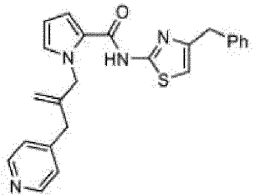 |
| **A204** | 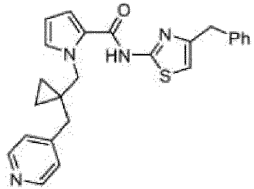 |
| **A205** | 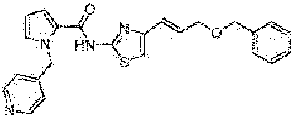 |
| **A206** | 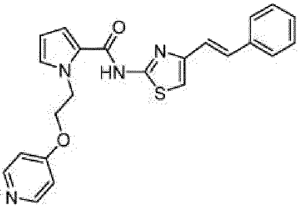 |
| **A207** | 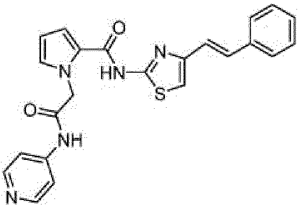 |
| **A208** | 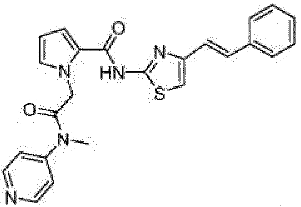 |
| **A209** | 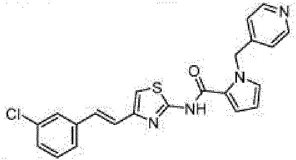 |
| **A210** | 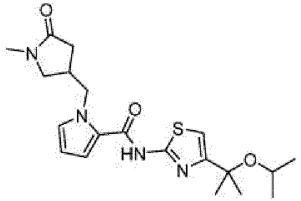 |
| **A211** | 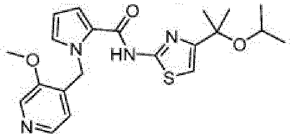 |
| **A212** | 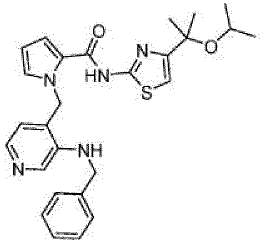 |
| **A213** | 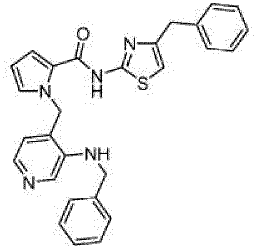 |
| **A214** | 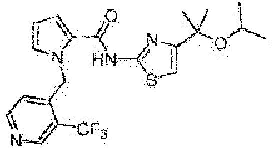 |
| **A215** | 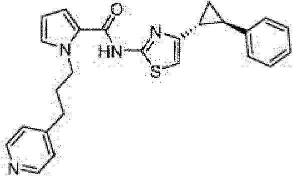 |
| **A216** | 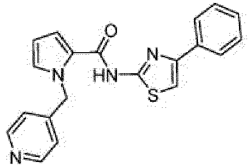 |
| **A217** | 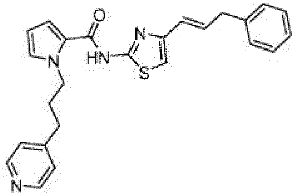 |
| **A218** | 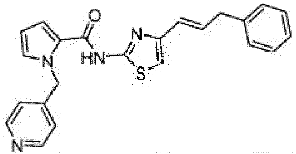 |
| **A219** | 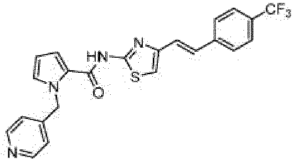 |
| **A220** | 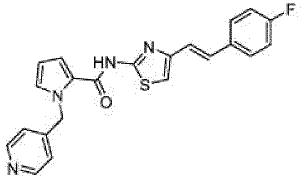 |
| **A221** | 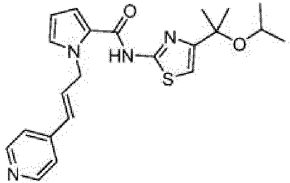 |
| **A222** | 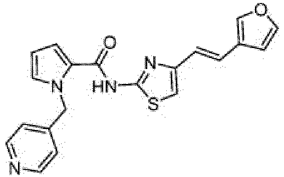 |
| **A223** | 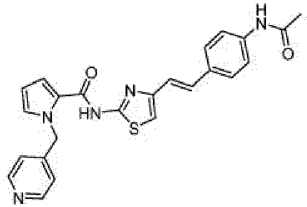 |
| **A224** | 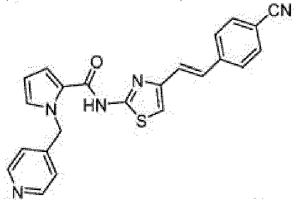 |
| **A225** | 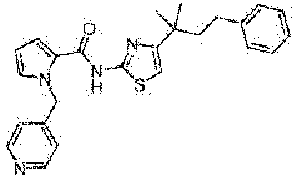 |
| **A226** | 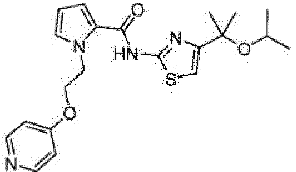 |
| **A227** | 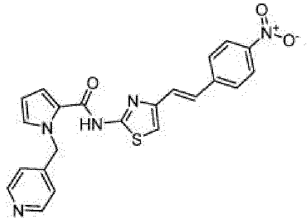 |
| **A228** | 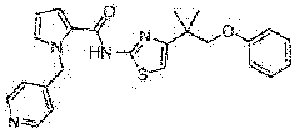 |
| **A229** | 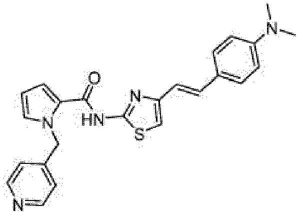 |
| **A230** | 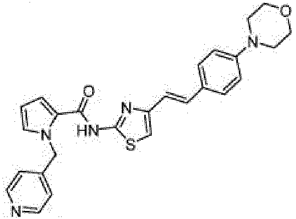 |
| **A231** | 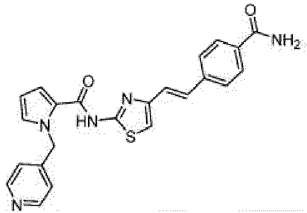 |
| **A232** | 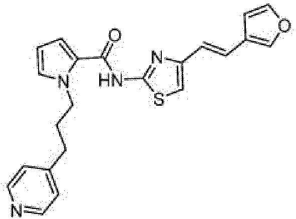 |
| **A233** | 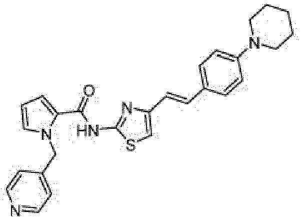 |
| **A234** | 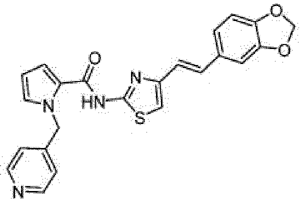 |
| **A235** | 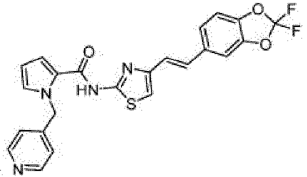 |
| **A236** | 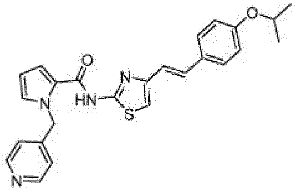 |
| **A237** | 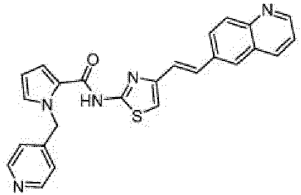 |
| **A238** | 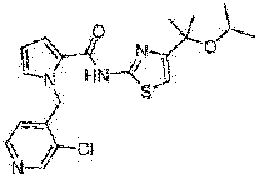 |
| **A239** | 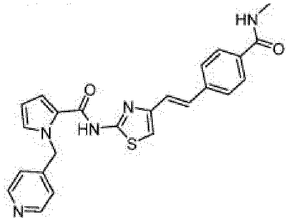 |
| **A240** | 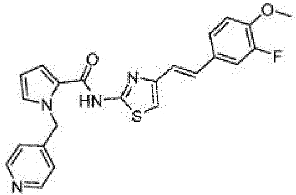 |
| **A241** | 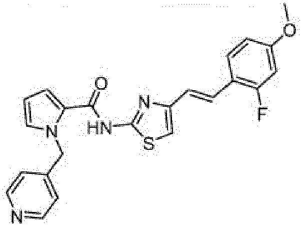 |
| **A242** | 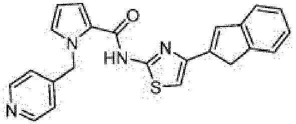 |
| **A243** | 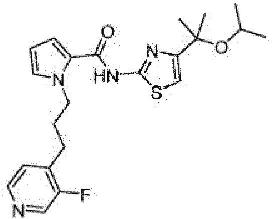 |
| **A244** | 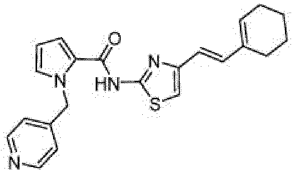 |
| **A245** | 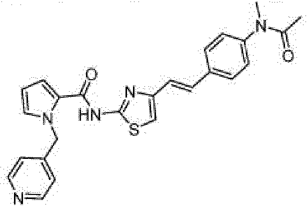 |
| **A246** | 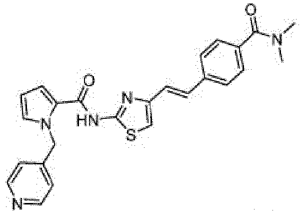 |
| **A247** | 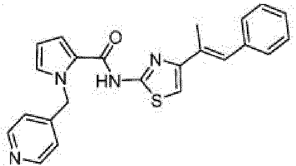 |
| **A248** | 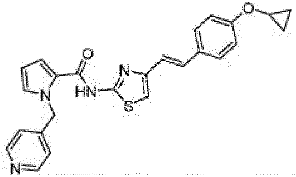 |
| **A249** | 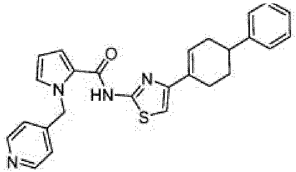 |
| **A250** | 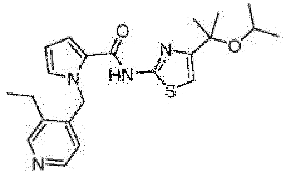 |
| **A251** | 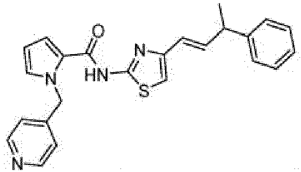 |
| **A252** | 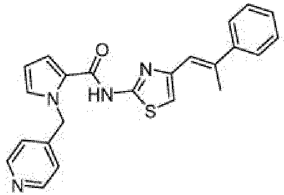 |
| **A253** | 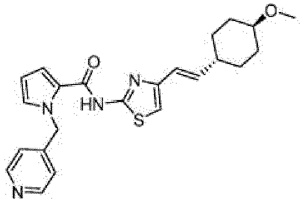 |
| **A254** | 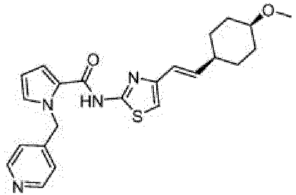 |
| **A255** | 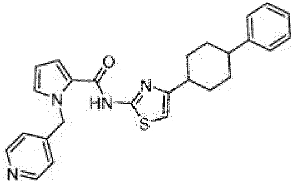 |
| **A256** | 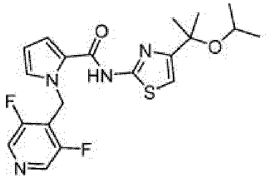 |
| **A257** | 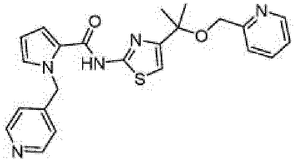 |
| **A258** | 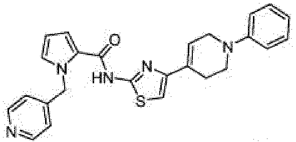 |
| **A259** | 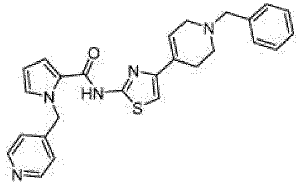 |
| **A260** | 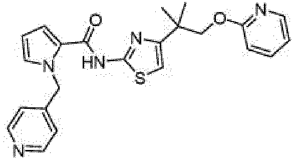 |
| **A261** | 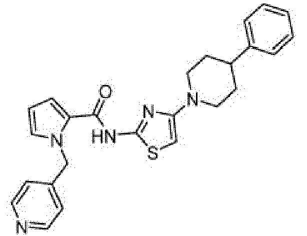 |
| **A262** | 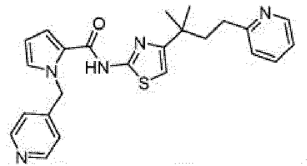 |
| **A263** | 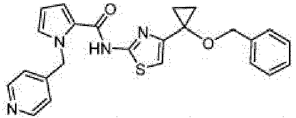 |
| **A264** | 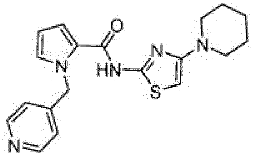 |
| **A265** | 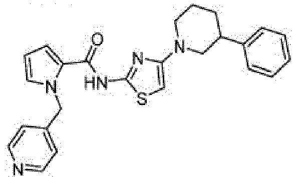 |
| **A266** | 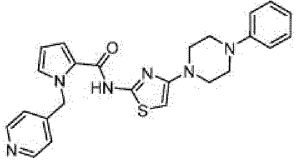 |
| **A267** | 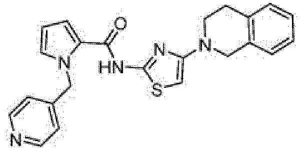 |
| **A268** | 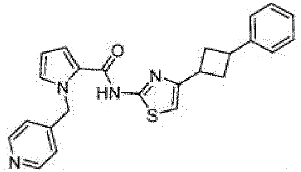 |
| **A269** | 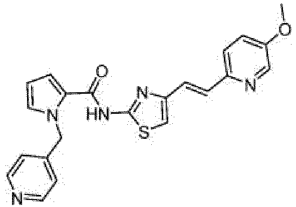 |
| **A270** | 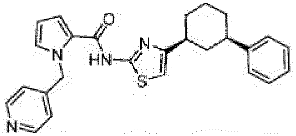 |
| **A271** | 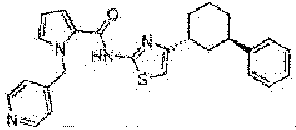 |
| **A272** | 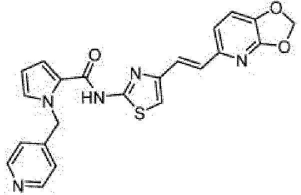 |
| **A273** | 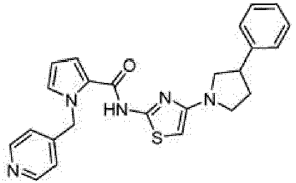 |
| **A274** | 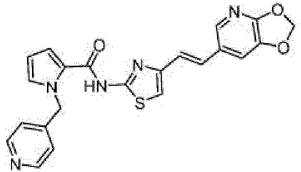 |
| **A275** | 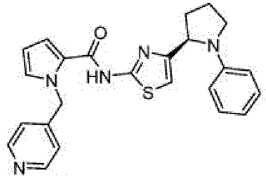 |
| **A276** | 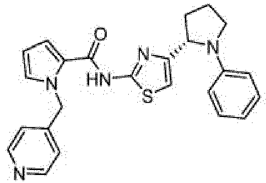 |
| **A277** | 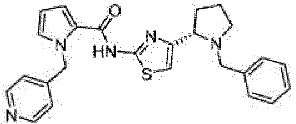 |
| **A278** | 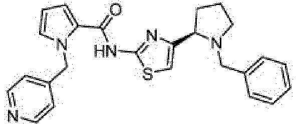 |
| **A279** | 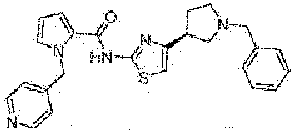 |
| **A280** | 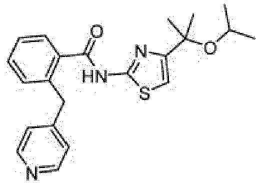 |
| **A281** | 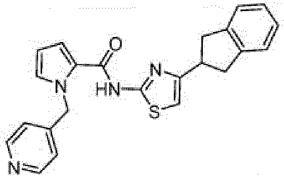 |
| **A282** | 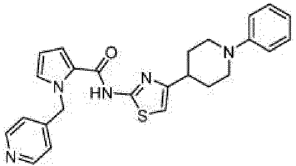 |
| **A283** | 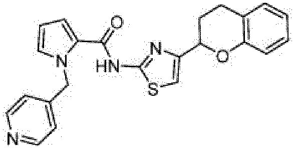 |
| **A284** | 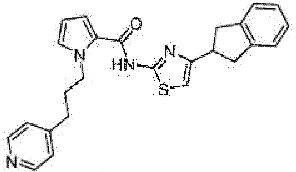 |
| **A285** | 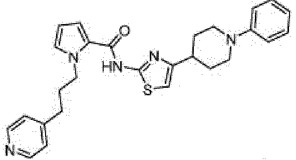 |
| **A286** | 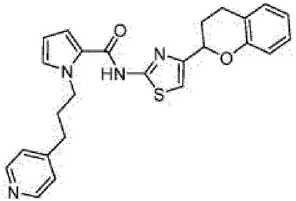 |
| **A287** | 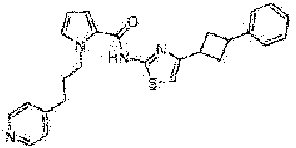 |
| **A288** | 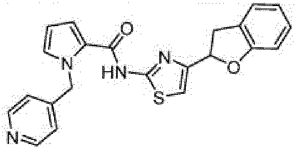 |
| **A289** | 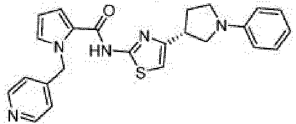 |
| **A290** | 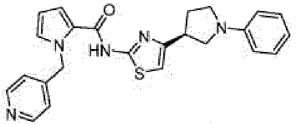 |
| **A291** | 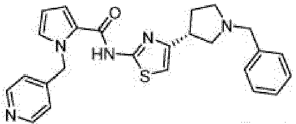 |
| **A292** | 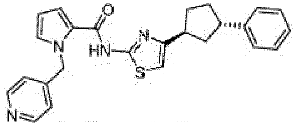 |
| **A293** | 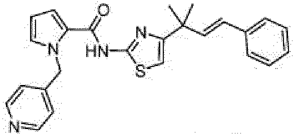 |
| **A294** | 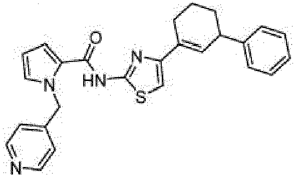 |
| **A295** | 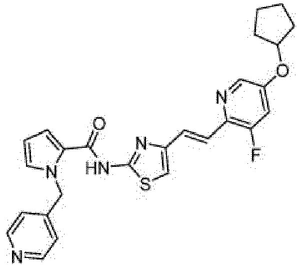 |
| **A296** | 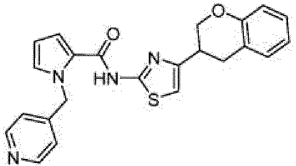 |
| **A297** | 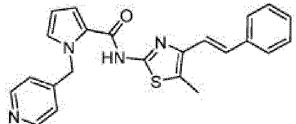 |
| **A298** | 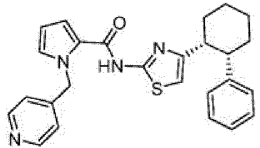 |
| **A299** | 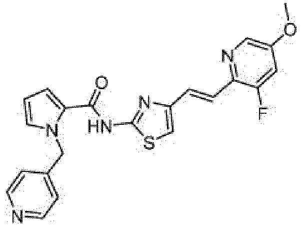 |
| **A300** | 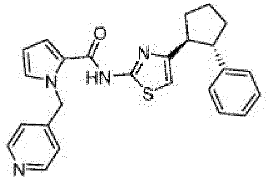 |
| **A301** | 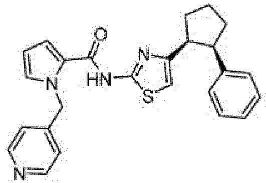 |
| **A302** | 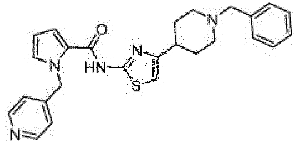 |
| **A303** | 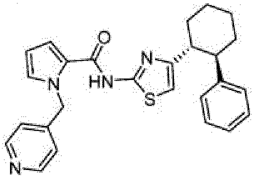 |
| **A304** | 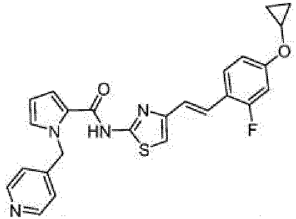 |
| **A305** | 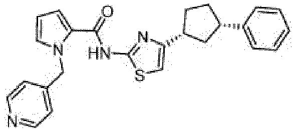 |
| **A306** | 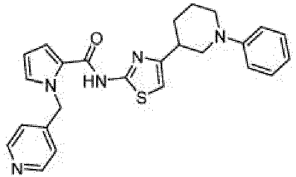 |
| **A307** | 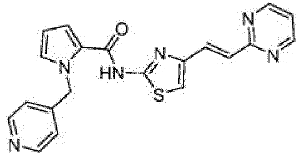 |
| **A308** | 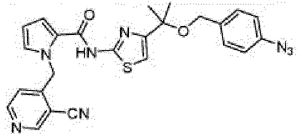 |
| **A309** | 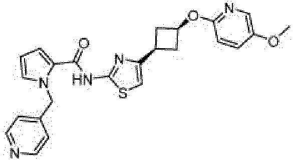 |
| **A310** | 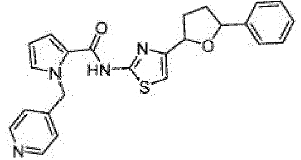 |
| **A311** | 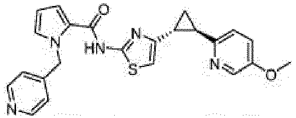 |
| **A312** | 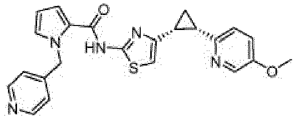 |
| **A313** | 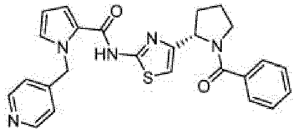 |
| **A314** | 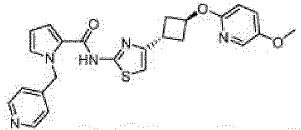 |
| **A315** | 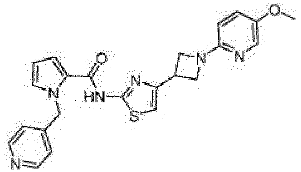 |
| **A316** | 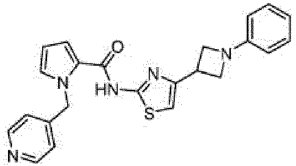 |
| **A317** | 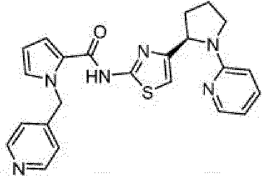 |
| **A318** | 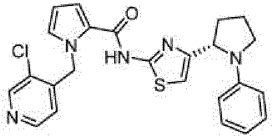 |
| **A319** | 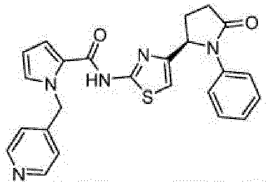 |
| **A320** | 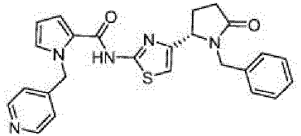 |
| **A321** | 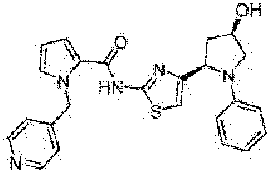 |
| **A322** | 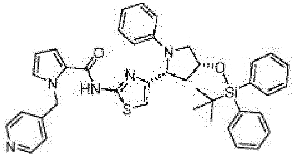 |
| **A323** | 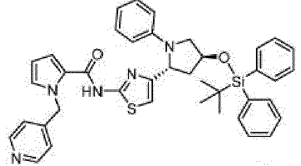 |
| **A324** | 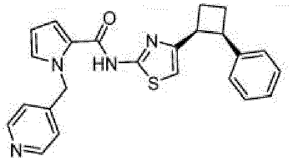 |
| **A325** | 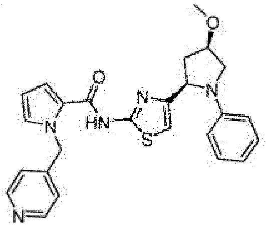 |
| **A326** | 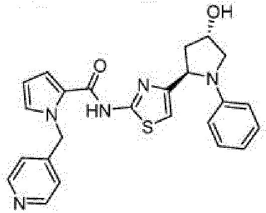 |
| **A327** | 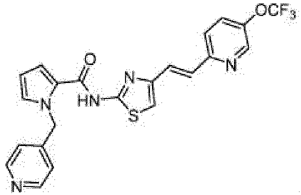 |
| **A328** | 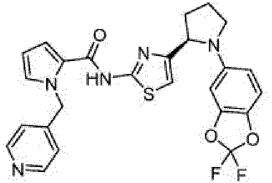 |
| **A329** | 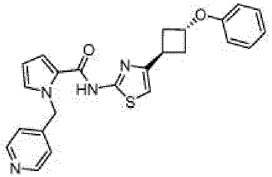 |
| **A330** | 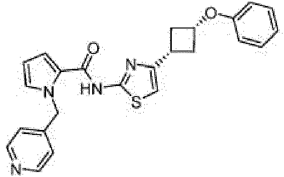 |
| **A331** | 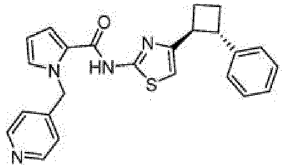 |
| **A332** | 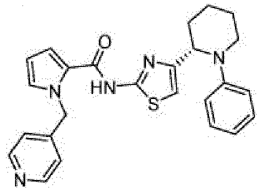 |
| **A333** | 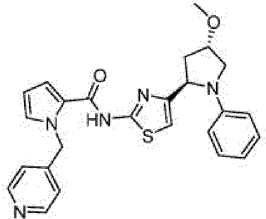 |
| **A334** | 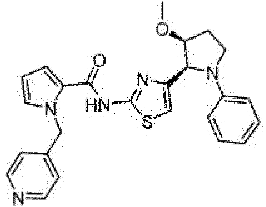 |
| **A335** | 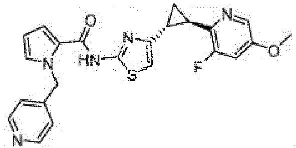 |
| **A336** | 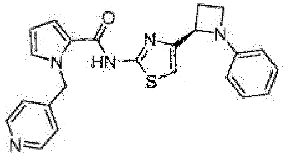 |
| **A337** | 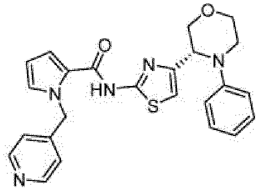 |
| **A338** | 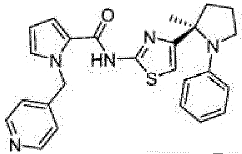 |
| **A339** | 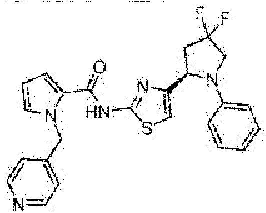 |
| **A340** | 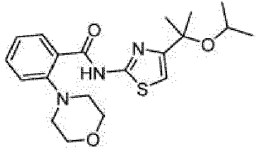 |
| **A341** | 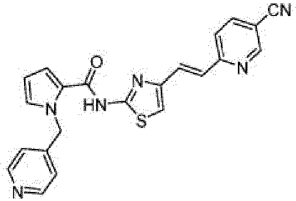 |
| **A342** | 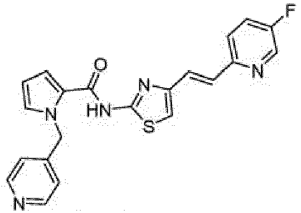 |
| **A343** | 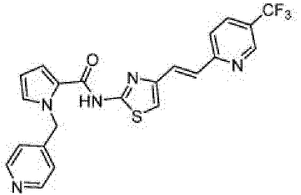 |
| **A344** | 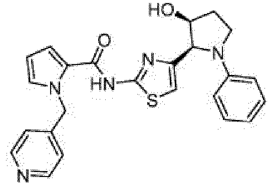 |
| **A345** | 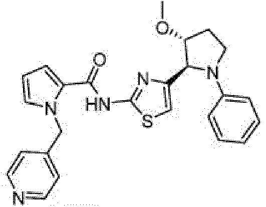 |
| **A346** | 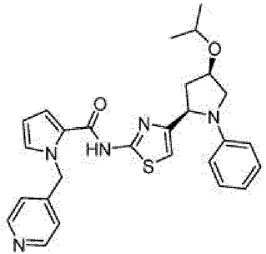 |
| **A347** | 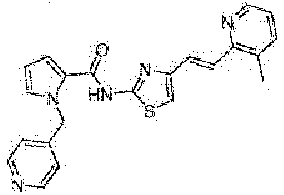 |
| **A348** | 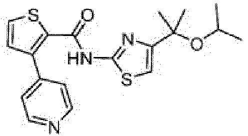 |
| **A349** | 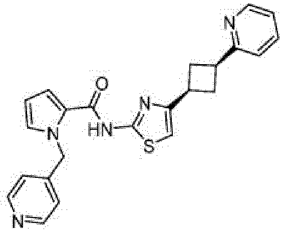 |
| **A350** | 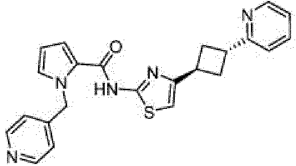 |
| **A351** | 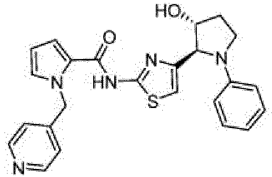 |
| **A352** | 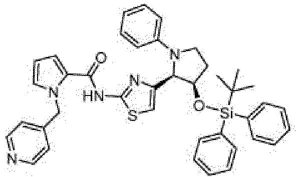 |
| **A353** | 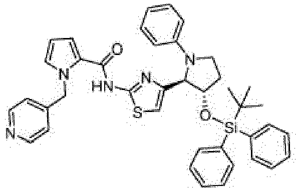 |
| **A354** | 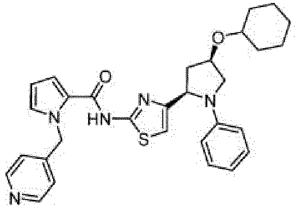 |
| **A355** | 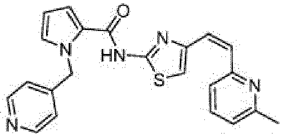 |
| **A356** | 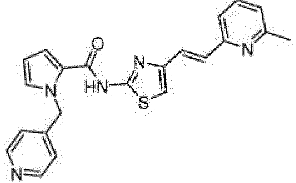 |
| **A357** | 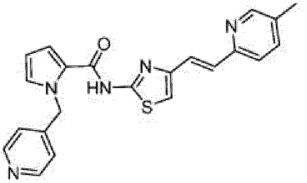 |
| **A358** | 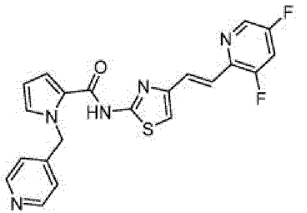 |
| **A359** | 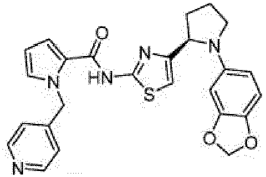 |
| **A360** | 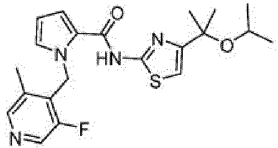 |
| **A361** | 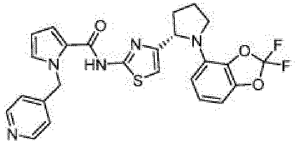 |
| **A362** | 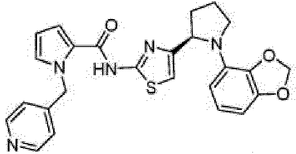 |
| **A363** | 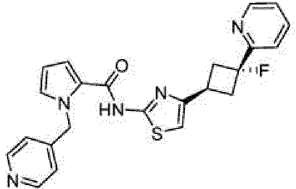 |
| **A364** | 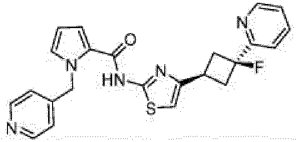 |
| **A356** | 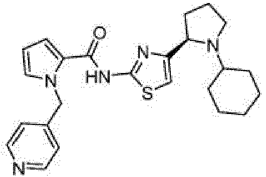 |
| **A366** | 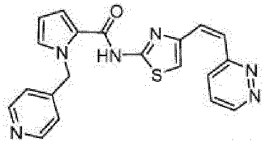 |
| **A367** | 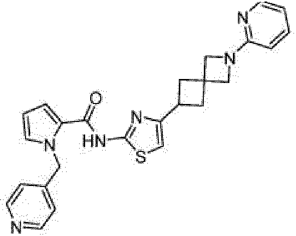 |
| **A368** | 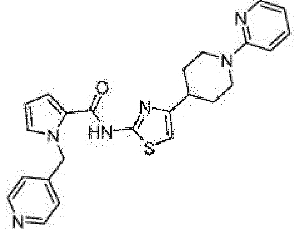 |
| **A369** | 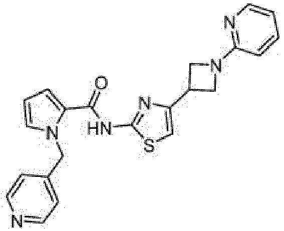 |
| **A370** | 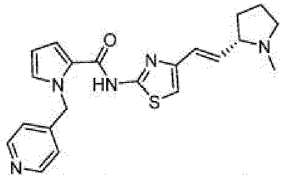 |
| **A371** | 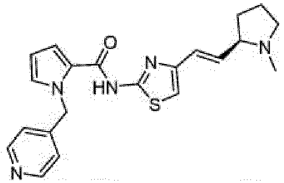 |
| **A372** | 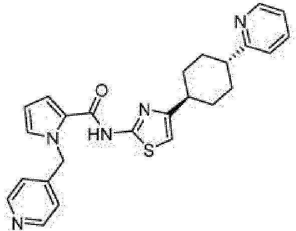 |
| **A373** | 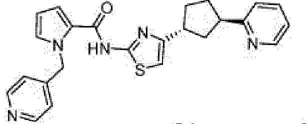 |
| **A374** | 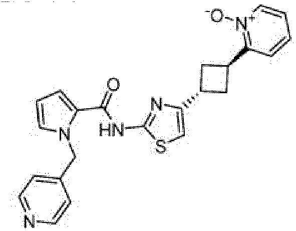 |
| **A375** | 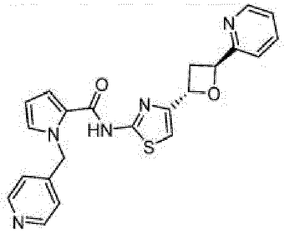 |
| **A376** | 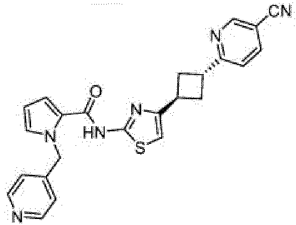 |
| **A377** | 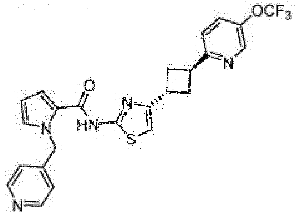 |
| **A378** | 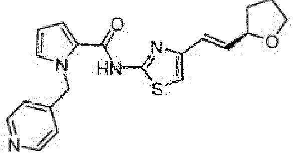 |
| **A379** | 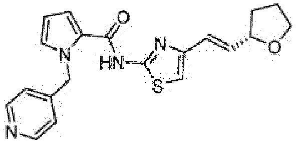 |
| **A380** | 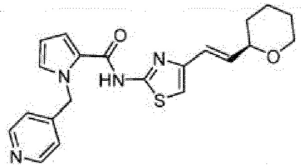 |
| **A381** | 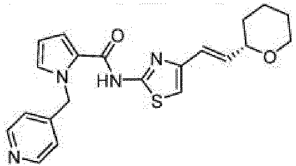 |
| **A382** | 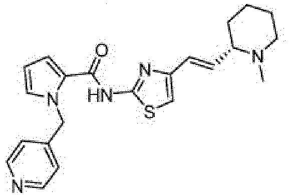 |
| **A383** | 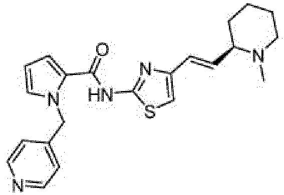 |
| **A384** | 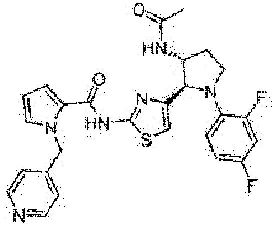 |
| **A385** | 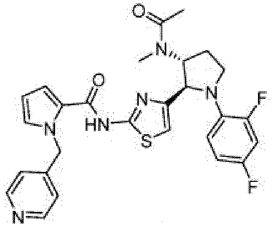 |
| **A386** | 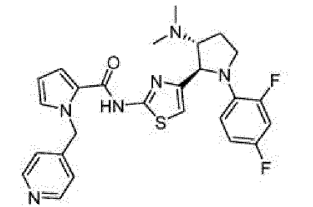 |
| **A387** | 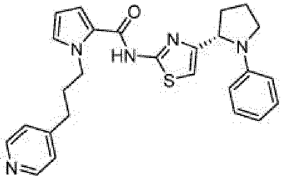 |
| **A388** | 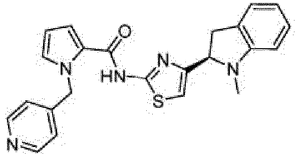 |
| **A389** | 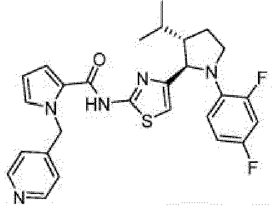 |
| **A390** | 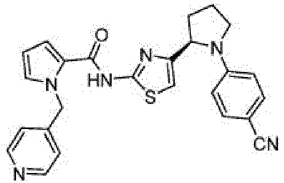 |
| **A391** | 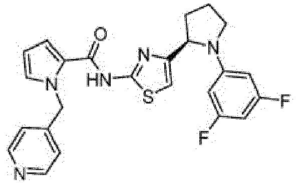 |
| **A392** | 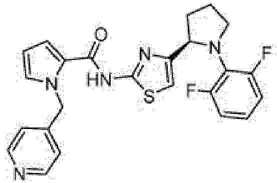 |
| **A393** | 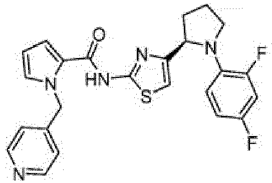 |
| **A394** | 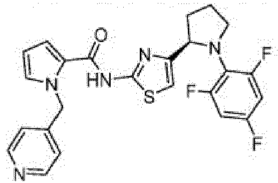 |
| **A395** | 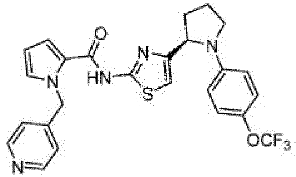 |
| **A396** | 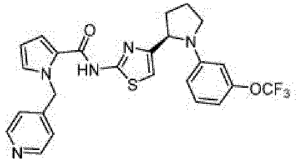 |
| **A397** | 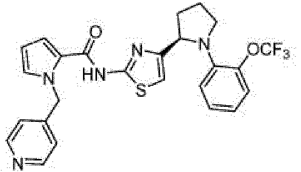 |
| **A398** | 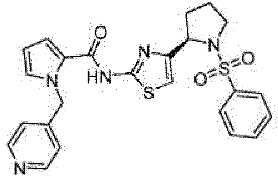 |
| **A399** | 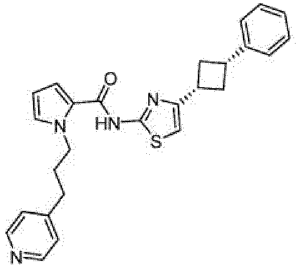 |
| **A400** | 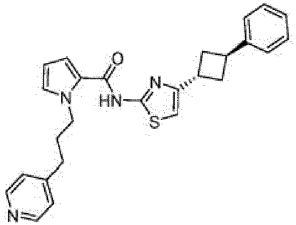 |
| **A401** | 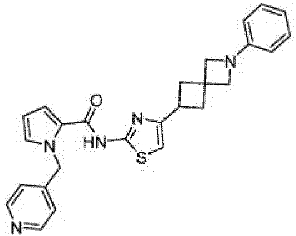 |
| **A402** | 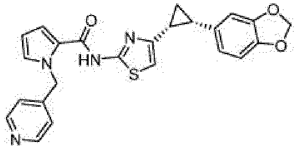 |
| **A403** | 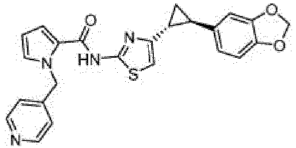 |

12. Verbindung oder Salz nach Anspruch 11, wobei die Verbindung oder das Salz aus der aus Folgendem bestehenden Gruppe ausgewählt ist: 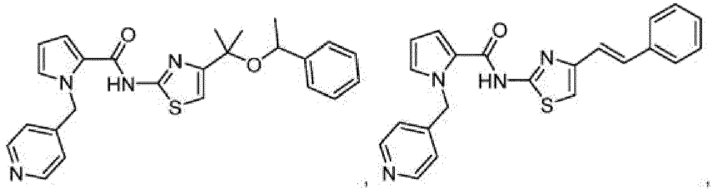 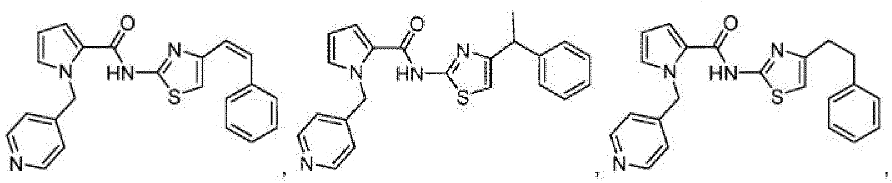 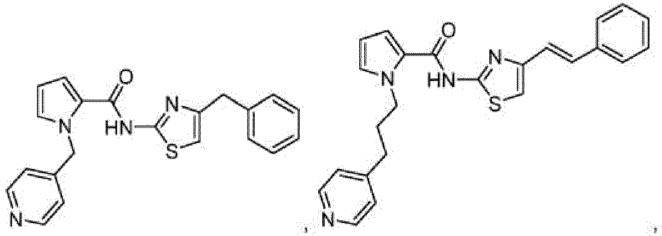 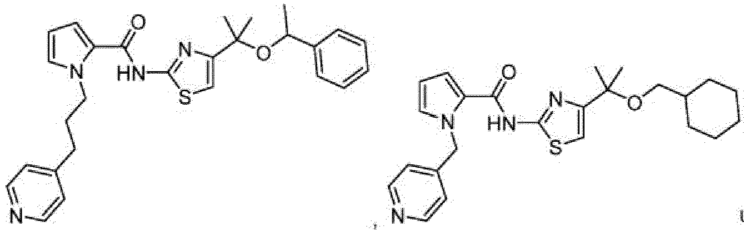 und 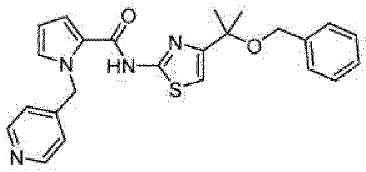 oder die Verbindung aus der aus Folgendem bestehenden Gruppe ausgewählt ist:

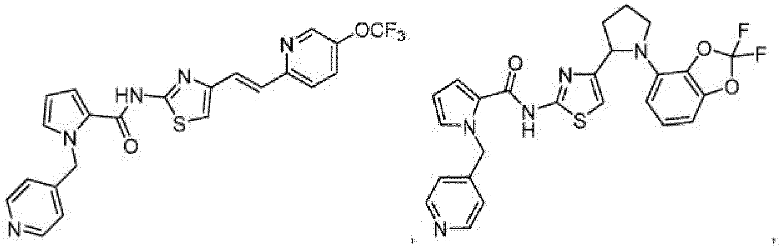

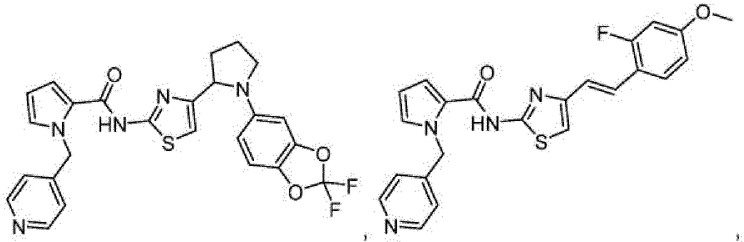

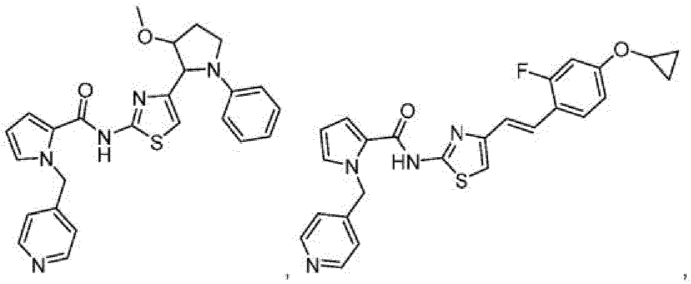

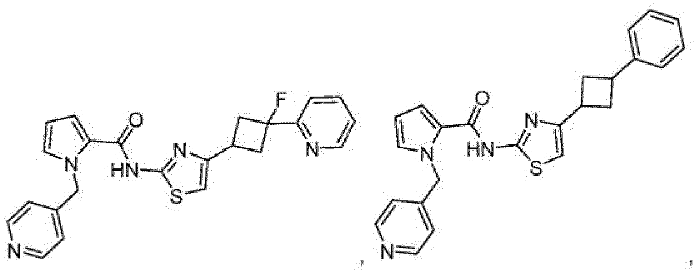

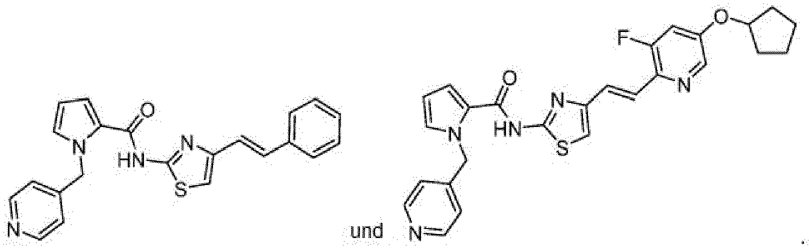

13. Verbindung oder Salz nach einem der Ansprüche 1 bis 12 zur Verwendung bei der Behandlung einer Entzündung, einer Autoimmunerkrankung, einer immunologischen Erkrankung, einer neurodegenerativen Erkrankung oder einer Entzündungserkrankung.

14. Verbindung oder Salz nach einem der Ansprüche 1 bis 12 zur Verwendung bei der Behandlung von Krebs.

15. Verbindung oder Salz zur Verwendung nach Anspruch 14, wobei es sich bei dem Krebs um ein Melanom, ein multiples Myelom, Prostatakrebs, Lungenkrebs, Bauchspeicheldrüsenkrebs, ein Plattenepithelkarzinom, Leukämie, ein Lymphom, einen neuroendokrinen Tumor, Blasenkrebs oder Dickdarmkrebs handelt oder es sich bei dem Krebs um ein nichtkleinzelliges Lungenkarzinom, ein Plattenepithelkarzinom, Leukämie, akute myeloische Leukämie, chronische myeloische Leukämie, ein Lymphom, ein NPM/ALK-transformiertes anaplastisches großzelliges Lymphom, ein diffuses großzelliges B-Zell-Lymphom, neuroendokrine Tumoren, Brustkrebs, ein Mantelzelllymphom, ein Nierenzellkarzinom, ein Rhabdomyosarkom, Eierstockkrebs, Endometriumkarzinom, ein kleinzelliges Karzinom, ein Adenokarzinom, ein Magenkarzinom, ein hepatozelluläres Karzinom, ein Pankreaskarzinom, ein Schilddrüsenkarzinom, ein anaplastisches großzelliges Lymphom, ein Hämangiom oder Kopf- und Halskrebs handelt.

16. Verbindung oder Salz zur Verwendung nach Anspruch 14, wobei es sich bei dem Krebs um einen soliden Tumor handelt.

## Revendications

1. Composé ayant une structure de formule (III) : 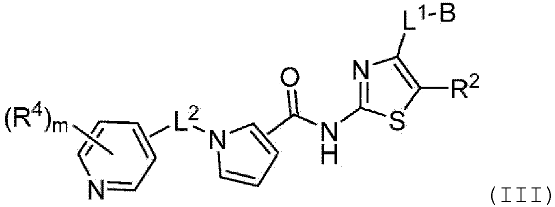 dans lequel :
L¹ est une liaison, alkylène en C₁₋₆, ou 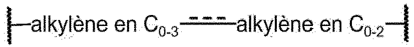
, dans lequel --- indique une double liaison, une triple liaison ou un cyclopropyle condensé ;
B est (alkylène en C₀₋₃) -X;
X est un carbocycle en C₄₋₁₀ aromatique ou non aromatique, ou un hétérocycle de 4 à 10 chaînons aromatique ou non aromatique ayant 1 à 3 hétéroatomes cycliques choisis parmi N, O et S ;
X est substitué par 1 à 3 G ;
chaque G est indépendamment sélectionné dans le groupe constitué d'halogéno, OH, =O, CN, NO₂, N(R^{N})₂, N(R^{N})C(O) (alkyle en C₁₋₃), alkyle en C_{1-3,} alcoxy en C_{1-3,} halogénoalkyle en C₁₋₃, halogénoalcoxy en C₁₋₃, C(O)(alkyle en C₁₋₃), S(O₂)-Z, C(O)-Z, C(O)N(R^{N})₂, éther silylique et [O]₀₋₁-(alkylène en C₀₋₃) -Z ;
chaque R^{N} est indépendamment H ou alkyle en C₁₋₄ ;
Z est un carbocycle en C₃₋₁₀ aromatique ou non aromatique, ou un hétérocycle à 4 à 10 chaînons aromatique ou non aromatique ayant 1 à 3 hétéroatomes choisis dans le groupe constitué de N, O et S ;
Z est facultativement substitué par 1 à 3 E ; et,
chaque E est indépendamment choisi parmi alkyle en C_{1-3,} alcoxy en C₁₋₃, =O, halogénoalcoxy en C₁₋₃, CN et halogéno
R² est H ;
L² est alkylène en C₀₋₃ ;
m est 0 à 2 ; et
chaque R⁴ est indépendamment alkyle en C₁₋₃, alcynyle en C₂₋₃, halogénoalkyle en C_{1-3,} alcoxy en C₁₋₃, halogéno ou NH(alkylène en C₁₋₃)-aryle ;
ou un sel pharmaceutiquement acceptable de celui-ci,
le composé ou un sel de celui-ci n'étant pas le N-[4-[(3-méthyl-1-pipéridinyl)méthyl]2-thiazolyl)-1-(4-pyridinylméthyl)-1H-pyrrole-2-carboxamide.

2. Composé ou sel selon la revendication 1, dans lequel L¹ est une liaison ou est 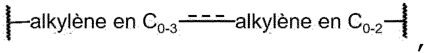 etindique une double liaison.

3. Composé ou sel selon l'une quelconque des revendications 1 à 2, dans lequel X est pyrrolidinyle, cyclobutyle, cyclopentyle, cyclohexyle, cyclohexényle, phényle, pipéridinyle, pyridinyle, pipérazinyle, tétrahydrofuranyle, furanyle, tétrahydropyranyle, quinoléinyle, morpholinyle, pyrrolidonyle, pyrimidinyle, pyridazinyle, indényle, dihydroindényle, dihydrobenzofuranyle, chromanyle, isochromanyle, dihydroisoquinoléinyle, orindolyle.

4. Procédé selon la revendication 1, dans lequel L¹-B est choisi dans le groupe constitué de :

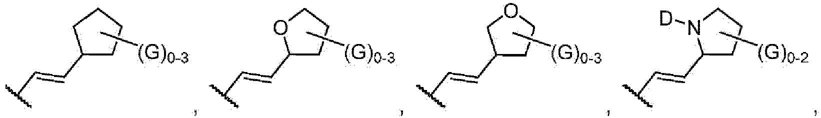

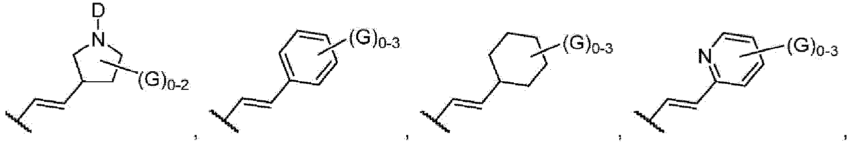

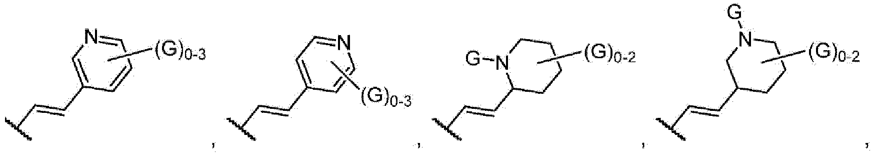

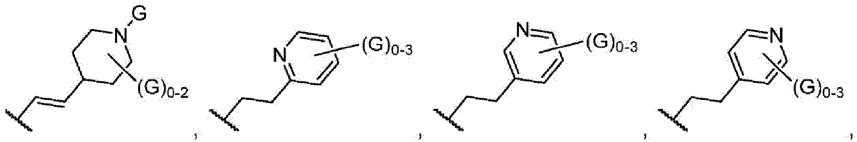

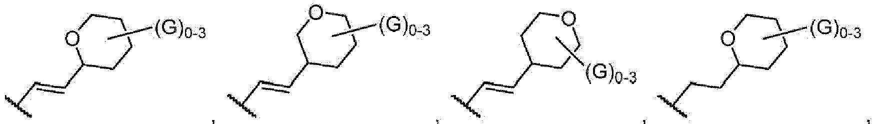

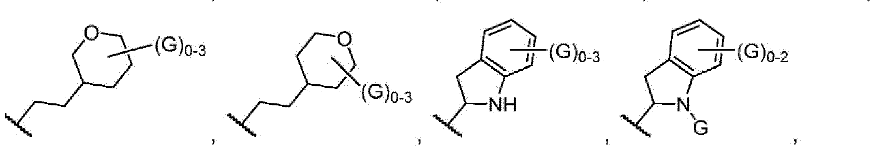

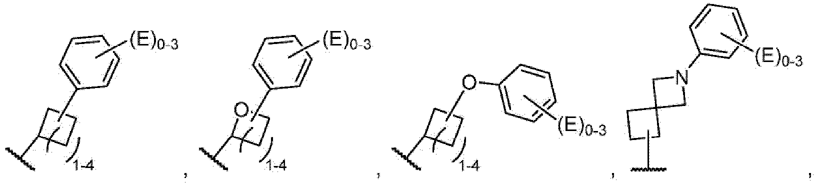

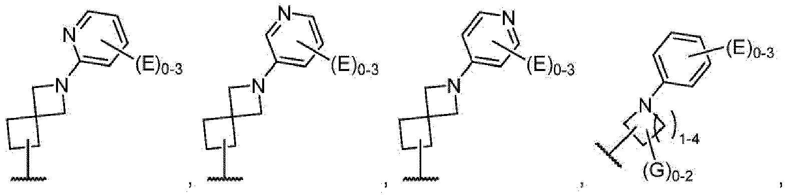

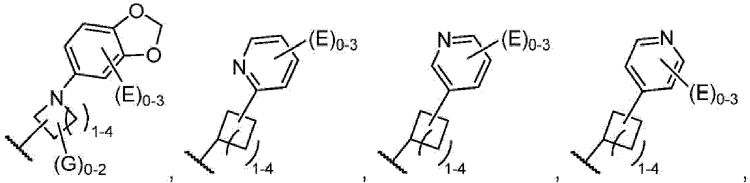

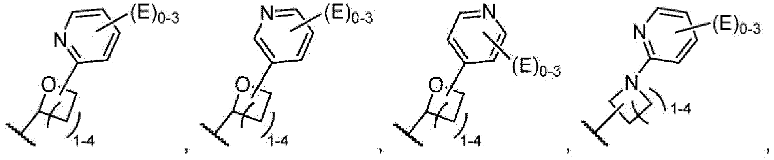

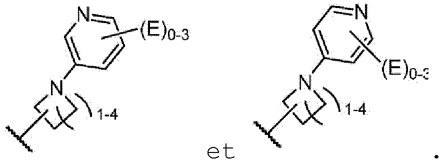

5. Procédé selon la revendication 1, dans lequel L¹-B est choisi dans le groupe constitué de :

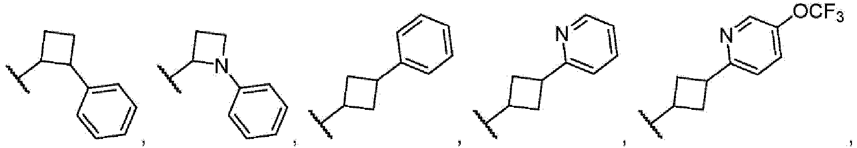

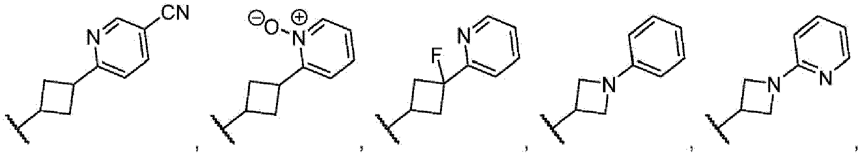

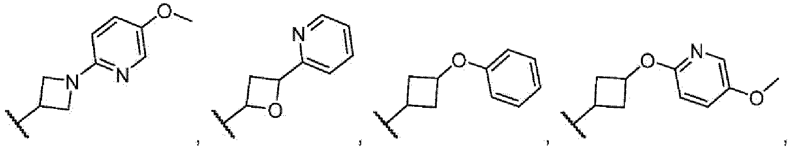

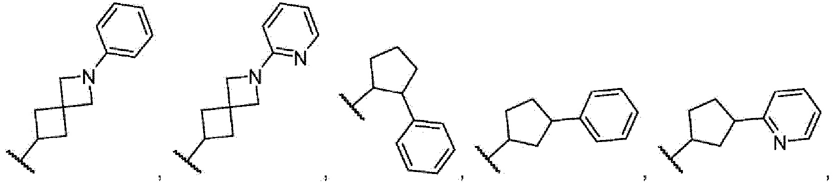

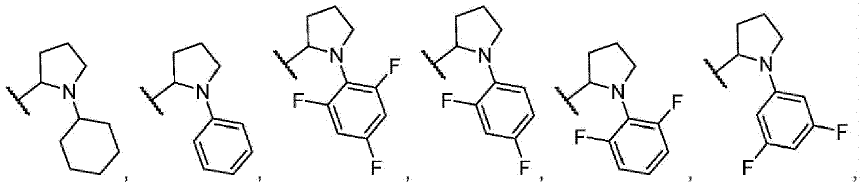

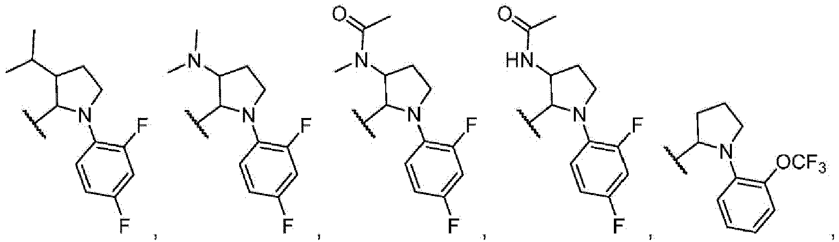

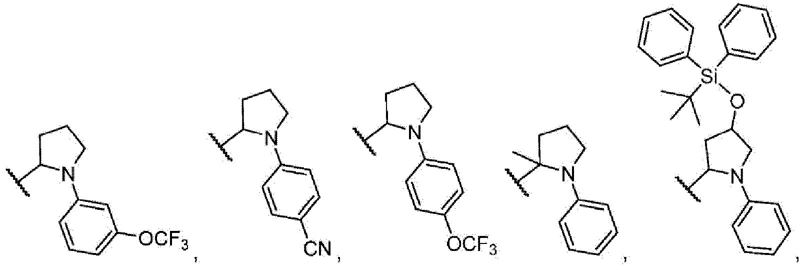

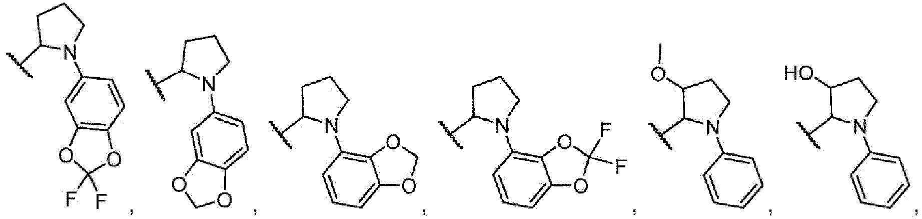

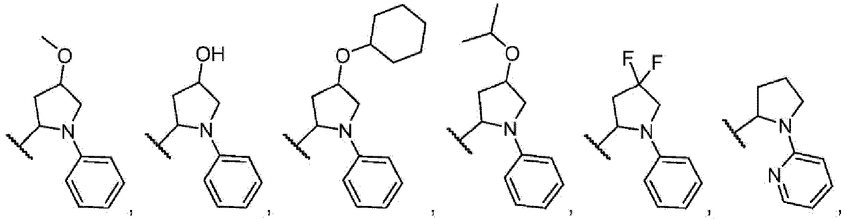

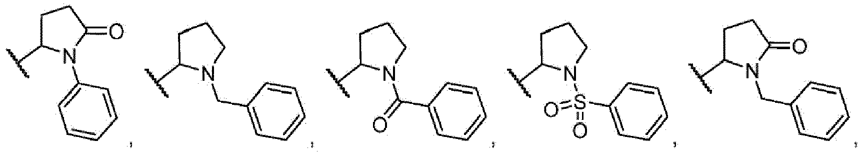

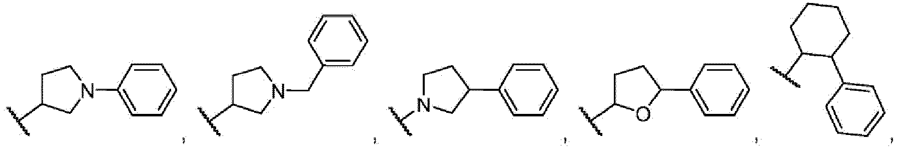

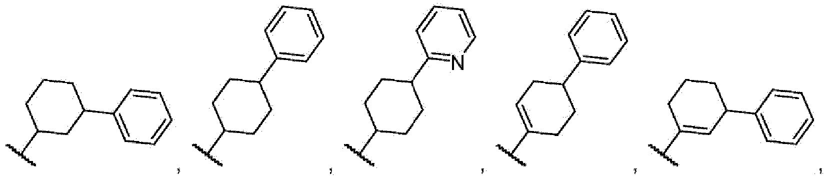

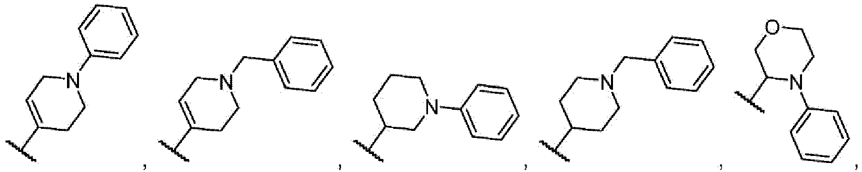

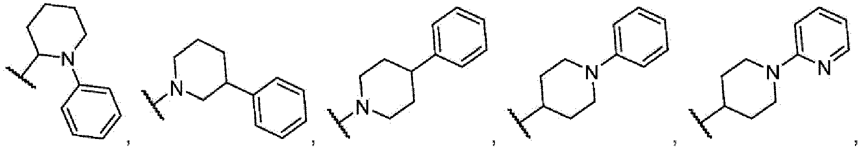

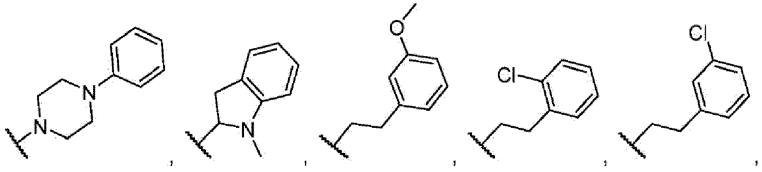

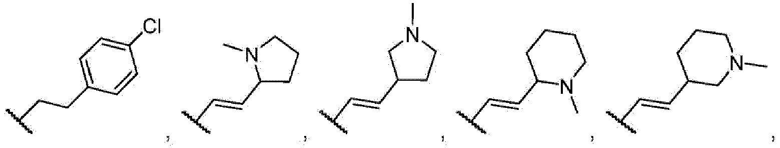

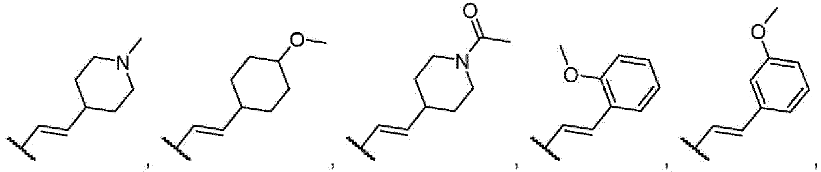

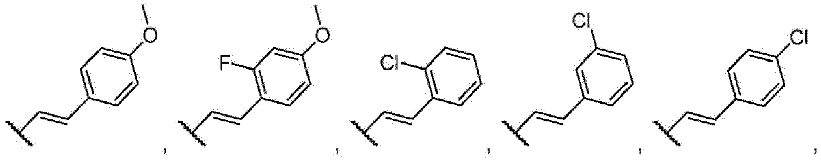

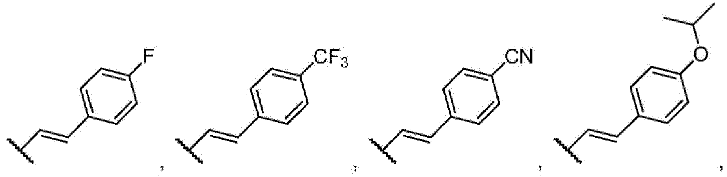

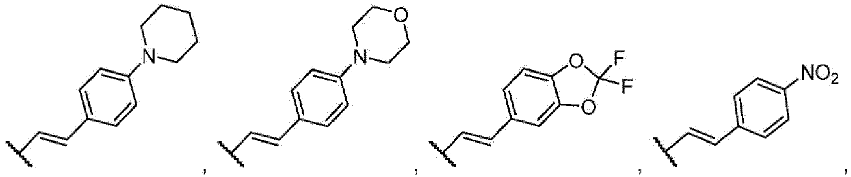

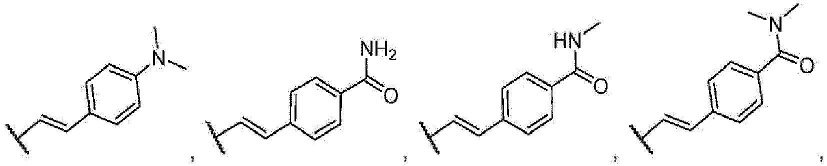

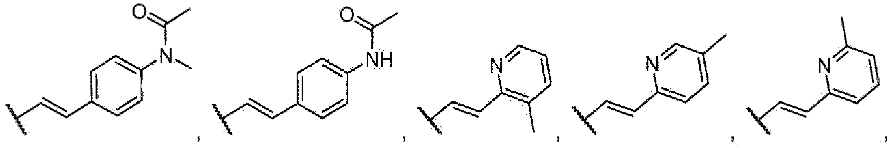

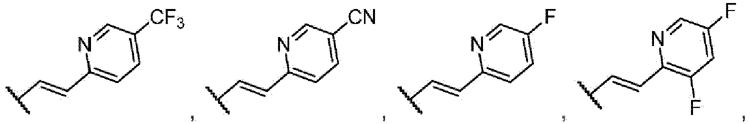

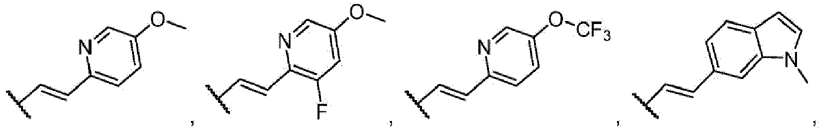

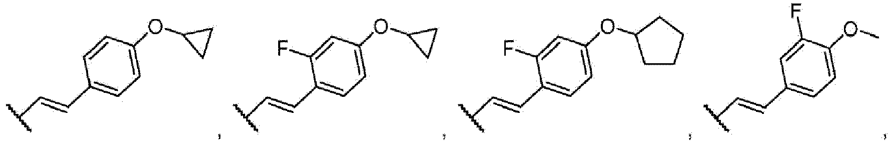

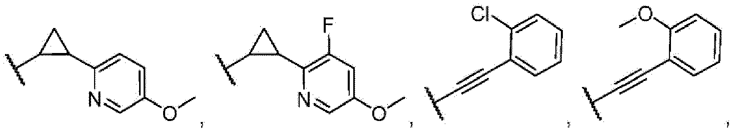

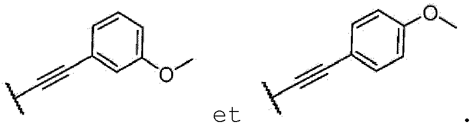

6. Composé ou sel selon l'une quelconque des revendications 1 à 5, dans lequel L² est alkylène en C₁.

7. Composé ou sel selon l'une quelconque des revendications 1 à 6, dans lequel m est 1 ou 2.

8. Composé ou sel selon la revendication 1, ayant la structure de formule (IIIA) : 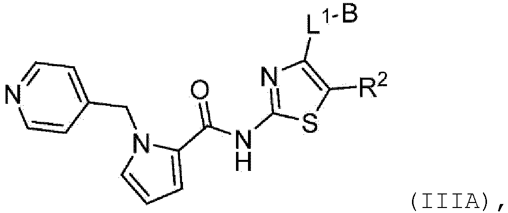 et dans lequel L¹-B est choisi dans le groupe constitué de :

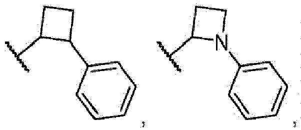

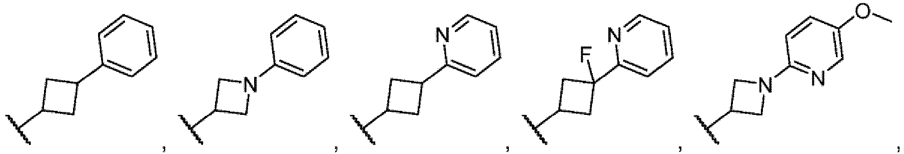

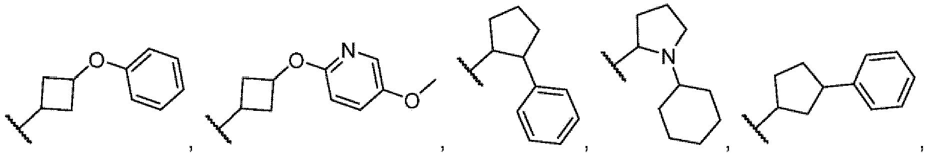

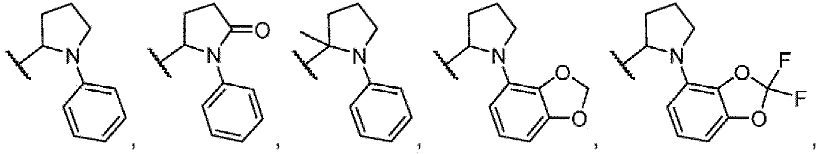

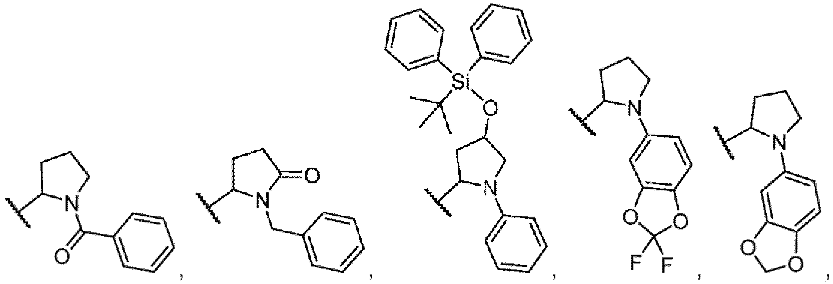

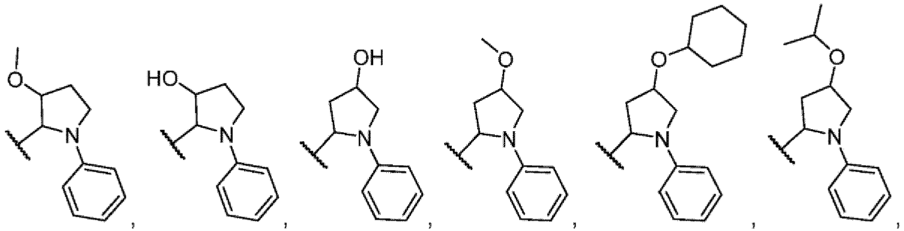

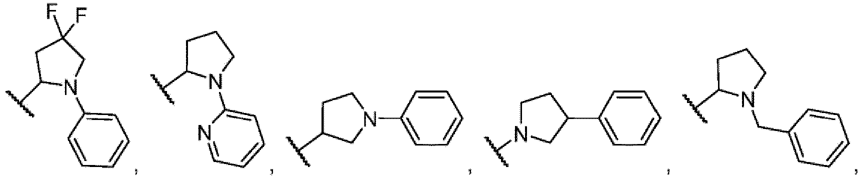

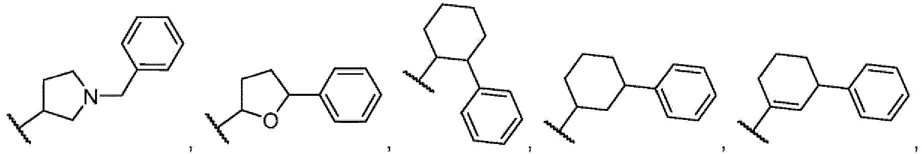

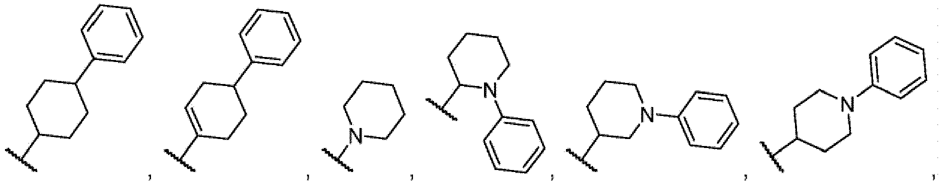

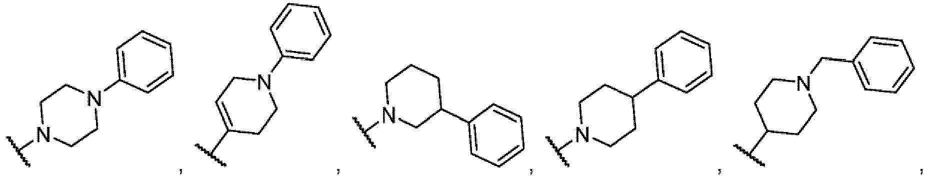

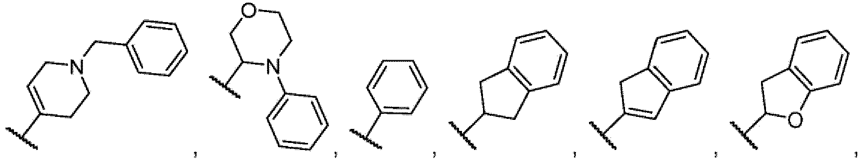

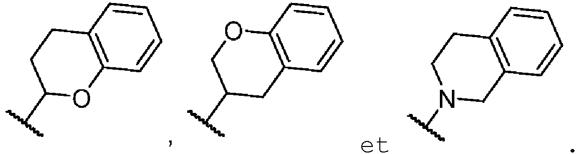

9. Composé ou sel selon la revendication 1, ayant la structure de formule (IIIA) : 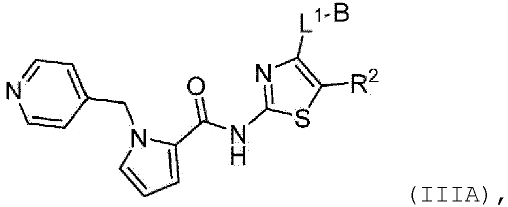 et dans lequel L¹-B est choisi dans le groupe constitué de :

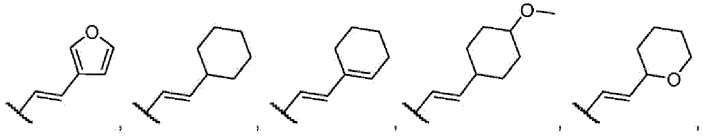

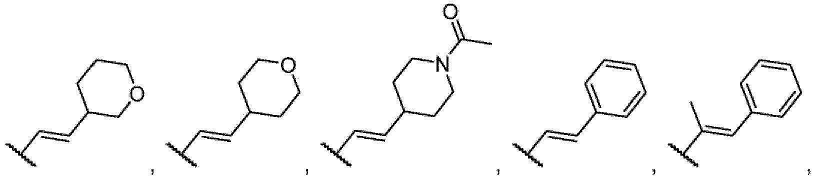

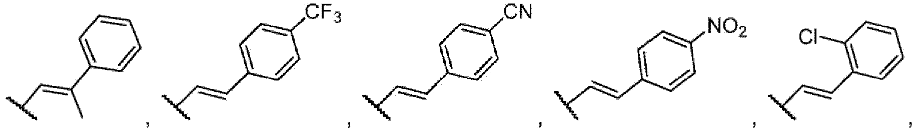

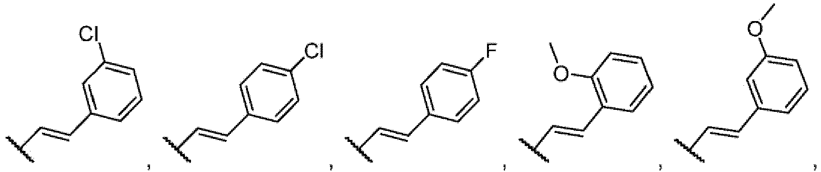

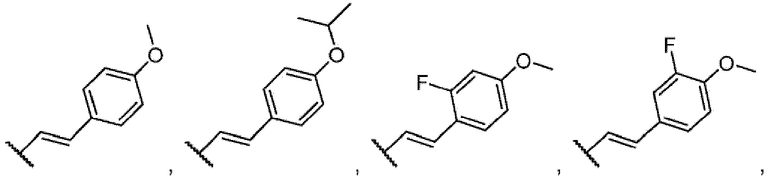

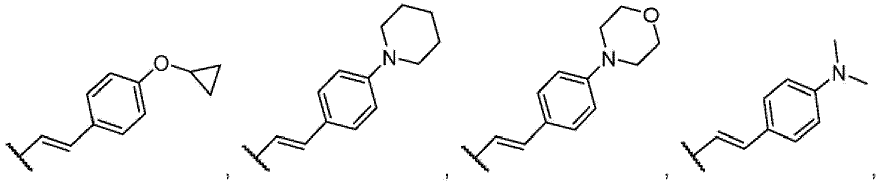

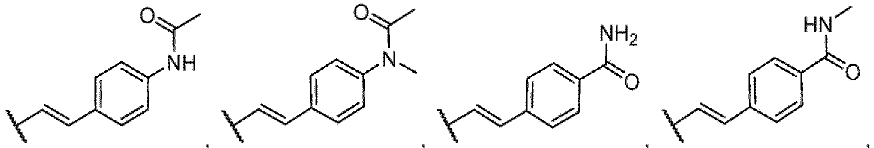

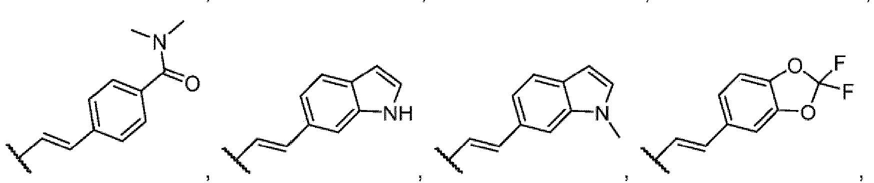

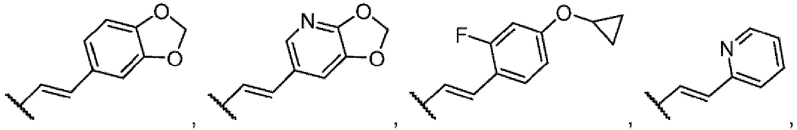

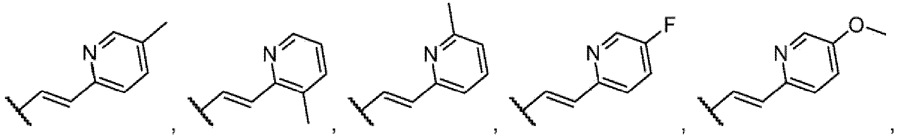

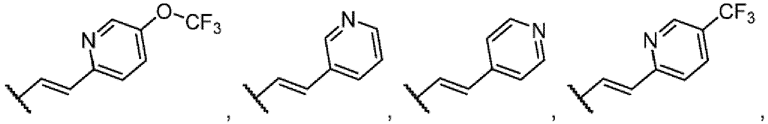

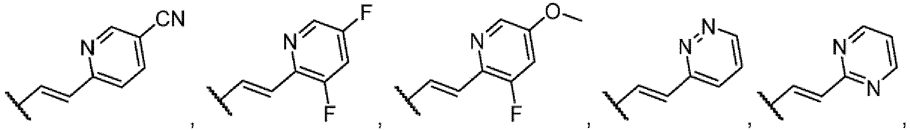

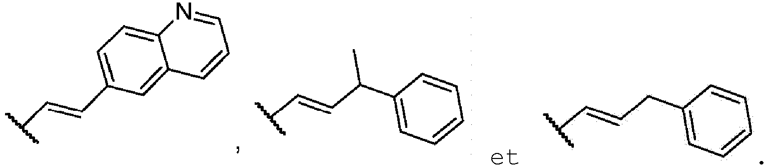

10. Composé ou sel selon la revendication 1, le composé ou le sel étant choisi dans le groupe constitué de :

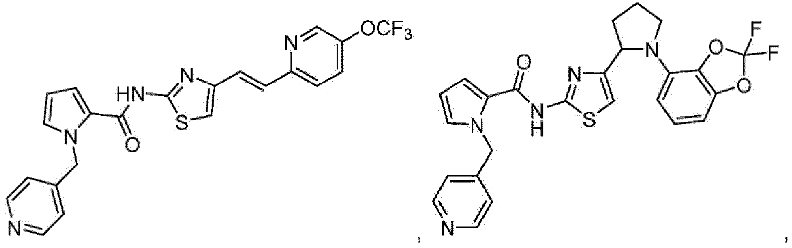

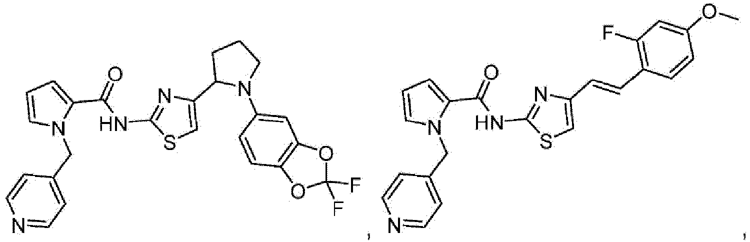

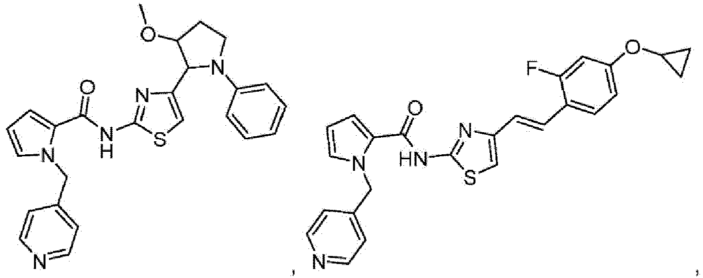

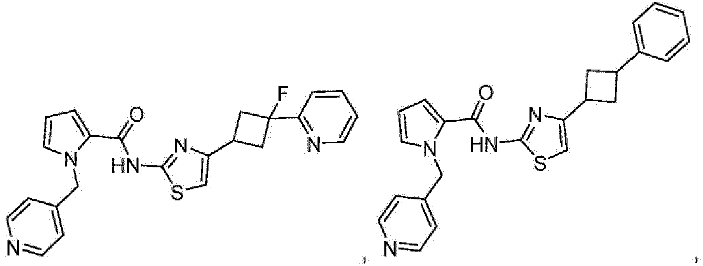

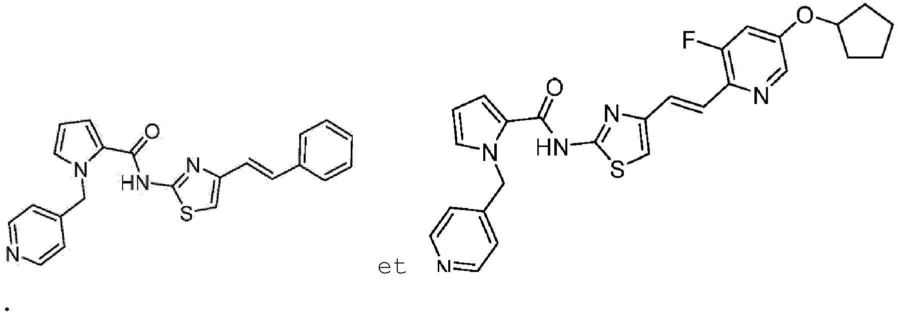

11. Composé répertorié dans le tableau A, ou un sel pharmaceutiquement acceptable de celui-ci.
**Tableau A**
| | |
|---|---|
| **A1** | 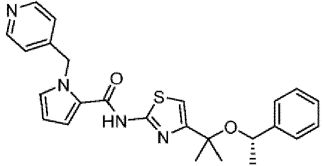 |
| **A2** | 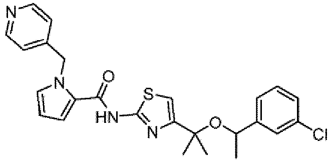 |
| **A3** | 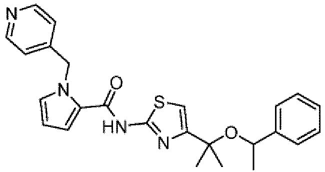 |
| **A4** | 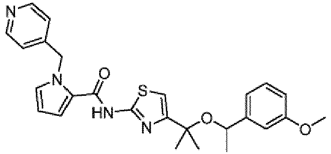 |
| **A5** | 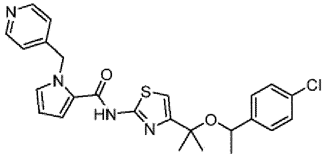 |
| **A6** | 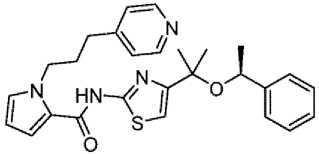 |
| **A7** | 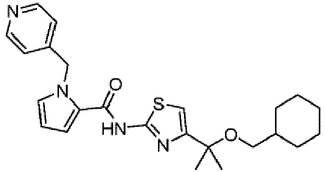 |
| **A8** | 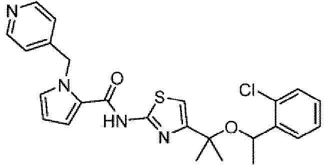 |
| **A9** | 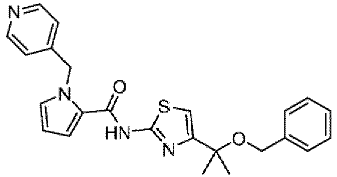 |
| **A10** | 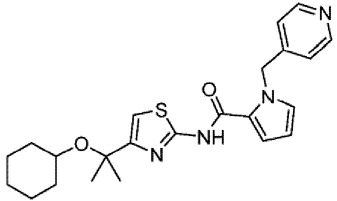 |
| **A11** | 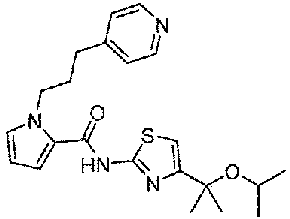 |
| **A12** | 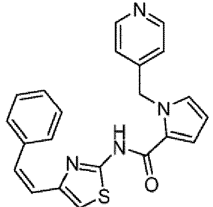 |
| **A13** | 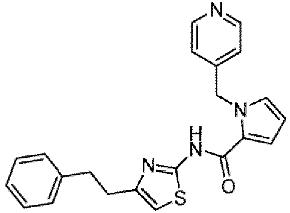 |
| **A14** | 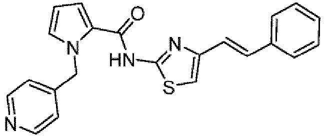 |
| **A15** | 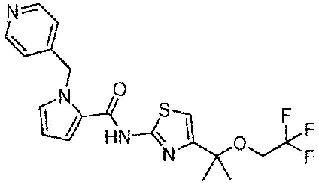 |
| **A16** | 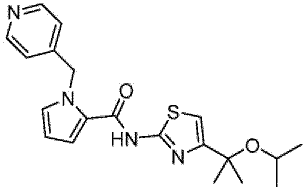 |
| **A17** | 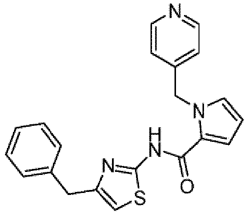 |
| **A18** | 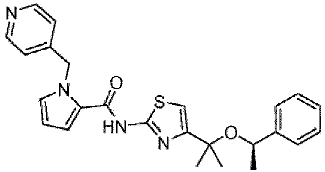 |
| **A19** | 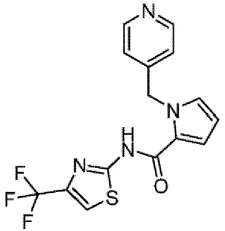 |
| **A20** | 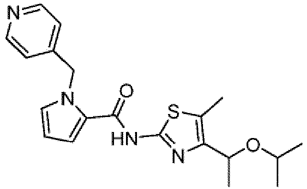 |
| **A21** | 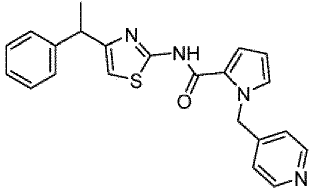 |
| **A22** | 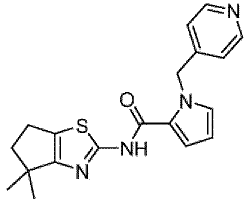 |
| **A23** | 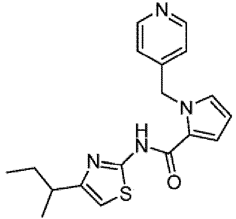 |
| **A24** | 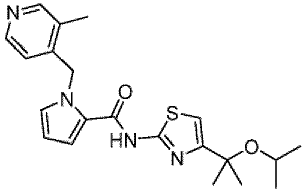 |
| **A25** | 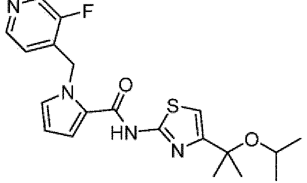 |
| **A26** | 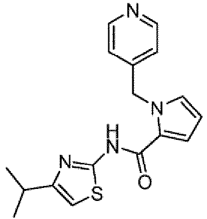 |
| **A27** | 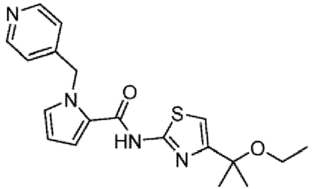 |
| **A28** | 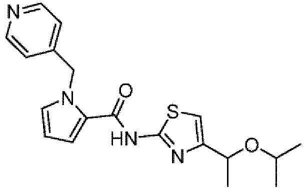 |
| **A29** | 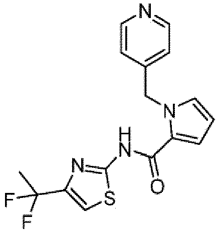 |
| **A30** | 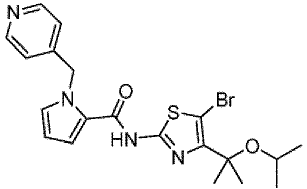 |
| **A31** | 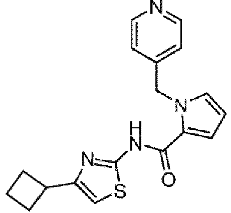 |
| **A32** | 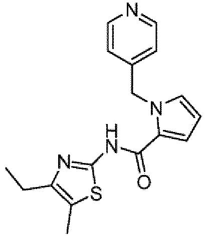 |
| **A33** | 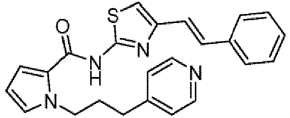 |
| **A34** | 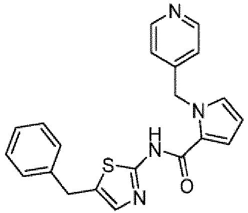 |
| **A35** | 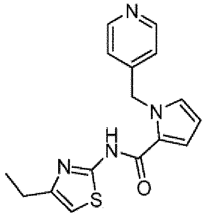 |
| **A36** | 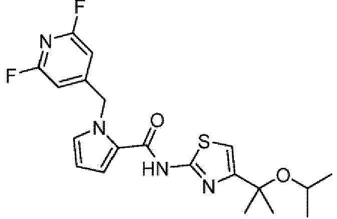 |
| **A37** | 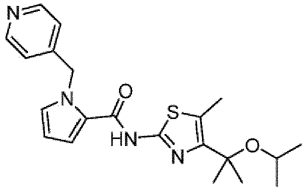 |
| **A38** | 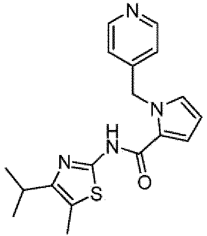 |
| **A39** | 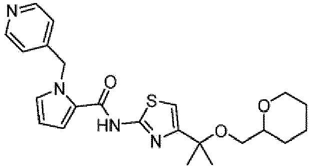 |
| **A40** | 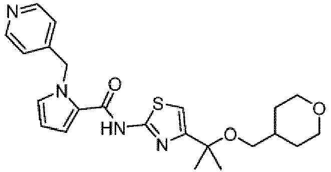 |
| **A41** | 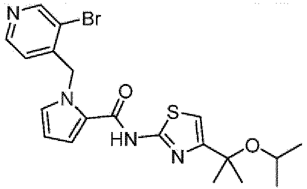 |
| **A42** | 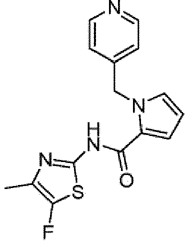 |
| **A43** | 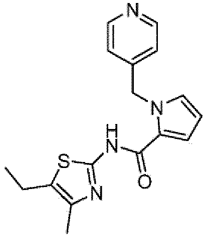 |
| **A44** | 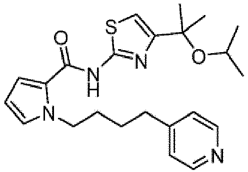 |
| **A45** | 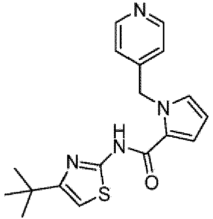 |
| **A46** | 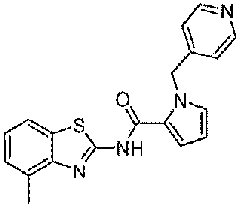 |
| **A47** | 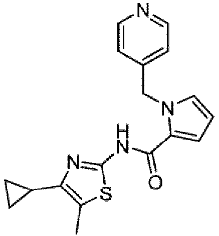 |
| **A48** | 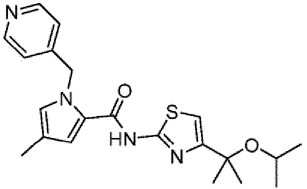 |
| **A49** | 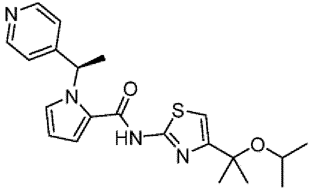 |
| **A50** | 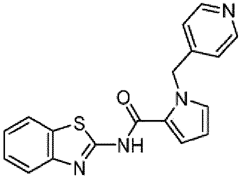 |
| **A51** | 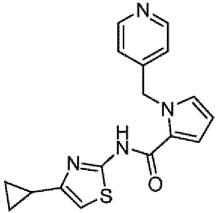 |
| **A52** | 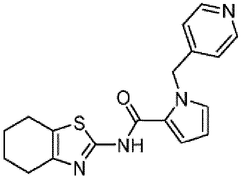 |
| **A53** | 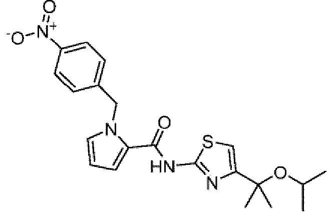 |
| **A54** | 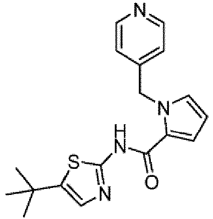 |
| **A55** | 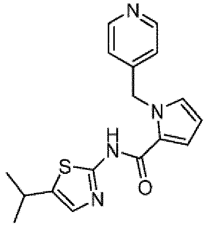 |
| **A56** | 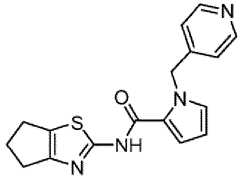 |
| **A57** | 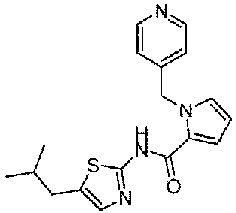 |
| **A58** | 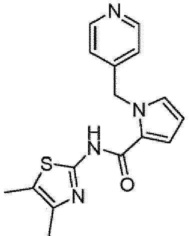 |
| **A59** | 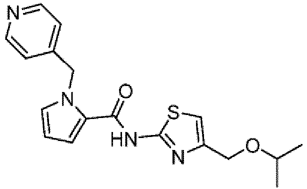 |
| **A60** | 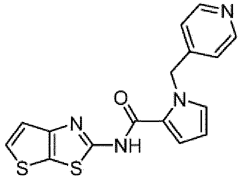 |
| **A61** | 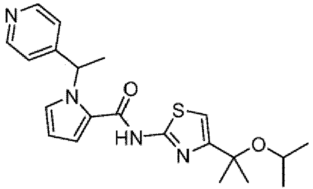 |
| **A62** | 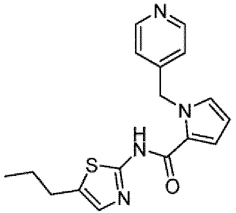 |
| **A63** | 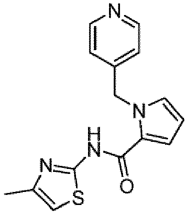 |
| **A64** | 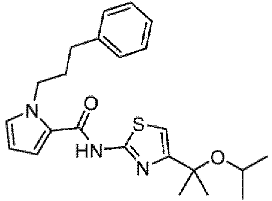 |
| **A65** | 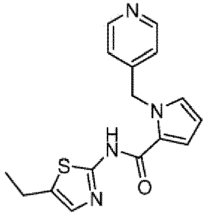 |
| **A66** | 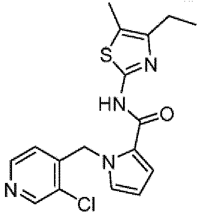 |
| **A67** | 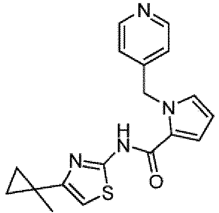 |
| **A68** | 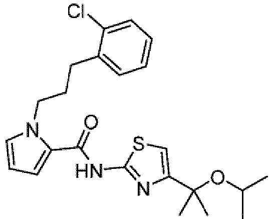 |
| **A69** | 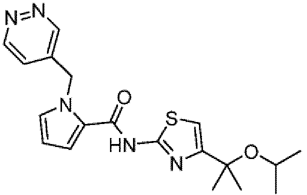 |
| **A70** | 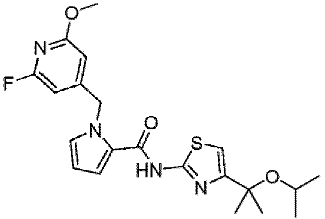 |
| **A71** | 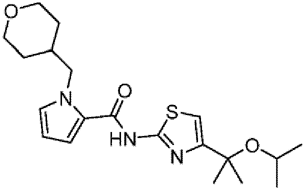 |
| **A72** | 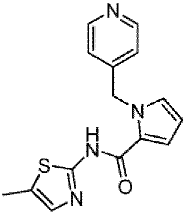 |
| **A73** | 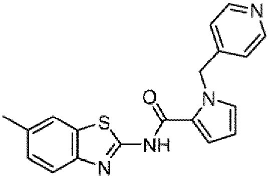 |
| **A74** | 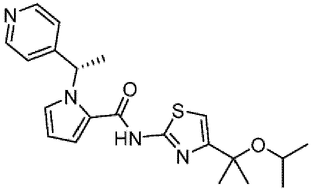 |
| **A75** | 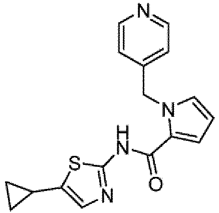 |
| **A76** | 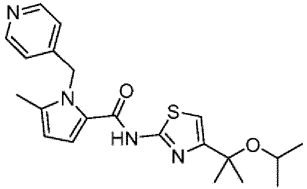 |
| **A77** | 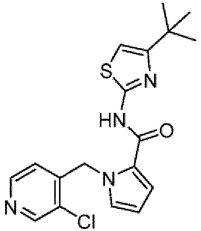 |
| **A78** | 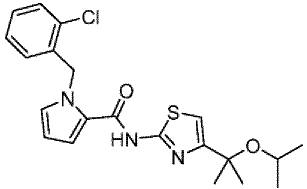 |
| **A79** | 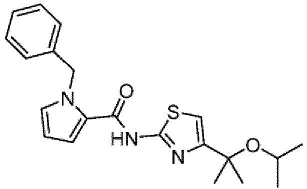 |
| **A80** | 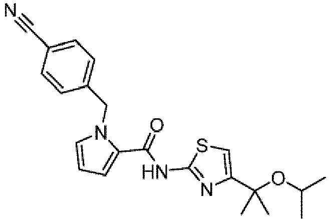 |
| **A81** | 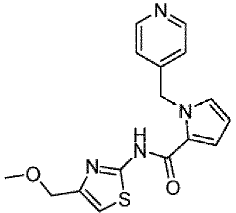 |
| **A82** | 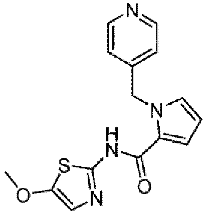 |
| **A83** | 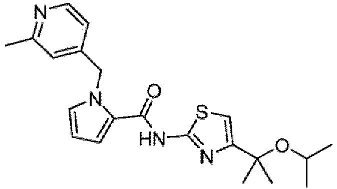 |
| **A84** | 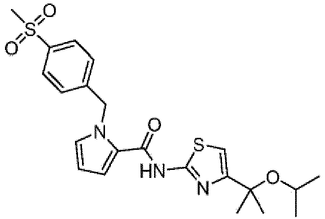 |
| **A85** | 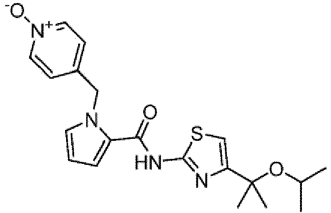 |
| **A86** | 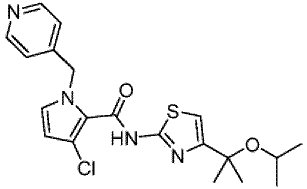 |
| **A87** | 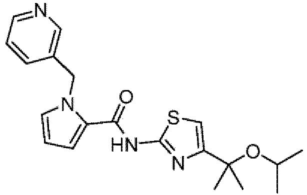 |
| **A88** | 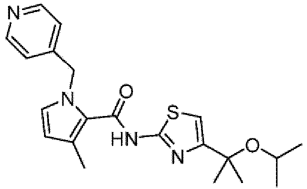 |
| **A89** | 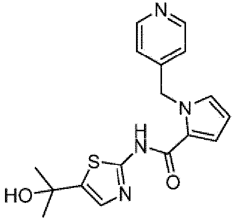 |
| **A90** | 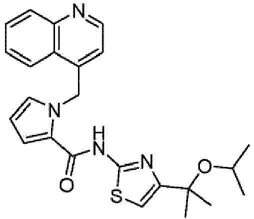 |
| **A91** | 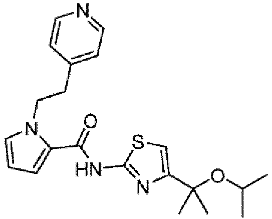 |
| **A92** | 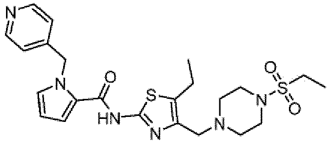 |
| **A93** | 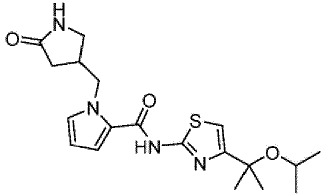 |
| **A94** | 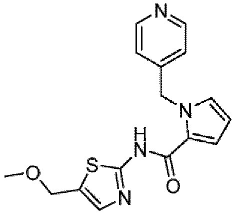 |
| **A95** | 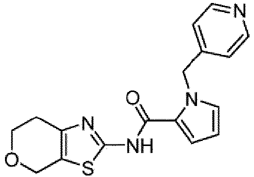 |
| **A96** | 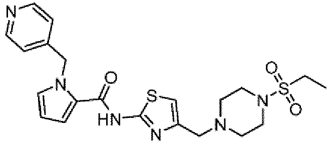 |
| **A97** | 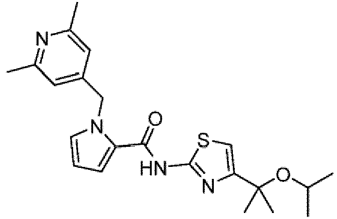 |
| **A98** | 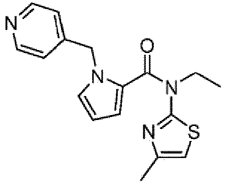 |
| **A99** | 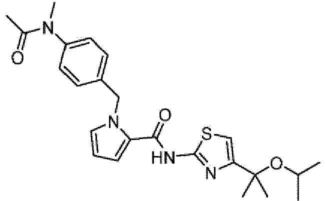 |
| **A100** | 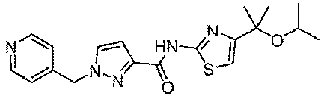 |
| **A101** | 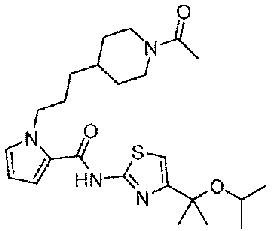 |
| **A102** | 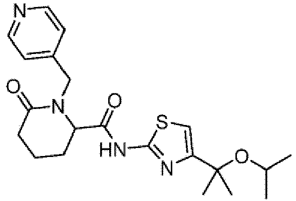 |
| **A103** | 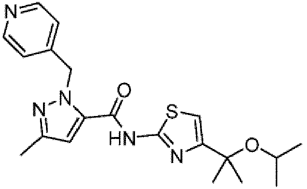 |
| **A104** | 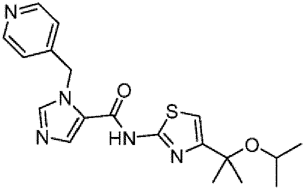 |
| **A105** | 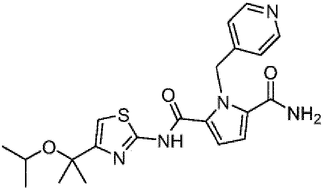 |
| **A106** | 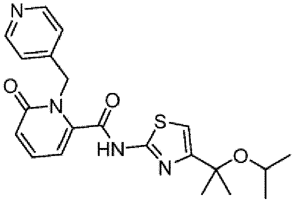 |
| **A107** | 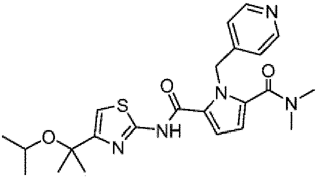 |
| **A108** | 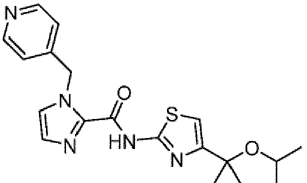 |
| **A109** | 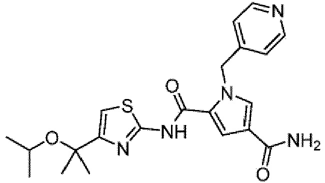 |
| **A110** | 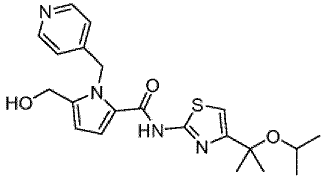 |
| **A111** | 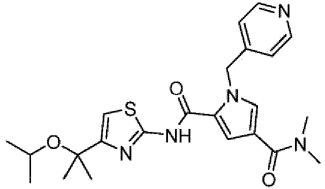 |
| **A112** | 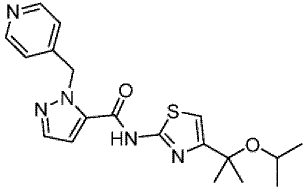 |
| **A113** | 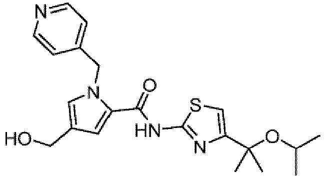 |
| **A114** | 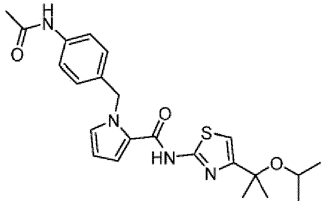 |
| **A115** | 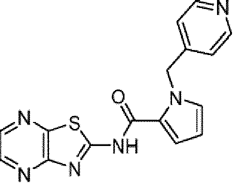 |
| **A116** | 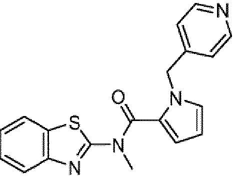 |
| **A117** | 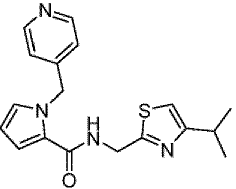 |
| **A118** | 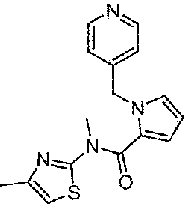 |
| **A119** | 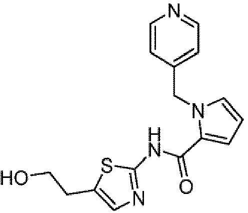 |
| **A120** | 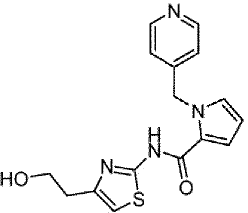 |
| **A121** | 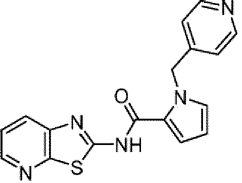 |
| **A122** | 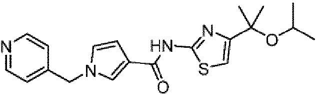 |
| **A123** | 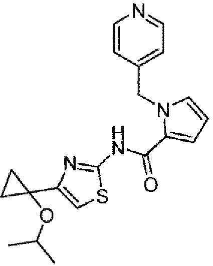 |
| **A124** | 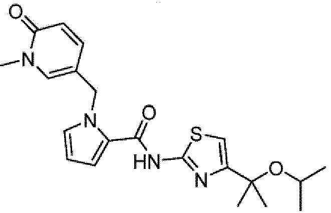 |
| **A125** | 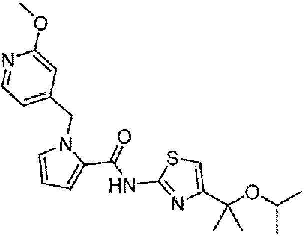 |
| **A126** | 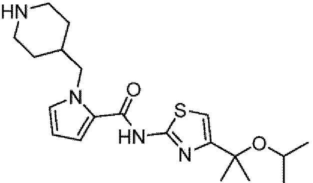 |
| **A127** | 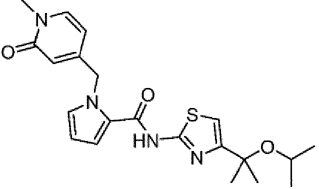 |
| **A128** | 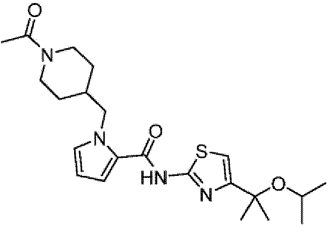 |
| **A129** | 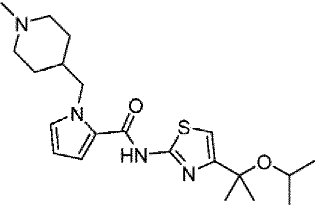 |
| **A130** | 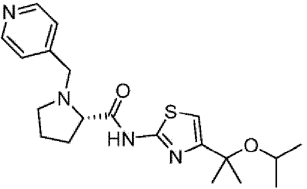 |
| **A131** | 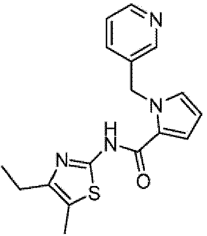 |
| **A132** | 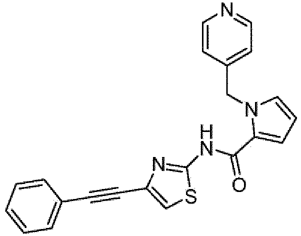 |
| **A133** | 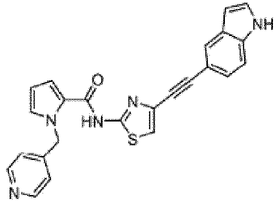 |
| **A134** | 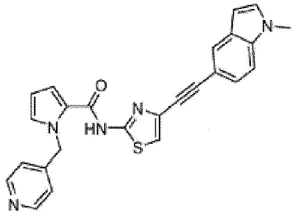 |
| **A135** | 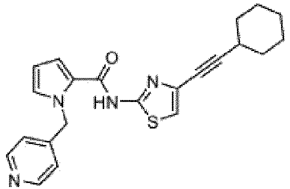 |
| **A136** | 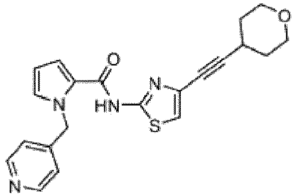 |
| **A137** | 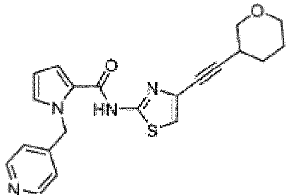 |
| **A138** | 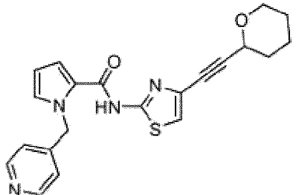 |
| **A139** | 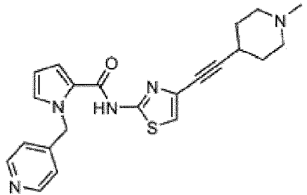 |
| **A140** | 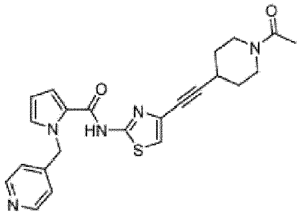 |
| **A141** | 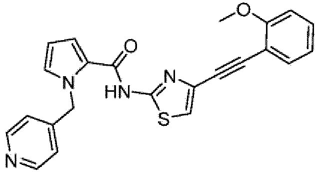 |
| **A142** | 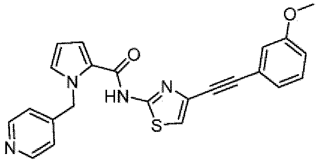 |
| **A143** | 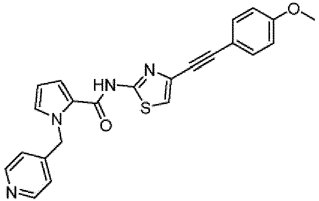 |
| **A144** | 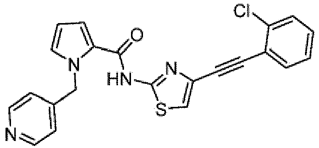 |
| **A145** | 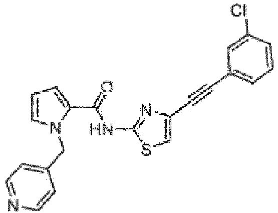 |
| **A146** | 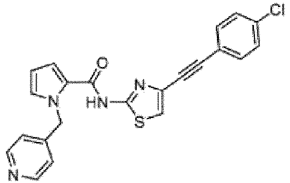 |
| **A147** | 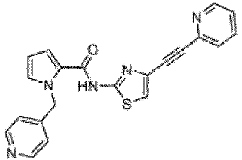 |
| **A148** | 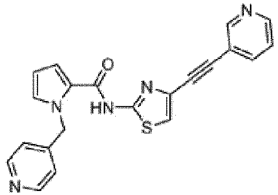 |
| **A149** | 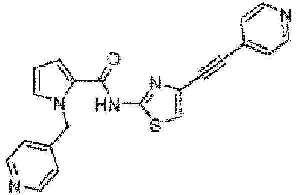 |
| **A150** | 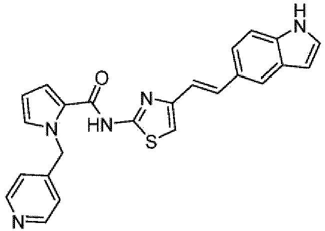 |
| **A151** | 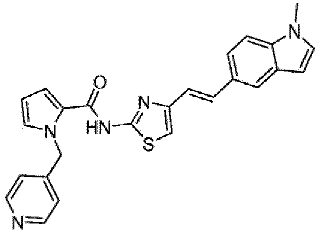 |
| **A152** | 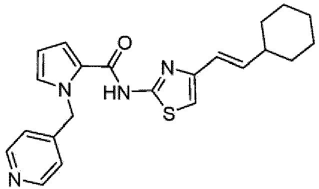 |
| **A153** | 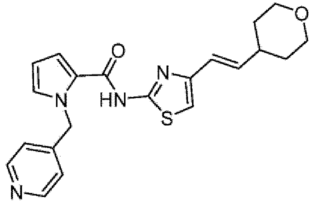 |
| **A154** | 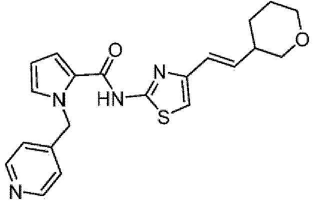 |
| **A155** | 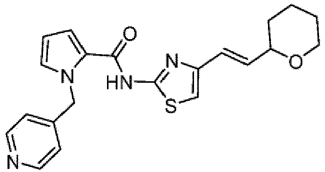 |
| **A156** | 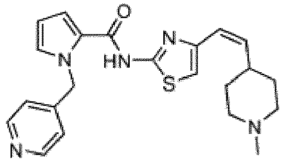 |
| **A157** | 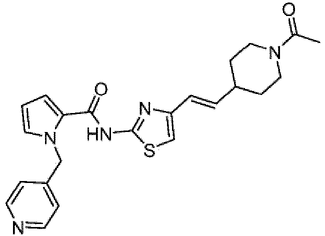 |
| **A158** | 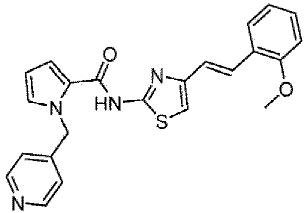 |
| **A159** | 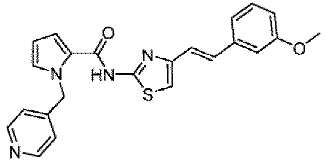 |
| **A160** | 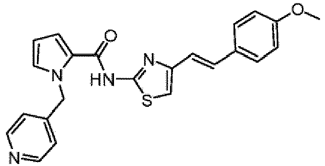 |
| **A161** | 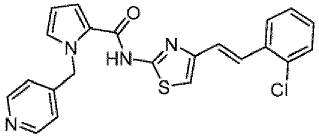 |
| **A162** | 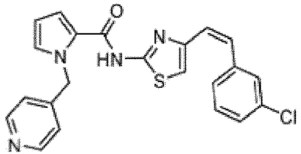 |
| **A163** | 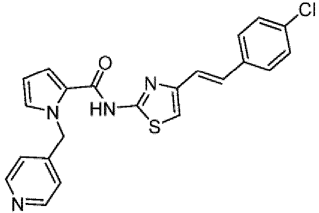 |
| **A164** | 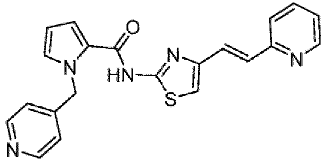 |
| **A165** | 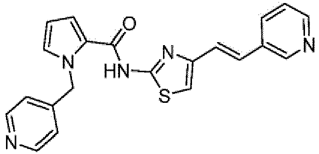 |
| **A166** | 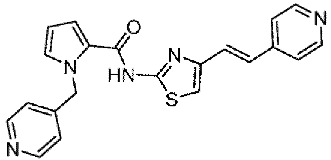 |
| **A167** | 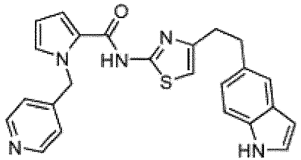 |
| **A168** | 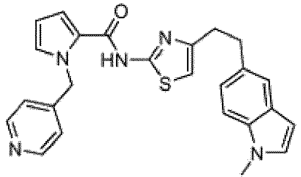 |
| **A169** | 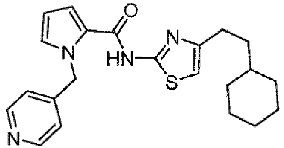 |
| **A170** | 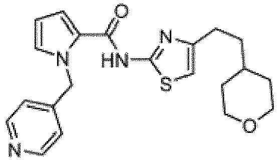 |
| **A171** | 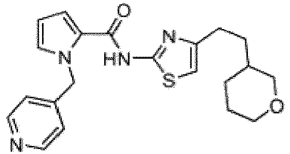 |
| **A172** | 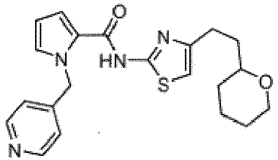 |
| **A173** | 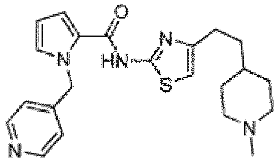 |
| **A174** | 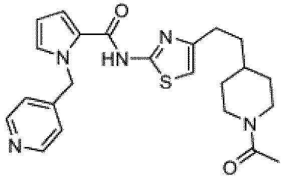 |
| **A175** | 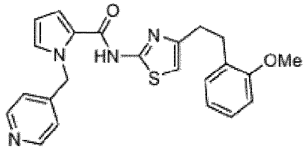 |
| **A176** | 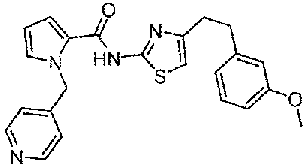 |
| **A177** | 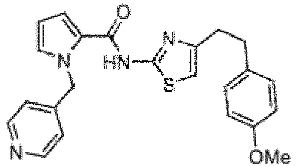 |
| **A178** | 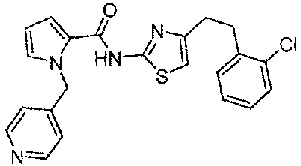 |
| **A179** | 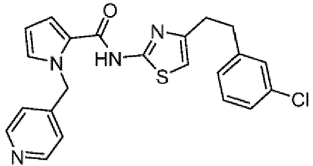 |
| **A180** | 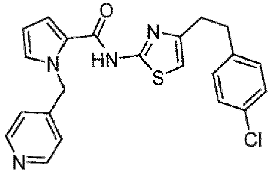 |
| **A181** | 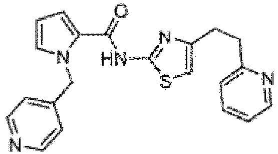 |
| **A182** | 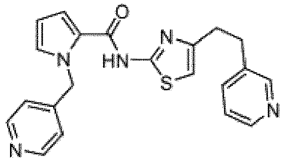 |
| **A183** | 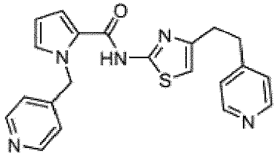 |
| **A184** | 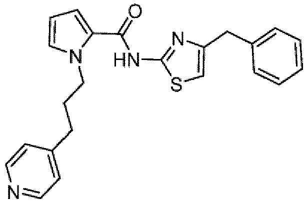 |
| **A185** | 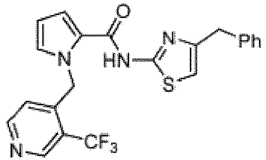 |
| **A186** | 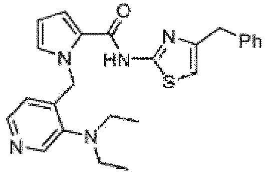 |
| **A187** | 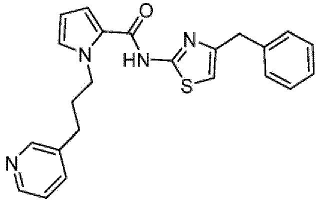 |
| **A188** | 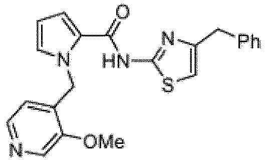 |
| **A189** | 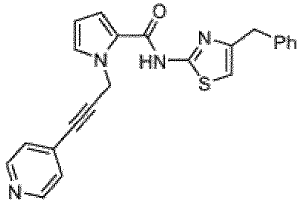 |
| **A190** | 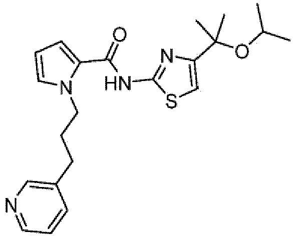 |
| **A191** | 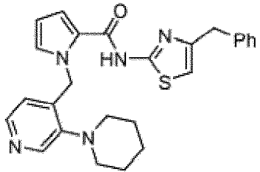 |
| **A192** | 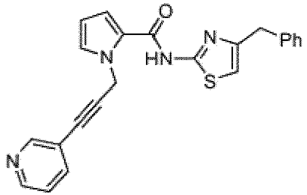 |
| **A193** | 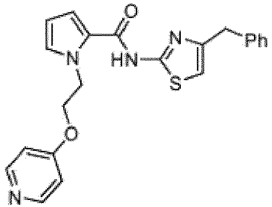 |
| **A194** | 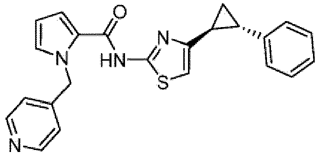 |
| **A195** | 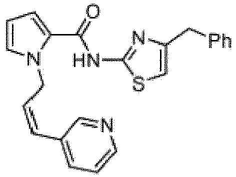 |
| **A196** | 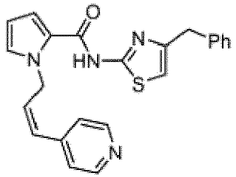 |
| **A197** | 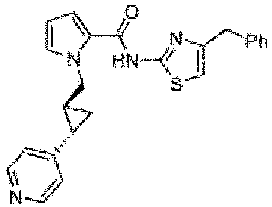 |
| **A198** | 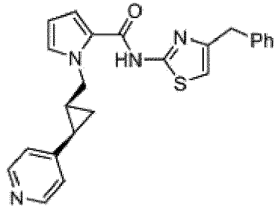 |
| **A199** | 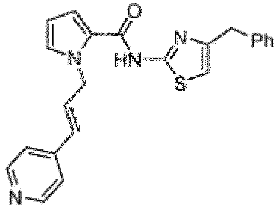 |
| **A200** | 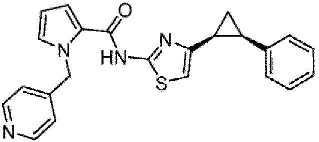 |
| **A201** | 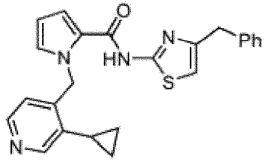 |
| **A202** | 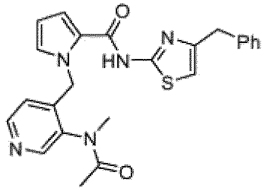 |
| **A203** | 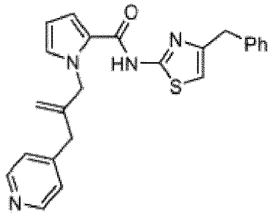 |
| **A204** | 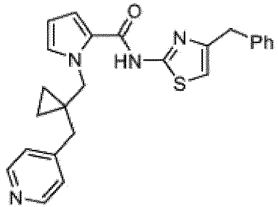 |
| **A205** | 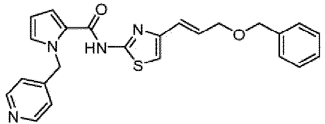 |
| **A206** | 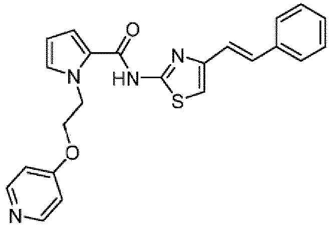 |
| **A207** | 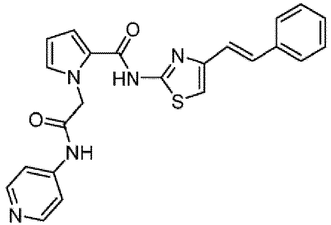 |
| **A208** | 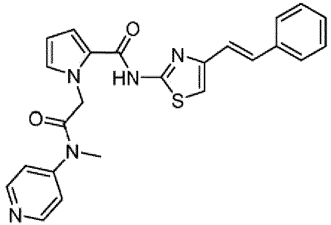 |
| **A209** | 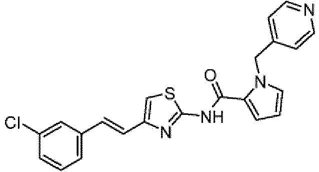 |
| **A210** | 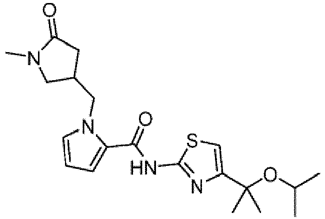 |
| **A211** | 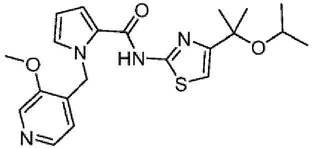 |
| **A212** | 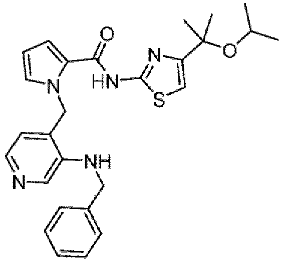 |
| **A213** | 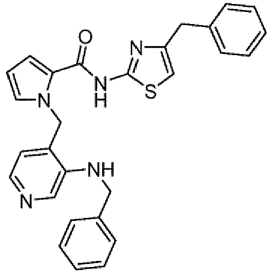 |
| **A214** | 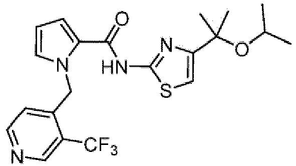 |
| **A215** | 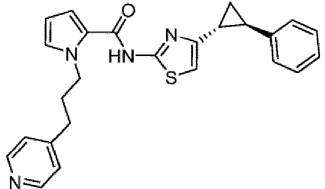 |
| **A216** | 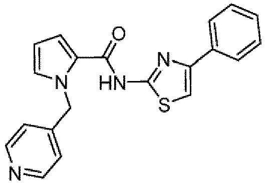 |
| **A217** | 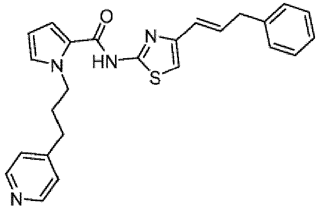 |
| **A218** | 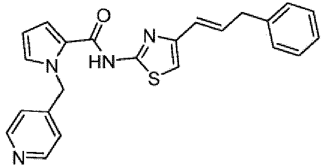 |
| **A219** | 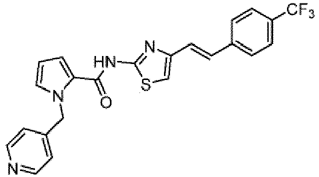 |
| **A220** | 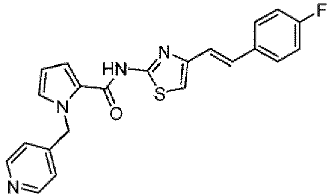 |
| **A221** | 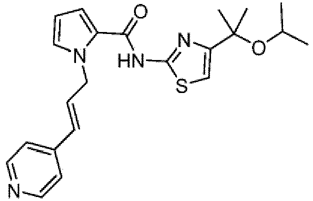 |
| **A222** | 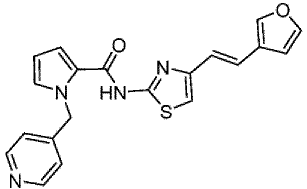 |
| **A223** | 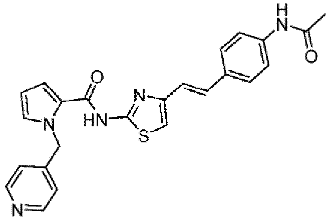 |
| **A224** | 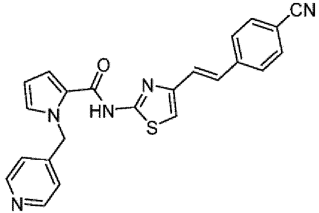 |
| **A225** | 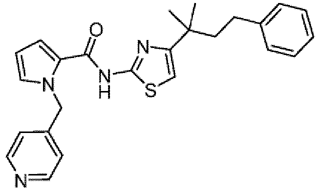 |
| **A226** | 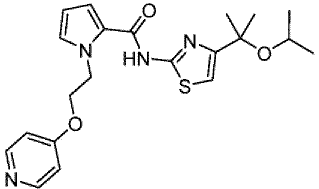 |
| **A227** | 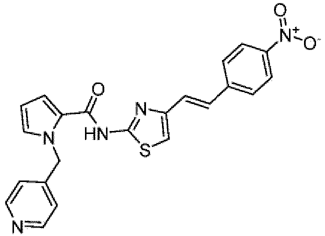 |
| **A228** | 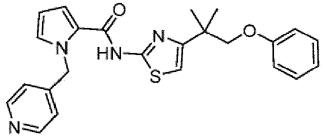 |
| **A229** | 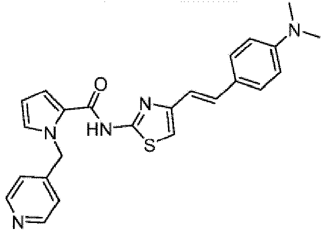 |
| **A230** | 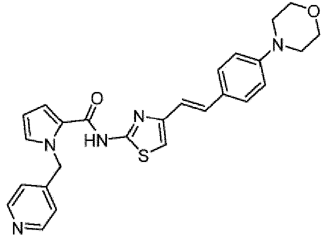 |
| **A231** | 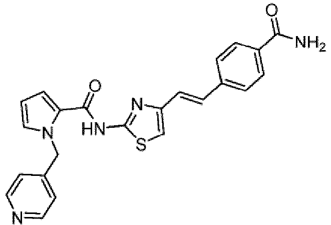 |
| **A232** | 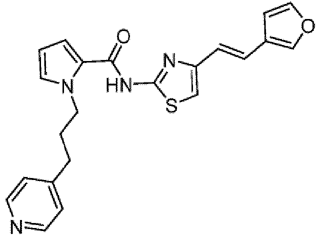 |
| **A233** | 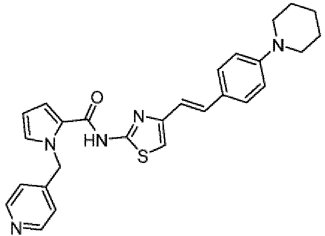 |
| **A234** | 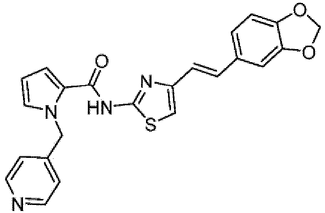 |
| **A235** | 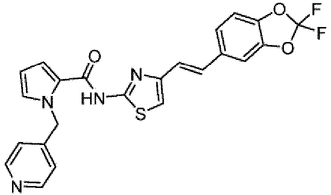 |
| **A236** | 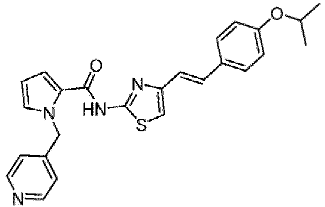 |
| **A237** | 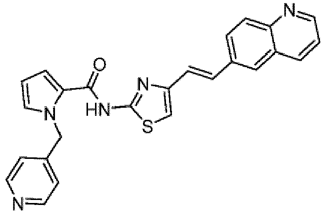 |
| **A238** | 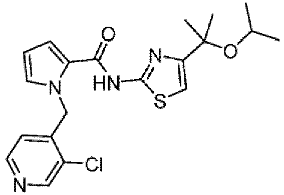 |
| **A239** | 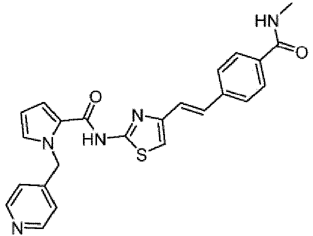 |
| **A240** | 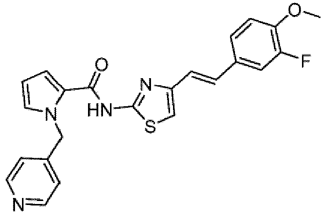 |
| **A241** | 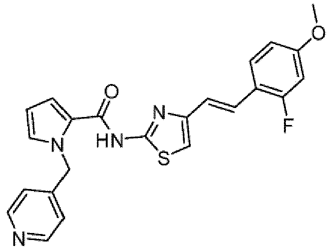 |
| **A242** | 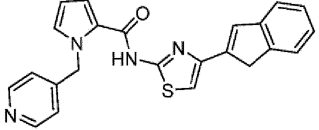 |
| **A243** | 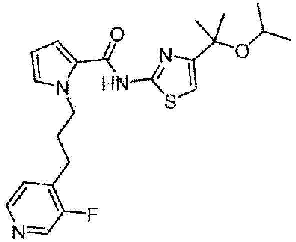 |
| **A244** | 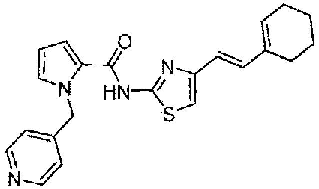 |
| **A245** | 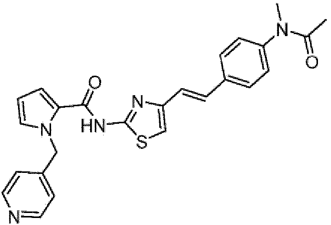 |
| **A246** | 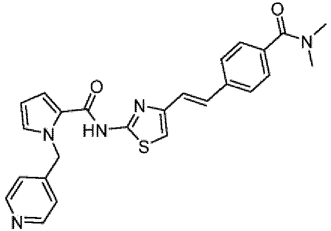 |
| **A247** | 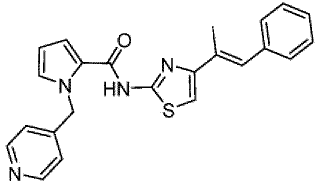 |
| **A248** | 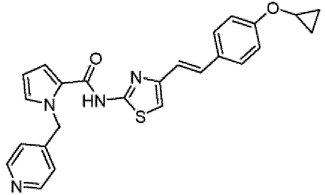 |
| **A249** | 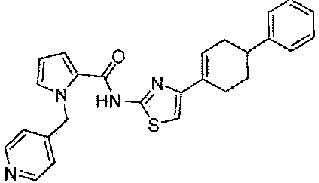 |
| **A250** | 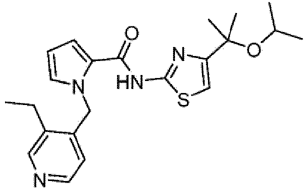 |
| **A251** | 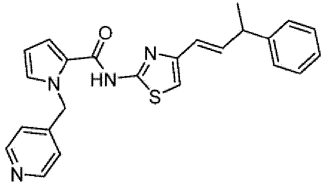 |
| **A252** | 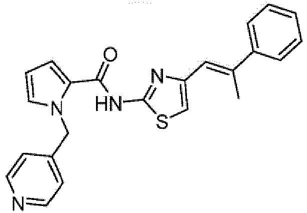 |
| **A253** | 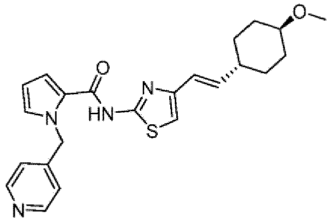 |
| **A254** | 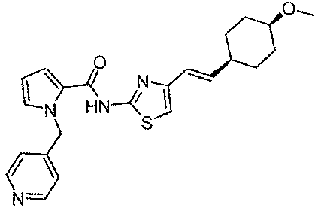 |
| **A255** | 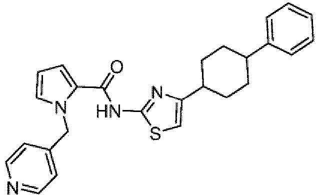 |
| **A256** | 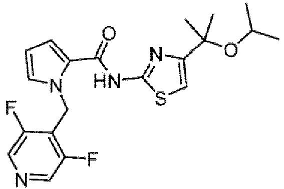 |
| **A257** | 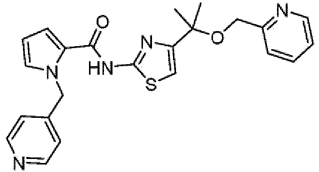 |
| **A258** | 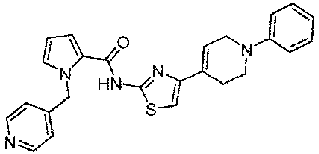 |
| **A259** | 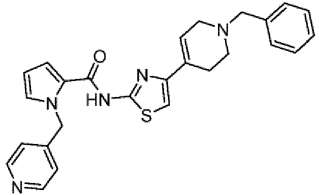 |
| **A260** | 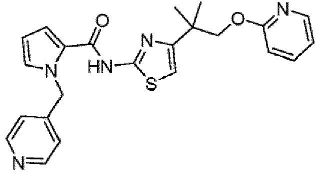 |
| **A261** | 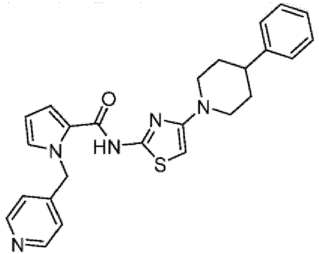 |
| **A262** | 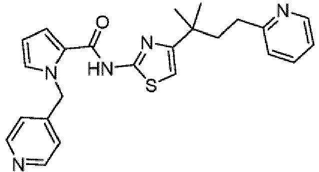 |
| **A263** | 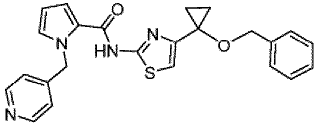 |
| **A264** | 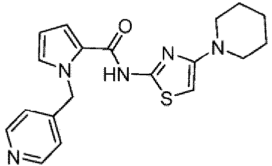 |
| **A265** | 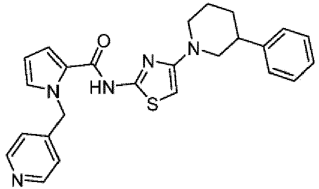 |
| **A266** | 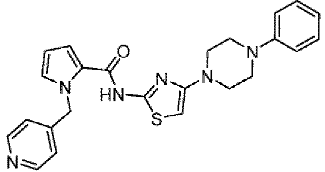 |
| **A267** | 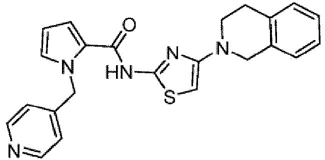 |
| **A268** | 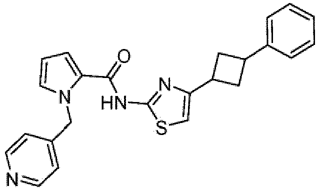 |
| **A269** | 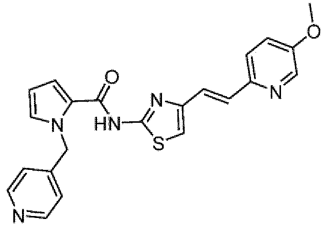 |
| **A270** | 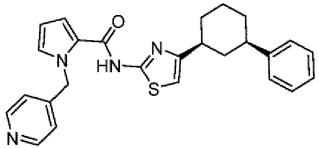 |
| **A271** | 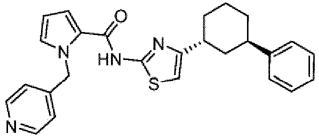 |
| **A272** | 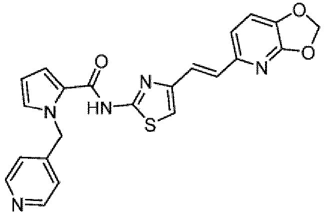 |
| **A273** | 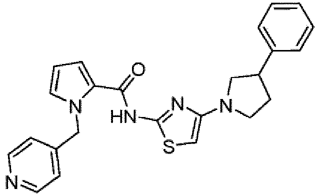 |
| **A274** | 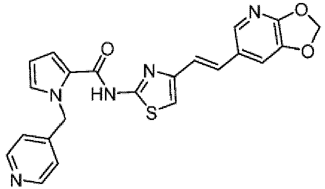 |
| **A275** | 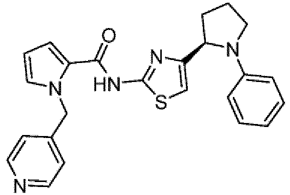 |
| **A276** | 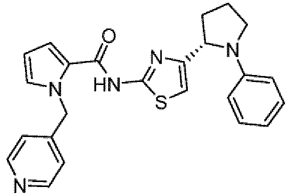 |
| **A277** | 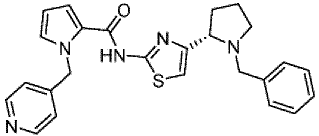 |
| **A278** | 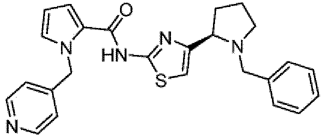 |
| **A279** | 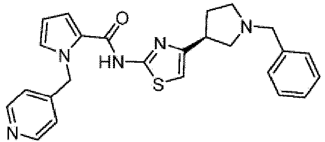 |
| **A280** | 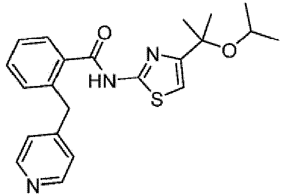 |
| **A281** | 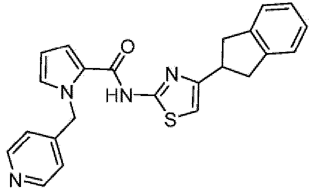 |
| **A282** | 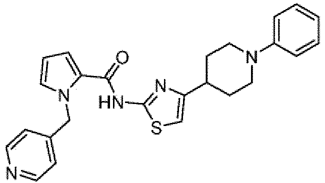 |
| **A283** | 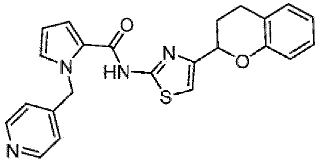 |
| **A284** | 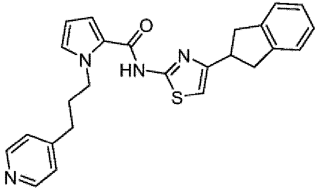 |
| **A285** | 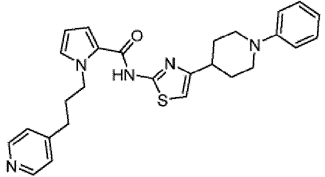 |
| **A286** | 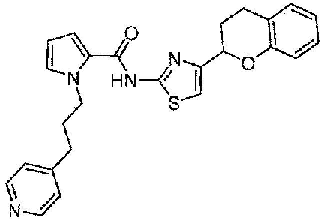 |
| **A287** | 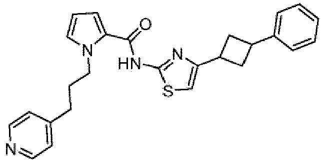 |
| **A288** | 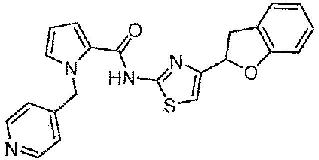 |
| **A289** | 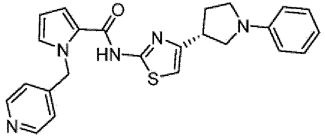 |
| **A290** | 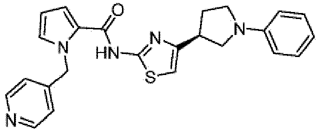 |
| **A291** | 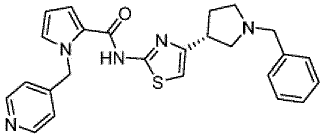 |
| **A292** | 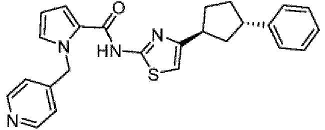 |
| **A293** | 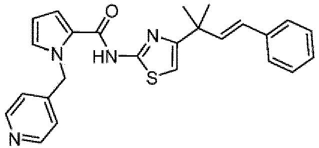 |
| **A294** | 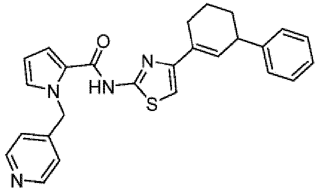 |
| **A295** | 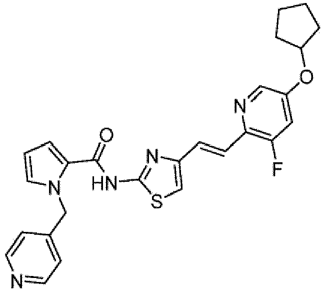 |
| **A296** | 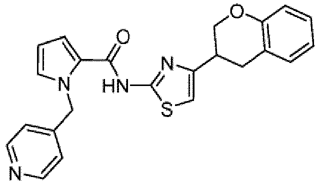 |
| **A297** | 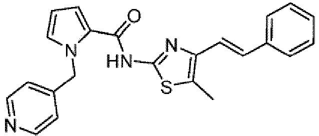 |
| **A298** | 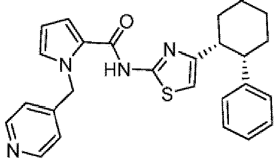 |
| **A299** | 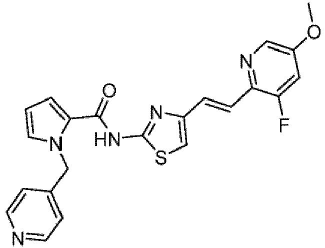 |
| **A300** | 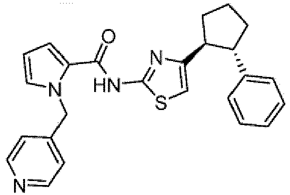 |
| **A301** | 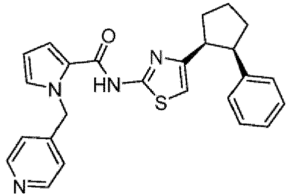 |
| **A302** | 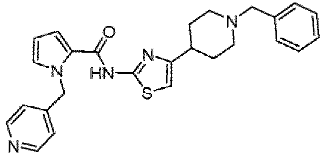 |
| **A303** | 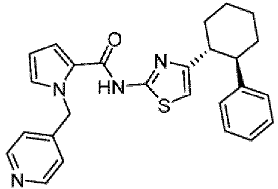 |
| **A304** | 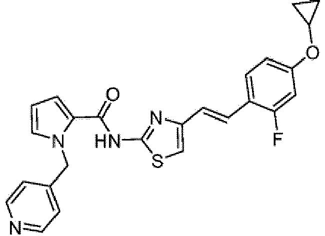 |
| **A305** | 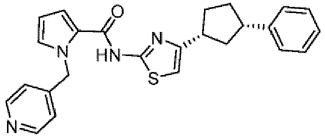 |
| **A306** | 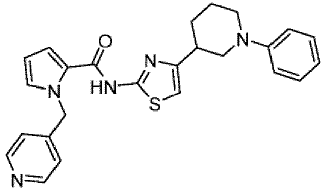 |
| **A307** | 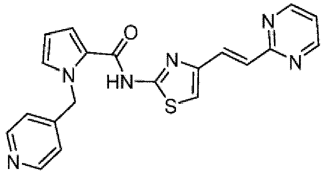 |
| **A308** | 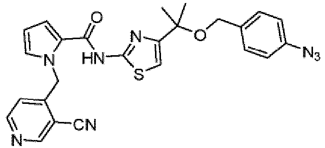 |
| **A309** | 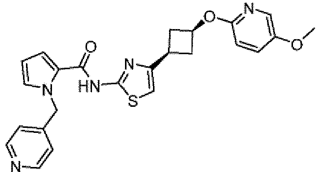 |
| **A310** | 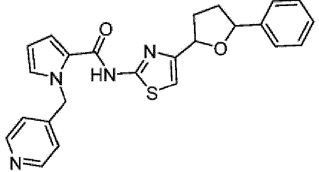 |
| **A311** | 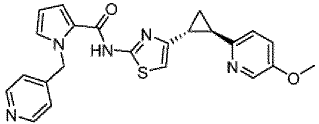 |
| **A312** | 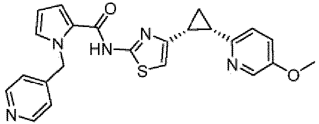 |
| **A313** | 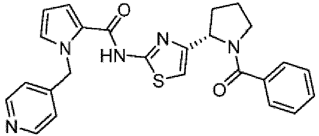 |
| **A314** | 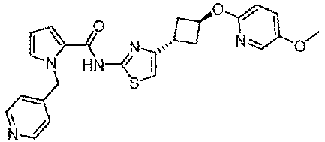 |
| **A315** | 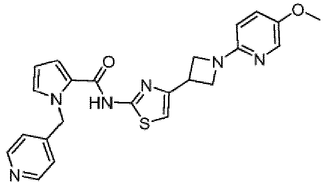 |
| **A316** | 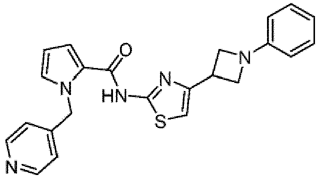 |
| **A317** | 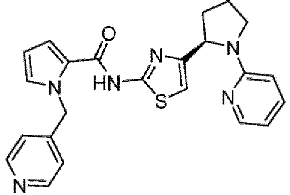 |
| **A318** | 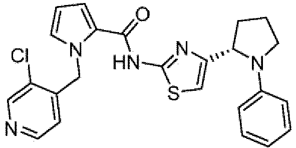 |
| **A319** | 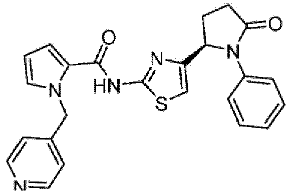 |
| **A320** | 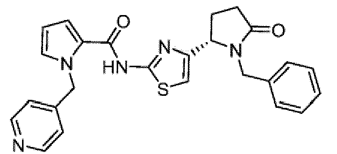 |
| **A321** | 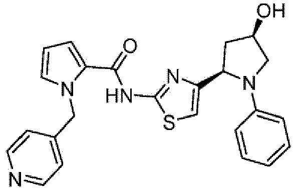 |
| **A322** | 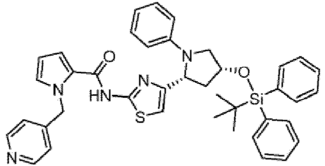 |
| **A323** | 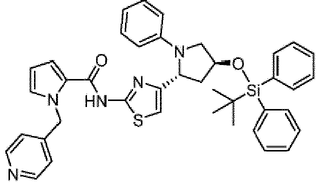 |
| **A324** | 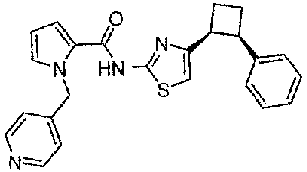 |
| **A325** | 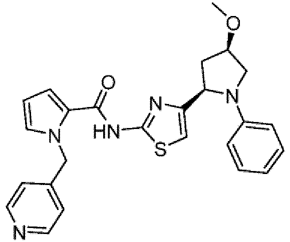 |
| **A326** | 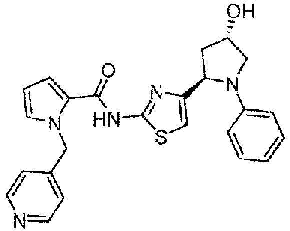 |
| **A327** | 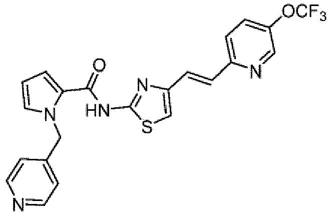 |
| **A328** | 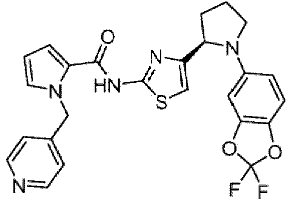 |
| **A329** | 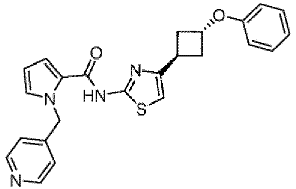 |
| **A330** | 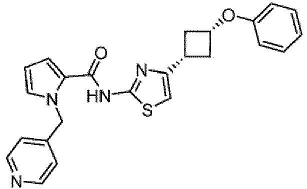 |
| **A331** | 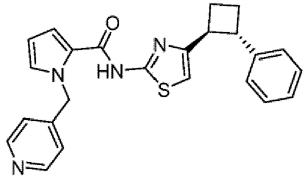 |
| **A332** | 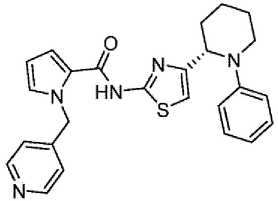 |
| **A333** | 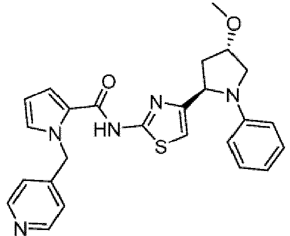 |
| **A334** | 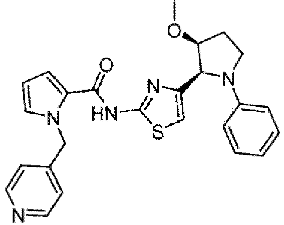 |
| **A335** | 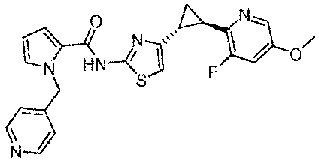 |
| **A336** | 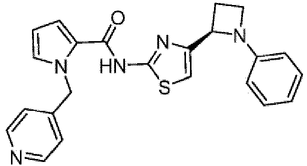 |
| **A337** | 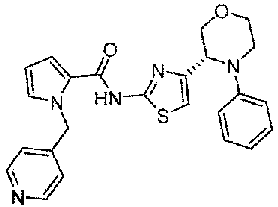 |
| **A338** | 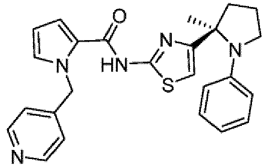 |
| **A339** | 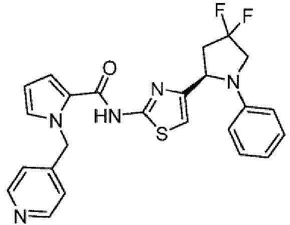 |
| **A340** | 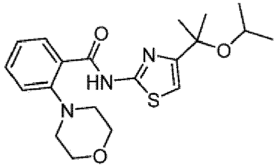 |
| **A341** | 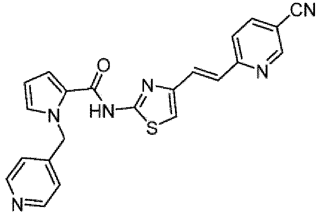 |
| **A342** | 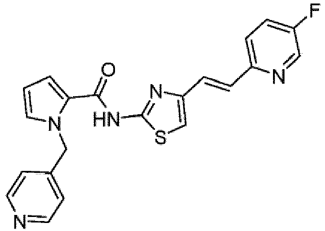 |
| **A343** | 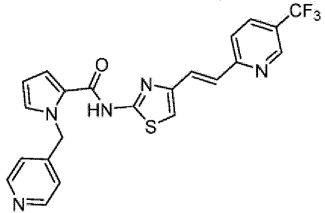 |
| **A344** | 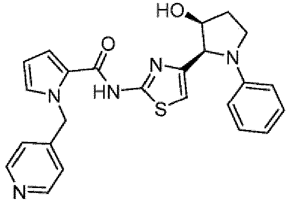 |
| **A345** | 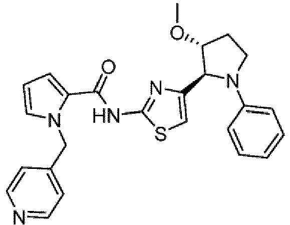 |
| **A346** | 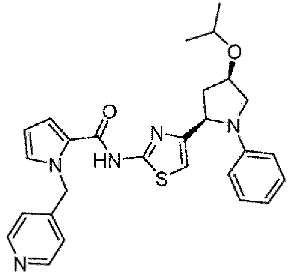 |
| **A347** | 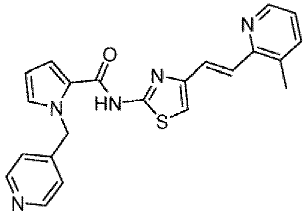 |
| **A348** | 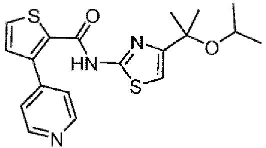 |
| **A349** | 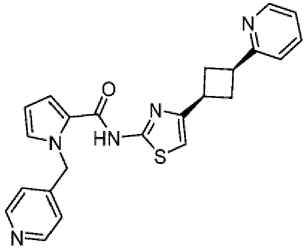 |
| **A350** | 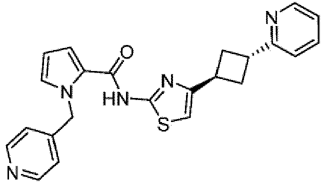 |
| **A351** | 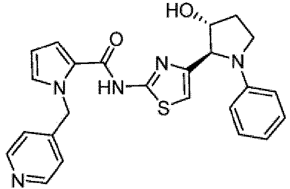 |
| **A352** | 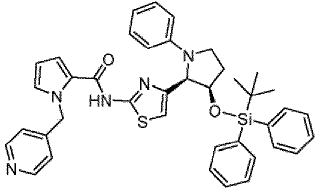 |
| **A353** | 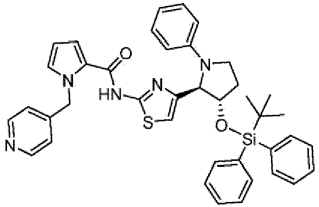 |
| **A354** | 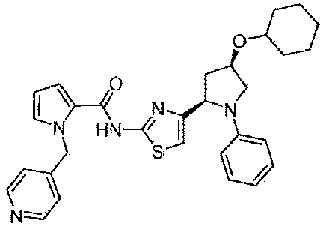 |
| **A355** | 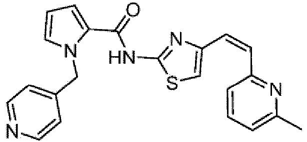 |
| **A356** | 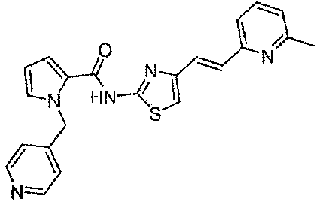 |
| **A357** | 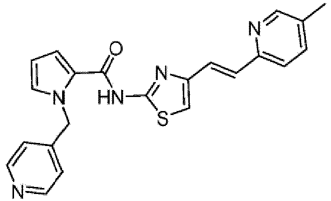 |
| **A358** | 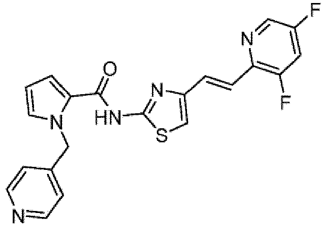 |
| **A359** | 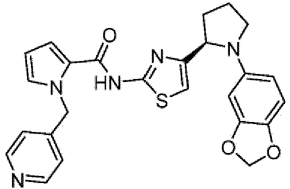 |
| **A360** | 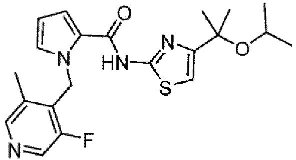 |
| **A361** | 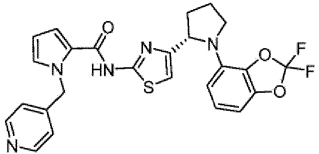 |
| **A362** | 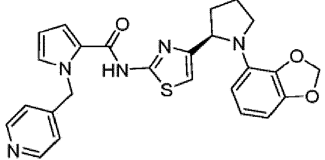 |
| **A363** | 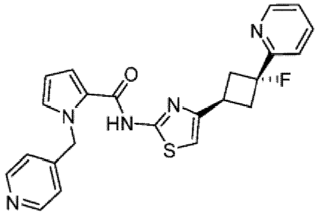 |
| **A364** | 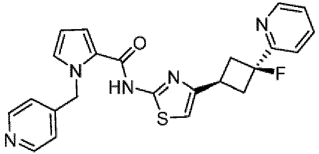 |
| **A356** | 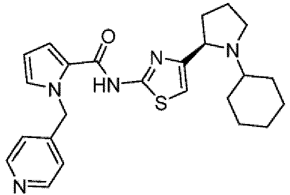 |
| **A366** | 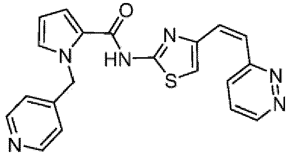 |
| **A367** | 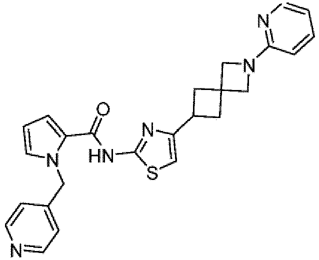 |
| **A368** | 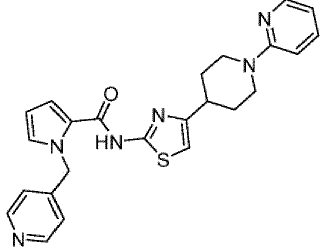 |
| **A369** | 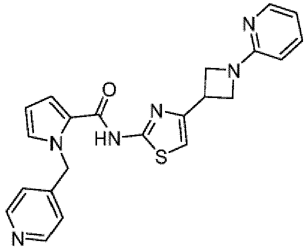 |
| **A370** | 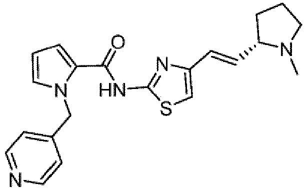 |
| **A371** | 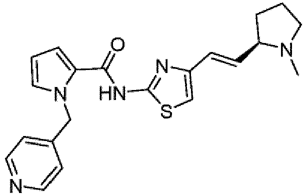 |
| **A372** | 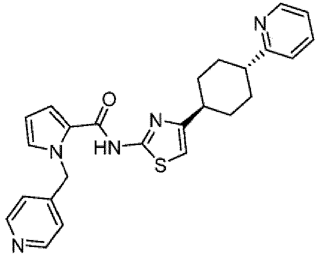 |
| **A373** | 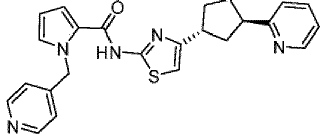 |
| **A374** | 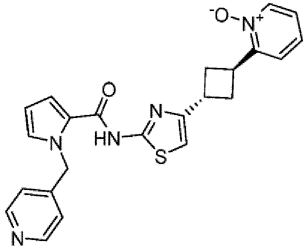 |
| **A375** | 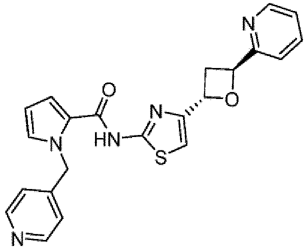 |
| **A376** | 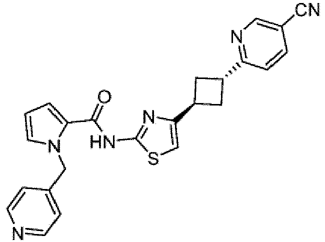 |
| **A377** | 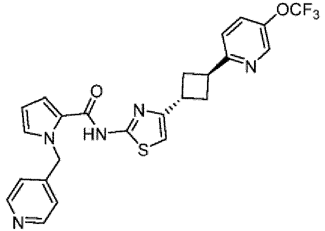 |
| **A378** | 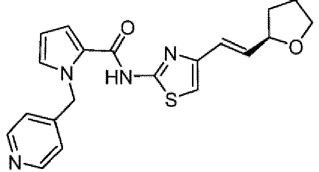 |
| **A379** | 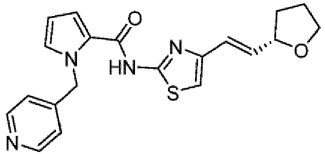 |
| **A380** | 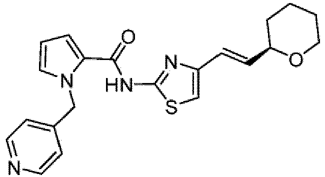 |
| **A381** | 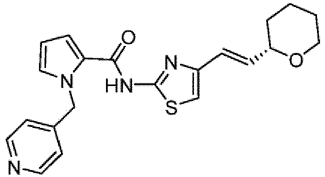 |
| **A382** | 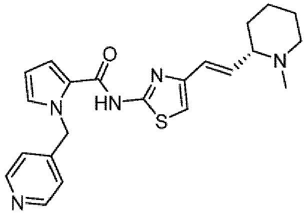 |
| **A383** | 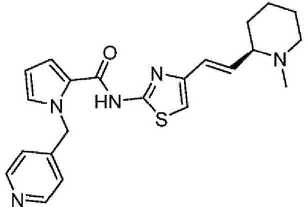 |
| **A384** | 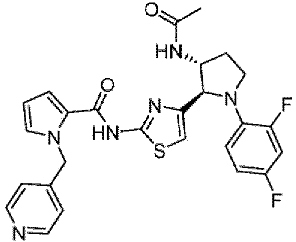 |
| **A385** | 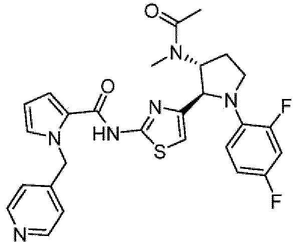 |
| **A386** | 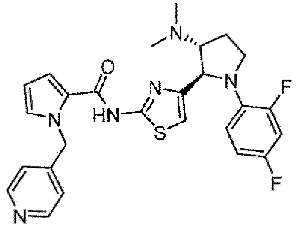 |
| **A387** | 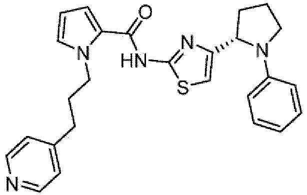 |
| **A388** | 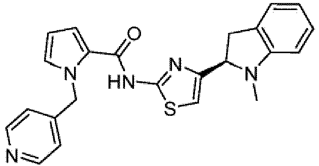 |
| **A389** | 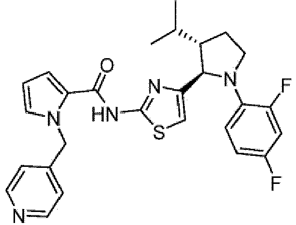 |
| **A390** | 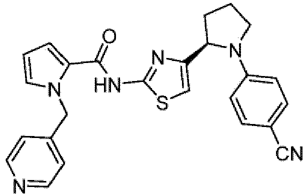 |
| **A391** | 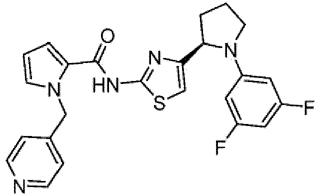 |
| **A392** | 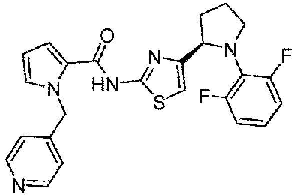 |
| **A393** | 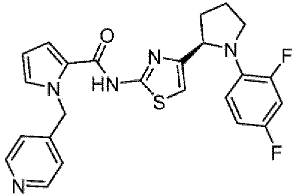 |
| **A394** | 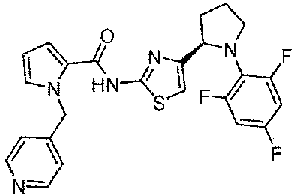 |
| **A395** | 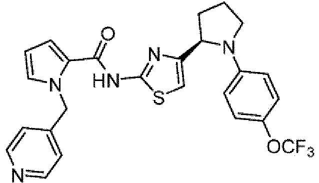 |
| **A396** | 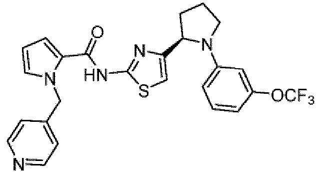 |
| **A397** | 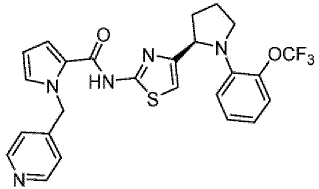 |
| **A398** | 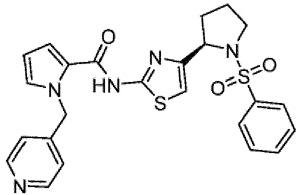 |
| **A399** | 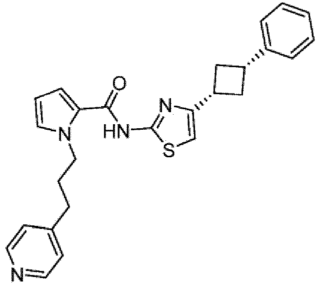 |
| **A400** | 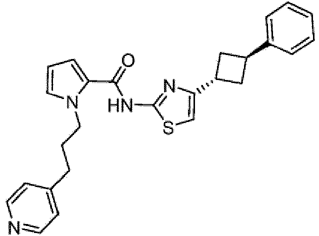 |
| **A401** | 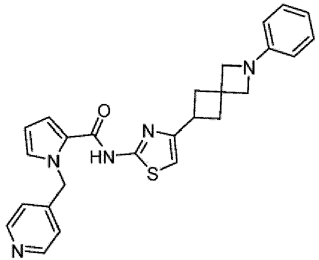 |
| **A402** | 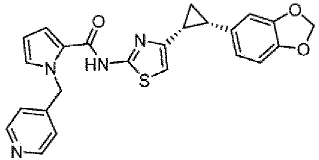 |
| **A403** | 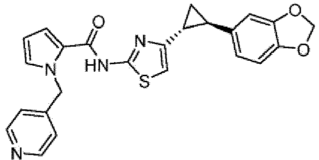 |

12. Composé ou sel selon la revendication 11, le composé ou le sel étant choisi dans le groupe constitué de : 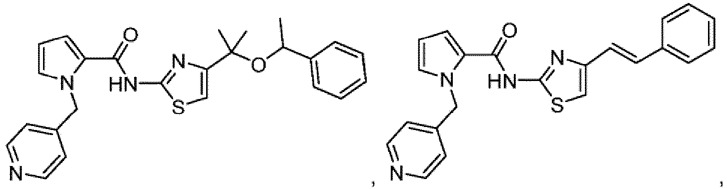 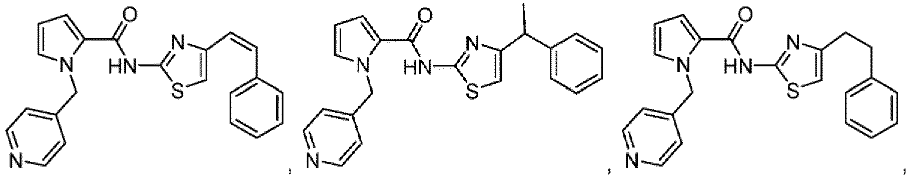 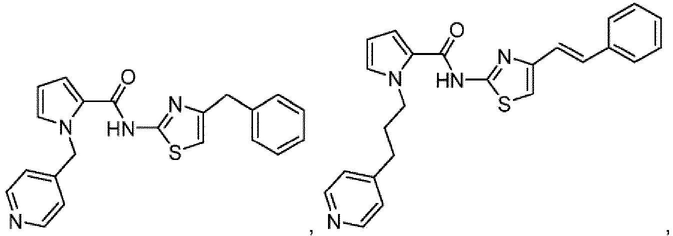 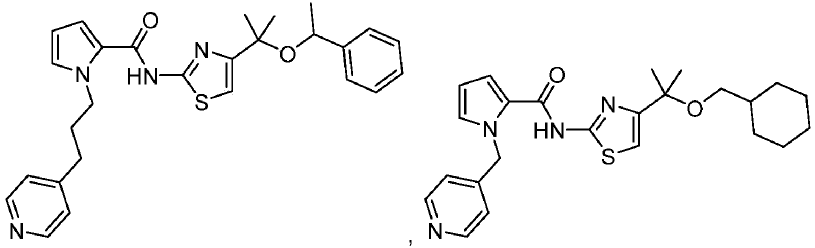 et 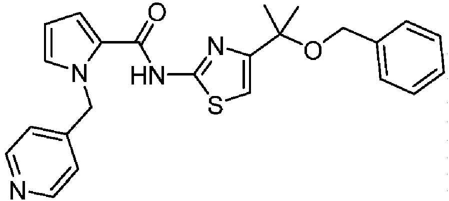 ou
le composé étant choisi dans le groupe constitué de :

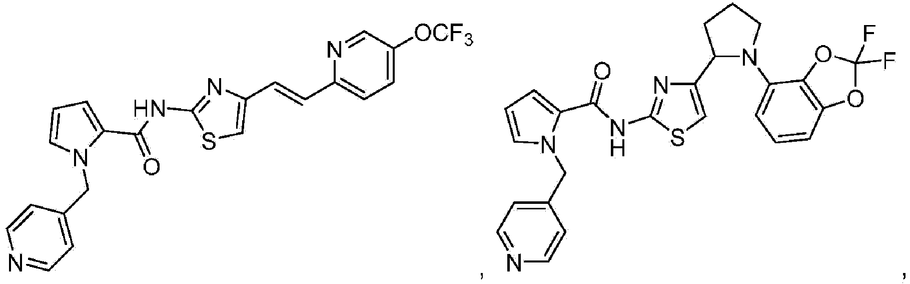

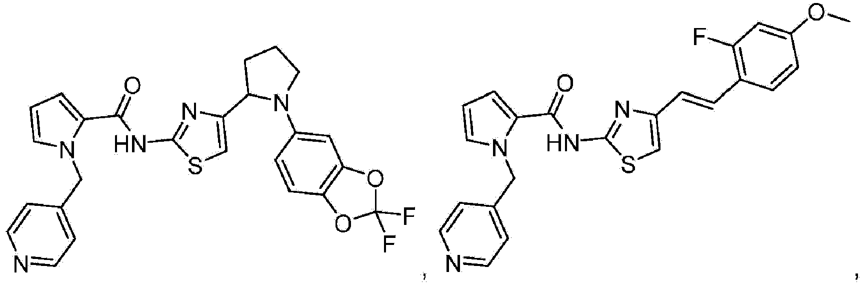

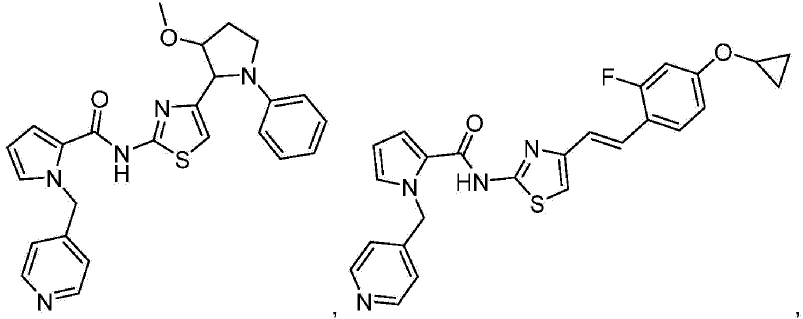

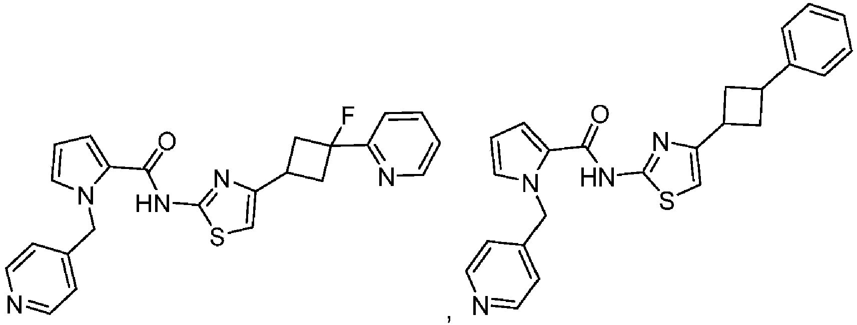

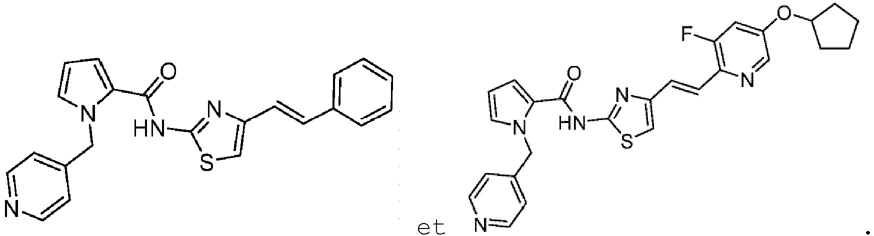

13. Composé ou sel selon l'une quelconque des revendications 1 à 12 destiné à être utilisé dans le traitement de l'inflammation ou d'une maladie auto-immune ou d'une maladie liée à l'immunité ou d'une maladie neurodégénérative ou d'une maladie inflammatoire.

14. Composé ou sel selon l'une quelconque des revendications 1 à 12 destiné à être utilisé dans le traitement du cancer.

15. Composé ou sel destiné à être utilisé selon la revendication 14, le cancer étant un mélanome, un myélome multiple, un cancer de la prostate, un cancer du poumon, un cancer du pancréas, un carcinome épidermoïde, une leucémie, un lymphome, une tumeur neuroendocrine, un cancer de la vessie ou un cancer colorectal ou le cancer est un carcinome du poumon non à petites cellules, un carcinome épidermoïde, une leucémie, une leucémie myéloïde aiguë, une leucémie myéloïde chronique, un lymphome, un lymphome anaplasique à grandes cellules transformé par NPM/ALK, un lymphome diffus à grandes cellules B, des tumeurs neuroendocrines, un cancer du sein, un lymphome à cellules du manteau, un carcinome à cellules rénales, un rhabdomyosarcome, un cancer de l'ovaire, un cancer de l'endomètre, un carcinome à petites cellules, un adénocarcinome, un carcinome gastrique, un carcinome hépatocellulaire, un cancer du pancréas, un carcinome de la thyroïde, un lymphome anaplasique à grandes cellules, un hémangiome ou un cancer de la tête et du cou.

16. Composé ou sel destiné à être utilisé selon la revendication 14, le cancer étant une tumeur solide.
